# EUROPEAN PATENT SPECIFICATION

(11) **EP 4 522 609 B1**
(45) Date of publication and mention of the grant of the patent: **30.09.2026**
(21) Application number: 23726330.6
(22) Date of filing: 10.05.2023
(51) Int. Cl.: C07D 417/14, C07D 413/14, C07D 403/14, A61K 31/506, A61P 29/00, A61P 35/00, A61P 37/00

(54) **PYRROLIDIONE DERIVATIVES AS INHIBITORS OF NF KAPPA B INDUCING KINASE**
PYRROLIDINONDERIVATE ALS INHIBITOREN DER NF KAPPA B INDUZIERENDEN KINASE
DERIVES DER PYRROLIDINONE COMME DES INHIBITEURS DE KINASE INDUISANT NF KAPPA B

(30) Priority: 11.05.2022 US 202263364550 P
(43) Date of publication of application: 19.03.2025
(73) Proprietor: JANSSEN Pharmaceutica NV, 2340 Beerse (BE)
(72) Inventor: MURPHY, Stephen K., San Diego, California 92121-1126 (US); ROVIRA, Alexander R., San Diego, California 92121-1126 (US); MAERTENS, Alexander J., San Diego, California 92121-1126 (US); LEBSACK, Alec D., San Diego, California 92121-1126 (US); WOLIN, Ronald L., San Diego, California 92121-1126 (US); CHAI, Wenying, San Diego, California 92121-1126 (US); MATVIITSUK, Anastassia, San Diego, California 92121-1126 (US)
(74) Representative: Carpmaels & Ransford LLP
(86) International application number: PCT/EP2023/062453
(87) International publication number: WO 2023/217879

(56) References cited:
- WO-A1-2020/239999

## Description

### CROSS-REFERENCE TO RELATED APPLICATIONS

This application claims priority to and the benefits of U.S. Provisional Application No. 63/364,550, filed on May 11, 2022.

### FIELD

The present disclosure relates to the field of chemistry and medicine. More particularly, the present disclosure relates to NF-κB-inducing kinase inhibitors and their use in medical treatment.

### BACKGROUND

NF-κB inducing kinase (NIK) is a serine/threonine kinase transcription factor regulating the expression of various genes involved in immune response disorders. Because of this immune system regulatory role, inhibition of NIK blocks several downstream pathways that produce inflammatory molecules. Clinical validation with biologics has confirmed a key role for several NIK-dependent pathways in autoimmune diseases. See, e.g., S. V. Navarra, et al., The Lancet, 2011;377(9767):721-31. One way to mitigate or eliminate the adverse effects associated with NIK activity is to increase NIK inhibition.

Thus, there is a need to develop effective NIK inhibitors that can be used to treat various diseases.

### SUMMARY

The present application discloses a compound of Formula I: 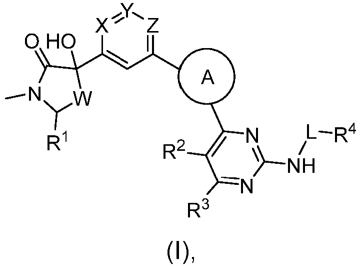 or a pharmaceutically acceptable salt thereof, wherein A, W, X, Y, Z, R¹, R², R³, L, and R⁴ are as defined herein.

The present application also discloses a pharmaceutical composition comprising a compound of Formula I, or a pharmaceutically acceptable salt thereof, and a pharmaceutically acceptable carrier.

The present application also discloses a method for treating a disease, disorder, or medical condition mediated by NIK activity, comprising administering to a subject in need of such treatment an effective amount of (i) a compound of Formula I, or a pharmaceutically acceptable carrier thereof, or (ii) a pharmaceutical composition comprising a compound of Formula I, or a pharmaceutically acceptable salt thereof, and a pharmaceutically acceptable carrier. In some embodiments, the disease, disorder, or medical condition mediated by NIK activity is selected from the group consisting of inflammatory disorders, autoimmune disorders, cancers, metabolic disorders, and osteoporosis. In some embodiments, the disease, disorder, or medical condition mediated by NIK activity is selected from the group consisting of systemic lupus erythematosus ("SLE"), rheumatoid arthritis ("RA"), Sjogren's syndrome, lupus nephritis, inflammatory bowel disease ("IBD"), ANCA associated vasculitis, myositis, IgG4 associated diseases, bullous pemphigoid, neuromyelitis optica spectrum disorders ("NMOSD"), atopic dermatitis "AD"), hidradenitis supperativa ("HS"), steatosis, nonalcoholic steatohepatitis ("NASH"), primary biliary cirrhosis, leukemias, lymphomas, pancreatic cancer, breast cancer, melanoma, obesity, diabetes, acute kidney injury, IgAN, autosomal dominant polycystic kidney disease ("ADCKD"), membranous nephropathy, osteoporosis, bone resorption (periodontitis), multiple sclerosis ("MS"), immune thrombocytopenic purpura, transplantation, myasthenia gravis, scleroderma, myositis, IgG4 associated diseases, and bullous pemphigoid.

### DETAILED DESCRIPTION

Discussion of documents, acts, materials, devices, articles or the like which has been included in the present specification is for the purpose of providing context for the present disclosure. Such discussion is not an admission that any or all of these matters form part of the prior art with respect to any present disclosures disclosed or claimed. In the present description all references to methods for treatment are to be interpreted as directed to compounds for use in such methods.

NF-κB-inducing kinase (referred to as NIK, also known as MAP3K14) is a regulator and driver of the non-canonical NIK cascade, and thus represents an attractive target for therapeutic intervention. Embodiments described herein relate to compounds that inhibit NIK and pharmaceutical compositions comprising such compounds. Compounds described herein and pharmaceutical compositions thereof are useful for preventing or treating diseases such as but not limited to inflammatory disorders and autoimmune disorders.

NIK-dependent transcriptional activation is a tightly controlled signaling pathway, through sequential events including phosphorylation and protein degradation. In a NIK activation pathway, known as a non-canonical pathway, activation is accomplished by phosphorylating the catalytic complex subunit IKKα, leading to the partial proteolysis of the gene product p100, liberating DNA-binding protein p52 which then heterodimerizes with another DNA-binding protein RelB, translocates to the nucleus and mediates gene expression. The non-canonical pathway is activated by ligands such as but not limited to CD40 ligands, B-cell activating factor (BAFF), lymphotoxin β receptor ligands, TNF-related weak inducer of apoptosis (TWEAK) cytokine, and receptor activator of nuclear factor kappa-B ligand (RANKL), also known as tumor necrosis factor ligand superfamily member 11 (TNFSF11). NIK has been shown to be required for activation of the pathway by these ligands (S.-C. Sun, Nat Rev Immunol. 2017, 17(9), 545-558). Because of its role, NIK expression is tightly regulated. Under normal non-stimulated conditions NIK protein levels are very low. This is due to its interaction with baculoviral-IAP-repeat-containing-3 (BIRC3, also known as CIAP2) and a range of TNF receptor associated factors (TRAF2 and TRAF3), which are ubiquitin ligases and result in degradation of NIK. It is believed that when the non-canonical pathway is stimulated by ligands under pathological/abnormal conditions, the activated receptors now compete for TRAFs, dissociating the TRAF-BIRC3-NIK complexes and thereby increasing the levels of NIK (For a more detailed analysis of this background, see e.g., S.-C. Sun (cited above) and Thu and Richmond, Cytokine Growth F. R. 2010, 21, 213-226). NIK plays a role propitiating immune response disorders, so a NIK level increase is undesirable, and one way to mitigate or eliminate the adverse effect associated with such increase is NIK inhibition.

BAFF/BAFF-R is a clinically validated therapeutic target whose inhibition is deemed beneficial for systemic lupus erythematosus (SLE) treatment. Belimumab (anti-BAFF antibody) has been approved to treat serum positive SLE patients (S. V. Navarra, et al., The Lancet, 2011;377(9767):721-31). The CD40L/CD40 pathway plays a key role in T-dependent B cell activation, dendritic cell maturation and tissue inflammation/immunity (R. Elgueta, et al., Immunol. Rev. 2009;229(1):152-72). An anti-CD40L antibody has demonstrated promising efficacy in phase 2 clinical studies in SLE patients (P.I. Sidiropoulos and D.T. Boumpas, Lupus 2004 May;13(5):391-7). Mice lacking NIK (R. Shinkura, et al., Nature Genetics 1999;22(1):74-7; H. D. Brightbill, et al., J Immunol. 2015;195(3):953-64) or conditional knockout of NIK (H. D. Brightbill, et al., J Immunol. 2015;195(3):953-64) or human patients carrying NIK gene mutations (K. L. Willmann, et al., Nature Comm. 2014;5:5360) showed deficiency in NIK non-canonical activation pathways such as but not limited to BAFF and CD40L pathway, reduced B lymphocytes in peripheral blood, and lymphoid organs and lower T cell dependent antibody responses supporting NIK as a therapeutic target for SLE.

NIK has been characterized as being "important in the immune and bonedestructive components of inflammatory arthritis and represents a possible therapeutic target for these diseases." K. Aya, et al. (J. Clin. Invest. 2005, 115, 1848-1854). Mice lacking functional NIK have no peripheral lymph nodes, defective B and T cells, and impaired receptor activator of NIK ligand-stimulated osteoclastogenesis. K. Aya, et al. (J. Clin. Invest. 2005, 115, 1848-1854) investigated the role of NIK in murine models of inflammatory arthritis using NIK-/- mice. The serum transfer arthritis model was initiated by preformed antibodies and required only intact neutrophil and complement systems in recipients. While NIK-/- mice had inflammation equivalent to that of NIK+/+ controls, Ada, *et al.,* (cited above) showed significantly less periarticular osteoclastogenesis and less bone erosion. In contrast, NIK-/- mice were completely resistant to antigen-induced arthritis (AIA), which requires intact antigen presentation and lymphocyte function but not lymph nodes. Additionally, transfer of NIK+/+ splenocytes or T cells to Rag2-/- mice conferred susceptibility to AIA, while transfer of NIK-/- cells did not. NIK-/- mice were also resistant to a genetic, spontaneous form of arthritis, generated in mice expressing both the KRN T cell receptor and H-2g7. Transgenic mice were used with OC-lineage expression of NIK lacking its TRAF3 binding domain (NT3), to demonstrate that constitutive activation of NIK drives enhanced osteoclastogenesis and bone resorption, both in basal conditions and in response to inflammatory stimuli. See Aya, *et al.,* cited above. Furthermore, constitutive activation of NIK drives enhanced osteoclastogenesis and bone resorption, both in basal conditions and in response to inflammatory stimuli. (C. Yang, et al., PLoS ONE 2010, 5(11): e15383, doi:10.1371/journal.pone.0015383).

NIK is also a therapeutic target for other BAFF, CD40L or lymphotoxin β receptor ligands driven autoimmune disorders such as Sjogren's syndrome (J. Groom, et al., J. Clin. Invest. 2002;109(1):59-68) and proliferative lupus glomerulonephritis (D.T. Boumpas, et al., Arthritis & Rheumatism 2003;48(3):719-27).

### Definitions

Unless defined otherwise, all technical and scientific terms used herein have the same meaning as commonly understood to one of ordinary skill in the art to which this present disclosure pertains. Otherwise, certain terms used herein have the meanings as set forth in the specification.

In an attempt to help the reader of the application, the description has been separated in various paragraphs or sections or is directed to various embodiments of the application. These separations should not be considered as disconnecting the substance of a paragraph or section or embodiments from the substance of another paragraph or section or embodiments. To the contrary, one skilled in the art will understand that the description has broad application and encompasses all the combinations of the various sections, paragraphs and sentences that can be contemplated. The discussion of any embodiment is meant only to be exemplary and is not intended to suggest that the scope of the disclosure, including the claims, is limited to these examples.

It must be noted that as used herein and in the appended claims, the singular forms "a," "an," and "the" include plural reference unless the context clearly dictates otherwise.

As used herein, the terms "including," "containing," and "comprising" are used in their open, non-limiting sense.

To provide a more concise description, some of the quantitative expressions given herein are not qualified with the term "about." It is understood that, whether the term "about" is used explicitly or not, every quantity given herein is meant to refer to the actual given value, and it is also meant to refer to the approximation to such given value that would reasonably be inferred based on the ordinary skill in the art, including equivalents and approximations due to the experimental and/or measurement conditions for such given value.

The term "administering" with respect to the methods of the present disclosure, means a method for therapeutically or prophylactically preventing, treating or ameliorating a syndrome, disorder or disease as described herein by using a compound of the disclosure, or pharmaceutically acceptable salt thereof, composition thereof, or medicament thereof. Such methods include administering a therapeutically effective amount of a compound of the disclosure, or pharmaceutically acceptable salt thereof, composition thereof, or medicament thereof, at different times during the course of a therapy or concurrently or sequentially as a combination therapy.

The term "subject" refers to a patient, which may be an animal, preferably a mammal, most preferably a human, whom will be or has been treated by a method according to an embodiment of the application. Examples of mammals include, but are not limited to, cows, horses, sheep, pigs, cats, dogs, mice, rats, rabbits, guinea pigs, non-human primates (NHPs) such as monkeys or apes, humans, etc., more preferably a human.

The term "therapeutically effective amount" or "effective amount" means that amount of active compound or pharmaceutical agent that elicits the biological or medicinal response in a tissue system, animal or human, that is being sought by a researcher, veterinarian, medical doctor, or other clinician, which includes preventing, treating or ameliorating the symptoms of a syndrome, disorder or disease being treated.

As used herein, the term "treatment" or "treating," is defined as the application or administration of a therapeutic agent, i.e., a compound of the present disclosure (alone or in combination with another pharmaceutical agent), to a patient, or application or administration of a therapeutic agent to an isolated tissue or cell line from a patient (e.g., for diagnosis or ex vivo applications), who has a disorder or disease as described herein, a symptom thereof; or the potential to develop such disorder or disease, where the purpose of the application or administration is to cure, heal, alleviate, relieve, alter, remedy, ameliorate, improve or affect the disorder or disease, its symptoms, or the potential to develop said disorder or disease. Such treatments may be specifically tailored or modified, based on knowledge obtained from the field of pharmacogenomics.

As used herein, the term "prevent" or "prevention" means no disorder or disease development if none had occurred, or no further disorder or disease development if there had already been development of the disorder or disease. Also considered is the ability of one to prevent some or all of the symptoms associated with the disorder or disease.

The term "C_{(***a-b)***}" (where *a* and *b* are integers referring to a designated number of carbon atoms) refers, for example, to an alkyl, alkenyl, alkynyl, alkoxy or cycloalkyl radical or to the alkyl portion of a radical in which alkyl appears as the prefix root containing from *a* to *b* carbon atoms inclusive. For example, C₍₁₋₄₎ denotes a radical containing 1, 2, 3 or 4 carbon atoms.

The term "alkyl" is a straight or branched saturated hydrocarbon. For example, an alkyl group can have 1 to 12 carbon atoms (i.e., (C₁-C₁₂)alkyl), 1 to 6 carbon atoms (i.e., (C₁-C₆)alkyl), 1 to 4 carbon atoms (i.e., (C₁-C₄)alkyl), or 1 to 3 carbon atoms (i.e., (C₁-C₃)alkyl). Examples of alkyl groups include, but are not limited to, methyl (Me, - CH₃), ethyl (Et, -CH₂CH₃), 1-propyl (*n*-Pr, *n*-propyl, -CH₂CH₂CH₃), isopropyl (*i*-Pr, *i-*propyl, -CH(CH₃)₂), 1-butyl (*n*-bu, *n*-butyl, -CH₂CH₂CH₂CH₃), 2-butyl (s-bu, s-butyl, - CH(CH₃)CH₂CH₃), *tert*-butyl (*t*-bu, *t*-butyl, -CH(CH₃)₃), 1-pentyl (*n*-pentyl, - CH₂CH₂CH₂CH₂CH₃), 2-pentyl (-CH(CH₃) CH₂CH₂CH₃), neopentyl (-CH₂C(CH₃)₃), 1-hexyl (-CH₂CH₂CH₂CH₂CH₂CH₃), 2-hexyl (-CH(CH₃)CH₂CH₂CH₂CH₃), heptyl (-(CH₂)₆CH₃), octyl (-(CH₂)₇CH₃), 2,2,4-trimethylpentyl (-CH₂C(CH₃)₂CH₂CH(CH₃)₂), nonyl (-(CH₂)₈CH₃), decyl (-(CH₂)₉CH₃), undecyl (-(CH₂)₁₀CH₃), and dodecyl (-(CH₂)₁₁CH₃). In an embodiment, alkyl refers to C₍₁₋₆₎alkyl. In another embodiment, alkyl refers to C₍₁₋₄₎alkyl. In another embodiment, alkyl refers to C₍₁₋₃₎alkyl.

The term "alkylene" refers to a linear or branched saturated divalent hydrocarbon moiety derived from an alkane having 1 to 12 carbon atoms (i.e., (C₁-C₁₂)alkylene), 1 to 6 carbon atoms (i.e., (C₁-C₆)alkylene), 1 to 4 carbon atoms (i.e., (C₁-C₄)alkylene), or 1 to 3 carbon atoms (i.e., (C₁-C₃)alkylene). Examples of alkylene groups include, but are not limited to, methylene (-CH₂-), ethylene (-CH₂CH₂-), -C(CH₃)H-, propylene (-CH₂CH₂CH₂-), isopropylene (-CH(CH₃)CH₂-), and -CH₂CH(CH₃)-. In an embodiment, alkylene refers to C₍₁₋₄₎alkylene. In another embodiment, alkylene refers to C₍₁₋₂₎alkylene.

The term "halo" or "halogen" refers to bromo (-Br), chloro (-Cl), fluoro (-F), or iodo (-I). In an embodiment, halo refers to fluoro.

The term "haloalkyl" refers to a straight- or branched-chain alkyl group having from 1 to 12 carbon atoms, 1 to 6 carbon atoms, 1 to 4 carbon atoms, or 1 to 3 carbon atoms in the chain optionally substituting one or more H with halo. Examples of "haloalkyl" groups include trifluoromethyl (CF₃), difluoromethyl (CF₂H), monofluoromethyl (CH₂F), pentafluoroethyl (CF₂CF₃), tetrafluoroethyl (CHFCF₃), monofluoroethyl (CH₂CH₂F), trifluoroethyl (CH₂CF₃), tetrafluorotrifluoromethylethyl (CF(CF₃)₂), and groups that in light of the ordinary skill in the art and the teachings provided herein would be considered equivalent to any one of the foregoing examples. In an embodiment, haloalkyl refers to C₍₁₋₆₎haloalkyl. In another embodiment, haloalkyl refers to C₍₁₋₄₎haloalkyl. In another embodiment, alkyl refers to C₍₁₋₃₎haloalkyl.

The term "cycloalkyl" refers to a saturated or partially unsaturated all carbon ring system having, for example, 3 to 10 carbon atoms (i.e., C₍₃₋₁₀₎cycloalkyl), 3 to 8 carbon atoms (i.e., C₍₃₋₈₎cycloalkyl), or 3 to 6 carbon atoms (i.e., C₍₃₋₆₎cycloalkyl), wherein the cycloalkyl ring system has a single ring or multiple rings in a fused, spirocyclic, or bridged configuration. Exemplary cycloalkyls include, but are not limited to, cyclopropyl, cyclobutyl, cyclopentyl, cyclohexyl, cycloheptyl, and cyclooctyl. Some cycloalkyl groups may exist as spirocycloalkyls, wherein two cycloalkyl rings are fused through a single carbon atom; for example and without limitation, an example of a spiropentyl group is 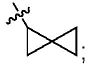 for example and without limitation, examples of spirohexyl groups include 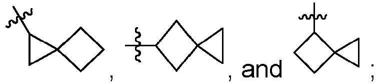 for example and without limitation examples of cycloheptyl groups include 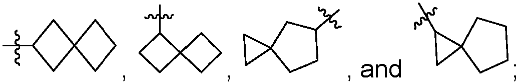 for example and without limitation examples of cyclooctyl groups include 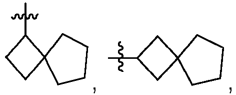 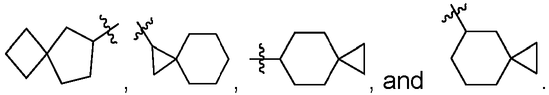 In an embodiment, cycloalkyl refers to C₍₃₋₁₀₎cycloalkyl. In another embodiment, cycloalkyl refers to C₍₃₋₈₎cycloalkyl. In another embodiment, cycloalkyl refers to C₍₃₋₆₎cycloalkyl.

The term "cycloalkylene" refers to a divalent saturated or partially unsaturated all carbon ring system derived from a cycloalkane having, for example, 3 to 10 carbon atoms (i.e., C₍₃₋₁₀₎cycloalkylene), 3 to 8 carbon atoms (i.e., C₍₃₋₈₎cycloalkylene), or 3 to 6 carbon atoms (i.e., C₍₃₋₆₎cycloalkylene), wherein the cycloalkyl ring system has a single ring or multiple rings in a fused, spirocyclic, or bridged configuration. Examples of alkylene groups include, but are not limited to, 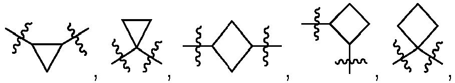 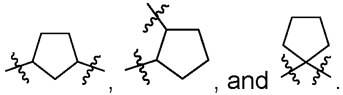 In an embodiment, cycloalkylene refers to C₍₃₋₁₀₎cycloalkylene. In another embodiment, cycloalkylene refers to C₍₃₋₈₎cycloalkylene. In another embodiment, cycloalkyl refers to C₍₃₋₆₎cycloalkylene.

The term "aryl," unless otherwise stated, refers to a polyunsaturated, typically aromatic, hydrocarbon group which can be a single ring or multiple rings (up to three rings) which are fused together or linked covalently. The term aromatic is well known to a person skilled in the art and designates cyclically conjugated systems of 4n + 2 electrons, that is with 6, 10, 14 etc. π-electrons (rule of Hückel). Examples of aryl groups include phenyl, naphthyl, anthracenyl. In an embodiment, aryl refers to C₍₆₋₁₀₎aryl. In another embodiment, aryl refers to phenyl.

The term "heterocyclyl" or "heterocycloalkyl" refers to a single saturated or partially unsaturated ring having 3 to 12 ring members, 3 to 10 ring members, 3 to 8 ring members, or 3 to 6 ring members and which contains carbon atoms and at least one atom other than carbon in the ring, wherein the atom is selected from the group consisting of N, O, and S. The terms "heterocyclyl" and "heterocycloalkyl" include cyclic esters (e.g., lactones) and cyclic amides (e.g., lactams). Exemplary heterocycles include, but are not limited to oxetanyl, aziridinyl, azetidinyl, pyrrolidinyl, pyrrolidonyl, piperidinyl, piperazinyl, morpholinyl, tetrahydropyranyl, tetrahydrofuranyl, and thiomorpholinyl. Unless otherwise noted, the heterocyclyl group is attached to its pendant group at any heteroatom or carbon atom that results in a stable structure. In an embodiment, heterocyclyl refers to 3- to 10-membered heterocyclyl. In another embodiment, heterocyclyl refers to 3- to 8-membered heterocyclyl. In another embodiment, heterocyclyl refers to 3- to 6-membered heterocyclyl.

As used herein, the term "5- to 12-membered bi- or tricyclic ring system containing one or more heteroatoms" refers to a saturated or partially saturated bridged polycyclic, fused polycyclic, or spiro polycyclic ring system having 5 to 12 ring members (or 7 to 12 ring members or 7 to 10 ring members) and which contains carbon atoms and from 1 to 7 heteroatoms, 1 to 5 heteroatoms, 1 to 4 heteroatoms, or 1 to 3 heteroatoms, wherein the heteroatoms are independently selected from the group consisting of N, O, and S. The ring system may include a fully unsaturated aromatic ring; however, at least one other ring in the polycyclic ring system must be saturated or partially saturated. In some embodiments, the term refers to a fused bicyclic ring system. In some embodiments, the term refers to a fused bicyclic ring system wherein one of the rings is an aromatic ring. The term includes cyclic esters (e.g., lactones) and cyclic amides (e.g., lactams). Nonlimiting examples of 5- to 12-membered bi- or tricyclic ring system containing one or more heteroatoms include 3-oxabicyclo[3.1.0]hexyl, indolinyl, 6,7-dihydro-5H-cyclopenta[b]pyridinyl, 4,5,6,7-tetrahydro-1H-indazolyl, 6,7-dihydro-5H-cyclopenta[c]pyridazinyl, 4,5,6,7-tetrahydropyrazolo[1,5-a]pyridinyl, 4,5,6,7-tetrahydropyrazolo[1,5-a]pyrimidinyl, 3,4-dihydro-2H-pyrano[3,2-b]pyridinyl, 3-methyl-6,7-dihydro-5H-pyrazolo[5,1-b][1,3]oxazinyl, and 6,7-dihydropyrazolo[1,5-a]pyrazin-4(5H)-onyl. Unless otherwise noted, the bi- or tricyclic ring system is attached to its pendant group at any heteroatom or carbon atom that results in a stable structure. In an embodiment, a 5- to 12-membered bi- or tricyclic ring system containing one or more heteroatoms refers to a 5- to 12-membered bicyclic ring system containing one or more heteroatoms. In another embodiment, a 5- to 12-membered bi- or tricyclic ring system containing one or more heteroatoms refers to a 7- to 12-membered bicyclic ring system containing one or more heteroatoms. In another embodiment, a 5- to 12-membered bi- or tricyclic ring system containing one or more heteroatoms refers to a 7- to 10-membered bicyclic ring system containing one or more heteroatoms.

The term "heteroaryl" refers to a monocyclic or bicyclic aryl ring system having 5 to 12 ring members, 5 to 10 ring members, or 5 to 6 ring members, and which contains carbon atoms and from 1 to 5 heteroatoms, 1 to 3 heteroatoms, or 1 to 2 heteroatoms, wherein the heteroatoms are independently selected from the group consisting of N, O, and S. Included within the term heteroaryl are aromatic rings of 5 or 6 members wherein the ring consists of carbon atoms and has at least one heteroatom member. Suitable heteroatoms include nitrogen, oxygen, and sulfur. In the case of 5-membered rings, in some embodiments, the heteroaryl ring contains one member of nitrogen, oxygen or sulfur and, in addition, up to 3 additional nitrogens. In the case of 6-membered rings, in some embodiments, the heteroaryl ring contains from 1 to 3 nitrogen atoms. For the case wherein the 6-membered ring has 3 nitrogens, at most 2 nitrogen atoms are adjacent. Examples of heteroaryl groups include furyl, thienyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl, indolyl, isoindolyl, benzofuryl, benzothienyl, indazolyl, benzimidazolyl, benzothiazolyl, benzoxazolyl, benzisoxazolyl, benzothiadiazolyl, benzotriazolyl, quinolinyl, isoquinolinyl, quinazolinyl, pyrazolopyridinyl, and pyrazolopyrimidinyl. Those skilled in the art will recognize that the species of heteroaryl groups listed are not exhaustive, and that additional species within the scope of these defined terms may also be selected. Unless otherwise noted, the heteroaryl is attached to its pendant group at any heteroatom or carbon atom that results in a stable structure. In an embodiment, heteroaryl refers to 5- to 10-membered heteroaryl. In another embodiment, heteroaryl refers to 5- to 8-membered heteroaryl. In another embodiment, heteroaryl refers to 5- to 6-membered heteroaryl. In another embodiment, heteroaryl refers to 5-membered heteroaryl.

The term "substituted" means that the specified group or moiety bears one or more substituents. The term "unsubstituted" means that the specified group bears no substituents. The term "optionally substituted" means that the specified group is unsubstituted or substituted by one or more substituents. Where the term "substituted" is used to describe a structural system, the substitution is meant to occur at any valency-allowed position on the system.

Where the compounds disclosed herein have at least one stereocenter, they may accordingly exist as enantiomers or diastereomers. It is to be understood that all such isomers and mixtures thereof are encompassed within the scope of the present disclosure.

"Diastereoisomers" are stereoisomers that have at least two asymmetric atoms, but which are not mirror images of each other.

"Enantiomers" are a pair of stereoisomers that are non-superimposable mirror images of each other. A "racemic" mixture is a 1:1 mixture of a pair of enantiomers. A "scalemic" mixture of enantiomers is mixture of enantiomers at a ratio other than 1:1.

Where the processes for the preparation of the compounds according to the disclosure give rise to mixture of stereoisomers, these isomers may be separated by conventional techniques such as but not limited to preparative chromatography. The compounds may be prepared in racemic form, a scalemic mixture, or individual enantiomers may be prepared either by enantiospecific synthesis or by resolution. The compounds may, for example, be resolved into their component enantiomers by standard techniques, such as the formation of diastereomeric pairs by salt formation with an optically active acid, such as but not limited to (-)-di-*p*-toluoyl-D-tartaric acid and/or (+)-di-*p*-toluoyl-L-tartaric acid followed by fractional crystallization and regeneration of the free base. The compounds may also be resolved by formation of diastereomeric esters or amides, followed by chromatographic separation and removal of the chiral auxiliary. Alternatively, the compounds may be resolved using a chiral column vial HPLC or SFC. In some instances rotamers of compounds may exist which are observable by ¹H NMR leading to complex multiplets and peak integration in the¹H NMR spectrum.

The absolute stereochemistry is specified according to the Cahn-Ingold-Prelog R-S system. Chiral centers, of which the absolute configurations are known, are labelled by prefixes *R* and *S*, assigned by the standard sequence-rule procedure, and preceded when necessary by the appropriate locants (Pure & Appl. Chem. 45, 1976, 11-30). Certain pairs of enantiomers and diastereomers are presented together in the Examples. These enantiomers/diastereomers may be designated in the following synthetic method and characterized as enantiomer 1 or enantiomer 2 (or, alternately, diastereomer 1 or diastereomer 2). The presentation of stereoisomers in this manner conveys the separate preparation or isolation of the compounds as pure single enantiomers or diastereomers at the identified stereocenter(s). However, unless otherwise specified, when a pure single enantiomer (or diastereomer) is presented together with the corresponding pure single enantiomer (or diastereomer) in the Examples of the present disclosure, the order in which the chemical structures/IUPAC names are presented do not necessarily correspond to the order in which the Example numbers are listed. By way of example, where R and S enantiomers of a compound are presented side-by-side under the header "Example X and Example Y," then Example X may be either the R enantiomer or the S enantiomer, and Example Y is the opposite enantiomer, regardless of the order in which the IUPAC names or chemical structures of the compounds are presented, unless otherwise specified in the method and characterization that follows.

Pseudoasymmetric stereogenic centers are treated in the same way as chiral centers, but are given lower-case symbols, *r* or *s* (Angew. Chem. Int. Ed. Engl. 1982, 21, 567-583).

During any of the processes for preparation of the compounds disclosed herein, it may be necessary and/or desirable to protect sensitive or reactive groups on any of the molecules concerned. This may be achieved by means of conventional protecting groups, such as but not limited to those described in Protective Groups in Organic Chemistry, ed. J.F.W. McOmie, Plenum Press, 1973; and T.W. Greene & P.G.M. Wuts, Protective Groups in Organic Synthesis, John Wiley & Sons, 1991. The protecting groups may be removed at a convenient subsequent stage using methods known from the art.
Furthermore, it is intended that within the scope of the present disclosure, any element, in particular when mentioned in relation to a compound of the disclosure, or pharmaceutically acceptable salt thereof, shall comprise all isotopes and isotopic mixtures of said element, either naturally occurring or synthetically produced, either with natural abundance or in an isotopically enriched form. For example, a reference to hydrogen or "H" includes within its scope ¹H, ²H (*i.e*., deuterium or D), and ³H (i.e., tritium or T). In some embodiments, the compounds described herein include a ²H (*i.e*., deuterium) isotope. By way of example, the group denoted -C₍₁₋₆₎alkyl includes not only -C¹H₃, but also C¹HD₂, C¹H₂D, CD₃ and other isotopic forms; not only C¹H₂C¹H₃, but also C¹HDC¹HD₂, C¹HDC¹H₂D, C¹H₂C¹H₃, CD₂CD₃, *etc.* Likewise, where nonexplicit hydrogen atoms are present in a chemical structure, those hydrogen atoms may be¹H, ²H (*i.e.*, deuterium or D), or ³H (i.e., tritium or T). By way of illustration, the group 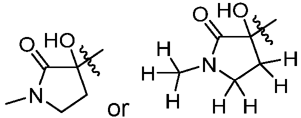 also encompasses 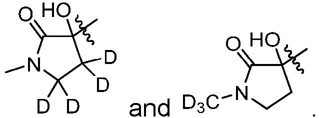 Similarly, references to carbon and oxygen include within their scope respectively ¹²C, ¹³C and ¹⁴C and ¹⁵O and ¹⁶O and ¹⁷O and ¹⁸O. The isotopes may be radioactive or nonradioactive. Radiolabelled compounds of the disclosure may include a radioactive isotope selected from the group comprising ³H, ¹¹C, ¹⁸F, ³⁵S, ¹²²I, ¹²³I, ¹²⁵I, ¹³¹I, ⁷⁵Br, ⁷⁶Br, ⁷⁷Br and ⁸²Br. In some embodiments, the radioactive isotope is selected from the group of ³H, ¹¹C and ¹⁸F.

Reference to a compound herein stands for a reference to any one of: (a) the actually recited form of such compound, and (b) any of the forms of such compound in the medium in which the compound is being considered when named. For example, reference herein to a compound such as but not limited to R-COOH, encompasses reference to any one of, for example, R-COOH₍ₛ₎, R-COOH₍ₛₒₗ₎, and R-COO⁻₍ₛₒₗ₎. In this example, R-COOH₍ₛ₎ refers to the solid compound, as it could be for example in a tablet or some other solid pharmaceutical composition or preparation; R-COOH₍ₛₒₗ₎ refers to the undissociated form of the compound in a solvent; and R-COO⁻₍ₛₒₗ₎ refers to the dissociated form of the compound in a solvent, such as but not limited to the dissociated form of the compound in an aqueous environment, whether such dissociated form derives from R-COOH, from a salt thereof, or from any other entity that yields R-COO⁻ upon dissociation in the medium being considered. In another example, an expression such as "exposing an entity to compound of formula R-COOH" refers to the exposure of such entity to the form, or forms, of the compound R-COOH that exists, or exist, in the medium in which such exposure takes place. In still another example, an expression such as "reacting an entity with a compound of formula R-COOH" refers to the reacting of (a) such entity in the chemically relevant form, or forms, of such entity that exists, or exist, in the medium in which such reacting takes place, with (b) the chemically relevant form, or forms, of the compound R-COOH that exists, or exist, in the medium in which such reacting takes place. In this regard, if such entity is for example in an aqueous environment, it is understood that the compound R-COOH is in such same medium, and therefore the entity is being exposed to species such as but not limited to R-COOH_{(aq)} and/or R-COO⁻_{(aq)}, where the subscript "(aq)" stands for "aqueous" according to its conventional meaning in chemistry and biochemistry. A carboxylic acid functional group has been chosen in these nomenclature examples; this choice is not intended, however, as a limitation but it is merely an illustration. It is understood that analogous examples can be provided in terms of other functional groups, including but not limited to hydroxyl, basic nitrogen members, such as those in amines, and any other group that interacts or transforms according to known manners in the medium that contains the compound. Such interactions and transformations include, but are not limited to, dissociation, association, tautomerism, solvolysis, including hydrolysis, solvation, including hydration, protonation, and deprotonation. No further examples in this regard are provided herein because these interactions and transformations in a given medium are known by any one of ordinary skill in the art.

The term "pharmaceutically acceptable" means approved or approvable by a regulatory agency of Federal or a state government or the corresponding agency in countries other than the United States, or that is listed in the U. S. Pharmcopoeia or other generally recognized pharmacopoeia for use in animals, and more particularly, in humans.

A "pharmaceutically acceptable salt" is intended to mean a salt of a free acid or base of a compound disclosed herein that is non-toxic, biologically tolerable, or otherwise biologically suitable for administration to the subject. It should possess the desired pharmacological activity of the parent compound. See, generally, G.S. Paulekuhn, et al., "Trends in Active Pharmaceutical Ingredient Salt Selection based on Analysis of the Orange Book Database", J. Med. Chem., 2007, 50:6665-72, S.M. Berge, et al., "Pharmaceutical Salts", J Pharm Sci., 1977, 66:1-19, and Handbook of Pharmaceutical Salts, Properties, Selection, and Use, Stahl and Wermuth, Eds., Wiley-VCH and VHCA, Zurich, 2002**.** Examples of pharmaceutically acceptable salts are those that are pharmacologically effective and suitable for contact with the tissues of patients without undue toxicity, irritation, or allergic response. A compound of the disclosure may possess a sufficiently acidic group, a sufficiently basic group, or both types of functional groups, and accordingly react with a number of inorganic or organic bases, and inorganic and organic acids, to form a pharmaceutically acceptable salt.

### Compounds of the Disclosure

The present application discloses a compound of Formula I: 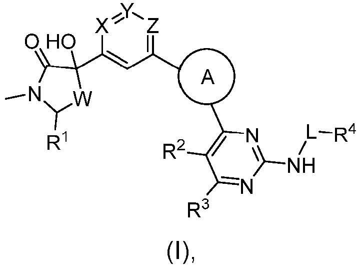 or a pharmaceutically acceptable salt thereof, wherein:
A is a 5- to 6-membered heteroaryl that is optionally substituted with one to three -C₍₁₋₄₎alkyl groups;
W is CH₂, CHF, or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein the -C₍₁₋₆₎alkyl, -C₍₃₋₁₀₎cycloalkyl, 3- to 10-membered heterocyclyl, 5- to 12-membered bi- or tricyclic ring system containing one or more heteroatoms, -C(O)-(3- to 10-membered heterocyclyl), -C₍₆₋₁₀₎aryl, and 5- to 10-membered heteroaryl are each optionally substituted with one to five R^{4x} groups;
each R^{4x}, independently for each occurrence, is halo, -OH, -N(R^{N1})(R^{N2}), -CN, - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, - C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C(₁₋₄₎alkyl; and
R^{N1} and R^{N2} are each independently for each occurrence H, -C₍₁₋₃₎alkyl, or -C₍₁₋₃₎haloalkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one -C₍₁₋₄₎alkyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, -C(O)-(3- to 10-membered heterocyclyl), -C₍₆₋₁₀₎aryl, or 5- to 10-membered heteroaryl, each of which is optionally substituted with one to five R^{4x} groups;
each R^{4x}, independently for each occurrence, is halo, -OH, -N(R^{N1})(R^{N2}), -CN, - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one methyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, -C(O)-(3- to 10-membered heterocyclyl), -C₍₆₋₁₀₎aryl, or 5- to 10-membered heteroaryl, each of which is optionally substituted with one to five R^{4x} groups;
each R^{4x}, independently for each occurrence, is halo, -OH, -N(R^{N1})(R^{N2}), -CN, - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -C₍₁₋₄₎alkyl, -C₍₁₋₄₎haloalkyl, - OC₍₁₋₄₎alkyl, -OC₍₁₋₆₎haloalkyl, and -C(O)N(R^{N1})(R^{N2}); and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one methyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, each of which is optionally substituted with one to five R^{4x} groups;
each R^{4x}, independently for each occurrence, is halo, -OH, -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, 3- to 8-membered heterocyclyl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, and 3- to 8-membered heterocyclyl, are optionally further substituted with one to five groups selected from halo, -OH, -C₍₁₋₄₎alkyl, -C₍₁₋₄₎haloalkyl, -OC₍₁₋₄₎alkyl, -OC₍₁₋₆₎haloalkyl, and - C(O)N(R^{N1})(R^{N2}); and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one to three -C₍₁₋₄₎alkyl groups;
W is CH₂, CHF, or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R' is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups;
R^{3a}, R^{3b}, R^{3c}, R^{3d}, R^{3e}, R^{3f} and R^{3g} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, - OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H, -C₍₁₋₃₎alkyl, or -C₍₁₋₃₎haloalkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one to three -C₍₁₋₄₎alkyl groups;
W is CH₂, or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R' is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₆₋₁₀₎aryl, or 5- to 10-membered heteroaryl, wherein the -C₍₁₋₆₎haloalkyl, - C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
R^{4b}, R^{4c}, R^{4d}, and R^{4e} are each independently for each occurrence halo, -OH, - N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
R^{4f} and R^{4g} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, - C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one to three -C₍₁₋₄₎alkyl groups;
W is CH₂, or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R' is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₆₋₁₀₎aryl, or 5- to 10-membered heteroaryl, wherein the -C₍₁₋₆₎haloalkyl, - C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
R^{4b}, R^{4c}, and R^{4d} are each independently for each occurrence halo, -OH, - N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one to three -C₍₁₋₄₎alkyl groups;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, or 3- to 8-membered heterocyclyl, wherein the -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, and 3- to 8-membered heterocyclyl are optionally further substituted with one to five groups selected from halo, -OH, -N(R^{N1})(R^{N2}), -CN, -C₍₁₋₄₎alkyl, and - OC₍₁₋₄₎alkyl;
R^{4b}, R^{4c}, R^{4d}, and R^{4e} are each independently for each occurrence halo, -OH, - N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, 3- to 8-membered heterocyclyl, and 5- to 10-membered heteroaryl are optionally further substituted with one to five groups selected from halo, -OH, - N(R^{N1})(R^{N2}), -CN, -C₍₁₋₄₎alkyl, and -OC₍₁₋₄₎alkyl;
R^{4f} and R^{4g} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one -C₍₁₋₄₎alkyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, or 3- to 8-membered heterocyclyl, wherein the -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, and 3- to 8-membered heterocyclyl are optionally further substituted with one to five groups selected from halo, -OH, -N(R^{N1})(R^{N2}), -CN, -C₍₁₋₄₎alkyl, and - OC₍₁₋₄₎alkyl;
R^{4b}, R^{4c}, and R^{4d} are each independently for each occurrence halo, -OH, - N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, 3- to 8-membered heterocyclyl, and 5- to 10-membered heteroaryl are optionally further substituted with one to five groups selected from halo, -OH, - N(R^{N1})(R^{N2}), -CN, -C₍₁₋₄₎alkyl, and -OC₍₁₋₄₎alkyl;
each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one -C₍₁₋₄₎alkyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R' is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -C(O)N(R^{N1})(R^{N2}), -S(O)₂C₍₁₋₄₎alkyl, -N(H)S(O)₂C₍₁₋₄₎alkyl, or 3- to 8-membered heterocyclyl, wherein the -C₍₁₋₆₎haloalkyl, -C₍₃₋₈₎cycloalkyl, -OC₍₁₋₆₎alkyl, -OC₍₁₋₆₎haloalkyl, and 3- to 8-membered heterocyclyl are optionally further substituted with one to five groups selected from halo, -OH, -N(R^{N1})(R^{N2}), -CN, -C₍₁₋₄₎alkyl, and -OC₍₁₋₄₎alkyl;
each R^{4b} independently for each occurrence is halo, -OH, -N(R^{N1})(R^{N2}), -CN, -C₍₁₋₆₎alkyl, -C₍₁₋₆₎haloalkyl, -OC₍₁₋₆₎alkyl, -OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo, -OH, -N(R^{N1})(R^{N2}), and -CN;
R^{4c} and R^{4d} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, - N(R^{N1})(R^{N2}), -CN, -C₍₁₋₄₎alkyl, and -OC₍₁₋₄₎alkyl;
each R^{4e} independently for each occurrence is halo, -OH, -N(R^{N1})(R^{N2}), -CN, -C₍₁₋₆₎alkyl, -C₍₁₋₆₎haloalkyl, -OC₍₁₋₆₎alkyl, or -OC₍₁₋₆₎haloalkyl;
each R^{4f} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, or -N(H)S(O)₂C₍₁₋₄₎alkyl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, and -OC₍₃₋₈₎cycloalkyl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, and -OC₍₁₋₆₎haloalkyl;
each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, - OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, - N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one -C₍₁₋₄₎alkyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -C(O)N(R^{N1})(R^{N2}), -S(O)₂C₍₁₋₄₎alkyl, -N(H)S(O)₂C₍₁₋₄₎alkyl, or 3- to 8-membered heterocyclyl, wherein the -C₍₁₋₆₎haloalkyl, -C₍₃₋₈₎cycloalkyl, -OC₍₁₋₆₎alkyl, -OC₍₁₋₆₎haloalkyl, and 3- to 8-membered heterocyclyl are optionally further substituted with one to five groups selected from halo, -OH, -N(R^{N1})(R^{N2}), -CN, -C₍₁₋₄₎alkyl, and -OC₍₁₋₄₎alkyl;
each R^{4b} independently for each occurrence is halo, -OH, -N(R^{N1})(R^{N2}), -CN, -C₍₁₋₆₎alkyl, -C₍₁₋₆₎haloalkyl, -OC₍₁₋₆₎alkyl, -OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo, -OH, -N(R^{N1})(R^{N2}), and -CN;
R^{4c} and R^{4d} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, - N(R^{N1})(R^{N2}), -CN, -C₍₁₋₄₎alkyl, and -OC₍₁₋₄₎alkyl;
each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, - OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, - N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

In some embodiments, disclosed herein is a compound of Formula I, or a pharmaceutically acceptable salt thereof wherein:
A is a 5- to 6-membered heteroaryl that is optionally substituted with one methyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R' is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to three -C₍₁₋₄₎alkyl groups, the -C₍₆₋₁₀₎aryl is optionally substituted with one to five R^{4f} groups, and the 5- to 10-membered heteroaryl is optionally substituted with one to five R^{4g} groups;
each R^{4a} independently for each occurrence is halo, -OH, -C₍₁₋₆₎haloalkyl, -C₍₃₋₈₎cycloalkyl, -OC₍₁₋₆₎alkyl, -OC₍₁₋₆₎haloalkyl, -S(O)₂C₍₁₋₄₎alkyl, -N(H)S(O)₂C₍₁₋₄₎alkyl, or 3-to 8-membered heterocyclyl, wherein the -C₍₁₋₆₎haloalkyl, -C₍₃₋₈₎cycloalkyl, -OC₍₁₋₆₎alkyl, - OC₍₁₋₆₎haloalkyl, and 3- to 8-membered heterocyclyl are optionally further substituted with one to five groups selected from halo and -OH;
each R^{4b} independently for each occurrence is halo, -OH, -CN, -C₍₁₋₆₎alkyl, -C₍₁₋₆₎haloalkyl, -OC₍₁₋₆₎alkyl, -OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo and -OH;
R^{4c} and R^{4d} are each independently for each occurrence -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, and -C₍₁₋₄₎alkyl;
each R^{4f} independently for each occurrence is halo, -OH, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, or -C(O)N(R^{N1})(R^{N2}), wherein the -C₍₁₋₆₎alkyl, -C₍₁₋₆₎haloalkyl, -C₍₃₋₈₎cycloalkyl, - OC₍₁₋₆₎alkyl, -OC₍₁₋₆₎haloalkyl, and -OC₍₃₋₈₎cycloalkyl are optionally further substituted with one to five groups selected from halo and -OH;
each R^{4g} independently for each occurrence is halo, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)N(R^{N1})(R^{N2}), 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, -C₍₁₋₄₎alkyl, -C₍₁₋₄₎haloalkyl, -OC₍₁₋₄₎alkyl, -OC₍₁₋₆₎haloalkyl, and - C(O)N(R^{N1})(R^{N2}); and
R^{N1} and R^{N2} are each independently for each occurrence H or -C₍₁₋₃₎alkyl;
provided that at least two of X, Y, and Z are C-H.

In some embodiments, disclosed herein is a compound of Formula I, or a pharmaceutically acceptable salt thereof wherein:
A is a 5- to 6-membered heteroaryl that is optionally substituted with one methyl group;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen or -C₍₁₋₄₎alkyl;
L is absent, -C₍₁₋₄₎alkylene or -C₍₃₋₆₎cycloalkylene, wherein the -C₍₁₋₄₎alkylene is optionally substituted with one -OC₍₁₋₃₎alkyl group;
R⁴ is-C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, and the 5- to 10-membered heteroaryl is optionally substituted with one to five R^{4g} groups;
each R^{4a} independently for each occurrence is halo, -OH, -C₍₁₋₆₎haloalkyl, -C₍₃₋₈₎cycloalkyl, -OC₍₁₋₆₎alkyl, -OC₍₁₋₆₎haloalkyl, -S(O)₂C₍₁₋₄₎alkyl, -N(H)S(O)₂C₍₁₋₄₎alkyl, or 3-to 8-membered heterocyclyl, wherein the -C₍₁₋₆₎haloalkyl, -C₍₃₋₈₎cycloalkyl, -OC₍₁₋₆₎alkyl, - OC₍₁₋₆₎haloalkyl, and 3- to 8-membered heterocyclyl are optionally further substituted with one to five groups selected from halo and -OH;
each R^{4b} independently for each occurrence is halo, -OH, -CN, -C₍₁₋₆₎alkyl, -C₍₁₋₆₎haloalkyl, -OC₍₁₋₆₎alkyl, -OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo and -OH;
R^{4c} and R^{4d} are each independently for each occurrence -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, and -C₍₁₋₄₎alkyl;
each R^{4g} independently for each occurrence is halo, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)N(R^{N1})(R^{N2}), 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, -C₍₁₋₄₎alkyl, -C₍₁₋₄₎haloalkyl, -OC₍₁₋₄₎alkyl, -OC₍₁₋₆₎haloalkyl, and - C(O)N(R^{N1})(R^{N2}); and
R^{N1} and R^{N2} are each independently for each occurrence H or -C₍₁₋₃₎alkyl;
provided that at least two of X, Y, and Z are C-H.

In some embodiments, disclosed herein is a compound of Formula I, or a pharmaceutically acceptable salt thereof, which is a compound of Formula la-1:

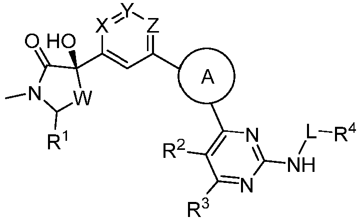

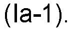

In some embodiments, disclosed herein is a compound of Formula I, or a pharmaceutically acceptable salt thereof, which is a compound of Formula la-2:

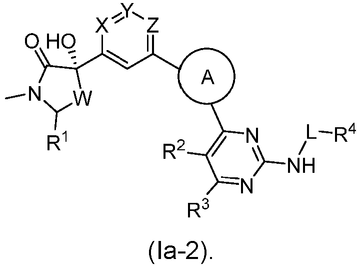

In some embodiments, disclosed herein is a compound of Formula I, or a pharmaceutically acceptable salt thereof, wherein A is a 5-membered heteroaryl that is optionally substituted with one to three groups -C₍₁₋₄₎alkyl groups. In some embodiments, disclosed herein is a compound of Formula I, or a pharmaceutically acceptable salt thereof, wherein A is a 5-membered heteroaryl that is optionally substituted with one methyl group. In some embodiments, disclosed herein is a compound of Formula I, or a pharmaceutically acceptable salt thereof, wherein A is a 5-membered heteroaryl.

In some embodiments, disclosed herein is a compound of Formula I, or a pharmaceutically acceptable salt thereof, wherein A is a 6-membered heteroaryl that is optionally substituted with one to three -C₍₁₋₄₎alkyl groups. In some embodiments, disclosed herein is a compound of Formula I, or a pharmaceutically acceptable salt thereof, wherein A is a 6-membered heteroaryl that is optionally substituted with one methyl group. In some embodiments, disclosed herein is a compound of Formula I, or a pharmaceutically acceptable salt thereof, wherein A is a 6-membered heteroaryl.

In some embodiments, disclosed herein is a compound of Formula I, or a pharmaceutically acceptable salt thereof, wherein A is a 5- to 6-membered heteroaryl that is optionally substituted with one -C₍₁₋₄₎alkyl group. In some embodiments, disclosed herein is a compound of Formula I, or a pharmaceutically acceptable salt thereof, wherein A is a 5-membered heteroaryl or pyridinyl, each of which is optionally substituted with one -C₍₁₋₄₎alkyl group. In some embodiments, disclosed herein is a compound of Formula I, or a pharmaceutically acceptable salt thereof, wherein A is a 5-membered heteroaryl or pyridinyl, each of which is optionally substituted with one methyl group. In some embodiments, disclosed herein is a compound of Formula I, or a pharmaceutically acceptable salt thereof, wherein A is a 5-membered heteroaryl or pyridinyl.

In some embodiments, disclosed herein is a compound of Formula I, or a pharmaceutically acceptable salt thereof, wherein A is pyrazolyl, oxazolyl, thiazolyl, or pyridinyl, each of which is optionally substituted with one methyl group. In some embodiments, disclosed herein is a compound of Formula I, or a pharmaceutically acceptable salt thereof, wherein A is pyrazolyl, oxazolyl, thiazolyl, or pyridinyl. In some embodiments, disclosed herein is a compound of Formula I, or a pharmaceutically acceptable salt thereof, wherein A is pyrazolyl or thiazolyl. In some embodiments, disclosed herein is a compound of Formula I, or a pharmaceutically acceptable salt thereof, wherein A is pyrazolyl. In some embodiments, disclosed herein is a compound of Formula I, or a pharmaceutically acceptable salt thereof, wherein A is oxazolyl. In some embodiments, disclosed herein is a compound of Formula I, or a pharmaceutically acceptable salt thereof, wherein A is thiazolyl. In some embodiments, disclosed herein is a compound of Formula I, or a pharmaceutically acceptable salt thereof, wherein A is pyridinyl.

In some embodiments, disclosed herein is a compound of Formula I, or a pharmaceutically acceptable salt thereof, wherein A is:

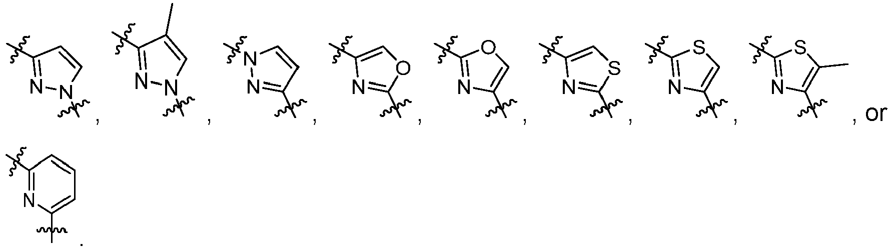

In some embodiments, disclosed herein is a compound of Formula I, or a pharmaceutically acceptable salt thereof, wherein A is

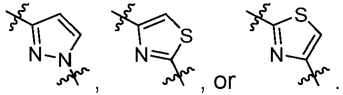

In some embodiments, disclosed herein is a compound of Formula I, or a pharmaceutically acceptable salt thereof, wherein A is

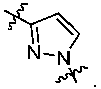

In some embodiments, disclosed herein is a compound of Formula I, or a pharmaceutically acceptable salt thereof, wherein A is

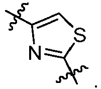

In some embodiments, disclosed herein is a compound of Formula I, or a pharmaceutically acceptable salt thereof, wherein A is

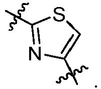

In some embodiments, disclosed herein is a compound of Formula I, or a pharmaceutically acceptable salt thereof, which is a compound of Formula Ib-1:

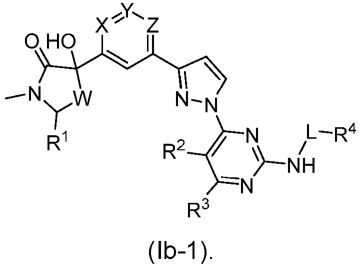

In some embodiments, disclosed herein is a compound of Formula I, or a pharmaceutically acceptable salt thereof, which is a compound of Formula Ib-2:

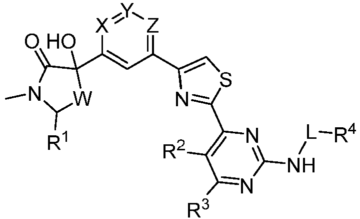

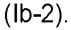

In some embodiments, disclosed herein is a compound of Formula I, or a pharmaceutically acceptable salt thereof, which is a compound of Formula Ib-3:

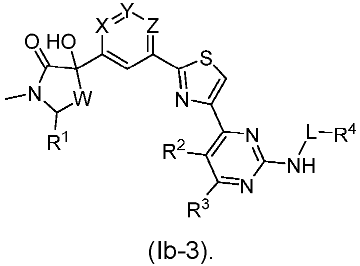

In some embodiments, disclosed herein is a compound of Formula I, or a pharmaceutically acceptable salt thereof, wherein W is CH₂, CHD, CD₂, or CF₂. In some embodiments, disclosed herein is a compound of Formula I, or a pharmaceutically acceptable salt thereof, wherein W is CH₂, CD₂, or CF₂. In some embodiments, disclosed herein is a compound of Formula I, or a pharmaceutically acceptable salt thereof, wherein W is CH₂ or CF₂. In some embodiments, disclosed herein is a compound of Formula I, or a pharmaceutically acceptable salt thereof, wherein W is CH₂. In some embodiments, disclosed herein is a compound of Formula I, or a pharmaceutically acceptable salt thereof, wherein W is CF₂.

In some embodiments, disclosed herein is a compound of Formula I, or a pharmaceutically acceptable salt thereof, wherein at least two of X, Y, and Z are C-H. In some embodiments, disclosed herein is a compound of Formula I, or a pharmaceutically acceptable salt thereof, wherein no more than one of X, Y, and Z is N.

In some embodiments, disclosed herein is a compound of Formula I, or a pharmaceutically acceptable salt thereof, wherein X is C-H or C-R^{X}. In some embodiments, disclosed herein is a compound of Formula I, or a pharmaceutically acceptable salt thereof, wherein X is C-H or C-F. In some embodiments, disclosed herein is a compound of Formula I, or a pharmaceutically acceptable salt thereof, wherein X is C-H. In some embodiments, disclosed herein is a compound of Formula I, or a pharmaceutically acceptable salt thereof, wherein X is C-R^{X}. In some embodiments, disclosed herein is a compound of Formula I, or a pharmaceutically acceptable salt thereof, wherein X is C-F.

In some embodiments, disclosed herein is a compound of Formula I, or a pharmaceutically acceptable salt thereof, wherein Y is N, C-H, C-F, or C-CN. In some embodiments, disclosed herein is a compound of Formula I, or a pharmaceutically acceptable salt thereof, wherein Y is N or C-H. In some embodiments, disclosed herein is a compound of Formula I, or a pharmaceutically acceptable salt thereof, wherein Y is N. In some embodiments, disclosed herein is a compound of Formula I, or a pharmaceutically acceptable salt thereof, wherein Y is C-H. In some embodiments, disclosed herein is a compound of Formula I, or a pharmaceutically acceptable salt thereof, wherein Y is C-R^{Y}. In some embodiments, disclosed herein is a compound of Formula I, or a pharmaceutically acceptable salt thereof, wherein Y is C-F. In some embodiments, disclosed herein is a compound of Formula I, or a pharmaceutically acceptable salt thereof, wherein Y is C-CN.

In some embodiments, disclosed herein is a compound of Formula I, or a pharmaceutically acceptable salt thereof, wherein Z is N, C-H, or C-F. In some embodiments, disclosed herein is a compound of Formula I, or a pharmaceutically acceptable salt thereof, wherein Z is N. In some embodiments, disclosed herein is a compound of Formula I, or a pharmaceutically acceptable salt thereof, wherein Z is C-H. In some embodiments, disclosed herein is a compound of Formula I, or a pharmaceutically acceptable salt thereof, wherein Z is C-R^{Z}. In some embodiments, disclosed herein is a compound of Formula I, or a pharmaceutically acceptable salt thereof, wherein Z is C-F.

In some embodiments, disclosed herein is a compound of Formula I, or a pharmaceutically acceptable salt thereof, wherein R^{X}, R^{Y}, and R^{Z} are each, independently, halo, -CN, -C₍₁₋₄₎alkyl, or -C₍₁₋₄₎haloalkyl. In some embodiments, disclosed herein is a compound of Formula I, or a pharmaceutically acceptable salt thereof, wherein R^{X}, R^{Y}, and R^{Z} are each, independently, fluoro or -CN.

In some embodiments, disclosed herein is a compound of Formula I, or a pharmaceutically acceptable salt thereof, wherein: X is C-H or C-F; Y is N, C-H, C-F, or C-CN; and Z is N, C-H, or C-F. In some embodiments, disclosed herein is a compound of Formula I, or a pharmaceutically acceptable salt thereof, wherein: X is C-H; Y is N, C-H, or C-F; and Z is N, C-H, or C-F. In some embodiments, disclosed herein is a compound of Formula I, or a pharmaceutically acceptable salt thereof, wherein: X is C-H; Y is N or C-H; and Z is N, C-H, or C-F.

In some embodiments, disclosed herein is a compound of Formula I, or a pharmaceutically acceptable salt thereof, which is a compound of any one of Formulas Ic-1 to Ic-4:

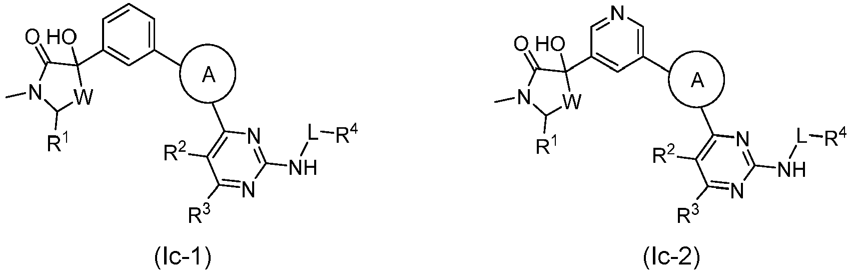

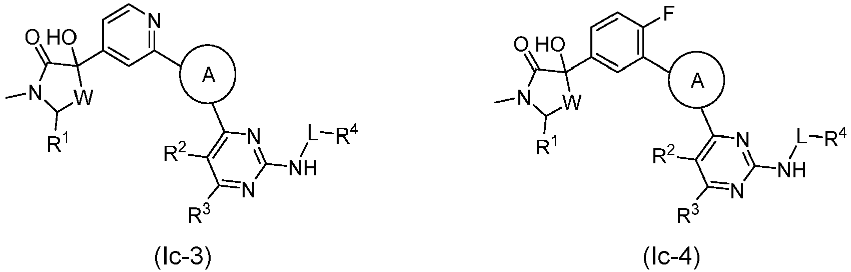

In some embodiments, disclosed herein is a compound of Formula I, or a pharmaceutically acceptable salt thereof, wherein R¹ is hydrogen or -C₍₁₋₄₎haloalkyl. In some embodiments, disclosed herein is a compound of Formula I, or a pharmaceutically acceptable salt thereof, wherein R¹ is hydrogen or -CF₃. In some embodiments, disclosed herein is a compound of Formula I, or a pharmaceutically acceptable salt thereof, wherein R¹ is hydrogen. In some embodiments, disclosed herein is a compound of Formula I, or a pharmaceutically acceptable salt thereof, wherein R¹ is -CF₃.

In some embodiments, disclosed herein is a compound of Formula I, or a pharmaceutically acceptable salt thereof, which is a compound of any one of Formulas Id-1 to Id-5:

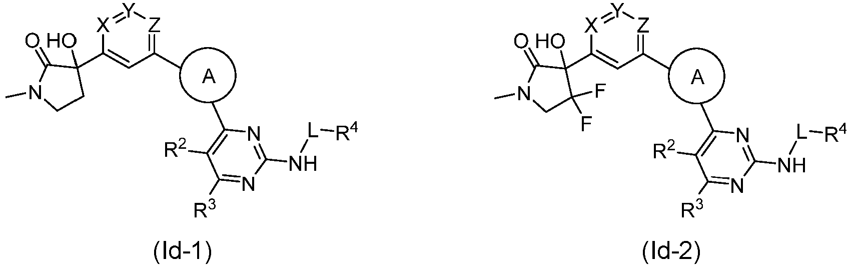

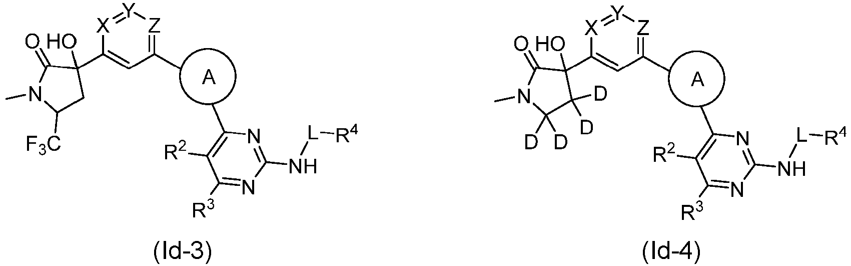

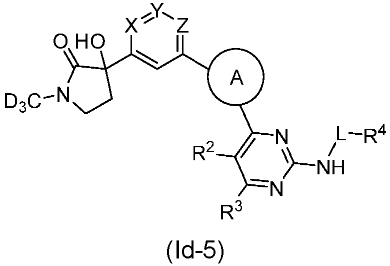

In some embodiments, disclosed herein is a compound of Formula I, or a pharmaceutically acceptable salt thereof, wherein R² and R³ are each independently hydrogen, halo, or -C₍₁₋₄₎alkyl. In some embodiments, disclosed herein is a compound of Formula I, or a pharmaceutically acceptable salt thereof, wherein R² and R³ are each independently hydrogen, fluoro, or methyl. In some embodiments, disclosed herein is a compound of Formula I, or a pharmaceutically acceptable salt thereof, wherein R² and R³ are hydrogen.

In some embodiments, disclosed herein is a compound of Formula I, or a pharmaceutically acceptable salt thereof, wherein R² is hydrogen, halo, or -C₍₁₋₄₎alkyl. In some embodiments, disclosed herein is a compound of Formula I, or a pharmaceutically acceptable salt thereof, wherein R² is hydrogen, fluoro, or methyl. In some embodiments, disclosed herein is a compound of Formula I, or a pharmaceutically acceptable salt thereof, wherein R² is hydrogen.

In some embodiments, disclosed herein is a compound of Formula I, or a pharmaceutically acceptable salt thereof, wherein R³ is hydrogen, halo, or -C₍₁₋₄₎alkyl. In some embodiments, disclosed herein is a compound of Formula I, or a pharmaceutically acceptable salt thereof, wherein R³ is hydrogen or -C₍₁₋₄₎alkyl. In some embodiments, disclosed herein is a compound of Formula I, or a pharmaceutically acceptable salt thereof, wherein R³ is hydrogen.

In some embodiments, disclosed herein is a compound of Formula I, or a pharmaceutically acceptable salt thereof, wherein L is absent, -C₍₁₋₄₎alkylene, or -C₍₃₋₆₎cycloalkylene, wherein the -C₍₁₋₄₎alkylene is optionally substituted with one -OC₍₁₋₃₎alkyl group. In some embodiments, disclosed herein is a compound of Formula I, or a pharmaceutically acceptable salt thereof, wherein L is absent, -C₍₁₋₄₎alkylene, or cyclopropylene, wherein the -C₍₁₋₄₎alkylene is optionally substituted with one -OC₍₁₋₃₎alkyl group.

In some embodiments, disclosed herein is a compound of Formula I, or a pharmaceutically acceptable salt thereof, wherein L is absent,

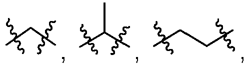

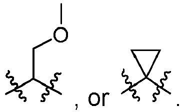

In some embodiments, disclosed herein is a compound of Formula I, or a pharmaceutically acceptable salt thereof, wherein L is absent or

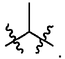

In some embodiments, disclosed herein is a compound of Formula I, or a pharmaceutically acceptable salt thereof, wherein L is absent.

In some embodiments, disclosed herein is a compound of Formula I, or a pharmaceutically acceptable salt thereof, wherein L is

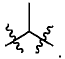

In some embodiments, disclosed herein is a compound of Formula I, or a pharmaceutically acceptable salt thereof, which is a compound of any one of Formulas le-1 to le-8:

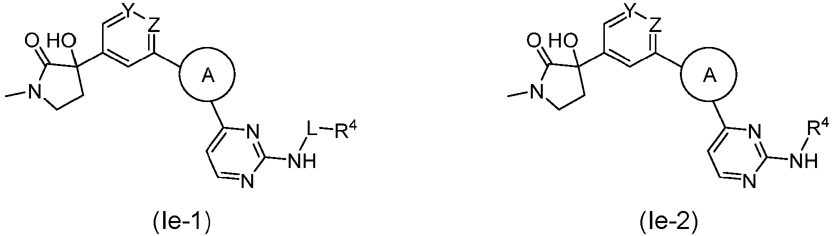

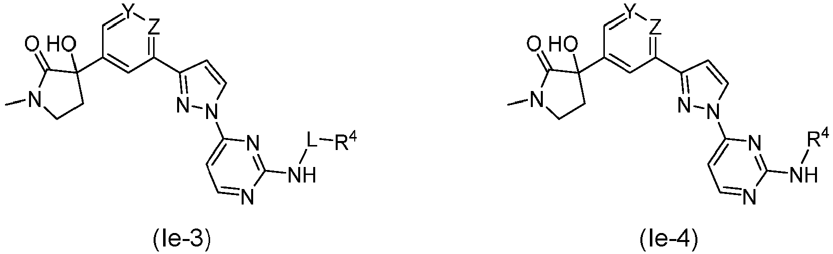

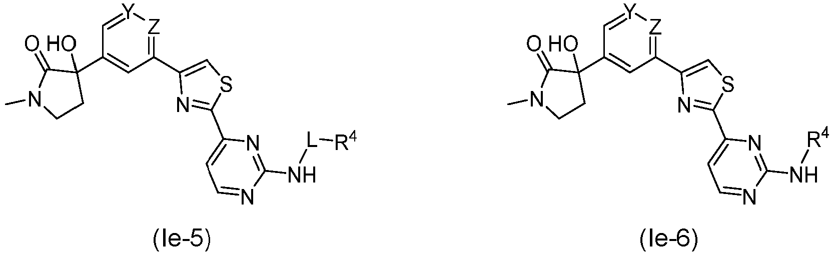

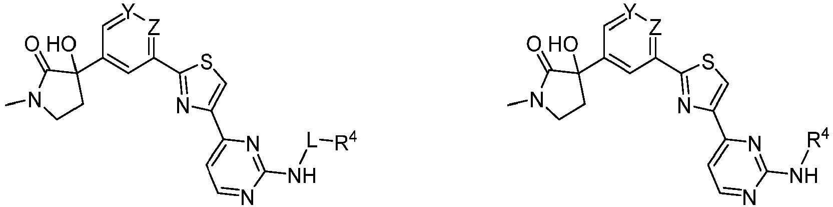

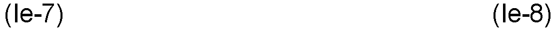

In some embodiments, disclosed herein is a compound of Formula I, or a pharmaceutically acceptable salt thereof, wherein R⁴ is -C₍₁₋₆₎alkyl, -C₍₃₋₁₀₎cycloalkyl, 3-to 10-membered heterocyclyl, a 5- to 12-membered bi- or tricyclic ring system containing one or more heteroatoms, -C(O)-(3- to 10-membered heterocyclyl), -C₍₆₋₁₀₎aryl, or 5- to 10-membered heteroaryl, each of which is optionally substituted with one to five R^{4x} groups. In some embodiments, disclosed herein is a compound of Formula I, or a pharmaceutically acceptable salt thereof, wherein R⁴ is -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, or 5- to 10-membered heteroaryl, each of which is optionally substituted with one to five R^{4x} groups.

In some embodiments, disclosed herein is a compound of Formula I, or a pharmaceutically acceptable salt thereof, wherein each R^{4x}, independently for each occurrence, is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl.

In some embodiments, disclosed herein is a compound of Formula I, or a pharmaceutically acceptable salt thereof, wherein each R^{4x}, independently for each occurrence, is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -C₍₁₋₄₎alkyl, -C₍₁₋₄₎haloalkyl, -OC₍₁₋₄₎alkyl, -OC₍₁₋₆₎haloalkyl, and - C(O)N(R^{N1})(R^{N2}).

In some embodiments, disclosed herein is a compound of Formula I, or a pharmaceutically acceptable salt thereof, wherein each R^{4x}, independently for each occurrence, is halo, -OH, -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, - C(O)N(R^{N1})(R^{N2}), -S(O)₂C₍₁₋₄₎alkyl, 3- to 8-membered heterocyclyl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, and 3- to 8-membered heterocyclyl, are optionally further substituted with one to five groups selected from halo, -OH, -C₍₁₋₄₎alkyl, -C₍₁₋₄₎haloalkyl, - OC₍₁₋₄₎alkyl, -OC₍₁₋₆₎haloalkyl, and -C(O)N(R^{N1})(R^{N2}).

In some embodiments, disclosed herein is a compound of Formula I, or a pharmaceutically acceptable salt thereof, wherein R⁴ is -C₍₁₋₆₎alkyl, -C₍₃₋₁₀₎cycloalkyl, 3-to 10-membered heterocyclyl, a 5- to 12-membered bi- or tricyclic ring system containing one or more heteroatoms, -C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups.

In some embodiments, disclosed herein is a compound of Formula I, or a pharmaceutically acceptable salt thereof, wherein R⁴ is -C₍₁₋₆₎alkyl, -C₍₃₋₁₀₎cycloalkyl, 3-to 10-membered heterocyclyl, a 5- to 12-membered bi- or tricyclic ring system containing one or more heteroatoms, or 5- to 10-membered heteroaryl, wherein: the - C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, and the 5- to 10-membered heteroaryl is optionally substituted with one to five R^{4g} groups.

In some embodiments, disclosed herein is a compound of Formula I, or a pharmaceutically acceptable salt thereof, wherein R⁴ is -C₍₁₋₆₎alkyl, which is optionally substituted with one to five R^{3a} groups.

In some embodiments, disclosed herein is a compound of Formula I, or a pharmaceutically acceptable salt thereof, wherein each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₆₋₁₀₎aryl, or 5- to 10-membered heteroaryl, wherein the -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl.

In some embodiments, disclosed herein is a compound of Formula I, or a pharmaceutically acceptable salt thereof, wherein each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, or 3- to 8-membered heterocyclyl, wherein the -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, and 3- to 8-membered heterocyclyl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -C(O)N(R^{N1})(R^{N2}), -S(O)₂C₍₁₋₄₎alkyl, -N(H)S(O)₂C₍₁₋₄₎alkyl, or 3-to 8-membered heterocyclyl, wherein the -C₍₁₋₆₎haloalkyl, -C₍₃₋₈₎cycloalkyl, -OC₍₁₋₆₎alkyl, - OC₍₁₋₆₎haloalkyl, and 3- to 8-membered heterocyclyl are optionally further substituted with one to five groups selected from halo, -OH, -N(R^{N1})(R^{N2}), -CN, -C₍₁₋₄₎alkyl, and - OC₍₁₋₄₎alkyl.

In some embodiments, disclosed herein is a compound of Formula I, or a pharmaceutically acceptable salt thereof, wherein each R^{4a} independently for each occurrence is halo, -OH, -C₍₁₋₆₎haloalkyl, -C₍₃₋₈₎cycloalkyl, -OC₍₁₋₆₎alkyl, -OC₍₁₋₆₎haloalkyl, -S(O)₂C₍₁₋₄₎alkyl, -N(H)S(O)₂C₍₁₋₄₎alkyl, or 3- to 8-membered heterocyclyl, wherein the - C₍₁₋₆₎haloalkyl, -C₍₃₋₈₎cycloalkyl, -OC₍₁₋₆₎alkyl, -OC₍₁₋₆₎haloalkyl, and 3- to 8-membered heterocyclyl are optionally further substituted with one to five groups selected from halo and -OH.

In some embodiments, disclosed herein is a compound of Formula I, or a pharmaceutically acceptable salt thereof, wherein R⁴ is:

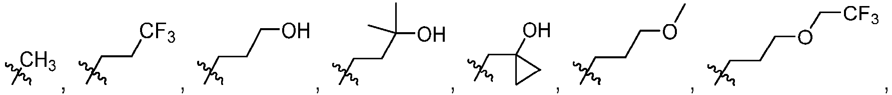

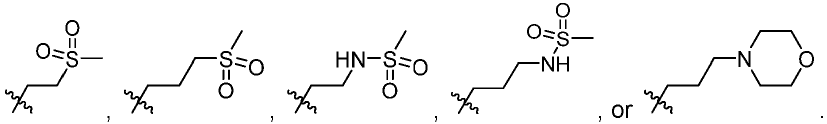

In some embodiments, disclosed herein is a compound of Formula I, or a pharmaceutically acceptable salt thereof, wherein R⁴ is -C₍₃₋₁₀₎cycloalkyl, which is optionally substituted with one to five R^{4b} groups.

In some embodiments, disclosed herein is a compound of Formula I, or a pharmaceutically acceptable salt thereof, wherein R⁴ is cyclopropyl, cyclobutyl, cyclopentyl, cyclohexyl, bicyclo[2.2.1]heptyl, or spirocyclo[3.3]heptyl, each of which is optionally substituted with one to three R^{4b} groups.

In some embodiments, disclosed herein is a compound of Formula I, or a pharmaceutically acceptable salt thereof, wherein each R^{4b} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and - N(H)S(O)₂C₍₁₋₄₎alkyl.

In some embodiments, disclosed herein is a compound of Formula I, or a pharmaceutically acceptable salt thereof, wherein each R^{4b} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4b} independently for each occurrence is halo, -OH, -N(R^{N1})(R^{N2}), -CN, -C₍₁₋₆₎alkyl, -C₍₁₋₆₎haloalkyl, -OC₍₁₋₆₎alkyl, - OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo, - OH, -N(R^{N1})(R^{N2}), and -CN.

In some embodiments, disclosed herein is a compound of Formula I, or a pharmaceutically acceptable salt thereof, wherein each R^{4b} independently for each occurrence is halo, -OH, -CN, -C₍₁₋₆₎alkyl, -C₍₁₋₆₎haloalkyl, -OC₍₁₋₆₎alkyl, -OC₍₁₋₆₎haloalkyl, wherein the -C₍₁₋₆₎alkyl, -C₍₁₋₆₎haloalkyl, -OC₍₁₋₆₎alkyl, and -OC₍₁₋₆₎haloalkyl are optionally further substituted with one to five groups selected from halo and -OH.

In some embodiments, disclosed herein is a compound of Formula I, or a pharmaceutically acceptable salt thereof, wherein each R^{4b} independently for each occurrence is halo, -OH, -CN, -C₍₁₋₆₎alkyl, or -OC₍₁₋₆₎alkyl, wherein the -C₍₁₋₆₎alkyl is optionally further substituted with one -OH group.

In some embodiments, disclosed herein is a compound of Formula I, or a pharmaceutically acceptable salt thereof, wherein R⁴ is:

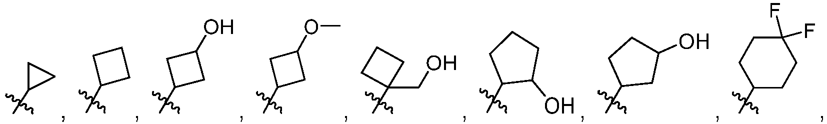

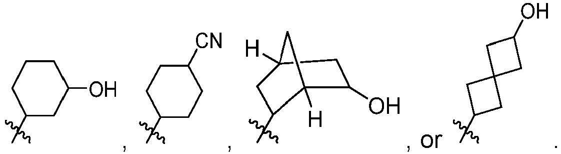

In some embodiments, disclosed herein is a compound of Formula I, or a pharmaceutically acceptable salt thereof, wherein R⁴ is 3- to 10-membered heterocyclyl, which is optionally substituted with one to five R^{4c} groups.

In some embodiments, disclosed herein is a compound of Formula I, or a pharmaceutically acceptable salt thereof, wherein R⁴ is oxetanyl, tetrahydrofuranyl, tetrahydropyranyl, azetidinyl, pyrrolidinyl, piperidinyl, pyrrolidin-2-onyl, piperidin-2-onyl, morpholinyl, or piperazinyl, each of which is optionally substituted with one to three R^{4c} groups.

In some embodiments, disclosed herein is a compound of Formula I, or a pharmaceutically acceptable salt thereof, wherein each R^{4c} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and - N(H)S(O)₂C₍₁₋₄₎alkyl.

In some embodiments, disclosed herein is a compound of Formula I, or a pharmaceutically acceptable salt thereof, wherein each R^{4c} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4c} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, - C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4c} independently for each occurrence is -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, and -C₍₁₋₄₎alkyl.

In some embodiments, disclosed herein is a compound of Formula I, or a pharmaceutically acceptable salt thereof, wherein each R^{4c} independently for each occurrence is -C₍₁₋₆₎alkyl, -C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, 3- to 8-membered heterocyclyl, and 5- to 10-membered heteroaryl are optionally further substituted with one to five groups selected from halo, -OH, and -C₍₁₋₄₎alkyl.

In some embodiments, disclosed herein is a compound of Formula I, or a pharmaceutically acceptable salt thereof, wherein R⁴ is:

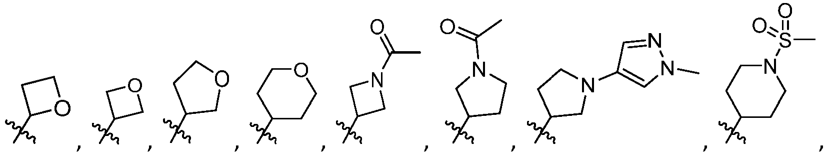

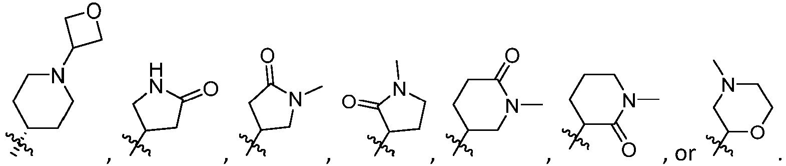

In some embodiments, disclosed herein is a compound of Formula I, or a pharmaceutically acceptable salt thereof, wherein R⁴ is a 5- to 12-membered bi- or tricyclic ring system containing one or more heteroatoms, which is optionally substituted with one to five R^{4d} groups. In some embodiments, disclosed herein is a compound of Formula I, or a pharmaceutically acceptable salt thereof, wherein R⁴ is a 5- to 12-membered bicyclic ring system containing one or more heteroatoms, which is optionally substituted with one to five R^{4d} groups.

In some embodiments, disclosed herein is a compound of Formula I, or a pharmaceutically acceptable salt thereof, wherein R⁴ is a 5- to 12-membered bicyclic ring system containing one to five heteroatoms selected from O, N, and S, wherein the 5- to 12-membered bicyclic ring system is optionally substituted with one to five R^{4d} groups. In some embodiments, disclosed herein is a compound of Formula I, or a pharmaceutically acceptable salt thereof, wherein R⁴ is a 5- to 12-membered bicyclic ring system containing one or more heteroatoms, which is optionally substituted with one to five R^{4d} groups, wherein the 5- to 12-membered bicyclic ring system is a 3,5-fused ring system, a 5,6-fused ring system, or a 6,6-fused ring system.

In some embodiments, disclosed herein is a compound of Formula I, or a pharmaceutically acceptable salt thereof, wherein R⁴ is 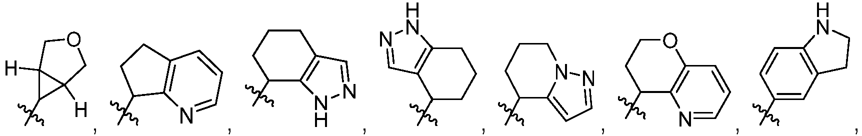 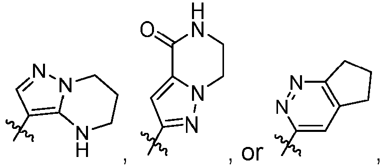 each of which is optionally substituted with one to three R^{4d} groups.

In some embodiments, disclosed herein is a compound of Formula I, or a pharmaceutically acceptable salt thereof, wherein each R^{4d} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and - N(H)S(O)₂C₍₁₋₄₎alkyl.

In some embodiments, disclosed herein is a compound of Formula I, or a pharmaceutically acceptable salt thereof, wherein each R^{4d} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4d} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, - C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4d} independently for each occurrence is -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -C(O)C₍₁₋₄₎alkyl, -S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, 3- to 8-membered heterocyclyl, and 5- to 10-membered heteroaryl are optionally further substituted with one to five groups selected from halo, -OH, and -C₍₁₋₄₎alkyl.

In some embodiments, disclosed herein is a compound of Formula I, or a pharmaceutically acceptable salt thereof, wherein each R^{4d} independently for each occurrence is -C₍₁₋₆₎alkyl, -OC₍₁₋₆₎alkyl, or -C(O)C₍₁₋₄₎alkyl, wherein the -C₍₁₋₆₎alkyl and - OC₍₁₋₆₎alkyl are optionally further substituted with one to five groups selected from halo and -OH.

In some embodiments, disclosed herein is a compound of Formula I, or a pharmaceutically acceptable salt thereof, wherein R⁴ is

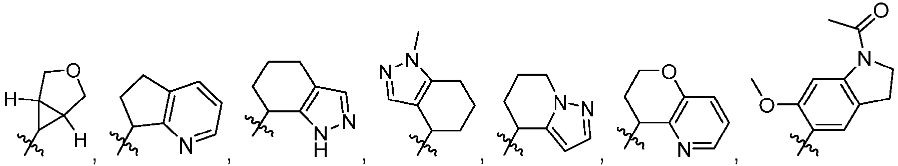

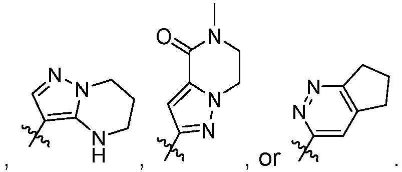

In some embodiments, disclosed herein is a compound of Formula I, or a pharmaceutically acceptable salt thereof, wherein R⁴ is -C(O)-(3- to 10-membered heterocyclyl), which is optionally substituted with one to five R^{4e} groups. In some embodiments, disclosed herein is a compound of Formula I, or a pharmaceutically acceptable salt thereof, wherein R⁴ is -C(O)-(3- to 10-membered heterocyclyl), which is optionally substituted with one to three -C₍₁₋₄₎alkyl groups.

In some embodiments, disclosed herein is a compound of Formula I, or a pharmaceutically acceptable salt thereof, wherein each R^{4e} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}),-S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and - N(H)S(O)₂C₍₁₋₄₎alkyl.

In some embodiments, disclosed herein is a compound of Formula I, or a pharmaceutically acceptable salt thereof, wherein each R^{4e} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, -N(H)S(O)₂C₍₁₋₄₎alkyl, 3- to 8-membered heterocyclyl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, and 5- to 10-membered heteroaryl are optionally further substituted with one to five groups selected from halo, -OH, -N(R^{N1})(R^{N2}), -CN, -C₍₁₋₄₎alkyl, and -OC₍₁₋₄₎alkyl.

In some embodiments, disclosed herein is a compound of Formula I, or a pharmaceutically acceptable salt thereof, wherein each R^{4e} independently for each occurrence is halo, -OH, -N(R^{N1})(R^{N2}), -CN, -C₍₁₋₆₎alkyl, -C₍₁₋₆₎haloalkyl, -OC₍₁₋₆₎alkyl, or - OC₍₁₋₆₎haloalkyl.

In some embodiments, disclosed herein is a compound of Formula I, or a pharmaceutically acceptable salt thereof, wherein each R^{4e} independently for each occurrence is -C₍₁₋₄₎alkyl groups.

In some embodiments, disclosed herein is a compound of Formula I, or a pharmaceutically acceptable salt thereof, wherein R⁴ is

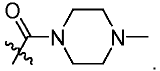

In some embodiments, disclosed herein is a compound of Formula I, or a pharmaceutically acceptable salt thereof, wherein R⁴ is -C₍₆₋₁₀₎aryl, which is optionally substituted with one to five R^{4f} groups. In some embodiments, disclosed herein is a compound of Formula I, or a pharmaceutically acceptable salt thereof, wherein R⁴ is phenyl, which is optionally substituted with one to five R^{4f} groups. In some embodiments, disclosed herein is a compound of Formula I, or a pharmaceutically acceptable salt thereof, wherein R⁴ is phenyl, which is optionally substituted with one to three R^{4f} groups.

In some embodiments, disclosed herein is a compound of Formula I, or a pharmaceutically acceptable salt thereof, wherein each R^{4f} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl.

In some embodiments, disclosed herein is a compound of Formula I, or a pharmaceutically acceptable salt thereof, wherein each R^{4f} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), - S(O)₂C₍₁₋₄₎alkyl, or -N(H)S(O)₂C₍₁₋₄₎alkyl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, and -OC₍₃₋₈₎cycloalkyl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, and -OC₍₁₋₆₎haloalkyl.

In some embodiments, disclosed herein is a compound of Formula I, or a pharmaceutically acceptable salt thereof, wherein each R^{4f} independently for each occurrence is halo, -OH, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, or -C(O)N(R^{N1})(R^{N2}), wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, and -OC₍₃₋₈₎cycloalkyl are optionally further substituted with one to five groups selected from halo and -OH.

In some embodiments, disclosed herein is a compound of Formula I, or a pharmaceutically acceptable salt thereof, wherein each R^{4f} independently for each occurrence is halo, -C₍₁₋₆₎alkyl, -C₍₃₋₈₎cycloalkyl, -OC₍₁₋₆₎alkyl, -OC₍₃₋₈₎cycloalkyl, or - C(O)N(R^{N1})(R^{N2}).

In some embodiments, disclosed herein is a compound of Formula I, or a pharmaceutically acceptable salt thereof, wherein R⁴ is:

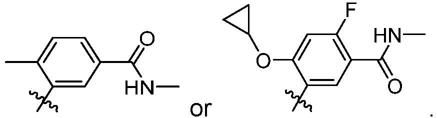

In some embodiments, disclosed herein is a compound of Formula I, or a pharmaceutically acceptable salt thereof, wherein R⁴ is 5- to 10-membered heteroaryl is optionally substituted with one to five R^{4g} groups. In some embodiments, disclosed herein is a compound of Formula I, or a pharmaceutically acceptable salt thereof, wherein R⁴ is a 5- to 6-membered monocyclic heteroaryl or a 9- to 10-membered bicyclic heteroaryl, each of which is optionally substituted with one to three R^{4g} groups. In some embodiments, disclosed herein is a compound of Formula I, or a pharmaceutically acceptable salt thereof, wherein R⁴ is a 5- to 6-membered monocyclic heteroaryl or a 9-membered bicyclic heteroaryl, each of which is optionally substituted with one to three R^{4g} groups.

In some embodiments, disclosed herein is a compound of Formula I, or a pharmaceutically acceptable salt thereof, wherein R⁴ is pyrazolyl, imidazolyl, triazolyl, oxazolyl, isoxazolyl, oxadiazolyl, thiazolyl, isothiazolyl, pyridinyl, pyridin-2-onyl, pyridazinyl, pyrimidinyl, pyrazinyl, indazolyl, benzoimidazolyl, pyrazolopyridinyl, imidazopyridinyl, imidazopyridinyl, or pyrazolopyrimidinyl, each of which is optionally substituted with one to three R^{4g} groups. In some embodiments, disclosed herein is a compound of Formula I, or a pharmaceutically acceptable salt thereof, wherein R⁴ is pyrazolyl, imidazolyl, oxazolyl, pyridinyl, or pyrazolopyridinyl, each of which is optionally substituted with one to three R^{4g} groups. In some embodiments, disclosed herein is a compound of Formula I, or a pharmaceutically acceptable salt thereof, wherein R⁴ is pyrazolyl or pyrazolopyridinyl, each of which is optionally substituted with one to three R^{4g} groups. In some embodiments, disclosed herein is a compound of Formula I, or a pharmaceutically acceptable salt thereof, wherein R⁴ is pyrazolyl, which is optionally substituted with one to three R^{4g} groups. In some embodiments, disclosed herein is a compound of Formula I, or a pharmaceutically acceptable salt thereof, wherein R⁴ is pyrazolopyridinyl, which is optionally substituted with one to three R^{4g} groups.

In some embodiments, disclosed herein is a compound of Formula I, or a pharmaceutically acceptable salt thereof, wherein R⁴ is

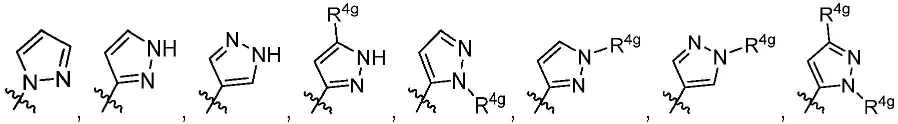

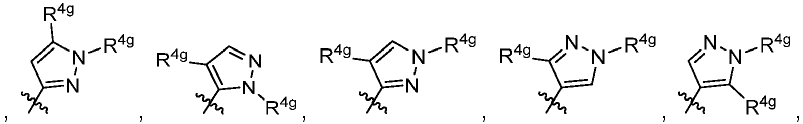

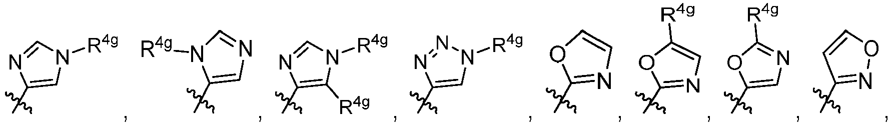

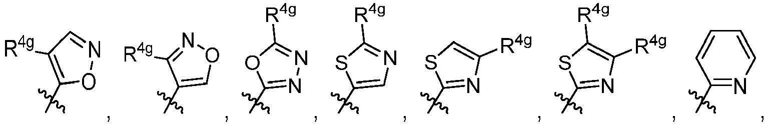

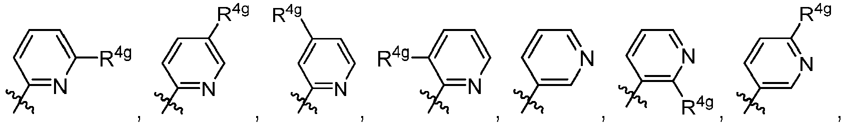

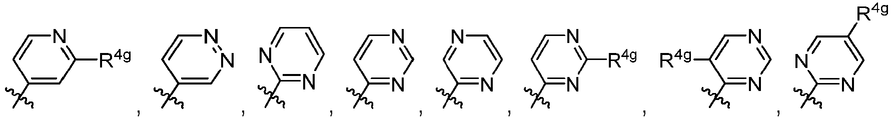

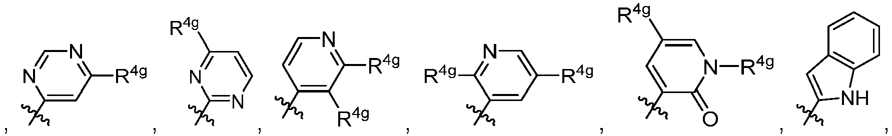

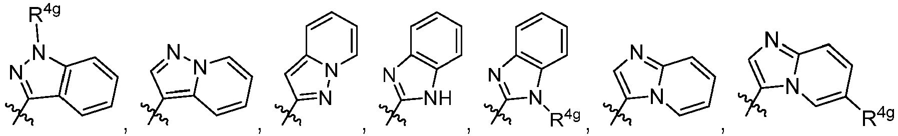

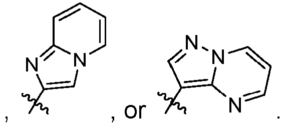

In some embodiments, disclosed herein is a compound of Formula I, or a pharmaceutically acceptable salt thereof, wherein R⁴ is

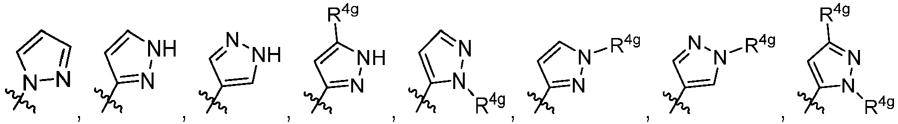

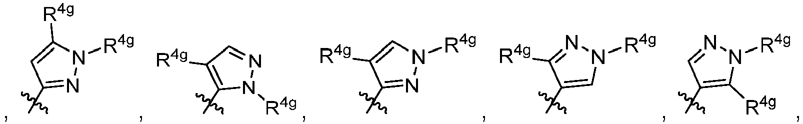

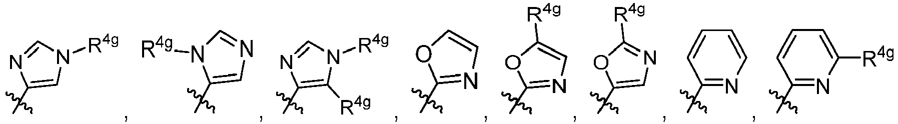

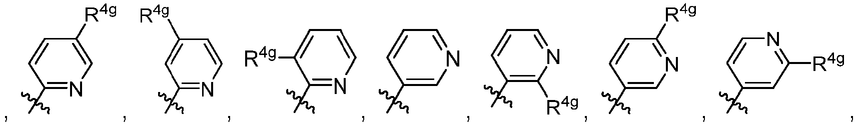

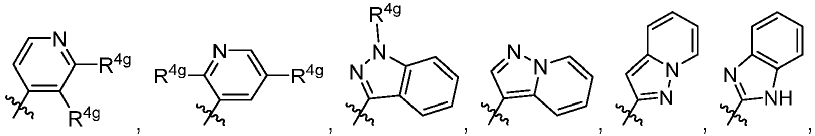

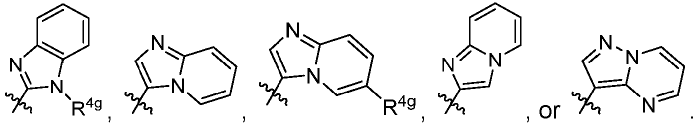

In some embodiments, disclosed herein is a compound of Formula I, or a pharmaceutically acceptable salt thereof, wherein R⁴ is 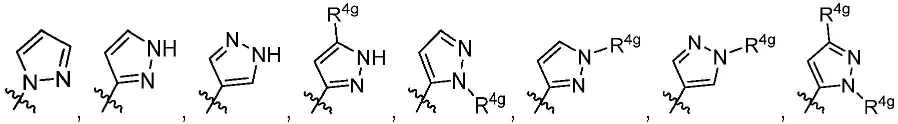 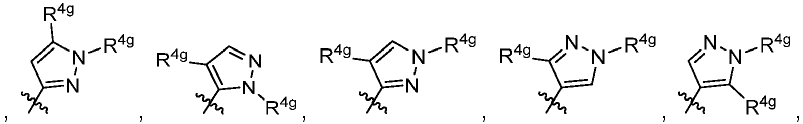 or

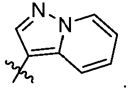

In some embodiments, disclosed herein is a compound of Formula I, or a pharmaceutically acceptable salt thereof, wherein R⁴ is:

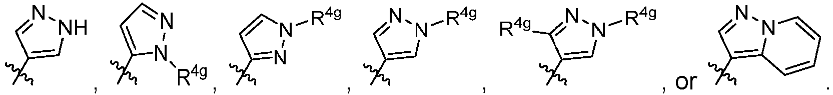

In some embodiments, disclosed herein is a compound of Formula I, or a pharmaceutically acceptable salt thereof, wherein each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl.

In some embodiments, disclosed herein is a compound of Formula I, or a pharmaceutically acceptable salt thereof, wherein each R^{4g} independently for each occurrence is halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl.

In some embodiments, disclosed herein is a compound of Formula I, or a pharmaceutically acceptable salt thereof, wherein each R^{4g} independently for each occurrence is halo, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C(O)N(R^{N1})(R^{N2}), 3- to 8-membered heterocyclyl, -C₍₁₋₃₎alkyl(3- to 8-membered heterocyclyl), or -C₍₆₋₁₀₎aryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, 3- to 8-membered heterocyclyl, -C₍₁₋₃₎alkyl(3- to 8-membered heterocyclyl), and -C₍₆₋₁₀₎aryl are optionally further substituted with one to five groups selected from halo, -OH, -C₍₁₋₄₎alkyl, -C₍₁₋₄₎haloalkyl, - OC₍₁₋₄₎alkyl, -OC₍₁₋₆₎haloalkyl, and -C(O)N(R^{N1})(R^{N2}).

In some embodiments, disclosed herein is a compound of Formula I, or a pharmaceutically acceptable salt thereof, wherein each R^{4g} independently for each occurrence is halo, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, or 3- to 8-membered heterocyclyl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, and 3-to 8-membered heterocyclyl are optionally further substituted with one to five groups selected from halo and -OH.

In some embodiments, disclosed herein is a compound of Formula I, or a pharmaceutically acceptable salt thereof, wherein each R^{4g} independently for each occurrence is -C₍₁₋₆₎alkyl, -C₍₁₋₆₎haloalkyl, -OC₍₁₋₆₎alkyl, or -OC₍₁₋₆₎haloalkyl, each of which is optionally further substituted with one to five groups selected from halo and -OH. In some embodiments, disclosed herein is a compound of Formula I, or a pharmaceutically acceptable salt thereof, wherein each R^{4g} independently for each occurrence is -C₍₁₋₆₎alkyl, -C₍₁₋₆₎haloalkyl, -OC₍₁₋₆₎alkyl, or -OC₍₁₋₆₎haloalkyl, each of which is optionally further substituted with one to five groups selected from halo and -OH. In some embodiments, each R^{4g} independently for each occurrence is -C₍₁₋₆₎alkyl or -OC₍₁₋₆₎alkyl, each of which is optionally further substituted with one to three groups selected from halo and -OH.

In some embodiments, disclosed herein is a compound of Formula I, or a pharmaceutically acceptable salt thereof, wherein each R^{4g} independently for each occurrence is:

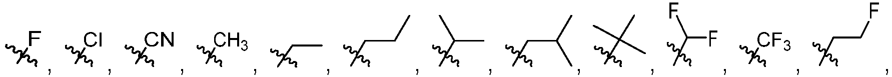

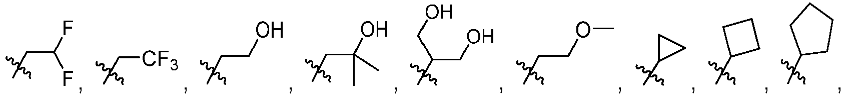

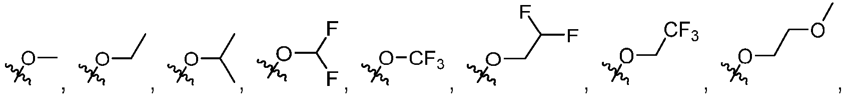

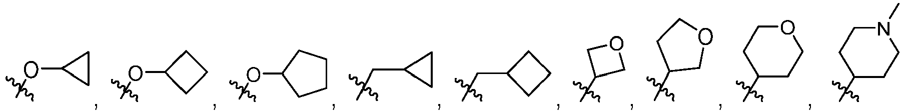

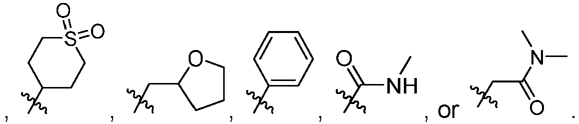

In some embodiments, disclosed herein is a compound of Formula I, or a pharmaceutically acceptable salt thereof, wherein each R^{4g} independently for each occurrence is:

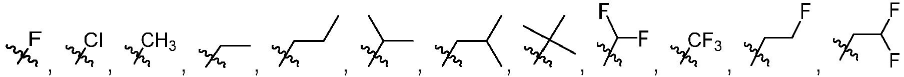

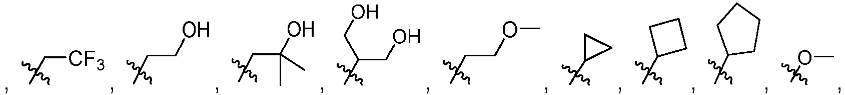

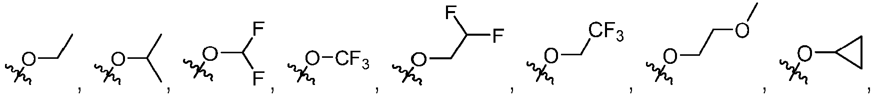

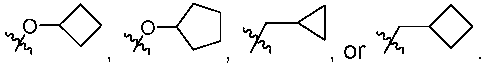

In some embodiments, disclosed herein is a compound of Formula I, or a pharmaceutically acceptable salt thereof, wherein each R^{4g} independently for each occurrence is:

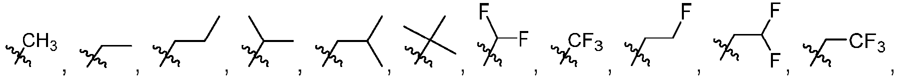

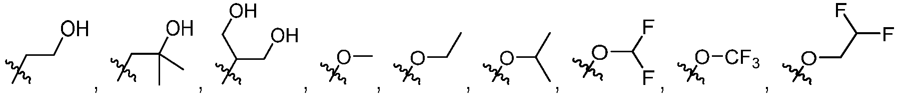

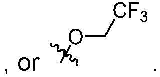

In some embodiments, disclosed herein is a compound of Formula I, or a pharmaceutically acceptable salt thereof, wherein each R^{4g} independently for each occurrence is:

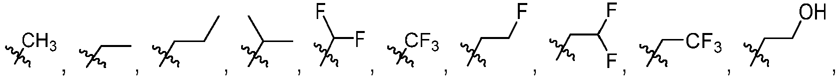

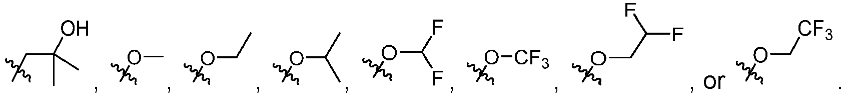

In some embodiments, disclosed herein is a compound of Formula I, or a pharmaceutically acceptable salt thereof, wherein each R^{4g} independently for each occurrence is:

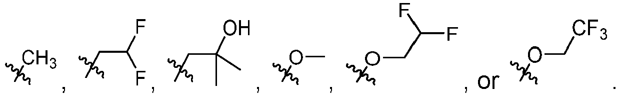

In some embodiments, disclosed herein is a compound of Formula I, or a pharmaceutically acceptable salt thereof, wherein each R^{4g} independently for each occurrence is:

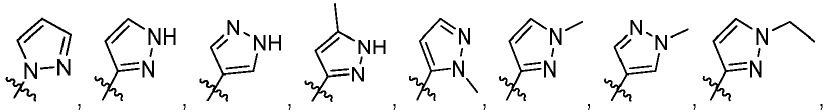

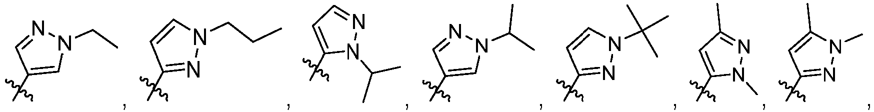

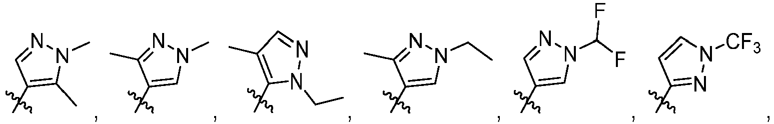

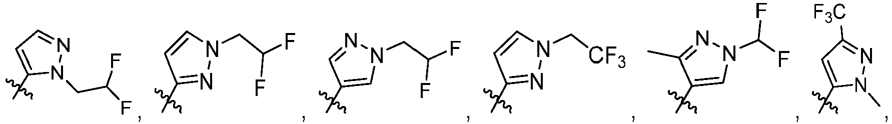

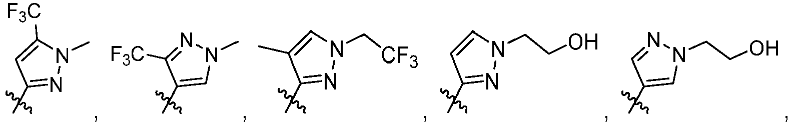

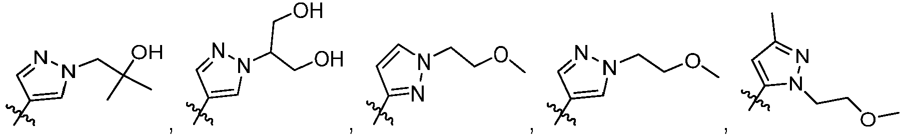

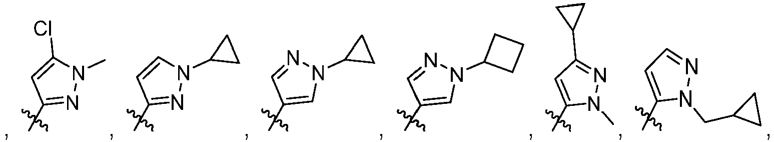

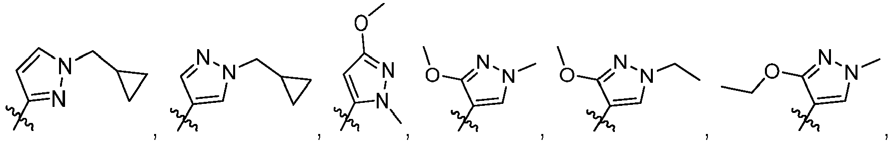

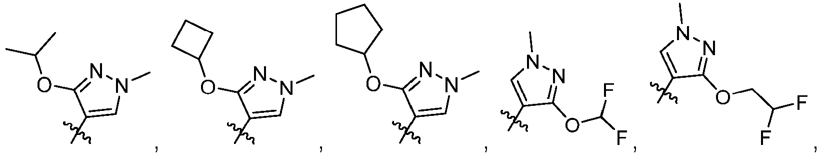

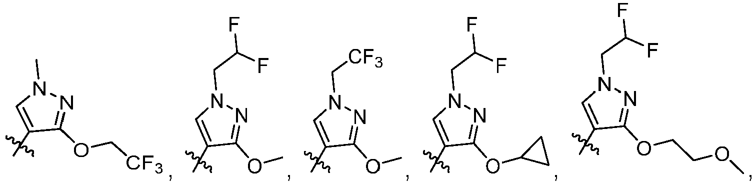

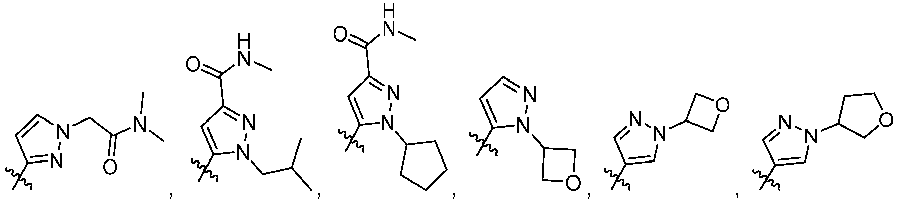

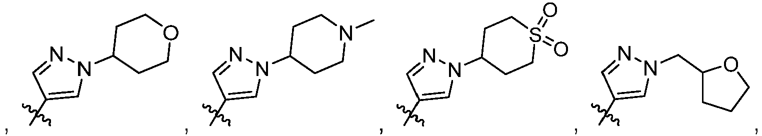

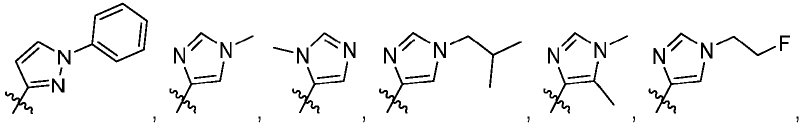

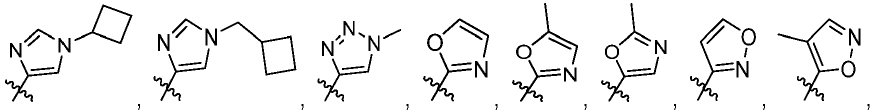

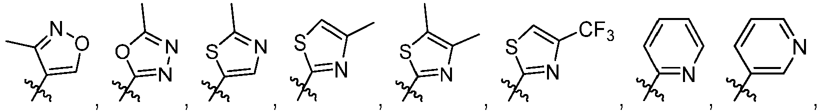

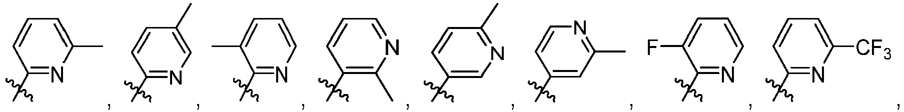

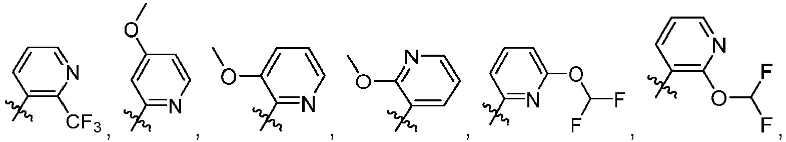

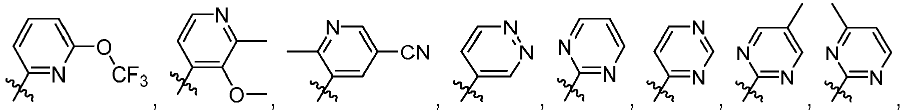

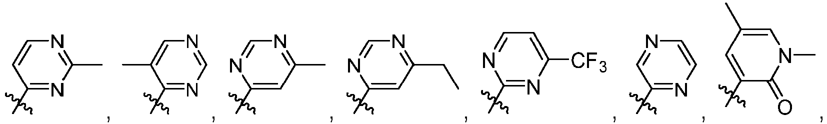

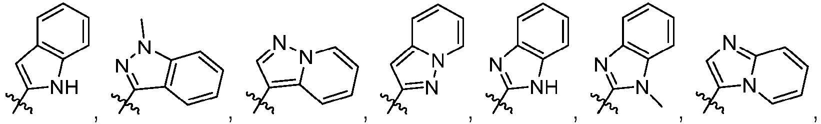

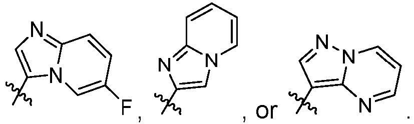

In some embodiments, disclosed herein is a compound of Formula I, or a pharmaceutically acceptable salt thereof, wherein each R^{4g} independently for each occurrence is:

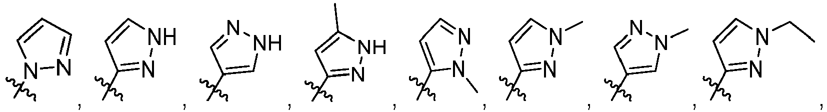

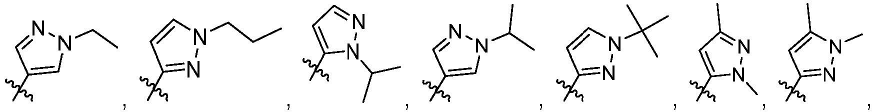

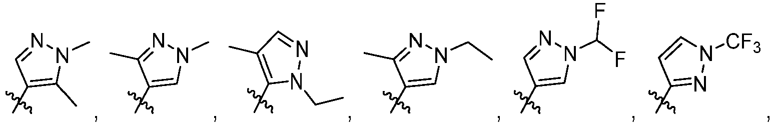

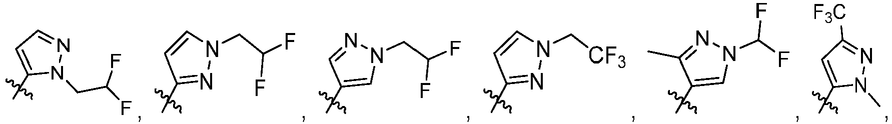

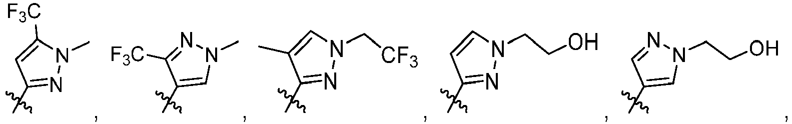

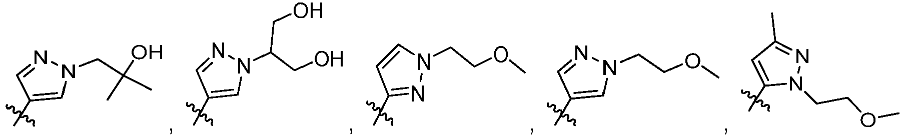

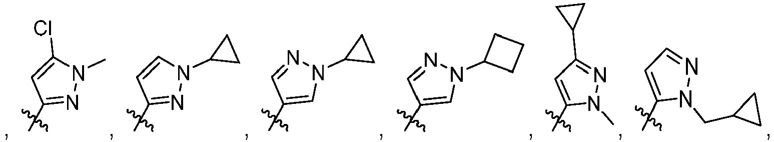

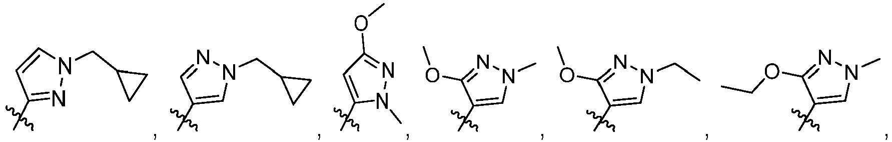

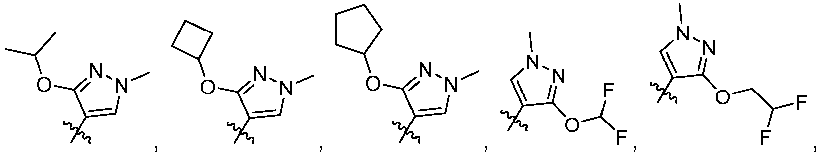

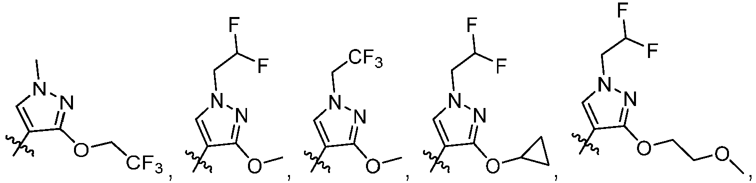

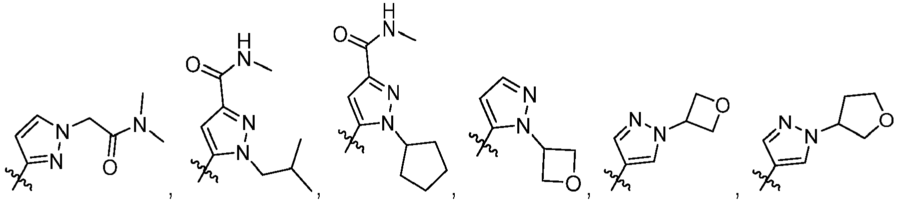

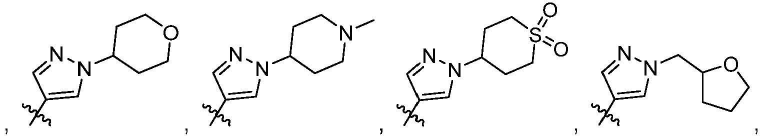

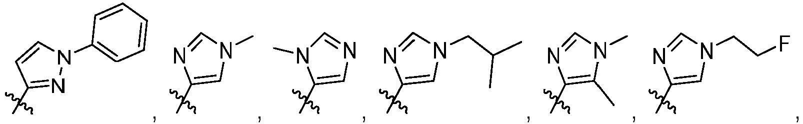

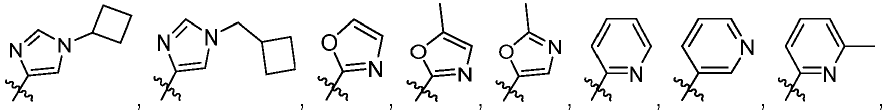

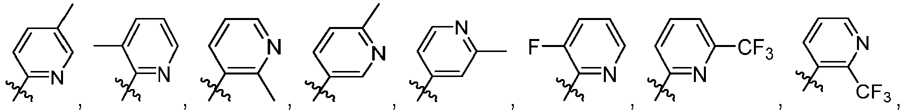

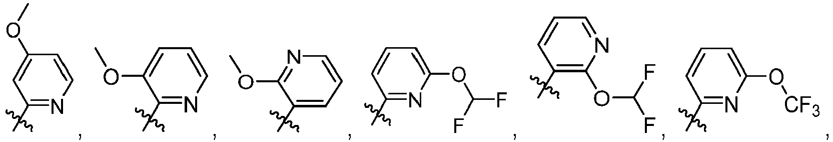

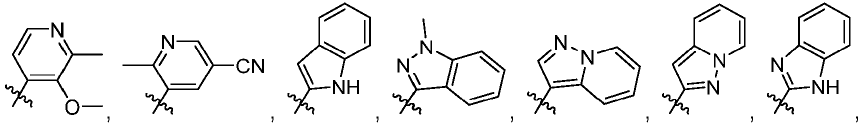

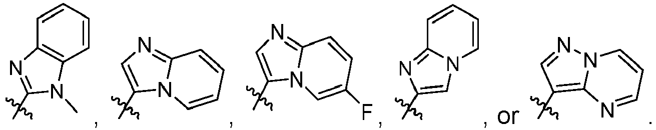

In some embodiments, disclosed herein is a compound of Formula I, or a pharmaceutically acceptable salt thereof, wherein each R^{4g} independently for each occurrence is:

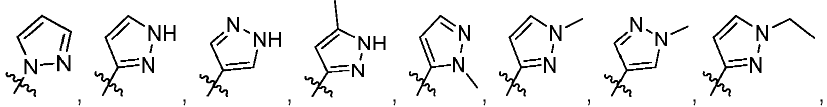

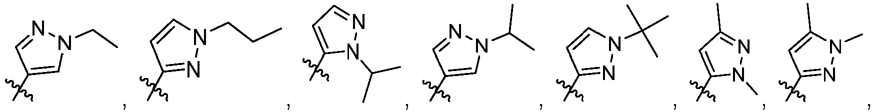

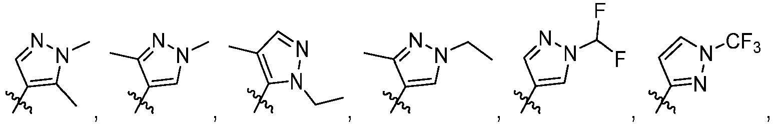

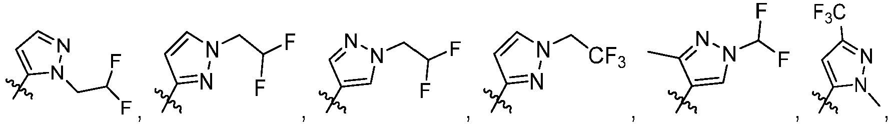

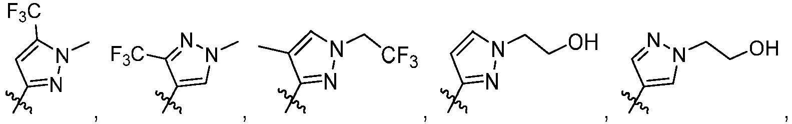

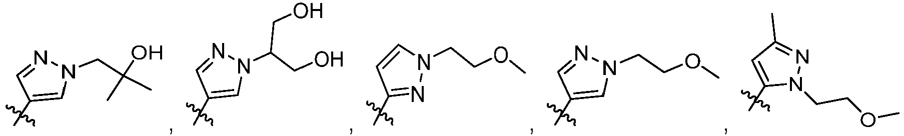

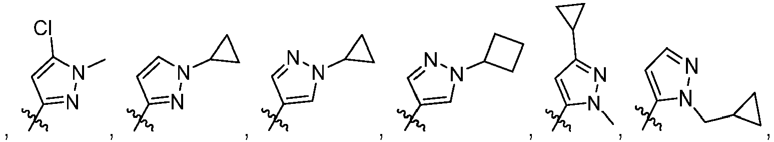

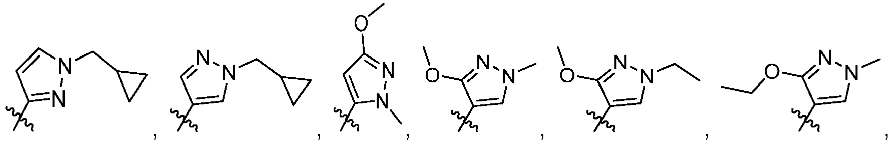

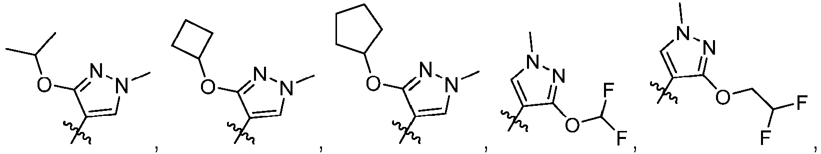

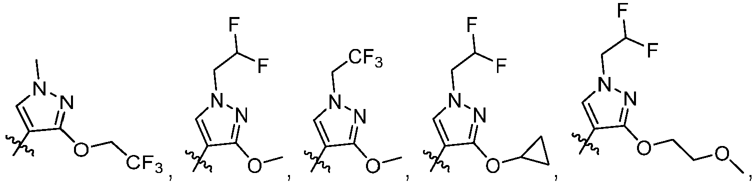

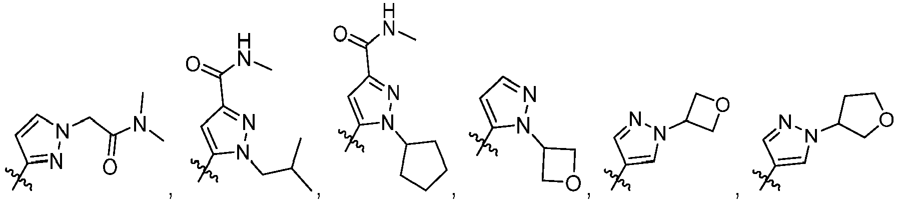

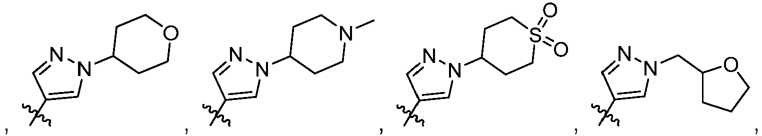

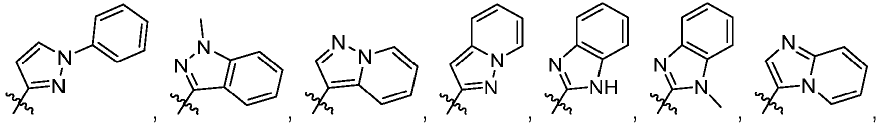

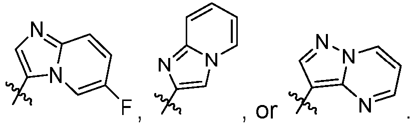

In some embodiments, disclosed herein is a compound of Formula I, or a pharmaceutically acceptable salt thereof, wherein each R^{4g} independently for each occurrence is:

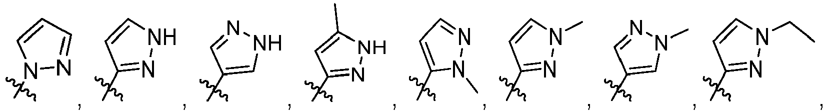

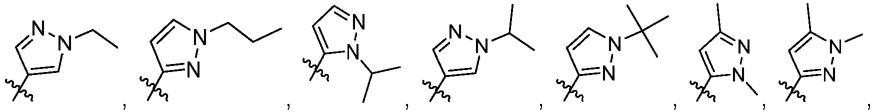

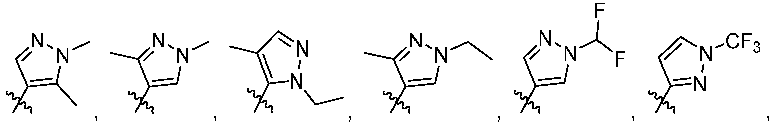

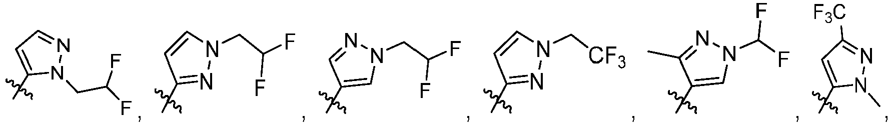

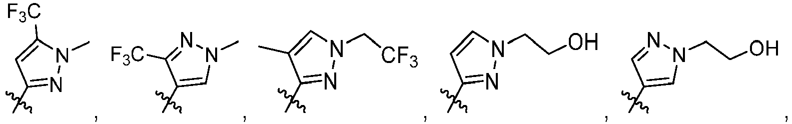

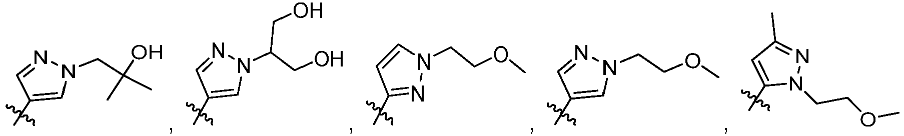

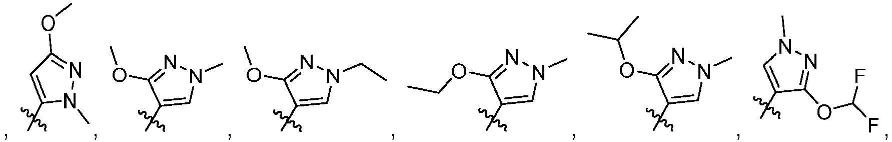

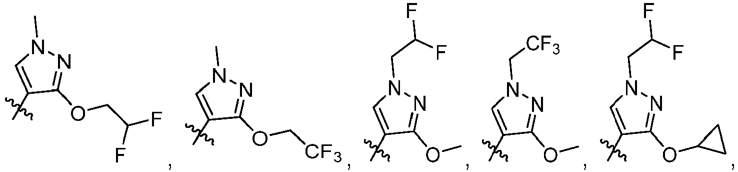

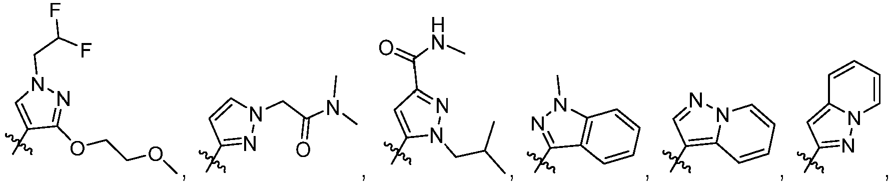

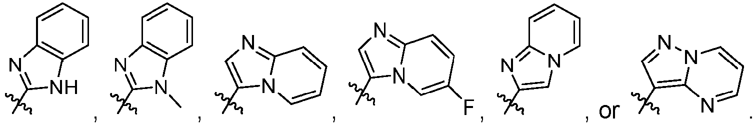

In some embodiments, disclosed herein is a compound of Formula I, or a pharmaceutically acceptable salt thereof, wherein each R^{4g} independently for each occurrence is:

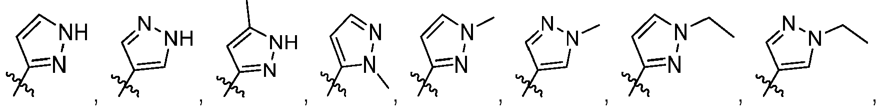

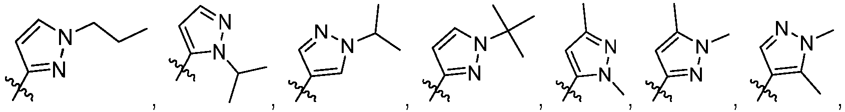

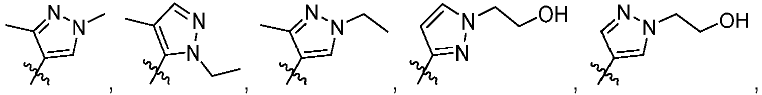

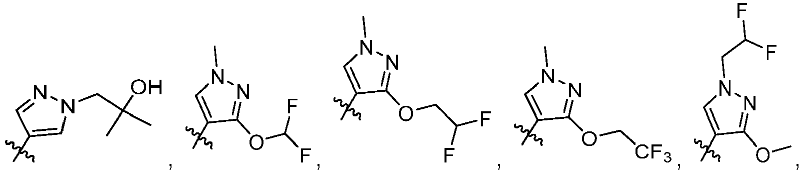

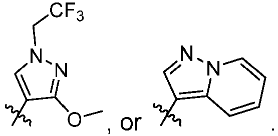

In some embodiments, disclosed herein is a compound of Formula I, or a pharmaceutically acceptable salt thereof, wherein each R^{4g} independently for each occurrence is:

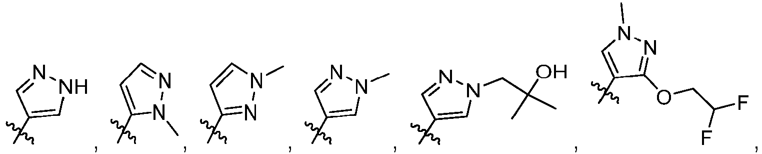

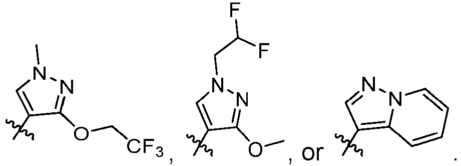

In some embodiments, disclosed herein is a compound of Formula I, or a pharmaceutically acceptable salt thereof, wherein R^{N1} and R^{N2} are each independently for each occurrence H or -C₍₁₋₃₎alkyl. In some embodiments, disclosed herein is a compound of Formula I, or a pharmaceutically acceptable salt thereof, wherein R^{N1} and R^{N2} are each hydrogen.

In some embodiments, disclosed herein is a compound of Formula I, or a pharmaceutically acceptable salt thereof, which is a compound of Formula le-1: 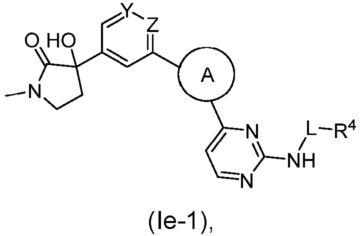
A is a pyrazolyl or thiazolyl;
Y is N, C-H, or C-F;
Z is N, C-H, or C-F;
L is absent or -C₍₁₋₄₎alkylene;
R⁴ is 5- to 10-membered heteroaryl, which is optionally substituted with one to five R^{4g} groups;
each R^{4g} independently for each occurrence is -C₍₁₋₆₎alkyl, -C₍₁₋₆₎haloalkyl, -OC₍₁₋₆₎alkyl, or -OC₍₁₋₆₎haloalkyl, each of which is optionally further substituted with one to five groups selected from halo and -OH;
provided that at least one of Y and Z is C-H.

In some embodiments, disclosed herein is a compound of Formula I, or a pharmaceutically acceptable salt thereof, having a structure as shown in any one of Tables 1A to 1M.

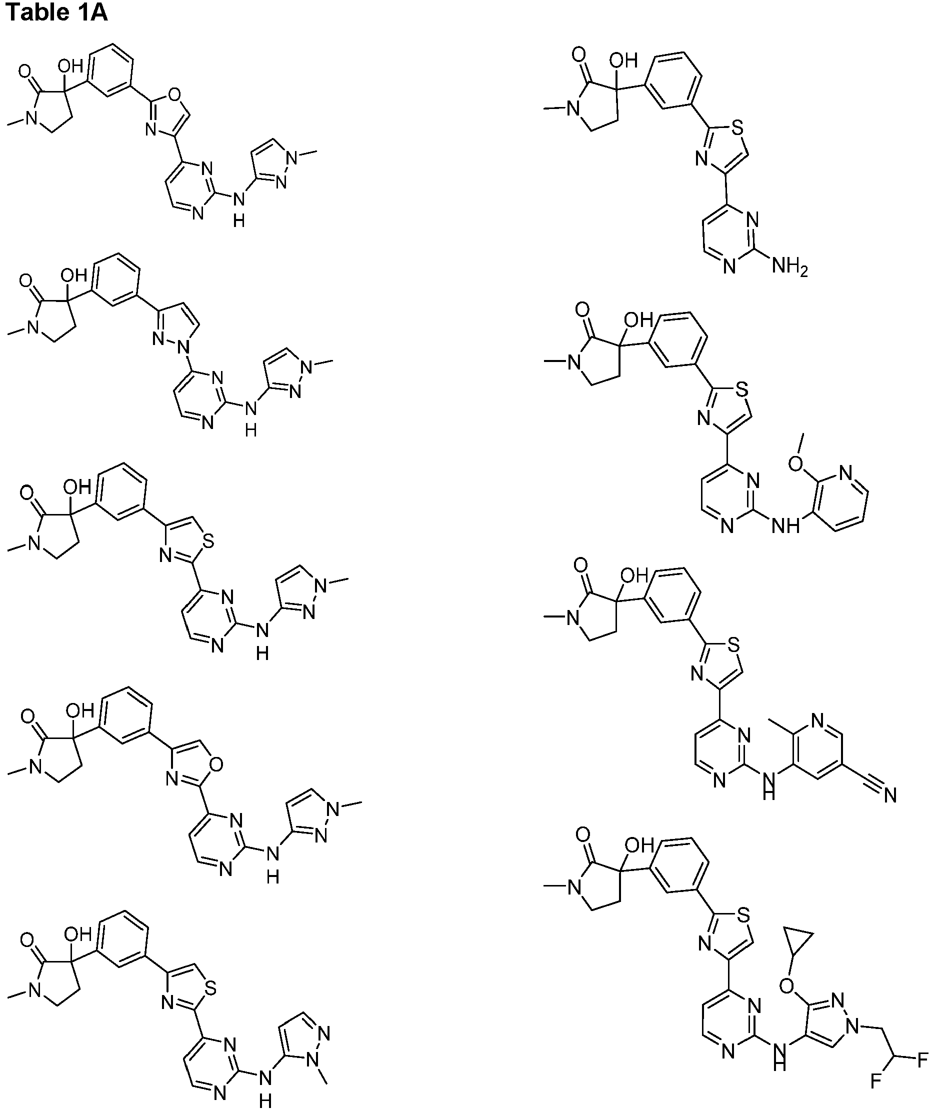

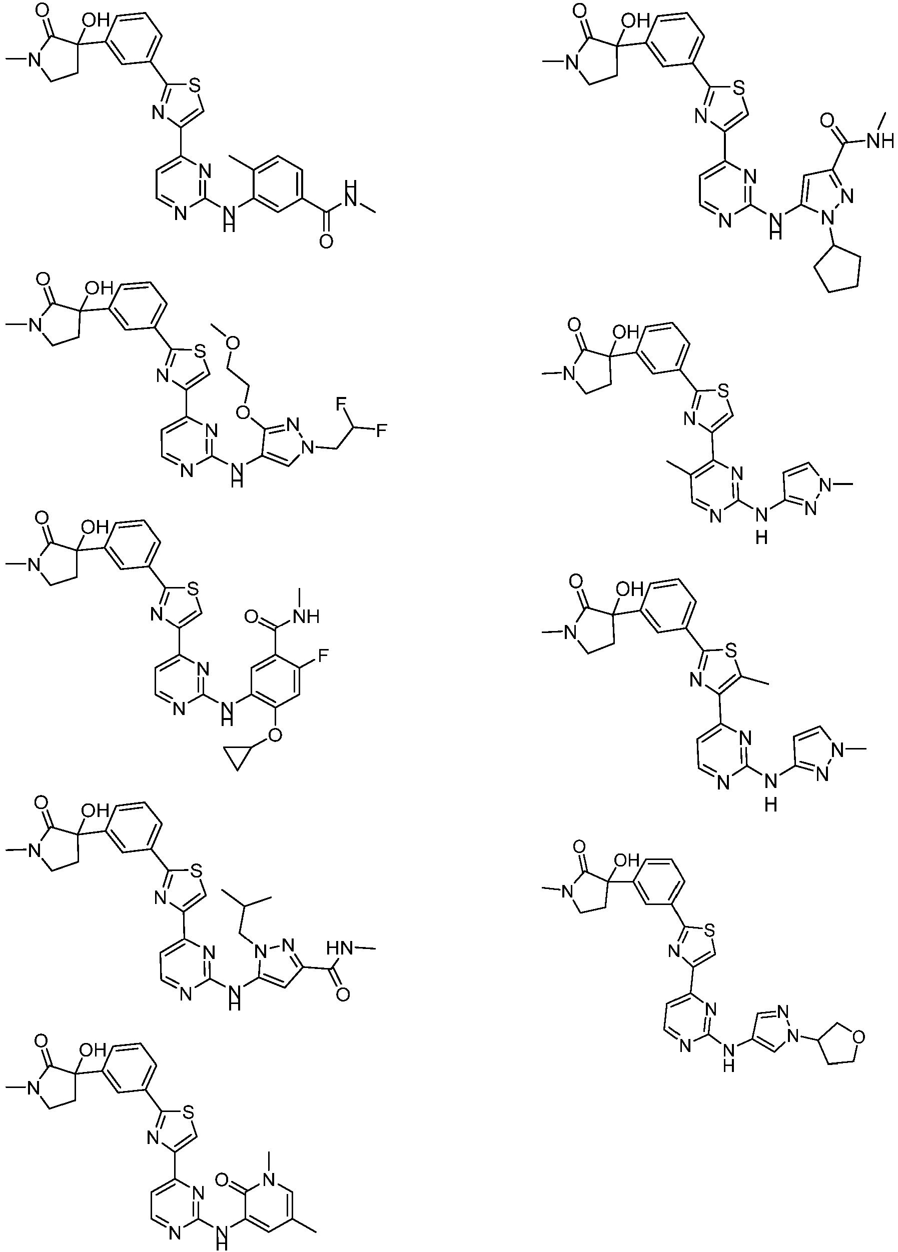

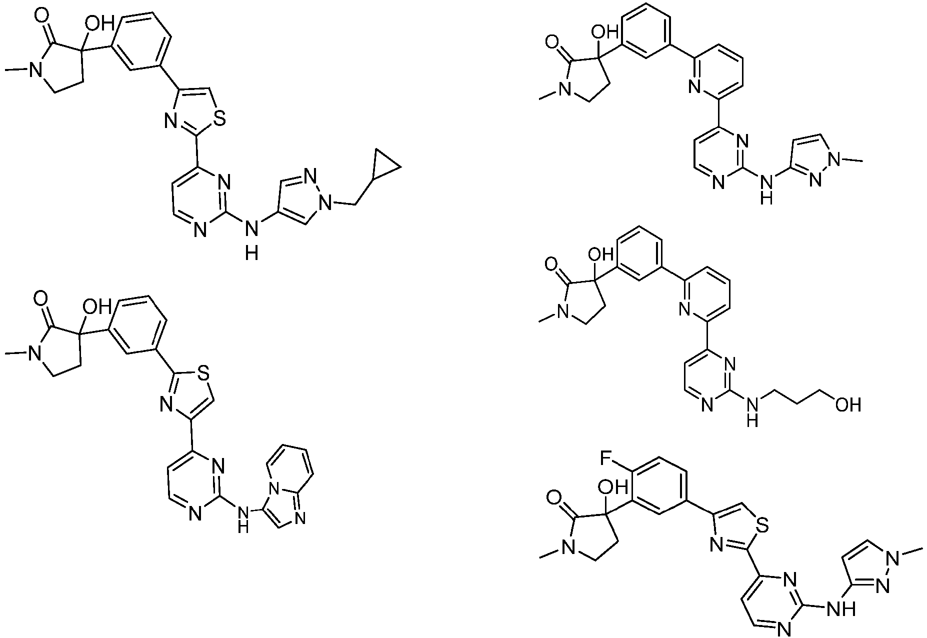

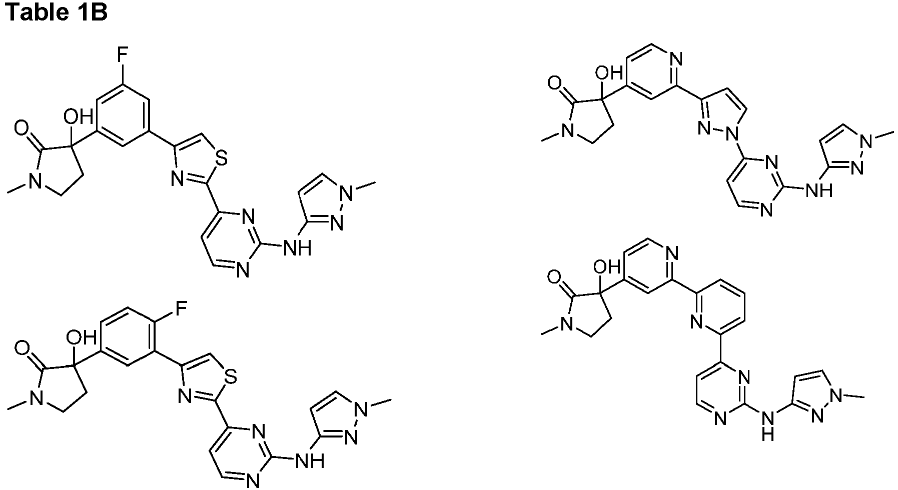

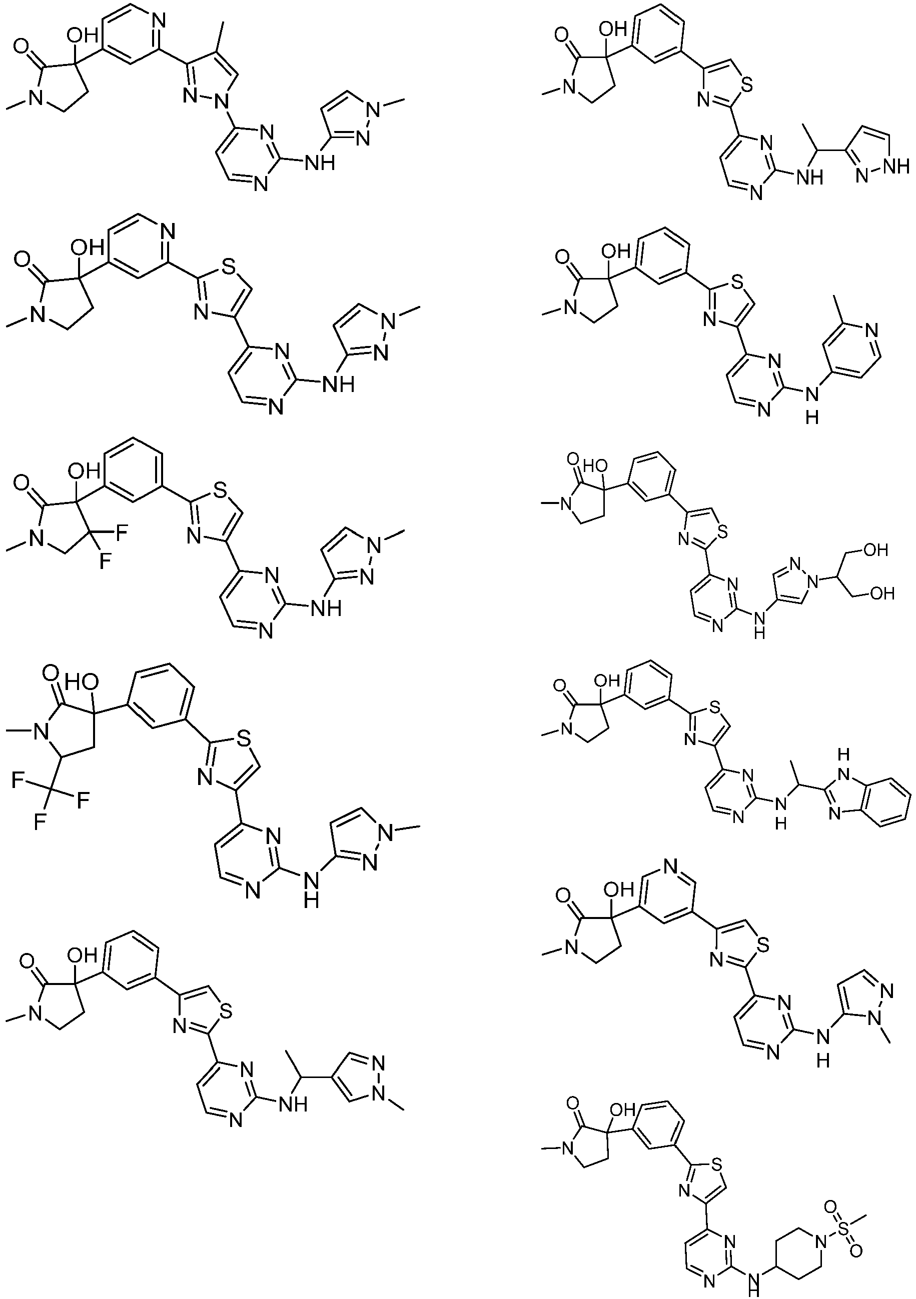

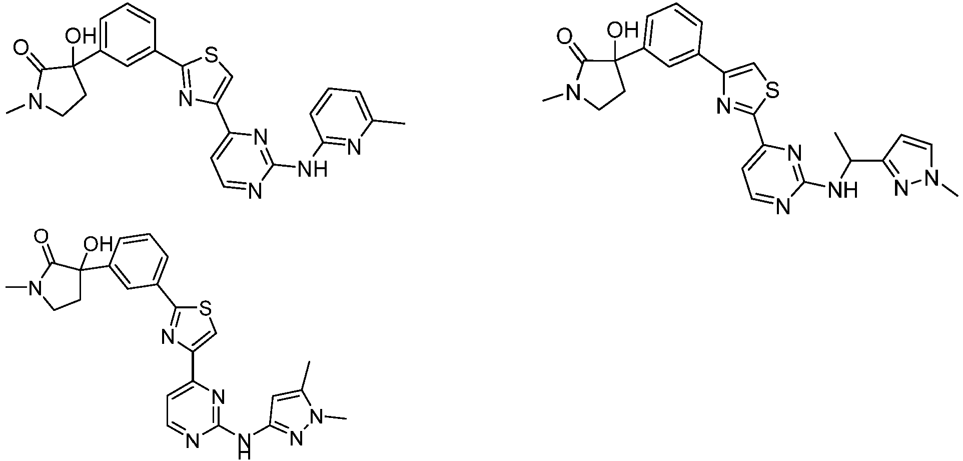

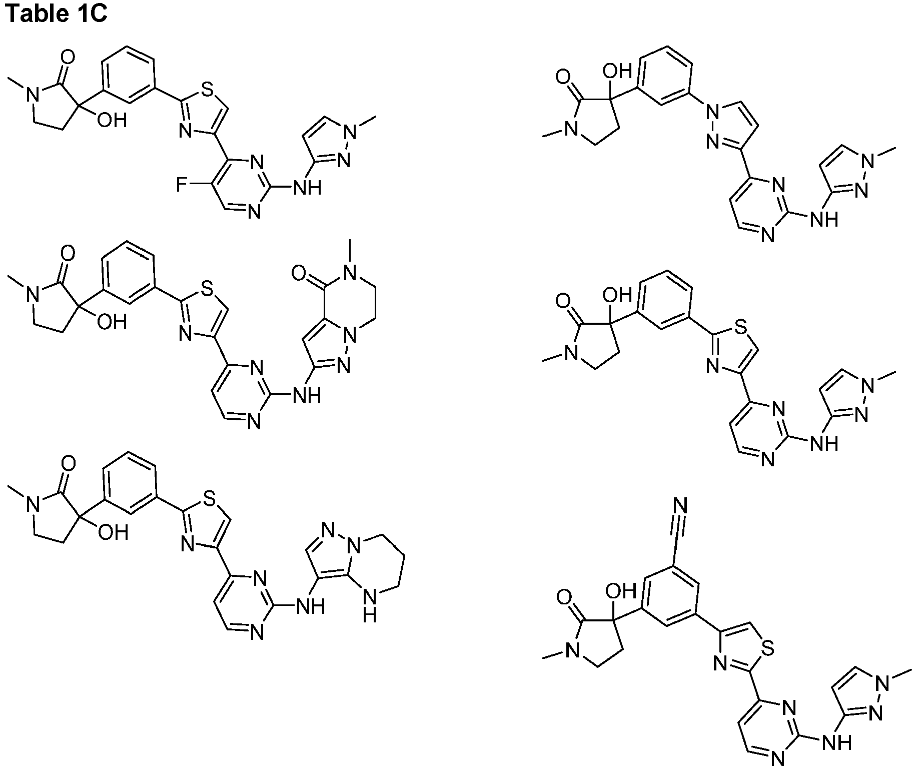

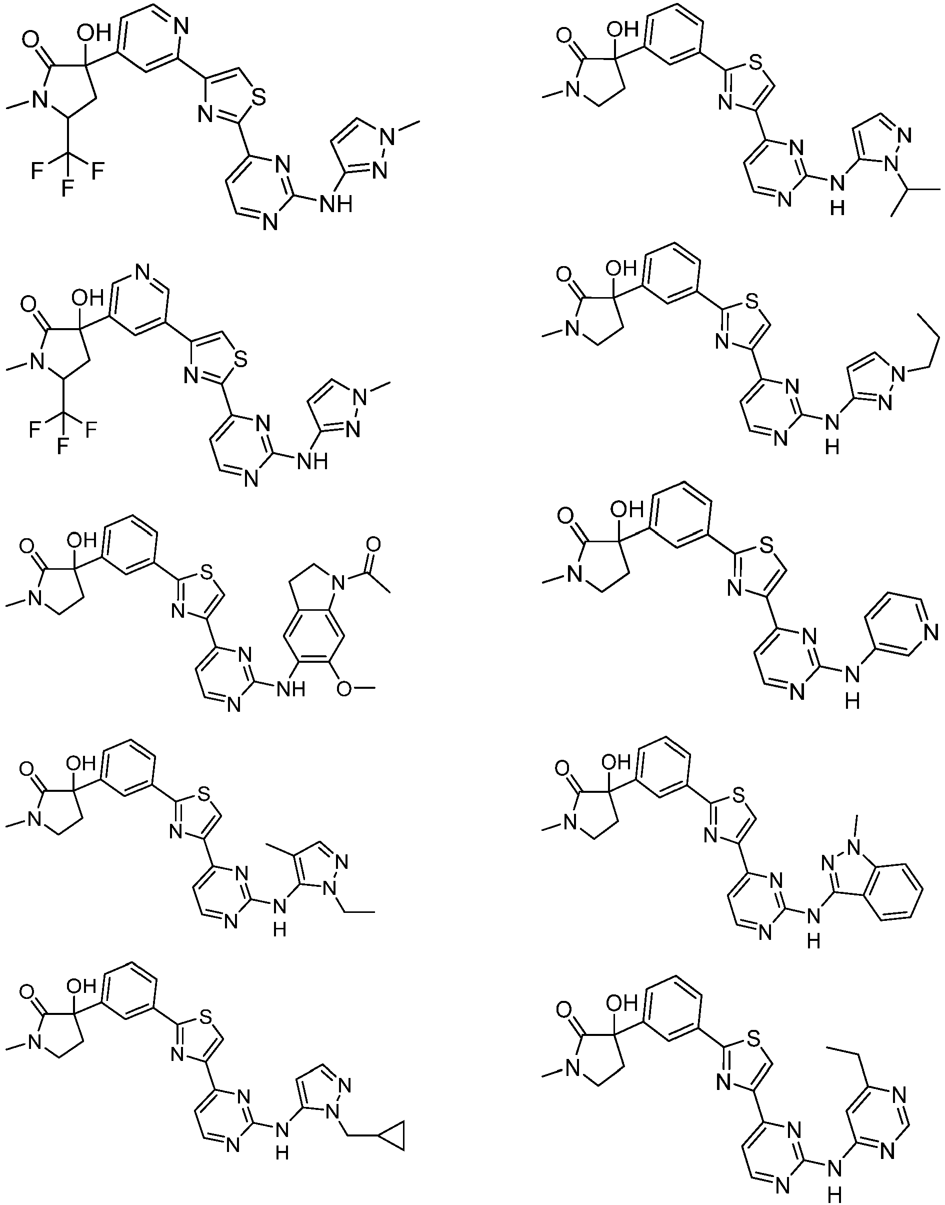

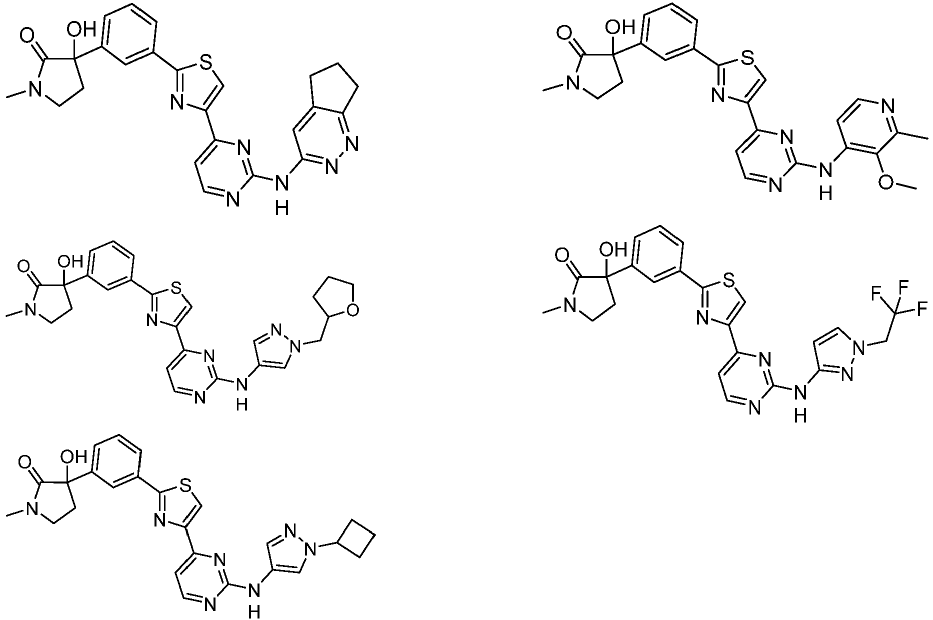

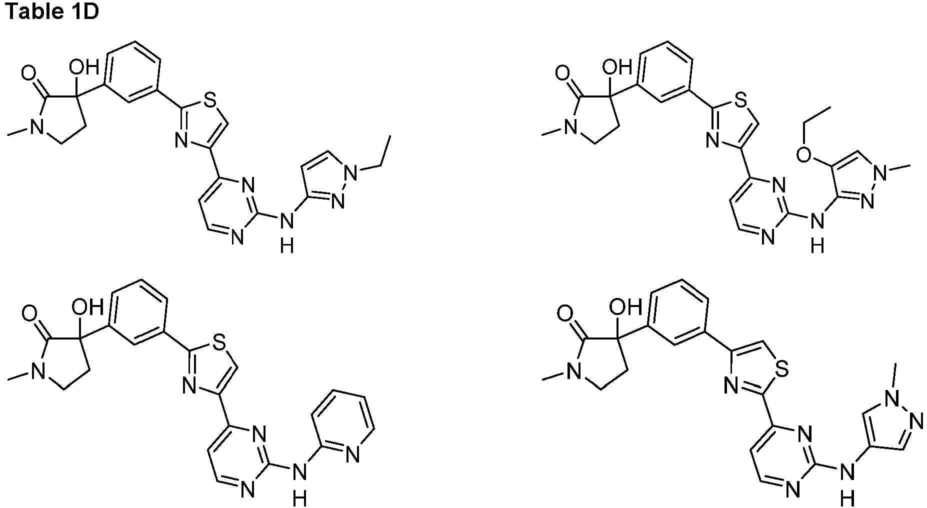

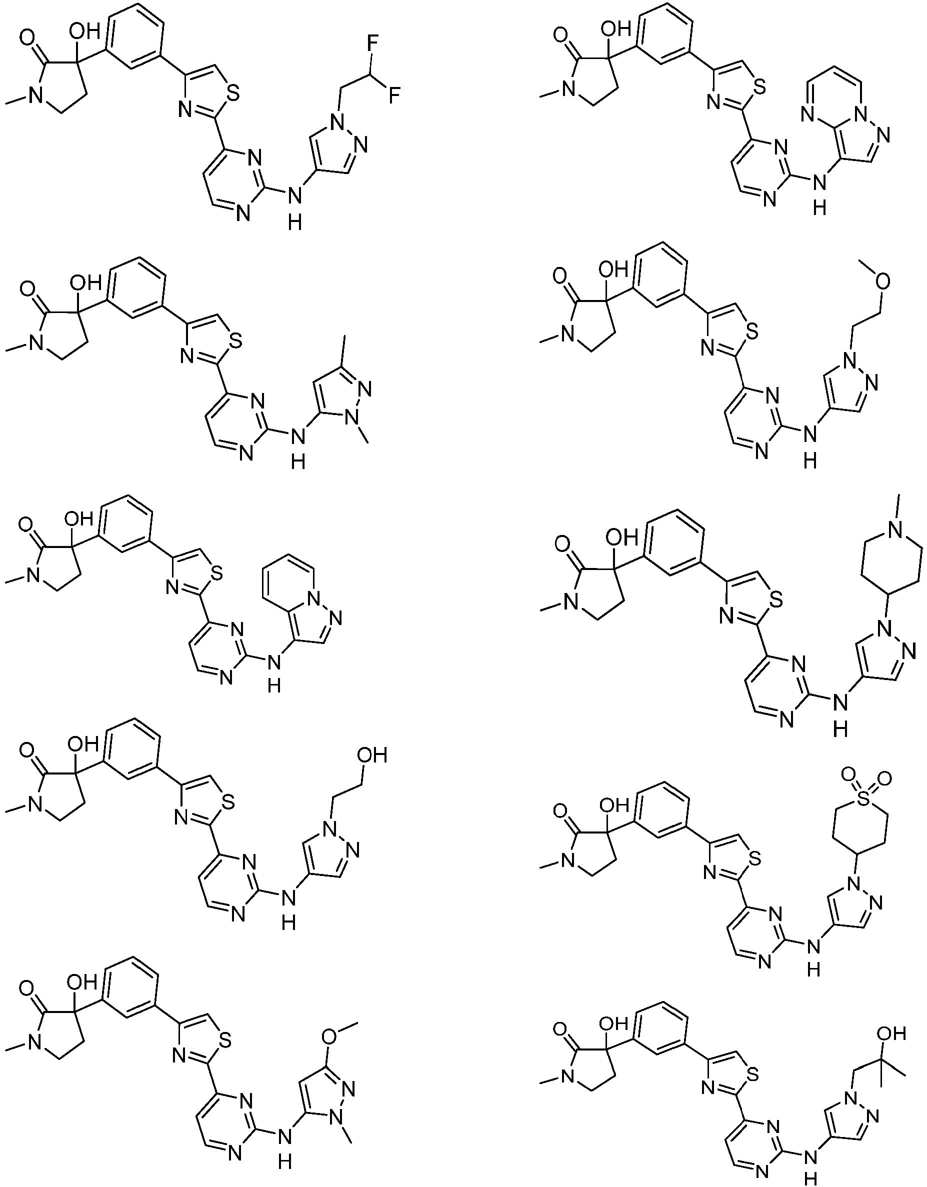

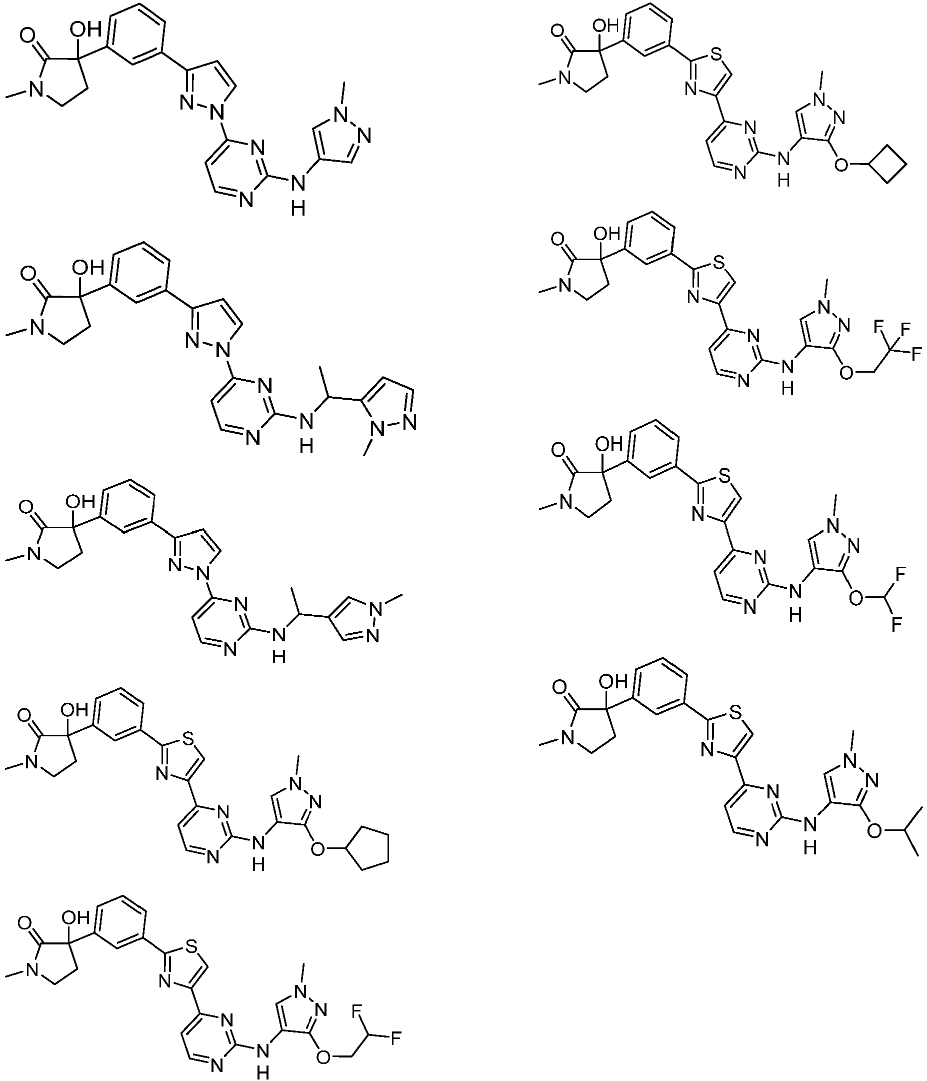

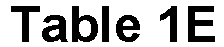

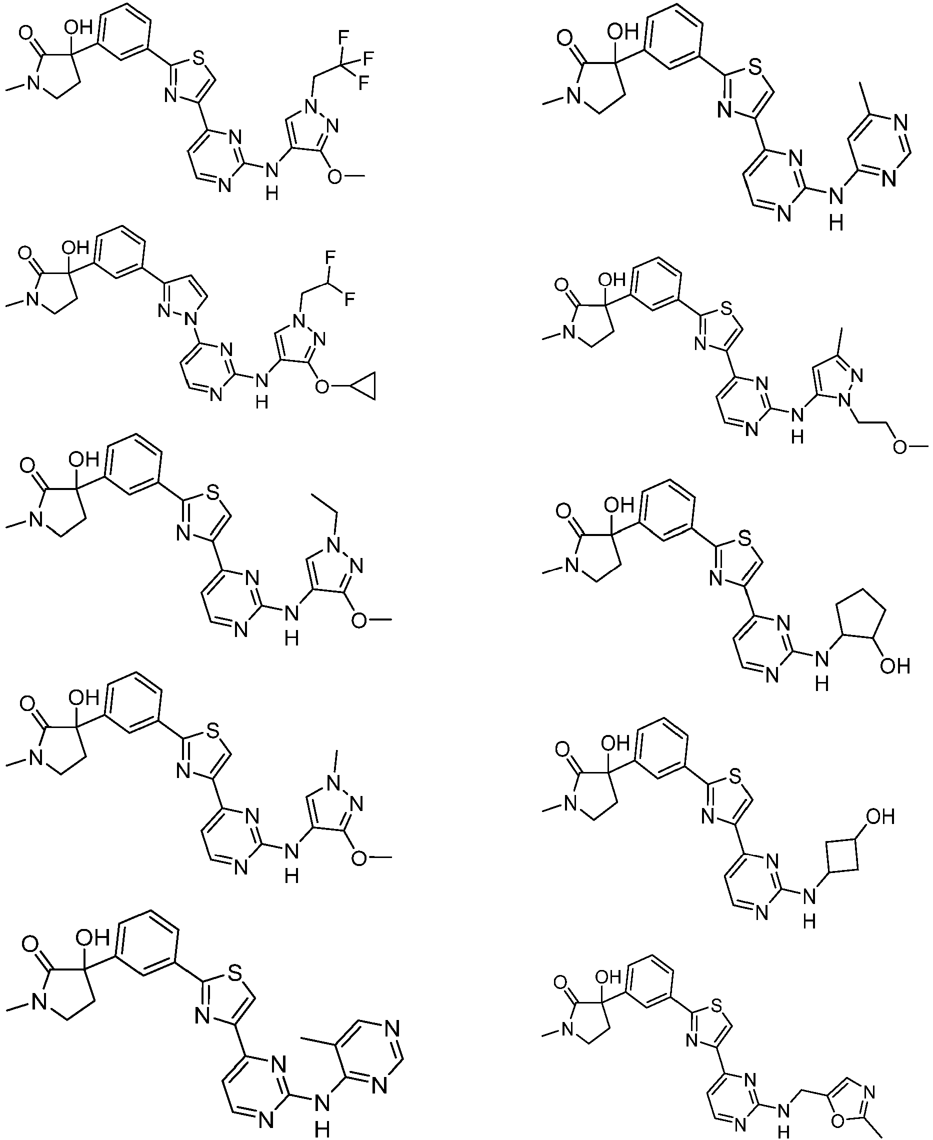

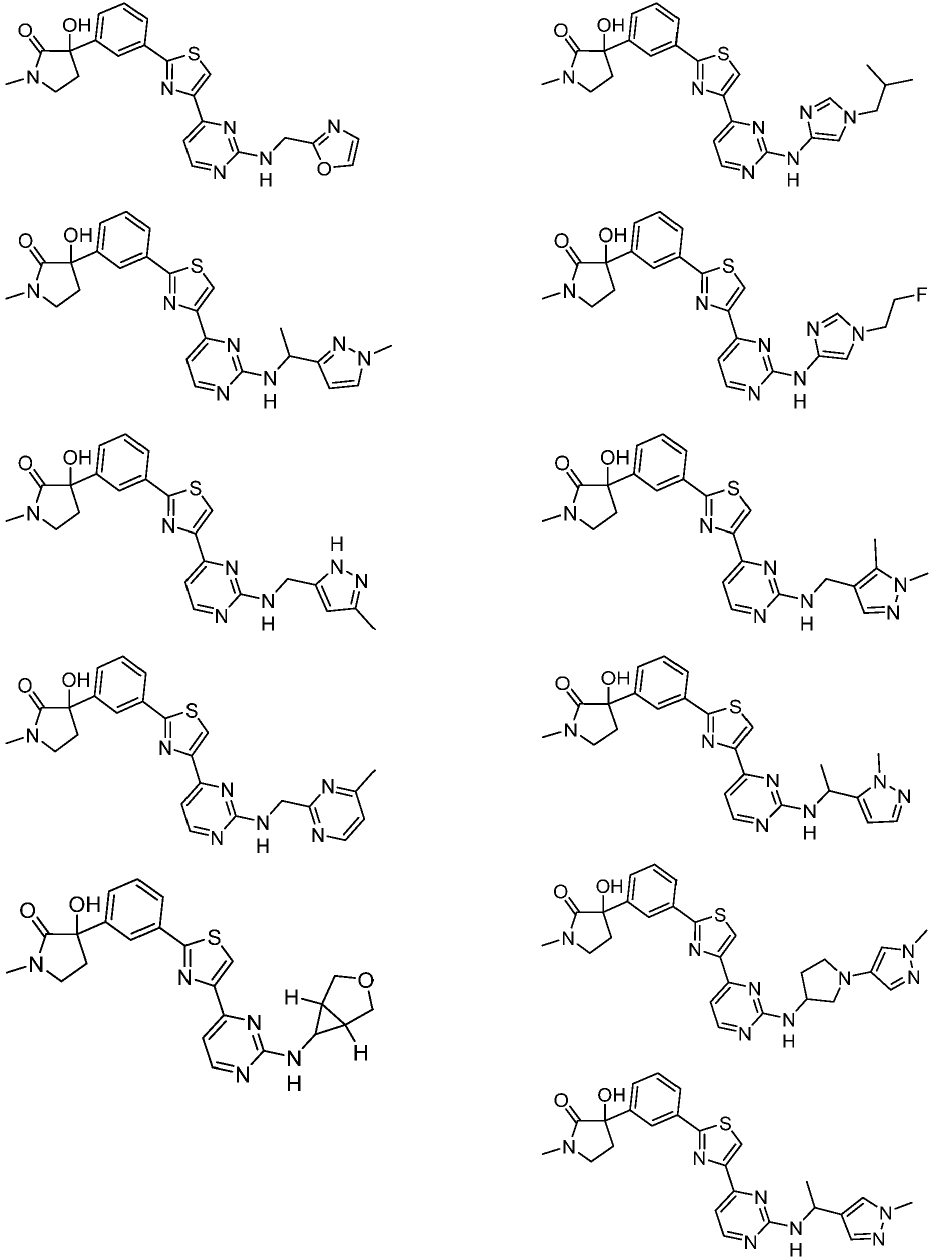

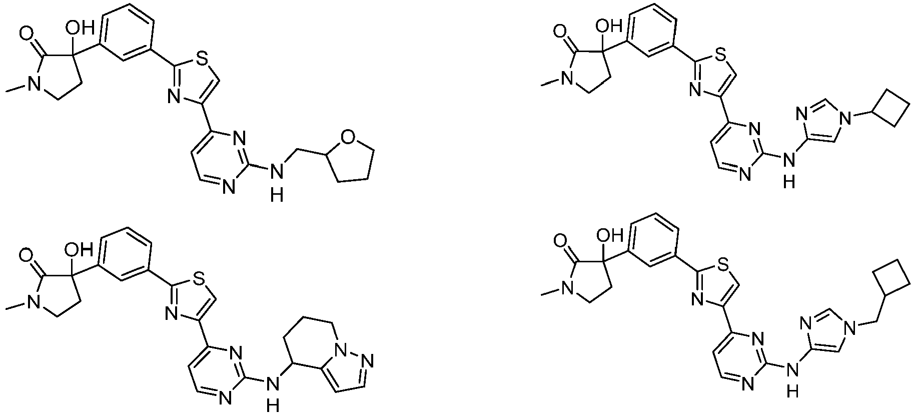

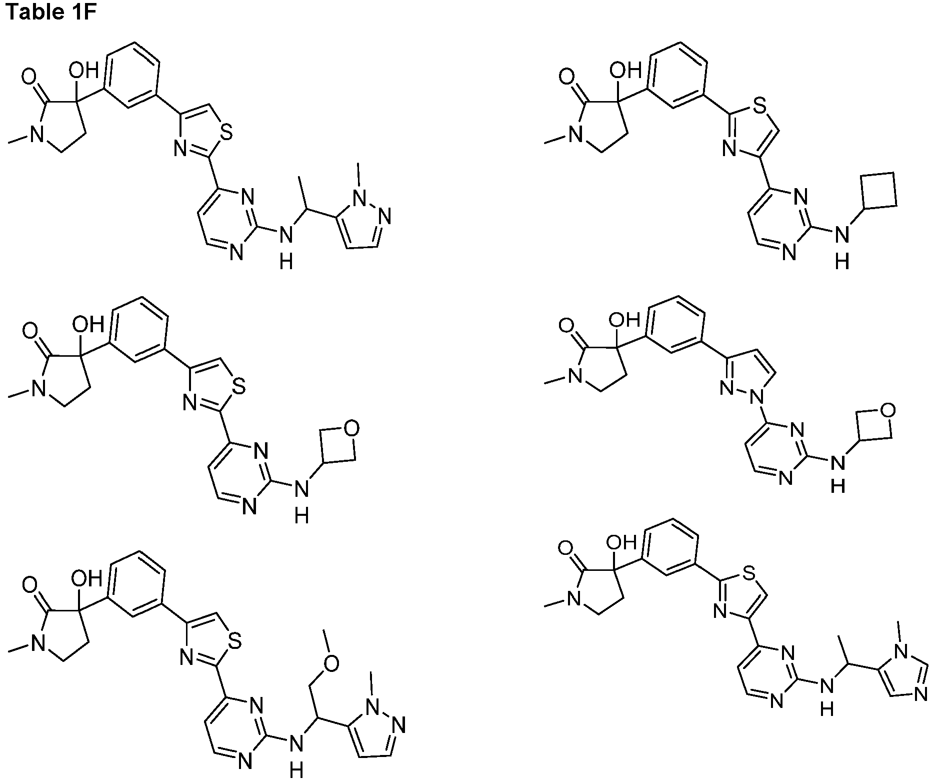

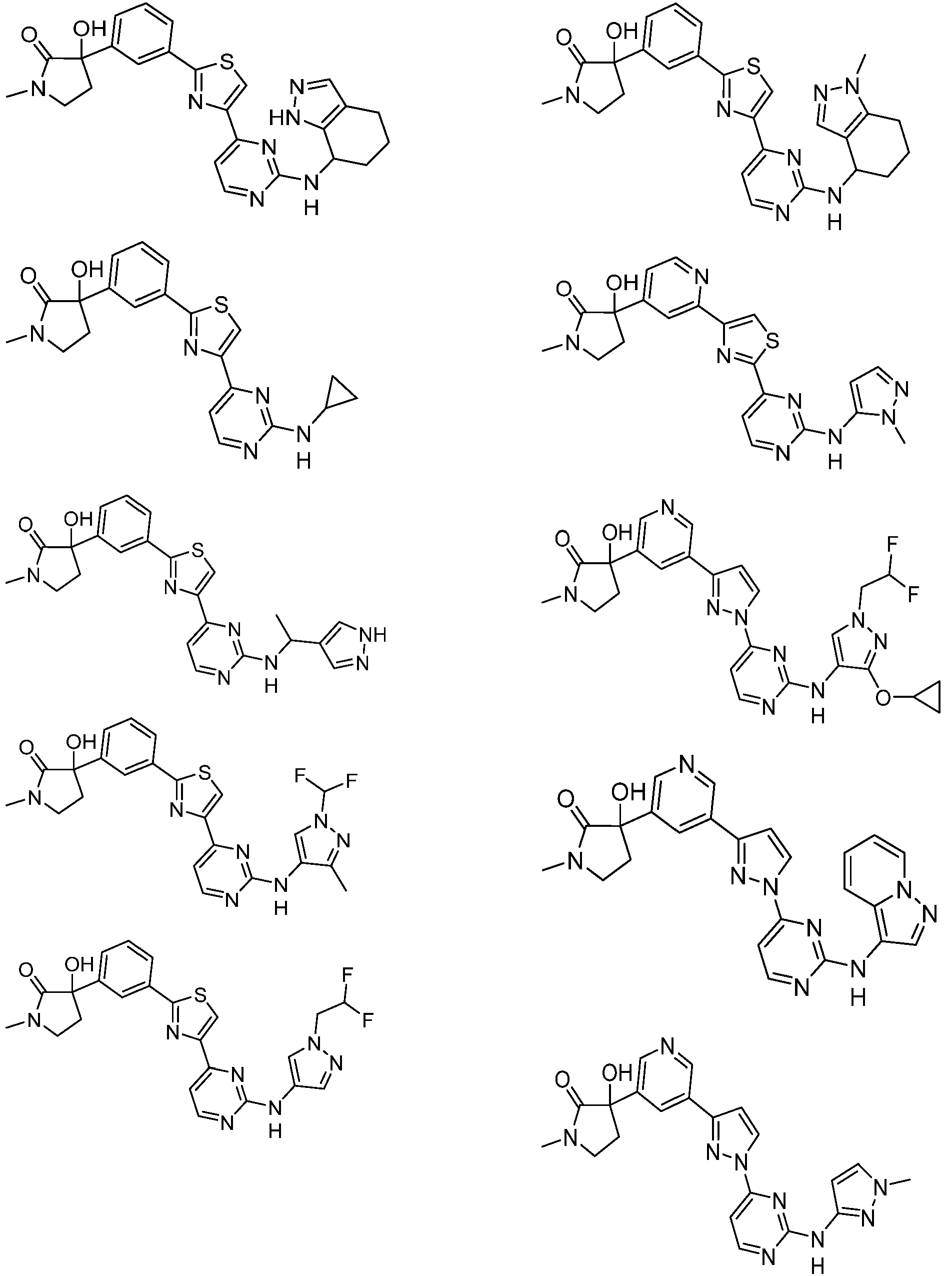

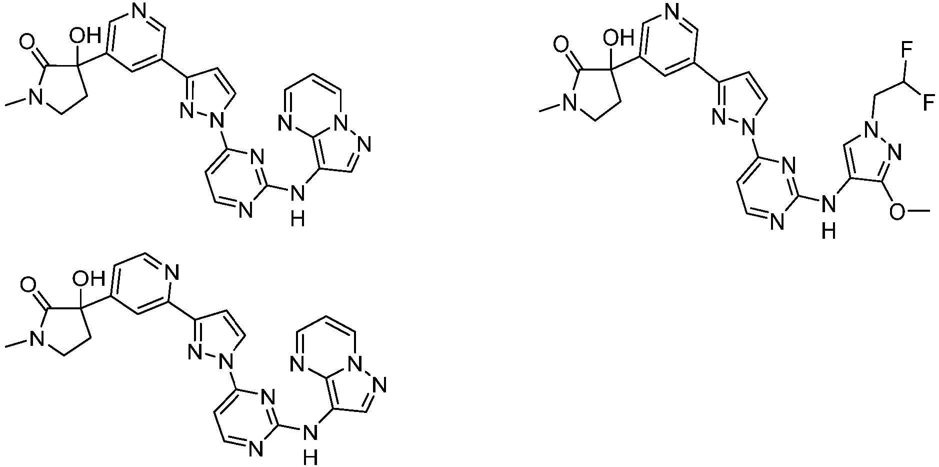

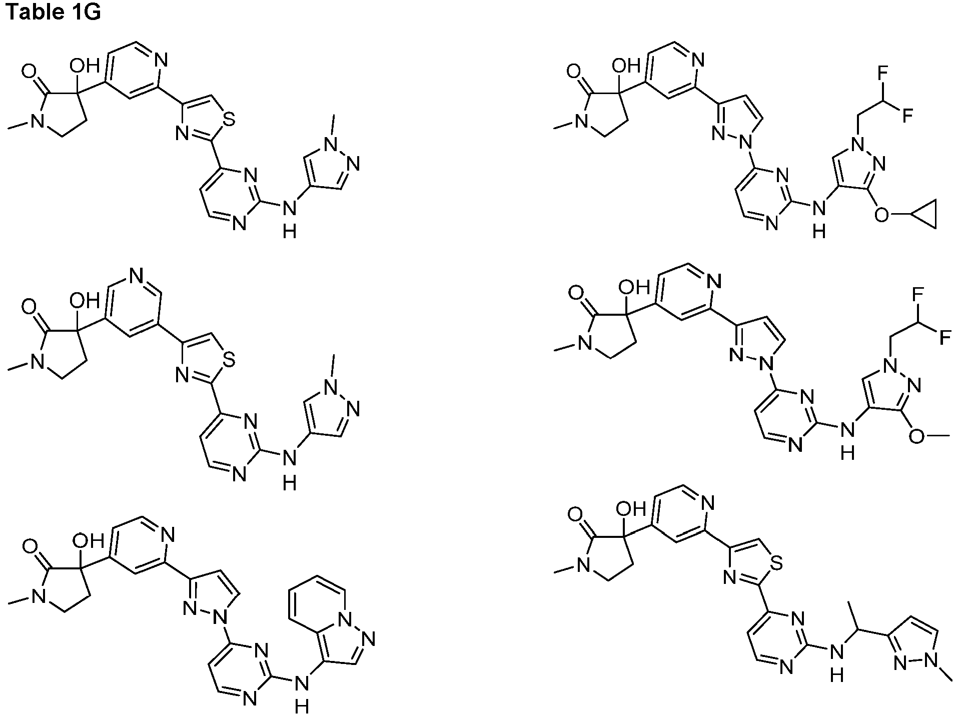

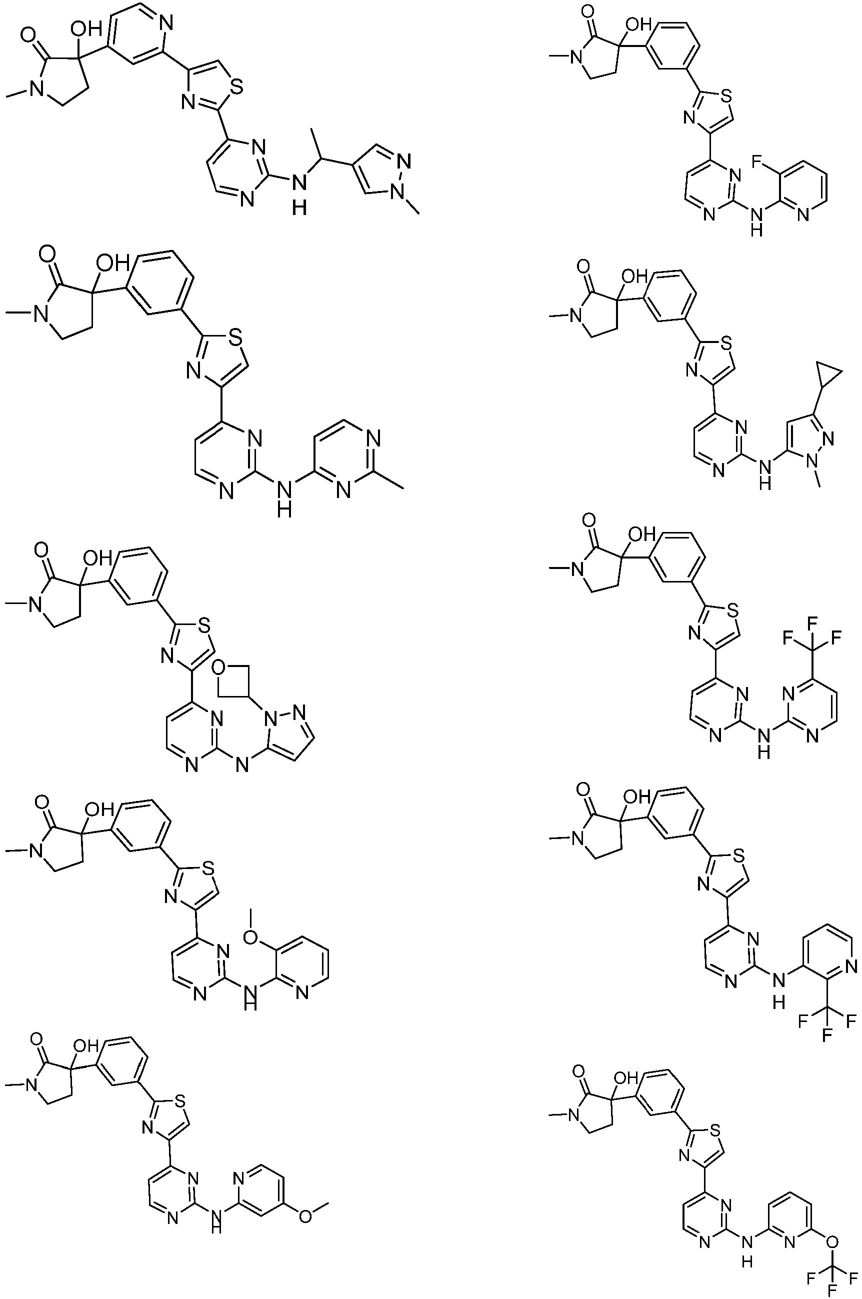

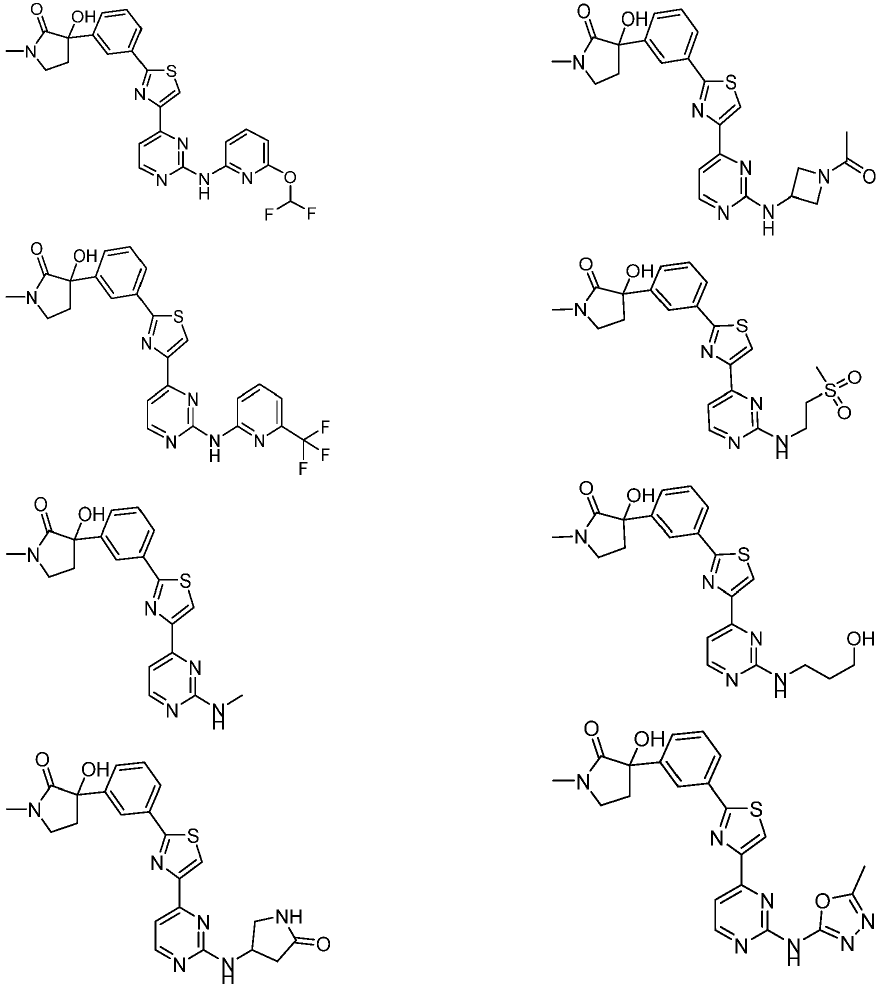

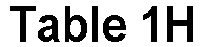

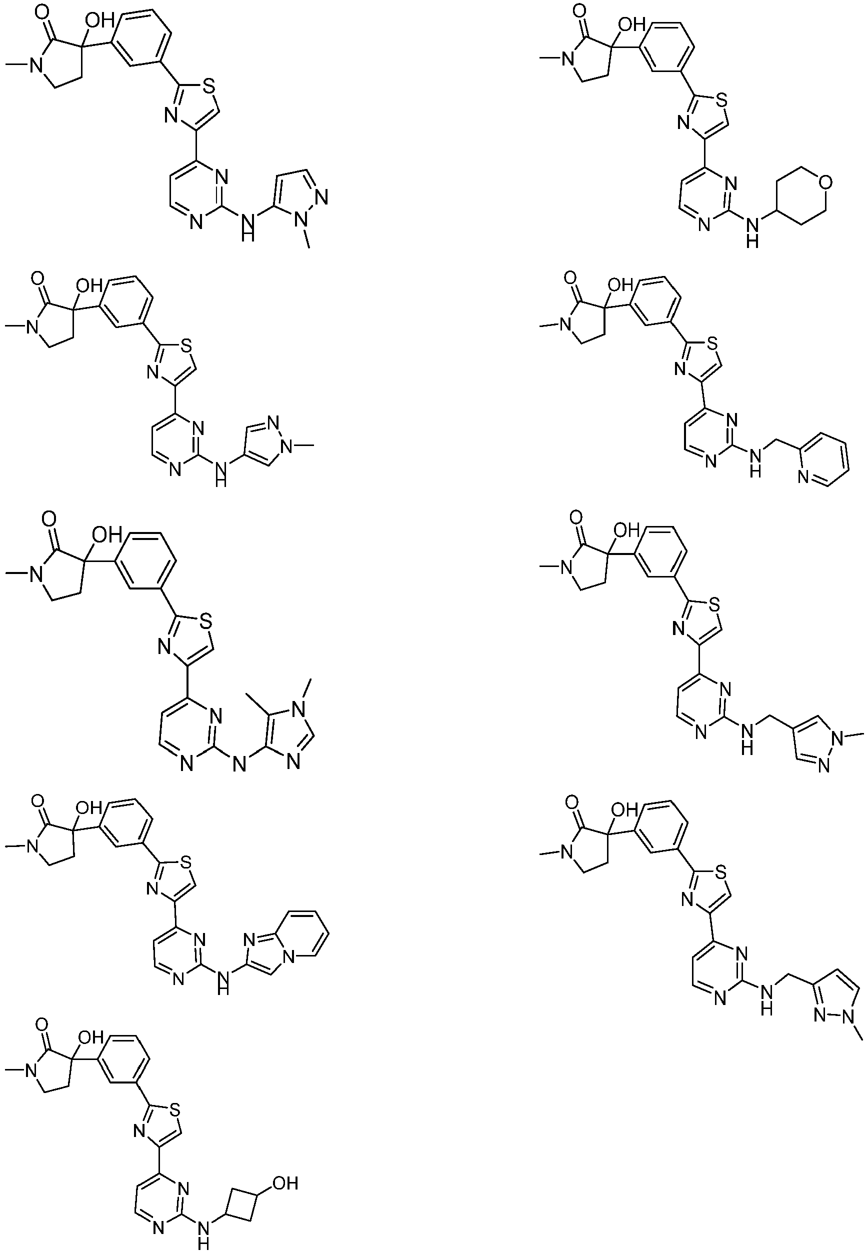

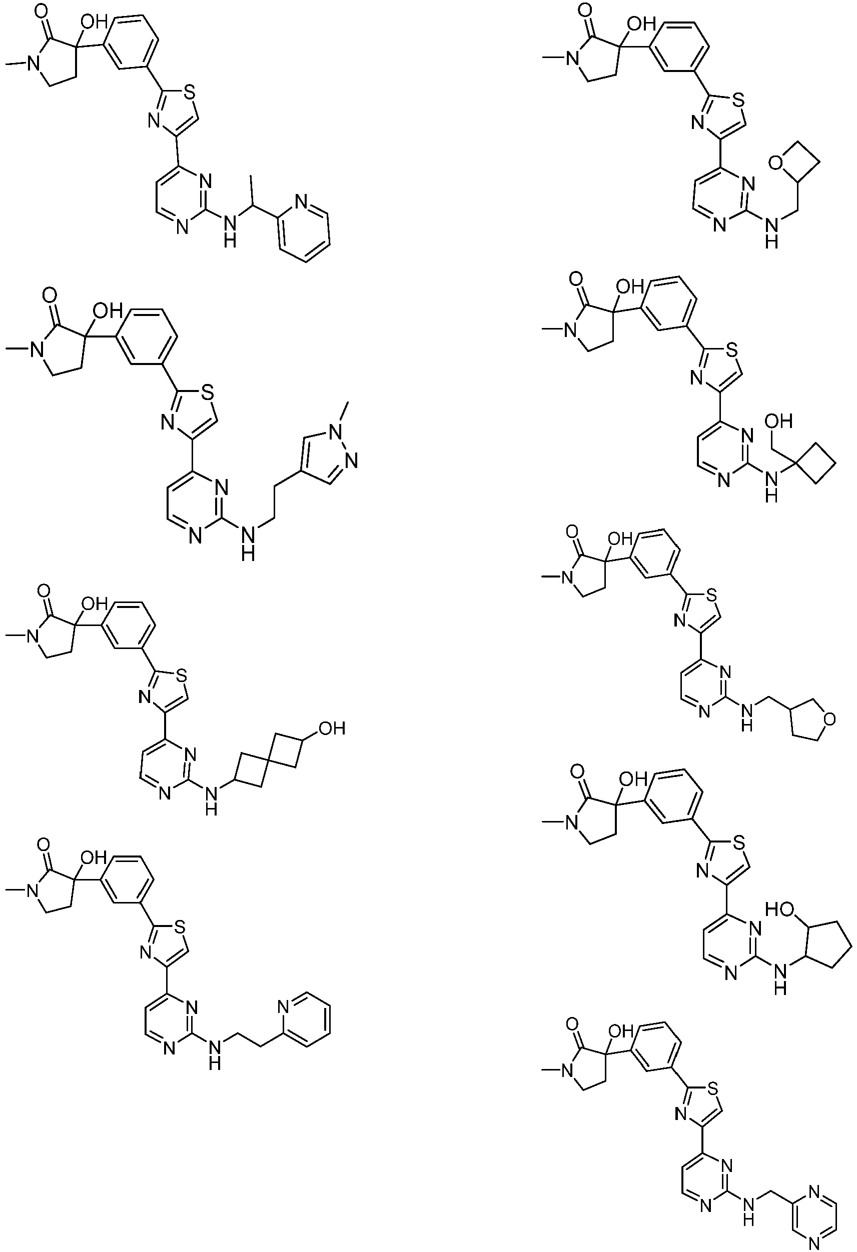

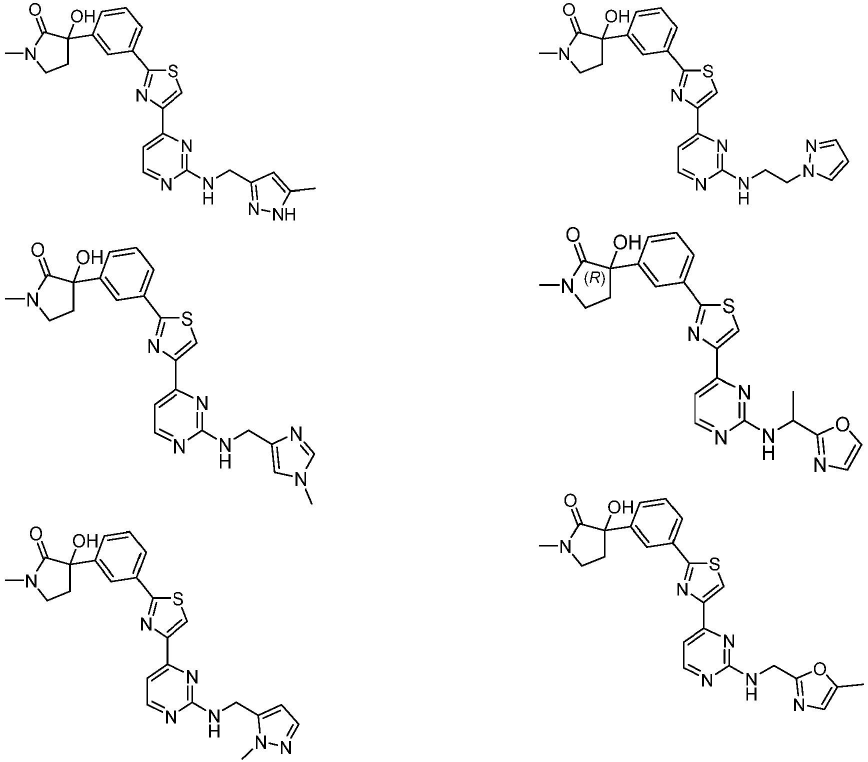

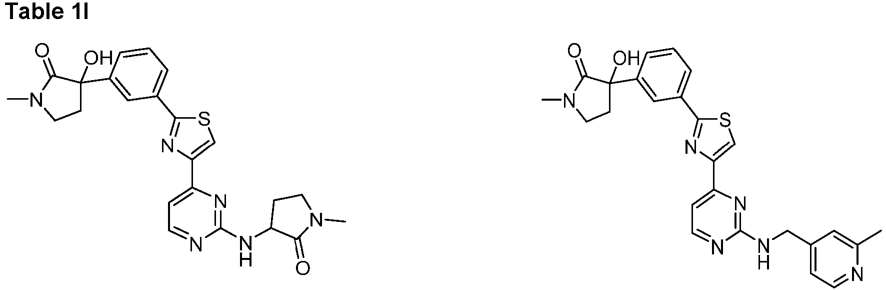

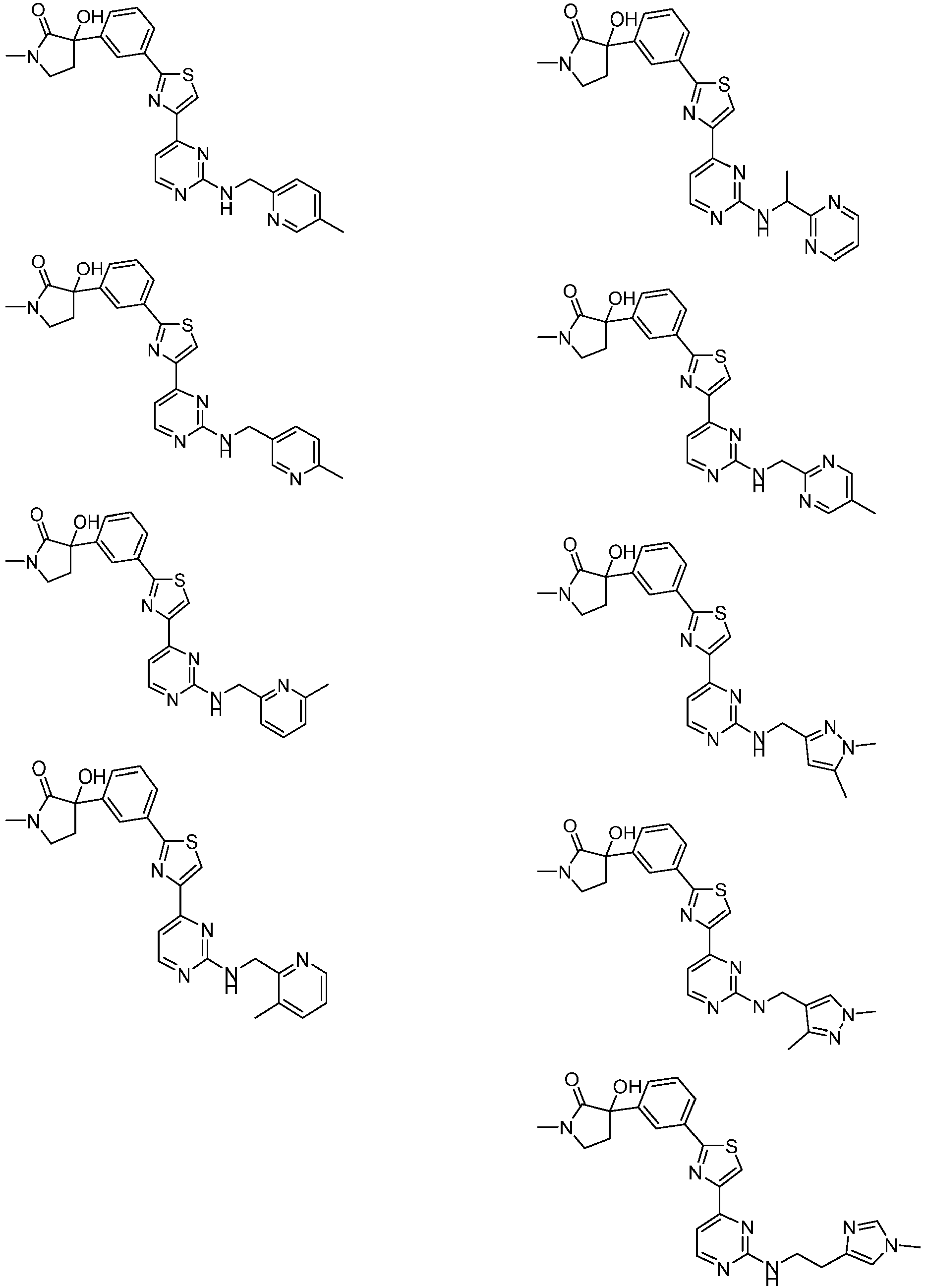

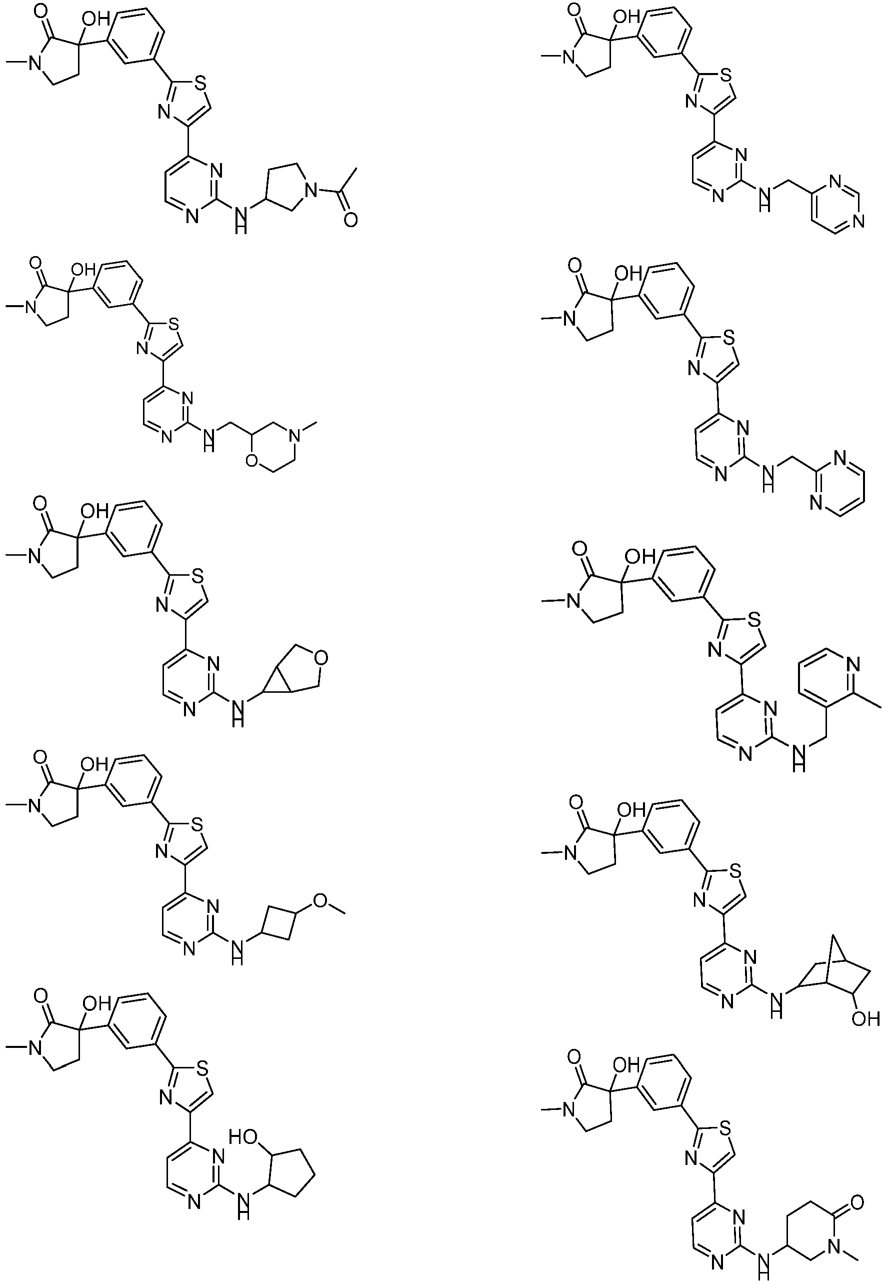

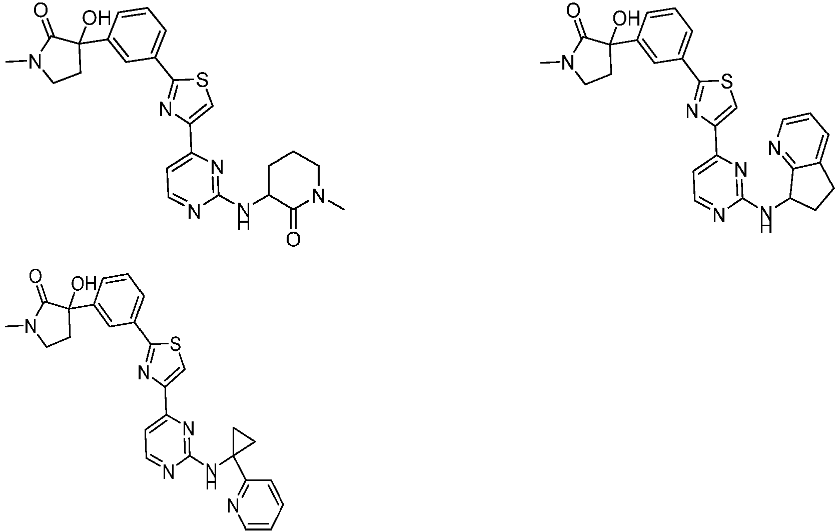

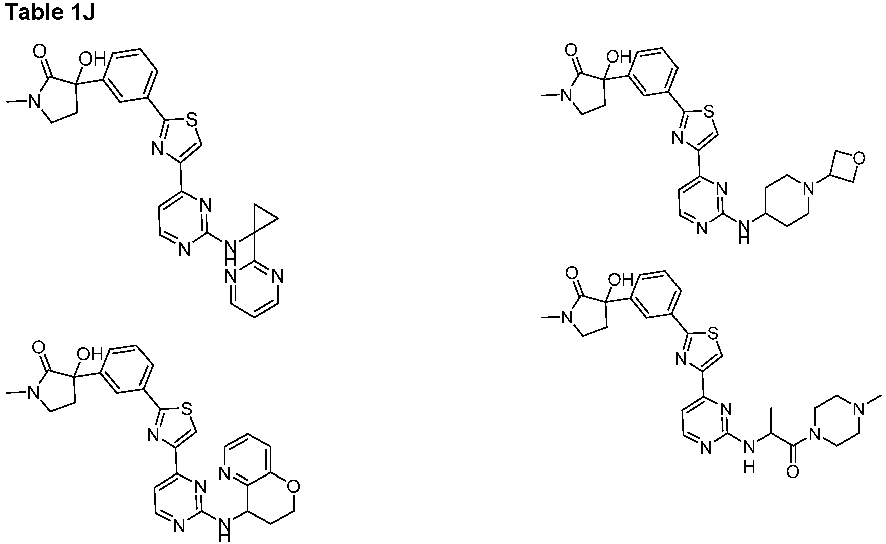

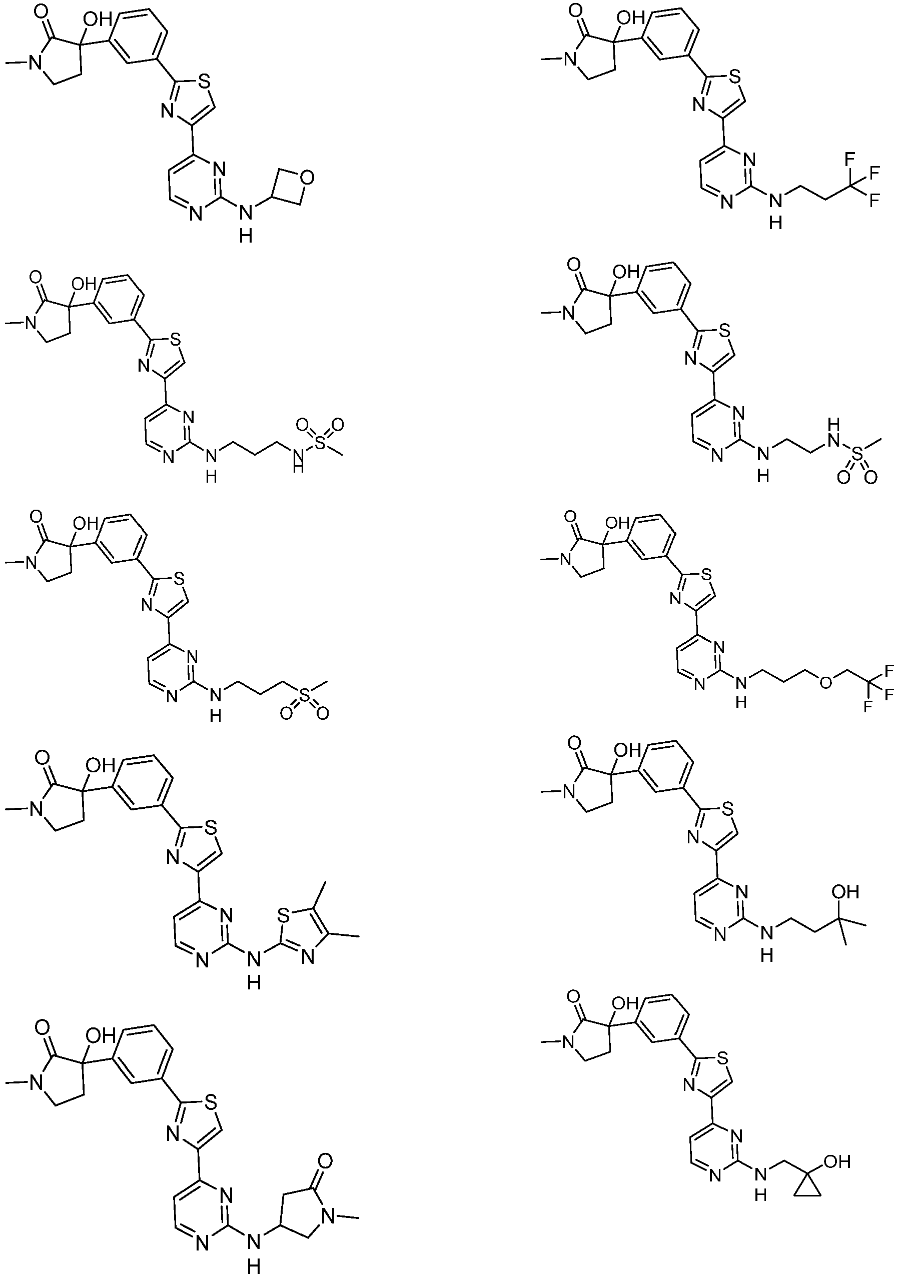

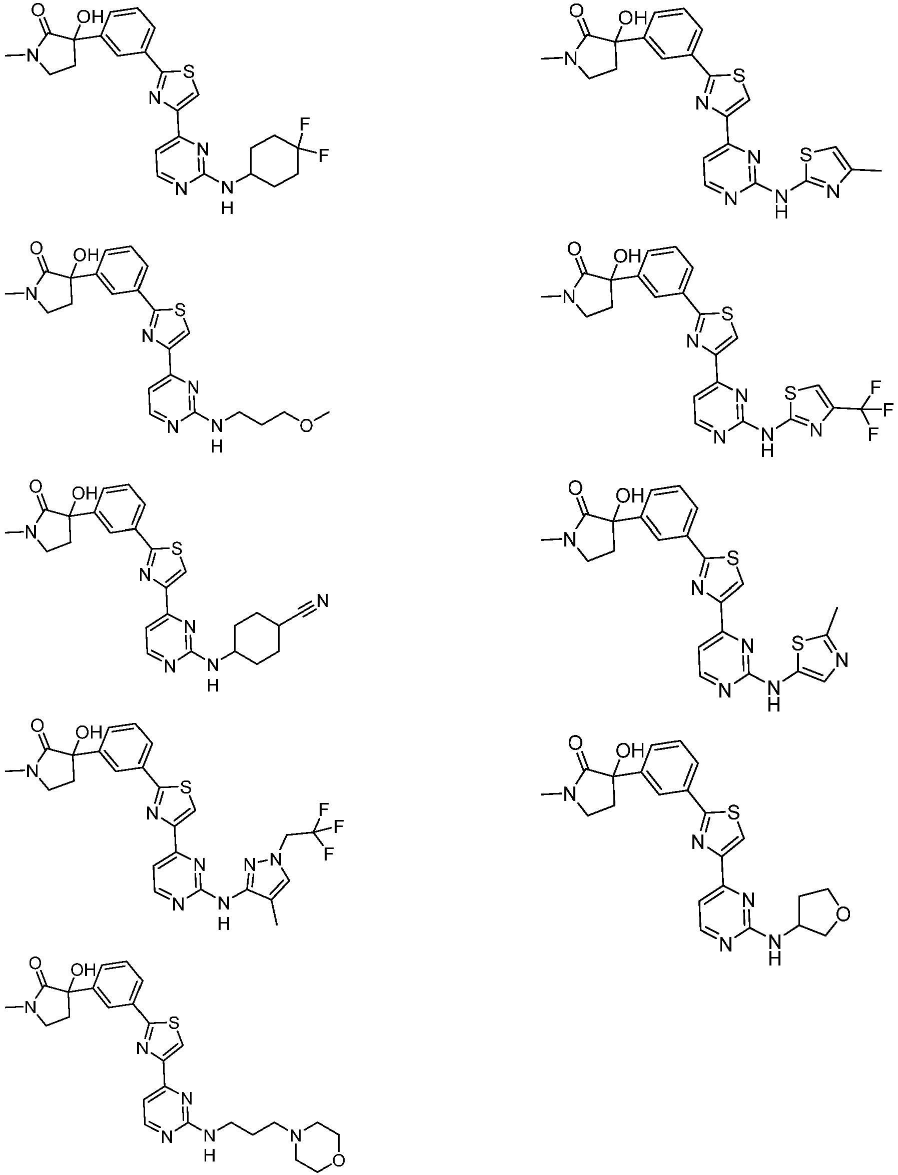

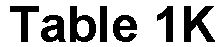

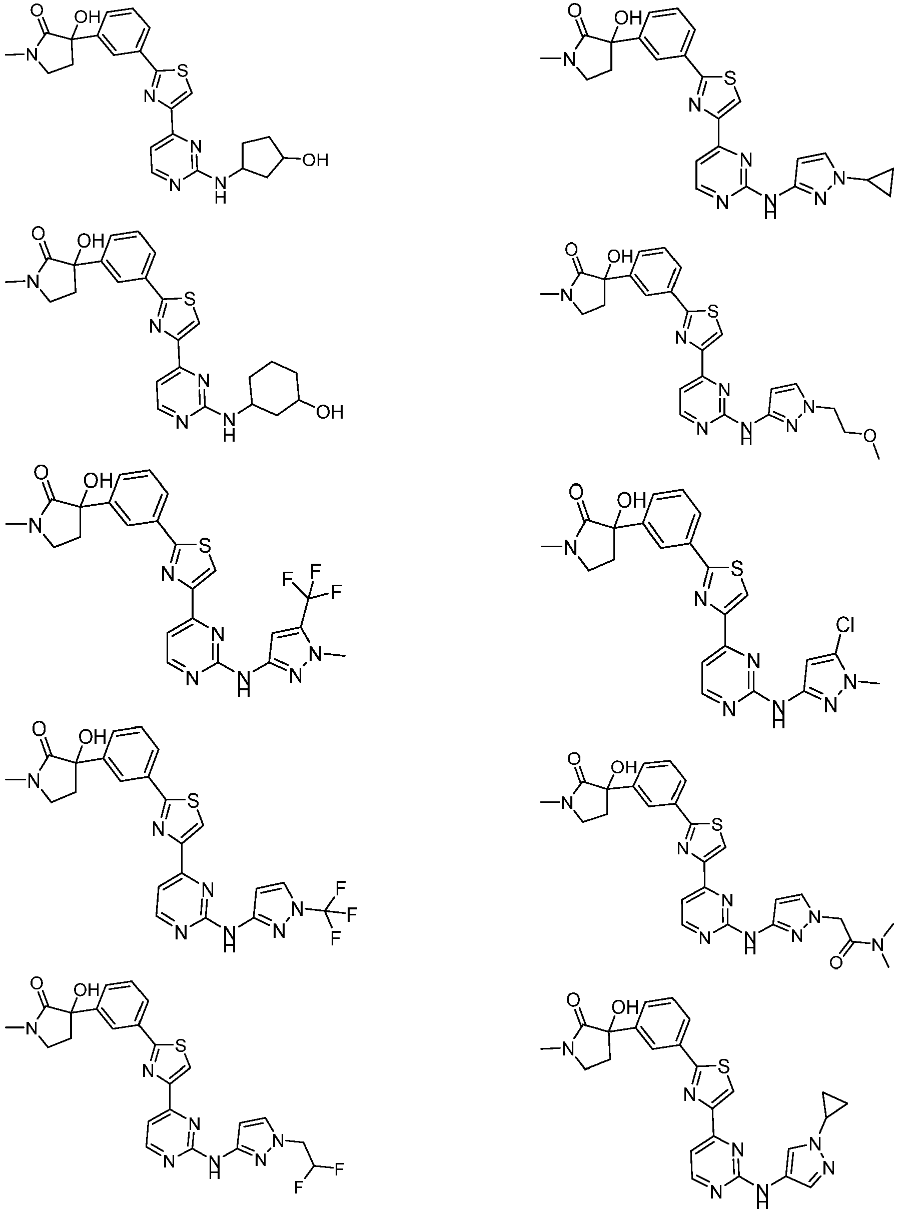

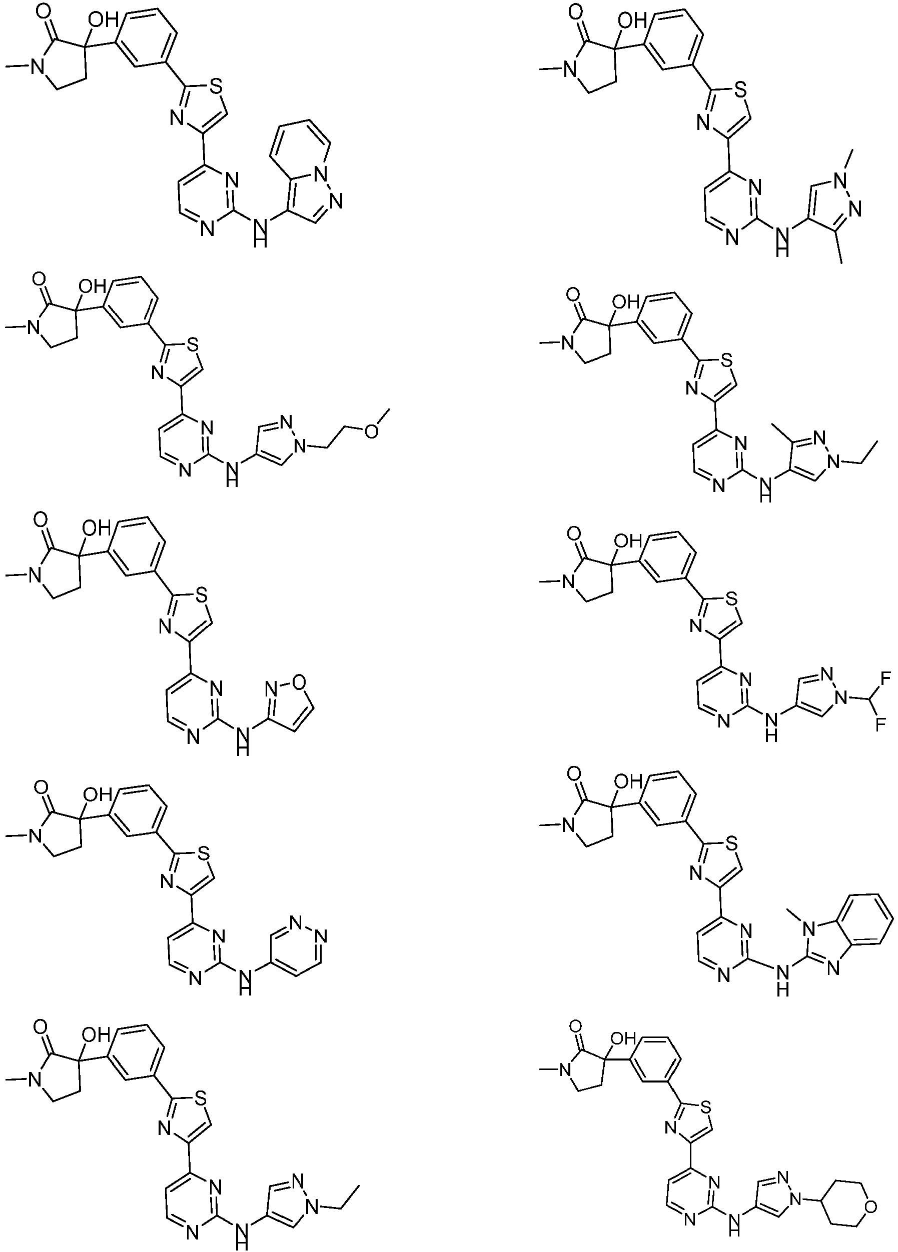

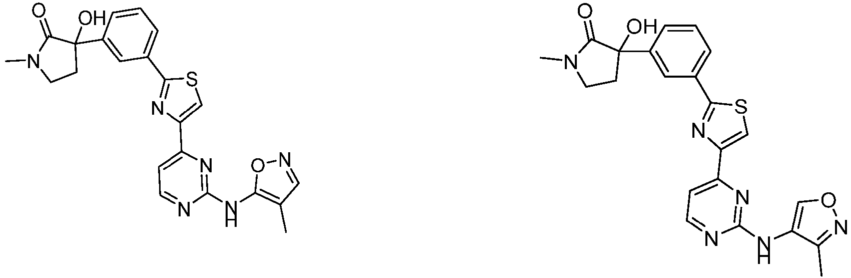

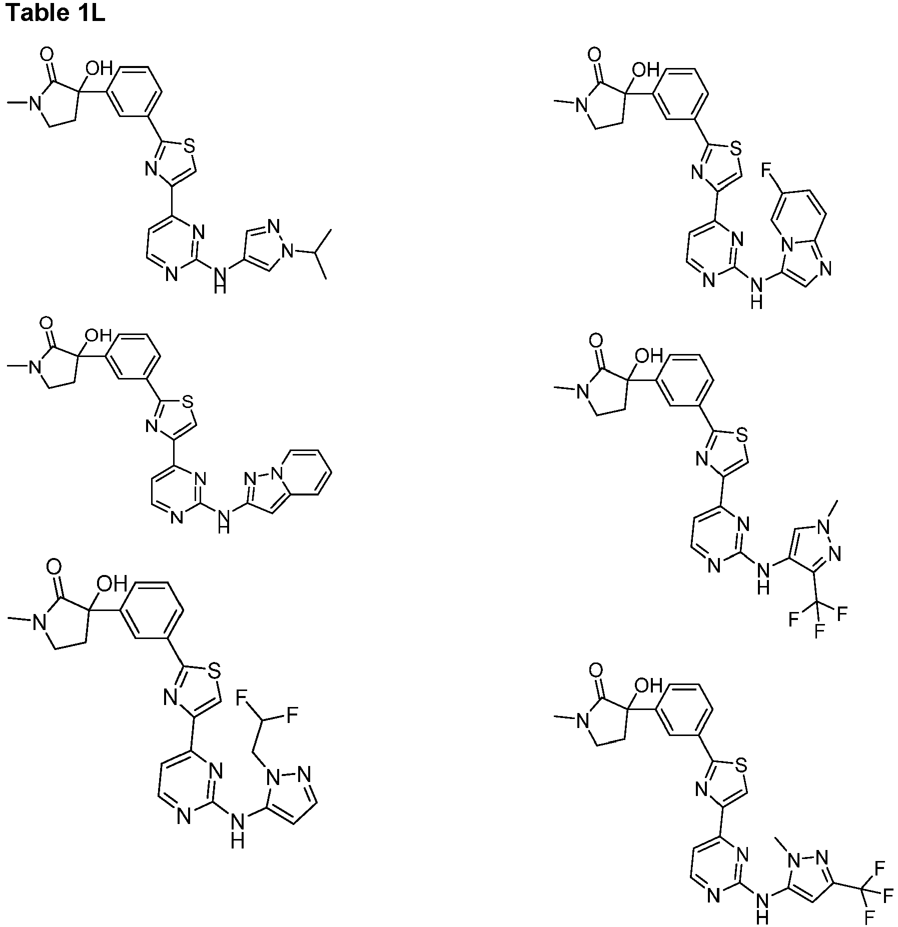

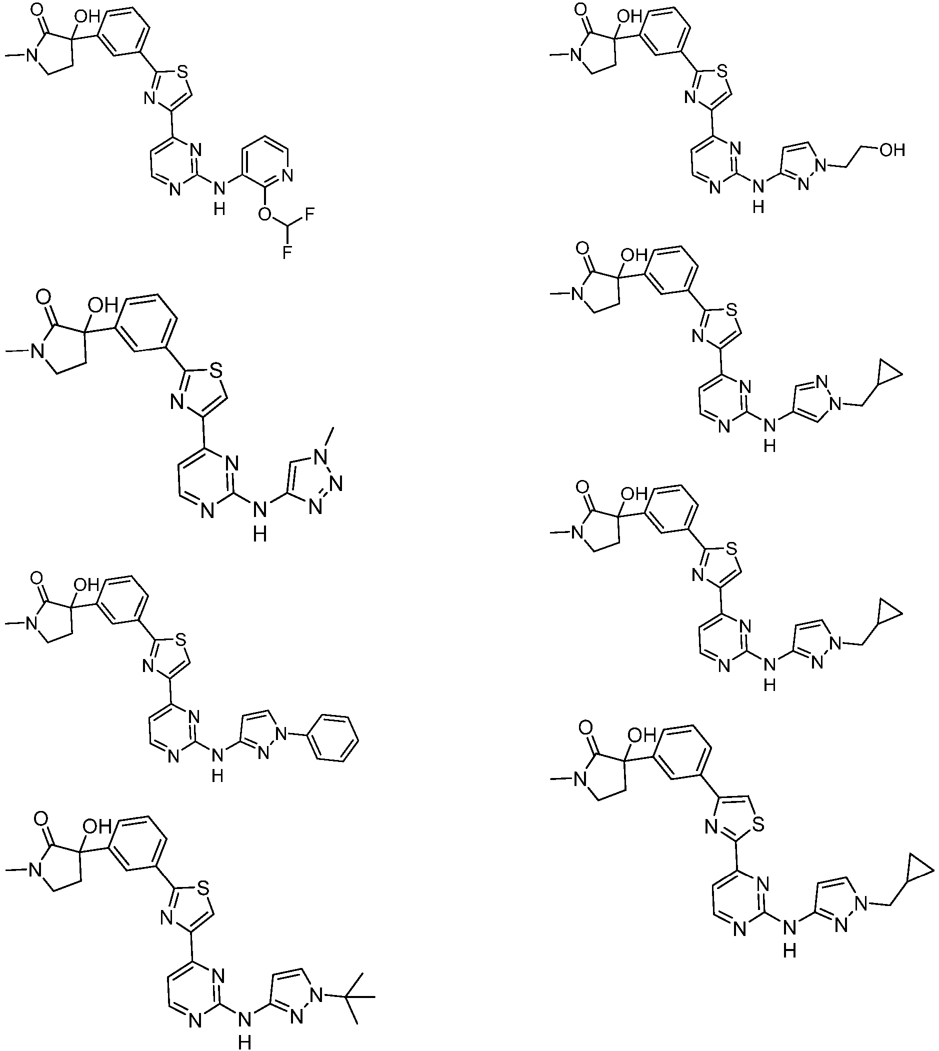

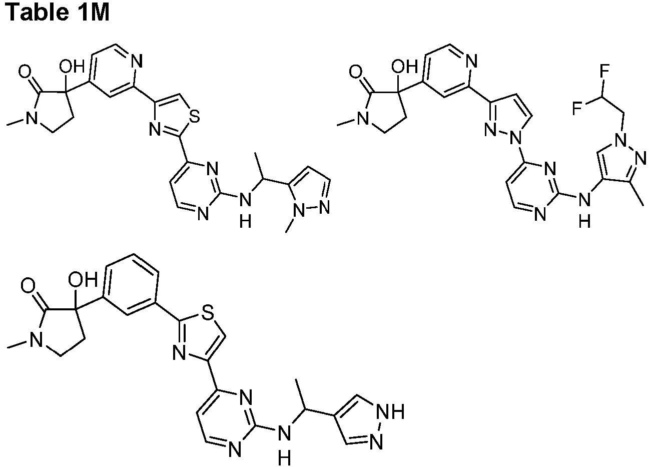

In some embodiments, disclosed herein is a compound of Formula I, or a pharmaceutically acceptable salt thereof, having a structure as shown in any one of Tables 2A to 2M.

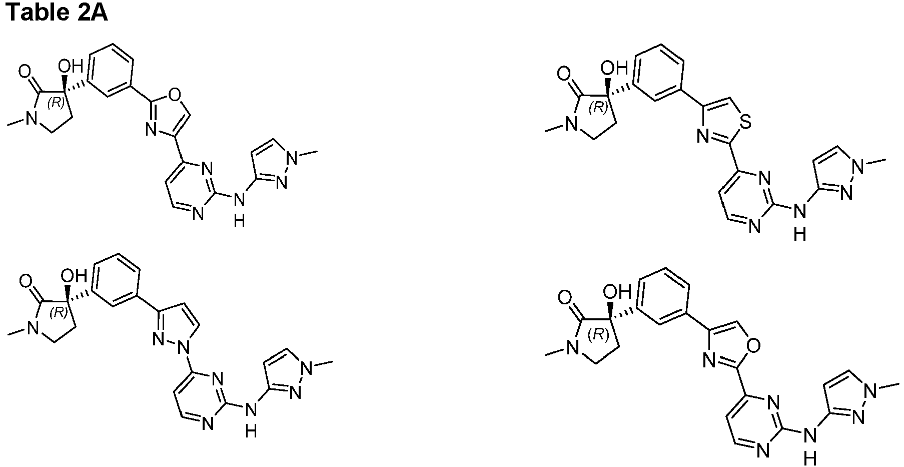

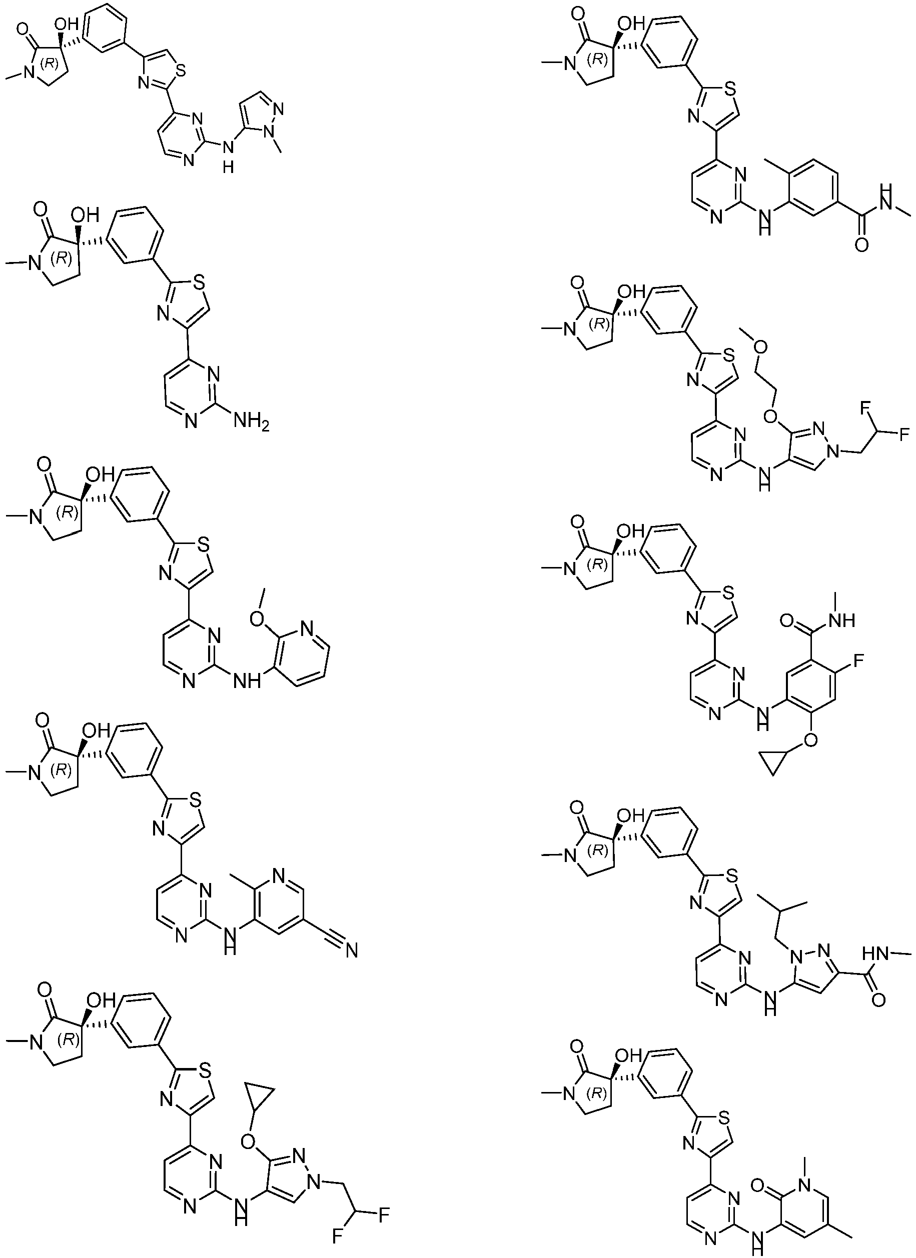

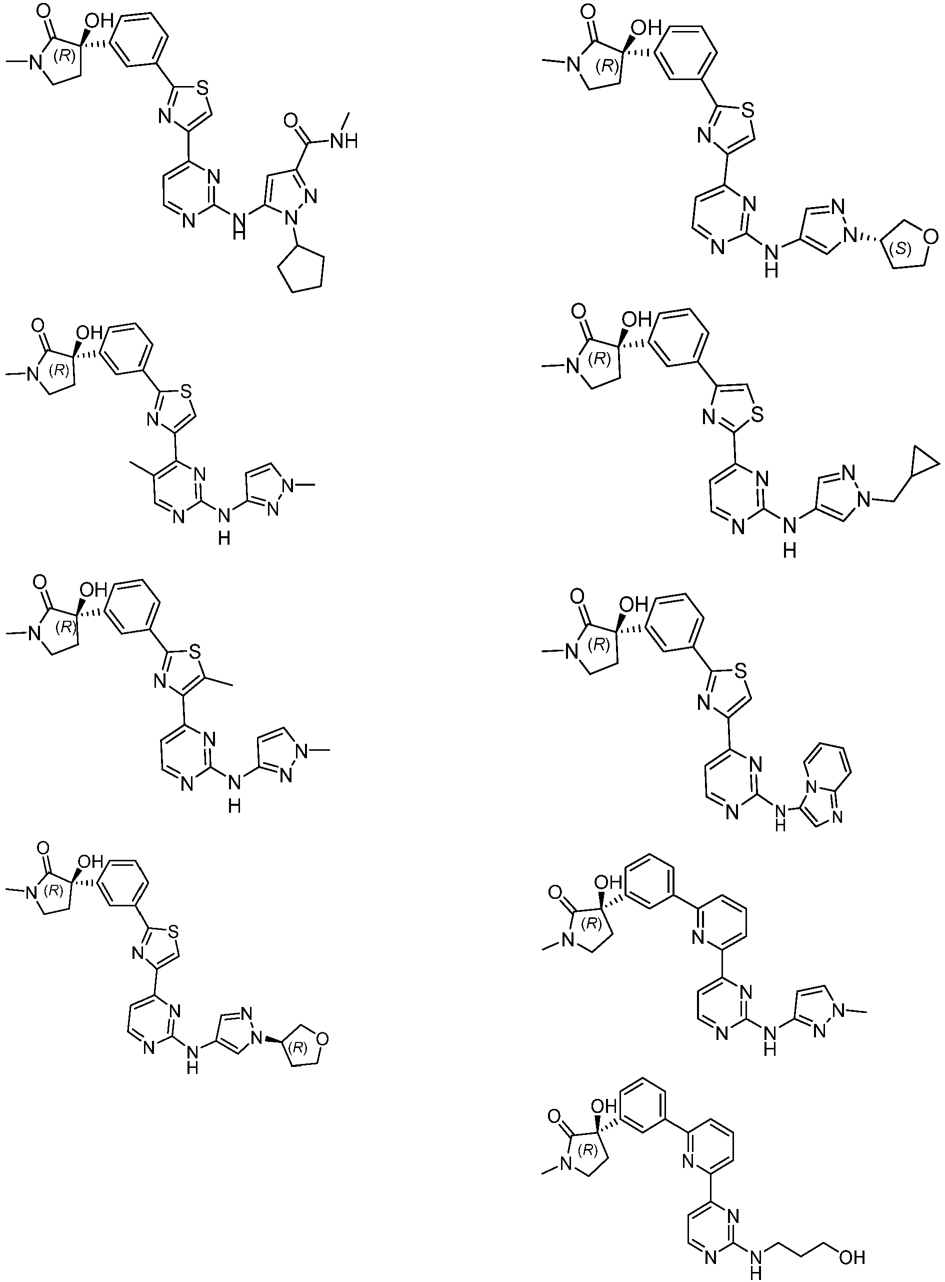

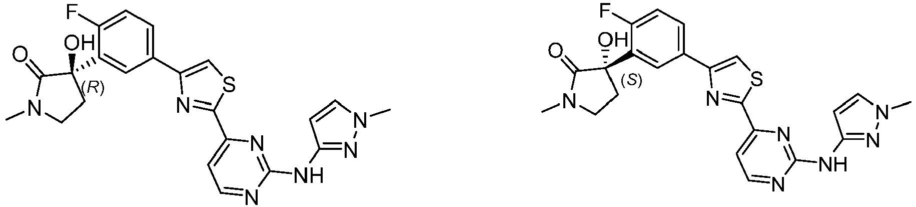

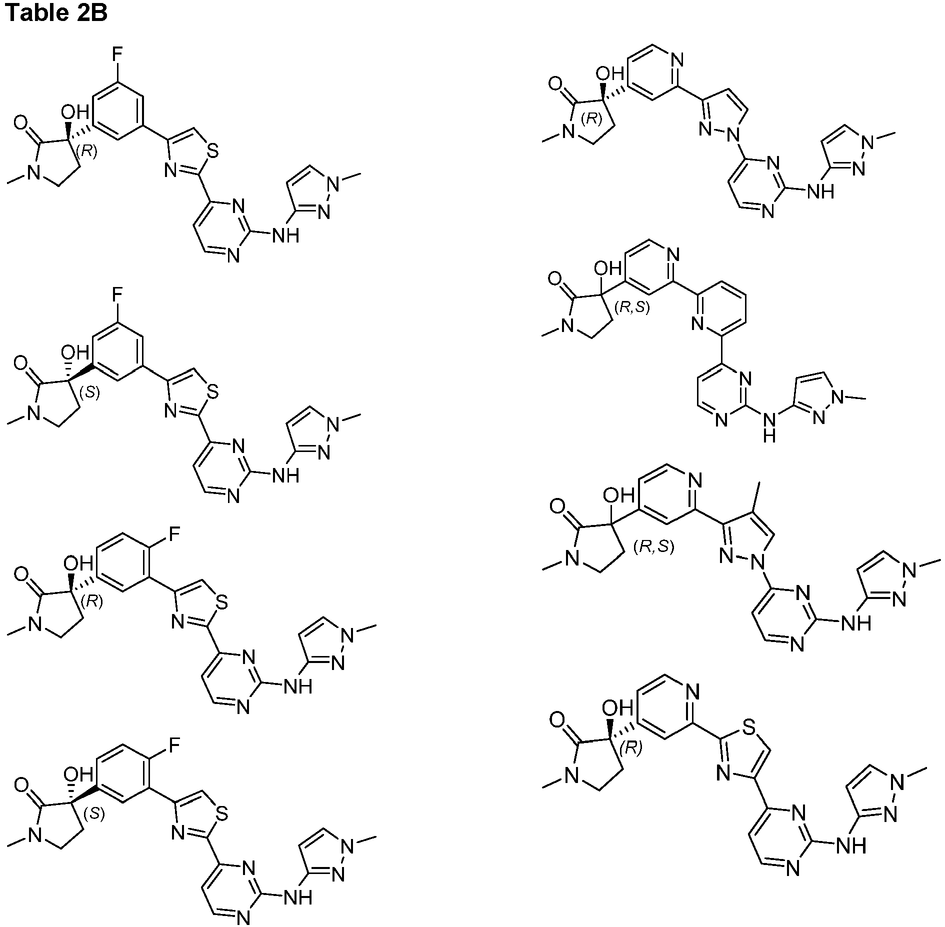

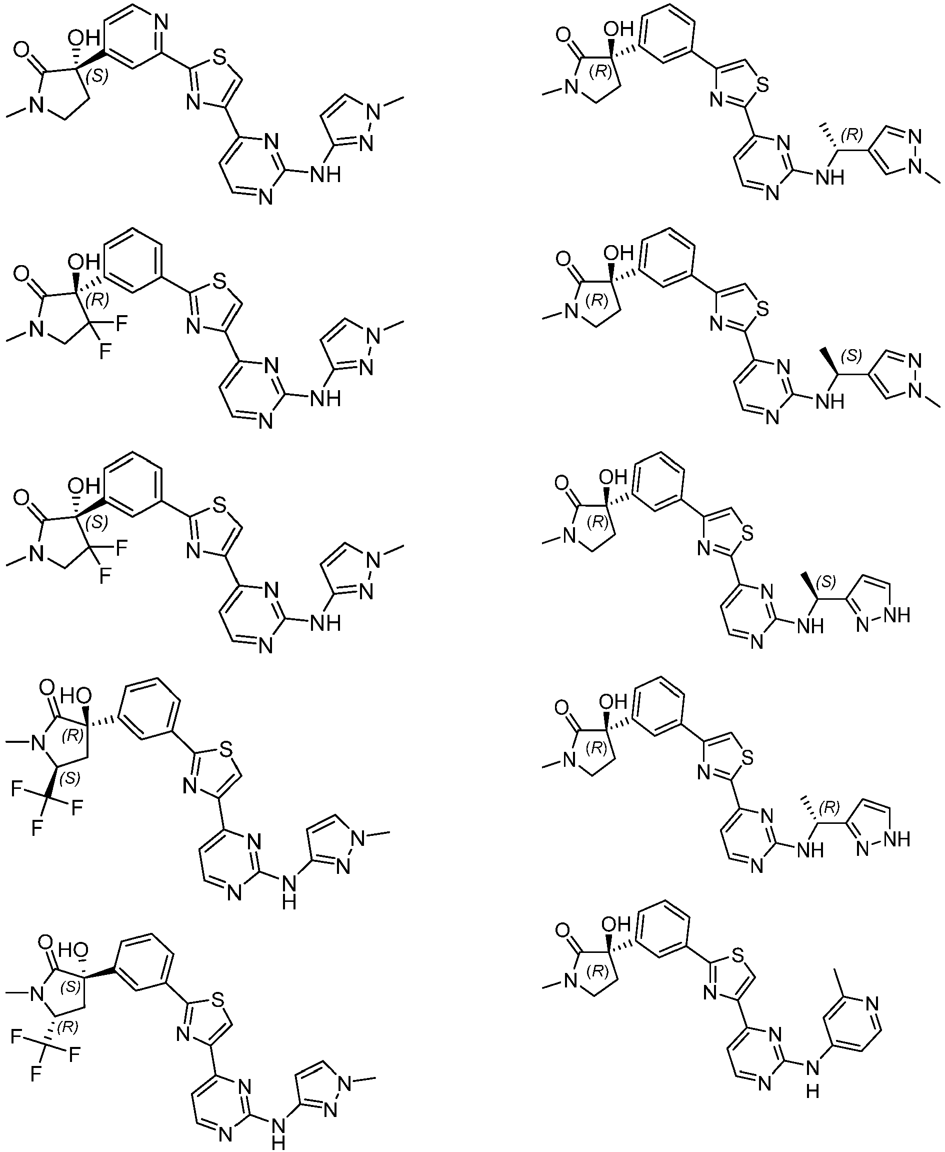

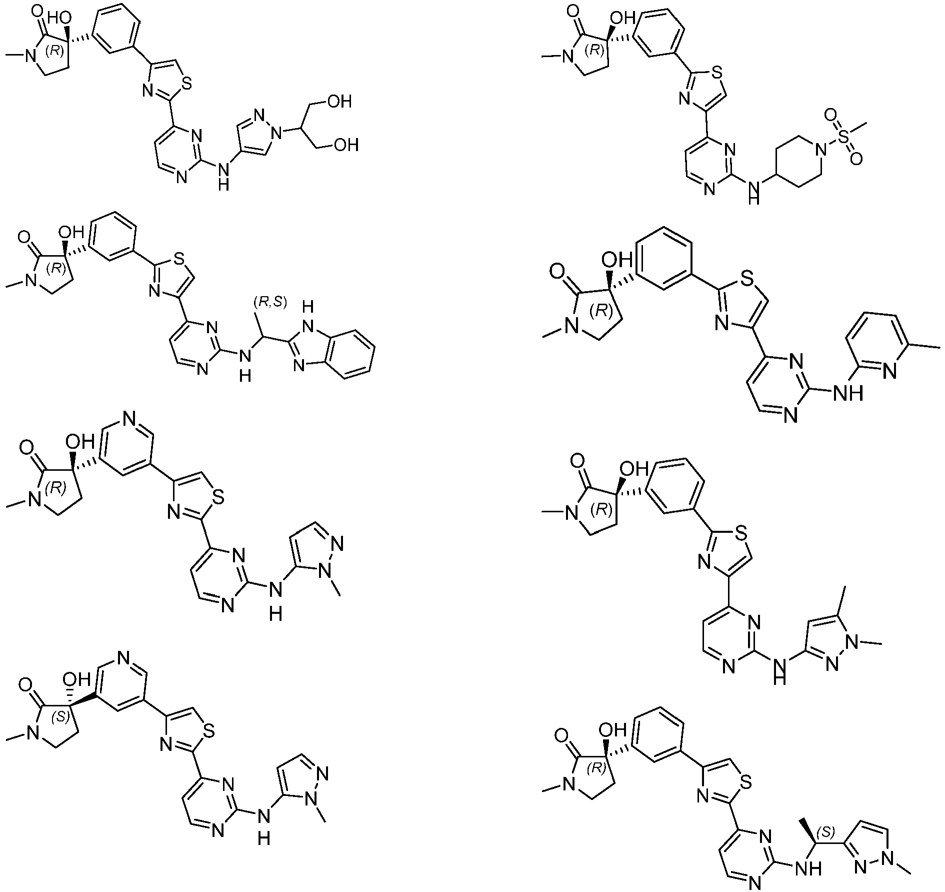

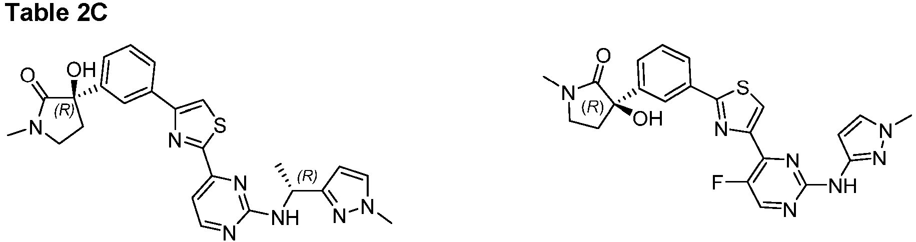

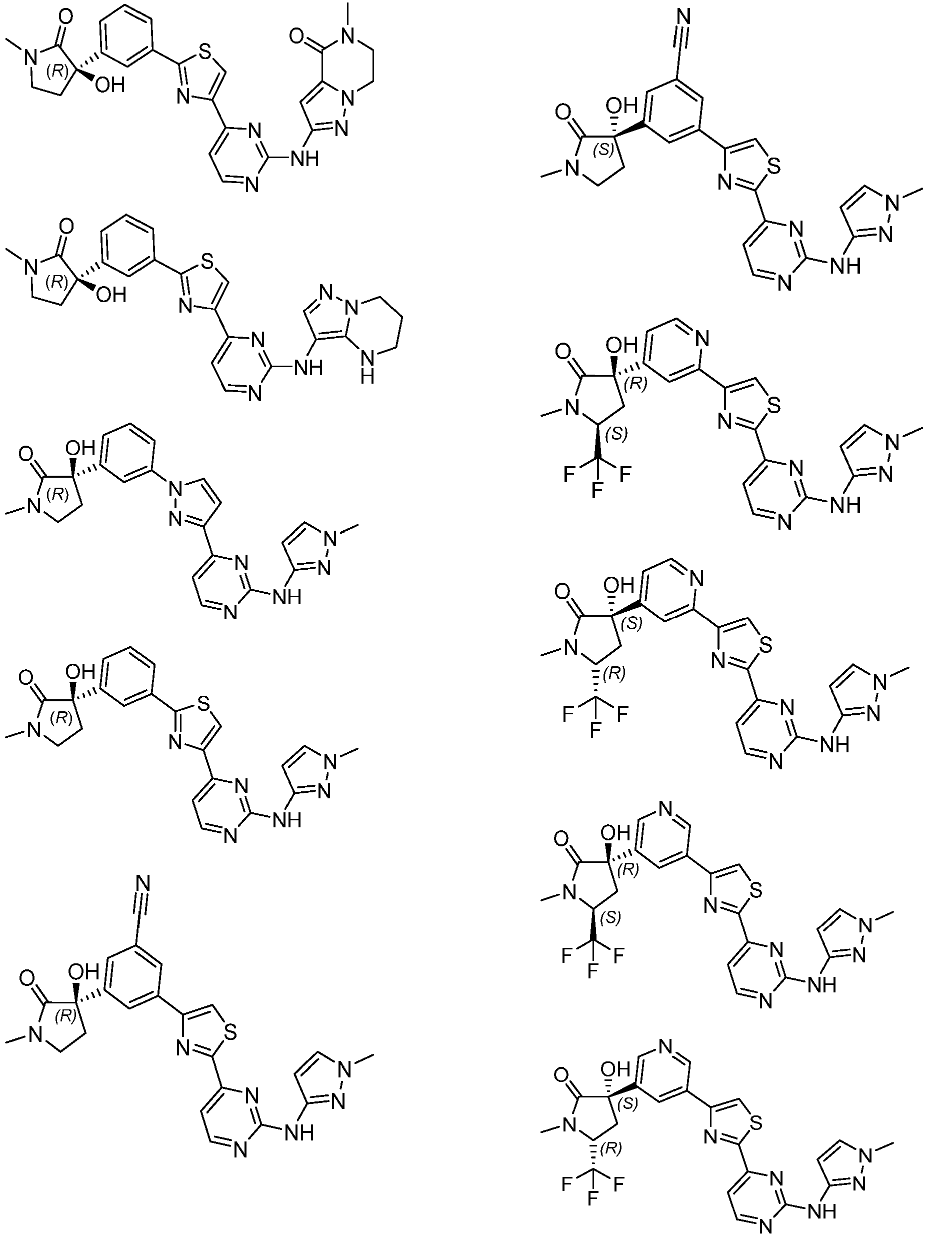

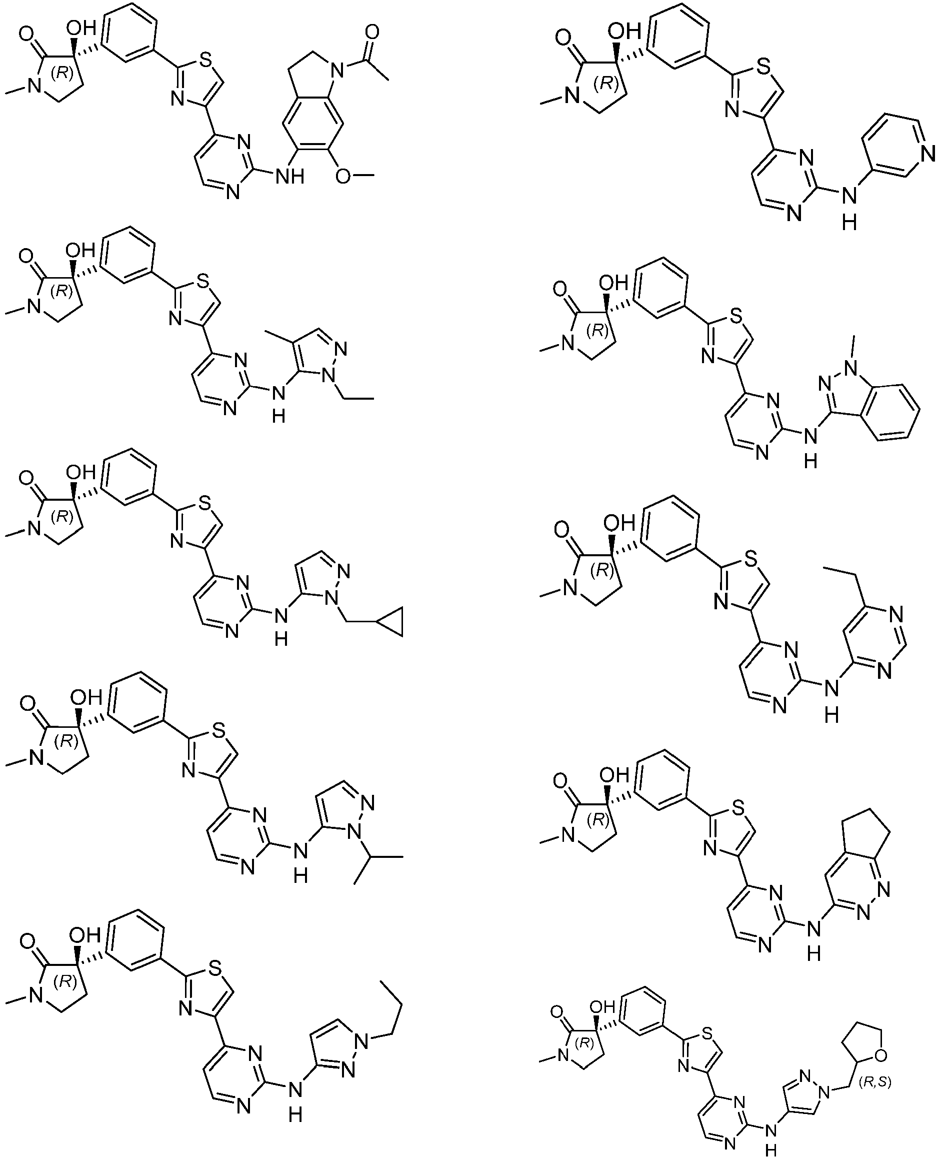

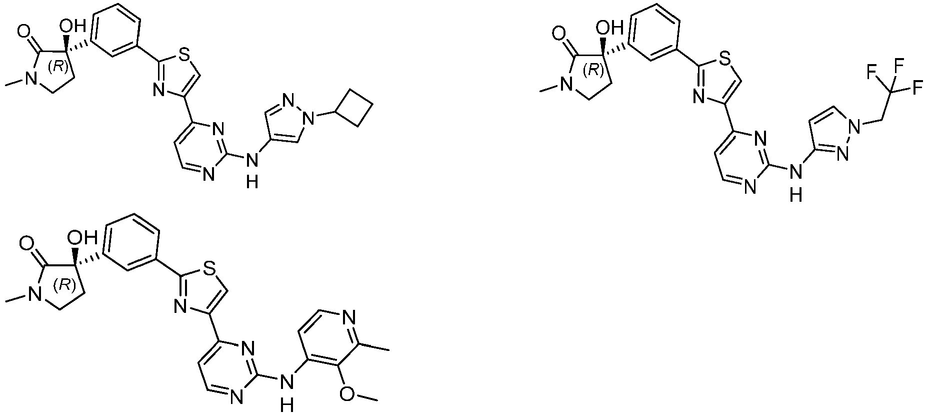

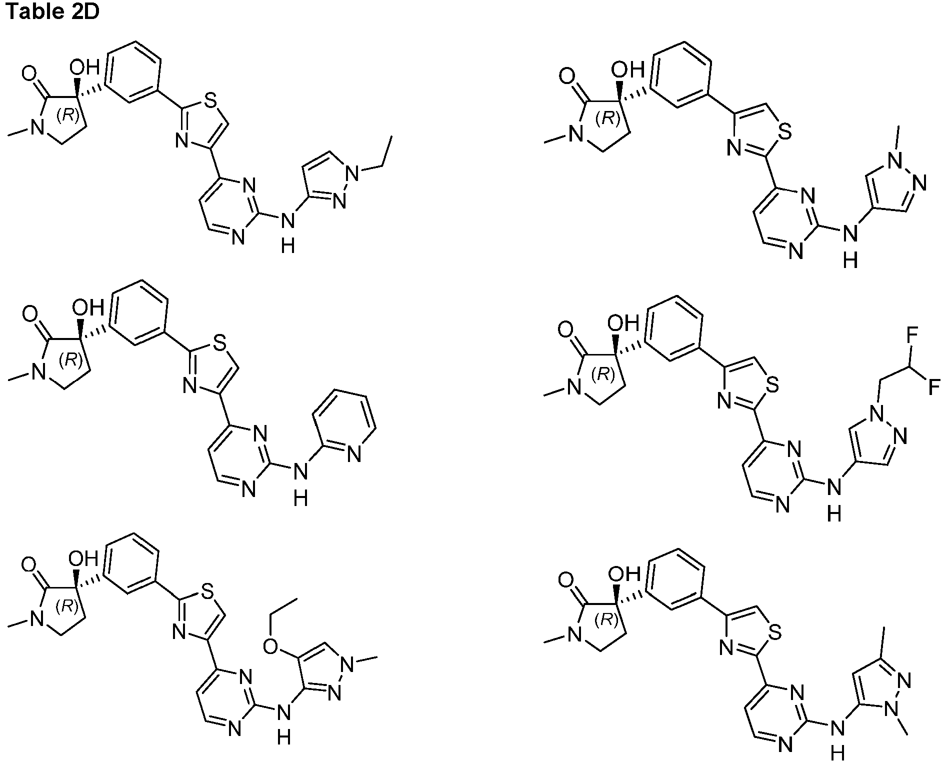

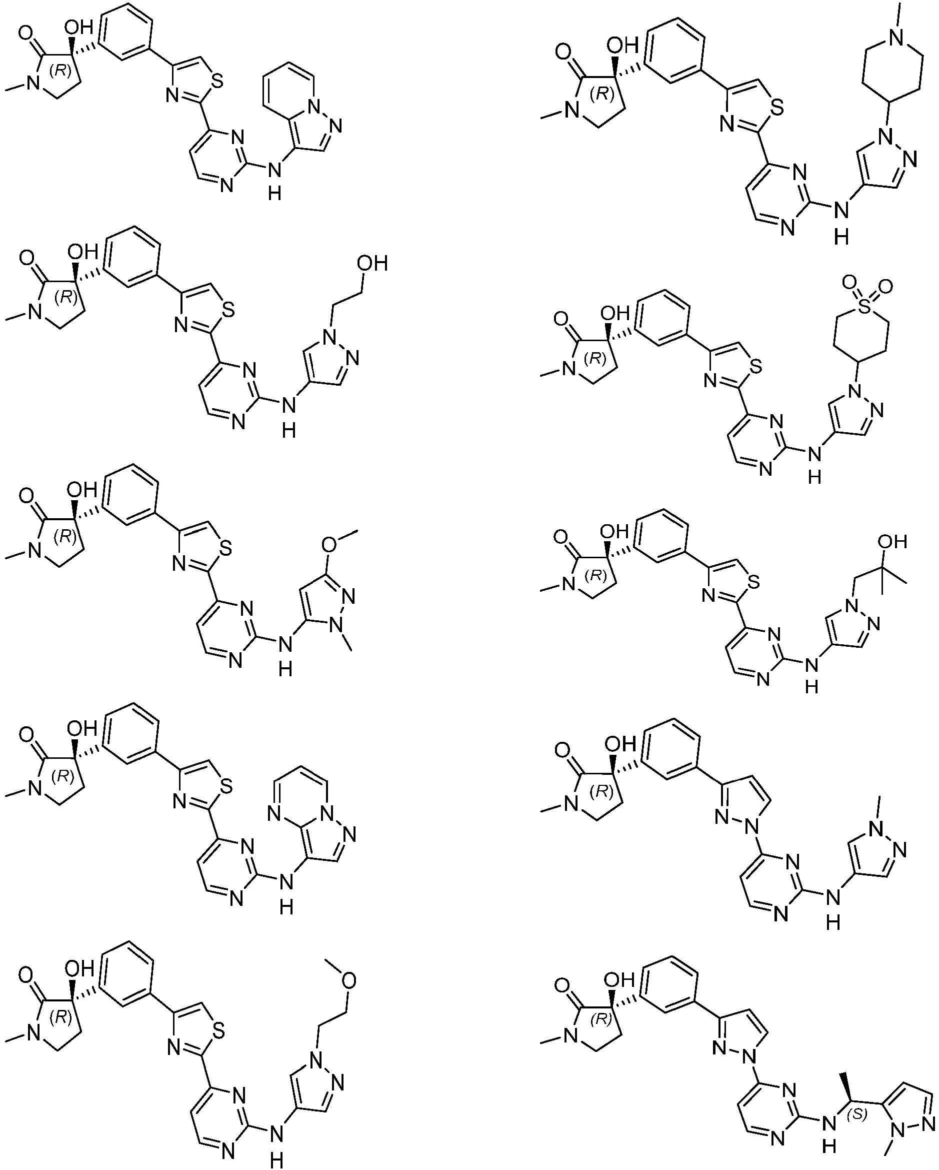

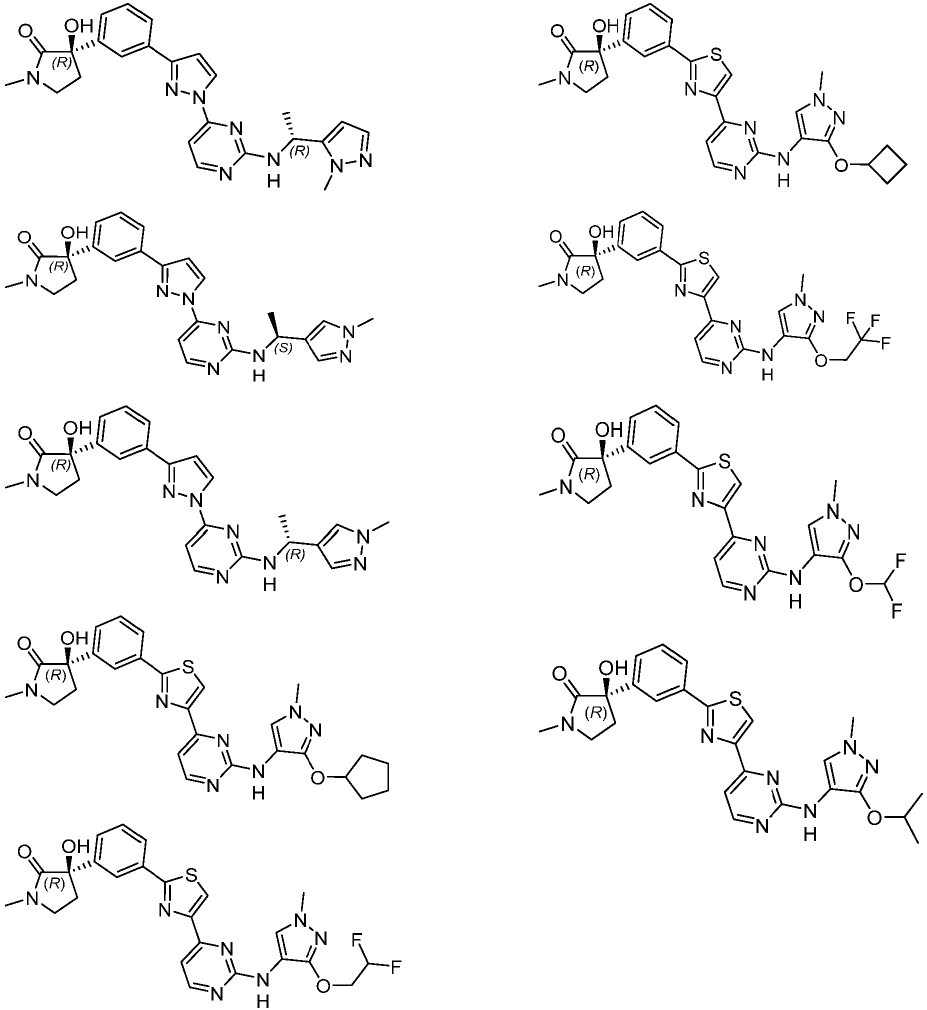

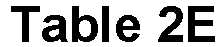

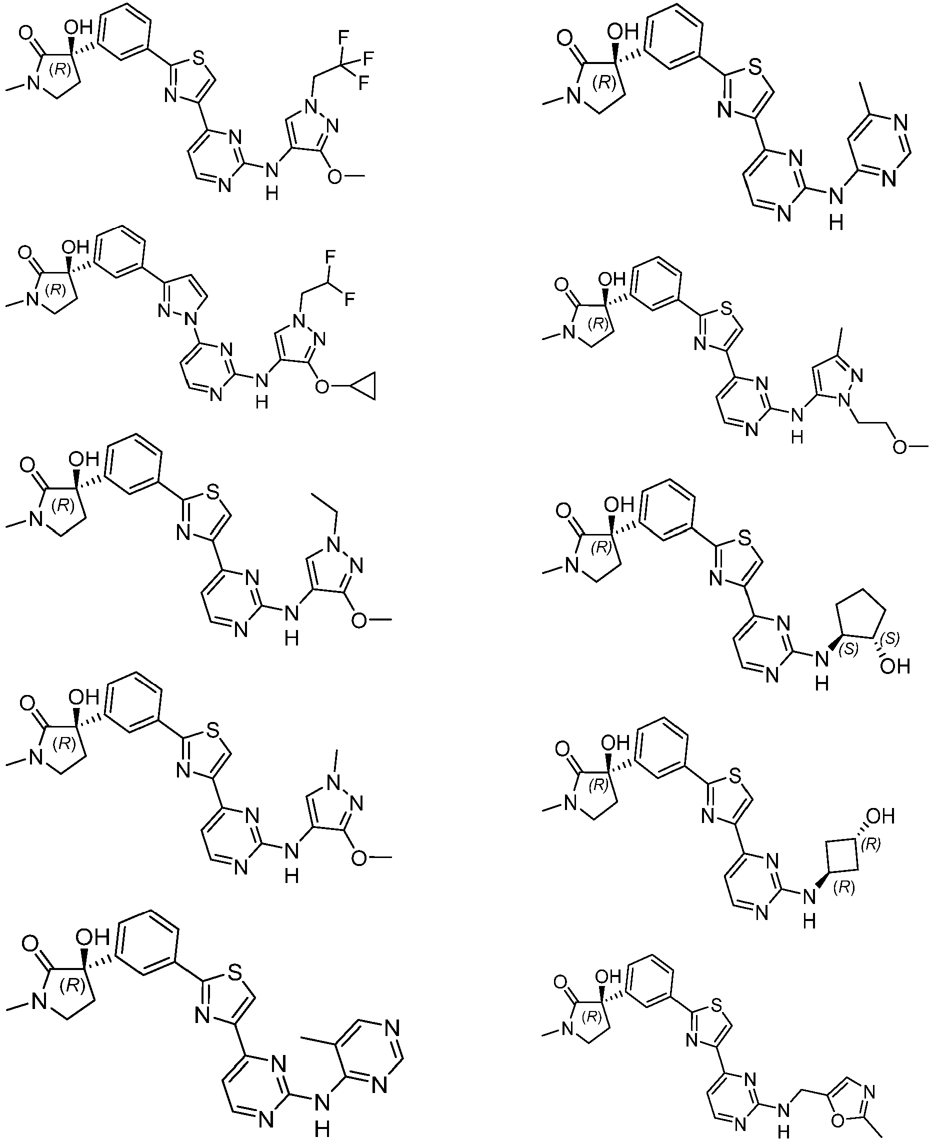

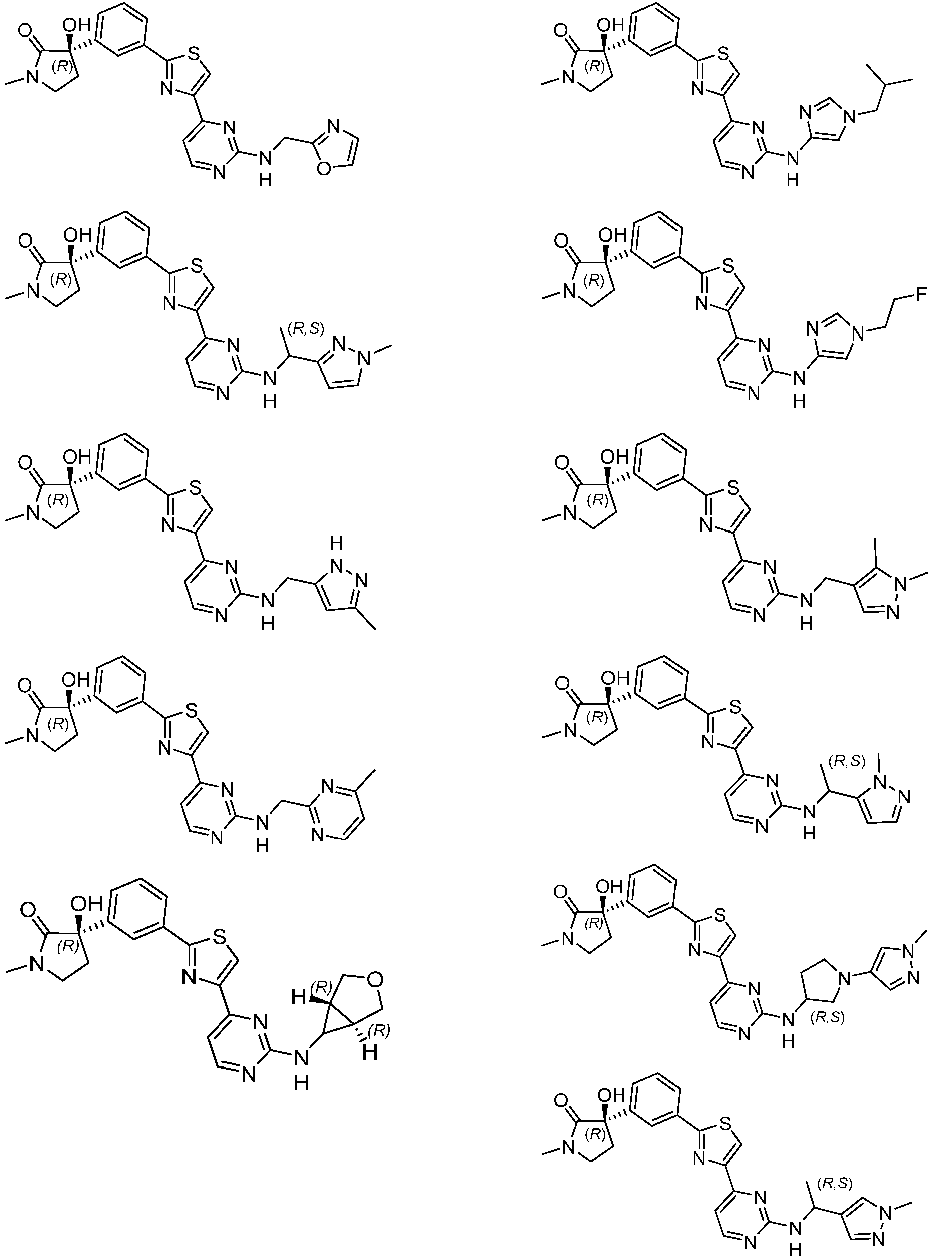

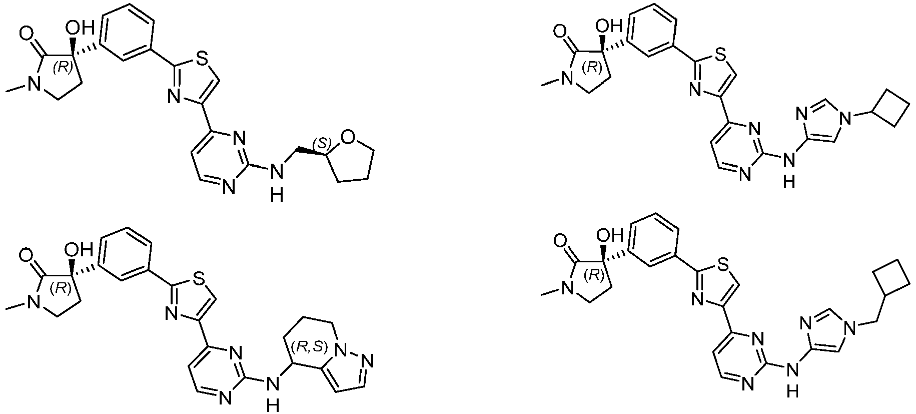

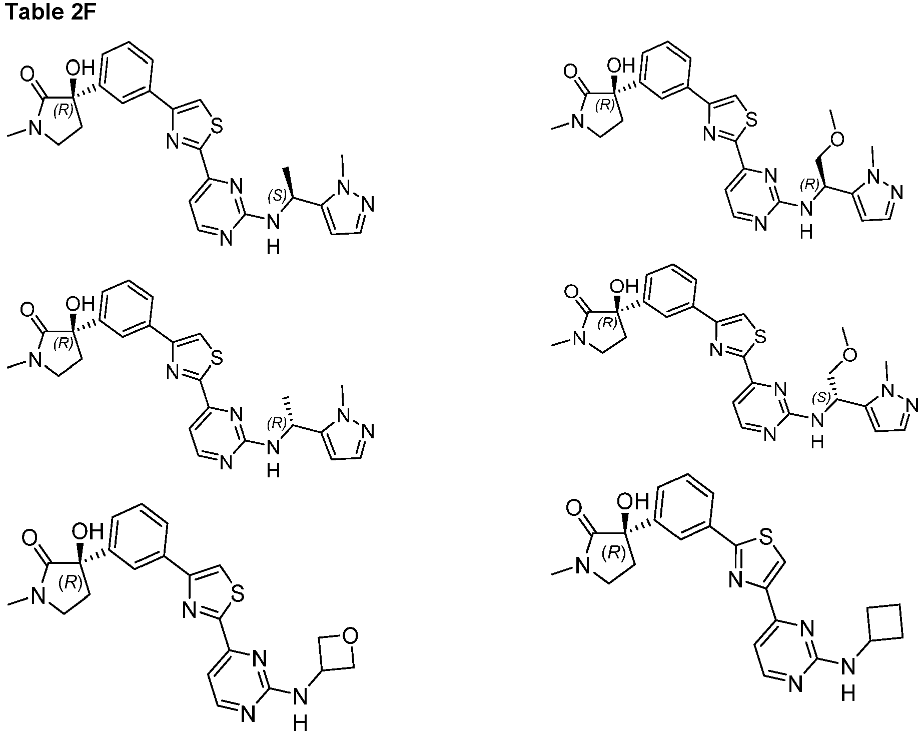

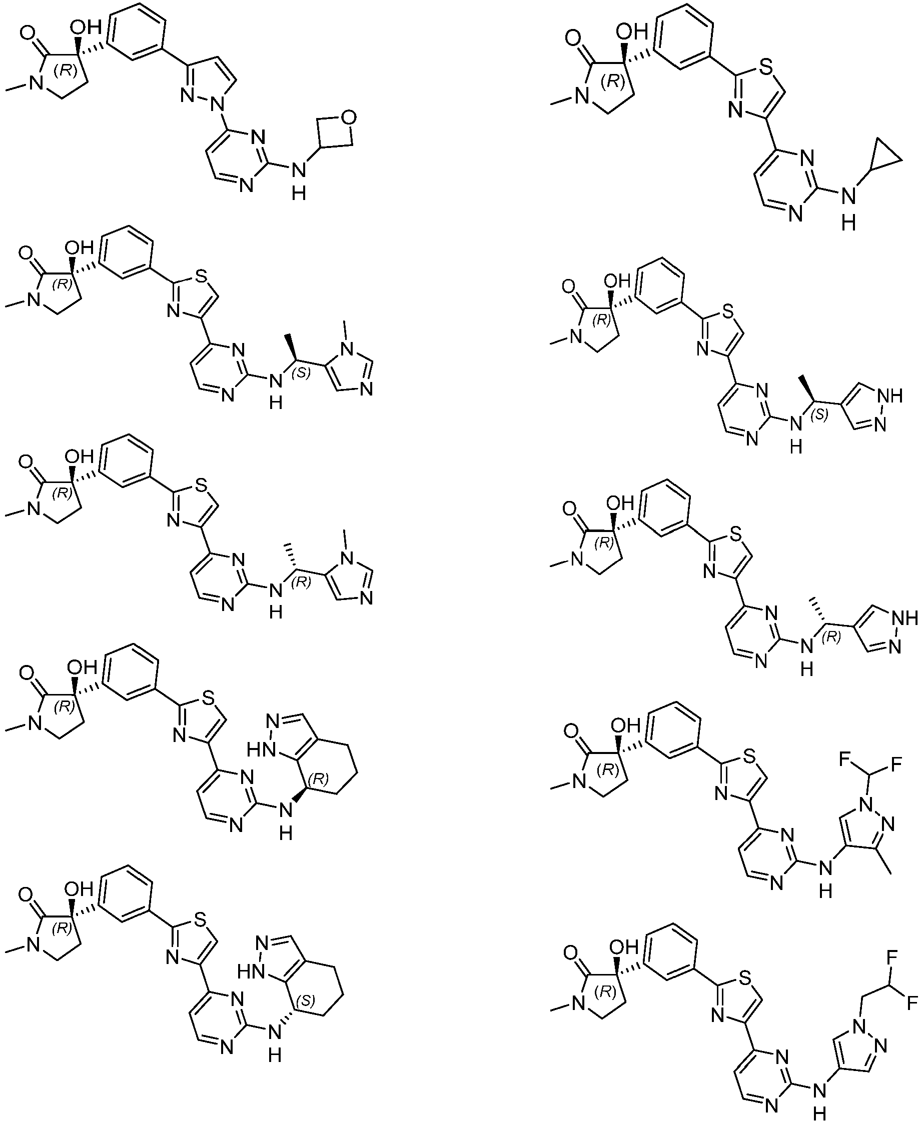

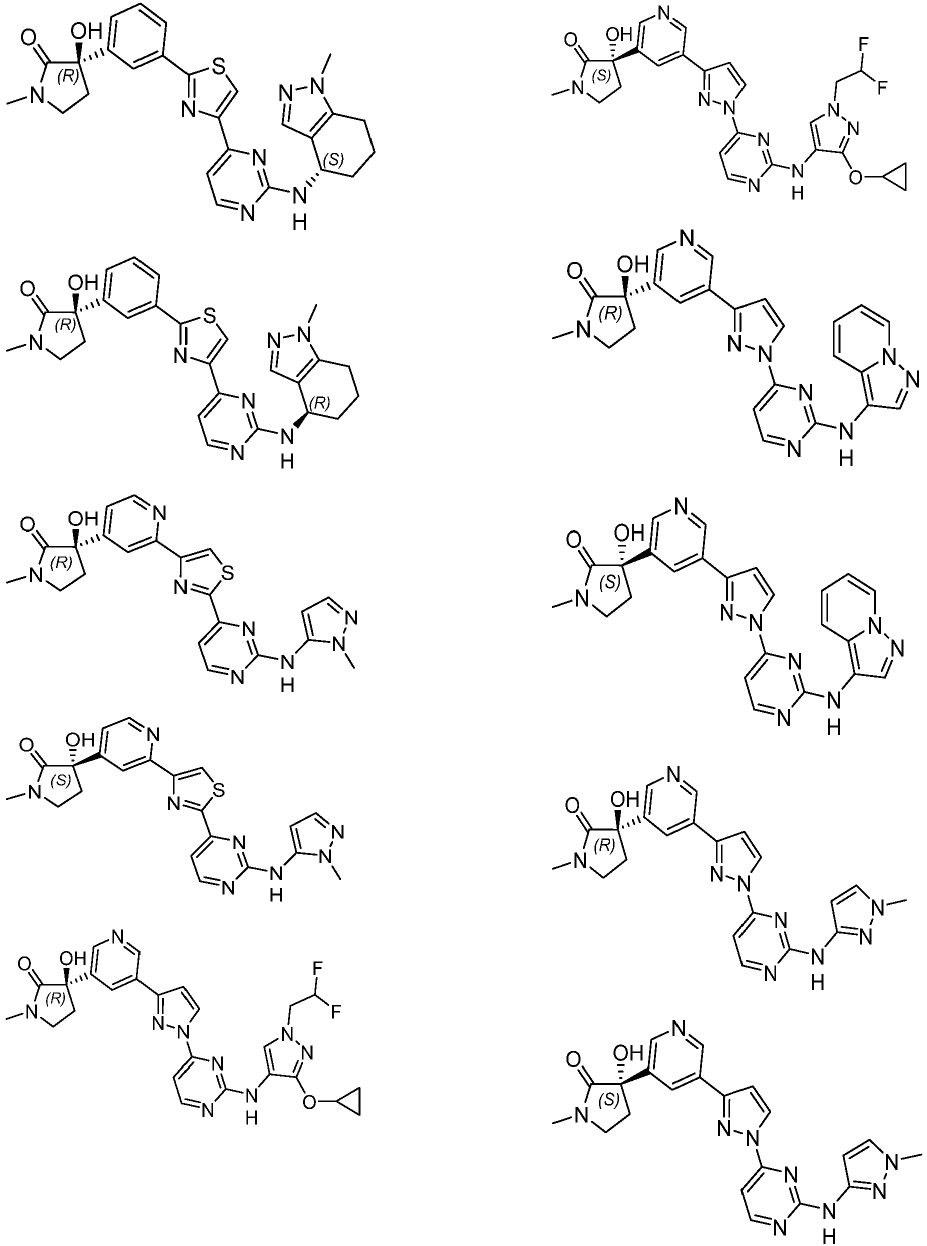

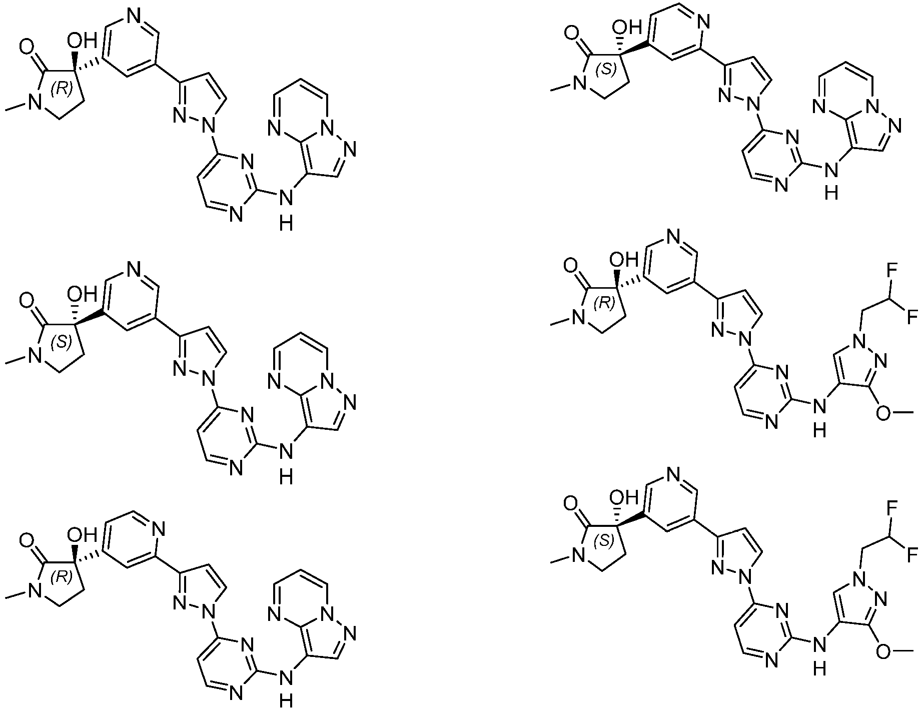

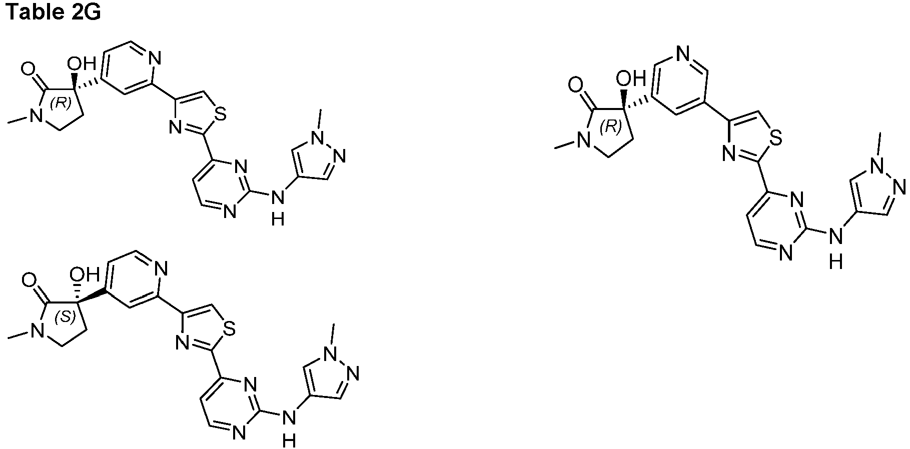

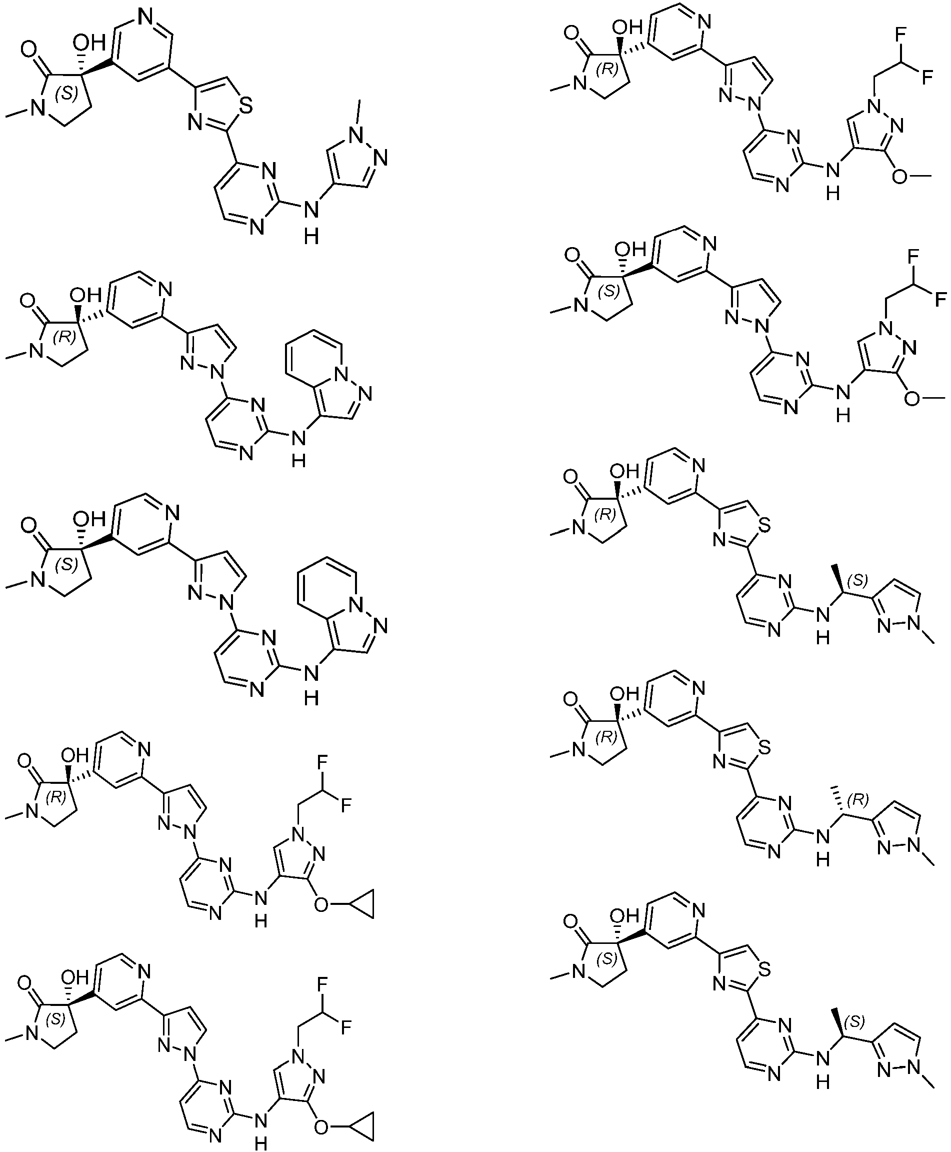

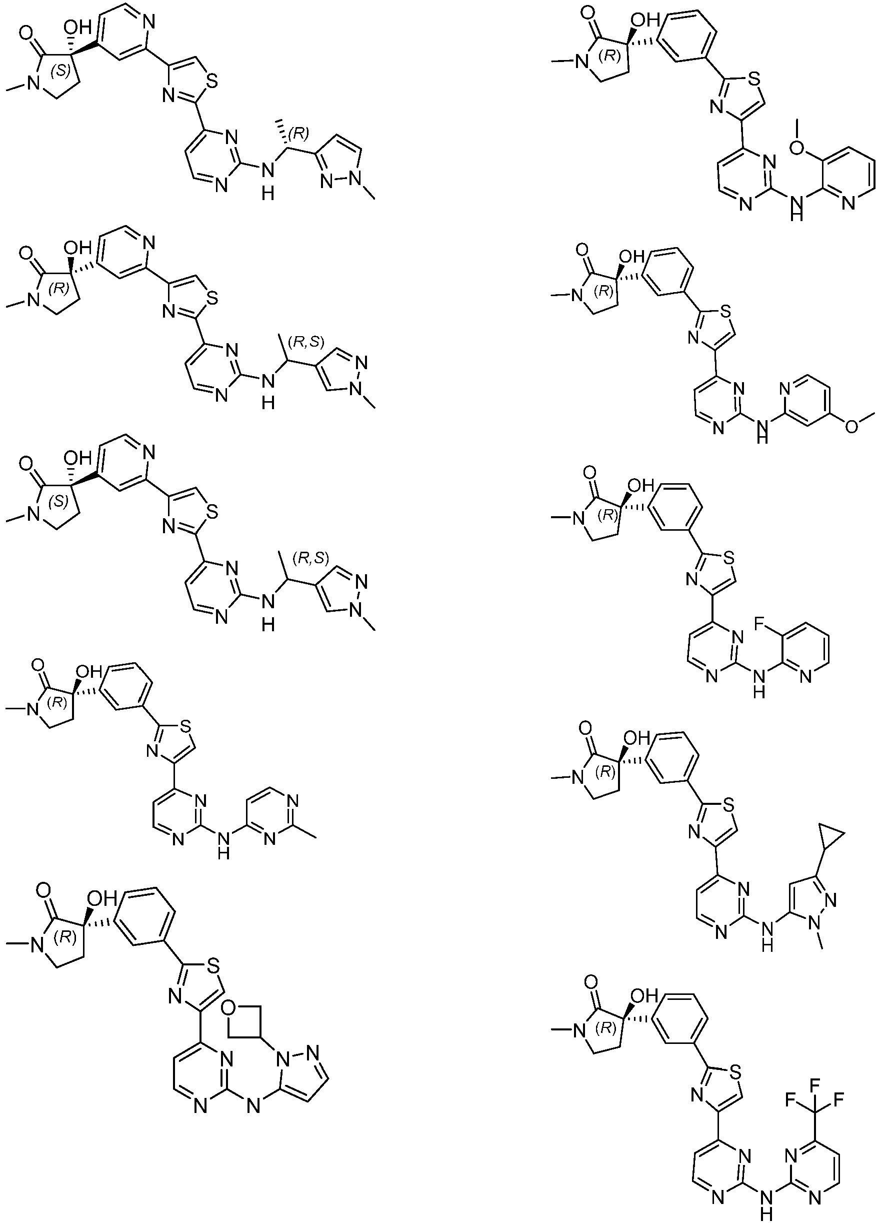

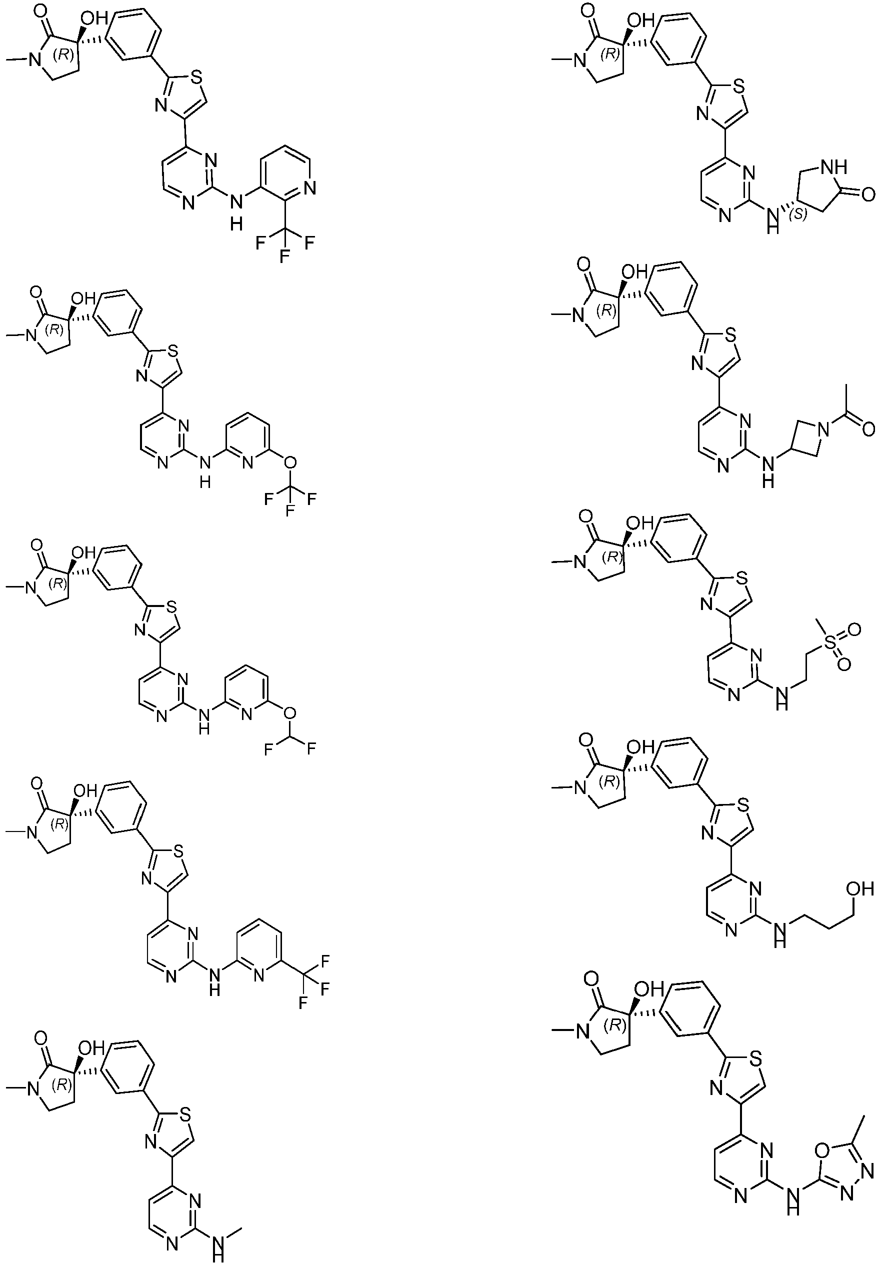

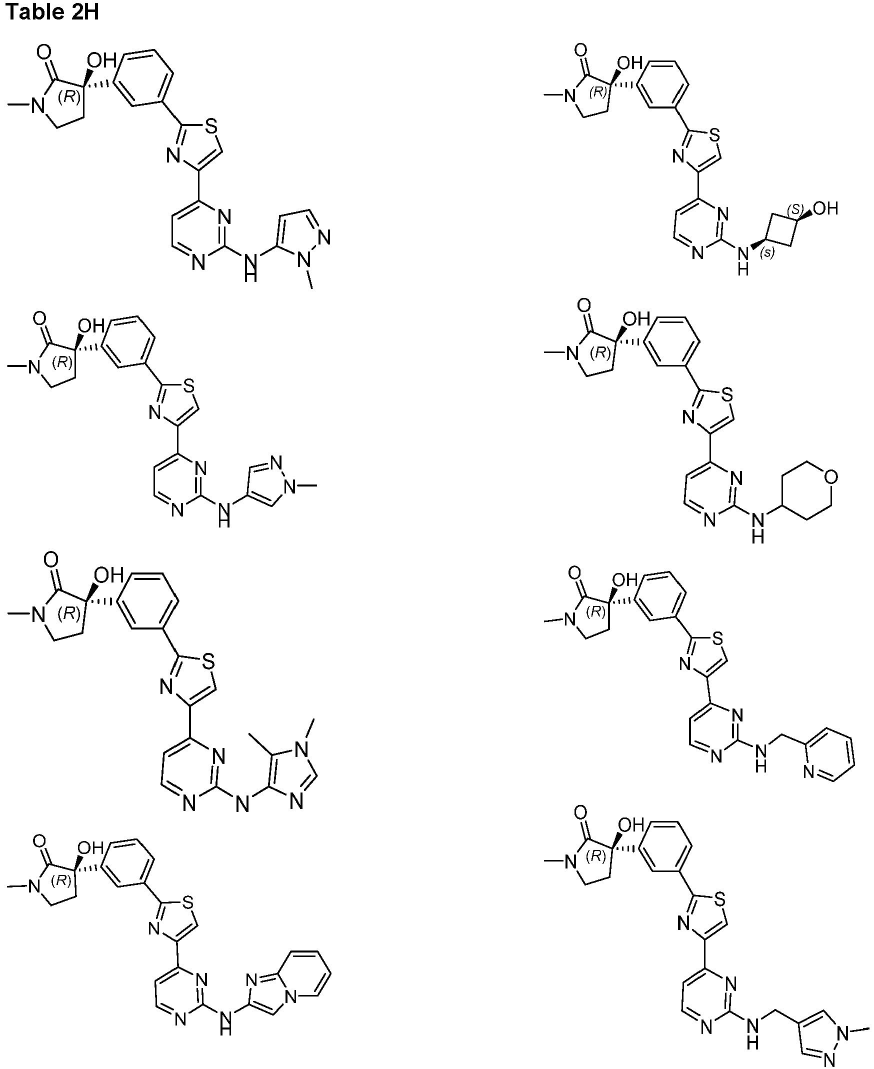

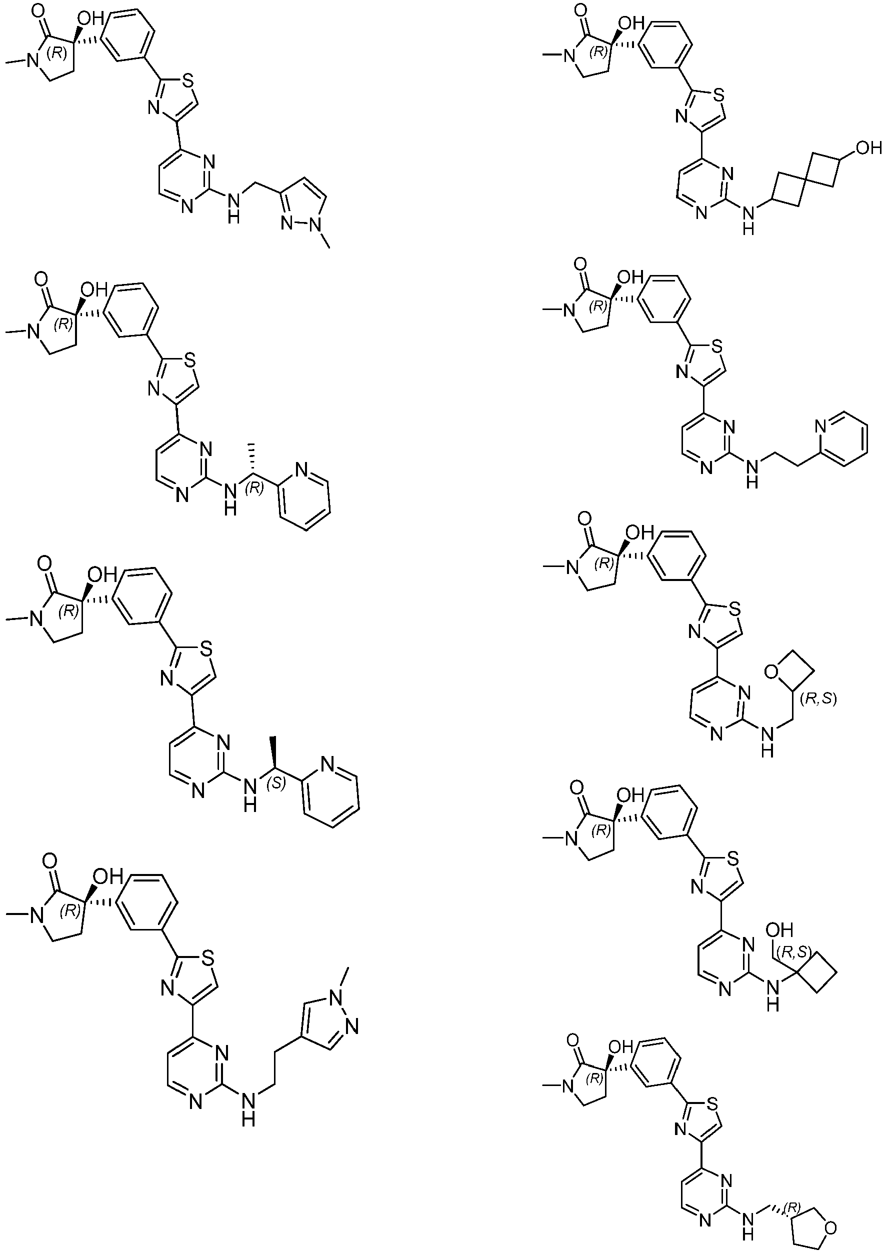

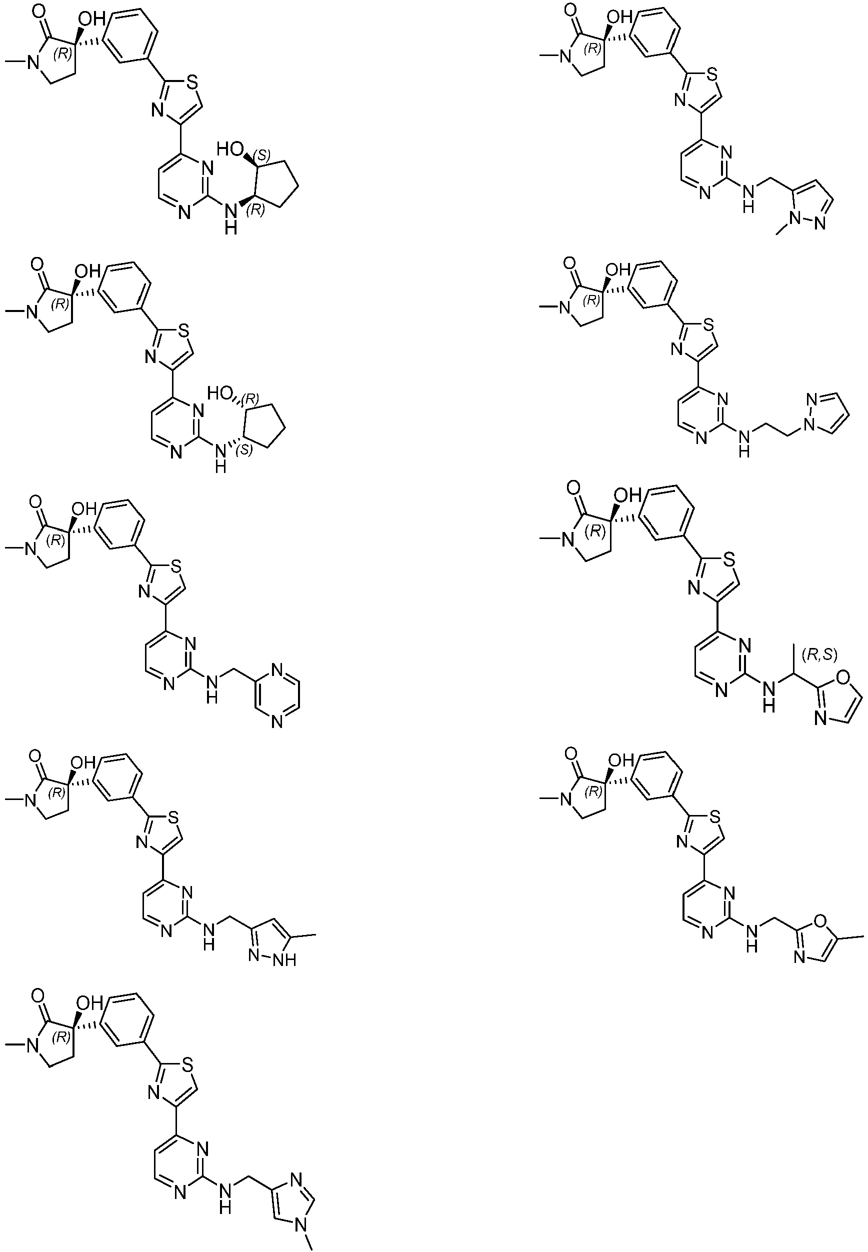

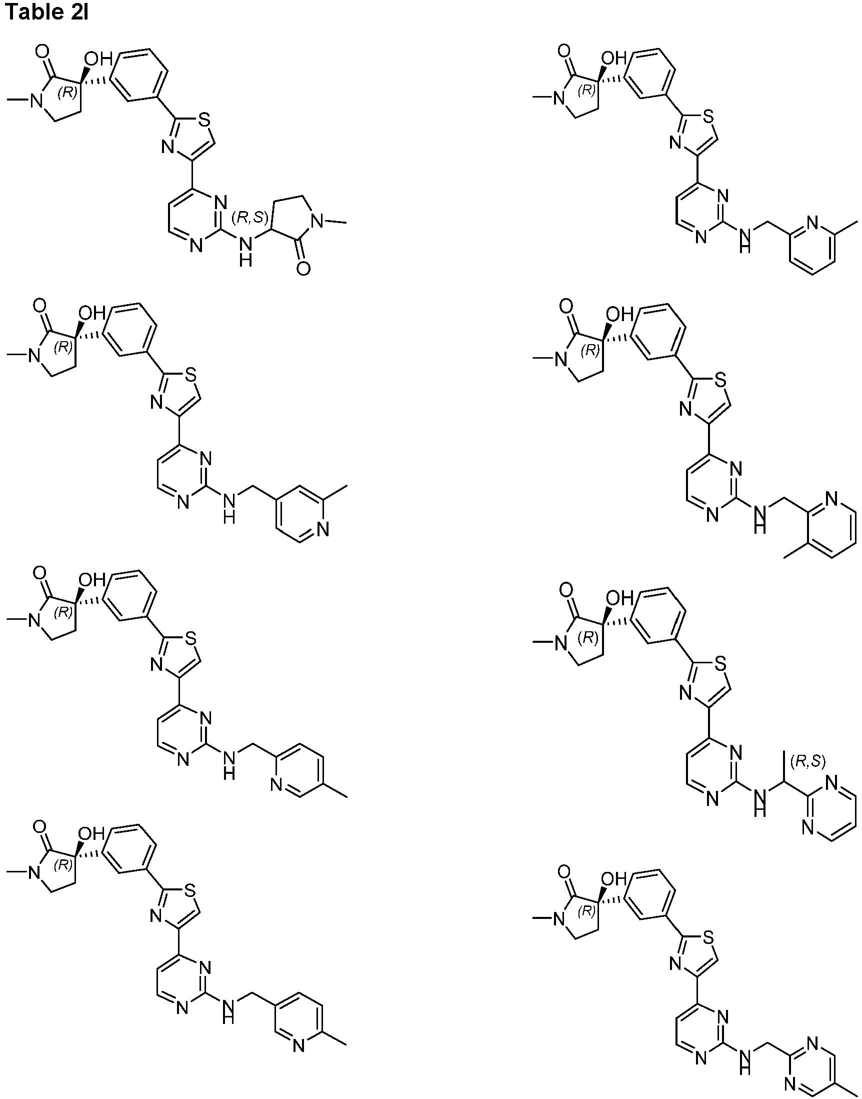

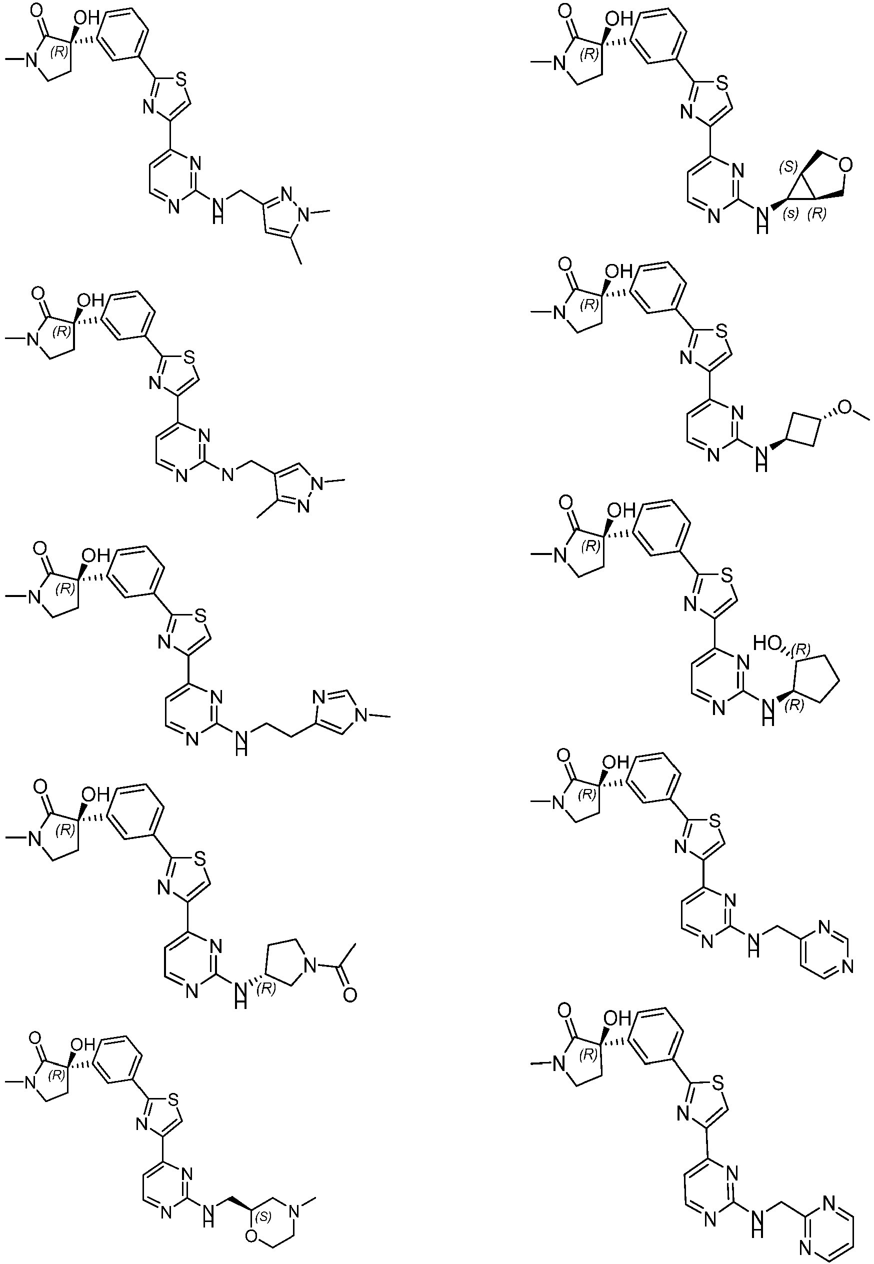

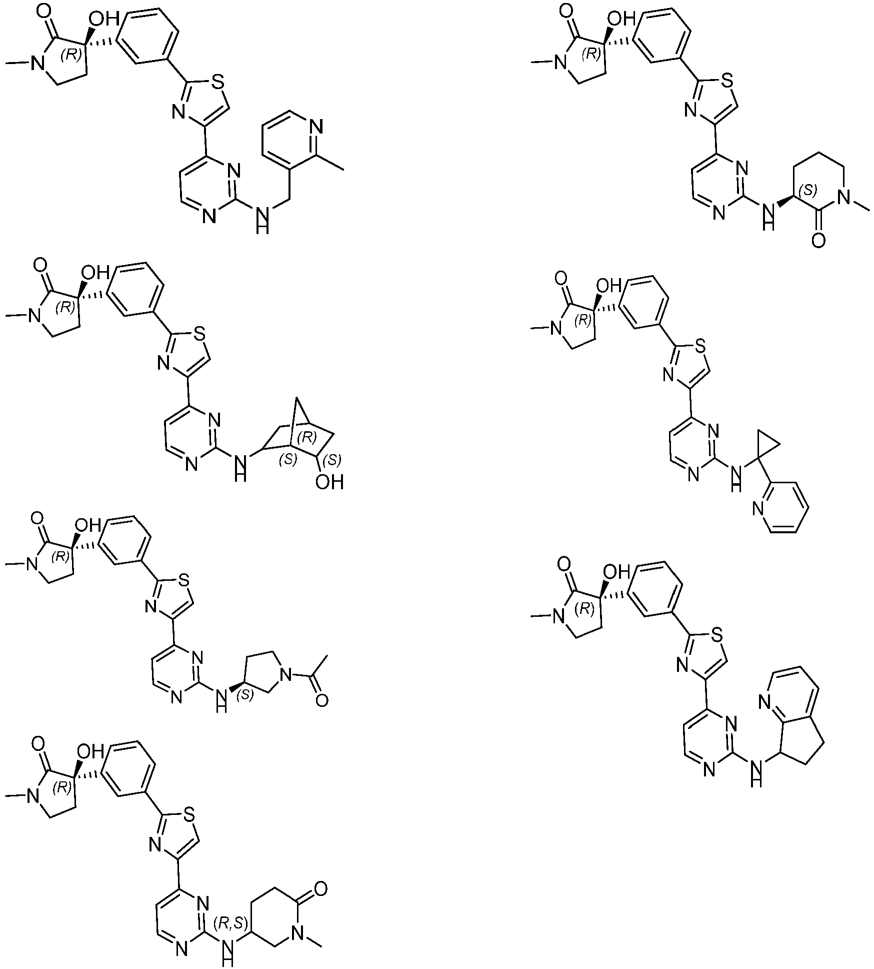

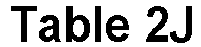

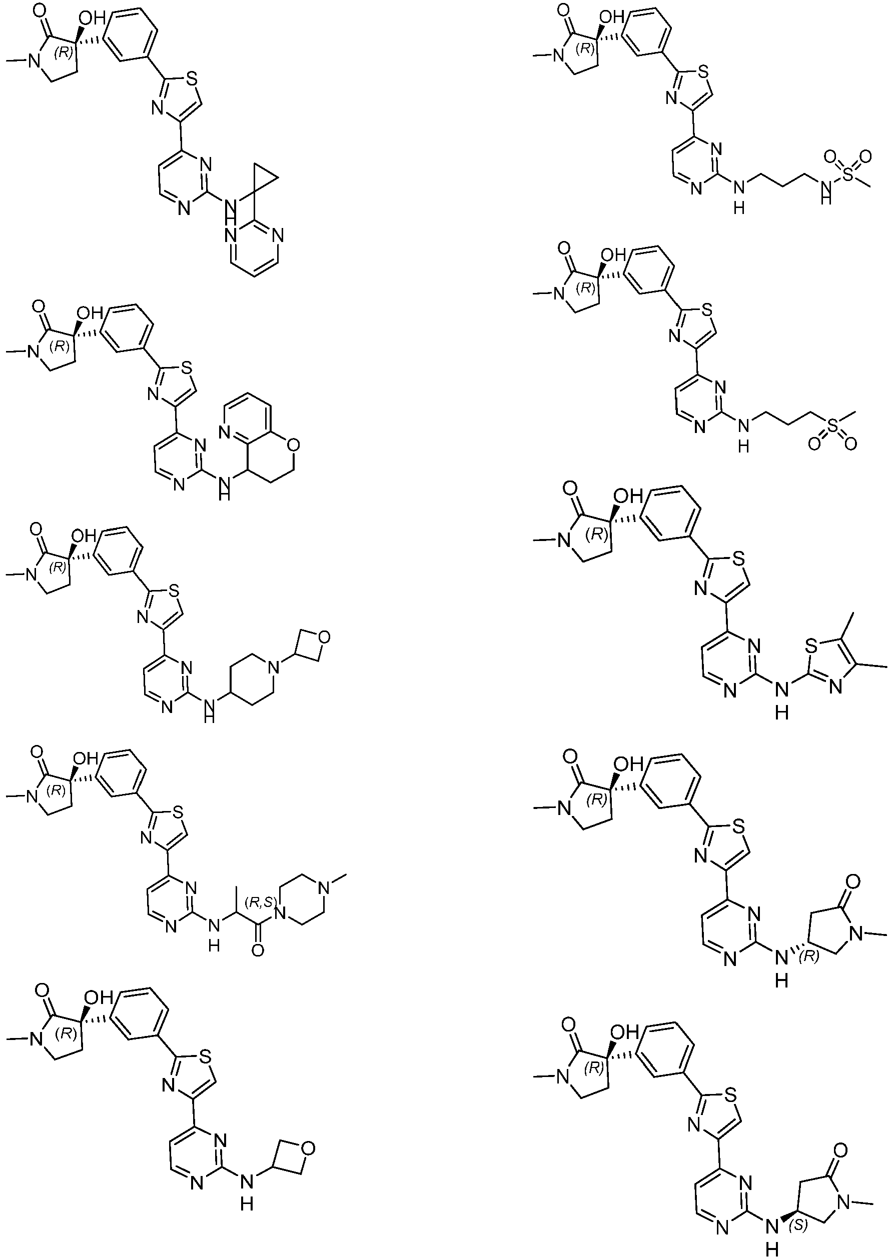

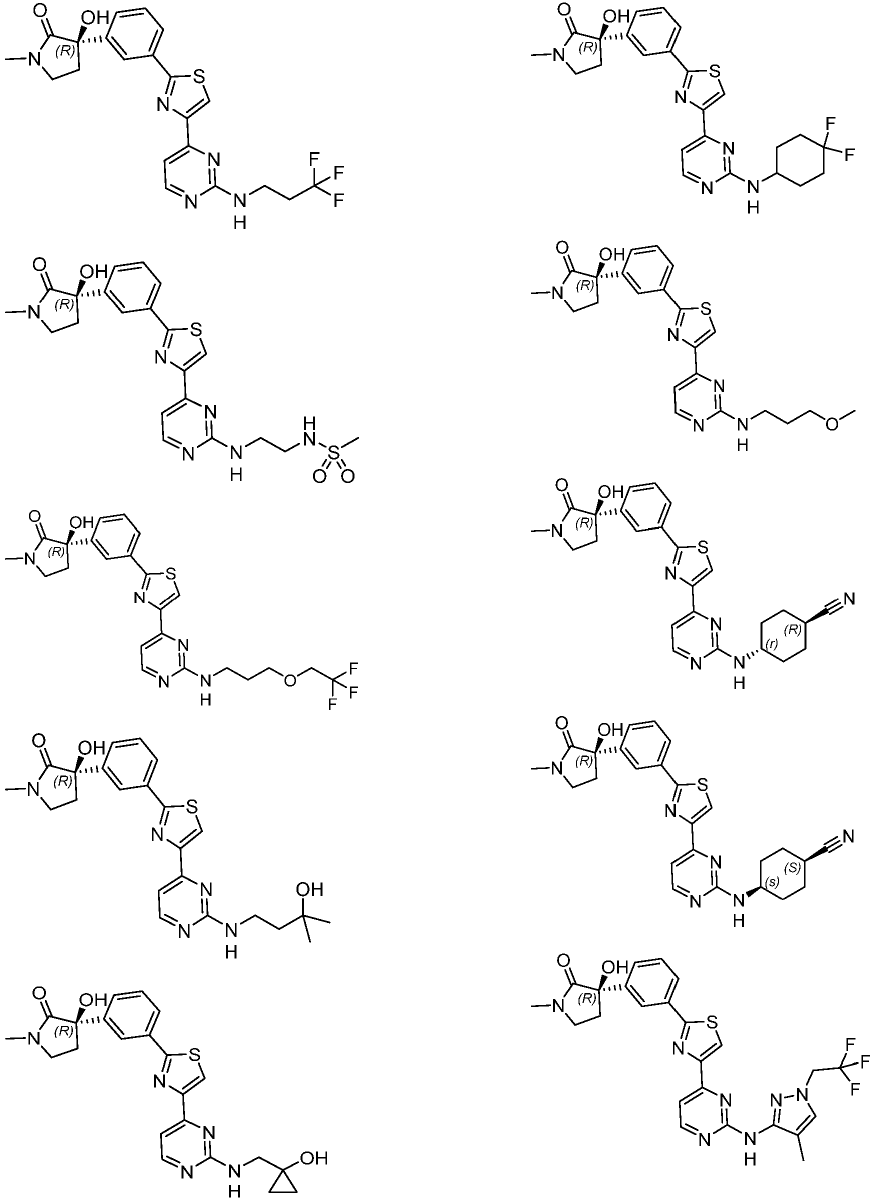

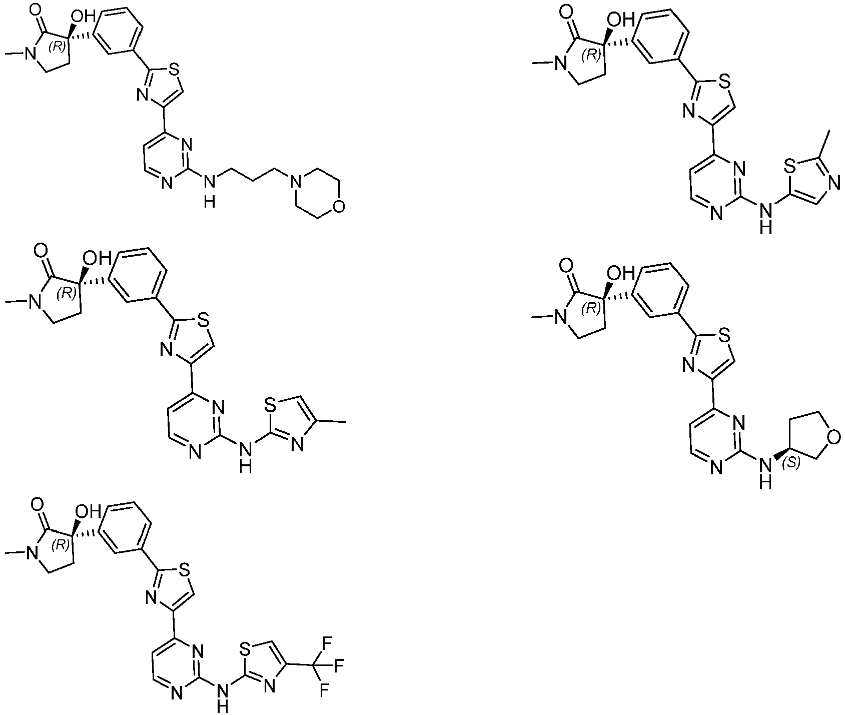

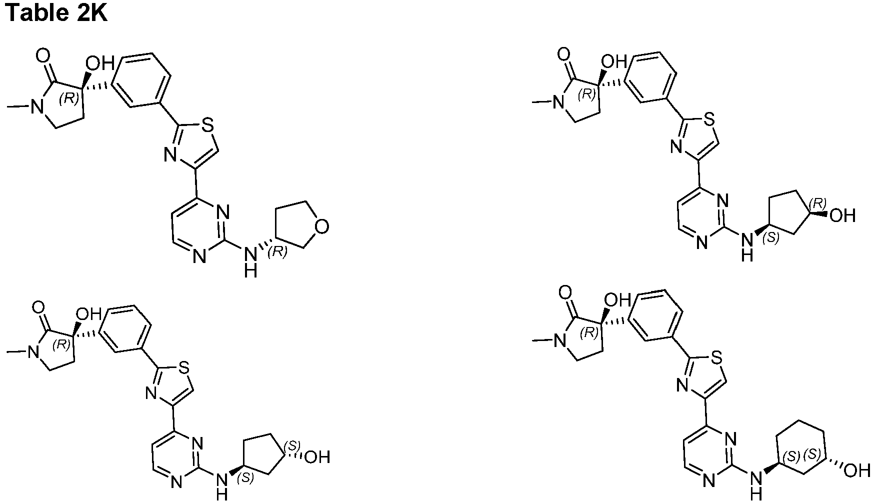

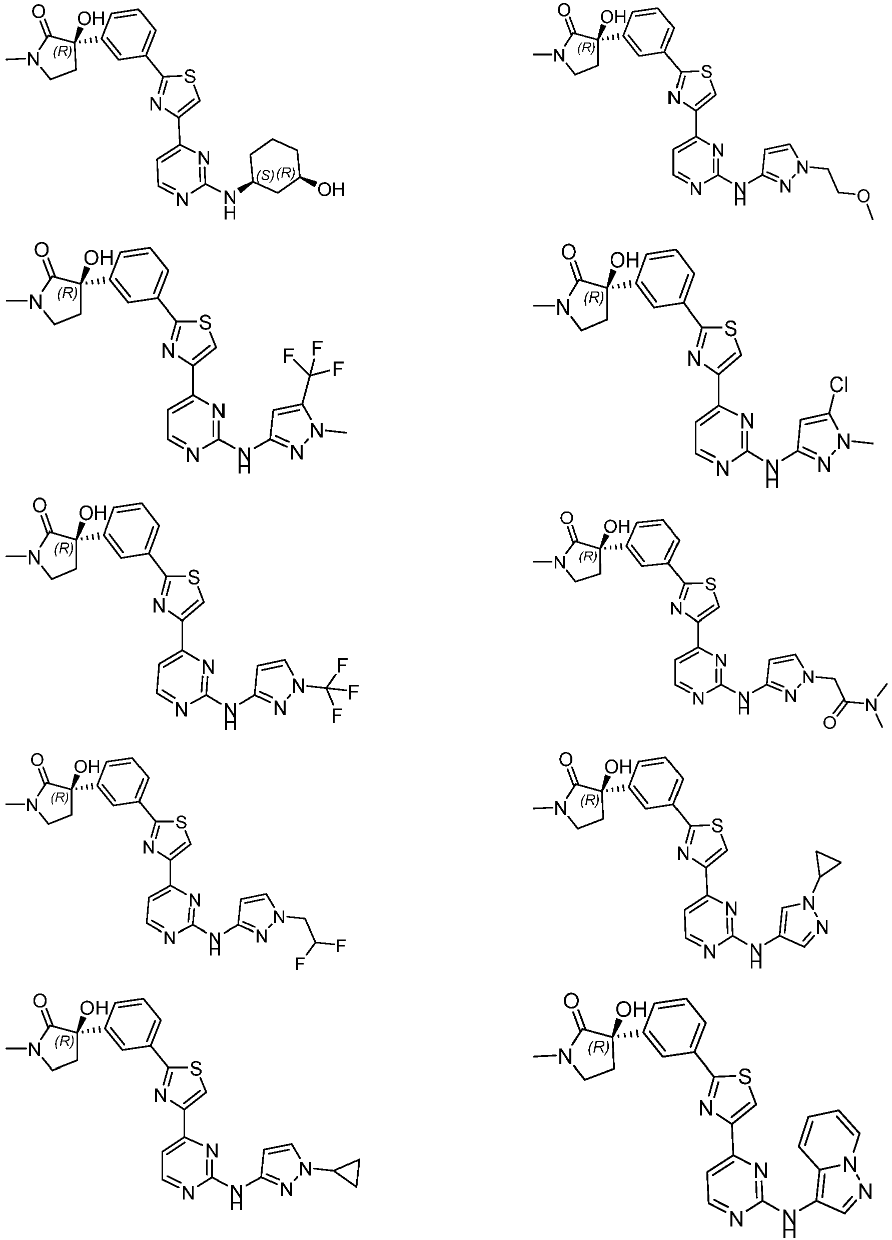

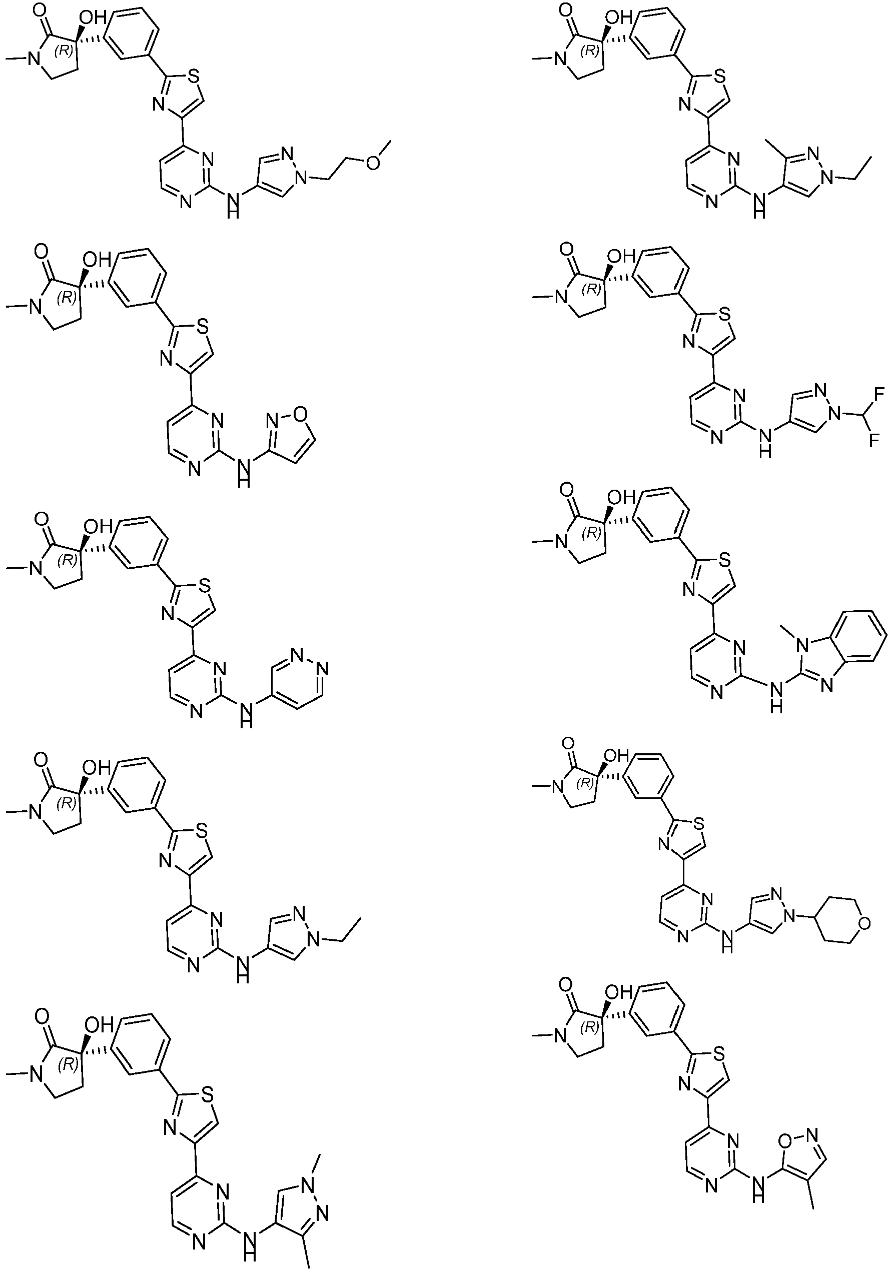

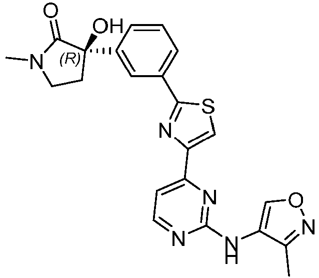

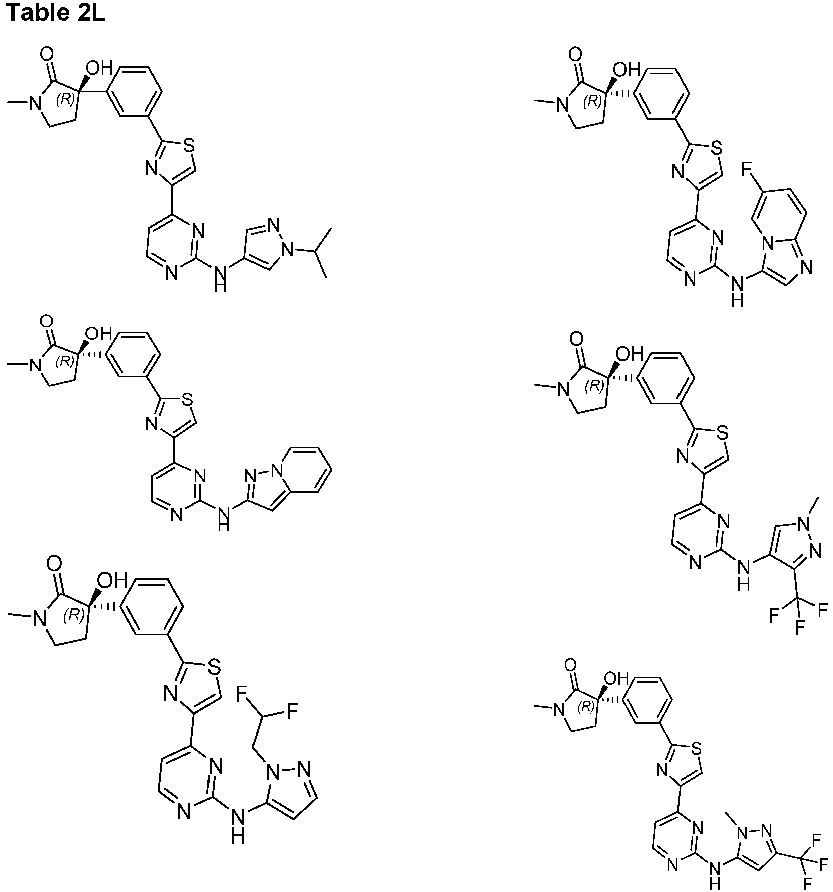

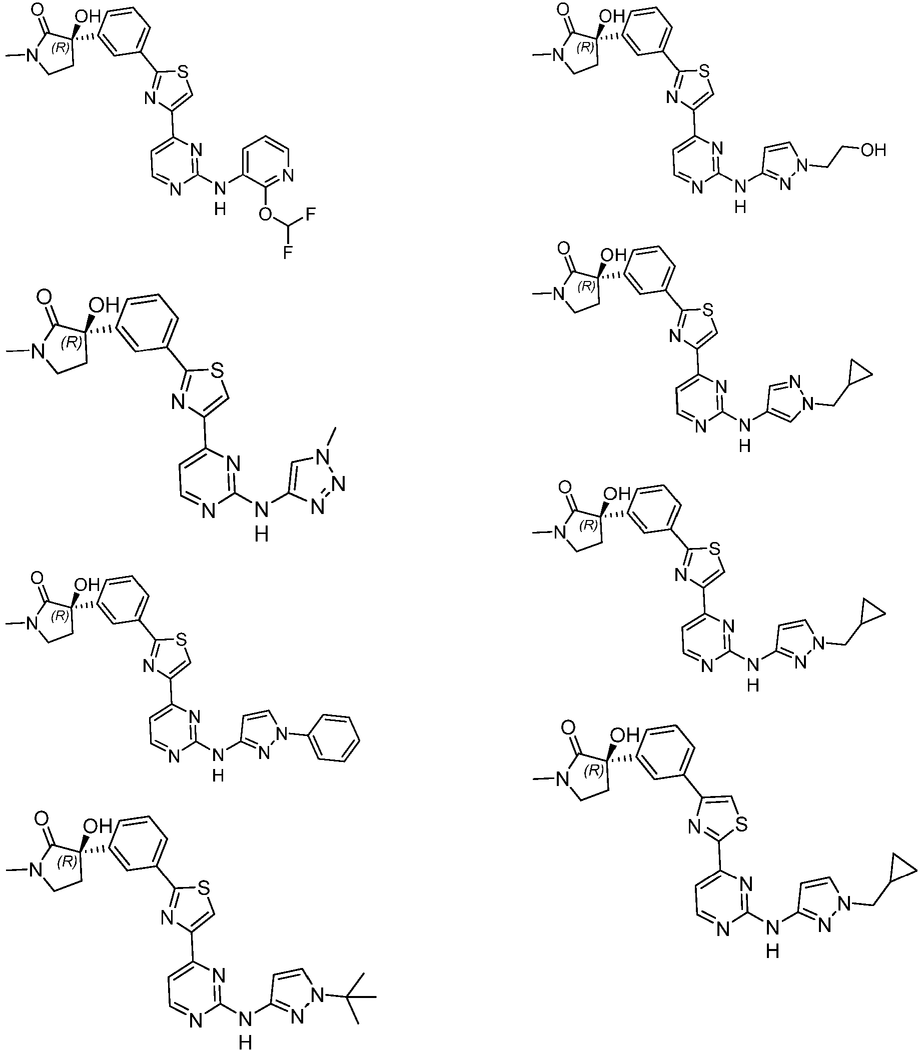

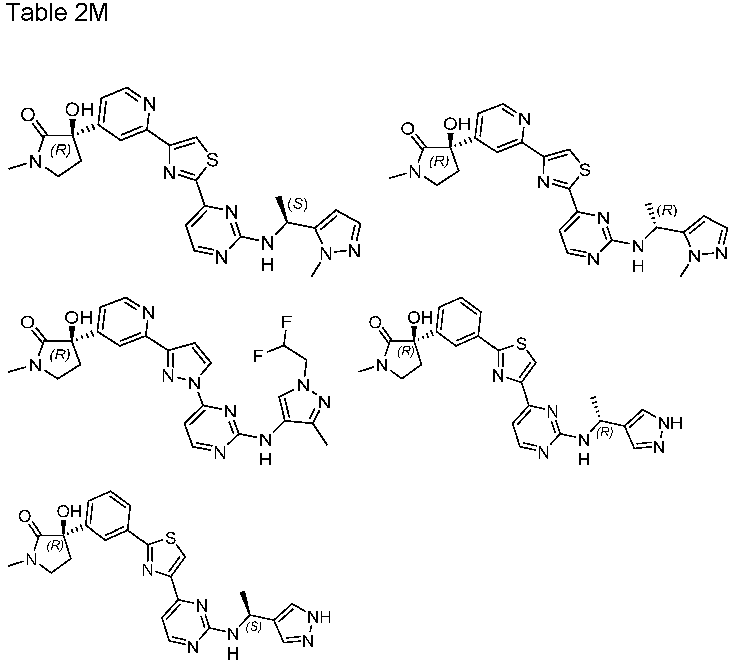

In some embodiments, disclosed herein is a compound of Formula I, or a pharmaceutically acceptable salt thereof, selected from the group consisting of: 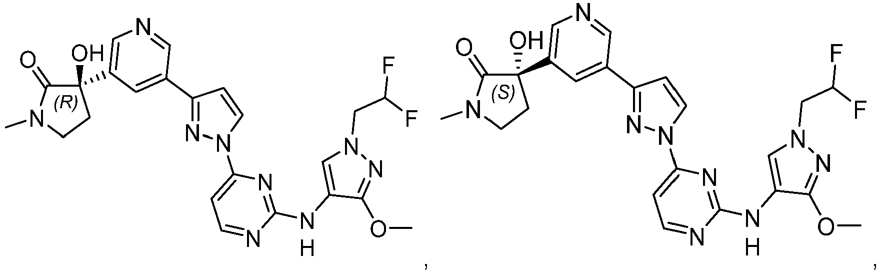 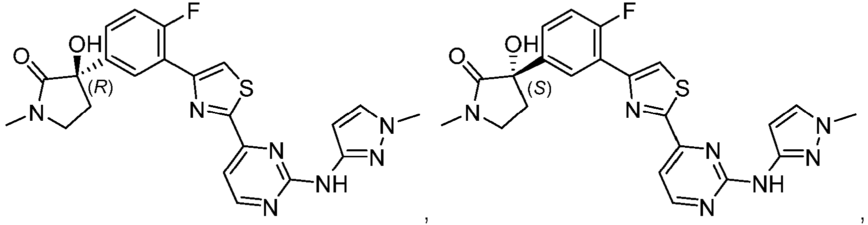 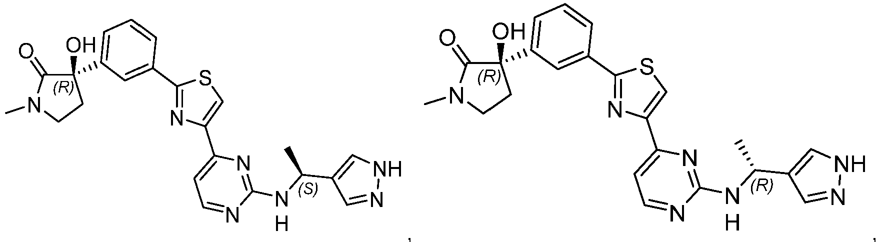 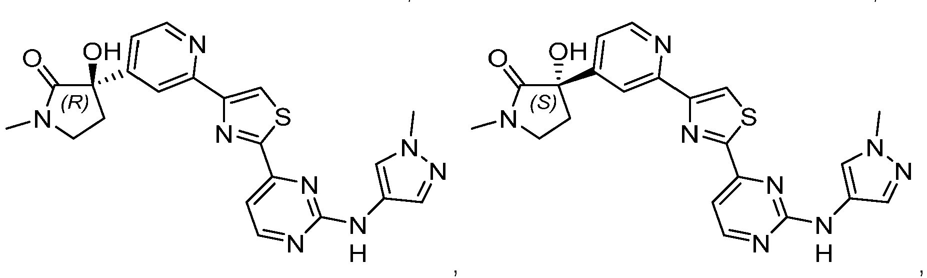 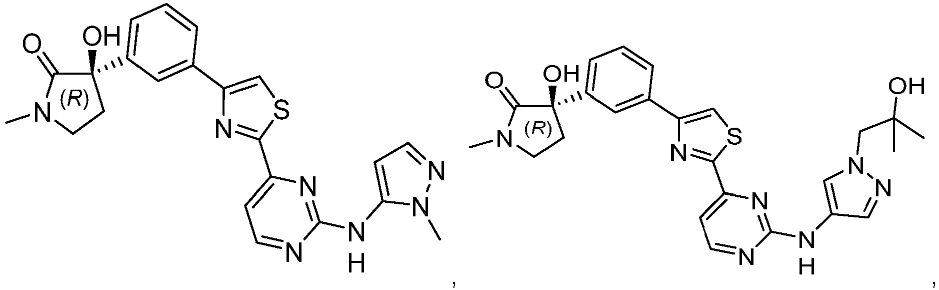 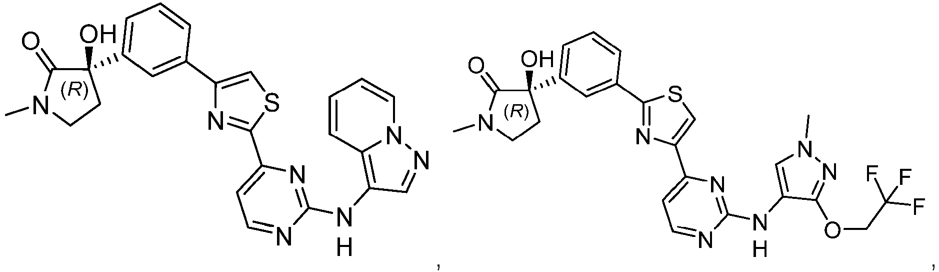 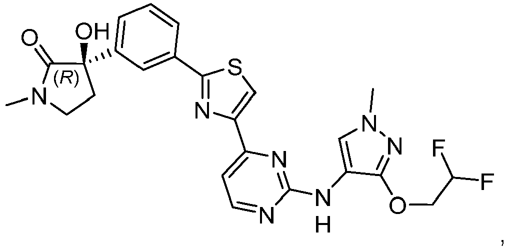 and 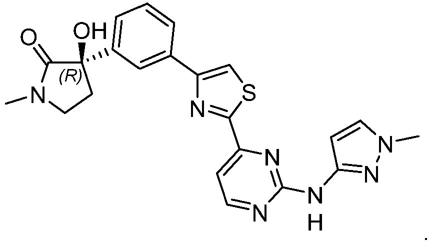 In some embodiments, disclosed herein is a compound of Formula I, or a pharmaceutically acceptable salt thereof, selected from the group consisting of: 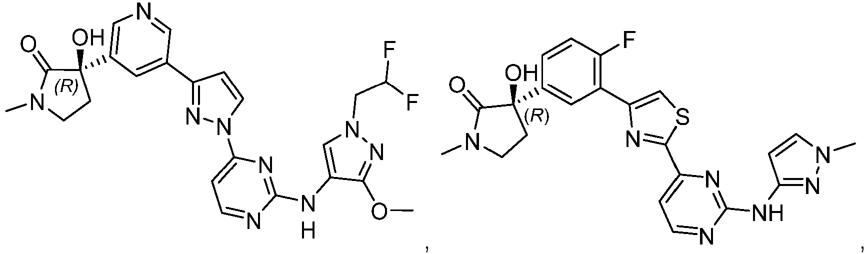 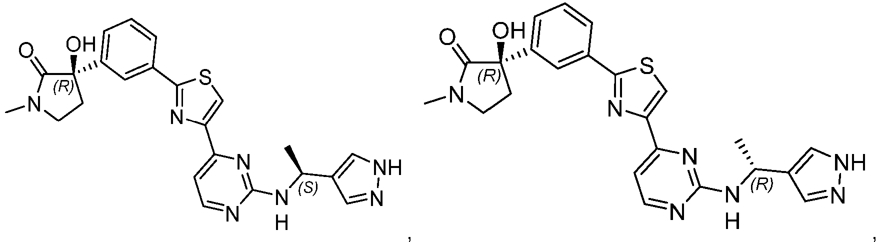 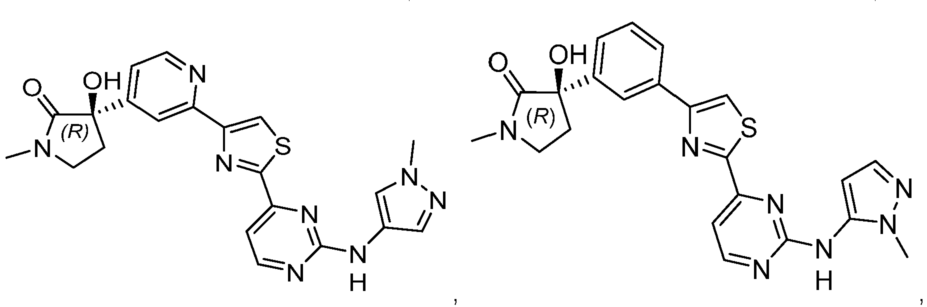 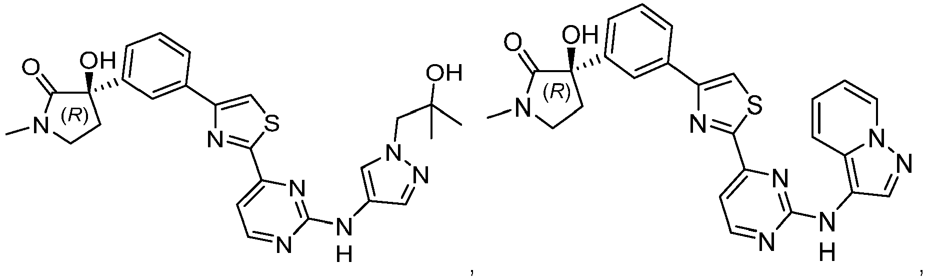 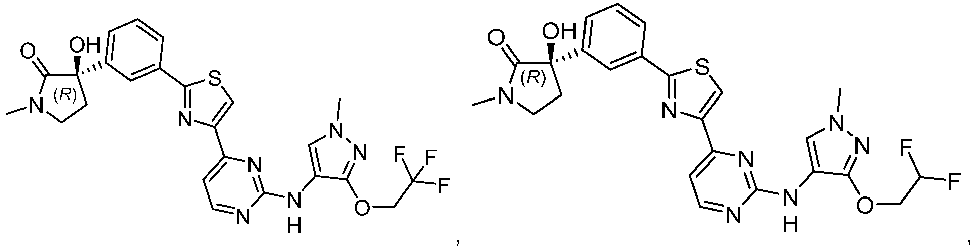 and

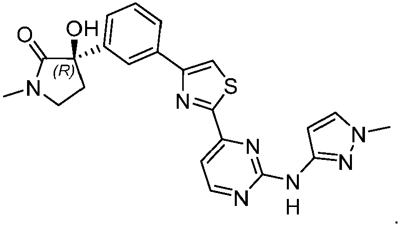

In some embodiments, disclosed herein is a compound of Formula I having the following formula: 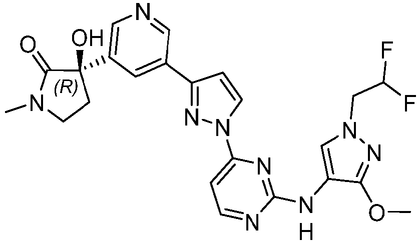 or a pharmaceutically acceptable salt thereof.

In some embodiments, disclosed herein is a compound of Formula I having the following formula: 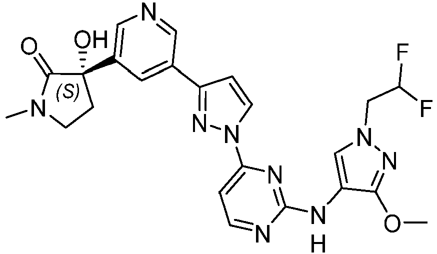 or a pharmaceutically acceptable salt thereof.

In some embodiments, disclosed herein is a compound of Formula I having the following formula: 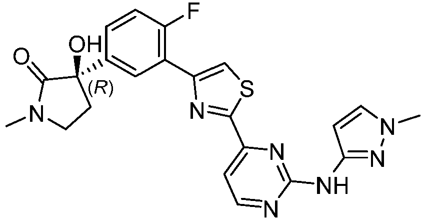 or a pharmaceutically acceptable salt thereof.

In some embodiments, disclosed herein is a compound of Formula I having the following formula: 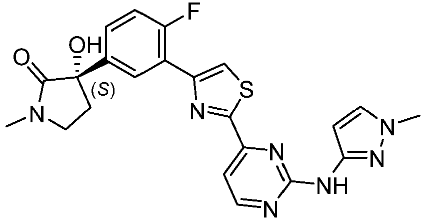 or a pharmaceutically acceptable salt thereof.

In some embodiments, disclosed herein is a compound of Formula I having the following formula: 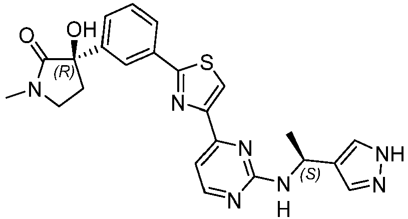 or a pharmaceutically acceptable salt thereof.

In some embodiments, disclosed herein is a compound of Formula I having the following formula: 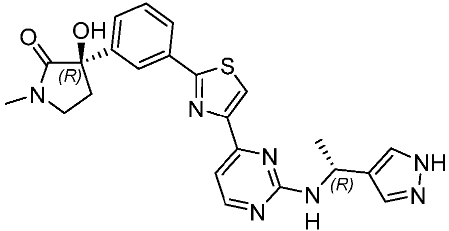 or a pharmaceutically acceptable salt thereof.

In some embodiments, disclosed herein is a compound of Formula I having the following formula: 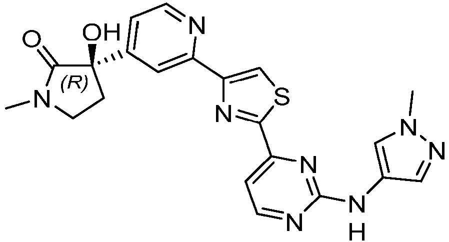 or a pharmaceutically acceptable salt thereof.

In some embodiments, disclosed herein is a compound of Formula I having the following formula: 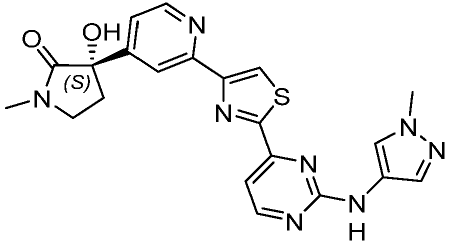 or a pharmaceutically acceptable salt thereof.

In some embodiments, disclosed herein is a compound of Formula I having the following formula: 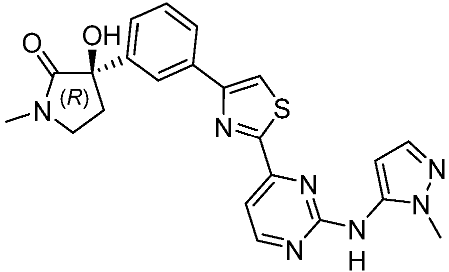 or a pharmaceutically acceptable salt thereof.

In some embodiments, disclosed herein is a compound of Formula I having the following formula: 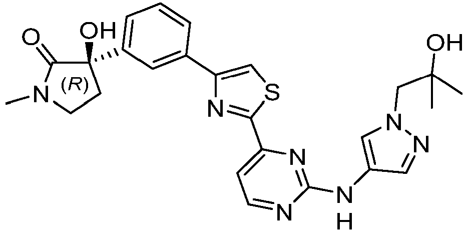 or a pharmaceutically acceptable salt thereof.

In some embodiments, disclosed herein is a compound of Formula I having the following formula: 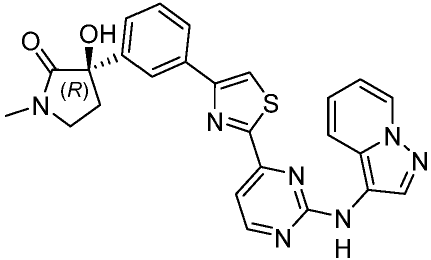 or a pharmaceutically acceptable salt thereof.

In some embodiments, disclosed herein is a compound of Formula I having the following formula: 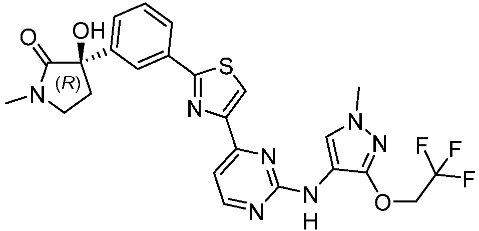 or a pharmaceutically acceptable salt thereof.

In some embodiments, disclosed herein is a compound of Formula I having the following formula: 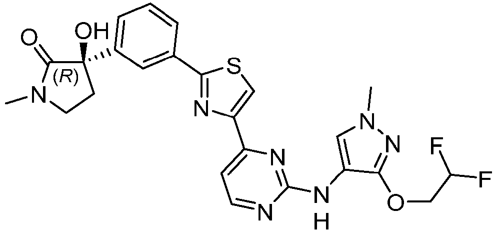 or a pharmaceutically acceptable salt thereof.

In some embodiments, disclosed herein is a compound of Formula I having the following formula: 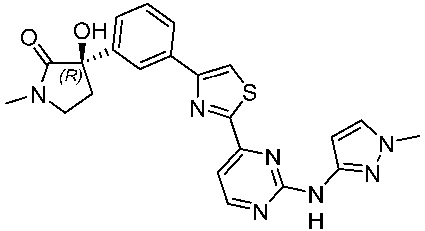 or a pharmaceutically acceptable salt thereof.

In some embodiments, disclosed herein is a pharmaceutical composition comprising a compound of Formula I, or a pharmaceutically acceptable salt thereof, and a pharmaceutically acceptable carrier.

### Therapeutic Use

The present disclosure is also directed toward a method for treating a disease, disorder, or medical condition mediated by NIK activity, comprising administering to a subject in need of such treatment an effective amount of a compound of the disclosure, or a pharmaceutically acceptable salt thereof, or a pharmaceutical composition of the disclosure.

The present disclosure is also directed toward a method for preventing a disease, disorder, or medical condition mediated by NIK activity, comprising administering to a subject in need of such treatment an effective amount of a compound of the disclosure, or a pharmaceutically acceptable salt thereof, or a pharmaceutical composition of the disclosure. In some embodiments, the compound disclosed herein can be used in combination with another therapeutic agent.

The present disclosure is also directed toward a method for improving or ameliorating a symptom of a disease, disorder, or medical condition mediated by NIK activity, comprising administering to a subject in need of such treatment an effective amount of a compound of the disclosure, or a pharmaceutically acceptable salt thereof, or a pharmaceutically acceptable salt thereof.

In some embodiments of the methods of treatment disclosed herein, the disease, disorder, or medical condition is selected from the group consisting of inflammatory disorders and autoimmune disorders.

In some embodiments, the disease, disorder, or medical condition is selected from the group consisting of systemic lupus erythematosus, rheumatoid arthritis, Sjogren's syndrome, and lupus nephritis.

In some embodiments of the methods of treatment disclosed herein, the disease, disorder, or medical condition is selected from the group consisting of inflammatory disorders, autoimmune disorders, cancers, metabolic disorders, and osteoporosis.

In some embodiments of the methods of treatment disclosed herein, the disease, disorder, or medical condition is an autoantibody associated disease. In some embodiments, the autoantibody associated diseases is selected from the group consisting of anti-neutrophil cytoplasmic antibody ("ANCA") associated vasculitis, scleroderma, Sjogren's disease, myositis, IgG4 associated diseases, bullous pemphigoid, and neuromyelitis optica spectrum disorders ("NMOSD").

In some embodiments of the methods of treatment disclosed herein, the disease, disorder, or medical condition is an immune mediated dermatitis indication. In some embodiments, the immune mediated dermatitis indication is selected from the group consisting of atopic dermatitis and hidradenitis supperativa.

In some embodiments of the methods of treatment disclosed herein, the disease, disorder, or medical condition is a liver inflammation or a liver injury. In some embodiments, the liver inflammation or a liver injury is selected from the group consisting of steatosis, non-alcoholic steatohepatitis ("NASH") and primary biliary cirrhosis.

In some embodiments of the methods of treatment disclosed herein, the disease, disorder, or medical condition is cancer. In some embodiments, the cancer is selected from the group consisting of leukemias, lymphomas, pancreatic cancer, breast cancer, and melanoma.

In some embodiments of the methods of treatment disclosed herein, the disease, disorder, or medical condition is a metabolic disorder. In some embodiments, the metabolic disorder is selected from the group consisting of obesity and diabetes. In some embodiments, the diabetes is a type 2 diabetes.

In some embodiments of the methods of treatment disclosed herein, the disease, disorder, or medical condition is a kidney disease. In some embodiments, the kidney disease is selected from the group consisting of acute kidney injury, Berger's disease (IgA nephropathy (IgAN)), autosomal dominant polycystic kidney disease ("ADCKD"), and membranous nephropathy.

In some embodiments of the methods of treatment disclosed herein, the disease, disorder, or medical condition is osteoporosis.

In some embodiments of the methods of treatment disclosed herein, the disease, disorder, or medical condition is selected from the group consisting of RA, IBD, SLE, IgAN, metabolic syndrome, multiple sclerosis, Immune thrombocytopenic purpura, primary biliary cirrhosis, transplantation, myasthenia gravis, osteoporosis, and bone resorption (periodontitis).

In some embodiments of the methods of treatment disclosed herein, the disease, disorder, or medical condition is selected from the group consisting of systemic lupus erythematosus ("SLE"), rheumatoid arthritis ("RA"), Sjogren's syndrome, lupus nephritis, inflammatory bowel disease ("IBD"), ANCA associated vasculitis, myositis, IgG4 associated diseases, bullous pemphigoid, neuromyelitis optica spectrum disorders ("NMOSD"), atopic dermatitis "AD"), hidradenitis supperativa ("HS"), steatosis, non-alcoholic steatohepatitis ("NASH"), primary biliary cirrhosis, leukemias, lymphomas, pancreatic cancer, breast cancer, melanoma, obesity, diabetes, acute kidney injury, IgAN, autosomal dominant polycystic kidney disease ("ADCKD"), membranous nephropathy, osteoporosis, bone resorption (periodontitis), multiple sclerosis ("MS"), immune thrombocytopenic purpura, transplantation, myasthenia gravis, scleroderma, myositis, IgG4 associated diseases, and bullous pemphigoid.

In some embodiments, disclosed herein is a method for preventing or controlling an excessive inflammatory response, comprising administering to a subject in need of such treatment an effective amount of a compound of the disclosure, or a pharmaceutically acceptable salt thereof, or a pharmaceutical composition of the disclosure.

In some embodiments of the methods disclosed herein, the subject is a human subject.

The present disclosure also provides a method for modulating NIK activity, comprising exposing NIK to an effective amount of a compound of the disclosure, or a pharmaceutically acceptable salt thereof.

In some embodiments, the disclosure provides a method for inhibiting NIK activity, comprising exposing NIK to an effective amount of a compound of the disclosure, or a pharmaceutically acceptable salt thereof.

### Dosing and Administration

In the methods disclosed herein, an effective amount of at least one compound according to the disclosure is administered to a subject suffering from or diagnosed as having such a disease, disorder, or medical condition. An "effective amount" means an amount or dose sufficient to generally bring about the desired therapeutic or prophylactic benefit in patients in need of such treatment for the designated disease, disorder, or medical condition. For a 70-kg human, an illustrative range for a dosage amount is from about 1 to 1000 mg/day in single or multiple dosage units.

In some embodiments, the dosage amount is about 1 mg to 500 mg of a compound of the disclosure, or a pharmaceutically acceptable salt thereof. In some embodiments, the dosage amount is about 1, 5, 10, 15, 20, 25, 30, 35, 40, 45, 50, 55, 60, 65, 70, 75, 80, 85, 90, 95, or 100 mg of a compound of the disclosure, or pharmaceutically acceptable salt thereof. In some embodiments, the dosage amount is about 100, 105, 110, 115, 120, 125, 130, 135, 140, 145, 150, 155, 160, 165, 170, 175, 180, 185, 190, 195, or 200 mg of a compound of the disclosure, or pharmaceutically acceptable salt thereof. In some embodiments, the dosage amount is about 200, 205, 210, 215, 220, 225, 230, 235, 240, 245, 250, 255, 260, 265, 270, 275, 280, 285, 290, 295, or 300 mg of a compound of the disclosure, or pharmaceutically acceptable salt thereof. In some embodiments, the dosage amount is about 300, 315, 320, 325, 330, 335, 340, 345, 350, 355, 360, 365, 370, 375, 380, 385, 390, 395, or 400 mg of a compound of the disclosure, or pharmaceutically acceptable salt thereof. In some embodiments, the dosage amount is about 400, 405, 410, 415, 420, 425, 430, 435, 440, 445, 450, 455, 460, 465, 470, 475, 480, 485, 490, 495, or 500 mg of a compound of the disclosure, or pharmaceutically acceptable salt thereof

The dosage administered will be affected by factors such as but not limited to the route of administration, the health, weight and age of the recipient, the frequency of the treatment and the presence of concurrent and unrelated treatments.

It is also apparent to one skilled in the art that the therapeutically effective dose for compounds of the present disclosure or a pharmaceutical composition thereof will vary according to the desired effect. Therefore, optimal dosages to be administered may be readily determined by one skilled in the art and will vary with the particular compound used, the mode of administration, the strength of the preparation, and the advancement of the disease condition. In addition, factors associated with the particular subject being treated, including subject age, weight, diet and time of administration, will result in the need to adjust the dose to an appropriate therapeutic level. The above dosages are thus exemplary of the average case. There can, of course, be individual instances where higher or lower dosage ranges are merited, and such are within the scope of this present disclosure.

Once improvement of the patient's disease, disorder, or condition has occurred, the dose may be adjusted for preventive or maintenance treatment. For example, the dosage or the frequency of administration, or both, may be reduced as a function of the symptoms, to a level at which the desired therapeutic or prophylactic effect is maintained. Of course, if symptoms have been alleviated to an appropriate level, treatment may cease. Patients may, however, require intermittent treatment on a longterm basis upon any recurrence of symptoms.

The compounds of the present disclosure, or pharmaceutically acceptable salts thereof, may be formulated into pharmaceutical compositions comprising any known pharmaceutically acceptable carriers. Pharmaceutically acceptable carriers commonly used in pharmaceutical compositions are substances that are non-toxic, biologically tolerable, and otherwise biologically suitable for administration to a subject, such as an inert substance, added to a pharmacological composition or otherwise used as a vehicle or diluent to facilitate administration of an agent and that is compatible therewith. Exemplary carriers include, but are not limited to, any suitable solvents, dispersion media, coatings, antibacterial and antifungal agents and isotonic agents. Exemplary excipients that may also be components of the formulation include fillers, binders, disintegrating agents and lubricants.

Delivery forms of the pharmaceutical compositions containing one or more compounds of the disclosure may be prepared using pharmaceutically acceptable excipients and compounding techniques known or that become available to those of ordinary skill in the art. The compositions may be administered in the inventive methods by a suitable route of delivery, e.g., oral, parenteral, rectal, topical, or ocular routes, or by inhalation.

The preparation may be in the form of tablets, capsules, sachets, dragees, powders, granules, lozenges, powders for reconstitution, liquid preparations, or suppositories. The compositions may be formulated for any one of a plurality of administration routes, such as but not limited to intravenous infusion, subcutaneous injection, topical administration, or oral administration.

For oral administration, the compounds of the present disclosure can be provided in the form of tablets, capsules, or beads, or as a solution, emulsion, or suspension. To prepare the oral compositions, the active agents may be formulated to yield a dosage of, e.g., for a 70-kg human, from about 1 to 1000 mg/day in single or multiple dosage units as an illustrative range.

Oral tablets may include a compound of the disclosure mixed with compatible pharmaceutically acceptable excipients such as but not limited to diluents, disintegrating agents, binding agents, lubricating agents, sweetening agents, flavoring agents, coloring agents and preservative agents. Suitable inert fillers include sodium and calcium carbonate, sodium and calcium phosphate, lactose, starch, sugar, glucose, methyl cellulose, magnesium stearate, mannitol, sorbitol, and the like. Illustrative examples of liquid oral excipients include ethanol, glycerol, water, and the like. Starch, polyvinyl-pyrrolidone (PVP), sodium starch glycolate, microcrystalline cellulose, and alginic acid are examples of disintegrating agents. Binding agents may include starch and gelatin. The lubricating agent, if present, may be magnesium stearate, stearic acid or talc. If desired, the tablets may be coated with a material such as but not limited to glyceryl monostearate or glyceryl distearate to delay absorption in the gastrointestinal tract or may be coated with an enteric coating. Additional coatings that may be used include coatings that are designed to release the compound or active agent as a function of time, pH or bacterial content.

Capsules for oral administration include hard and soft gelatin or (hydroxypropyl)methyl cellulose capsules. To prepare hard gelatin capsules, active ingredient(s) may be mixed with a solid, semi-solid, or liquid diluent. Soft gelatin capsules may be prepared by mixing the active ingredient with an oil such as but not limited to peanut oil or olive oil, liquid paraffin, a mixture of mono and di-glycerides of short chain fatty acids, polyethylene glycol 400, or propylene glycol. Liquids for oral administration may be in the form of suspensions, solutions, emulsions or syrups or may be lyophilized or presented as a dry product for reconstitution with water or other suitable vehicle before use. Such liquid compositions may optionally contain: pharmaceutically-acceptable excipients such as but not limited to suspending agents (for example, sorbitol, methyl cellulose, sodium alginate, gelatin, hydroxyethylcellulose, carboxymethylcellulose, aluminum stearate gel and the like); non-aqueous vehicles, e.g., oil (for example, almond oil or fractionated coconut oil), propylene glycol, ethyl alcohol, or water; preservatives (for example, methyl or propyl p-hydroxybenzoate or sorbic acid); wetting agents such as but not limited to lecithin; and, if desired, flavoring or coloring agents.

The compounds of the present disclosure may also be administered by non-oral routes. For example, compositions may be formulated for rectal administration as a suppository, enema or foam. For parenteral use, including intravenous, intramuscular, intraperitoneal, or subcutaneous routes, the compounds of the disclosure may be provided in sterile aqueous solutions or suspensions, buffered to an appropriate pH and isotonicity or in parenterally acceptable oil. Suitable aqueous vehicles include Ringer's solution and isotonic sodium chloride. Such forms may be presented in unit-dose form such as but not limited to ampules or disposable injection devices, in multi-dose forms such as vials from which the appropriate dose may be withdrawn, or in a solid form or pre-concentrate that can be used to prepare an injectable formulation. Illustrative infusion doses range from about 1 to 1000 µg/kg/minute of agent admixed with a pharmaceutical carrier over a period ranging from several minutes to several days.

For topical administration, the compounds of the disclosure may be mixed with a pharmaceutical carrier. Another mode of administering the compounds of the disclosure may utilize a patch formulation to effect transdermal delivery.

Compounds of the disclosure may alternatively be administered in methods of this present disclosure by inhalation, via the nasal or oral routes, e.g., in a spray formulation also containing a suitable carrier.

Although the present embodiments have been described in connection with certain specific embodiments for instructional purposes, the present embodiments are not limited thereto. Accordingly, various modifications, adaptations, and combinations of various features of the described embodiments can be practiced without departing from the scope of the invention as set forth in the claims. Furthermore, the following examples are illustrative, but not limiting, of the compounds, compositions and methods described herein. Other suitable modifications and adaptations known to those skilled in the art are within the scope of the following embodiments.

### EXAMPLES

The following specific examples are provided to further illustrate embodiments within the scope of the present disclosure.

### Abbreviations

Herein and throughout the application, the following abbreviations may be used.

| | |
|---|---|
| Ac | acyl or acetyl |
| ACN or MeCN | acetonitrile |
| AcOH | acetic acid |
| BINAP | 1,1'-binaphthalene]-2,2'-diylbis[diphenylphosphine |
| br | broad |
| BrettPhos-Pd-G3 | [(2-Di-cyclohexylphosphino-3,6-dimethoxy-2',4',6'-triisopropyl-1,1'-biphenyl)-2-(2'-amino-1,1'-biphenyl)]palladium(II) methanesulfonate methanesulfonate |
| Bu | butyl |
| *n*-BuLi | *n*-butyllithium |
| *t*-BuOH | *t*-butanol |
| *t*-BuOK | potassium *t*-butoxide |
| *t*-BuONa | sodium *t*-butoxide |
| Cu(OAc)₂ | copper(II) acetate |
| d | doublet |
| DAC | dynamic axial compression |
| DBU | 1,8-diazabicyclo(5.4.0)undec-7-ene |
| DCM | dichloromethane |
| DEA | diethylamine |
| DIAD | diisopropyl azodicarboxylate |
| DIEA or DIPEA | diisoproylethylamine |
| DMA | dimethylacetamide |
| DMAP | 4-(dimethylamino)pyridine |
| DMF | *N,N*-dimethylformamide |
| DMSO | dimethyl sulfoxide |
| ESI | electrospray ionization |
| Et | ethyl |
| Et₂NH | diethylamine |
| Et₂O | diethyl ether |
| EtOAc or EA | ethyl acetate |
| EtOH | ethanol |
| FCC | flash column chromatography |
| h or hr | hour(s) |
| HATU | 1-Bis(dimethylamino)methylene]-1*H*-1,2,3-triazolo[4,5-*b*]pyridinium 3-oxid hexafluorophosphate |
| HPLC | high pressure liquid chromatography |
| Hz | Hertz |
| ICl | (2*R*,3*R*)-*rel*-3-isopropylamino-1-(7-methylindan-4-yloxy)-butan-2-ol hydrochloride |
| IPA or *i*-PrOH | isopropanol |
| KOAc | potassium acetate |
| Lawesson's reagent | 2,4-Bis(4-methoxyphenyl)-1,3,2,4-dithiadiphosphetane-2,4-disulfide |
| LCMS | liquid chromatography mass spectrometry |
| LDA | lithium diisopropylamide |
| LiHMDS | lithium bis(trimethylsilyl)amide |
| LiOtBu | lithium *t*-butoxide |
| mCPBA or m-CPBA | meta-chloroperoxybenzoic acid |
| mmol | millimoles |
| m/z | mass-to-charge ratio |
| M+ | parent molecular ion |
| Me | methyl |
| MeNH₂ | methylamine |
| MeOH | methanol |
| min | minute(s) |
| MS | mass spectrometry |
| MTBE or TBME | *tert*-butyl methyl ether |
| NaHMDS | sodium bis(trimethylsilyl)amide |
| NaOMe | sodium methoxide |
| NaSO₂Me | sodium methyl sulfate |
| NCS | *N*-chlorosuccinimide |
| NBS | *N*-bromosuccinimide |
| NMP | *N*-methyl-2-pyrrolidone |
| NMR | nuclear magnetic resonance |
| nt | not tested |
| Pd₂(dba)₃ | tris(dibenzylideneacetone)dipalladium(0) |
| Pd(*t*-Bu₃P)₂ | bis(tri-tert-butylphosphine)palladium(0) |
| PdCl₂(dppf) or Pd(dppf)Cl₂ | [1,1-bis(diphenylphosphino)ferrocene]dichloropalladium(II) |
| Pd(dtbpf)Cl₂ | [1,1-bis(di-*tert-*butylphosphino)ferrocene]dichloropalladium(II) |
| Pd(PPh₃)₄ | tetrakis(triphenylphosphine)palladium(0) |
| PdCl₂(PPh₃)₂ or Pd(PPh₃)₂Cl₂ | bis(triphenylphosphine)palladium(II) dichloride |
| Pd(OAc)₂ | palladium(II) acetate |
| PE or pet ether | petroleum ether |
| PivOH | 2,2-dimethylpropanoic acid |
| RP | reversed-phase |
| rt | room temperature |
| RuPhos Pd G3 | [2'-(Amino-κN)[1,1'-biphenyl]-2-yl-κC][[2',6'-bis(1-methylethoxy)[1,1'-biphenyl]-2-yl]dicyclohexylphosphine-κP](methanesulfonato-κO)palladium |
| RuPhos Pd G4 | (SP-4-3)-[[2',6'-Bis(1-methylethoxy)[1,1'-biphenyl]-2-yl]dicyclohexylphosphine-κP](methanesulfonato-κO)[2'-(methylamino-κN)[1,1'-biphenyl]-2-yl-κC]palladium |
| sat. | saturated |
| SFC | supercritical fluid chromatography |
| Select-F | selectfluor |
| SEM-Cl | 2-(trimethylsilyl)ethoxymethyl chloride |
| (SnBu₃)₂ | bis(tributyltin) |
| TBAF | tetrabutylammonium fluoride |
| TBSCl | *tert*-butyldimethylsilyl chloride |
| *t*-BuOK | potassium tert-butoxide |
| TEA or Et₃N | triethylamine |
| TFA | trifluoroacetic acid |
| THF | tetrahydrofuran |
| TLC | thin layer chromatography |
| TosCl or TsCl | *p*-toluenesulfonyl chloride |
| TsOH | *p*-toluenesulfonic acid |
| UV | ultraviolet |
| v/v | volume-to-volume ratio |
| XPhos Pd G2 | Chloro(2-dicyclohexylphosphino-2',4',6'-triisopropyl-1,1'-biphenyl)[2-(2'-amino-1,1'-biphenyl)]palladium(II) |
| Xantphos | (9,9-Dimethyl-9H-xanthene-4,5-diyl)bis(diphenylphosphane) |

In some embodiments, provided herein are processes and intermediates disclosed herein that are useful for preparing a compound of the disclosure or pharmaceutically acceptable salts thereof.

By way of illustration, but not as limitation, compounds of the present disclosure are prepared according to the following general preparation procedures given by Schemes 1-5. One of ordinary skill in the art will recognize that, to obtain the various compounds herein, starting materials may be suitably selected so that the ultimately desired substituents will be carried through the reaction scheme with or without protection as appropriate to yield the desired product. Alternately, in the place of the ultimately desired substituent, a suitable group may be carried through the reaction scheme and replaced, as appropriate, with the desired substituent. Unless otherwise specified, the variables in Schemes 1-5 are as defined above in reference to Formula I.

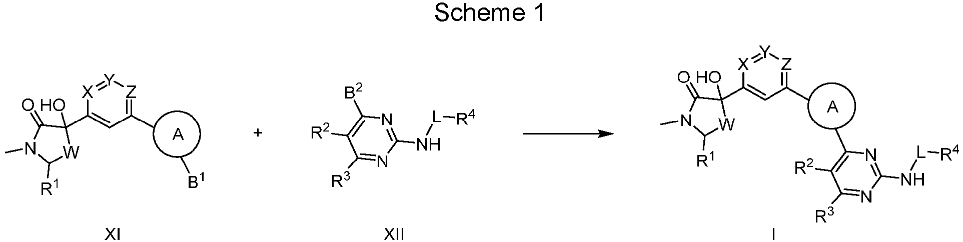

The compounds of Formula I of the present disclosure can be prepared, for example, as shown in Scheme 1. When substituent B¹ in compound XI is a halide (e.g., Cl, Br, or I) and when substituent B² in compound XII is a stannyl group (e.g., Sn(Me)₃ or Sn(n-Bu)₃), this coupling is achieved by reaction under Stille conditions. One having skill in the art would recognize that coupling under Stille conditions also provides a compound of Formula I when B¹ in compound XI is a stannyl group and B² in compound XII is a halide. Typical Stille coupling conditions involve the use of a catalyst (usually palladium, but sometimes nickel), a suitable solvent, and other optional reagents including ligands, TEA, or CuI. Examples of suitable catalysts include, but are not limited to Pd(PPh₃)₄, Pd(dppf)Cl₂, and Pd₂(dba)₃. Suitable solvents include, but are not limited to, toluene, 1,4-dioxane, DMF, or mixtures thereof. The reaction may be heated to a temperature from about 80 °C to about 130 °C for a time period of about 1 to 16 hours, employing microwave or conventional heating to provide the compound of Formula I.

Alternately, Suzuki coupling conditions can be used to couple compound XI to compound XII when B¹ is a halide such as but not limited to Cl, Br, or I, and when B² is a boron-based coupling agent such as but not limited to a boronic acid or a boronic ester. One having skill in the art would recognize that coupling under Suzuki conditions also provides a compound of Formula I when B¹ is a boron-based coupling agent and when B² is a halide. Typical Suzuki coupling conditions involve the use of a palladium catalyst, a base, a suitable solvent, and other optional reagents including ligands (e.g., tricyclohexylphosphine). Examples of suitable palladium catalysts include, but are not limited to, Pd(dppf)Cl₂, BrettPhos-Pd-G3, and Pd₂(dba)₃. Suitable bases include, but are not limited to, K₂CO₃, CS₂CO₃, KOAc, and combinations thereof. Suitable solvents include, but are not limited to, 1,4-dioxane, 2-methyl-2-butanol, water, or mixtures thereof. The reaction may be heated to a temperature of about 80 °C to about 120 °C for a time period of about 0.5 to 16 hours, employing microwave or conventional heating to provide the compound of Formula I.

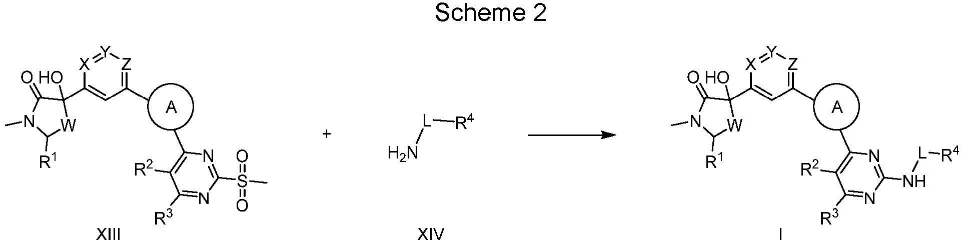

The compounds of Formula I of the present disclosure can be prepared, for example, as shown in Scheme 2. Compounds XIII and XIV may be combined with a suitable acid, such as but not limited to TFA, TsOH, or HCl, in a solvent, such as but not limited to isopropanol, DMSO, DMF, or 1,4-dioxane. The reaction may be heated to a temperature from about 100 °C to about 160 °C for a time period of about 1 to 16 hours, employing microwave or conventional heating to provide the compound of Formula I.

Alternately, compounds XIII and XIV may be combined with a suitable base, such as but not limited to NaHMDS or DIPEA, in a solvent such as but not limited to DMA or THF. The reaction mixture may be heated to a temperature from about 120 °C to about 160 °C for a time period of about 1.5 to 3.5 hours, employing microwave or conventional heating to provide the compound of Formula I. Alternately, the reaction mixture may be cooled to about -72 °C for about 1 hour to provide the compound of Formula I.

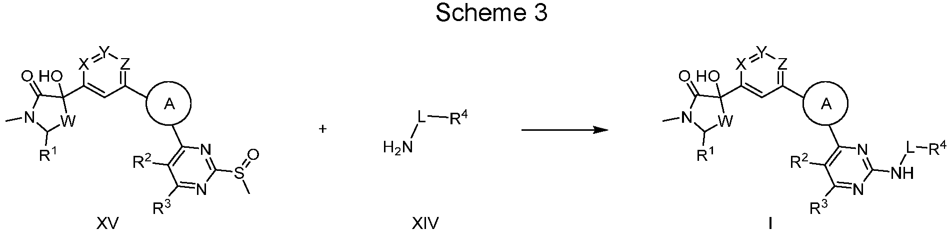

The compounds of Formula I of the present disclosure can be prepared, for example, as shown in Scheme 3. Compounds XV and XIV may be combined with a suitable acid, such as but not limited to TFA, TsOH, or HCl, in a solvent, such as but not limited to isopropanol, DMSO, DMF, or 1,4-dioxane. The reaction may be heated to a temperature from about 100 °C to about 160 °C for a time period of about 1 to 16 hours, employing microwave or conventional heating to provide the compound of Formula I.

Alternately, compounds XV and XIV may be combined with a suitable base, such as but not limited to DIEA, in a solvent such as but not limited to isopropanol, DMA, or THF. The reaction mixture may be heated to a temperature from about 120 °C to about 160 °C for a time period of about 1.5 to 3.5 hours, employing microwave or conventional heating to provide the compound of Formula I.

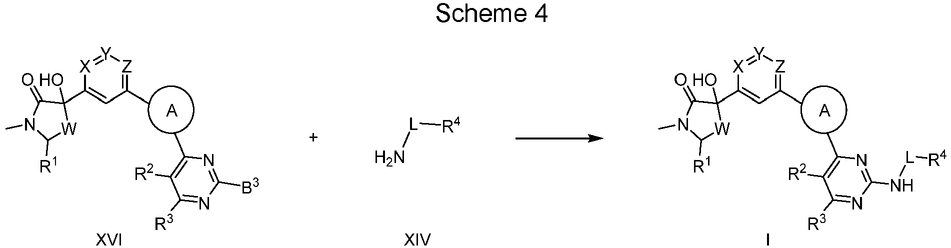

The compounds of Formula I of the present disclosure can be prepared, for example, as shown in Scheme 4. When substituent B³ in compound XVI is a halide (e.g., Cl, Br, or I), preparation of the compound of Formula I can be achieved under Buchwald-Hartwig amination conditions. Typical Buchwald-Hartwig reactions involve the use of a catalyst (usually palladium, but sometimes other metals; e.g., Pd(OAc)₂), a base (e.g., Cs₂CO₃), and a suitable solvent (e.g., 1,4-dioxane). A phosphate ligand, such as but not limited to Xantphos, may also be included. The reaction may be heated to a temperature from about 90 °C to about 120 °C for a time period of about 1 to 12 hours to provide the compound of Formula I.

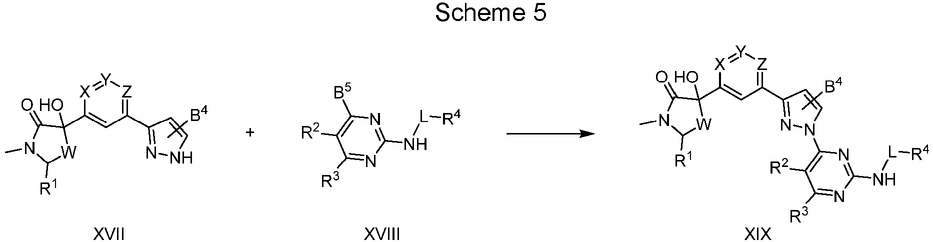

The compounds of Formula XIX of the present disclosure can be prepared, for example, as shown in Scheme 5 where B⁴ is halo, -C₍₁₋₄₎alkyl, -C₍₁₋₄₎haloalkyl, -OC₍₁₋₄₎alkyl, and -OC₍₁₋₄₎haloalkyl, and where B⁵ is a halide (e.g., F or Cl). Compounds XVII and XVIII may be combined with a suitable base (e.g., Cs₂CO₃) in a suitable solvent (e.g., DMF). The reaction may be heated to a temperature from about 100 °C to about 120 °C for a time period of about 0.5 to 2 hours to provide the compound of Formula I.

In obtaining the compounds described in the examples below and the corresponding analytical data, the following experimental and analytical protocols were followed unless otherwise indicated.

Unless otherwise specified, reaction solutions were stirred at room temperature under a N_{2(g)} or Ar_{(g)} atmosphere. When solutions were "concentrated to dryness", they were concentrated using a rotary evaporator under reduced pressure. When solutions were dried, they are typically dried over a drying agent such as but not limited to MgSO₄ or Na₂SO₄. Normal phase flash column chromatography (FCC) was performed on silica gel with prepackaged silica gel columns, such as but not limited to RediSep^{®}, using ethyl acetate (EtOAc)/hexanes, CH₂Cl₂/MeOH, or CH₂Cl₂/10% 2N NH₃ in MeOH, as eluent, unless otherwise indicated.

The compounds described in the examples may also be purified via preparative reverse-phase HPLC. A typical HPLC chromatographic separation ranges from about 10 to about 20 minutes. Suitable solvent gradients and conditions for purification may be determined by one having skill in the art. Unless otherwise specified, where reverse-phase HPLC is used to purify a compound described in one of the examples below, the solvent used is a gradient of 10% to 80% acetonitrile in water. When the purification is performed under "acidic conditions" or in "acidic media," the acetonitrile and water both contain 0.16% TFA. When performed under "basic conditions" or in "basic media," the pH of the acetonitrile and water have been adjusted to pH 10 with ammonium hydroxide. The following column abbreviations are also used throughout the examples:

| | **Column Type** |
|---|---|
| C1 | Waters XSelect CSH C18, 5 µm, 19x100 mm |
| C2 | Waters XBridge BEH C18, 5 µm, 19x100 mm |
| C3 | Waters XSelect CSH C18, 5 µm, 19x150 mm |
| C4 | Waters XBridge BEH C18, 5 µm, 19x150 mm |
| C5 | Waters XSelect CSH Fluoro Phenyl, 5 µm, 19x100 mm |
| C6 | Waters XSelect CSH Fluoro Phenyl, 5 µm, 19x150 mm |
| C7 | Waters XSelect CSH C18, 5 µm, 30x150 mm |
| C8 | Waters XBridge BEH C18, 5 µm, 30x150 mm |
| C9 | Waters XBridge OBD C18, 5 µm, 50x100 mm |
| C10 | Boston Prime C18 column, 5 µm, 150 x 30 mm |
| C11 | Boston Green ODS C18 column, 5 µm, 150 x 30 mm |
| C12 | Xtimate C18, 5 µm, 150 x 25 mm |
| SFC1 | CHIRALPAK^{®} IB N3 5 µm 250 x 21 mm, |
| SFC2 | CHIRALCEL^{®} OZ-H 5 µm 250 x 21 mm |

| Descriptor | Mobile Phases |
|---|---|
| acidic conditions/ acidic media | water containing 0.16% TFA and acetonitrile containing 0.16% TFA |
| basic conditions/ basic media | water at pH 10 with ammonium hydroxide and acetronitrile at pH 10 ammonium hydroxide |

Thin-layer chromatography was performed using silica gel plates, such as but not limited to Merck silica gel 60 F₂₅₄ 2.5 cm x 7.5 cm 250 mm or 5.0 cm x 10.0 cm 250 µm pre-coated silica gel plates. Preparative thin-layer chromatography was performed using silica gel plates such as but not limited to EM Science silica gel 60 F₂₅₄ 20 cm x 20 cm 0.5 mm pre-coated plates with a 20 cm x 4 cm concentrating zone. Microwave reactions were carried out in a microwave reactor, such as but not limited to a CEM Discover^{â}, a Biotage Initiator^{™} or Optimizer^{™} microwave, at specified temperatures. Mass spectra were obtained on a mass spectrometer, such as but not limited to Agilent series 1100 MSD using electrospray ionization (ESI) in positive mode unless otherwise indicated. Calculated mass corresponds to the exact mass. NMR spectra were obtained on an NMR spectrometer, such as but not limited to a Bruker model DPX400 (400 MHz), DPX500 (500 MHz), DRX600 (600 MHz) spectrometer. The format of the ¹H NMR data below is as follows: Chemical shift in ppm down field of the tetramethylsilane reference (multiplicity, coupling constant J in Hz, integration).

Whenever a yield is given as a percentage, such yield refers to a mass of the entity for which the yield is given with respect to the maximum amount of the same entity that could be obtained under the particular stoichiometric conditions. Reagent concentrations that are given as percentages refer to mass ratios, unless indicated differently. Whether expressly indicated or not, yields given in the following examples are computed with respect to the dried form of the compound for which any such yield is given.

Chemical names were generated using ChemDraw Ultra 17.1 (CambridgeSoft Corp., Cambridge, MA) or OEMetaChem V1.4.0.4 (Open Eye).

### Intermediate 1: N-(1-methyl-1H-pyrazol-3-yl)-4-(trimethylstannyl)pyrimidin-2-amine

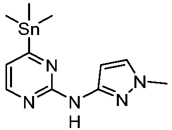

A mixture of 4-chloro-N-(1-methylpyrazol-3-yl)pyrimidin-2-amine (7.50 g, 35.8 mmol), tetrakis(triphenylphosphine)palladium(0) (4.13 g, 3.58 mmol), hexamethylditin (17.2 g, 52.5 mmol) and 1,4-dioxane (250 mL) was heated at 100 °C for 16h. The mixture was cooled to rt, diluted with aqueous KF (2 M, 150 mL), and stirred for 1h. The mixture was then extracted with ethyl acetate, washed with brine, dried with Na₂SO₄, filtered, and concentrated under reduced pressure to afford N-(1-methyl-1H-pyrazol-3-yl)-4-(trimethylstannyl)pyrimidin-2-amine (15.3 g, about 38% purity, 48%) as a brown powder which was used without further purification. MS (ESI⁺): m/z = 339.5.

### Intermediate 2: (R)-3-(3-(4-bromooxazol-2-yl)phenyl)-3-hydroxy-1-methylpyrrolidin-2-one

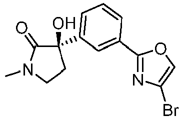

A mixture of (*R*)-3-hydroxy-1-methyl-3-[3-(4,4,5,5-tetramethyl-1,3,2-dioxaborolan-2-yl)phenyl]pyrrolidin-2-one (250 mg, 0.788 mmol), 2,4-dibromooxazole (358 mg, 1.58 mmol), [1,1'-bis(diphenylphosphino)ferrocene]dichloropalladium(II) (58 mg, 0.079 mmol), K₂CO₃ (1.58 mL, 1 M in H₂O, 1.58 mmol) and 1,4-dioxane (6 mL) was heated at 100 °C for 2 h. The mixture was cooled to rt, diluted with water, and extracted with ethyl acetate. The organic extract was dried with MgSO₄, filtered, and concentrated under reduced pressure. The residue was purified by FCC (gradient 30% to 100% EtOAc in hexanes) to afford (*R*)-3-(3-(4-bromooxazol-2-yl)phenyl)-3-hydroxy-1-methylpyrrolidin-2-one (225 mg, 84) as a tacky white solid. ¹H NMR (600 MHz, DMSO-*d₆*) δ 8.49 (s, 1H), 8.02 (dt, *J* = 2.4, 1.0 Hz, 1H), 7.86 (ddd, *J* = 6.1, 3.0, 1.8 Hz, 1H), 7.55 - 7.47 (m, 2H), 6.21 (s, 1H), 3.47 (ddd, *J* = 9.9, 8.0, 5.2 Hz, 1H), 3.36 (ddd, *J* = 9.9, 7.8, 5.3 Hz, 1H), 2.85 (s, 3H), 2.34 (ddd, *J* = 13.2, 7.9, 5.3 Hz, 1H), 2.26 (ddd, *J* = 13.3, 8.0, 5.2 Hz, 1H). MS (ESI⁺): m/z = 337.1.

### Intermediate 3: (R)-3-(3-(1H-pyrazol-3-yl)phenyl)-3-hydroxy-1-methylpyrrolidin-2-one

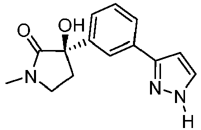

Step A: A mixture of K₂CO₃ (7.8 mL, 1 M in H₂O, 7.8 mmol) and 1,4-dioxane (40 mL) was sparged with N₂ for 15 minutes and then added to a mixture of (R)-3-hydroxy-1-methyl-3-[3-(4,4,5,5-tetramethyl-1,3,2-dioxaborolan-2-yl)phenyl]pyrrolidin-2-one (1.00 g, 3.15 mmol), 3-bromo-1-(tetrahydro-2H-pyran-2-yl)-1H-pyrazole (947 mg, 4.10 mmol) and bis(triphenylphosphine)palladium(II) dichloride (143 mg, 0.205 mmol) to afford a reaction mixture. The reaction mixture was heated to 100 °C for 1 hr, cooled to rt, diluted with water, and extracted with ethyl acetate. The organic extract was dried with MgSO₄, filtered, and concentrated under reduced pressure. The residue was purified by FCC (100% EtOAc) to afford (R)-3-hydroxy-1-methyl-3-(3-(1-(tetrahydro-2H-pyran-2-yl)-1H-pyrazol-3-yl)phenyl)pyrrolidin-2-one (517 mg, 48%) as a white powder. MS (ESI⁺): m/z = 342.2.

Step B: A mixture of (R)-3-hydroxy-1-methyl-3-(3-(1-(tetrahydro-2H-pyran-2-yl)-1H-pyrazol-3-yl)phenyl)pyrrolidin-2-one (517 mg, 1.514 mmol), TFA (1 mL) and DCM (5 mL) was stirred at rt overnight. The reaction mixture was concentrated and then diluted with DCM and washed with K₂CO₃ solution (1 M in H₂O). The organic layer was dried with MgSO₄, filtered, and concentrated to afford (*R*)-3-(3-(1H-pyrazol-3-yl)phenyl)-3-hydroxy-1-methylpyrrolidin-2-one (533 mg). MS (ESI⁺): m/z = 258.15.

### Intermediate 4: 4-(4-bromothiazol-2-yl)-N-(1-methyl-1H-pyrazol-3-yl)pyrimidin-2-amine

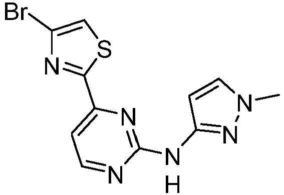

A mixture of 2,4-dibromothiazole (151 mg, 0.621 mmol), N-(1-methyl-1H-pyrazol-3-yl)-4-(trimethylstannyl)pyrimidin-2-amine (Intermediate 1, 140 mg, 0.414 mmol), tetrakis(triphenylphosphine)palladium(0) (48 mg, 0.041 mmol) and 1,4-dioxane (3.5 mL) was heated at 95 °C overnight then at 110 °C for 5 h. The reaction mixture was diluted with water and extracted with ethyl acetate. The organic extract was dried with MgSO₄, filtered, and concentrated under reduced pressure. The residue was purified by FCC (30% to 100% EtOAc in hexanes) to afford 4-(4-bromothiazol-2-yl)-N-(1-methyl-1H-pyrazol-3-yl)pyrimidin-2-amine (20 mg, 14%). MS (ESI⁺): m/z = 338.0.

### Intermediate 5: 4-(4-bromooxazol-2-yl)-N-(1-methyl-1H-pyrazol-3-yl)pyrimidin-2-amine

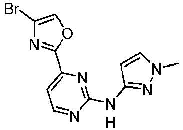

A mixture of N-(1-methyl-1H-pyrazol-3-yl)-4-(trimethylstannyl)pyrimidin-2-amine (Intermediate 1, 150 mg, 0.444 mmol), 2,4-dibromooxazole (201 mg, 0.888 mmol), tetrakis(triphenylphosphine)palladium(0) (77 mg, 0.067 mmol) and DMF (2 mL) was heated in a microwave reactor at 130 °C for 2 h. The reaction mixture was cooled to rt, diluted with water, and extracted with ethyl acetate. The organic extract was dried with MgSO₄, filtered, and concentrated under reduced pressure. The residue was purified by FCC (30% to 100% EtOAc in hexanes) to afford 4-(4-bromooxazol-2-yl)-N-(1-methyl-1H-pyrazol-3-yl)pyrimidin-2-amine (10 mg, 7%) as a yellow powder. MS (ESI⁺): m/z = 321.0.

### Intermediate 6: 4-bromo-2-(2-(methylthio)pyrimidin-4-yl)thiazole

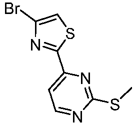

A mixture of 2,4-dibromothiazole (7.0 g, 29 mmol), 2-(methylthio)-4-(tributylstannyl)pyrimidine (10 g, 24 mmol), tetrakis(triphenylphosphine)palladium(0) (1.4 g, 1.2 mmol) and DMF (200 mL) was heated to 140 °C for 5 hr. The mixture was cooled to rt, diluted with ethyl acetate and washed with brine. The organic extract was dried with MgSO₄, filtered, and concentrated under reduced pressure. The residue was purified by FCC (20% to 90% EtOAc in hexanes) to afford 4-bromo-2-(2-(methylthio)pyrimidin-4-yl)thiazole (2.9 g, 41%) as a white powder. ¹H NMR (500 MHz, DMSO) δ 8.82 (d, *J* = 5.1 Hz, 1H), 8.20 (s, 1H), 7.75 (d, *J* = 5.1 Hz, 1H), 2.59 (s, 3H). MS (ESI⁺): m/z = 287.9.

### Intermediate 7: (R)-3-(3-(4-bromothiazol-2-yl)phenyl)-3-hydroxy-1-methylpyrrolidin-2-one

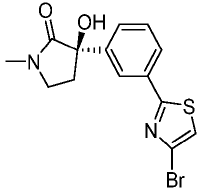

A mixture of (*R*)-3-hydroxy-1-methyl-3-(3-(4,4,5,5-tetramethyl-1,3,2-dioxaborolan-2-yl)phenyl)pyrrolidin-2-one (16.0 g, 50.4 mmol), Pd(dppf)Cl₂•CH₂Cl₂ (4.12 g, 5.04 mmol), 2,4-dibromothiazole (12.3 g, 50.4 mmol), K₃PO₄ (32.1 g, 151 mmol), water (50 mL), and anhydrous 1,4-dioxane (200 mL) was heated at 100 °C for 16 h and then cooled to rt. The resulting mixture was diluted with water and extracted with ethyl acetate. The organic extract was washed with brine, dried over anhydrous Na₂SO₄, filtered, and concentrated. The residue was purified by FCC (gradient 5:1 to 0:1 petroleum ether:ethyl acetate) to afford (*R*)-3-(3-(4-bromothiazol-2-yl)phenyl)-3-hydroxy-1-methylpyrrolidin-2-one as a brown solid (10 g, 43%). ¹H NMR (400 MHz, CDCl₃) δ 8.13 - 7.96 (m, 1H), 7.90 - 7.80 (m, 1H), 7.42 (br s, 2H), 7.26 - 7.21 (m, 1H), 3.68 - 3.35 (m, 3H), 3.02 (s, 3H), 2.61 - 2.36 (m, 2H). MS (ESI⁺): m/z = 353.0.

### Intermediate 8: (R)-3-hydroxy-1-methyl-3-(3-(4-(2-(methylthio)pyrimidin-4-yl)thiazol-2-yl)phenyl)pyrrolidin-2-one

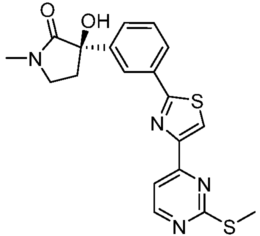

A mixture of (*R*)-3-(3-(4-bromothiazol-2-yl)phenyl)-3-hydroxy-1-methylpyrrolidin-2-one (Intermediate 7, 500 mg, 0.142 mmol), Pd(PPh₃)₄ (176 mg, 0.142 mmol), 2-(methylthio)-4-(tributylstannyl)pyrimidine (647 mg, 1.56 mmol), TEA (0.395 mL, 1.13 mmol), and anhydrous toluene (10 mL) was heated at 120 °C for 16 h and then cooled to rt. The resulting mixture was poured into aqueous KF (10 mL). The resulting suspension was filtered through a pad of diatomaceous earth and the pad washed with EtOAc (20 mL). The filtrate was further extracted with EtOAc (20 mL x 3) and the combined organic extracts were washed with brine, dried over anhydrous Na₂SO₄, filtered, and concentrated. The residue was purified by FCC (silica, gradient 5:1-0:1 petroleum ether-ethyl acetate) to afford (R)-3-hydroxy-1-methyl-3-(3-(4-(2-(methylthio)pyrimidin-4-yl)thiazol-2-yl)phenyl)pyrrolidin-2-one as a brown oil (397 mg, 57%). MS (ESI⁺): m/z = 399.0.

### Intermediate 9: (R)-3-hydroxy-1-methyl-3-(3-(2-(2-(methylsulfonyl)pyrimidin-4-yl)thiazol-4-yl)phenyl)pyrrolidin-2-one

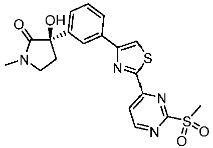

Step A: A mixture of 4-bromo-2-(2-(methylthio)pyrimidin-4-yl)thiazole (Intermediate 6, 645 mg, 2.24 mmol), (R)-3-hydroxy-1-methyl-3-[3-(4,4,5,5-tetramethyl-1,3,2-dioxaborolan-2-yl)phenyl]pyrrolidin-2-one (854 mg, 2.69 mmol), [1,1'-bis(diphenylphosphino)ferrocene]dichloropalladium(II) (82 mg, 0.11 mmol), K₂CO₃ (5.6 mL, 1 M in H₂O, 5.6 mmol), and 1,4-dioxane (10 mL) was heated to 80 °C for 1 h. The reaction mixture was cooled to rt, diluted with water, and extracted with ethyl acetate. The organic extract was dried with MgSO₄, filtered, and concentrated under reduced pressure. The residue was purified by FCC (50% to 100% EtOAc in hexanes) to afford (*R*)-3-hydroxy-1-methyl-3-(3-(2-(2-(methylthio)pyrimidin-4-yl)thiazol-4-yl)phenyl)pyrrolidin-2-one (715 mg, 80%) as a white powder. ¹H NMR (500 MHz, DMSO) δ 8.83 (d, *J* = 5.1 Hz, 1H), 8.44 (s, 1H), 8.12 - 8.06 (m, 1H), 7.99 - 7.91 (m, 1H), 7.89 (d, *J* = 5.0 Hz, 1H), 7.49 - 7.41 (m, 1H), 7.40 - 7.32 (m, 1H), 6.10 (s, 1H), 3.52 - 3.43 (m, 1H), 3.43 - 3.33 (m, 1H), 2.87 (s, 3H), 2.61 (s, 3H), 2.44 - 2.34 (m, 1H), 2.33 - 2.21 (m, 1H). MS (ESI⁺): m/z = 399.0.

Step B: A mixture of (R)-3-hydroxy-1-methyl-3-(3-(2-(2-(methylthio)pyrimidin-4-yl)thiazol-4-yl)phenyl)pyrrolidin-2-one (715 mg, 1.79 mmol), potassium peroxymonosulfate (2.4 g, 3.9 mmol), acetone (5 mL), water (5 mL) and THF (5 mL) was stirred at rt overnight. The mixture was diluted with ice water (30 mL) and stirred for 10 minutes. The resulting suspension was filtered and the precipitate was collected and washed with water and then dried under reduced pressure. The solid was washed with water and then dried under high vacuum to afford (*R*)-3-hydroxy-1-methyl-3-(3-(2-(2-(methylsulfonyl)pyrimidin-4-yl)thiazol-4-yl)phenyl)pyrrolidin-2-one (642 mg, 83%) as a white solid. The material contained approximately 20% (R)-3-hydroxy-1-methyl-3-(3-(2-(2-(methylsulfinyl)pyrimidin-4-yl)thiazol-4-yl)phenyl)pyrrolidin-2-one. ¹H NMR (400 MHz, DMSO-*d₆*) δ 9.24 (d, *J =* 5.2 Hz, 1H), 8.57 (s, 1H), 8.46 (d, *J* = 5.1 Hz, 1H), 8.12 (q, *J* = 2.0 Hz, 1H), 8.08 - 7.92 (m, 1H), 7.55 - 7.44 (m, 1H), 7.44 - 7.32 (m, 1H), 6.12 (d, *J =* 1.0 Hz, 1H), 3.56 - 3.44 (m, 4H), 3.44 - 3.36 (m, 1H), 2.87 (s, 4H), 2.49 - 2.34 (m, 1H), 2.33 - 2.20 (m, 1H). MS (ESI⁺): m/z = 432.0.

### Intermediate 10: 4-bromo-2-(2-(methylsulfonyl)pyrimidin-4-yl)thiazole

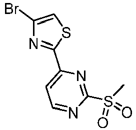

A mixture of 4-bromo-2-(2-(methylthio)pyrimidin-4-yl)thiazole (Intermediate 6, 500 mg, 1.74 mmol), potassium peroxymonosulfate (2.35 g, 3.82 mmol), acetone (3.6 mL), water (3.6 mL) and MeOH (3.6 mL) was stirred at rt overnight. Potassium peroxymonosulfate (2.35 g, 3.82 mmol) was added and the mixture was stirred at rt for an additional 24 h. The reaction mixture was diluted with 20 mL water and the resulting precipitate was collected by filtration, washed with water, and dried under reduced pressure. The solid obtained was re-suspended in water (20 mL) and stirred vigorously for 5 minutes. The resulting precipitate was collected by filtration, washed with water, and dried under reduced pressure to afford 4-bromo-2-(2-(methylsulfonyl)pyrimidin-4-yl)thiazole (383 mg, 69%) as a white powder. ¹H NMR (500 MHz, DMSO-*d₆*) δ 9.24 (d, *J* = 5.2 Hz, 1H), 8.34 (d, *J* = 5.2 Hz, 1H), 8.32 (s, 1H), 3.49 (s, 3H). MS (ESI⁺): m/z = 320.1.

### Intermediate 11: 4-(4-bromothiazol-2-yl)-N-(1-methyl-1H-pyrazol-4-yl)pyrimidin-2-amine

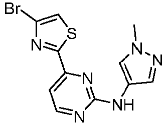

A mixture of 4-bromo-2-(2-(methylsulfonyl)pyrimidin-4-yl)thiazole (Intermediate 10, 150 mg, 0.468 mmol), 4-amino-1-methylpyrazole (91 mg, 0.94 mmol), TFA (0.090 mL, 1.2 mmol) and DMSO (0.75 mL) was heated to 150 °C in a microwave reactor for 1 h. The reaction mixture was cooled to rt and then poured into a stirring solution of K₂CO₃ (0.5 M in H₂O). The precipitate was collected by filtration, washed with water, and dried under reduced pressure to afford 4-(4-bromothiazol-2-yl)-N-(1-methyl-1H-pyrazol-4-yl)pyrimidin-2-amine (111 mg, 70%) as a greenish yellow powder. ¹H NMR (500 MHz, DMSO-*d₆*) δ 9.81 (s, 1H), 8.62 (d, *J* = 4.9 Hz, 1H), 8.14 (s, 1H), 7.90 (s, 1H), 7.59 (s, 1H), 7.31 (d, *J* = 4.9 Hz, 1H), 3.84 (s, 3H). MS (ESI⁺): m/z = 336.9.

### Intermediate 12: (R)-3-hydroxy-1-methyl-3-(3-(4-(2-(methylsulfonyl)pyrimidin-4-yl)thiazol-2-yl)phenyl)pyrrolidin-2-one

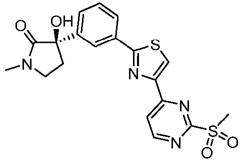

A mixture of potassium peroxymonosulfate (3.95 g, 6.43 mmol) in water (10 mL) was added to a mixture of (*R*)-3-hydroxy-1-methyl-3-(3-(4-(2-(methylthio)pyrimidin-4-yl)thiazol-2-yl)phenyl)pyrrolidin-2-one (Intermediate 8, 1.28 g, 3.21 mmol) in THF (10 mL) and acetone (10 mL). The resulting mixture was stirred at rt for 16 h and then EtOAc (30 mL) and water (25 mL) were added and the mixture was stirred for 30 min. The precipitate was collected by filtration and rinsed with ethyl acetate (30 mL) to afford (*R*)-3-hydroxy-1-methyl-3-(3-(4-(2-(methylsulfonyl)pyrimidin-4-yl)thiazol-2-yl)phenyl)pyrrolidin-2-one (1.10 g, 80% yield) as a white solid. ¹H NMR (400 MHz, DMSO-*d₆*) δ 9.18 (d, *J =* 5.1 Hz, 1H), 8.88 (s, 1H), 8.39 (d, *J* = 5.1 Hz, 1H), 8.14 (t, *J* = 1.6 Hz, 1H), 8.01 - 7.97 (m, 1H), 7.57 - 7.51 (m, 1H), 7.50 - 7.46 (m, 1H), 6.27 (s, 1H), 3.53 (s, 3H), 3.51 - 3.46 (m, 1H), 3.43 - 3.41 (m, 1H), 2.87 (s, 3H), 2.43 - 2.35 (m, 1H), 2.34 - 2.25 (m, 1H). MS (ESI⁺): m/z = 453.0.

### Intermediate 13: (R)-3-hydroxy-1-methyl-3-(3-(1-(2-(methylsulfonyl)pyrimidin-4-yl)-1H-pyrazol-3-yl)phenyl)pyrrolidin-2-one

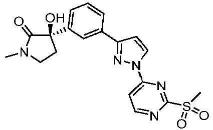

A mixture of Intermediate 21 (95.3 mg, 0.25 mmol), potassium peroxymonosulfate (460.764 mg, 0.749 mmol), acetone (0.524 mL, 0.79 g/mL, 7.124 mmol), water, distilled (0.524 mL, 29.071 mmol) and MeOH (0.524 mL, 0.791 g/mL, 12.929 mmol) was stirred at room temperature overnight. LCMS revealed full conversion of the starting material to the desired product. The reaction mixture was diluted with 50mL of water and stirred for 5 minutes. The precipitate was collected by vacuum filtration and dried to yield (R)-3-hydroxy-1-methyl-3-(3-(1-(2-(methylsulfonyl)pyrimidin-4-yl)-1H-pyrazol-3-yl)phenyl)pyrrolidin-2-one as a white solid (38.8 mg, 38%). ¹H NMR (500 MHz, DMSO-*d₆*) δ 9.16 - 9.09 (m, 1H), 8.95 - 8.88 (m, 1H), 8.27 - 8.20 (m, 1H), 8.09 - 8.03 (m, 1H), 7.95 - 7.89 (m, 1H), 7.52 - 7.46 (m, 1H), 7.45 - 7.39 (m, 1H), 7.33 = 7.27 (m, 1H), 6.15 - 6.10 (m, 1H), 3.54 (s, 3H), 3.51 - 3.45 (m, 1H), 3.45 - 3.37 (m, 2H), 2.34 - 2.24 (m, 2H). MS (ESI⁺): m/z = 414.2.

### Intermediate 14: 4-(4-bromothiazol-2-yl)-N-(1-methyl-1H-pyrazol-5-yl)pyrimidin-2-amine

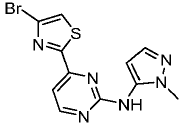

A mixture of 4-bromo-2-(2-(methylsulfonyl)pyrimidin-4-yl)thiazole (Intermediate 10, 500 mg, 1.562 mmol), 1-methyl-1h-pyrazol-5-amine (170.2 mg, 1.718 mmol), sodium bis(trimethylsilyl)amide (3.123 mL, 1 M, 3.123 mmol), and THF (10 mL) was heated at 50 °C under nitrogen for 1 hour. The mixture was cooled to rt, diluted with aqueous ammonium chloride solution (100 mL), and extracted with ethyl acetate (3 x 100 mL). The organic layer was dried with MgSO₄, filtered and concentrated to yield a red solid, 4-(4-bromothiazol-2-yl)-N-(1-methyl-1H-pyrazol-5-yl)pyrimidin-2-amine (350.5 mg, 67%). ¹H NMR (500 MHz, DMSO-*d₆*) δ 9.76 (s, 1H), 8.65 (d, *J* = 5.0 Hz, 1H), 8.10 (d, *J* = 2.4 Hz, 1H), 7.45 (d, *J* = 5.0 Hz, 1H), 7.37 (d, *J* = 1.9 Hz, 1H), 6.29 (d, *J* = 2.0 Hz, 1H), 3.70 (s, 3H). MS (ESI⁺): m/z = 337.

### Intermediate 15: 4-(3-bromo-1H-pyrazol-1-yl)-N-(3-cyclopropoxy-1-(2,2-difluoroethyl)-1H-pyrazol-4-yl)pyrimidin-2-amine

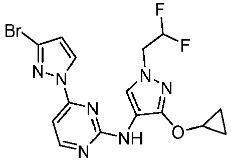

To a 2-dram vial, 4-(3-bromo-1H-pyrazol-1-yl)-2-chloropyrimidine (Intermediate 18, 100 mg, 0.385 mmol), pyrazolo[1,5-a]pyridin-3-amine (66.707 mg, 0.501 mmol), TFA (0.0737 mL, 1.49 g/mL, 0.963 mmol), DMSO (1.2 mL, 1.092 g/mL, 16.771 mmol) were added under nitrogen to react in a microwave reactor at 150 °C for 1 hour. The mixture was diluted with 100 mL of 1 M aqueous K₂CO₃ and extracted with 3 x 100 mL EtOAc. The organic extracts were dried with MgSO₄, filtered, and concentrated. The product was then purified by FCC (12 g silica, 0-100% ethyl acetate in hexanes) to yield 4-(3-bromo-1H-pyrazol-1-yl)-N-(3-cyclopropoxy-1-(2,2-difluoroethyl)-1H-pyrazol-4-yl)pyrimidin-2-amine (36.5 mg, 22%). ¹H NMR (400 MHz, DMSO-*d₆*) δ 8.83 (s, 1H), 8.48 (d, *J* = 2.4 Hz, 1H), 8.47 (s, 1H), 7.90 (s, 1H), 7.09 (d, *J* = 5.4 Hz, 1H), 6.81 (d, *J* = 2.7 Hz, 1H), 6.47 - 6.15 (m, 2H), 4.56 - 4.42 (m, 2H), 4.13 - 4.05 (m, 1H), 0.67 (q, *J* = 2.8 Hz, 2H), 0.66 - 0.61 (m, 2H). MS (ESI⁺): m/z = 427.

### Intermediate 16: N-(4-(3-bromo-1H-pyrazol-1-yl)pyrimidin-2-yl)pyrazolo[1,5-a]pyridin-3-amine

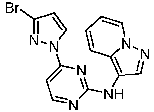

To a 2 dram vial, 4-(3-bromo-1H-pyrazol-1-yl)-2-chloropyrimidine (Intermediate 18, 100 mg, 0.385 mmol), pyrazolo[1,5-a]pyridin-3-amine (113.396 mg, 0.852 mmol), TFA (0.0737 mL, 1.49 g/mL, 0.963 mmol), and DMSO (0.827 mL, 1.092 g/mL, 11.561 mmol) were added and stirred under nitrogen at 150 °C for 1 hour. The mixture was diluted with 50 mL 1M K₂CO₃ and extracted with 3x 50 mL EtOAc. The organic extracts were dried with MgSO₄, filtered, and concentrated. The product was then purified by FCC (0-100% EtOAc in hexanes) to yield N-(4-(3-bromo-1H-pyrazol-1-yl)pyrimidin-2-yl)pyrazolo[1,5-a]pyridin-3-amine (55.1 mg, 40%). ¹H NMR (400 MHz, DMSO-*d₆*) δ 8.83 (s, 1H), 8.48 (d, *J* = 2.4 Hz, 1H), 8.47 (s, 1H), 7.90 (s, 1H), 7.09 (d, *J* = 5.4 Hz, 1H), 6.81 (d, *J* = 2.7 Hz, 1H), 6.47 - 6.15 (m, 2H), 4.56 - 4.42 (m, 2H), 4.13 - 4.05 (m, 1H), 0.67 (q, *J* = 2.8 Hz, 2H), 0.66 - 0.61 (m, 2H). MS (ESI⁺): m/z = 357.

### Intermediate 17: 4-(3-bromo-1H-pyrazol-1-yl)-N-(1-methyl-1H-pyrazol-3-yl)pyrimidin-2-amine

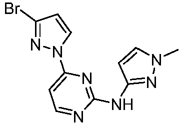

3-Bromopyrazole (40 mg, 0.272 mmol), 4-chloro-N-(1-methylpyrazol-3-yl)pyrimidin-2-amine (68.465 mg, 0.327 mmol), aqueous potassium carbonate (0.544 mL, 1 M, 0.544 mmol), and DMF (1.406 mL, 0.944 g/mL, 18.156 mmol) were added to a vial and heated at 60 °C for 1 hour. The temperature was raised to 100 °C and stirred for another hour.. After cooling to room temperature, the reaction mixture was diluted in 5 mL ethyl acetate and extracted with water (3 x 5 mL). The organic layer was dried with MgSO₄, filtered and concentrated. The residue was purified by silica gel chromatography (0-100% hexanes:10%MeOH in ethyl acetate) to yield 4-(3-bromo-1H-pyrazol-1-yl)-N-(1-methyl-1H-pyrazol-3-yl)pyrimidin-2-amine (44 mg, 50%). MS (ESI⁺): m/z = 321.

### Intermediate 18: 4-(3-bromo-1H-pyrazol-1-yl)-2-chloropyrimidine

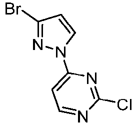

To a 100 mL round-bottom flask, 3-bromopyrazole (500 mg, 3.402 mmol), 2,4-dichloropyrimidine (1013 mg, 6.804 mmol), and DMF (12 mL, 155 mmol) were added under nitrogen followed by aqueous K₂CO₃ (8.505 mL, 1 M, 8.505 mmol). Then the reaction was heated at 60 °C for 1 hour. The reaction mixture was diluted in ethyl acetate (30 mL) and washed with water (3 x 30 mL). The organic layer was dried with MgSO₄, filtered, concentrated under reduced pressure and purified by FCC (silica, 0-60% hexanes:EtOAc) to yield 4-(3-bromo-1H-pyrazol-1-yl)-2-chloropyrimidine (531.6 mg, 60%) as white solid. ¹H NMR (600 MHz, DMSO-*d₆*) δ 8.84 (d, *J* = 5.6 Hz, 1H), 8.68 (d, *J* = 2.8 Hz, 1H), 7.89 (d, *J =* 5.5 Hz, 1H), 6.89 (d, *J* = 2.8 Hz, 1H). MS (ESI⁺): m/z = 260.

### Intermediate 19: N-(4-(3-bromo-1H-pyrazol-1-yl)pyrimidin-2-yl)pyrazolo[1,5-a]pyrimidin-3-amine

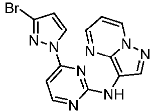

4-(3-Bromo-1H-pyrazol-1-yl)-2-chloropyrimidine (Intermediate 18, 100 mg, 0.385 mmol), 3-aminopyrazolo[1,5-a]pyrimidine (103.387 mg, 0.771 mmol), TFA (0.0737 mL, 1.49 g/mL, 0.963 mmol), and DMSO (1.2 mL, 1.092 g/mL, 16.771 mmol) were added to a vial under nitrogen and subjected to microwave irradiation at 150 °C for 1 hour. The mixture was diluted with 100 mL 1 M aqueous K₂CO₃ and extracted with 3 x 100 mL EtOAc. The organic extracts were dried with MgSO₄, filtered, and concentrated. The product was then purified by FCC (0-100% EtOAc in hexanes) to yield N-(4-(3-bromo-1H-pyrazol-1-yl)pyrimidin-2-yl)pyrazolo[1,5-a]pyrimidin-3-amine (26.7 mg, 19%). ¹H NMR (500 MHz, DMSO-*d₆*) δ 9.43 (s, 2H), 9.06 (d, *J* = 1.7 Hz, 1H), 9.04 (d, *J* = 1.7 Hz, 1H), 8.50 (d, *J =* 1.7 Hz, 2H), 8.49 (d, *J* = 1.7 Hz, 1H), 7.13 (s, 1H), 7.12 (s, 1H), 7.02 (d, *J* = 4.0 Hz, 1H), 7.01 (d, *J* = 3.9 Hz, 1H). MS (ESI⁺): m/z = 358.

### Intermediate 20: 4-(3-bromo-1H-pyrazol-1-yl)-N-(1-(2,2-difluoroethyl)-3-methoxy-1H-pyrazol-4-yl)pyrimidin-2-amine

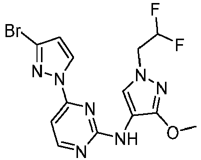

4-(3-Bromo-1H-pyrazol-1-yl)-2-chloropyrimidine (Intermediate 18, 100 mg, 0.385 mmol), 1-(2,2-difluoroethyl)-3-methoxy-1h-pyrazol-4-amine (136.539 mg, 0.771 mmol), TFA (0.0737 mL, 1.49 g/mL, 0.963 mmol), and DMSO (1.2 mL, 1.092 g/mL, 16.771 mmol) were combined under nitrogen and subjected to microwave irradiation at 150 °C for 1 hour. The mixture was diluted with 100 mL 1 M aqueous K₂CO₃ and extracted with EtOAc (3 x 100 mL). The organic extracts were dried with MgSO₄, filtered, and concentrated. The product was then purified by FCC (0-100% EtOAc in hexanes) to yield 4-(3-bromo-1H-pyrazol-1-yl)-N-(1-(2,2-difluoroethyl)-3-methoxy-1H-pyrazol-4-yl)pyrimidin-2-amine (49.4 mg, 32%). ¹H NMR (500 MHz, DMSO-*d₆*) δ 8.88 (s, 1H), 8.48 (s, 1H), 8.47 (s, 1H), 7.88 (s, 1H), 7.08 (d, *J* = 5.3 Hz, 1H), 6.81 (d, *J* = 2.7 Hz, 1H), 6.30 (tt, *J* = 55.2, 3.9 Hz, 2H), 4.51 - 4.41 (m, 2H), 3.83 (s, 3H). MS (ESI⁺): m/z = 401.

### Intermediate 21: (R)-3-Hydroxy-1-methyl-3-(3-(1-(2-(methylthio)pyrimidin-4-yl)-1H-pyrazol-3-yl)phenyl)pyrrolidin-2-one

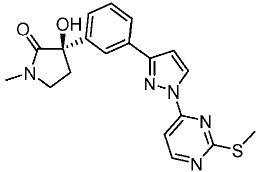

A mixture of (*R*)-3-(3-(1H-pyrazol-3-yl)phenyl)-3-hydroxy-1-methylpyrrolidin-2-one (Intermediate 3, 120 mg, 0.466 mmol), 4-chloro-2-(methylthio)pyrimidine (107.552 mg, 0.67 mmol), Cs₂CO₃ (759.803 mg, 2.332 mmol), and DMSO (0.747 mL, 1.092 g/mL, 10.435 mmol) were combined under an atmosphere of nitrogen and heated at 150 °C in a microwave reactor for 1 hour. LCMS revealed conversion to the desired product. After cooling to room temperature, the reaction mixture was diluted with EtOAc (30 mL) and washed with water (3 x 50 mL). The extracted organic layer was dried over MgSO₄, filtered, and concentrated before purification via FCC (12 g silica, 0-100% EtOAc in hexanes) to yield (R)-3-hydroxy-1-methyl-3-(3-(4-(2-(methylsulfonyl)pyrimidin-4-yl)thiazol-2-yl)phenyl)pyrrolidin-2-one (95.3 mg, 54%) as a white solid. ¹H NMR (500 MHz, DMSO-*d₆*) δ 8.79 (d, *J* = 2.8 Hz, 1H), 8.73 (d, *J* = 5.5 Hz, 1H), 8.02 (t, *J* = 1.8 Hz, 1H), 7.88 (dt, *J* = 7.7, 1.4 Hz, 1H), 7.69 (d, *J* = 5.5 Hz, 1H), 7.46 (t, *J* = 7.7 Hz, 1H), 7.40 - 7.37 (m, 1H), 7.18 (d, *J* = 2.8 Hz, 1H), 6.11 (s, 1H), 3.50 - 3.45 (m, 1H), 3.41 - 3.37 (m, 1H), 2.87 (s, 3H), 2.62 (s, 4H), 2.42 - 2.36 (m, 2H), 2.31 - 2.25 (m, 1H). MS (ESI⁺): m/z = 382.2.

### Intermediate 22: (R)-3-(3-(4-(2-((2,4-Dimethoxybenzyl)amino)pyrimidin-4-yl)thiazol-2-yl)phenyl)-3-hydroxy-1-methylpyrrolidin-2-one

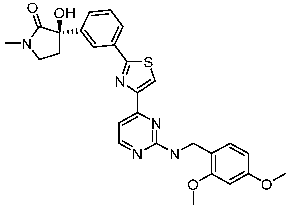

(*R*)-3-Hydroxy-1-methyl-3-(3-(4-(2-(methylsulfonyl)pyrimidin-4-yl)thiazol-2-yl)phenyl)pyrrolidin-2-one (Intermediate 12, 200 mg, 0.465 mmol), (2,4-dimethoxyphenyl)methanamine (0.42 mL, 2.3 mmol), DIPEA (1.44 mL, 8.36 mmol) and DMA (2.4 mL) were added to a 5 mL flask, and the resulting mixture heated for 16 h at 150 °C. The reaction vessel was removed from the oil bath and allowed to gradually cool to r.t. The reaction mixture was concentrated to dryness *in vacuo* to give a yellow solid. The yellow solid was purified by silica gel chromatography (0-100% ethyl acetate/petroleum ether) to give (*R*)-3-(3-(4-(2-((2,4-dimethoxybenzyl)amino)pyrimidin-4-yl)thiazol-2-yl)phenyl)-3-hydroxy-1-methylpyrrolidin-2-one (400 mg, 95%) as a colorless oil. MS (ESI⁺): m/z = 518.1.

### Intermediate 23: N-(2-Methoxypyridin-3-yl)-4-(tributylstannyl)pyrimidin-2-amine

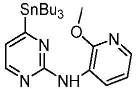

Step A. 4-Chloro-*N*-(2-methoxypyridin-3-yl)pyrimidin-2-amine. 2-Methoxypyridin-3-amine (440 mg, 3.54 mmol), and THF (10 mL) were added to an oven-dried and nitrogen-purged 50 mL three-necked round-bottomed flask, which was subsequently cooled to -72 °C, and the resulting mixture treated with LDA (2.0 mL, 2 M in THF, 4.0 mmol), portion-wise over 3 min. After being stirred for 30 min at -78 °C, the resulting mixture was treated with 4-chloro-2-(methylsulfonyl)pyrimidine (400 mg, 2.08 mmol) in THF (5 mL). The mixture was stirred for 1 h at room temperature. After this time, the mixture was poured into water (15 mL) and extracted with ethyl acetate (20 mL x 3). The combined organic extracts were washed with brine (30 mL), dried with anhydrous Na₂SO₄, filtered, and concentrated to dryness *in vacuo* to give a yellow viscous oil. The oil was purified by silica gel chromatography (0-100% EtOAc/pet ether) to give 4-chloro-*N*-(2-methoxypyridin-3-yl)pyrimidin-2-amine as a white solid (490 mg, 72%). MS (ESI⁺): m/z =237.0.

Step B. *N*-(2-Methoxypyridin-3-yl)-4-(tributylstannyl)pyrimidin-2-amine. 4-Chloro-*N*-(2-methoxypyridin-3-yl)pyrimidin-2-amine (490 mg, 2.07 mmol), a stir bar, 1,1,1,2,2,2-hexabutyldistannane (2.26 g, 3.90 mmol), LiCl (526 mg, 12.4 mmol) and 1,4-dioxane (30 mL) were added to a 100 mL round-bottomed flask, which was subsequently evacuated and refilled with argon (x 3), then treated with Pd₂(dba)₃ (97 mg, 0.11 mmol) and tricyclohexylphosphine (62 mg, 0.22 mmol), and the resulting mixture stirred for 16 h at 120 °C. The reaction mixture was then cooled to room temperature, quenched with sat. aqueous KF (40 mL), extracted with ethyl acetate (50 mL x 3), and the combined extracts washed with brine (300 mL), dried over anhydrous Na₂SO₄, filtered, and concentrated to dryness *in vacuo* to give the product (2.5 g) as a brown oil. MS (ESI⁺): m/z = 493.0.

### Intermediate 24: 6-Methyl-5-((4-(tributylstannyl)pyrimidin-2-yl)amino)nicotinonitrile

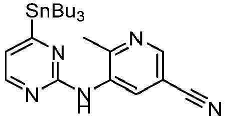

Step A. 5-((4-Chloropyrimidin-2-yl)amino)-6-methylnicotinonitrile. 5-Amino-6-methylnicotinonitrile (249 mg, 1.87 mmol) and THF (1 mL) were added to an oven-dried and nitrogen-purged 10 mL three-neck flask, which was subsequently cooled to -72 °C and charged with LDA (1.04 mL, 2.08 mmol, 2 M in hexanes and THF), dropwise over 5 min. The resulting mixture stirred for 30 min at -72 °C. 4-Chloro-2-(methylsulfonyl)pyrimidine (200 mg, 1.04 mmol) was added dropwise to the mixture at - 72 °C. The resulting mixture was stirred for 1 h with gradual warming to room temperature, and then diluted with H₂O (5 mL), and extracted with EtOAc (5 mL x 3). The combined organic extracts was washed with brine (5 mL), dried over anhydrous Na₂SO₄, filtered, and concentrated to dryness *in vacuo* to give a brown solid, which was purified by silica gel chromatography (0-30% EtOAc/pet ether) to give 5-((4-chloropyrimidin-2-yl)amino)-6-methylnicotinonitrile as a yellow solid (240 mg, 93%). MS (ESI⁺): m/z =246.1 [M+H⁺].

Step B. 6-Methyl-5-((4-(tributylstannyl)pyrimidin-2-yl)amino)nicotinonitrile. 5-((4-Chloropyrimidin-2-yl)amino)-6-methylnicotinonitrile (Intermediate 24, 240 mg, 0.977 mmol), a stir bar, LiCl (248 mg, 5.86 mmol) and 1,4-dioxane (10 mL) were added to a 50 mL round-bottomed flask fitted with a reflux condenser and charged with 1,1,1,2,2,2-hexabutyldistannane (1.03 g, 1.78 mmol). The resulting mixture was sparged with Ar for 5 min, and then treated with Pd₂(dba)₃ (45 mg, 0.049 mmol) and tricyclohexylphosphine (27 mg, 0.098 mmol), and stirred while heating at 120 °C for 16 hours. The reaction vessel was removed from the oil bath and allowed to gradually cool to r.t. The mixture was then quenched with sat. aqueous KF (30 mL), extracted with EtOAc (20 mL x 3), and the combined extracts washed with brine (20 mL), dried over anhydrous Na₂SO₄, filtered, and concentrated to dryness *in vacuo* to give a brown solid which was purified by silica gel chromatography (0-30% EtOAc/pet ether) to give 6-methyl-5-((4-(tributylstannyl)pyrimidin-2-yl)amino)nicotinonitrile as a white solid (76 mg, 15%). MS (ESI⁺): m/z = 500.7.

### Intermediate 25: 3-Cyclopropoxy-1-(2,2-difluoroethyl)-1H-pyrazol-4-amine

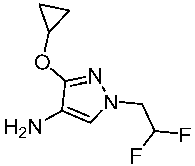

Step A. 1,4-Dinitro-1*H*-pyrazole. KNO₃ (0.89 g, 8.84 mmol), 4-nitro-1*H*-pyrazole (1.00 g, 8.84 mmol), and anhydrous CH₂Cl₂ (5 mL) were added to a 50 mL single-neck round-bottomed flask at 15 °C, and then 2,2,2-trifluoroacetic anhydride (2.46 mL, 17.7 mmol) in anhydrous CH₂Cl₂ (5 mL) was added. The light-yellow mixture was stirred at room temperature for 5 h under N₂, and then poured into ice water, and extracted with ethyl acetate (20 mL x 3). The combined organic extracts were washed with brine (50 mL), dried over anhydrous Na₂SO₄, filtered, and concentrated to dryness *in vacuo* to give a light-yellow oil, which was subjected to silica gel chromatography (0-20% EtOAc/pet ether) to afford 1,4-dinitro-1H-pyrazole as a yellow oil (1.2 g, 86%). ¹H NMR (400 MHz, CDCl₃) δ 8.97 (d, *J* = 0.8 Hz, 1H), 8.15 - 8.09 (m, 1H).

Step B. 3-Cyclopropoxy-4-nitro-1*H*-pyrazole. NaH (1.35 g, 33.7 mmol, 60 wt% in mineral oil) was added in portions to a 100 mL three-necked round-bottomed flask containing cyclopropanol (1.96 g, 33.7 mmol), and 2-Me-THF (15 mL) at 0 °C. The mixture was stirred for 10 min with gradual warming to room temperature. The above-described suspension was added dropwise to a 100 mL three-necked round-bottomed flask containing 1,4-dinitro-1*H*-pyrazole (Intermediate 25, 8.00 g, 50.6 mmol) and 2-Me-THF (15 mL) at -78 °C. The grey mixture was stirred at -78 °C for 1 h and then allowed to warm to room temperature and stir for 5 h. After this time, the mixture was poured into water, treated with aq. HCl (10 mL, 3 N), and extracted with CH₂Cl₂ (20 mL x 3). The combined organic extracts were washed with brine (50 mL), dried over anhydrous Na₂SO₄, filtered, and concentrated to dryness *in vacuo* to give a light-yellow oil, which was subjected to silica gel chromatography (0-30% EtOAC/pet ether) to afford 3-cyclopropoxy-4-nitro-1*H*-pyrazole as a yellow oil (1.1 g, 19%). ¹H NMR (400 MHz, CDCl₃) δ 8.30 - 8.17 (m, 1H), 6.47 (s, 1H), 4.27 - 4.23 (m, 1H), 1.03 - 0.94 (m, 2H), 0.88 - 0.79 (m, 2H).

Step C. 3-Cyclopropoxy-1-(2,2-difluoroethyl)-4-nitro-1*H*-pyrazole. 2-(Tributylphosphoranylidene)acetonitrile (3.00 g, 12.4 mmol) was added to a 100 mL three-necked round-bottomed flask containing 3-cyclopropoxy-4-nitro-1*H*-pyrazole (700 mg, 4.14 mmol), 2,2-difluoroethanol (1.02 g, 12.4 mmol), and anhydrous toluene (30 mL) at room temperature under N₂. The mixture was heated at 60 °C for 12 h. The reaction vessel was removed from the oil bath and allowed to gradually cool to room temperature. The mixture was then concentrated to dryness *in vacuo* to give a red oil, which was subjected to silica gel chromatography (20-50% EtOAc/pet ether) to give 3-cyclopropoxy-1-(2,2-difluoroethyl)-4-nitro-1H-pyrazole (500 mg, 52%) as a red solid. ¹H NMR (400 MHz, CDCl₃) δ 8.10 (s, 1H), 6.34 - 5.94 (m, 1H), 4.38 - 4.30 (m, 2H), 4.22 - 4.19 (m, 1H), 0.96 - 0.90 (m, 2H), 0.84 - 0.78 (m, 2H).

Step D. 3-Cyclopropoxy-1-(2,2-difluoroethyl)-1*H*-pyrazol-4-amine. Iron powder (838 mg, 15.0 mmol) was added to a 50 mL three-necked round-bottomed flask containing 3-cyclopropoxy-1-(2,2-difluoroethyl)-4-nitro-1*H*-pyrazole (700 mg, 3.00 mmol, isomer mixture), NH₄Cl (642 mg, 12.0 mmol), anhydrous EtOH (10 mL) and H₂O (2 mL) at room temperature. The mixture was heated at 70 °C for 12 h. The reaction vessel was removed from the oil bath and allowed to gradually cool to room temperature. The mixture was filtered through a pad of diatomaceous earth and the pad washed with MeOH (30 mL x 3). The filtrate was concentrated to dryness *in vacuo* to give a black oil, which was subjected to silica gel chromatography (0-50% EtOAc/pet ether) to give 3-cyclopropoxy-1-(2,2-difluoroethyl)-1H-pyrazol-4-amine (300 mg, 41%) as a black oil. MS (ESI⁺): m/z = 203.6.

### Intermediate 26: 3-Amino-N,4-dimethylbenzamide

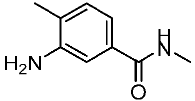

Step A: 4-Dimethyl-3-nitrobenzamide. DIPEA (2.74 mL, 16.6 mmol), 4-methyl-3-nitrobenzoic acid (1.00 g, 5.52 mmol), methanamine hydrochloride (0.69 g, 6.63 mmol), HATU (3.15 g, 8.28 mmol) and anhydrous DMF (20 mL) were added to a 100 mL flask. The resultant mixture was stirred at room temperature for 12 h under N₂. After this time, the mixture was diluted with H₂O (50 mL) and extracted with CH₂Cl₂ (50 mL x 3). The combined organic extracts were washed with brine (50 mL x 2), dried over anhydrous Na₂SO₄, filtered, and concentrated *in vacuo.* The residue was subjected to silica gel chromatography (0-100% EtOAc/pet ether) to give *N*,4-dimethyl-3-nitrobenzamide as a white solid (1 g, 92%). MS (ESI⁺): m/z = 194.6 [M+H]⁺. ¹H NMR (400 MHz, CDCl₃) δ 8.35 (d, *J* = 1.6 Hz, 1H), 7.97 - 7.94 (m, 1H), 7.44 (d, *J* = 7.6 Hz, 1H), 6.26 (s, 1H), 3.05 (d, *J* = 4.8 Hz, 3H), 2.66 (s, 3H).

Step B: 3-Amino-*N*,4-dimethylbenzamide. A mixture of *N*, 4-dimethyl-3-nitrobenzamide (1.70 g, 8.75 mmol), wet Pd/C (500 mg, 10 wt.%, 0.46 mmol) and EtOH (30 mL) was stirred at room temperature under H₂ (50 psi) for 12 h. After this time, the mixture was filtered through a pad of diatomaceous earth and the pad washed with EtOAc (20 mL x 3). The filtrate was concentrated to dryness *in vacuo* to give a liquid, which was subjected to silica gel chromatography (0-50% EtOAc/pet ether) to obtain 3-amino-*N*,4-dimethylbenzamide as a white solid (790 mg, 55%). MS (ESI⁺): m/z = 164.7. ¹H NMR (400 MHz, CDCl₃) δ 7.15 (d, *J =* 1.2 Hz, 1H), 7.08 (d, *J* = 7.6 Hz, 1H), 7.00 - 6.98 (m, 1H), 6.07 (s, 1H), 3.73 (s, 2H), 3.00 (d, *J* = 4.8 Hz, 3H), 2.20 (s, 3H).

### Intermediate 27: 1-(2,2-Difluoroethyl)-3-(2-methoxyethoxy)-1H-pyrazol-4-amine

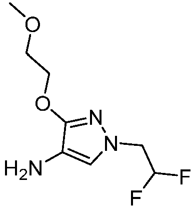

Step A. 1-(3-(2-Methoxyethoxy)-1*H*-pyrazol-1-yl)ethanone. 1-Acetyl-1*H*-pyrazol-3(2*H*)-one (5.5 g, 43.6 mmol), 1-bromo-2-methoxyethane (6.37 g, 45.79 mmol), K₂CO₃ (9.04 g, 65.42 mmol), and anhydrous DMF (45 mL) were added to an oven-dried and nitrogen-purged 100 mL three-necked round-bottomed flask, and the resulting mixture heated for 2 h at 80 °C. The reaction mixture was then diluted with EtOAc (200 mL), washed with brine (100 mL), dried over anhydrous Na₂SO₄, filtered, and concentrated to dryness *in vacuo* to give a brown solid, which was subjected to silica gel chromatography (0-50% EtOAc/pet ether) to give 1-(3-(2-methoxyethoxy)-1*H*-pyrazol-1-yl)ethanone as a colorless liquid (6.7 g, 83%).

Step B. 3-(2-Methoxyethoxy)-1*H*-pyrazole. 1-(3-(2-Methoxyethoxy)-1*H*-pyrazol-1-yl)ethanone (6.7 g, 36.4 mmol), NaOH (4.37 g, 109 mmol), MeOH (40 mL), and H₂O (12 mL) were added to a 100 mL round-bottomed flask fitted with a reflux condenser, and the resulting mixture was heated for 1 h at 50 °C. The resulting white suspension was concentrated to dryness *in vacuo* to give a white solid, which was acidified with aq. HCl (2 N, 40 mL) to pH = 3 - 4 and extracted with EtOAc (40 mL x 4). The combined extracts were washed with brine (30 mL), dried over anhydrous Na₂SO₄, filtered, and concentrated to dryness *in vacuo* to give a colorless oil, which was subjected to silica gel chromatography (0-50% EtOAc/pet ether) to give 3-(2-methoxyethoxy)-1*H*-pyrazole as a colorless liquid (5 g, 97%).

Step C. 3-(2-Methoxyethoxy)-4-nitro-1*H*-pyrazole. 3-(2-Methoxyethoxy)-1*H-*pyrazole (2 g, 14.1 mmol) and *conc.* H₂SO₄ (8 mL) were added to a 50 mL round-bottomed flask, and the resulting mixture was carefully treated with fuming HNO₃ (2.31 g, 36.6 mmol), dropwise over 10 min at room temperature and then heated for 12 h at 50 °C. The yellow solution was carefully added into ice-water (20 mL) dropwise and then extracted with EtOAc (30 mL x 3). The combined extracts were washed with brine (10 mL), dried over anhydrous Na₂SO₄, filtered, and the filtrate concentrated to dryness *in vacuo* to give a white solid. The white solid was subjected to silica gel chromatography (30-50% EtOAc/pet ether) to give 3-(2-methoxyethoxy)-4-nitro-1*H-*pyrazole as a white solid (1.3 g, 49%).

Step D. 1-(2,2-Difluoroethyl)-3-(2-methoxyethoxy)-4-nitro-1*H*-pyrazole. 2-(Tributylphosphoranylidene)acetonitrile (5.03 g, 20.8 mmol), 3-(2-methoxyethoxy)-4-nitro-1*H*-pyrazole (1.3 g, 6.95 mmol), 2,2-difluoroethanol (1.71 g, 20.8 mmol), and anhydrous toluene (25 mL) were added to an oven-dried and nitrogen-purged 100 mL three-necked round-bottomed flask fitted with a reflux condenser, and the resulting mixture was heated for 12 h at 60 °C. The reaction vessel was removed from the oil bath and allowed to gradually cool to room temperature. The reaction mixture was concentrated to dryness *in vacuo* to give a red oil, which was subjected to silica gel chromatography (20-50% EtOAc/pet ether) to give 1-(2,2-difluoroethyl)-3-(2-methoxyethoxy)-4-nitro-1*H*-pyrazole as a red oil (1.1 g, 63%).

Step E. 1-(2,2-Difluoroethyl)-3-(2-methoxyethoxy)-1*H*-pyrazol-4-amine. Fe powder (1.11 g, 19.9 mmol), 1-(2,2-difluoroethyl)-3-(2-methoxyethoxy)-4-nitro-1*H-*pyrazole (1 g, 3.98 mmol), NH₄Cl (852 mg, 15.9 mmol), anhydrous EtOH (50 mL) and H₂O (10 mL) were added to an oven-dried and nitrogen-purged 100 mL three-necked round-bottomed flask fitted with a reflux condenser, and the resulting mixture was heated for 12 h at 70 °C. The reaction vessel was removed from the oil bath and allowed to gradually cool to r.t. The black suspension was filtered through a pad of diatomaceous earth and the pad washed with MeOH (50 mL x 3). The filtrate was concentrated to dryness *in vacuo* to give a black oil, which was partitioned between EtOAc (60 mL) and brine (20 mL). The aqueous phase was subsequently extracted with EtOAc (20 mL x 2), and the combined organic extracts were washed with brine (20 mL), dried over anhydrous Na₂SO₄, filtered, and the filtrate concentrated to dryness *in vacuo* to give a brown solid. The brown solid was subjected to silica gel chromatography (30-50% EtOAc/pet ether) to give 1-(2,2-difluoroethyl)-3-(2-methoxyethoxy)-1H-pyrazol-4-amine as a brown oil (490 mg, 56%).¹H NMR (400 MHz, CDCl₃) δ 7.00 - 6.85 (m, 1H), 6.13 - 5.79 (m, 1H), 4.34 - 4.32 (m, 2H),4.15 - 4.14 (m, 2H), 3.77 - 3.69 (m, 2H), 3.44 (s, 3H).

### Intermediate 28: 2,4-Difluoro-N-methyl-5-nitrobenzamide

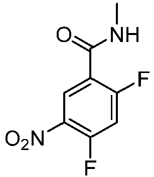

SOCl₂ (25 mL) was added to a 100 mL single-necked round bottomed flask containing 2,4-difluoro-5-nitrobenzoic acid (5.00 g, 24.6 mmol) under nitrogen. The resulting brown suspension was heated at reflux for 3 h. The reaction vessel was removed from the oil bath and allowed to gradually cool to rt. The mixture was concentrated to dryness in *vacuo* to give a yellow solid, which was dissolved in anhydrous CH₂Cl₂ (50 mL), cooled to -40 °C, and treated with CH₃NH₂•HCl (1.66 g, 24.6 mmol) and Et₃N (9.97 g, 98.5 mmol). The resultant white suspension was stirred at -40 °C for 1 h and allowed to warm to 0 °C and stirred for 10 min. The mixture was concentrated to dryness *in vacuo* to give a yellow solid. The yellow solid was partitioned between EtOAc (60 mL) and *sat.* aqueous NaHCO₃ (20 mL). The aqueous phase was subsequently extracted with EtOAc (30 mL x 2), and the combined organic extracts washed with brine (20 mL), dried over anhydrous Na₂SO₄, filtered, and the filtrate concentrated to dryness *in vacuo* to give a yellow solid. The yellow solid was subjected to silica gel chromatography (20-50% EtOAc/pet ether) to give 2,4-difluoro-*N*-methyl-5-nitrobenzamide as a yellow solid (3 g, 56%).

### Intermediate 29 and Intermediate 30: 4-Cyclopropoxy-2-fluoro-N-methyl-5-nitrobenzamide & 2-cyclopropoxy-4-fluoro-N-methyl-5-nitrobenzamide

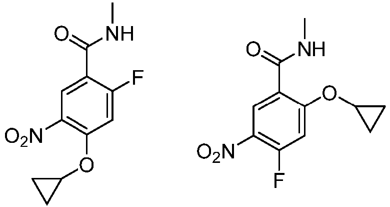

Cs₂CO₃ (8.14 g, 25.0 mmol) was added to a 100 mL three-necked round-bottomed flask containing 2,4-difluoro-*N*-methyl-5-nitrobenzamide (Intermediate 28, 2.70 g, 12.5 mmol), cyclopropanol (726 mg, 12.49 mmol) a stir bar, and anhydrous 1,4-dioxane (55 mL) at room temperature. The resultant yellow suspension was stirred for 1 h at 80 °C. The reaction vessel was removed from the oil bath and allowed to gradually cool to rt. The reaction mixture was concentrated to dryness *in vacuo* to obtain a yellow solid. The yellow solid was partitioned between EtOAc (100 mL) and H₂O (40 mL). The aqueous phase was subsequently extracted with EtOAc (30 mL x 2), and the combined organic extracts were washed with brine (30 mL), dried over anhydrous Na₂SO₄, filtered, and the filtrate concentrated to dryness *in vacuo* to give a yellow solid. The yellow solid was subjected to silica gel chromatography (20-50% EtOAc/pet ether) to give a mixture of 4-cyclopropoxy-2-fluoro-*N*-methyl-5-nitrobenzamide and 2-cyclopropoxy-4-fluoro-*N-*methyl-5-nitrobenzamide as a yellow solid (2200 mg, 69%).

### Intermediate 31 and Intermediate 32: 5-Amino-4-cyclopropoxy-2-fluoro-N-methylbenzamide & 5-amino-2-cyclopropoxy-4-fluoro-N-methylbenzamide

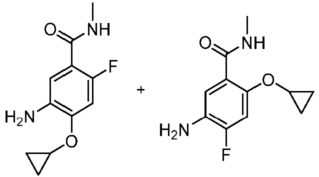

Fe powder (2.42 g, 43.3 mmol) was added to a 100 mL three-necked round-bottomed flask containing 4-cyclopropoxy-2-fluoro-*N*-methyl-5-nitrobenzamide (2.20 g, 8.65 mmol, isomer mixture, Intermediate 29 & Intermediate 30), NH₄Cl (1.85 g, 34.6 mmol), a stir bar, anhydrous EtOH (50 mL) and H₂O (10 mL) at room temperature. The resulting black suspension was heated at 70 °C for 1 h. The reaction vessel was removed from the oil bath and allowed to gradually cool to rt. The reaction mixture was filtered through a pad of diatomaceous earth and the pad washed with MeOH (50 mL x 3). The filtrate was concentrated to dryness *in vacuo* to give a black oil, which was partitioned between EtOAc (60 mL) and brine (20 mL). The aqueous phase was subsequently extracted with EtOAc (20 mL x 2), and the combined organic extracts were washed with brine (20 mL), dried over anhydrous Na₂SO₄, filtered, and the filtrate concentrated to dryness *in vacuo* to give a brown solid. The brown solid was subjected to silica gel chromatography (25-50% EtOAc/pet ether) to give a mixture of 5-amino-4-cyclopropoxy-2-fluoro-*N-*methylbenzamide and 5-amino-2-cyclopropoxy-4-fluoro-*N*-methylbenzamide as a yellow solid (1600 mg, 82%). MS (ESI⁺): m/z = 224.9.

### Intermediate 33: 5-Amino-4-cyclopropoxy-2-fluoro-N-methylbenzamide

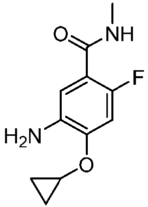

5-Amino-4-cyclopropoxy-2-fluoro-*N*-methylbenzamide (1.6 g, 7.14 mmol, mixture of isomers, Intermediate 31 and Intermediate 32) was purified by HPLC (Boston Uni C18 column, 5 µm, 40 x 150 mm, 8-38% (v/v) CH₃CN/H₂O with 0.225% HCOOH) to afford, after lyophilization, 5-amino-4-cyclopropoxy-2-fluoro-N-methylbenzamide as a yellow solid (390 mg, 24%). ¹H NMR (400 MHz, CDCl₃) δ 7.41 (d, *J* = 8.0 Hz, 1H), 6.92 - 6.89 (m, 1H), 6.79 - 6.59 (m, 1H), 3.83 - 3.73 (m, 1H), 3.67 - 3.07 (m, 2H), 3.01 (d, *J* = 4.8 Hz, 3H), 0.89 - 0.80 (m, 4H).

### Intermediate 34: 5-Amino-1-isobutyl-N-methyl-1H-pyrazole-3-carboxamide

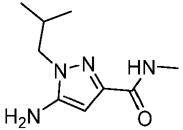

Step A. Ethyl 5-amino-1-isobutyl-1*H*-pyrazole-3-carboxylate. Conc. HCl (7.01 mL, 83.90 mmol, 1.18 g/ mL, 37 wt.%) was added to a 250 mL three-necked round-bottomed flask containing ethyl 3-cyano-2-oxopropanoate (4 g, 28.3 mmol), isobutylhydrazine hydrochloride (3.53 g, 28.3 mmol), and anhydrous EtOH (150 mL) at room temperature. The resultant brown suspension was heated for 12 h at 80 °C. The reaction vessel was removed from the oil bath and allowed to gradually cool to room temperature. The mixture was concentrated to dryness *in vacuo* to give a yellow solid. The yellow solid was cooled ice-water and then the pH was adjusted to pH = 9 - 10 with sat. aqueous NaHCO₃ (30 mL). The resulting brown suspension was extracted with EtOAc (60 mL x 3). The combined organic extracts were washed with brine (30 mL), dried over anhydrous Na₂SO₄, filtered, and concentrated *in vacuo* to give a brown solid. The brown solid was subjected to silica gel chromatography (50-100% EtOAc/pet ether) to give ethyl 5-amino-1-isobutyl-1*H*-pyrazole-3-carboxylate as a yellow solid (2.5 g, 42%). ¹H NMR (400 MHz, CDCl₃) δ 6.14 (s, 1H), 4.41 - 4.35 (m, 2H), 3.95 - 3.93 (m, 2H), 3.58 (s, 2H), 2.36 - 2.26 (m, 1H), 1.42 - 1.33 (m, 3H), 0.95 - 0.93 (m, 6H).

Step B. 5-Amino-1-isobutyl-1*H*-pyrazole-3-carboxylic acid. LiOH•H₂O (2.68 g, 63.9 mmol) was added to a 250 mL three-necked round-bottomed flask containing ethyl 5-amino-1-isobutyl-1*H*-pyrazole-3-carboxylate (2.70 g, 12.8 mmol), THF (75 mL) and H₂O (15 mL) at room temperature. The resultant yellow suspension was heated for 3 h at 70 °C. The reaction vessel was removed from the oil bath and allowed to gradually cool to room temperature. The reaction mixture was concentrated to dryness *in vacuo* to obtain a yellow solid. The yellow solid was treated with aq. HCl (3 N, 20 mL) and the resulting mixture was extracted with EtOAc (30 mL x 5). The combined organic extracts were washed with brine, dried over anhydrous Na₂SO₄, filtered, and concentrated in *vacuo* to give 5-amino-1-isobutyl-1*H*-pyrazole-3-carboxylic acid as a yellow solid (2.3 g, 94%). MS (ESI⁺): m/z = 183.9.

Step C. 5-Amino-1-isobutyl-*N*-methyl-1*H*-pyrazole-3-carboxamide. HATU (4.11 g, 10.8 mmol) was added to 100 mL one-necked round-bottomed flask containing 5-amino-1-isobutyl-1*H*-pyrazole-3-carboxylic acid (1.8 g, 9.83 mmol), CH₃NH₂•HCl (1.00 g, 14.7 mmol), DIPEA (3.81 g, 29.5 mmol) a stir bar, and anhydrous DMF (65 mL) at room temperature. The resultant brown solution was stirred for 12 h at rt. After this time, the mixture was diluted with H₂O (60 mL) and extracted with EtOAc (50 mL x 5). The combined organic extracts were washed with H₂O (30 mL x 2) and brine (30 mL), dried over anhydrous Na₂SO₄, filtered, and the filtrate concentrated to dryness *in vacuo* to give a yellow oil. The yellow oil was combined with another batch of this material (500 mg) and subjected to silica gel chromatography (50-100% EtOAc/pet ether) to give 5-amino-1-isobutyl-*N*-methyl-1*H*-pyrazole-3-carboxamide as a brown oil (1.8 g, 73%). MS (ESI⁺): m/z = 196.9.

### Intermediate 35: (R)-3-(3-(4-(2-Chloropyrimidin-4-yl)thiazol-2-yl)phenyl)-3-hydroxy-1-methylpyrrolidin-2-one

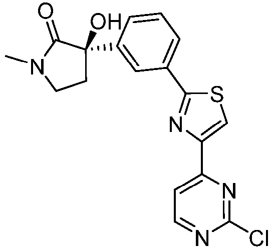

(*R*)-3-(3-(4-Bromothiazol-2-yl)phenyl)-3-hydroxy-1-methylpyrrolidin-2-one (Intermediate 7, 600 mg, 1.70 mmol), 2-chloro-4-(tributylstannyl)pyrimidine (754 mg, 1.868 mmol), Et₃N (0.47 mL, 3.40 mmol) and anhydrous toluene (15 mL) were added to an oven-dried and nitrogen-purged 50 mL round-bottomed flask fitted with a reflux condenser, and treated with Pd(PPh₃)₄ (294 mg, 0.255 mmol). The mixture was sparged with N₂ for 5 min, and heated for 12 h at 120 °C to give a brown suspension. The reaction vessel was removed from the oil bath and allowed to gradually cool to room temperature. The suspension was concentrated to dryness *in vacuo* to give a brown solid. The brown solid was subjected to silica gel chromatography (0-100% EtOAc/pet ether) to give (R)-3-(3-(4-(2-chloropyrimidin-4-yl)thiazol-2-yl)phenyl)-3-hydroxy-1-methylpyrrolidin-2-one as a yellow solid (350 mg, 53%).

### Intermediate 36: 3-Amino-1,5-dimethylpyridin-2(1H)-one

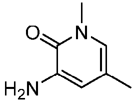

Step A. 1,5-Dimethyl-3-nitropyridin-2(1*H*)-one. CH₃I (1.38 g, 9.73 mmol) was added drop-wise over 5 minutes under N₂ to a 100 mL three-necked round-bottomed flask containing a 0 °C mixture of 5-methyl-3-nitropyridin-2(1H)-one (1 g, 6.49 mmol), K₂CO₃ (1.79 g, 12.98 mmol), and anhydrous DMF (10 mL). The resultant mixture was stirred for 12 h with gradual warming to room temperature. After this time, the mixture diluted with water (30 mL) and extracted with EtOAc (40 mL x 3). The combined organic extracts were washed with brine (10 mL), dried over anhydrous Na₂SO₄, filtered, and the filtrate concentrated to dryness *in vacuo* to give a yellow oil. The yellow oil was subjected to silica gel chromatography (50-100% EtOAc/pet ether) to give 1,5-dimethyl-3-nitropyridin-2(1*H*)-one as a yellow solid (690 mg, 63%). MS (ESI⁺): m/z = 168.8. ¹H NMR (400 MHz, CDCl₃) δ 8.20 (d, *J* = 2.4 Hz, 1H), 7.52 (d, *J* = 1.8 Hz,1H), 3.71 - 3.58 (m, 3H), 2.20 (s, 3H).

Step B. 3-Amino-1,5-dimethylpyridin-2(1*H*)-one. Pd/C (wet, 500 mg, 50% H₂O) was added to a 50 mL single-necked round-bottomed flask containing a mixture of 1,5-dimethyl-3-nitropyridin-2(1*H*)-one (Intermediate 36, 850 mg, 5.06 mmol), anhydrous MeOH (15 mL), and THF (15 mL) at rt. The resulting black suspension was stirred under H₂ (15 psi) for 12 h at rt. The suspension was then filtered through a pad of diatomaceous earth and the pad washed with MeOH (15 mL x 5). The filtrate, combined with an additional the batch of material, was concentrated to dryness *in vacuo* to give a brown solid. The brown solid was subjected to silica gel chromatography (2-5% MeOH/DCM) to give 3-amino-1,5-dimethylpyridin-2(1*H*)-one as a yellow solid (850 mg). MS (ESI⁺): m/z = 138.8. ¹H NMR (400 MHz, CDCl₃) δ 6.49 - 6.48 (m, 1H), 6.41- 6.40 (m, 1H), 4.77 - 3.56 (m, 2H), 3.53 (s, 3H), 2.01 (s, 3H).

### Intermediate 37: (R)-3-Hydroxy-1-methyl-3-(3-(4-(2-(methylthio)pyrimidin-4-yl)thiazol-2-yl)phenyl)pyrrolidin-2-one

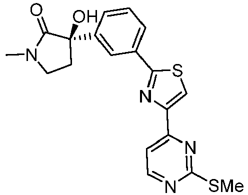

((*R*)-3-(3-(4-Bromothiazol-2-yl)phenyl)-3-hydroxy-1-methylpyrrolidin-2-one (Intermediate 7, 1.00 g, 2.83 mmol), Pd(PPh₃)₄ (351 mg, 0.283 mmol), 2-(methylthio)-4-(tributylstannyl)pyrimidine (1.29 g, 3.11 mmol), Et₃N (0.789 mL, 5.66 mmol), and anhydrous toluene (30 mL) were added to a 100 mL three-necked round-bottomed flask fitted with a reflux condenser. The resultant mixture was heated at 120 °C for 16 h under N₂. The reaction vessel was removed from the oil bath and allowed to gradually cool to room temperature. The reaction mixture was poured into aq. KF (20 mL), filtered through a pad of diatomaceous earth and the pad washed with EtOAc (50 mL). The filtrate was extracted with EtOAc (40 mL x 3). The combined organic extracts were washed with brine (50 mL), dried over anhydrous Na₂SO₄, filtered, and the filtrate concentrated to dryness *in vacuo* to give a brown oil. The brown oil was subjected to silica gel chromatography (20-100% EtOAc/pet ether) to afford (*R*)-3-hydroxy-1-methyl-3-(3-(4-(2-(methylthio)pyrimidin-4-yl)thiazol-2-yl)phenyl)pyrrolidin-2-one as a brown oil (810 mg, 66%). MS (ESI⁺): m/z = 399.0 [M+H]⁺.

### Intermediate 38: (R)-3-Hydroxy-1-methyl-3-(3-(4-(2-(methylsulfinyl)pyrimidin-4-yl)thiazol-2-yl)phenyl)pyrrolidin-2-one

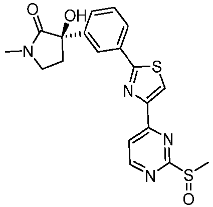

(*R*)-3-Hydroxy-1-methyl-3-(3-(4-(2-(methylthio)pyrimidin-4-yl)thiazol-2-yl)phenyl)pyrrolidin-2-one (Intermediate 37, 134 mg, 0.336 mmol), hydrogen peroxide (68.7 µL, 0.673 mmol), and hexafluoro-2-propanol (4 mL) were added to a 10 mL round-bottomed flask. The resultant mixture was stirred at rt for 16 h. After this time the mixture was quenched with saturated aq. Na₂O₃S₂ (5 mL) and extracted with EtOAc (20 mL x 3). The combined organic extracts were dried over anhydrous Na₂SO₄, filtered, and the filtrate concentrated to dryness *in vacuo* to give a brown oil. The brown oil was subjected to silica gel chromatography (20-100% EtOAc/pet ether) to afford (R)-3-hydroxy-1-methyl-3-(3-(4-(2-(methylsulfinyl)pyrimidin-4-yl)thiazol-2-yl)phenyl)pyrrolidin-2-one as a yellow solid (110 mg, 74%). MS (ESI⁺): m/z = 415.0.

### Intermediate 39: 5-Amino-1-cyclopentyl-N-methyl-1H-pyrazole-3-carboxamide

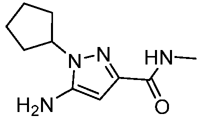

Step A. Ethyl 5-amino-1-cyclopentyl-1*H*-pyrazole-3-carboxylate. Conc. HCl (5.26 mL, 62.92 mmol, 1.18 g/ mL, 37 wt.%) was added to a 250 mL three-necked round-bottomed flask containing a mixture of ethyl 3-cyano-2-oxopropanoate (3 g, 21.26 mmol), cyclopentylhydrazine hydrochloride (2.90 g, 21.26 mmol), and anhydrous EtOH (120 mL) at room temperature. The resultant brown suspension was heated for 12 h at 80 °C. The reaction vessel was removed from the oil bath and allowed to gradually cool to room temperature. The reaction mixture was then concentrated to dryness *in vacuo* to give a yellow solid. The yellow solid was cooled to 0 °C and then the pH was adjusted to pH = 9 ~ 10 with sat. aqueous NaHCO₃ (30 mL). The resultant suspension was extracted with EtOAc (50 mL x 3). The combined organic extracts were washed with brine (30 mL), dried over anhydrous Na₂SO₄, filtered, and concentrated *in vacuo* to give a yellow solid. The yellow solid was subjected to silica gel chromatography (50% - 100% EtOAc/pet ether) to give ethyl 5-amino-1-cyclopentyl-1*H*-pyrazole-3-carboxylate as a yellow solid (1.5 g, 32%). ¹H NMR (400 MHz, CDCl₃) δ 6.09 (s, 1H), 4.56 - 4.48 (m, 1H), 4.36 (q, *J* = 7.2 Hz, 2H), 2.19 - 2.04 (m, 4H), 1.99 - 1.89 (m, 2H), 1.73 - 1.60 (m, 2H), 1.37 (t, *J* = 7.2 Hz, 3H).

Step B. 5-Amino-1-cyclopentyl-1*H*-pyrazole-3-carboxylic acid. LiOH•H₂O (1.28 g, 30.5 mmol) was added to a 100 mL three-necked round-bottomed flask containing a solution consisting of ethyl 5-amino-1-cyclopentyl-1H-pyrazole-3-carboxylate (1.70 g, 7.61 mmol), THF (50 mL) and H₂O (10 mL) at room temperature. The resultant yellow suspension was heated for 3 h at 70 °C. The reaction vessel was removed from the oil bath and allowed to gradually cool to room temperature. The solution was concentrated to dryness *in vacuo* to obtain a yellow solid. The yellow solid was treated with aq. HCl (3 N, 10 mL) and the resulting mixture was extracted with EtOAc (30 mL x 5). The combined organic extracts were washed with brine (10 mL), dried over anhydrous Na₂SO₄, filtered, and concentrated *in vacuo* to give 5-amino-1-cyclopentyl-1H-pyrazole-3-carboxylic acid as a yellow solid (1.2 g, 81%). MS (ESI⁺): m/z = 195.9. ¹H NMR (400 MHz, DMSO-*d₆*) δ 12.17 (br s, 1H), 5.66 (s, 1H), 5.32 (br s, 2H), 4.64 - 4.47 (m, 1H), 2.02 -1.93 (m, 2H), 1.88 - 1.74 (m, 4H), 1.64 - 1.52 (m, 2H).

Step C. 5-Amino-1-cyclopentyl-*N*-methyl-1*H*-pyrazole-3-carboxamide. HATU (2.57 g, 6.76 mmol) was added to 100 mL one-necked round-bottomed flask containing 5-amino-1-cyclopentyl-1*H*-pyrazole-3-carboxylic acid (1.2 g, 6.1 mmol), CH₃NH₂•HCl (0.623 g, 9.22 mmol), DIPEA (2.38 g, 18.4 mmol), and anhydrous DMF (40 mL) at rt. The resultant brown solution was stirred for 12 h at room temperature under N₂. After this time, the mixture was extracted with EtOAc (40 mL x 2), and the combined extracts washed with H₂O (20 mL x 2) and brine (30 mL), dried over anhydrous Na₂SO₄, filtered, and the filtrate concentrated to dryness *in vacuo* to give a yellow solid. The yellow solid was subjected to silica gel chromatography (50-100% EtOAc/pet ether) to give 5-amino-1-cyclopentyl-*N*-methyl-1*H*-pyrazole-3-carboxamide as a brown oil (1.1 g, 86%). MS (ESI⁺): m/z = 208.9.

### Intermediate 40: 4-Chloro-5-methyl-N-(1-methyl-1H-pyrazol-3-yl)pyrimidin-2-amine

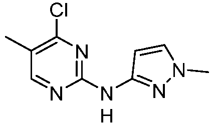

1-Methyl-1*H*-pyrazol-3-amine (592 mg, 6.10 mmol) and anhydrous THF (20 mL) were added to an oven-dried and nitrogen-purged 100 mL three-neck round-bottom flask, which was subsequently cooled to -70 °C. LDA (3.39 mL, 6.78 mmol, 2 M in hexanes and THF) was added, dropwise over 5 min. The resulting mixture was stirred for 30 min at -70 °C. 4-Chloro-5-methyl-2-(methylsulfonyl)pyrimidine (700 mg, 3.39 mmol, in 5 mL of dry THF) was added dropwise over 5 min to the mixture at -70 °C and the resulting mixture stirred for 1 h with gradual warming to rt. The reaction mixture was diluted with H₂O (50 mL) and extracted with ethyl acetate (100 mL x 3). The combined organic extracts were washed with brine (50 mL), dried over anhydrous Na₂SO₄, filtered, and concentrated to dryness *in vacuo* to give a brown solid, which was then subjected to silica gel chromatography (25-50% EtOAc/pet ether) to give 4-chloro-5-methyl-*N*-(1-methyl-1*H*-pyrazol-3-yl)pyrimidin-2-amine as a yellow solid (260 mg, 34%).

### Intermediate 41: (R)-3-Hydroxy-1-methyl-3-(3-(4-(trimethylstannyl)thiazol-2-yl)phenyl)pyrrolidin-2-one

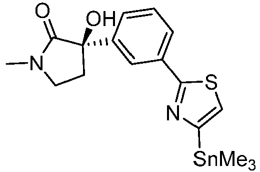

Pd(PPh₃)₄ (327 mg, 0.283 mmol), 1,1,1,2,2,2-hexamethyldistannane (1.39 g, 4.25 mmol), (*R*)-3-(3-(4-bromothiazol-2-yl)phenyl)-3-hydroxy-1-methylpyrrolidin-2-one (Intermediate 7, 1.00 g, 2.83 mmol), and anhydrous toluene (10 mL) were added to a 100 mL three-necked round-bottom flask fitted with a reflux condenser and under an atmosphere of nitrogen. The resultant mixture was heated at 110 °C for 16 hours before cooling to room temperature. The mixture was treated with aq. KF (100 mL, 2 N), and was stirred at room temperature for 2 hours. The mixture was then extracted with ethyl acetate (100 mL x 3). The combined organic extracts were washed with brine (50 mL), dried over anhydrous Na₂SO₄, filtered, and concentrated to dryness *in vacuo* to give the product which was subjected to silica gel chromatography (0-100% EtOAc/pet ether) to afford a yellow oil. The yellow oil was further purified by HPLC (Boston Prime C18 column, 5 µM, 150 x 30 mm, 55-85% (v/v) CH₃CN/ H₂O with 0.05% NH₃•H₂O + 10 mM NH₄HCO₃) to give, after lyophilization, (*R*)-3-hydroxy-1-methyl-3-(3-(4-(trimethylstannyl)thiazol-2-yl)phenyl)pyrrolidin-2-one a yellow oil (100 mg, 8%). MS (ESI⁺): m/z = 439.0 [M+H]⁺.

### Intermediate 42: N-(1-Methyl-1H-pyrazol-3-yl)-4-(tributylstannyl)pyrimidin-2-amine

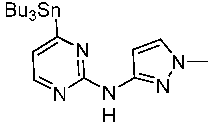

4-Chloro-*N*-(1-methyl-1*H*-pyrazol-3-yl)pyrimidin-2-amine (300 mg, 1.43 mmol), LiCl (364 mg, 8.59 mmol), 1,1,1,2,2,2-hexabutyldistannane (1.79 g, 3.09 mmol), and 1,4-dioxane (10 mL) were added to a 50 mL flask fitted with a reflux condenser. The resulting mixture was purged with N₂ three times and then treated with Pd₂(dba)₃ (66 mg, 0.072 mmol) and tricyclohexylphosphine (40 mg, 0.14 mmol). The resultant mixture was purged with N₂ for another three times and then heated at 120 °C for 16 hours. After this time, the reaction vessel was removed from the oil bath and allowed to gradually cool to rt. The reaction mixture was quenched with sat. aqueous KF (30 mL), stirred for 1 h, and extracted with EtOAc (20 mL x 3). The combined organic extracts were washed with brine (20 mL), dried over anhydrous Na₂SO₄, filtered, and concentrated to dryness *in vacuo* to give a brown solid. The solid was subjected to silica gel chromatography (0-30% EtOAc/pet ether) to give *N*-(1-methyl-1*H*-pyrazol-3-yl)-4-(tributylstannyl)pyrimidin-2-amine as a white solid (350 mg, 53%). MS (ESI⁺): m/z = 466.0.

### Intermediate 43: (R)-3-(3-(4-Bromo-5-methylthiazol-2-yl)phenyl)-3-hydroxy-1-methylpyrrolidin-2-one

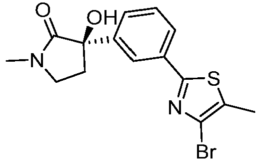

(*R*)-3-Hydroxy-1-methyl-3-(3-(4,4,5,5-tetramethyl-1,3,2-dioxaborolan-2-yl)phenyl)pyrrolidin-2-one (200 mg, 0.631 mmol), 2,4-dibromo-5-methylthiazole (162 mg, 0.631 mmol), K₃PO₄ (402 mg, 1.89 mmol) and 1,4-dioxane/H₂O (3 mL, v/v = 4:1) were added to a 20 mL microwave tube. The mixture was sparged with N₂ for 5 min, treated with Pd(dtbpf)Cl₂ (41 mg, 0.063 mmol), and heated at 80 °C via microwave irradiation for 1 h. After this time, the reaction vessel was removed from the microwave reactor and allowed to gradually cool to rt. The reaction mixture was then diluted with H₂O (10 mL) and extracted with EtOAc (10 mL x 3). The combined organic extracts were washed with brine (10 mL), dried over anhydrous Na₂SO₄, filtered, and concentrated to dryness *in vacuo* to give a brown oil. The oil was purified by silica gel chromatography (0-100% EtOAc/pet ether) to give (*R*)-3-(3-(4-bromo-5-methylthiazol-2-yl)phenyl)-3-hydroxy-1-methylpyrrolidin-2-one as a yellow oil (190 mg, 78%). MS (ESI⁺): m/z = 369.0 [M+H]⁺.

### Intermediate 44: (R)-3-(3-(2-bromothiazol-4-yl)phenyl)-3-hydroxy-1-methylpyrrolidin-2-one

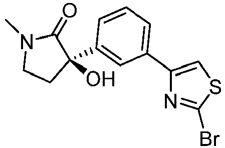

Step A. (*R*)-3-hydroxy-1-methyl-3-(3-(thiazol-4-yl)phenyl)pyrrolidin-2-one. A mixture of 1,4-dioxane (8 mL), water (2 mL), (R)-3-hydroxy-1-methyl-3-(3-(4,4,5,5-tetramethyl-1,3,2-dioxaborolan-2-yl)phenyl)pyrrolidin-2-one (2.0 g, 6.3 mmol), 4-bromothiazole (562 µL, 6.31 mmol), K₃PO₄ (4.0 g, 19 mmol) and Pd(dtbpf)Cl₂ (411 mg, 0.631 mmol) was heated to 90 °C for 1 h in a microwave reactor. The mixture was then cooled to rt, diluted with water, and extracted with ethyl acetate. The organic extract was dried with brine, Na₂SO₄, filtered, and concentrated. The residue was purified by FCC (silica, gradient 0-100% EtOAc-petroleum ether) to give (*R*)-3-hydroxy-1-methyl-3-(3-(thiazol-4-yl)phenyl)pyrrolidin-2-one as a brown oil (1.4 g, 80%). MS (ESI⁺): m/z/ = 274.6.

Step B. (*R*)-3-(3-(2-bromothiazol-4-yl)phenyl)-3-hydroxy-1-methylpyrrolidin-2-one. To a stirring mixture of THF (30 mL) and (*R*)-3-hydroxy-1-methyl-3-(3-(thiazol-4-yl)phenyl)pyrrolidin-2-one (1.4 g, 5.1 mmol) at -72 °C was added LDA (9 mL, 1 M solution in THF, 18 mmol). After 30 min, the resulting mixture was treated with bromine (471 µL, 9.19 mmol). After an additional 30 min, water (50 mL) was added and the resulting mixture was extracted with EtOAc. The organic extracts were dried with Na₂SO₄, filtered, and concentrated. The residue was purified by FCC (silica gel, gradient 0-100% EtOAc/petroleum ether) to give (*R*)-3-(3-(2-bromothiazol-4-yl)phenyl)-3-hydroxy-1-methylpyrrolidin-2-one (1 g, 49%) as a brown oil. MS (ESI⁺): m/z = 353.0.

### Intermediate 45: (R,S)-7-(3-(4-Bromothiazol-2-yl)phenyl)-6,7-dihydro-5H-pyrrolo[1,2-a]imidazol-7-ol

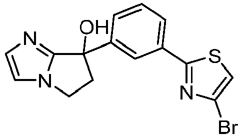

Step A. (*R*,*S*)-7-(3-Bromophenyl)-6,7-dihydro-5*H*-pyrrolo[1,2-*a*]imidazol-7-ol. 1,3-Dibromobenzene (2.32 g, 9.83 mmol) and anhydrous THF (10 mL) were added into an 100 mL three-necked round-bottom flask, which was subsequently cooled to -72 °C and treated with n-butyllithium (2.5 M solution in hexane, 4.3 mL, 11 mmol). The resulting light yellow mixture was stirred at -72 °C for 30 min, treated with 5H-pyrrolo[1,2-a]imidazol-7(6H)-one (1.00 g, 8.19 mmol) in anhydrous THF (5 mL), and stirred for 30 min at about -72 °C. After this time, the brown mixture was quenched with water (40 mL), warmed to r.t., and extracted with EtOAc (50 mL x 2). The combined organic extracts were dried over anhydrous Na₂SO₄, filtered, and the filtrate concentrated to dryness *in vacuo* to give a yellow solid. The yellow solid was purified by silica gel chromatography (0-50% EtOAc/pet ether) to give a yellow solid. The yellow solid was further purified by HPLC (Xtimate C18 column, 10 mm, 150 x 40 mm; 21-51% (v/v) CH₃CN/water 0.05% NH₃•H₂O + 10 mM NH₄HCO₃), to give, after lyophilization, (*R*,*S*)-7-(3-bromophenyl)-6,7-dihydro-5H-pyrrolo[1,2-a]imidazol-7-ol as a white solid (600 mg, 26%). MS (ESI⁺): m/z = 279.0.

Step B. (*R*,*S*)-7-(3-(4,4,5,5-Tetramethyl-1,3,2-dioxaborolan-2-yl)phenyl)-6,7-dihydro-5*H*-pyrrolo[1,2-a]imidazol-7-ol. (*R*,*S*)-7-(3-Bromophenyl)-6,7-dihydro-5H-pyrrolo[1,2-a]imidazol-7-ol (700 mg, 2.51 mmol), 4,4,4',4',5,5,5',5'-octamethyl-2,2'-bi(1,3,2-dioxaborolane) (1.15 g, 4.51 mmol), Pd(dppf)Cl₂•CH₂Cl₂ (367 mg, 0.502mmol), KOAc (738 mg, 7.52 mmol), and 1,4-dioxane (7 mL) were added to a 50 mL round-bottomed flask fitted with a reflux condenser and under an atmosphere of nitrogen. The resultant mixture was heated at 100 °C for 16 h and turned black. After this time, the reaction mixture was cooled to r.t., poured into H₂O (50 mL), and extracted with ethyl acetate (100 mL x 3). The combined organic extracts were washed with brine (200 mL), dried over anhydrous Na₂SO₄, filtered, and the filtrate concentrated to dryness *in vacuo* to give a black solid. The black solid was subjected to silica gel chromatography (10-100% EtOAc/pet ether) to give (*R*,*S*)-7-(3-(4,4,5,5-tetramethyl-1,3,2-dioxaborolan-2-ylphenyl)-6,7-dihydro-5*H*-pyrrolo[1,2-a]imidazol-7-ol as a solid (300 mg, 37%).

Step C. (*R*,*S*)-7-(3-(4-Bromothiazol-2-yl)phenyl)-6,7-dihydro-5H-pyrrolo[1,2-a]imidazol-7-ol. 2,4-Dibromothiazole (232 mg, 0.956 mmol), (*R*,*S*)-7-(3-(4,4,5,5-tetramethyl-1,3,2-dioxaborolan-2-yl)phenyl)-6,7-dihydro-5*H*-pyrrolo[1,2-a]imidazol-7-ol ( 260 mg, 0.797 mmol), K₂CO₃ (330 mg 2.39 mmol), and 1,4-dioxane/H₂O (4:1) (1 mL) were added to a nitrogen-purged 5 mL microwave tube, then treated with Pd(dppf)Cl₂ (58 mg, 0.080 mmol). The resultant mixture was purged with N₂ for 5 min, and heated at 90 °C via microwave irradiation for 1 h to give a black solution. After this time, the mixture was cooled to r.t., poured into H₂O (50 mL) and extracted with ethyl acetate (100 mL x 3). The combined organic extracts were washed with brine (200 mL), dried over anhydrous Na₂SO₄, filtered, and the filtrate concentrated to dryness *in vacuo* to give a black solid. The black solid was subjected to silica gel chromatography (0-10% MeOH/DCM) to afford (*R*,*S*)-7-(3-(4-bromothiazol-2-yl)phenyl)-6,7-dihydro-5*H-*pyrrolo[1,2-a]imidazol-7-ol as a yellow solid (160 mg, 55%).

### Intermediate 46: (R)-3-(3-(6-Bromopyridin-2-yl)phenyl)-3-hydroxy-1-methylpyrrolidin-2-one

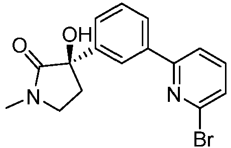

2,6-Dibromopyridine (934 mg, 3.94 mmol), (*R*)-3-hydroxy-1-methyl-3-(3-(4,4,5,5-tetramethyl-1,3,2-dioxaborolan-2-yl)phenyl)pyrrolidin-2-one (500 mg, 1.58 mmol), K₂CO₃ (654 mg, 4.73 mmol) and anhydrous 1,4-dioxane/H₂O (4:1) (15 mL) were added to a nitrogen-purged 30 mL microwave tube, then treated with Pd(dppf)Cl₂ (115 mg, 0.158 mmol). The resultant mixture was purged with N₂ for 5 min, and then heated at 110 °C via microwave irradiation for 1 h to give a black solution. The solution was cooled to r.t., poured into H₂O (50 mL) and extracted with ethyl acetate (20 mL x 3). The combined organic extracts were washed with brine (20 mL), dried over anhydrous Na₂SO₄, filtered, and the filtrate concentrated to dryness *in vacuo* to give a black solid. The black solid was subjected to silica gel chromatography (0-100% EtOAc/pet ether) to afford (*R*)-3-(3-(6-bromopyridin-2-yl)phenyl)-3-hydroxy-1-methylpyrrolidin-2-one as a yellow solid (340 mg, 62%). MS (ESI⁺): m/z = 347.0.

### Intermediate 47: (R)-3-Hydroxy-1-methyl-3-(3-(6-(2-(methylsulfinyl)pyrimidin-4-yl)pyridin-2-yl)phenyl)pyrrolidin-2-one

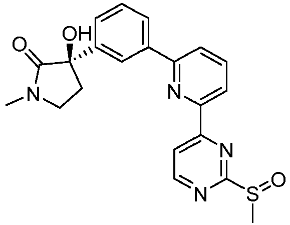

Step A. (*R*)-3-Hydroxy-1-methyl-3-(3-(6-(2-(methylthio)pyrimidin-4-yl)pyridin-2-yl)phenyl)pyrrolidin-2-one. (*R*)-3-(3-(6-Bromopyridin-2-yl)phenyl)-3-hydroxy-1-methylpyrrolidin-2-one (Intermediate 46, 190 mg, 0.55 mmol), Pd(PPh₃)₄ (63 mg, 0.055 mmol), 2-(methylthio)-4-(tributylstannyl)pyrimidine (341 mg, 0.821 mmol), TEA (0.228 mL, 1.64 mmol), and anhydrous toluene (6 mL) were added to a 50 mL single-necked round-bottomed flask fitted with a reflux condenser and under an atmosphere of nitrogen. The resultant mixture heated at 120 °C for 16 h and turned brown. The reaction mixture was concentrated to dryness *in vacuo* to give a brown oil. The brown oil was purified by silica gel chromatography (50-100% EtOAc/pet ether) to afford (*R*)-3-hydroxy-1-methyl-3-(3-(6-(2-(methylthio)pyrimidin-4-yl)pyridin-2-yl)phenyl)pyrrolidin-2-one as a yellow oil (200 mg, 93%).

Step B. (*R*)-3-Hydroxy-1-methyl-3-(3-(6-(2-(methylsulfinyl)pyrimidin-4-yl)pyridin-2-yl)phenyl)pyrrolidin-2-one. ((*R*)-3-Hydroxy-1-methyl-3-(3-(6-(2-(methylthio)pyrimidin-4-yl)pyridin-2-yl)phenyl)pyrrolidin-2-one (200 mg, 0.510 mmol), hydrogen peroxide (0.625 mL, 6.12 mmol), and 1,1,1,3,3,3-hexafluoro-2-propanol (12 mL) were added to a 50 mL round-bottomed flask. Then the resultant mixture was stirred at r.t. for 16 h and turned yellow. The reaction mixture was quenched with aq. Na₂O₃S₂ (50 mL, 2 N) and extracted with EtOAc (50 mL x 3). The combined organic extracts were dried over anhydrous Na₂SO₄, filtered, and the filtrate concentrated to dryness *in vacuo* to give (*R*)-3-hydroxy-1-methyl-3-(3-(6-(2-(methylsulfinyl)pyrimidin-4-yl)pyridin-2-yl)phenyl)pyrrolidin-2-one as a yellow solid (200 mg). MS (ESI⁺): m/z = 409.0.

### Intermediate 48: (R,S)-3-(2-Fluoro-5-(4,4,5,5-tetramethyl-1,3,2-dioxaborolan-2-yl)phenyl)-3-hydroxy-1-methylpyrrolidin-2-one

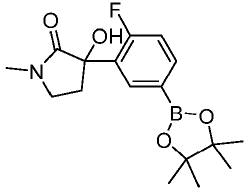

Step A. (*R*,*S*)-3-(5-Bromo-2-fluorophenyl)-3-hydroxy-1-methylpyrrolidin-2-one. 4-Bromo-1-fluoro-2-iodobenzene (3.46 g, 11.5 mmol) and anhydrous THF (20 mL) were added to a oven-dried 100 mL three-necked round-bottomed flask, which was subsequently cooled to -70 °C, and the resuting mixture was treated with n-BuLi (2.5 M in hexanes, 4.60 mL, 11.5 mmol), portion-wise over 10 min. The reaction mixture was stirred for 30 min at this temperature, then treated with a second solution of 1-methylpyrrolidine-2,3-dione (1.00 g, 8.84 mmol) and anhydrous THF (3 mL), portion-wise over 10 min. The final mixture was stirred for another 30 min. The reaction mixture was quenched with *aq*. NH₄Cl (10 mL, 2 N) and H₂O (10 mL) before removing from the dry ice/EtOH bath, then allowed to warm to r.t., extracted with EtOAc (20 mL x 3), and the combined extracts washed with brine (10 mL), dried over anhydrous Na₂SO₄, filtered, and concentrated to dryness *in vacuo* to give an off-white solid. The solid was subjected to sillica gel chromatography (0-60% ethyl acetate/petroleum ether) followed by HPLC (Welch Xtimate C18 column, 5 µm, 150 x 30 mm; eluent: 20-50% (v/v) CH₃CN and H₂O with 0.225% HCOOH) to give, after lyophillization, (*R*,*S*)-3-(5-bromo-2-fluorophenyl)-3-hydroxy-1-methylpyrrolidin-2-one obtained as a white solid (670 mg, 34%). MS (ESI⁺): m/z = 287.5. ¹H NMR (400 MHz, CD₃OD) δ 7.84 - 7.81 (m, 1H), 7.49 - 7.45 (m, 1H), 7.04 - 7.00 (m, 1H), 3.67 - 3.57 (m, 1H), 3.50 - 3.45 (m, 1H), 2.94 (s, 3H), 2.48 - 2.40 (m, 1H), 2.29 - 2.22 (m, 1H).

Step B. (*R*,*S*)-3-(2-Fluoro-5-(4,4,5,5-tetramethyl-1,3,2-dioxaborolan-2-yl)phenyl)-3-hydroxy-1-methylpyrrolidin-2-one. (*R*,*S*)-3-(5-Bromo-2-fluorophenyl)-3-hydroxy-1-methylpyrrolidin-2-one (100 mg, 346 µmol), B₂pin₂ (176 mg, 692 µmol), Pd(dppf)Cl₂•CH₂Cl₂ (28.3 mg, 34.6 µmol), KOAc (102 mg, 1.04 mmol), a stir bar, and anhydrous 1,4-dioxane (6 mL) were added to an oven-dried and N₂-purged 10 mL single-necked round-bottomed flask. The resulting mixture was heated to 100 °C for 16 h and turned black. The reaction vessel removed from the oil bath, and allowed to cool to room temperature, then filtered through a pad of diatomaceous earth (e.g., Celite^{®}), and the filter cake rinsed with EtOAc (10 mL x 3). The filtrate was concentrated to dryness *in vacuo* to give a brown solid, which was subjected to sillica gel chromatography (0-60% ethyl acetate/petroleum ether) to give (*R*,*S*)-3-(2-fluoro-5-(4,4,5,5-tetramethyl-1,3,2-dioxaborolan-2-yl)phenyl)-3-hydroxy-1-methylpyrrolidin-2-one as a white solid (100 mg, 69%). MS (ESI⁺): m/z = 335.7. ¹H NMR (400 MHz, CDCl₃) δ 8.09 - 8.06 (m, 1H), 7.77 - 7.73 (m, 1H), 7.05 - 7.01 (m, 1H), 3.57 - 3.41 (m, 2H), 2.99 (s, 3H), 2.51 - 2.35 (m, 2H), 1.34 (s, 12H).

### Intermediate 49: (R,S)-3-(3-Chloro-5-fluorophenyl)-3-hydroxy-1-methylpyrrolidin-2-one

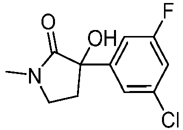

Step A. (3-Chloro-5-fluorophenyl)magnesium bromide. Mg (790 mg, 30.0 mmol), iodine (61 mg, 0.24 mmol), and anhydrous THF (3 mL) were added to a 100 mL three-necked round-bottomed flask at rt and then heated at 60 °C for 5 min. 1-Bromo-3-chloro-5-fluorobenzene (5.0 g, 24 mmol) in anhydrous THF (7 mL) was added over about 5 min and the mixture was heated at 65 °C for 30 min. The mixture was allowed to cool to room temperature and was used in the next step without purification.

Step B. (*R*,*S*)-3-(3-Chloro-5-fluorophenyl)-3-hydroxy-1-methylpyrrolidin-2-one. 1-Methylpyrrolidine-2,3-dione (1.00 g, 8.84 mmol) and anhydrous THF (3 mL) were combined and subsequently cooled to -78 °C, and charged with (3-chloro-5-fluorophenyl)magnesium bromide (5.17 mL, 8.84 mmol). The mixture was stirred at -78 °C for 2 h. The mixture was added into saturated aqueous NH₄Cl (5 mL) and extracted with EtOAc (10 mL x 3). The combined organic extracts were washed with brine (10 mL), dried over anhydrous Na₂SO₄, filtered, and the filtrate concentrated to dryness *in vacuo* to give a yellow oil. The yellow oil was subjected to silica gel chromatography (0-100% EtOAc/pet ether) to afford (*R*,*S*)-3-(3-chloro-5-fluorophenyl)-3-hydroxy-1-methylpyrrolidin-2-one (200 mg, 9%) as a yellow solid.

### Intermediate 50: (R,S)-3-(3-Chloro-4-fluorophenyl)-3-hydroxy-1-methylpyrrolidin-2-one

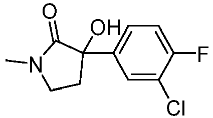

Step A. (3-Chloro-4-fluorophenyl)magnesium bromide. Mg (754 mg, 28.6 mmol), iodine (61 mg, 0.24 mmol), and anhydrous THF (3 mL) were added to a three-necked round-bottomed flask at r.t and heated at 60 °C for 5 min. 4-Bromo-2-chloro-1-fluorobenzene (5.0 g, 24 mmol) in anhydrous THF (7 mL) was added over about 5 min and the mixture was heated at 65 °C for 30 min. The mixture was allowed to cool to room temperature and was used in the next step without purification.

Step B. (*R*,*S*)-3-(3-Chloro-4-fluorophenyl)-3-hydroxy-1-methylpyrrolidin-2-one. 1-Methylpyrrolidine-2,3-dione (1.5 g, 13 mmol) and anhydrous THF (5 mL) were combined and subsequently cooled to -78 °C. The mixture was then charged with (3-chloro-4-fluorophenyl)magnesium bromide (6.20 mL, 13.3 mmol) and the resulting mixture was stirred at -78 °C for 2 h. The mixture was added into saturated aqueous NH₄Cl (15 mL) and extracted with EtOAc (10 mL x 3). The combined organic extracts were washed with brine (10 mL), dried over anhydrous Na₂SO₄, filtered, and the filtrate concentrated to dryness *in vacuo* to give a yellow oil. The yellow oil was subjected to silica gel chromatography (eluent: 50-100% EtOAc/pet ether) to afford (*R*,*S*)-3-(3-chloro-4-fluorophenyl)-3-hydroxy-1-methylpyrrolidin-2-one (480 mg, 15%) as a yellow solid.

### Intermediate 51: (R,S)-3-(2-bromopyridin-4-yl)-3-hydroxy-1-methylpyrrolidin-2-one

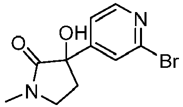

To a solution of 2-bromo-4-iodopyridine (90.0 g, 317 mmol, 1.00 eq) in THF (100 mL) was added dropwise iso-propylmagnesium chloride lithium chloride complex (1.30 M in THF, 244 mL, 1.00 eq) at 0 °C. The reaction mixture was stirred at this temperature for 30 mins. 1-Methylpyrrolidine-2,3-dione (35.9 g, 317 mmol, 1.00 eq) was added in one portion as a solid and the resulting mixture was stirred at 0 °C for 1 hr. Then the temperature was raised to 25 °C and the reaction mixture was stirred for 12 hrs. The reaction mixture was diluted by the addition of saturated aq. NH₄Cl (200 mL) at 0 °C, and then further diluted with water (300 mL) and extracted with 1500 mL EtOAc (300 mL × 5). The combined organic layers were washed with brine (300 mL × 1), dried over anhydrous Na₂SO₄, filtered and concentrated under reduced pressure to give a residue. The residue was purified by FCC (silica, 0-100% ethyl acetate/petroleum ether) to afford (R,S)-3-(2-bromopyridin-4-yl)-3-hydroxy-1-methylpyrrolidin-2-one (37.0 g, 121.05 mmol, 38%) as a light yellow solid. ¹H NMR (400 MHz, DMSO-*d*₆) δ 8.35 (d, *J* = 4.75 Hz, 1H), 7.59 (d, *J* = 1.00 Hz, 1H), 7.38 (dd, *J* = 5.19, 1.56 Hz, 1H), 6.43 (s, 1H), 3.35 - 3.51 (m, 2H), 2.83 (s, 3H), 2.29 - 2.40 (m, 1H), 2.22 (ddd, *J* = 13.38, 8.13, 5.00 Hz, 1H). MS (ESI⁺): m/z = 273.1.

### Intermediate 52 and Intermediate 53: (R)-3-(2-bromopyridin-4-yl)-3-hydroxy-1-methylpyrrolidin-2-one and (S)-3-(2-bromopyridin-4-yl)-3-hydroxy-1-methylpyrrolidin-2-one

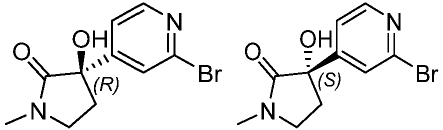

The (*R*) and (*S*) enantiomers of (*R*,*S*)-3-(2-bromopyridin-4-yl)-3-hydroxy-1-methylpyrrolidin-2-one (Intermediate 51, 37.0 g, 121.05 mmol) were separated by chiral SFC using a CHIRALPAK-AD column, 250 × 50 mm I.D., 10 µm column (Mobile phase: A for CO₂ and B for methanol (0.1% NH₃•H₂O); Gradient: B 25%; Flow rate: 140 mL /min; Back pressure: 100 bar; Column temperature: 40 °C; Wavelength: 220 nM) to provide the first eluting peak which was designated as enantiomer 1 of 3-(2-bromopyridin-4-yl)-3-hydroxy-1-methylpyrrolidin-2-one (Intermediate 52) (10.0 g, 27%). The enantiomeric excess (retention time = 1.324 min [first eluting peak], ee% = 100%) was determined by analytical SFC using a Waters UPC2 analytical SFC (SFC-E) instrument equipped with a CHIRALPAK AD, 50 × 4.6 mm I.D., 3 µm column (mobile phase: A for CO₂ and B for methanol (0.05% DEA); Gradient: from 5% to 40% of B in 2.5 min and hold 40% for 0.5 min, then 5% of B for 1 min; Flow rate: 4 mL/min; Back pressure: 100 bar; Column temperature: 35 °C; Wavelength: 220 nM). ¹H NMR (400 MHz, DMSO-*d*₆) δ 8.34 (d, *J* = 5.25 Hz, 1H), 7.59 (d, *J* = 0.88 Hz, 1H), 7.38 (dd, *J =* 5.19, 1.44 Hz, 1H), 6.43 (s, 1H), 3.35 - 3.50 (m, 2H), 2.83 (s, 3H), 2.30 - 2.39 (m, 1H), 2.23 (br dd, *J* = 8.07, 5.07 Hz, 1H). MS (ESI⁺): m/z = 273.2. [α]²⁰_{D} = -32 (c = 0.1 in MeOH).

The second eluting peak which was designated as enantiomer 2 of 3-(2-bromopyridin-4-yl)-3-hydroxy-1-methylpyrrolidin-2-one (Intermediate 53) (10.0 g, 27%, ee% = 100%). ¹H NMR (400 MHz, DMSO-*d*₆) δ 8.34 (d, *J* = 5.13 Hz, 1H), 7.59 (d, *J* = 0.88 Hz, 1H), 7.38 (dd, *J* = 5.19, 1.44 Hz, 1H), 6.43 (s, 1H), 3.35 - 3.51 (m, 2H), 2.83 (s, 3H), 2.30 - 2.39 (m, 1H), 2.17 - 2.26 (m, 1H). MS (ESI⁺): m/z = 271.2. [α]²⁰_{D} = +21 (c = 0.1 in MeOH). SFC Rt = 1.217 min.

### Intermediate 54: (R,S)-3-(5-bromopyridin-3-yl)-3-hydroxy-1-methylpyrrolidin-2-one

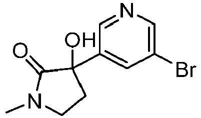

To a solution of 3,5-dibromopyridine (100 g, 422 mmol) in THF (1000 mL) was added dropwise iso-propylmagnesium chloride lithium chloride complex (1.30 M in THF, 357 mL) at 0 °C. The reaction mixture was stirred at this temperature for 30 min. 1-Methylpyrrolidine-2,3-dione (43.0 g, 380 mmol) was added in one portion as a solid and the resulting mixture was stirred at 0 °C for 1 hr. The reaction mixture was diluted with saturated aq. NH₄Cl (200 mL) at 0 °C, and then further diluted with water (300 mL) and extracted with ethyl acetate (1500 mL, 300 mL × 5). The combined organic layers were washed with brine (300 mL × 1), dried over anhydrous Na₂SO₄, filtered and concentrated under reduced pressure to give a residue. The residue was purified by FCC (silica, 0-100% ethyl acetate/ petroleum ether) to afford (*R*,*S*)-3-(5-bromopyridin-3-yl)-3-hydroxy-1-methylpyrrolidin-2-one (28.9 g, 99.0 mmol, 23%) as a light yellow solid. ¹H NMR (400 MHz, DMSO-*d*₆) δ 8.63 (d, *J* = 2.13 Hz, 1H), 8.55 (d, *J* = 1.88 Hz, 1H), 8.00 (t, *J* = 2.13 Hz, 1H), 6.38 (s, 1H), 3.35 - 3.50 (m, 2H), 2.83 (s, 3H), 2.43 (ddd, *J* = 13.35, 7.91, 5.38 Hz, 1H), 2.24 (ddd, *J* = 13.35, 8.04, 5.13 Hz, 1H). MS (ESI⁺): m/z = 273.1.

### Intermediate 55 and Intermediate 56: (R)-3-(5-bromopyridin-3-yl)-3-hydroxy-1-methylpyrrolidin-2-one and (S)-3-(5-bromopyridin-3-yl)-3-hydroxy-1-methylpyrrolidin-2-one

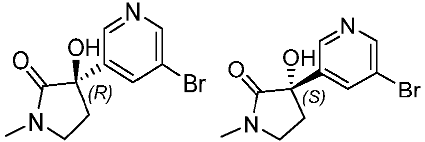

The (R) and (S) enantiomers of (R,S)-3-(5-bromopyridin-3-yl)-3-hydroxy-1-methylpyrrolidin-2-one (Intermediate 54, 28.9 g, 99.0 mmol) were separated by chiral SFC using a CHIRALPAK-AD column, 300 × 50mm I.D., 10 µm column (Mobile phase: A for CO₂ and B for methanol; Gradient: B 40%; Flow rate: 200 mL/min; Back pressure: 100 bar; Column temperature: 38 °C; Wavelength: 220 nM) to provide the first eluting peak which was designated as enantiomer 1 of 3-(5-bromopyridin-3-yl)-3-hydroxy-1-methylpyrrolidin-2-one (Intermediate 55) (10.0 g, 35%). The enantiomeric excess (retention time = 1.763 min [2^{nd} eluting peak], ee% = 99.7%) was determined by analytical SFC using a Waters UPC2 analytical SFC (SFC-H) instrument equipped with a CHIRALPAK AD, 150 × 4.6 mm I.D., 3 µm column (mobile phase: A for CO₂ and B for methanol (0.05% DEA); Gradient: B 40%; Flow rate: 2.5 mL/min; Back pressure: 100 bar; Column temperature: 35 °C; Wavelength: 220 nM). ¹H NMR (400 MHz, DMSO-*d*_{6):} δ 8.63 (d, *J* = 2.13 Hz, 1H), 8.55 (d, *J* = 1.88 Hz, 1H), 8.00 (t, *J* = 2.06 Hz, 1H), 6.38 (s, 1H), 3.37 - 3.49 (m, 2H), 2.83 (s, 3H), 2.43 (ddd, *J* = 13.41, 7.91, 5.32 Hz, 1H), 2.24 (ddd, *J* = 13.35, 8.04, 5.13 Hz, 1H). MS (ESI⁺): m/z = 271.2. [α]²⁰_{D} = -36 (c = 0.1 in MeOH).

The second eluting peak was designated as enantiomer 2 of 3-(5-bromopyridin-3-yl)-3-hydroxy-1-methylpyrrolidin-2-one (Intermediate 56) (10.0 g, 35%, ee% = 100%). ¹H NMR (400 MHz, DMSO-*d*₆): δ 8.62 (d, *J* = 2.13 Hz, 1H), 8.54 (d, *J* = 1.88 Hz, 1H), 8.00 (t, *J* = 2.13 Hz, 1H), 6.38 (s, 1H), 3.37 - 3.49 (m, 2H), 2.82 (s, 3H), 2.42 (ddd, *J* = 13.35, 7.91, 5.38 Hz, 1H), 2.19 - 2.28 (m, 1H). MS (ESI⁺): m/z = 271.2. [α]²⁰_{D} = +26 (c = 0.1 in MeOH). SFC Rt = 1.473 min.

### Intermediate 57: (R,S)-tert-butyl 3-(4-(3-hydroxy-1-methyl-2-oxopyrrolidin-3-yl)pyridin-2-yl)-1H-pyrazole-1-carboxylate

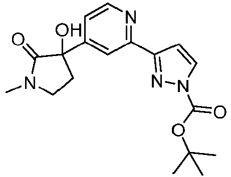

A mixture of (*R*,*S*)-3-(2-bromopyridin-4-yl)-3-hydroxy-1-methylpyrrolidin-2-one (Intermediate 51, 0.2 g, 0.738 mmol), 1-tert-butoxycarbonyl-3-(4,4,5,5-tetramethyl-1,3,2-dioxaborolane-2-yl)pyrazole (282.1 mg, 0.959 mmol), bis(triphenylphosphine)palladium(ii) dichloride (51.78 mg, 0.0738 mmol), K₂CO₃ (1 M in H₂O, 1.844 mL, 1.844 mmol) and 1,4-dioxane (8 mL) was heated at 70 °C for 2 h and then 40 °C for 16 h. The mixture was cooled to rt, diluted with water and extracted with ethyl acetate. The organic extract was dried with anhydrous MgSO₄, filtered, and concentrated. The residue was purified by FCC (gradient 0->15% MeOH in DCM) to afford (*R*,*S*)-tert-butyl 3-(4-(3-hydroxy-1-methyl-2-oxopyrrolidin-3-yl)pyridin-2-yl)-1H-pyrazole-1-carboxylate (22 mg, 8%) as a colorless oil. MS (ESI⁺): m/z = 359.1.

### Intermediate 58: (R,S)-3-(6'-chloro-[2,2'-bipyridin]-4-yl)-3-hydroxy-1-methylpyrrolidin-2-one

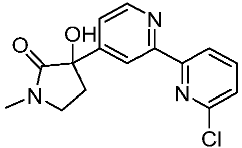

A mixture of (*R*,*S*)-3-(2-bromopyridin-4-yl)-3-hydroxy-1-methylpyrrolidin-2-one (Intermediate 51, 250 mg, 0.922 mmol), 6-chloro-2-(tributylstannyl)pyridine (408.4 mg, 1.014 mmol), tetrakis(triphenylphosphine)palladium(0) (213 mg, 0.184 mmol) and toluene (2 mL, 0.867 g/mL, 18.819 mmol) was heated in a microwave reactor at 130 °C for 1 hour and then 160 °C for an additional hour. The mixture was cooled to rt, diluted with water, and extracted with ethyl acetate. The organic extract was dried with anhydrous MgSO₄, filtered, and concentrated. The residue was purified by FCC (silica, gradient 50-100% EtOAc in hexanes) to afford (R,S)-3-(6'-chloro-[2,2'-bipyridin]-4-yl)-3-hydroxy-1-methylpyrrolidin-2-one (90 mg, 32%) as a white powder. MS (ESI⁺): m/z = 304.0.

### Intermediate 59: (R,S)-3-(2-(4-Bromothiazol-2-yl)pyridin-4-yl)-3-hydroxy-1-methylpyrrolidin-2-one

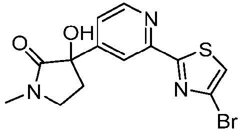

Step A: (*R*,*S*)-3-hydroxy-1-methyl-3-(2-(trimethylstannyl)pyridin-4-yl)pyrrolidin-2-one. A mixture of (*R*,*S*)-3-(2-bromopyridin-4-yl)-3-hydroxy-1-methylpyrrolidin-2-one (Intermediate 51, 350 mg, 1.291 mmol), hexamethylditin (423 mg, 1.29 mmol), tetrakis(triphenylphosphine)palladium(0) (149.183 mg, 0.129 mmol) and 1,4-dioxane (2.5 mL) was heated at 110 °C for 2 h. The mixture was cooled to rt, diluted with water, and extracted with ethyl acetate. The organic extract was dried with anhydrous MgSO₄, filtered, and concentrated to afford (R,S)-3-hydroxy-1-methyl-3-(2-(trimethylstannyl)pyridin-4-yl)pyrrolidin-2-one (262.2 mg, 57%) as a beige powder. MS (ESI⁺): m/z = 357.0.

Step B: (*R,S*)-3-(2-(4-bromothiazol-2-yl)pyridin-4-yl)-3-hydroxy-1-methylpyrrolidin-2-one. A mixture of (*R,S*)-3-hydroxy-1-methyl-3-(2-(trimethylstannyl)pyridin-4-yl)pyrrolidin-2-one (100 mg, 0.282 mmol), 2,4-dibromothiazole (205 mg, 0.845 mmol), tetrakis(triphenylphosphine)palladium(0) (48.8 mg, 0.0423 mmol) and DMF (2.8 mL) was heated in a microwave reactor at 120 °C for 1 h and then at 140 °C for an additional h. The mixture was diluted with water and extracted with ethyl acetate. The organic extract was dried with anhydrous MgSO₄, filtered, and concentrated. The residue was purified by chromatography (silica, gradient 50->100% of hexanes and 10% MeOH-EtOAc) to afford (*R,S*)-3-(2-(4-bromothiazol-2-yl)pyridin-4-yl)-3-hydroxy-1-methylpyrrolidin-2-one (17.2 mg, 17%).

### Intermediate 60: 4,4-difluoro-3,3-dihydroxy-1-methylpyrrolidin-2-one

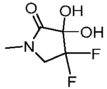

Step A. (E)-1-methyl-3-((1-phenylethyl)imino)pyrrolidin-2-one. Into a 20 L 4-necked round-bottom flask was added 1-methylpyrrolidine-2,3-dione (346 g, 3058.80 mmol), MgSO₄ (368.16 g, 3058.80 mmol) and DCM (7 L) at room temperature. To this was added (+/-)-α-methylbenzylamine (389.21 g, 3211.74 mmol) and TFA (0.2 mL) at room temperature. The resulting mixture was stirred for overnight at 40 °C under a nitrogen atmosphere. The mixture was allowed to cool down to room temperature. The solids were filtered out and washed with DCM (1x1 L). The filtrate was collected and concentrated under reduced pressure. The product was slurried with ether (1.5 L). The solids were collected by filtration. This resulted in (3E)-1-methyl-3-[(1-phenylethyl)imino]pyrrolidin-2-one (540 g) as a purple solid. ¹H NMR (300 MHz, DMSO-*d*₆) δ 7.39-7.08 (m, 5H), 5.41 (d, *J* = 7.0 Hz, 1H), 4.88 (t, *J* = 2.4 Hz, 1H), 4.30-4.13(m,1H), 3.76-3.52 (m, 2H), 2.89 (s, 3H), 1.40 (d, *J* = 6.8 Hz, 3H).

Step B. 4,4-difluoro-3,3-dihydroxy-1-methylpyrrolidin-2-one. Into a 10 L 4-necked round-bottom flask were added (3E)-1-methyl-3-[(1-phenylethyl)imino]pyrrolidin-2-one (540 g, 2496.71 mmol), Na₂SO₄ (510.5 g, 3595.26 mmol) and acetonitrile (5.4 L) at room temperature. To this was added Select-F (1945.87 g, 5492.77 mmol) in portions at 0 °C. The resulting mixture was stirred overnight at room temperature. The mixture was diluted with acetonitrile (5.4 L). To the mixture was added HCl/1,4-dioxane (4 N, 936 mL) dropwise at 0 °C. The resulting mixture was stirred for additional 1 h at room temperature. The resulting precipitate was filtered out and washed with acetonitrile (1x2 L). The filtrate was concentrated under vacuum. This filtration and concentration were repeated 2 times again. The residue was purified by DAC: CH₃CN/H₂O (0.1% NH₄HCO₃) = 1% to 15% in 17 min to afford 4,4-difluoro-3,3-dihydroxy-1-methylpyrrolidin-2-one (122 g, two steps for 23.9%) as an off-white solid. ¹H NMR (300 MHz, DMSO-*d*₆) δ 7.29 (s, 2H), 3.65 (t, *J* = 12.0 Hz, 2H), 2.80 (s, 3H).

### Intermediate 61: 1-Methyl-5-(trifluoromethyl)pyrrolidine-2,3-dione

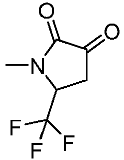

Step A. Ethyl 4,4,4-trifluoro-3-(methylamino)butanoate. To a solution of ethyl (E)-4,4,4-trifluorobut-2-enoate (4400 g, 26.17 mol, 3.89 L) in THF (18.0 L), was added MeNH₂ (3330 g, 32.17 mol, 30% purity). The light yellow solution was stirred at 25 °C for 3 hrs. The reaction mixture was concentrated under reduced pressure to provide the product as a yellow liquid (4.64 kg, 89%) which was used without further purification. ¹H NMR (400 MHz, chloroform-d) δ 4.08-4.26 (m, 2H), 3.40-3.56 (m, 1H), 2.61-2.68 (m, 1H), 2.51-2.55 (m, 3H), 2.41-2.50 (m, 1H), 1.22-1.29 (m, 3H).

Step B. Ethyl 4-hydroxy-1-methyl-5-oxo-2-(trifluoromethyl)-2,5-dihydro-1H-pyrrole-3-carboxylate. To a solution of ethyl 4,4,4-trifluoro-3-(methylamino)butanoate (2400 g, 12.05 mol) in 2-MeTHF (24000 mL) were added t-BuOK (1.35 kg, 12.05 mol) and diethyl oxalate (1.76 kg, 12.05 mol, 1.65 L) at 25 °C under a N₂ atmosphere. The reaction mixture was heated at 60 °C for 3 h under a N₂ atmosphere. The reaction mixture was quenched by the addition of NH₄Cl (saturated aqueous, 10.0 L) at 25 °C, the pH of the mixture was adjusted to pH = 2-3 with 1 M aqueous HCl (9.00 L), and the resulting mixture was extracted with EtOAc (5.00 L) twice. The combined organic extracts were washed with brine (15.0 L), dried over anhydrous Na₂SO₄, filtered and concentrated under reduced pressure to give a residue. The residue was purified by column chromatography (DCM/MeOH 0% to 3%) to afford the title compound as a brown oil (4.20 kg, 69%). ¹H NMR (400 MHz, chloroform-d) δ 4.63 (q, *J* = 5.0 Hz, 1H), 4.24-4.42 (m, 4H), 3.10-3.14 (m, 3H), 1.19-1.26 (m, 2H).

Step C. 1-Methyl-5-(trifluoromethyl)pyrrolidine-2,3-dione. A mixture of ethyl 4-hydroxy-1-methyl-5-oxo-2-(trifluoromethyl)-2,5-dihydro-1H-pyrrole-3-carboxylate (2000 g, 7.90 mol) in HCl (10.96 kg, 10.75 L, 36% aqueous solution) was heated at 110 °C for 16 hrs. The reaction mixture was cooled to 20 °C and extracted with isopropylacetate (5000 mL x 8). The combined organic extracts were washed with brine (10000 mL), dried over anhydrous Na₂SO₄, filtered and concentrated under reduced pressure to give a residue. The residue was triturated with MTBE (5000 mL) at 25 °C for 3 hrs. The solid was filtered, washed with MTBE (1000 mL) and dried under reduced pressure to afford the title compound as a yellow solid (1.15 kg, 6.35 mol, 40.2%). ¹H NMR (400 MHz, chloroform-d) δ 4.28-4.42 (m, 1H), 3.28 (s, 3H), 2.87-3.01 (m, 1H), 2.71-2.83 (m, 1H).

### Intermediate 62: (R,S)-3-(3-(4-bromothiazol-2-yl)phenyl)-4,4-difluoro-3-hydroxy-1-methylpyrrolidin-2-one

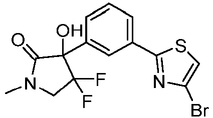

Step A: (3-Bromophenyl)lithium. n-BuLi (2.016 mL, 2.5 M in hexanes) was added over 5 minutes to a solution of dibromobenzene (0.581 mL, 1 equiv) in THF (4 mL, 1.2 M) at -78 °C. The resulting solution was stirred for 30 minutes before use in the next step.

Step B: (*R*,*S*)-3-(3-bromophenyl)-4,4-difluoro-3-hydroxy-1-methylpyrrolidin-2-one. 4,4-Difluoro-3,3-dihydroxy-1-methylpyrrolidin-2-one (Intermediate 60, 500 mg, 2.992 mmol) was dissolved in TFAA (7.48 mL, 2.992 mmol) and heated at 40 °C for 1 h. The resulting mixture was cooled to rt and concentrated. THF was then added and the solution was cooled down to -78 °C. (3-Bromophenyl)lithium from Step A (5.3 mL, 1.3 equiv) was added dropwise to the solution at -78 °C and the resulting mixture was stirred for 1 h. The resulting mixture was diluted with MeOH, filtered, and concentrated. The residue was purified by FCC to afford (*R,S*)-3-(3-bromophenyl)-4,4-difluoro-3-hydroxy-1-methylpyrrolidin-2-one (321.6 mg, 35%) as a white solid. MS (ESI⁺): m/z = 308.0.

Step C: (*R,S*)-4,4-difluoro-3-hydroxy-1-methyl-3-(3-(4,4,5,5-tetramethyl-1,3,2-dioxaborolan-2-yl)phenyl)pyrrolidin-2-one. A mixture of (*R,S*)-3-(3-bromophenyl)-4,4-difluoro-3-hydroxy-1-methylpyrrolidin-2-one (213 mg, 0.696 mmol), bis(pinacolato)diboron (247 mg, 0.974 mmol), chloro(2-dicyclohexylphosphino-2',4',6'-triisopropyl-1,1'-biphenyl)[2-(2'-amino-1,1'-biphenyl)]palladium(II) (55 mg, 0.0696 mmol), potassium acetate (273 mg, 2.783 mmol), and 1,4-dioxane (3.5 mL) was heated at 110 °C for 2 h. The mixture was cooled to rt and filtered through diatomaceous earth. The filtrate was concentrated and purified by FCC (100% hexanes) to afford (*R*,*S*)-4,4-difluoro-3-hydroxy-1-methyl-3-(3-(4,4,5,5-tetramethyl-1,3,2-dioxaborolan-2-yl)phenyl)pyrrolidin-2-one as a yellow oil (240 mg, 98%).

Step D: (*R,S*)-3-(3-(4-bromothiazol-2-yl)phenyl)-4,4-difluoro-3-hydroxy-1-methylpyrrolidin-2-one. A mixture of (*R,S*)-4,4-difluoro-3-hydroxy-1-methyl-3-(3-(4,4,5,5-tetramethyl-1,3,2-dioxaborolan-2-yl)phenyl)pyrrolidin-2-one (240 mg, 0.68 mmol), 2,4-dibromothiazole (214 mg, 0.883 mmol), PdCl₂(dppf) (50 mg, 0.068 mmol), K₂CO₃ (2 M in H₂O, 1.359 mL, 2.718 mmol), and 1,4-dioxane (4.5 mL) was heated at 110 °C for 4 h. The mixture was cooled to rt, filtered through diatomaceous earth, and purified by FCC (0-100% EtOAc in hexanes) to afford (*R*,*S*)-3-(3-(4-bromothiazol-2-yl)phenyl)-4,4-difluoro-3-hydroxy-1-methylpyrrolidin-2-one. MS (ESI⁺): m/z = 388.9.

### Intermediate 63: 4-(4-bromothiazol-2-yl)-N-(1-(1-methyl-1H-pyrazol-3-yl)ethyl)pyrimidin-2-amine

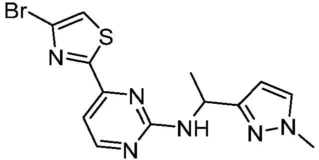

4-Bromo-2-(2-(methylsulfonyl)pyrimidin-4-yl)thiazole (Intermediate 10, 250 mg, 0.781 mmol), 1-(1-methyl-1H-pyrazol-3-yl)ethanamine (195.467 mg, 1.562 mmol), TFA (0.149 mL, 1.49 g/mL, 1.952 mmol), and DMSO (2 mL, 1.092 g/mL, 27.952 mmol) were combined and heated in a microwave reactor at 150 °C for 1 hr. After cooling to room temperature, the reaction mixture was washed with ethyl acetate (100 mL) and water (3x100 mL). The organic extracts were dried with anhydrous MgSO₄, filtered, and concentrated under reduced pressure. This material was purified by chromatography (gradient 0-100% EtOAc in hexanes) to yield a beige solid, 4-(4-bromothiazol-2-yl)-N-(1-(1-methyl-1H-pyrazol-3-yl)ethyl)pyrimidin-2-amine (111.3 mg, 39%). ¹H NMR (500 MHz, CDCl₃) δ 8.43 (d, J = 5.0 Hz, 1H), 7.37 (s, 1H), 7.31 (dd, J = 5.0, 0.7 Hz, 1H), 7.27 (d, J = 2.2 Hz, 1H), 6.19 (d, J = 2.2 Hz, 1H), 5.77 (d, J = 7.6 Hz, 1H), 5.34 - 5.26 (m, 1H), 3.88 (s, 3H), 1.62 (d, J = 6.8 Hz, 4H). MS (ESI⁺): m/z = 366.

### Intermediate 64: 4-(4-bromothiazol-2-yl)-N-(1-(1-methyl-1H-pyrazol-4-yl)ethyl)pyrimidin-2-amine

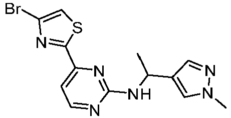

4-Bromo-2-(2-(methylsulfonyl)pyrimidin-4-yl)thiazole (Intermediate 10, 250 mg, 0.781 mmol), 1-(1-methyl-1h-pyrazol-4-yl)ethanamine (195.467 mg, 1.562 mmol), DIPEA (0.149 mL, 0.75 g/mL, 0.867 mmol), and DMA (2 mL, 0.941 g/mL, 21.602 mmol) were combined and heated in a microwave reactor at 150 °C for 1 hr. After cooling to room temperature, the reaction mixture was washed with ethyl acetate (100 mL) and water (3x100 mL). The organic extracts were dried with anhydrous MgSO₄, filtered, and concentrated under reduced pressure. This material was then purified by chromatography (gradient 0-100% EtOAc in hexanes) to afford a solid, 4-(4-bromothiazol-2-yl)-N-(1-(1-methyl-1H-pyrazol-4-yl)ethyl)pyrimidin-2-amine (191.9 mg, 67%). ¹H NMR (500 MHz, DMSO-*d₆*) δ 8.51 - 8.45 (m, 1H), 8.08 (s, 1H), 7.77 - 7.70 (m, 1H), 7.58 (s, 1H), 7.40 - 7.36 (m, 1H), 7.16 (d, J = 4.9 Hz, 1H), 5.20 - 5.06 (m, 2H), 3.76 (s, 3H), 1.47 (d, J = 6.9 Hz, 3H). MS (ESI⁺): m/z = 365.1.

### Intermediate 65: N-(1-(1H-pyrazol-3-yl)ethyl)-4-(4-bromothiazol-2-yl)pyrimidin-2-amine

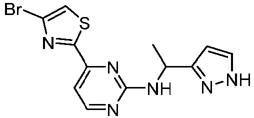

4-Bromo-2-(2-(methylsulfonyl)pyrimidin-4-yl)thiazole (Intermediate 10, 144.04 mg, 0.45 mmol), 1-(1*H*-pyrazol-3-yl)ethan-1-amine (100 mg, 0.9 mmol), DIPEA (0.31 mL, 0.75 g/mL, 1.799 mmol), and DMA (1 mL, 1.092 g/mL, 13.976 mmol) were combined and heated in a microwave reactor at 150 °C for 1 hr. After cooling to room temperature, the reaction mixture was diluted with ethyl acetate (100 mL) and extracted with water (3x100 mL). The organic extracts were dried with anhydrous MgSO₄, filtered and concentrated before purification by chromatography (gradient 0-100% EtOAc in hexanes) to afford *N*-(1-(1H-pyrazol-3-yl)ethyl)-4-(4-bromothiazol-2-yl)pyrimidin-2-amine (91 mg, yield 57%) as a yellow solid. ¹H NMR (500 MHz, DMSO-*d₆*) δ 12.65 - 12.38 (m, 1H), 8.49 (d, J = 4.9 Hz, 1H), 8.08 (s, 1H), 7.92 - 7.63 (m, 1H), 7.63 - 7.35 (m, 1H), 7.18 (d, J = 4.9 Hz, 1H), 6.20 (s, 1H), 5.28 (s, 1H), 1.50 (d, J = 6.9 Hz, 3H). MS (ESI⁺): m/z = 352.1.

### Example 1: (R)-3-hydroxy-1-methyl-3-(3-(4-(2-((1-methyl-1H-pyrazol-3-yl)amino)pyrimidin-4-yl)oxazol-2-yl)phenyl)pyrrolidin-2-one

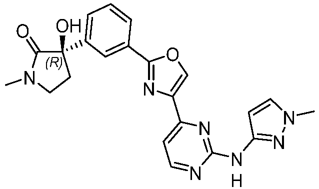

A mixture of N-(1-methyl-1H-pyrazol-3-yl)-4-(trimethylstannyl)pyrimidin-2-amine (Intermediate 1, 150 mg), (R)-3-(3-(4-bromooxazol-2-yl)phenyl)-3-hydroxy-1-methylpyrrolidin-2-one (Intermediate 2, 100 mg, 0.297 mmol), tetrakis(triphenylphosphine)palladium(0) (34 mg, 0.030 mmol), cuprous iodide (11 mg, 0.059 mmol) and DMF (1.3 mL) was heated to 80 °C for 2 h and then 100 °C overnight. The reaction mixture was diluted with water and extracted with ethyl acetate. The organic extract was dried with anhydrous MgSO₄, filtered, and concentrated under reduced pressure. (*R*)-3-Hydroxy-1-methyl-3-(3-(4-(2-((1-methyl-1H-pyrazol-3-yl)amino)pyrimidin-4-yl)oxazol-2-yl)phenyl)pyrrolidin-2-one was purified by FCC (0% to 15% MeOH in DCM) followed by RP HPLC (acidic conditions, column C1) to afford (*R*)-3-hydroxy-1-methyl-3-(3-(4-(2-((1-methyl-1H-pyrazol-3-yl)amino)pyrimidin-4-yl)oxazol-2-yl)phenyl)pyrrolidin-2-one as its trifluoroacetate salt (28 mg, 22%) as a yellow powder. ¹H NMR (500 MHz, DMSO-*d₆*) δ 9.90 (s, 1H), 8.86 (s, 1H), 8.55 (d, *J* = 5.0 Hz, 1H), 8.15 (t, *J* = 1.8 Hz, 1H), 7.97 (dt, *J* = 7.5, 1.5 Hz, 1H), 7.61 - 7.48 (m, 3H), 7.30 (d, *J* = 5.0 Hz, 1H), 6.69 (d, *J* = 2.2 Hz, 1H), 3.77 (s, 3H), 2.87 (s, 3H), 2.36 (ddd, *J* = 13.1, 7.8, 5.2 Hz, 1H), 2.32 - 2.24 (m, 1H). ¹⁹F NMR (471 MHz, DMSO-*d₆*) δ -74.65. MS (ESI⁺): m/z = 432.3.

### Example 2: (R)-3-hydroxy-1-methyl-3-(3-(1-(2-((1-methyl-1H-pyrazol-3-yl)amino)pyrimidin-4-yl)-1H-pyrazol-3-yl)phenyl)pyrrolidin-2-one

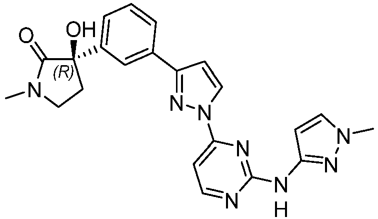

A mixture of (*R*)-3-(3-(1H-pyrazol-3-yl)phenyl)-3-hydroxy-1-methylpyrrolidin-2-one (Intermediate 3, 100 mg, 0.389 mmol), 4-chloro-N-(1-methylpyrazol-3-yl)pyrimidin-2-amine (122 mg, 0.583 mmol), Cs₂CO₃ (253 mg, 0.777 mmol) and DMF (2 mL) was heated at 100 °C for 30 minutes and then at 115 °C for 1 h. The reaction mixture was cooled to rt, diluted with water, and extracted with ethyl acetate. The organic extract was dried with anhydrous MgSO₄, filtered, and concentrated under reduced pressure. The residue was purified by RP HPLC (basic, column C2) to afford (*R*)-3-hydroxy-1-methyl-3-(3-(1-(2-((1-methyl-1H-pyrazol-3-yl)amino)pyrimidin-4-yl)-1H-pyrazol-3-yl)phenyl)pyrrolidin-2-one (61.5 mg, 36%) as a white powder. ¹H NMR (400 MHz, DMSO-*d₆*) δ 9.97 (s, 1H), 8.60 (d, *J* = 2.7 Hz, 1H), 8.54 (d, *J* = 5.4 Hz, 1H), 8.08 - 7.95 (m, 1H), 7.91 - 7.80 (m, 1H), 7.60 (d, *J* = 2.2 Hz, 1H), 7.45 (t, *J* = 7.7 Hz, 1H), 7.36 (ddd, *J* = 7.8, 1.9, 1.2 Hz, 1H), 7.29 (d, *J* = 5.4 Hz, 1H), 7.13 (d, *J* = 2.7 Hz, 1H), 6.63 (d, *J =* 2.2 Hz, 1H), 6.10 (s, 1H), 3.77 (s, 3H), 3.47 (ddd, *J* = 9.9, 8.1, 4.9 Hz, 1H), 3.38 (ddd, *J* = 9.9, 7.7, 6.2 Hz, 1H), 2.86 (s, 3H), 2.38 (ddd, *J* = 12.7, 7.6, 4.9 Hz, 1H), 2.27 (ddd, *J =* 13.4, 8.1, 5.7 Hz, 1H). MS (ESI⁺): m/z = 431.1.

### Example 3: (R)-3-hydroxy-1-methyl-3-(3-(2-(2-((1-methyl-1H-pyrazol-3-yl)amino)pyrimidin-4-yl)thiazol-4-yl)phenyl)pyrrolidin-2-one

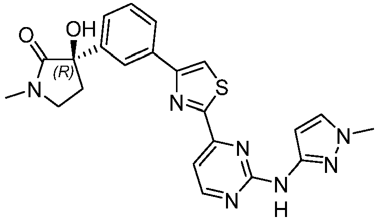

A mixture of 4-(4-bromothiazol-2-yl)-N-(1-methyl-1H-pyrazol-3-yl)pyrimidin-2-amine (Intermediate 4, 20 mg, 0.059 mmol), [1,1'-bis(diphenylphosphino)ferrocene]dichloropalladium(II) (4.4 mg, 0.0059 mmol), K₂CO₃ (0.15 mL, 1 M in H₂O, 0.15 mmol), 1,4-dioxane (0.5 mL) and (R)-3-hydroxy-1-methyl-3-[3-(4,4,5,5-tetramethyl-1,3,2-dioxaborolan-2-yl)phenyl]pyrrolidin-2-one (22.6 mg, 0.0712 mmol) was heated at 80 °C for 1 h and then cooled to rt. The reaction mixture was diluted with water and extracted with ethyl acetate. The organic extract was dried with anhydrous MgSO₄, filtered, and concentrated under reduced pressure. The residue was purified by RP HPLC (acidic conditions, column C1) and then filtered through a carbonate cartridge to afford (*R*)-3-hydroxy-1-methyl-3-(3-(2-(2-((1-methyl-1H-pyrazol-3-yl)amino)pyrimidin-4-yl)thiazol-4-yl)phenyl)pyrrolidin-2-one (9.3 mg, 35%) as a yellow powder. ¹H NMR (600 MHz, DMSO-*d₆*) δ 10.04 (s, 1H), 8.63 (d, *J* = 4.9 Hz, 1H), 8.35 (s, 1H), 8.09 (t, *J* = 1.8 Hz, 1H), 7.95 (ddd, *J* = 7.7, 1.8, 1.1 Hz, 1H), 7.63 (d, *J* = 2.2 Hz, 1H), 7.51 (d, *J* = 4.9 Hz, 1H), 7.45 (t, *J* = 7.7 Hz, 1H), 7.36 (ddd, *J* = 7.8, 1.8, 1.1 Hz, 1H), 6.80 - 6.64 (m, 2H), 6.10 (s, 1H), 3.78 (s, 3H), 3.51 - 3.43 (m, 1H), 3.42 - 3.35 (m, 1H), 2.87 (s, 3H), 2.43 - 2.36 (m, 1H), 2.32 - 2.24 (m, 1H). MS (ESI⁺): m/z = 448.1.

### Example 4: (R)-3-hydroxy-1-methyl-3-(3-(2-(2-((1-methyl-1H-pyrazol-3-yl)amino)pyrimidin-4-yl)oxazol-4-yl)phenyl)pyrrolidin-2-one

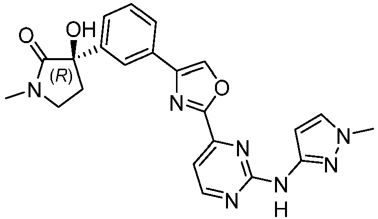

A mixture of 4-(4-bromooxazol-2-yl)-N-(1-methyl-1H-pyrazol-3-yl)pyrimidin-2-amine (Intermediate 5, 10 mg, 0.031 mmol), (R)-3-hydroxy-1-methyl-3-[3-(4,4,5,5-tetramethyl-1,3,2-dioxaborolan-2-yl)phenyl]pyrrolidin-2-one (12.8 mg, 0.0405 mmol), [1,1'-bis(diphenylphosphino)ferrocene]dichloropalladium(II) (2.3 mg, 0.0031 mmol), K₂CO₃ (0.080 mL, 1 M in H₂O, 0.080 mmol) and 1,4-dioxane (0.3 mL) was heated at 80 °C for 4 h. The reaction mixture was then diluted with water and extracted with ethyl acetate. The organic extract was dried with anhydrous MgSO₄, filtered, and concentrated under reduced pressure. The residue was purified by RP HPLC (basic, column C2) to afford (*R*)-3-hydroxy-1-methyl-3-(3-(2-(2-((1-methyl-1H-pyrazol-3-yl)amino)pyrimidin-4-yl)oxazol-4-yl)phenyl)pyrrolidin-2-one (6.9 mg, 51%) as a yellow solid. ¹H NMR (600 MHz, DMSO-*d₆*) δ 10.09 (s, 1H), 8.89 (s, 1H), 8.63 (d, *J* = 4.9 Hz, 1H), 7.96 - 7.89 (m, 1H), 7.81 - 7.74 (m, 1H), 7.62 - 7.55 (m, 1H), 7.49 - 7.39 (m, 2H), 7.37 - 7.29 (m, 1H), 6.77 (s, 1H), 6.10 (s, 1H), 3.76 (s, 2H), 3.51 - 3.43 (m, 1H), 3.41 - 3.35 (m, 1H), 2.87 (s, 2H), 2.39 - 2.31 (m, 1H), 2.31 - 2.23 (m, 1H). MS (ESI⁺): m/z = 432.4.

### Example 5: (R)-3-hydroxy-1-methyl-3-(3-(2-(2-((1-methyl-1H-pyrazol-5-yl)amino)pyrimidin-4-yl)thiazol-4-yl)phenyl)pyrrolidin-2-one

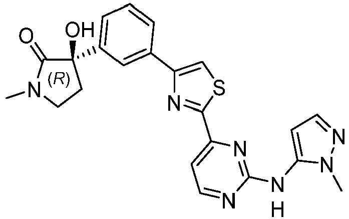

A mixture of 4-(4-bromothiazol-2-yl)-N-(1-methyl-1H-pyrazol-5-yl)pyrimidin-2-amine (Intermediate 14, 21 mg, 0.0623 mmol), (R)-3-hydroxy-1-methyl-3-[3-(4,4,5,5-tetramethyl-1,3,2-dioxaborolan-2-yl)phenyl]pyrrolidin-2-one (39.508 mg, 0.125 mmol), [1,1'-bis(diphenylphosphino)ferrocene]dichloropalladium(ii) (2.294 mg, 0.00311 mmol), K₂CO₃ (0.156 mL, 1 M, 0.156 mmol), and 1,4-dioxane (0.4 mL, 1.033 g/mL, 4.69 mmol) was heated at 80 °C for 1 hour. Then, the reaction mixture was diluted with ethyl acetate (15 mL) and extracted with (3x15 mL) water. The organic layer was dried with anhydrous MgSO₄, filtered, and concentrated. The residue was purified by RP HPLC (basic, column C8) to afford (*R*)-3-hydroxy-1-methyl-3-(3-(2-(2-((1-methyl-1H-pyrazol-5-yl)amino)pyrimidin-4-yl)thiazol-4-yl)phenyl)pyrrolidin-2-one (26.8 mg, 25%). ¹H NMR (500 MHz, DMSO-*d₆*) δ 9.73 (s, 1H), 8.67 (d, *J* = 5.0 Hz, 1H), 8.35 (s, 1H), 8.08 (t, *J* = 1.8 Hz, 1H), 7.94 (dt, *J* = 7.7, 1.8, 1.1 Hz, 1H), 7.60 (d, *J* = 5.0 Hz, 1H), 7.45 (t, *J* = 7.7 Hz, 1H), 7.39 (d, *J* = 1.9 Hz, 1H), 7.36 (dt, *J* = 7.8, 1.9, 1.1 Hz, 1H), 6.36 - 6.31 (m, 1H), 6.10 (s, 1H), 3.73 (s, 3H), 3.51 - 3.44 (m, 1H), 3.42 - 3.37 (m, 1H), 2.87 (s, 3H), 2.43 - 2.35 (m, 1H), 2.32 - 2.24 (m, 1H). MS (ESI⁺): m/z = 448.

### Example 6: (R)-3-(3-(4-(2-Aminopyrimidin-4-yl)thiazol-2-yl)phenyl)-3-hydroxy-1-methylpyrrolidin-2-one

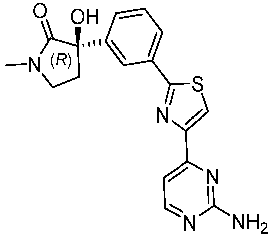

(*R*)-3-(3-(4-(2-((2,4-Dimethoxybenzyl)amino)pyrimidin-4-yl)thiazol-2-yl)phenyl)-3-hydroxy-1-methylpyrrolidin-2-one (Intermediate 22, 400 mg, 0.773 mmol), and DCM (4 mL) were added to a 25 mL round-bottomed flask, and charged with TFA (4 mL). The resulting mixture stirred for 2 h at room temperature. The pH of the reaction mixture was adjusted to pH = 9 with aq. NaOH (2 M) and extracted with DCM (30 mL x 3). The combined extracts were washed with brine (15 mL), dried over anhydrous Na₂SO₄, filtered, and concentrated to dryness *in vacuo* to give a colorless oil. The colorless oil was then subjected to HPLC purification (Xtimate C18, 5 µm, 150 x 40 mm column (Isocratic: 18-48% ACN (v/v) / water (0.05% NH₃H₂O))) to give (*R*)-3-(3-(4-(2-aminopyrimidin-4-yl)thiazol-2-yl)phenyl)-3-hydroxy-1-methylpyrrolidin-2-one (78.9 mg, 28%) as a colorless solid. MS (ESI⁺): m/z =368.1. ¹H NMR (400 MHz, DMSO-*d₆*): δ 8.43 - 8.38 (m, 1H), 8.33 (s, 1H), 8.12 - 8.08 (m, 1H), 7.95 - 7.90 (m, 1H), 7.54 - 7.48 (m, 1H), 7.46 - 7.41 (m, 1H), 7.32 - 7.28 (m, 1H), 6.76 - 6.69 (m, 2H), 6.27 (br s, 1H), 3.53 - 3.43 (m, 2H), 2.87 (s, 3H), 2.43 - 2.33 (m, 1H), 2.33 - 2.22 (m, 1H).

### Example 7: (R)-3-Hydroxy-3-(3-(4-(2-((2-methoxypyridin-3-yl)amino)pyrimidin-4-yl)thiazol-2-yl)phenyl)-1-methylpyrrolidin-2-one

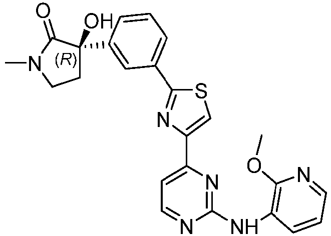

(*R*)-3-(3-(4-Bromothiazol-2-yl)phenyl)-3-hydroxy-1-methylpyrrolidin-2-one (Intermediate 7, 200 mg, 0.566 mmol), *N*-(2-methoxypyridin-3-yl)-4-(tributylstannyl)pyrimidin-2-amine (Intermediate 23, 1.0 g, 2.0 mmol), TEA (170 µL, 1.22 mmol) and toluene (13 mL) were added into a 100 mL round-bottomed flask, which was subsequently evacuated and refilled with argon (x 3), then treated with Pd(PPh₃)₄ (66 mg, 0.057 mmol). The resulting mixture was heated for 16 h at 120 °C. After the reaction mixture was cooled to room temperature, the resulting mixture stirred for 2 h before it was treated with sat. aq. KF (40 mL) over the course of 5 min. The mixture was then extracted with ethyl acetate (50 mL x 3), and the combined extracts washed with brine (300 mL), dried over anhydrous Na₂SO₄, filtered, and concentrated to dryness *in vacuo* to give a nearly black viscous oil. The oil was then subjected to HPLC purification (Xtimate C18 column, 5 µm, 150 x 40 mm; (40-70 % (v/v) ACN/H₂O (NH₃•H₂O))) to afford, after lyophilization, (*R*)-3-Hydroxy-3-(3-(4-(2-((2-methoxypyridin-3-yl)amino)pyrimidin-4-yl)thiazol-2-yl)phenyl)-1-methylpyrrolidin-2-one (51.9 mg, 19%) as a white solid. MS (ESI⁺): m/z = 475.2. ¹H NMR (400 MHz, DMSO-*d*₆): δ 8.66 (d, *J* = 5.0 Hz, 1H), 8.63 - 8.58 (m, 1H), 8.57 (s, 1H), 8.28 (s, 1H), 8.13 (s, 1H), 7.96 (d, *J* = 7.6 Hz, 1H), 7.88 - 7.82 (m, 1H), 7.61 (d, *J* = 5.1 Hz, 1H), 7.55 - 7.50 (m, 1H), 7.47 - 7.43 (m, 1H), 7.11 - 7.04 (m, 1H), 3.97 (s, 3H), 3.47 - 3.46 (m, 2H), 2.87 (s, 3H), 2.40 - 2.35 (m, 1H), 2.32 - 2.26 (m, 1H).

### Example 8: (R)-5-((4-(2-(3-(3-Hydroxy-1-methyl-2-oxopyrrolidin-3-yl)phenyl)thiazol-4-yl)pyrimidin-2-yl)amino)-6-methylnicotinonitrile

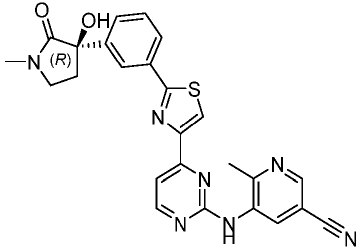

(*R*)-3-(3-(4-Bromothiazol-2-yl)phenyl)-3-hydroxy-1-methylpyrrolidin-2-one (Intermediate 7, 50 mg, 0.14 mmol), 6-methyl-5-((4-(tributylstannyl)pyrimidin-2-yl)amino)nicotinonitrile (Intermediate 24, 71 mg, 0.14 mmol), TEA (39 µL, 0.28 mmol) and toluene (1 mL) were added to a 8 mL glass vial, which was subsequently evacuated and refilled with argon (x 3) and then treated with Pd(PPh₃)₄ (16 mg, 0.014 mmol). The resultant mixture was heated at 120 °C for 16 hours. The reaction vessel was removed from the oil bath and allowed to gradually cool to rt. The mixture was then quenched with sat. aqueous KF (5 mL), extracted with EtOAc (5 mL x 3), and the combined extracts washed with brine (5 mL), dried over anhydrous Na₂SO₄, filtered, and concentrated to dryness *in vacuo* to give a brown solid, which was then purified by HPLC (Boston Prime C18 column, 5 µm, 150 x 30 mm; 40-70% (v/v) ACN (v/v)/H₂O (0.05% NH₃H₂O + 10 mM NH₄HCO₃)) to give, after lyophilization, (*R*)-5-((4-(2-(3-(3-hydroxy-1-methyl-2-oxopyrrolidin-3-yl)phenyl)thiazol-4-yl)pyrimidin-2-yl)amino)-6-methylnicotinonitrile as a colorless solid (46.6 mg, 68%). MS (ESI⁺): m/z = 484.2. ¹H NMR (400 MHz, DMSO-*d₆*) δ 9.38 (s, 1H), 8.68 - 8.61 (m, 2H), 8.57 (d, *J* = 1.9 Hz, 1H), 8.37 (s, 1H), 8.13 - 8.08 (m, 1H), 7.99 - 7.93 (m, 1H), 7.61 (d, *J* = 5.0 Hz, 1H), 7.55 - 7.49 (m, 1H), 7.47 - 7.43 (m, 1H), 6.26 (s, 1H), 3.49 - 3.47 (m, 2H), 2.87 (s, 3H), 2.60 (s, 3H), 2.43 - 2.24 (m, 2H).

### Example 9: (R)-3-(3-(4-(2-((3-Cyclopropoxy-1-(2,2-difluoroethyl)-1H-pyrazol-4-yl)amino)pyrimidin-4-yl)thiazol-2-yl)phenyl)-3-hydroxy-1-methylpyrrolidin-2-one

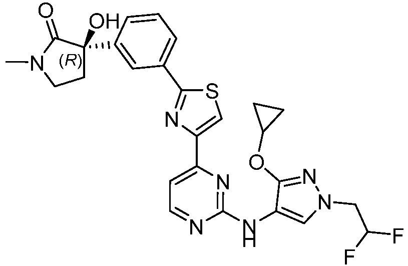

(*R*)-3-Hydroxy-1-methyl-3-(3-(4-(2-(methylsulfonyl)pyrimidin-4-yl)thiazol-2-yl)phenyl)pyrrolidin-2-one (Intermediate 12, 70.6 mg, 0.16 mmol), 3-cyclopropoxy-1-(2,2-difluoroethyl)-1*H*-pyrazol-4-amine (Intermediate 25, 100 mg, 0.49 mmol), 4-methylbenzenesulfonic acid hydrate (28.3 mg, 0.16 mmol) and 1,4-dioxane (3 mL) were added to a 10 mL round-bottomed flask fitted with a reflux condenser and the yellow mixture was heated for 12 h at 115 °C under N₂. The reaction vessel was removed from the oil bath and allowed to gradually cool to room temperature. The reaction mixture was combined with another batch (made from 100 mg 3-cyclopropoxy-1-(2,2-difluoroethyl)-1*H*-pyrazol-4-amine). This yellow mixture was diluted with H₂O (30 mL) and extracted with ethyl acetate (30 mL x 3). The combined organic extracts were washed with brine (30 mL x 2), dried over anhydrous Na₂SO₄, filtered, and concentrated *in vacuo* to give a yellow oil, which was subjected to silica gel chromatography (0-100% EtOAC/pet ether). The product was further purified by HPLC (Boston Green ODS column, 5 µm, 150 x 30 mm; 35-65% (v/v) CH₃CN/H₂O with 0.225% HCOOH) to give, after lyophilization, (*R*)-3-(3-(4-(2-((3-cyclopropoxy-1-(2,2-difluoroethyl)-1*H*-pyrazol-4-yl)amino)pyrimidin-4-yl)thiazol-2-yl)phenyl)-3-hydroxy-1-methylpyrrolidin-2-one as a white solid (41.1 mg). MS (ESI⁺): m/z = 554.3. ¹H NMR (400 MHz, DMSO-*d₆*) δ 8.54 (d, *J* = 4.8 Hz, 2H), 8.46 (s, 1H), 8.12 (s, 1H), 8.01 (s, 1H), 7.96 - 7.94 (m, 1H), 7.56 - 7.49 (m, 1H), 7.45 (d, *J* = 5.2 Hz, 2H), 6.49 - 6.15 (m, 2H), 4.55 - 4.48 (m, 2H), 4.12 - 4.11 (m, 1H), 3.54 - 3.43 (m, 2H), 2.87 (s, 3H), 2.43 - 2.34 (m, 1H), 2.33 - 2.24 (m, 1H), 0.76 - 0.69 (m, 2H), 0.67 - 0.61 (m, 2H).

### Example 10: (R)-3-((4-(2-(3-(3-Hydroxy-1-methyl-2-oxopyrrolidin-3-yl)phenyl)thiazol-4-yl)pyrimidin-2-yl)amino)-N,4-dimethylbenzamide

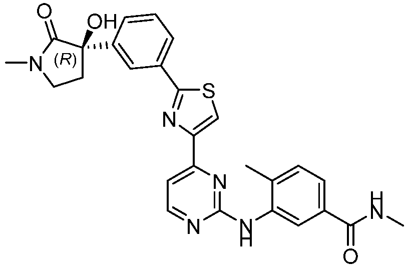

(*R*)-3-Hydroxy-1-methyl-3-(3-(4-(2-(methylsulfonyl)pyrimidin-4-yl)thiazol-2-yl)phenyl)pyrrolidin-2-one (Intermediate 12, 168 mg, 0.41 mmol), 3-amino-*N*,4-dimethylbenzamide (Intermediate 26, 200 mg, 1.22 mmol), 4-methylbenzenesulfonic acid hydrate (116 mg, 0.61 mmol) and isopropanol (3 mL) were added to 10 mL microwave tube. The yellow mixture heated at 120 °C via microwave irradiation for 2 h. The reaction vessel was removed from the microwave reactor and allowed to gradually cool to room temperature. The yellow mixture was diluted with H₂O (10 mL) and extracted with EtOAc (20 mL x 3). The combined organic phase was washed with brine (50 mL x 2), dried over anhydrous Na₂SO₄, filtered, and concentrated *in vacuo* to give a yellow oil, which was initially purified by silica gel chromatography (0-100% EtOAc/pet ether), and subsequently further purified by HPLC (Boston Green ODS column, 5 µm, 150 x 30 mm; 27-57% (v/v) CH₃CN/H₂O with 0.225% HCOOH) to give, after lyophilization, (*R*)-3-((4-(2-(3-(3-hydroxy-1-methyl-2-oxopyrrolidin-3-yl)phenyl)thiazol-4-yl)pyrimidin-2-yl)amino)-*N*,4-dimethylbenzamide as a light yellow solid (47.1 mg, 31%). MS (ESI⁺): m/z = 515.3. ¹H NMR (400 MHz, DMSO-*d₆*) δ 9.01 (s, 1H), 8.56 (d, *J* = 4.8 Hz, 1H), 8.40 - 8.32 (m, 2H), 8.19 (s, 1H), 8.11 (s, 1H), 7.94 (d, *J* = 8.0 Hz, 1H), 7.58 - 7.48 (m, 3H), 7.47 - 7.43 (m, 1H), 7.31 (d, *J* = 8.0 Hz, 1H), 6.28 - 6.20 (s, 1H), 3.51 - 3.45 (m, 2H), 2.87 (s, 3H), 2.78 (d, *J* = 4.8 Hz, 3H), 2.40 - 2.35 (m, 2H), 2.31 (s, 3H).

### Example 11: (R)-3-(3-(4-(2-((1-(2,2-Difluoroethyl)-3-(2-methoxyethoxy)-1H-pyrazol-4-yl)amino)pyrimidin-4-yl)thiazol-2-yl)phenyl)-3-hydroxy-1-methylpyrrolidin-2-one

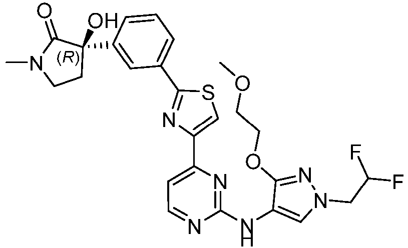

TsOH (150 mg, 0.871 mmol), (*R*)-3-hydroxy-1-methyl-3-(3-(4-(2-(methylsulfonyl)pyrimidin-4-yl)thiazol-2-yl)phenyl)pyrrolidin-2-one (Intermediate 12, 250 mg, 0.581 mmol), 1-(2,2-difluoroethyl)-3-(2-methoxyethoxy)-1*H*-pyrazol-4-amine (Intermediate 27, 488 mg, 2.21 mmol), and anhydrous 1,4-dioxane (15 mL) were added to an oven-dried and nitrogen-purged 50 mL one-necked round-bottomed flask fitted with a reflux condenser, and the resulting mixture was heated for 12 h at 115 °C. The reaction vessel was removed from the oil bath and allowed to gradually cool to rt. The mixture was concentrated to dryness *in vacuo* to give a green semi-solid, which was initially purified by silica gel chromatography (0-8% MeOH/CH₂Cl₂) and then further purified by HPLC (C18 column, 5 µm, 150 x 25 mm; 30-60% (v/v) CH₃CN/H₂O with 0.225% HCOOH) to give, after lyophilization, (*R*)-3-(3-(4-(2-((1-(2,2-difluoroethyl)-3-(2-methoxyethoxy)-1*H*-pyrazol-4-yl)amino)pyrimidin-4-yl)thiazol-2-yl)phenyl)-3-hydroxy-1-methylpyrrolidin-2-one as a yellow solid (57.1 mg, 17%). MS (ESI⁺): m/z = 572.3. ¹H NMR (400 MHz, DMSO-*d*₆) δ 8.55 - 8.54 (m, 2H), 8.48 (s, 1H), 8.12 (s, 1H), 8.08 - 7.85 (m, 2H), 7.56 - 7.48 (m, 1H), 7.48 - 7.40 (m, 2H), 6.49 - 6.16 (m, 2H), 4.55 - 4.49 (m, 2H), 4.32 - 4.20 (m, 2H), 3.70 - 3.61 (m, 2H), 3.52 - 3.44 (m, 1H), 3.44 - 3.39 (m, 1H), 3.29 (s, 3H), 2.87 (s, 3H), 2.42 - 2.33 (m, 1H), 2.33 - 2.25 (m, 1H).

### Example 12: (R)-4-Cyclopropoxy-2-fluoro-5-((4-(2-(3-(3-hydroxy-1-methyl-2-oxopyrrolidin-3-yl)phenyl)thiazol-4-yl)pyrimidin-2-yl)amino)-N-methylbenzamide

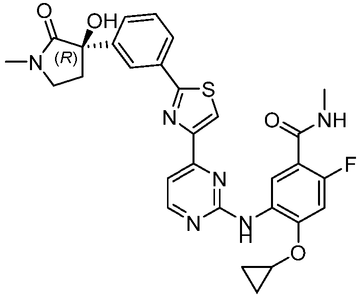

(*R*)-3-Hydroxy-1-methyl-3-(3-(4-(2-(methylsulfonyl)pyrimidin-4-yl)thiazol-2-yl)phenyl)pyrrolidin-2-one (Intermediate 12, 200 mg, 0.483 mmol), 5-amino-4-cyclopropoxy-2-fluoro-N-methylbenzamide (Intermediate 33, 216.4 mg, 0.965 mmol) and TsOH (99.7 mg, 0.579 mmol) were added to a 20 mL microwave tube and the resulting mixture dissolved in IPA (2.5 mL). The resultant mixture was heated at 120 °C via microwave irradiation for 3 hours. The reaction vessel was removed from the microwave reactor and allowed to gradually cool to room temperature. The mixture was concentrated to dryness *in vacuo* to give a green semi-solid, which was initially purified by silica gel chromatography (0-8% MeOH/CH₂Cl₂), and then further purified by HPLC (Welch Xtimate C18 column, 5 µm, 150 x 25 mm; 44-74% (v/v) CH₃CN/H₂O with 0.225% HCOOH) to give, after lyophilization, (*R*)-4-cyclopropoxy-2-fluoro-5-((4-(2-(3-(3-hydroxy-1-methyl-2-oxopyrrolidin-3-yl)phenyl)thiazol-4-yl)pyrimidin-2-yl)amino)-*N-*methylbenzamide as a gray solid (107.7 mg, 38%). MS (ESI⁺): m/z = 575.3. ¹H NMR (400 MHz, DMSO-*d₆*) δ 8.65 - 8.64 (m, 1H), 8.61 - 8.60 (m, 1H), 8.56 (s, 1H), 8.30 (s, 1H), 8.13 (s, 1H), 8.07 - 8.01 (m, 1H), 7.96 - 7.95(m, 1H), 7.57 - 7.50 (m, 1H), 7.52 (d, *J* = 7.6 Hz, 1H), 7.49 - 7.44 (m, 1H), 7.26 - 7.25 (m, 1H), 6.27 (s, 1H), 4.09 - 4.02 (m, 1H), 3.53 - 3.47 (m, 1H), 3.54 - 3.43 (m, 1H), 2.88 (s, 3H), 2.84 (d, *J =* 4.0 Hz, 3H), 2.43 - 2.35 (m, 1H), 2.34 - 2.26 (m, 1H), 0.88 - 0.81 (m, 2H), 0.81 -0.73 (m, 2H).

### Example 13: (R)-5-((4-(2-(3-(3-Hydroxy-1-methyl-2-oxopyrrolidin-3-yl)phenyl)thiazol-4-yl)pyrimidin-2-yl)amino)-1-isobutyl-N-methyl-1H-pyrazole-3-carboxamide

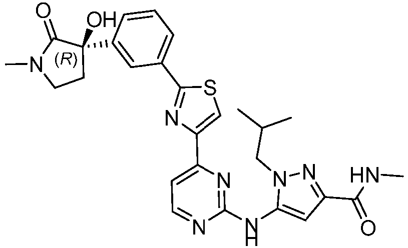

(*R*)-3-(3-(4-(2-Chloropyrimidin-4-yl)thiazol-2-yl)phenyl)-3-hydroxy-1-methylpyrrolidin-2-one (Intermediate 35, 150 mg, 0.388 mmol), 5-amino-1-isobutyl-*N*-methyl-1*H*-pyrazole-3-carboxamide (Intermediate 34, 98.9 mg, 0.504 mmol), Cs₂CO₃ (253 mg, 0.775 mmol), Xantphos (22.4 mg, 0.039 mmol), and 1,4-dioxane (15 mL) were added to an oven-dried and nitrogen-purged 50 mL round-bottomed flask, which was treated with Pd(OAc)₂ (17.4 mg, 0.078 mmol) and then subsequently evacuated and refilled with N₂ (x 3). The resulting mixture was heated for 12 h at 90 °C. The reaction vessel was removed from the oil bath and allowed to gradually cool to room temperature. The reaction mixture was then diluted with EtOAc (60 mL), washed with H₂O (20 mL) and brine (20 mL), dried over anhydrous Na₂SO₄, filtered, and concentrated to dryness in *vacuo* to give a brown solid. The brown solid was initially purified by silica gel chromatography (0-100% EtOAc/pet ether) and then further purified by HPLC (Welch Xtimate C18 column, 5 µm, 150 x 25 mm; 35-65% (v/v) CH₃CN/H₂O with 0.225% HCOOH) to give, after lyophilization, (*R*)-5-((4-(2-(3-(3-hydroxy-1-methyl-2-oxopyrrolidin-3-yl)phenyl)thiazol-4-yl)pyrimidin-2-yl)amino)-1-isobutyl*-N-*methyl-1*H-*pyrazole-3-carboxamide as a white solid (11.8 mg, 38%). MS (ESI⁺): m/z = 547.3. ¹H NMR (400 MHz, MeOH-*d*₄) δ 8.53 (d, *J* = 5.2 Hz, 1H), 8.38 (s, 1H), 8.19 (s, 1H), 7.98 - 7.97 (m, 1H), 7.67(d, *J* = 4.8 Hz, 1H), 7.52- 7.51 (m, 2H), 6.85 (s, 1H), 3.97 - 3.96 (m, 2H), 3.63 - 3.55 (m, 1H), 3.54 - 3.46 (m,1H), 3.00 (s, 3H), 2.92 (s, 3H), 2.57 - 2.48 (m, 1H), 2.47 - 2.38 (m, 1H), 2.35 - 2.24 (m, 1H), 0.91 - 0.89 (m, 6H).

### Example 14: (R)-3-((4-(2-(3-(3-Hydroxy-1-methyl-2-oxopyrrolidin-3-yl)phenyl)thiazol-4-yl)pyrimidin-2-yl)amino)-1,5-dimethylpyridin-2(1H)-one

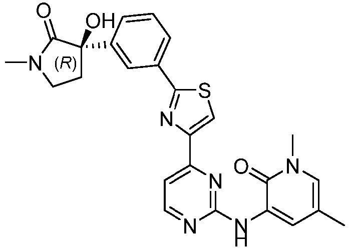

(*R*)-3-Hydroxy-1-methyl-3-(3-(4-(2-(methylsulfinyl)pyrimidin-4-yl)thiazol-2-yl)phenyl)pyrrolidin-2-one (Intermediate 38, 200 mg, 0.483 mmol), 3-amino-1,5-dimethylpyridin-2(1H)-one (Intermediate 36, 200 mg, 1.448 mmol) and TsOH (99.7 mg, 0.579 mmol) were added to a 20 mL microwave tube and the resulting mixture dissolved in IPA (3 mL). The resultant mixture was heated at 120 °C via microwave irradiation for 3 h before cooling to room temperature. The reaction mixture was then concentrated to dryness *in vacuo* to give a brown semi-solid, which was purified by silica gel chromatography (0-10% MeOH/CH₂Cl₂) and then further purified by prep. HPLC (Welch Xtimate C18 column, 5 µm, 150 x 25 mm; 36-66% (v/v) CH₃CN/H₂O with 0.225% HCOOH) to give, after lyophilization, (*R*)-3-((4-(2-(3-(3-hydroxy-1-methyl-2-oxopyrrolidin-3-yl)phenyl)thiazol-4-yl)pyrimidin-2-yl)amino)-1 ,5-dimethylpyridin-2(1*H*)-one as a brown solid (58.1 mg, 24%). MS (ESI⁺): m/z = 489.1. ¹H NMR (400 MHz, DMSO-*d₆*) δ 8.71 (d, *J* = 4.8 Hz, 1H), 8.60 (s, 1H), 8.36 (d, *J* = 1.6 Hz, 1H), 8.33 (s, 1H), 8.12 (s, 1H), 7.96 (d, *J* = 7.6 Hz, 1H), 7.63 (d, *J* = 4.8 Hz, 1H), 7.55 - 7.49 (m, 1H), 7.48 - 7.43 (m, 1H),7.13 (s, 1H), 6.26 (s, 1H), 3.52 (s, 3H), 3.50 - 3.42 (m, 2H), 2.88 (s, 3H), 2.43 - 2.35 (m, 1H), 2.34 - 2.26 (m,1H), 2.15 (s, 3H).

### Example 15: (R)-1-Cyclopentyl-5-((4-(2-(3-(3-hydroxy-1-methyl-2-oxopyrrolidin-3-yl)phenyl)thiazol-4-yl)pyrimidin-2-yl)amino)-N-methyl-1H-pyrazole-3-carboxamide

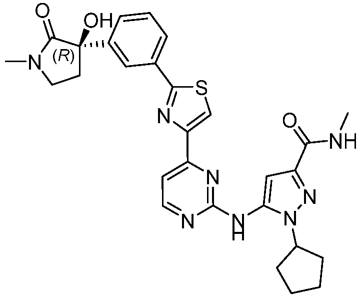

(*R*)-3-(3-(4-(2-Chloropyrimidin-4-yl)thiazol-2-yl)phenyl)-3-hydroxy-1-methylpyrrolidin-2-one (Intermediate 35, 80 mg, 0.207 mmol), 5-amino-1-cyclopentyl-*N*-methyl-1*H-*pyrazole-3-carboxamide (Intermediate 39, 64.6 mg, 0.310 mmol), Cs₂CO₃ (134.8 mg, 0.414 mmol), Xantphos (12 mg, 0.021 mmol), and 1,4-dioxane (5 mL) were added to an oven-dried and nitrogen-purged 10 mL round-bottomed flask. The mixture was treated with Pd(OAc)₂ (9.28 mg, 0.041 mmol) and then subsequently evacuated and refilled with N₂ (x 3). The resultant mixture was heated for 12 h at 90 °C. The reaction vessel was removed from the oil bath and allowed to gradually cool to room temperature. The reaction mixture was then concentrated to dryness *in vacuo* to give a yellow viscous solid. The solid was purified by silica gel chromatography (0-100% EtOAc/pet ether), and then further purified by HPLC (Welch Xtimate C18 column, 5 µm, 150 x 25 mm; 35-65% (v/v) CH₃CN/H₂O with 0.225% HCOOH) to give, after lyophilization, (*R*)-1-cyclopentyl-5-((4-(2-(3-(3-hydroxy-1-methyl-2-oxopyrrolidin-3-yl)phenyl)thiazol-4-yl)pyrimidin-2-yl)amino)-*N*-methyl-1*H*-pyrazole-3-carboxamide as a yellow solid (8.5 mg, 20%). MS (ESI⁺): m/z = 559.3. ¹H NMR (400 MHz, DMSO-*d₆*) δ 9.58 (s, 1H), 8.60 (d, *J =* 4.8 Hz, 1H), 8.31 (s, 1H), 8.10 (s, 1H), 7.99 - 7.83 (m, 2H), 7.58 (d, J = 4.8 Hz, 1H), 7.55 - 7.48 (m, 1H), 7.47 - 7.41 (m, 1H), 6.61 (s, 1H), 6.25 (s, 1H), 4.82 -4.78 (m, 1H), 3.51 - 3.48 (m, 1H), 2.87 (s, 3H), 2.76 (d, *J* = 4.8 Hz, 3H), 2.41 - 2.25 (m, 2H), 2.07 - 1.90 (m, 5H), 1.90 - 1.82 (m, 2H), 1.63 - 1.54 (m, 2H).

### Example 16: (R)-3-Hydroxy-1-methyl-3-(3-(4-(5-methyl-2-((1-methyl-1H-pyrazol-3-yl)amino)pyrimidin-4-yl)thiazol-2-yl)phenyl)pyrrolidin-2-one

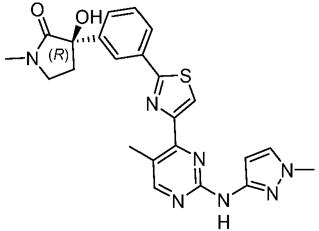

Pd(PPh₃)₄ (12 mg, 0.010 mmol), 4-chloro-5-methyl-N-(1-methyl-1H-pyrazol-3-yl)pyrimidin-2-amine (Intermediate 40, 34 mg, 0.15 mmol), (*R*)-3-hydroxy-1-methyl-3-(3-(4-(trimethylstannyl)thiazol-2-yl)phenyl)pyrrolidin-2-one (Intermediate 41, 45 mg, 0.10 mmol), TEA (31.3 mg, 0.309 mmol), and anhydrous toluene (1 mL) were added to 8 mL sealed tube under N₂. The resultant mixture was heated at 110 °C for 16 hours before cooling to room temperature to give a black solution. The mixture was filtered through a pad of diatomaceous earth and the pad washed with ethyl acetate (10 mL) and concentrated to dryness *in vacuo* to give a yellow oil. The yellow oil was purified by preparative HPLC using a Boston Prime C18 150 x 30 mm x 5 µm column (eluent: 25-55% (v/v) CH₃CN and H₂O with 0.05% NH₃) to afford pure product. The product was suspended in water (10 mL), the mixture frozen using dry ice/EtOH, and then lyophilized to dryness to afford (*R*)-3-Hydroxy-1-methyl-3-(3-(4-(5-methyl-2-((1-methyl-1H-pyrazol-3-yl)amino)pyrimidin-4-yl)thiazol-2-yl)phenyl)pyrrolidin-2-one as a white solid (30 mg, 63%). MS (ESI⁺): m/z = 462.2 [M+H]⁺. ¹H NMR (400 MHz, DMSO-*d₆*) δ 9.61 (s, 1H), 8.42 - 8.38 (m, 2H), 8.10 - 8.05 (m, 1H), 7.95 - 7.88 (m, 1H), 7.61 - 7.43 (m, 3H), 6.72 - 7.68 (m, 1H), 6.24 (s, 1H), 3.75 (s, 3H), 3.52 - 3.45 (m, 2H), 2.87 (s, 3H), 2.55 (s, 3H), 2.40 - 2.25 (m, 2H).

### Example 17: (R)-3-Hydroxy-1-methyl-3-(3-(5-methyl-4-(2-((1-methyl-1H-pyrazol-3-yl)amino)pyrimidin-4-yl)thiazol-2-yl)phenyl)pyrrolidin-2-one

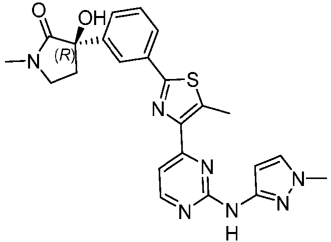

Toluene (3 mL), (*R*)-3-(3-(4-bromo-5-methylthiazol-2-yl)phenyl)-3-hydroxy-1-methylpyrrolidin-2-one (Intermediate 43, 150 mg, 0.408 mmol), *N*-(1-methyl-1*H*-pyrazol-3-yl)-4-(tributylstannyl)pyrimidin-2-amine (Intermediate 42, 190 mg, 0.408 mmol), and Et₃N (114 µL, 0.817 mmol) were added to an oven-dried and N₂-purged 10 mL roundbottom flask, and charged with Pd(PPh₃)₄ (47 mg, 0.041 mmol). The resultant mixture was sparged with N₂ for 5 min, and then heated at 120 °C for 16 hours. After this time, the reaction vessel was removed from oil bath and allowed to gradually cool to rt. The reaction mixture was then quenched with sat. aqueous KF (20 mL), stirred for 1 h at room temperature, and extracted with EtOAc (20 mL x 3). The combined organic extracts were washed with brine (20 mL), dried over anhydrous Na₂SO₄, filtered, and concentrated to dryness *in vacuo* to give a brown oil. The oil was purified by HPLC (Welch Xtimate C18 column, 5 µm, 150 x 30 mm; 33-63% MeCN/water with 0.05% NH₃•H₂O + 10 mM NH₄HCO₃) to give, after lyophilization, (*R*)-3-hydroxy-1-methyl-3-(3-(5-methyl-4-(2-((1-methyl-1*H*-pyrazol-3-yl)amino)pyrimidin-4-yl)thiazol-2-yl)phenyl)pyrrolidin-2-one as a colorless solid (44.6 mg, 23%). MS (ESI⁺): m/z = 462.2. ¹H NMR (400 MHz, DMSO-*d₆*) δ 9.67 (s, 1H), 8.54 (d, *J* = 5.1 Hz, 1H), 8.06 - 8.01 (m, 1H), 7.85 (d, *J* = 7.7 Hz, 1H), 7.58 - 7.56 (m, 1H), 7.52 - 7.45 (m, 2H), 7.43 - 7.39 (m, 1H), 6.56 - 6.50 (m, 1H), 6.23 (s, 1H), 3.76 (s, 3H), 3.52 - 3.48 (m, 2H), 2.93 (s, 3H), 2.86 (s, 3H), 2.40 - 2.34 (m, 1H), 2.32 - 2.24 (m, 1H).

### Example 18 and Example 19: (R)-3-Hydroxy-1-methyl-3-(3-(4-(2-((1-((R)-tetrahydrofuran-3-yl)-1H-pyrazol-4-yl)amino)pyrimidin-4-yl)thiazol-2-yl)phenyl)pyrrolidin-2-one (R)-3-Hydroxy-1-methyl-3-(3-(4-(2-((1-((S)-tetrahydrofuran-3-yl)-1H-pyrazol-4-yl)amino)pyrimidin-4-yl)thiazol-2-yl)phenyl)pyrrolidin-2-one

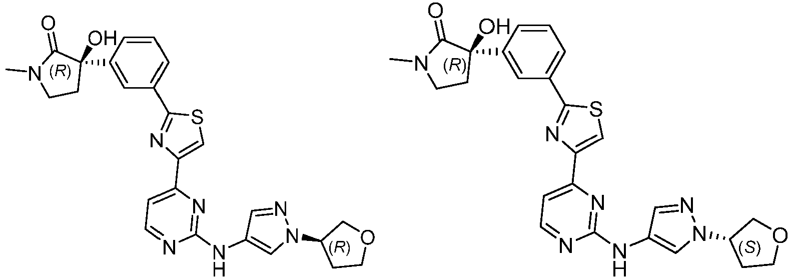

(*R*)-3-Hydroxy-1-methyl-3-(3-(4-(2-(methylsulfonyl)pyrimidin-4-yl)thiazol-2-yl)phenyl)pyrrolidin-2-one (Intermediate 12, 93.7 mg, 0.261 mmol), (*R,S*)-1-(tetrahydrofuran-3-yl)-1*H*-pyrazol-4-amine (100 mg, 0.653 mmol), TsOH (45.0 mg, 0.261 mmol) and anhydrous 1,4-dioxane (5 mL) were added to an oven-dried and nitrogen-purged 40 mL reaction vial, and the resulting mixture was heated for 12 h at 110 °C to give a brown solution. The reaction mixture was purified by HPLC (Welch Xtimate C18 column, 5 µm, 150 x 25 mm; 34-64% (v/v) CH₃CN/H₂O with 0.225% HCOOH) to give (*R*)-3-hydroxy-1-methyl-3-(3-(4-(2-((1-((*R*,*S*)-tetrahydrofuran-3-yl)-1*H-*pyrazol-4-yl)amino)pyrimidin-4-yl)thiazol-2-yl)phenyl)pyrrolidin-2-one as a white solid. The (R) and (S) enantiomers of (*R*)-3-hydroxy-1-methyl-3-(3-(4-(2-((1-((*R,S*)-tetrahydrofuran-3-yl)-1*H*-pyrazol-4-yl)amino)pyrimidin-4-yl)thiazol-2-yl)phenyl)pyrrolidin-2-one were separated by SFC (DAICEL CHIRALPAK IC column, 10 µm, 250 x 30 mm; 50% IPA (containing 0.1% of 25% aq. NH₃)/supercritical CO₂). The first eluting product was designated as diastereomer 1 of (*R*)-3-hydroxy-1-methyl-3-(3-(4-(2-((1-(tetrahydrofuran-3-yl)-1*H*-pyrazol-4-yl)amino)pyrimidin-4-yl)thiazol-2-yl)phenyl)pyrrolidin-2-one (Example 18) (18.0 mg, yellow solid, 16%). MS (ESI⁺): m/z = 504.2. ¹H NMR (400 MHz, DMSO-*d*₆) δ 9.63 (s, 1H), 8.63 - 8.45 (m, 2H), 8.17 - 8.07 (m, 2H), 7.98 - 7.92 (m, 1H), 7.63 (s, 1H), 7.55 - 7.48 (m, 1H), 7.47 - 7.42 (m, 2H), 6.26 (s, 1H), 5.08 (s, 1H), 4.07 - 3.96 (m, 2H), 3.93 -3.77 (m, 2H), 3.56 - 3.41 (m, 2H), 2.87 (s, 3H), 2.44 - 2.21 (m, 4H).

The second eluting product was designated as diastereomer 2 of (*R*)-3-hydroxy-1-methyl-3-(3-(4-(2-((1-(tetrahydrofuran-3-yl)-1*H*-pyrazol-4-yl)amino)pyrimidin-4-yl)thiazol-2-yl)phenyl)pyrrolidin-2-one (Example 19) (yellow solid, 17.9 mg, 16%). MS (ESI⁺): m/z = 504.2. ¹H NMR (400 MHz, DMSO-*d*₆) δ 9.63 (s, 1H), 8.65 -8.45 (m, 2H), 8.16 - 8.06 (m, 2H), 7.99 - 7.93 (m, 1H), 7.63 (s, 1H), 7.57 - 7.49 (m, 1H), 7.47 - 7.41 (m, 2H), 6.26 (s, 1H), 5.08 (s, 1H), 4.04 - 3.80 (m, 4H), 3.52 -3.40 (m, 2H), 2.87 (s, 3H), 2.43 - 2.24 (m, 4H).

### Example 20: (R)-3-(3-(2-(2-((1-(cyclopropylmethyl)-1H-pyrazol-4-yl)amino)pyrimidin-4-yl)thiazol-4-yl)phenyl)-3-hydroxy-1-methylpyrrolidin-2-one

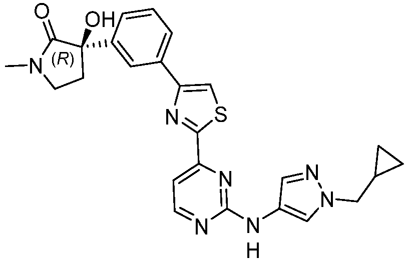

The title compound was prepared using conditions analogous to those described in Example 49, using 1-(cyclopropylmethyl)-1H-pyrazol-4-amine in place of 1,5-dimethyl-1H-pyrazol-3-amine. The compound was purified via FCC (100% DCM increasing to 5% MeOH-DCM). The resulting residue was then subjected to further purification using column C9 under basic conditions to yield the titular compound (89% yield). ¹H NMR (400 MHz, Methanol-*d₄*) δ 8.51 (d, J = 5.0 Hz, 1H), 8.19 - 8.09 (m, 2H), 8.03 (s, 1H), 7.97 - 7.86 (m, 1H), 7.63 (s, 1H), 7.55 - 7.35 (m, 3H), 4.00 (d, J = 7.0 Hz, 2H), 3.65 - 3.44 (m, 2H), 3.00 (s, 3H), 2.61 - 2.37 (m, 2H), 1.29-1.36 (m, 1H), 0.71 - 0.60 (m, 2H), 0.42-0.44 (m, 2H). MS (ESI⁺): m/z = 488.20.

### Example 21: (R)-3-Hydroxy-3-(3-(4-(2-(imidazo[1,2-a]pyridin-3-ylamino)pyrimidin-4-yl)thiazol-2-yl)phenyl)-1-methylpyrrolidin-2-one

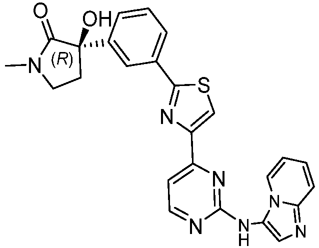

The title compound was prepared using conditions analogous to those described in Example 49, using imidazo[1,2-a]pyridin-3-amine in place of 1,5-dimethyl-1H-pyrazol-3-amine. The compound was purified via RP HPLC (Xtimate C18 150 x 25 mm x 5 µm column; eluent: 29% to 69% (v/v) ACN and H₂O with 0.05% ammonia hydroxide and 10 mM NH₄HCO₃) to afford the titular compound (1%). ¹H NMR (400 MHz, DMSO-*d*₆): δ 8.82 - 8.77 (m, 1H), 8.61 (s, 1H), 8.53 - 8.47 (m, 1H), 8.14 - 8.11 (m, 1H), 8.05 - 8.01 (m, 1H), 7.99 - 7.95 (m, 1H), 7.93 - 7.87 (m, 1H), 7.80 -7.75 (m, 1H), 7.56 - 7.42 (m, 2H), 7.28 - 7.22 (m, 1H), 6.25 (s, 1H), 5.38 - 5.31 (m, 2H), 3.54 - 3.45 (m, 1H), 3.43 - 3.38 (m, 1H), 2.87 (s, 3H), 2.43 - 2.35 (m, 1H), 2.34 - 2.25 (m, 1H). MS (ESI⁺): m/z = 484.2.

### Example 22: (R)-3-Hydroxy-1-methyl-3-(3-(6-(2-((1-methyl-1H-pyrazol-3-yl)amino)pyrimidin-4-yl)pyridin-2-yl)phenyl)pyrrolidin-2-one

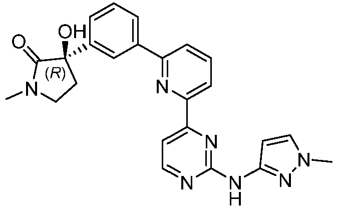

Pd(PPh₃)₄ (33 mg, 0.029 mmol), *N*-(1-methyl-1*H*-pyrazol-3-yl)-4-(trimethylstannyl)pyrimidin-2-amine (Intermediate 1, 214 mg, 0.634 mmol), (*R*)-3-(3-(6-bromopyridin-2-yl)phenyl)-3-hydroxy-1-methylpyrrolidin-2-one (Intermediate 46, 100 mg, 0.288 mmol), TEA (0.118 mL, 0.864 mmol), anhydrous toluene (1 mL), and anhydrous 1,4-dioxane (1 mL) were added to an 8 mL vial under N₂. The resultant mixture was heated at 100 °C for 16 hours before cooling to room temperature. The mixture was concentrated to dryness *in vacuo* to give a yellow oil. The oil was purified by HPLC (Phenomenex C18 column, 3 µm, 75 x 30 mm; 29-59% (v/v) CH₃CN/H₂O with 0.05% NH₃) followed by SFC (DAICEL CHIRALCEL OD-H column, 10 µm, 250 x 30 mm; 50% EtOH (containing 0.1% of 25% aq. NH₃)/supercritical CO₂) to give, after lyophilization, (*R*)-3-hydroxy-1-methyl-3-(3-(6-(2-((1-methyl-1*H*-pyrazol-3-yl)amino)pyrimidin-4-yl)pyridin-2-yl)phenyl)pyrrolidin-2-one as a white solid (60 mg, 47%). MS (ESI⁺): m/z = 442.2. ¹H NMR (400 MHz, DMSO-*d₆*) δ 9.87 (s, 1H), 8.65 (d, *J* = 4.8 Hz, 1H), 8.36 - 8.30 (m, 1H), 8.25 (s, 1H), 8.18 - 8.08 (m, 3H), 7.83 (d, *J* = 5.2 Hz, 1H), 7.66 - 7.59 (m, 1H), 7.55 - 7.42 (m, 2H), 6.73 (d, *J* = 2.0 Hz, 1H), 6.17 (s, 1H), 3.78 (s, 3H), 3.54 - 3.41 (m, 2H), 2.88 (s, 3H), 2.46 - 2.24 (m, 2H).

### Example 23: (R)-3-Hydroxy-3-(3-(6-(2-((3-hydroxypropyl)amino)pyrimidin-4-yl)pyridin-2-yl)phenyl)-1-methylpyrrolidin-2-one

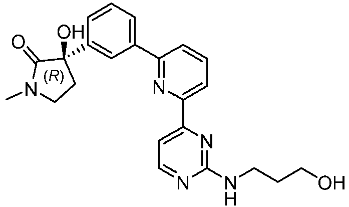

(*R*)-3-Hydroxy-1-methyl-3-(3-(6-(2-(methylsulfinyl)pyrimidin-4-yl)pyridin-2-yl)phenyl)pyrrolidin-2-one (Intermediate 47, 210 mg ,0.514 mmol), 3-aminopropan-1-ol (232 mg, 3.09 mmol) and DIEA (0.548 mL, 3.09 mmol) were added to a 20 mL microwave tube and the resulting mixture was dissolved in IPA (5 mL). The resultant mixture was heated at 120 °C via microwave irradiation for 2 hours to give a yellow solution. After this time, the mixture was cooled to r,t., and concentrated to dryness in *vacuo* to give a yellow oil. The yellow oil was purified by HPLC (Boston Prime C18 column, 5 µm, 150 x 30 mm; 19-39% (v/v) CH₃CN/H₂O with 0.05% HCOOH) to give (R)-3-hydroxy-3-(3-(6-(2-((3-hydroxypropyl)amino)pyrimidin-4-yl)pyridin-2-yl)phenyl)-1-methylpyrrolidin-2-one as a white solid (31.3 mg, 14%). MS (ESI⁺): m/z = 420.2. ¹H NMR (400 MHz, DMSO-*d₆*) δ 8.51 - 8.46 (m, 1H), 8.32 (s, 1H), 8.23 (s, 1H), 8.12 - 8.06 (m, 3H), 7.64 - 7.61 (m, 1H), 7.55 - 7.47 (m, 1H), 7.46 - 7.39 (m, 1H), 7.29 - 7.24 (m, 1H), 6.16 (s, 1H), 4.51 (t, *J* = 5.2 Hz, 1H), 3.58 - 3.41 (m, 6H), 2.88 (s, 3H), 2.41 - 2.25 (m, 2H), 1.82 - 1.69 (m, 2H).

### Example 24 and Example 25: (R)-3-(2-Fluoro-5-(2-(2-((1-methyl-1H-pyrazol-3-yl)amino)pyrimidin-4-yl)thiazol-4-yl)phenyl)-3-hydroxy-1-methylpyrrolidin-2-one and (S)-3-(2-Fluoro-5-(2-(2-((1-methyl-1H-pyrazol-3-yl)amino)pyrimidin-4-yl)thiazol-4-yl)phenyl)-3-hydroxy-1-methylpyrrolidin-2-one

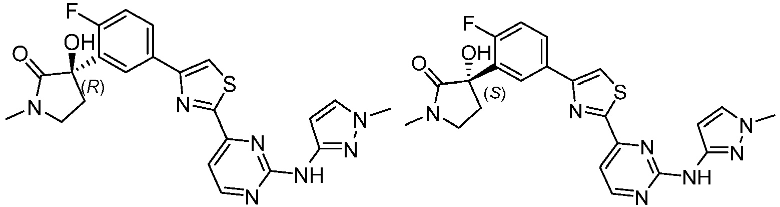

4-(4-Bromothiazol-2-yl)-*N*-(1-methyl-1*H*-pyrazol-3-yl)pyrimidin-2-amine (Intermediate 4, 357 mg, 212 µmol), (*R,S*)-3-(2-fluoro-5-(4,4,5,5-tetramethyl-1,3,2-dioxaborolan-2-yl)phenyl)-3-hydroxy-1-methylpyrrolidin-2-one (Intermediate 48, 100 mg, 240 µmol), Pd(dppf)Cl₂ (17.6 mg, 24.0 µmol), Cs₂CO₃ (235 mg, 720 µmol), anhydrous 1,4-dioxane (4 mL), and H₂O (1 mL) were added to an oven-dried and N₂-sparged 10 mL singlenecked round-bottomed flask. The resulting mixture was heated at 100 °C for 16 h. The reaction vessel was removed from the oil bath, allowed to cool to r.t., and the mixture was treated with H₂O (10 mL) and extracted with DCM (10 mL x 3). The combined extracts were dried over anhydrous Na₂SO₄, filtered, and concentrated *in vacuo* to give a black solid, which was subjected to sillica gel chromatography (eluent: 0-5% methanol/dichloromethane) to give a yellow solid. The solid was purified by HPLC (Boston Green ODS C18 column, 5 µm, 150 x 30 mm; eluent: 26-56% (v/v) CH₃CN and H₂O with 0.225% HCOOH) to give, after lyophillization, (*R,S*)-3-(2-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one as a yellow solid (100 mg, 89%). The (*R*) and (*S*) enantiomers of (*R,S*)-3-(2-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one (100 mg, 213 µmol) were separated by SFC (DAICEL CHIRALCEL OJ-H column, 5 µm, 250 mm x 30 mm; 40% IPA (containing 0.1% of 25% aq. NH₃)/supercritical CO₂). The first eluting product was designated as enantiomer 1, (*R*)-3-(2-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one (Example 24) (yellow solid, 18.9 mg, 18%). MS (ESI⁺): m/z = 466.2. ¹H NMR (400 MHz, DMSO-*d*₆) δ 10.10 (s, 1H), 8.64 (d, *J* = 4.8 Hz, 1H), 8.36 - 8.34 (m, 1H), 8.32 (s, 1H), 8.03 - 7.96 (m, 1H), 7.63 (d, *J* = 2.0 Hz, 1H), 7.50 (d, *J* = 4.8 Hz, 1H), 7.29 - 7.21 (m, 1H), 6.74 (s, 1H), 6.36 (s, 1H), 3.78 (s, 3H), 3.57 - 3.49 (m, 1H), 3.44 - 3.37 (m, 1H), 2.88 - 2.82 (m, 3H), 2.37 - 2.30 (m, 1H), 2.25 - 2.16 (m, 1H).

The second eluting product was designated as enantiomer 2, (*S*)-3-(2-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one (Example 25) (yellow solid, 19.3 mg, 19%). MS (ESI⁺): m/z = 466.2. ¹H NMR (400 MHz, DMSO-*d*₆) δ 10.10 (s, 1H), 8.64 (d, *J =* 5.2 Hz, 1H), 8.36 - 8.34 (m, 1H), 8.32 (s, 1H), 8.03 - 7.98 (m, 1H), 7.63 (d, *J* = 2.0 Hz, 1H), 7.50 (d, *J* = 5.2 Hz, 1H), 7.29 - 7.24 (m, 1H), 6.74 (s, 1H), 6.36 (s, 1H), 3.78 (s, 3H), 3.57 - 3.51 (m, 1H), 3.42 - 3.38 (m, 1H), 2.86 (s, 3H), 2.37 - 2.29 (m, 1H), 2.23 - 2.18 (m, 1H).

### Example 26 and Example 27: (R)-3-(3-Fluoro-5-(2-(2-((1-methyl-1H-pyrazol-3-yl)amino)pyrimidin-4-yl)thiazol-4-yl)phenyl)-3-hydroxy-1-methylpyrrolidin-2-one and (S)-3-(3-Fluoro-5-(2-(2-((1-methyl-1H-pyrazol-3-yl)amino)pyrimidin-4-yl)thiazol-4-yl)phenyl)-3-hydroxy-1-methylpyrrolidin-2-one

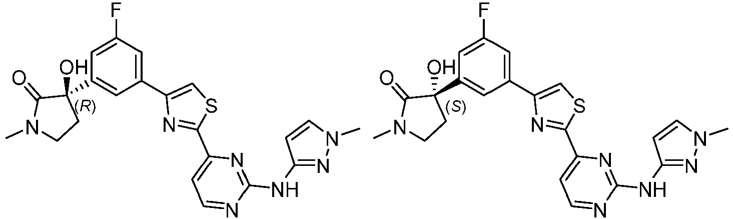

(*R,S*)-3-(3-Chloro-5-fluorophenyl)-3-hydroxy-1-methylpyrrolidin-2-one (Intermediate 49, 250 mg, 1.03 mmol), 4,4,4',4',5,5,5',5'-octamethyl-2,2'-bi(1,3,2-dioxaborolane) (313 mg, 1.23 mmol), Brettphos-Pd-G3 (93 mg, 0.10 mmol), KOAc (302 mg, 3.08 mmol), and anhydrous 1,4-dioxane (20 mL) were added to 100 mL three-necked round-bottomed flask fitted with a reflux condenser and under an atmosphere of nitrogen. The resultant mixture was heated at 100 °C for 16 h and turned black. The mixture was cooled to r.t. and treated with Pd(dppf)Cl₂ (75 mg, 0.10 mmol), Cs₂CO₃ (1.00 g, 3.08 mmol), 4-(4-bromothiazol-2-yl)-N-(1-methyl-1H-pyrazol-3-yl)pyrimidin-2-amine (Intermediate 4, 277 mg, 0.821 mmol), H₂O (5 mL), and anhydrous 1,4-dioxane (10 mL), and the resulting mixture was heated at 100 °C for 2 h. The reaction mixture was cooled to r.t., diluted with water (5 mL), and extracted with EtOAc (25 mL x 3). The combined organic extracts were washed with brine (20 mL), dried over anhydrous Na₂SO₄, and filtered. The filtrate was concentrated to give a yellow oil which was purified by sillica gel chromatography (10% MeOH/CH₂Cl₂) to give (*R,S*)-3-(3-fluoro-5-(2-(2-((1-methyl-1*H-*pyrazol-3-yl)amino)pyrimidin-4-yl)thiazol-4-yl)phenyl)-3-hydroxy-1-methylpyrrolidin-2-one as a yellow solid (300 mg, 63%). The (*R*) and (*S*) enantiomers of (*R,S*)-3-(3-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one were separted by SFC over a DAICEL CHIRALPAK AS column, 10 µm, 250 x 30 mm, 50% EtOH (containing 0.1% of 25% aq. NH₃): supercritical CO₂. The first elulting product was designated as enantiomer 1 of 3-(3-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one and was further purified by preparative HPLC using a Boston Green ODS column, 5 µm, 150 x 30 mm; 35-65% (v/v) CH₃CN and H₂O with 0.05% HCOOH to provide Example 26 (yellow solid, 84.4 mg, 27%). MS (ESI⁺): m/z = 466.1 [M+H]⁺. ¹H NMR (400 MHz, DMSO-*d*₆) δ 10.09 (s, 1H), 8.64 (d, *J* = 5.2 Hz, 1H), 8.50 (s, 1H), 7.91 (d, *J* = 1.6 Hz, 1H), 7.83 - 7.77 (m, 1H), 7.66 - 7.61 (m, 1H), 7.53 (d, *J* = 4.8 Hz, 1H), 7.23 - 7.16 (m, 1H), 6.73 (s, 1H), 6.28 (s, 1H), 3.78 (s, 3H), 3.50 - 3.41 (m, 2H), 2.87 (s, 3H), 2.46 - 2.39 (m, 1H), 2.35 - 2.24 (m, 1H).

The second eluting product was designated as enantiomer 2 of 3-(3-fluoro-5-(2-(2-((1-methyl-1*H*-pyrazol-3-yl)amino)pyrimidin-4-yl)thiazol-4-yl)phenyl)-3-hydroxy-1-methylpyrrolidin-2-one. It was further purified by preparative HPLC using a Boston Green ODS column, 5 µm, 150 x 30 mm (35-65% (v/v) CH₃CN and H₂O with 0.05% HCOOH) to afford Example 27 as a yellow solid (98.9 mg, 32%). MS (ESI⁺): m/z = 466.1 [M+H]⁺. ¹H NMR (400 MHz, DMSO-*d*₆) δ 10.09 (s, 1H), 8.64 (d, *J* = 5.2 Hz, 1H), 8.50 (s, 1H), 7.91 (s, 1H), 7.82 - 7.77 (m, 1H), 7.65 - 7.61 (m, 1H), 7.53 (d, *J* = 5.2 Hz, 1H), 7.22 - 7.16 (m, 1H), 6.73 (s, 1H), 6.28 (s, 1H), 3.78 (s, 3H), 3.49 - 3.40 (m, 2H), 2.87 (s, 3H), 2.46 - 2.39 (m, 1H), 2.34 - 2.27 (m, 1H).

### Example 28 and Example 29: (R)-3-(4-Fluoro-3-(2-(2-((1-methyl-1H-pyrazol-3-yl)amino)pyrimidin-4-yl)thiazol-4-yl)phenyl)-3-hydroxy-1-methylpyrrolidin-2-one and (S)-3-(4-Fluoro-3-(2-(2-((1-methyl-1H-pyrazol-3-yl)amino)pyrimidin-4-yl)thiazol-4-yl)phenyl)-3-hydroxy-1-methylpyrrolidin-2-one

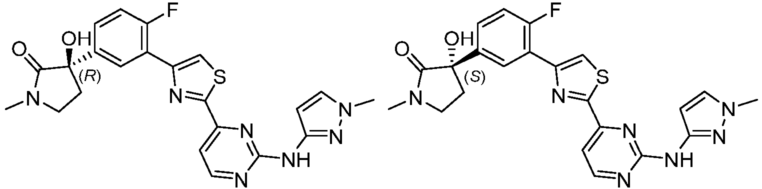

(*R,S*)-3-(3-Chloro-4-fluorophenyl)-3-hydroxy-1-methylpyrrolidin-2-one (Intermediate 50, 250 mg, 1.03 mmol), 4,4,4',4',5,5,5',5'-octamethyl-2,2'-bi(1,3,2-dioxaborolane) (313 mg, 1.23 mmol), Brettphos-Pd-G3 (93 mg, 0.10 mmol), KOAc (302 mg, 3.08 mmol) and 1,4-dioxane (20 mL) were added to a 100 mL three-necked round-bottomed flask fitted with a reflux condenser and under an atmosphere of nitrogen. The resultant mixture was heated at 100 °C for 16 h and turned black. The mixture was cooled to rt and treated with Pd(dppf)Cl₂ (75 mg, 0.10 mmol), Cs₂CO₃ (1.00 g, 3.08mmol), 4-(4-bromothiazol-2-yl)-N-(1-methyl-1H-pyrazol-3-yl)pyrimidin-2-amine (Intermediate 4, 277 mg, 0.821 mmol), H₂O (5 mL) and anhydrous 1,4-dioxane (10 mL) under an atmosphere of nitrogen. The resultant mixture was heated at 100 °C for 2 h and turned black. The reaction was diluted with water (5 mL), and extracted with EtOAc (25 mL x 3). The combined organics were washed with brine (20 mL), dried over anhydrous Na₂SO₄ and filtered. The filterate was concentrated to give a yellow oil which was subjected to sillica gel chromatography (10% EtOH/EtOAc) to give (*R,S*)-3-(4-fluoro-3-(2-(2-((1-methyl-1H-pyrazol-3-yl)amino)pyrimidin-4-yl)thiazol-4-yl)phenyl)-3-hydroxy-1-methylpyrrolidin-2-one as a yellow solid. The (*R*) and (*S*) enantiomers of (*R,S*)-3-(4-fluoro-3-(2-(2-((1-methyl-1H-pyrazol-3-yl)amino)pyrimidin-4-yl)thiazol-4-yl)phenyl)-3-hydroxy-1-methylpyrrolidin-2-one were separated by SFC over a DAICEL CHIRALPAK AS column, 10 µm, 250 x 30 mm, (45% EtOH (containing 0.1% of 25% aq. NH₃): supercritical CO₂). The first eluting product was designated as enantiomer 1 of 3-(4-fluoro-3-(2-(2-((1-methyl-1H-pyrazol-3-yl)amino)pyrimidin-4-yl)thiazol-4-yl)phenyl)-3-hydroxy-1-methylpyrrolidin-2-one (Example 28) (42.1 mg, 21%). MS (ESI⁺): m/z = 466.1 [M+H]⁺. ¹H NMR (400 MHz, DMSO-*d*₆) δ 10.10 (s, 1H), 8.66 (d, *J* = 5.2 Hz, 1H), 8.32 - 8.20 (m, 2H), 7.64 (d, *J* = 2.0 Hz, 1H), 7.49 (d, *J* = 4.8 Hz, 1H), 7.42 - 7.30 (m, 2H), 6.73 (s, 1H), 6.20 (s, 1H), 3.78 (s, 3H), 3.54 - 3.41 (m, 2H), 2.87 (s, 3H), 2.39 - 2.26 (m, 2H).

The second eluting product was designated as enantiomer 2 of 3-(4-fluoro-3-(2-(2-((1-methyl-1H-pyrazol-3-yl)amino)pyrimidin-4-yl)thiazol-4-yl)phenyl)-3-hydroxy-1-methylpyrrolidin-2-one (Example 29) (yellow solid, 27.1 mg, 13%). MS (ESI⁺): m/z = 466.2. ¹H NMR (400 MHz, DMSO-*d*₆) δ 10.09 (s, 1H), 8.66 (d, *J* = 5.2 Hz, 1H), 8.33 - 8.21 (m, 2H), 7.64 (s, 1H), 7.49 (d, *J* = 4.8 Hz, 1H), 7.43 - 7.26 (m, 2H), 6.73 (s, 1H), 6.20 (s, 1H), 3.78 (s, 3H), 3.52 - 3.39 (m, 2H), 2.87 (s, 3H), 2.40 - 2.26 (m, 2H).

### Example 30: (R,S)-3-Hydroxy-1-methyl-3-(2-(1-(2-((1-methyl-1H-pyrazol-3-yl)amino)pyrimidin-4-yl)-1H-pyrazol-3-yl)pyridin-4-yl)pyrrolidin-2-one

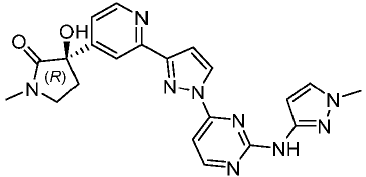

Step A: (*R,S*)-3-(4-(3-hydroxy-1-methyl-2-oxopyrrolidin-3-yl)pyridin-2-yl)-1H-pyrazole. A mixture of (*R,S*)-tert-butyl 3-(4-(3-hydroxy-1-methyl-2-oxopyrrolidin-3-yl)pyridin-2-yl)-1H-pyrazole-1-carboxylate (Intermediate 57, 22 mg, 0.0614 mmol), TFA (0.5 mL, 1.49 g/mL, 6.534 mmol), and DCM (1 mL) was stirred at rt for 3 h. The resulting mixture was concentrated to dryness and used directly in the next step without further purification. MS (ESI⁺): m/z = 259.05.

Step B: (*R,S*)-3-hydroxy-1-methyl-3-(2-(1-(2-((1-methyl-1H-pyrazol-3-yl)amino)pyrimidin-4-yl)-1H-pyrazol-3-yl)pyridin-4-yl)pyrrolidin-2-one. A mixture of (R,S)-3-(4-(3-hydroxy-1-methyl-2-oxopyrrolidin-3-yl)pyridin-2-yl)-1H-pyrazole ( 15.8 mg, 0.061 mmol), 4-chloro-N-(1-methylpyrazol-3-yl)pyrimidin-2-amine (18.0 mg, 0.0856 mmol), Cs₂CO₃ (99.7 mg, 0.306 mmol) and DMF (2 mL) was heated at 100 °C for 2 hr. The resulting mixture was cooled to rt, diluted with water, and extracted with ethyl acetate. The organic extracts were dried with anhydrous MgSO₄, filtered, and concentrated. The residue was purified by RP HPLC (acidic conditions, column C1) to afford (*R,S*)-3-hydroxy-1-methyl-3-(2-(1-(2-((1-methyl-1H-pyrazol-3-yl)amino)pyrimidin-4-yl)-1H-pyrazol-3-yl)pyridin-4-yl)pyrrolidin-2-one (17.7 mg, 67%, as its TFA salt) as a yellow powder. ¹H NMR (500 MHz, DMSO-*d₆*) δ 10.11 (s, 1H), 8.64 (d, *J* = 2.7 Hz, 1H), 8.63 (d, *J* = 5.6 Hz, 1H), 8.57 (d, *J* = 5.4 Hz, 1H), 8.18 (d, *J* = 1.6 Hz, 1H), 7.62 (d, *J =* 2.3 Hz, 1H), 7.38 (dd, *J* = 5.2, 1.8 Hz, 1H), 7.35 (d, *J* = 5.4 Hz, 1H), 7.22 (d, *J* = 2.7 Hz, 1H), 6.63 (d, *J* = 2.2 Hz, 1H), 3.78 (s, 3H), 2.88 (s, 3H), 2.40 (ddd, *J* = 13.3, 7.9, 5.4 Hz, 1H), 2.29 (ddd, *J* = 13.3, 8.0, 5.0 Hz, 1H). MS (ESI⁺): m/z = 432.3.

### Example 31: (R,S)-3-Hydroxy-1-methyl-3-(6'-(2-((1-methyl-1H-pyrazol-3-yl)amino)pyrimidin-4-yl)-[2,2'-bipyridin]-4-yl)pyrrolidin-2-one

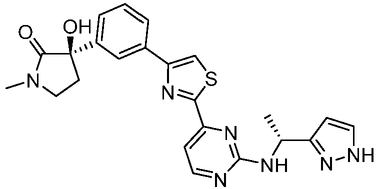

A mixture of (*R,S*)-3-(6'-chloro-[2,2'-bipyridin]-4-yl)-3-hydroxy-1-methylpyrrolidin-2-one (Intermediate 58, 45 mg, 0.148 mmol), N-(1-methyl-1H-pyrazol-3-yl)-4-(trimethylstannyl)pyrimidin-2-amine (Intermediate 1, 75.1 mg, 0.222 mmol), [1,1'-bis(diphenylphosphino)ferrocene]dichloropalladium(ii) (21.83 mg, 0.0296 mmol), cuprous iodide (11.286 mg, 0.0593 mmol) and DMF (0.5 mL) was heated at 130 °C in a microwave reactor for 1 h. The mixture was cooled to rt, diluted with DMSO, filtered through a syringe filter, and purified by RP HPLC (basic, column C2) to afford (*R,S*)-3-hydroxy-1-methyl-3-(6'-(2-((1-methyl-1H-pyrazol-3-yl)amino)pyrimidin-4-yl)-[2,2'-bipyridin]-4-yl)pyrrolidin-2-one (16.2 mg, 25%) as a light yellow powder. ¹H NMR (600 MHz, DMSO-*d₆*) δ 9.86 (s, 1H), 8.72 - 8.65 (m, 2H), 8.63 (dd, *J* = 1.9, 0.8 Hz, 1H), 8.53 (dd, *J* = 7.8, 1.0 Hz, 1H), 8.43 (dd, *J* = 7.8, 1.1 Hz, 1H), 8.19 (t, *J* = 7.8 Hz, 1H), 7.84 (d, *J* = 5.0 Hz, 1H), 7.62 (d, *J* = 2.2 Hz, 1H), 7.41 (dd, *J* = 5.1, 1.8 Hz, 1H), 6.73 (d, *J* = 2.2 Hz, 1H), 6.46 (s, 1H), 3.78 (s, 3H), 3.57 - 3.49 (m, 1H), 3.48 - 3.42 (m, 1H), 2.91 (s, 3H), 2.45 - 2.37 (m, 1H), 2.37 - 2.29 (m, 1H). MS (ESI⁺): m/z = 443.1.

### Example 32: (R,S)-3-Hydroxy-1-methyl-3-(2-(4-methyl-1-(2-((1-methyl-1H-pyrazol-3-yl)amino)pyrimidin-4-yl)-1H-pyrazol-3-yl)pyridin-4-yl)pyrrolidin-2-one

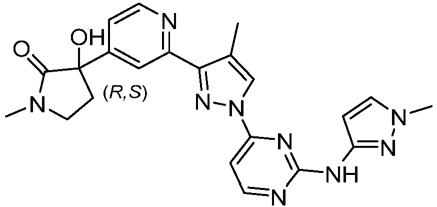

Step A: (*R,S*)-3-hydroxy-1-methyl-3-(2-(4-methyl-1H-pyrazol-3-yl)pyridin-4-yl)pyrrolidin-2-one. A mixture of (*R,S*)-3-(2-bromopyridin-4-yl)-3-hydroxy-1-methylpyrrolidin-2-one (Intermediate 51, 114 mg, 0.42 mmol), 4-methylpyrazole-5-boronic acid pinacol ester (131 mg, 0.631 mmol), [1,1'-bis(diphenylphosphino)ferrocene]dichloropalladium(ii) (31.0 mg, 0.042 mmol), K₂CO₃ (1.051 mL, 1 M in water, 1.051 mmol) and 1,4-dioxane (2 mL) was heated at 80 °C for 5 h and then stirred at rt for 16 h. The mixture was diluted with water and extracted with ethyl acetate. The organic extract was dried with anhydrous MgSO₄, filtered, and concentrated to afford (*R,S*)-3-hydroxy-1-methyl-3-(2-(4-methyl-1H-pyrazol-3-yl)pyridin-4-yl)pyrrolidin-2-one (100 mg, 87%) which was used in the next step without further purification. MS (ESI⁺): m/z = 273.1.

Step B: (*R,S*)-3-hydroxy-1-methyl-3-(2-(4-methyl-1-(2-((1-methyl-1H-pyrazol-3-yl)amino)pyrimidin-4-yl)-1H-pyrazol-3-yl)pyridin-4-yl)pyrrolidin-2-one. A mixture of (*R,S*)-3-hydroxy-1-methyl-3-(2-(4-methyl-1H-pyrazol-3-yl)pyridin-4-yl)pyrrolidin-2-one (100 mg, 0.367 mmol), 4-chloro-N-(1-methylpyrazol-3-yl)pyrimidin-2-amine (77.0 mg, 0.367 mmol) and Cs₂CO₃ (598.3 mg, 1.836 mmol) in DMF (2 mL) was heated at 100 °C for 2 hr. The mixture was cooled to rt, diluted with water, and extracted with ethyl acetate. The organic extract was dried with anhydrous MgSO₄, filtered, and concentrated. The residue was dissolved in DMSO, filtered through a syringe filter, and purified by RP HPLC (basic conditions, column C2) to afford (*R,S*)-3-hydroxy-1-methyl-3-(2-(4-methyl-1-(2-((1-methyl-1H-pyrazol-3-yl)amino)pyrimidin-4-yl)-1H-pyrazol-3-yl)pyridin-4-yl)pyrrolidin-2-one (12.5 mg, 7%) as a white powder.

### Example 33 and Example 34: (R)-3-Hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one and (S)-3-Hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one

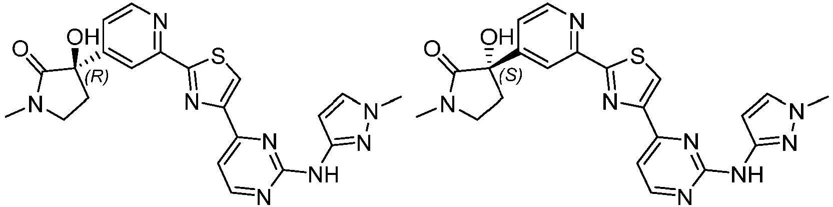

A mixture of (*R,S*)-3-(2-(4-bromothiazol-2-yl)pyridin-4-yl)-3-hydroxy-1-methylpyrrolidin-2-one (Intermediate 59, 17.3 mg, 0.0488 mmol), N-(1-methyl-1H-pyrazol-3-yl)-4-(trimethylstannyl)pyrimidin-2-amine (Intermediate 1, 29.7 mg, 0.0879 mmol), tetrakis(triphenylphosphine)palladium(0) (5.6 mg, 0.0049 mmol) and DMF (0.5 mL) was heated at 130 °C in a microwave reactor for 2 h. The resulting mixture was cooled to rt, diluted with DMSO, passed through a syringe filter, and initially purified by RP HPLC (acidic conditions, column C1) to provide (*R,S*)-3-hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one. The (*R*) and (*S*) enantiomers of (*R,S*)-3-hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one were separated by chiral SFC (Stationary phase: CHIRALPAK IH3, 5 µm, 250 x 21 mm, Mobile phase: 35% methanol, 65% CO₂), Flow Rate 73 mg/min, Monitor at 220nm) to afford the second eluting isomer which was designated as enantiomer 1 of 3-hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one (Example 33) (2.1 mg, 10%). ¹H NMR (500 MHz, DMSO-*d₆*) δ 9.80 (s, 1H), 8.63 (dd, *J* = 5.2, 0.8 Hz, 1H), 8.58 (d, *J* = 5.0 Hz, 1H), 8.53 (s, 1H), 8.31 - 8.26 (m, 1H), 7.58 (d, *J* = 2.2 Hz, 1H), 7.50 (d, *J* = 5.0 Hz, 1H), 7.48 - 7.41 (m, 1H), 6.75 (s, 1H), 6.49 (s, 1H), 3.77 (s, 3H), 3.57 - 3.50 (m, 1H), 3.48 - 3.41 (m, 1H), 2.88 (s, 3H), 2.44 - 2.37 (m, 1H), 2.35 - 2.25 (m, 1H). MS (ESI⁺): m/z = 449.2.

The first eluting isomer was designated as enantiomer 2 of 3-hydroxy-1-methyl-3-(2-(4-(2-((1-methyl-1H-pyrazol-3-yl)amino)pyrimidin-4-yl)thiazol-2-yl)pyridin-4-yl)pyrrolidin-2-one (Example 34) (1.6 mg, 7%). ¹H NMR (500 MHz, DMSO-*d₆*) δ 9.80 (s, 1H), 8.63 (dd, *J* = 5.2, 0.8 Hz, 1H), 8.58 (d, *J* = 4.9 Hz, 1H), 8.53 (s, 1H), 8.30 (dd, *J* = 1.8, 0.8 Hz, 1H), 7.58 (d, *J* = 2.3 Hz, 1H), 7.50 (d, *J* = 5.0 Hz, 1H), 7.46 (dd, *J* = 5.1, 1.8 Hz, 1H), 6.75 (s, 1H), 6.49 (s, 1H), 3.77 (s, 3H), 3.57 - 3.48 (m, 1H), 3.48 - 3.40 (m, 1H), 2.88 (s, 3H), 2.44 - 2.37 (m, 1H), 2.34 - 2.25 (m, 1H). MS (ESI⁺) = 449.2.

### Example 35 and Example 36: (R)-4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one and (S)-4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one

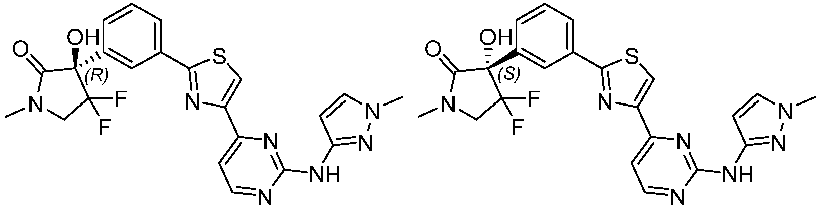

A mixture of (*R,S*)-3-(3-(4-bromothiazol-2-yl)phenyl)-4,4-difluoro-3-hydroxy-1-methylpyrrolidin-2-one (Intermediate 62, 100 mg, 0.257 mmol), N-(1-methyl-1H-pyrazol-3-yl)-4-(trimethylstannyl)pyrimidin-2-amine (Intermediate 1, 112 mg, 0.334 mmol), Pd(PPh₃)₄ (44.5 mg, 0.0385 mmol), and 1,4-dioxane (1.3 mL) was heated at 100 °C for 16 h. The resulting mixture was cooled to rt, concentrated, and purified by FCC (0-100% THF in hexanes) to afford (*R,S*)-4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one as a yellow oil. The (*R*) and (*S*) enantiomers of (*R,S*)-4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one were separated by SFC (Stationary phase: CHIRALPAK IH, 5 µm, 250 x 21 mm, Mobile phase: 20% methanol, 80% CO₂) to afford a second eluting peak which was designated as enantiomer 1 of 4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one (Example 35) (yellow powder, 15 mg, 12%). ¹H NMR (500 MHz, MeOH-*d*₄) δ 8.56 - 8.49 (m, 1H), 8.41 (s, 1H), 8.23 - 8.15 (m, 1H), 8.14 - 8.01 (m, 1H), 7.66 - 7.45 (m, 4H), 6.76 - 6.66 (m, 1H), 4.02 - 3.73 (m, 5H), 3.10 (s, 3H). ¹⁹F NMR (471 MHz, MeOH-*d*₄) δ -112.82 (d, *J* = 232.4 Hz), -119.44 (d, *J* = 232.4 Hz).

The first eluting peak was designated as enantiomer 2 of 4,4-difluoro-3-hydroxy-1-methyl-3-(3-(4-(2-((1-methyl-1H-pyrazol-3-yl)amino)pyrimidin-4-yl)thiazol-2-yl)phenyl)pyrrolidin-2-one (Example 36) (yellow powder, 20 mg, 16%). ¹H NMR (400 MHz, MeOH-*d*₄) δ 8.43 (d, *J* = 5.1 Hz, 1H), 8.31 (s, 1H), 8.10 - 8.05 (m, 1H), 7.99 - 7.94 (m, 1H), 7.54 - 7.38 (m, 4H), 6.67 - 6.59 (m, 1H), 3.91 - 3.67 (m, 5H), 3.01 (s, 3H). ¹⁹F NMR (376 MHz, MeOH-*d*₄) δ -112.79 (d, *J* = 232.7 Hz), -119.45 (d, *J* = 232.8 Hz).

### Example 37 and Example 38: (3R,5S)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one and (3S,5R)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one

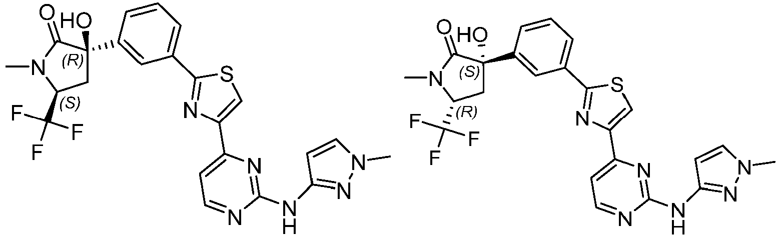

Step A. Ethyl 4-hydroxy-1-methyl-5-oxo-2-(trifluoromethyl)-2,5-dihydro-1H-pyrrole-3-carboxylate. To a solution of ethyl 4,4,4-trifluoro-3-(methylamino)butanoate (2400 g, 12.05 mol) in 2-MeTHF (24000 mL) was added t-BuOK (1.35 kg, 12.05 mol) and diethyl oxalate (1.76 kg, 12.05 mol, 1.65 L) at 25 °C under N₂ atmosphere. The mixture was heated at 60 °C for 3 hr under a N₂ atmosphere. The reaction mixture was quenched by the addition of saturated aqueous NH₄Cl (10.0 L) at 25 °C, and the pH was adjusted to pH = 2-3 with 1 M aqueous HCl (9.00 L). The resulting mixture was extracted with EtOAc (5.00 L) twice. The combined organic layers were washed with brine (15.0 L), dried over anhydrous Na₂SO₄, filtered and concentrated under reduced pressure to give a residue. The residue was purified by column chromatography (DCM/MeOH 0% to 3%) to give the title compound as a brown oil (4.20 kg, 16.59 mol, 68.8%). ¹H NMR (400 MHz, chloroform-*d*) δ 4.63 (q, *J* = 5.0 Hz, 1H), 4.24-4.42 (m, 4H), 3.10-3.14 (m, 3H), 1.19-1.26 (m, 2H).

Step B.1-Methyl-5-(trifluoromethyl)pyrrolidine-2,3-dione. A yellow mixture of ethyl 4-hydroxy-1-methyl-5-oxo-2-(trifluoromethyl)-2,5-dihydro-1H-pyrrole-3-carboxylate (2000 g, 7.90 mol) in HCl (10.96 kg, 108.23 mol, 10.75 L, 36% aqueous solution) was heated at 110 °C for 16 hrs. The reaction mixture was cooled to 20 °C and extracted with isopropylacetate (5000 mL x 8). The combined organic layers were washed with brine (10000 mL), dried over anhydrous Na₂SO₄, filtered and concentrated under reduced pressure to give a residue. The product was triturated with MTBE (5000 mL) at 25 °C for 3 hrs. The solid was filtered, washed with MTBE (1000 mL) and dried under high vacuum to afford the product as a yellow solid (1.15 kg, 6.35 mol, 40.2%). ¹H NMR (400 MHz, chloroform-*d*) δ 4.28-4.42 (m, 1H), 3.28 (s, 3H), 2.87-3.01 (m, 1H), 2.71-2.83 (m, 1H).

Step C. 3-(3-Bromophenyl)-3-hydroxy-1-methyl-5-(trifluoromethyl)pyrrolidin-2-one. (3-Bromophenyl)lithium (0.73 M, 0.91 mL, 0.33 mmol) was added dropwise to a solution of 1-methyl-5-(trifluoromethyl)pyrrolidine-2,3-dione (100 mg, 0.55 mmol) in THF (1.84 mL) at -78 °C over 5 minutes before warming the reaction mixture to 0 °C. After stirring for 90 minutes at 0 °C, the reaction was quenched with NH₄Cl (sat. aqueous, 1 mL). The resulting mixture was partitioned between water and EtOAc, the organic portion was separated, dried over anhydrous sodium sulfate, filtered and evaporated to dryness. The product was purified by flash column chromatography, with 0% to 100% EtOAc in hexanes over 20 minutes as the eluent to afford 3-(3-bromophenyl)-3-hydroxy-1-methyl-5-(trifluoromethyl)pyrrolidin-2-one as a yellow oil (118 mg, 63%).

Step D. 3-hydroxy-1-methyl-3-(3-(4,4,5,5-tetramethyl-1,3,2-dioxaborolan-2-yl)phenyl)-5-(trifluoromethyl)pyrrolidin-2-one. To a 5 mL microwave vial was added 3-(3-bromophenyl)-3-hydroxy-1-methyl-5-(trifluoromethyl)pyrrolidin-2-one (118 mg, 0.35 mmol), bis(pinacolato)diboron (124 mg, 0.49 mmol), potassium acetate (137 mg, 1.4 mmol), and 1,4-dioxane (1.75 mL). This solution was sparged with nitrogen for 10 minutes followed by the addition of XPhos Pd G2 (27 mg, 0.03 mmol). The vial was then sealed and heated at 110 °C for 2.5 hours. The dark solution was filtered through a syringe filter and concentrated to dryness to yield a dark brown oil. The resulting oil was purified by flash column chromatography, running 0% to 100% EtOAc in hexanes over 20 minutes to yield a yellow oil (93 mg, 69%). LC-MS (ESI): Mass calcd. for C₁₈H₂₃BF₃NO₄ 385.2 m/z found 386.1 [M+H]⁺. ¹H NMR (400 MHz, Chloroform-d) δ 7.78 - 7.71 (m, 2H), 7.43 (ddd, *J* = 7.9, 2.1, 1.4 Hz, 1H), 7.37 - 7.30 (m, 1H), 3.95 (dtd, *J* = 12.9, 6.4, 1.3 Hz, 1H), 3.07 (q, *J* = 1.3 Hz, 3H), 2.70 - 2.62 (m, 1H), 2.53 - 2.45 (m, 1H), 1.33 (s, 12H).

Step E. 3-(3-(4-Bromothiazol-2-yl)phenyl)-3-hydroxy-1-methyl-5-(trifluoromethyl)pyrrolidin-2-one. In a small vial 1,4-dioxane (1.61 mL) and aqueous K₂CO₃ (2 M, 0.48 mL) were combined and sparged for 15 min with nitrogen. To this solution were added 3-hydroxy-1-methyl-3-(3-(4,4,5,5-tetramethyl-1,3,2-dioxaborolan-2-yl)phenyl)-5-(trifluoromethyl)pyrrolidin-2-one (93 mg, 0.24 mmol), dibromothiazole (76 mg, 0.13 mmol), and PdCl₂(dppf) (18 mg, 0.02 mmol). The vial was sealed and heated at 100 °C. After 80 minutes, the mixture was filtered through a syringe filter and washed with MeOH. The solution was concentrated to give a dark brown gum which was subjected to flash column chromatography with 0% to 20% MeOH in DCM as the eluent to yield an orange gum (65 mg, 64%). LC-MS (ESI): Mass calcd. for C₁₅H₁₂BrF₃N₂O₂S 420 m/z found 420.9 [M+H]⁺.

Step F. (3R,5S)-3-Hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one and (3*S*,5*R*)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one. To a 5 mL vial was added 3-(3-(4-bromothiazol-2-yl)phenyl)-3-hydroxy-1-methyl-5-(trifluoromethyl)pyrrolidin-2-one (65 mg, 0.15 mmol) and 1,4-dioxane. This mixture was sparged under nitrogen for 5 minutes before the addition of N-(1-methyl-1H-pyrazol-3-yl)-4-(trimethylstannyl)pyrimidin-2-amine (Intermediate 1, 68 mg, 0.2 mmol) and Pd(PPh₃)₄ (27 mg, 0.02 mmol). The vial was capped and heated at 100 °C overnight. The reaction mixture was then directly subjected to flash column chromatography, running from 0% to 20% MeOH in DCM over 20 minutes to provide a mixture of (3*R*,5*S*)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one and (3*S*,5*R*)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one. This mixture was separated by SFC: Stationary phase: CHIRALPAK IH 5 µm, 250 x 21 mm, Mobile phase: 20% methanol, 80% CO₂, Flow Rate 73 mg/min, monitoring at 220 nm to provide a first eluting peak which was designated as diastereomer 1 of (*trans*)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one (Example 37) (light yellow residue, 11 mg, 14%). ¹H NMR (600 MHz, Methanol-*d₄*) δ 8.55 (d, *J* = 5.1 Hz, 1H), 8.42 (s, 1H), 8.27 - 8.21 (m, 1H), 8.03 (dt, *J* = 7.2, 1.7 Hz, 1H), 7.61 (d, *J* = 5.1 Hz, 1H), 7.56 - 7.50 (m, 3H), 6.77 (d, *J* = 2.3 Hz, 1H), 3.85 (s, 3H), 3.10 (d, *J* = 1.1 Hz, 3H), 2.89 (dd, *J* = 14.5, 8.6 Hz, 1H), 2.50 (dd, *J* = 14.5, 5.3 Hz, 1H).
The second eluting peak was designated as diastereomer 2 of (*trans*)-3-hydroxy-1-methyl-3-(3-(4-(2-((1-methyl-1H-pyrazol-3-yl)amino)pyrimidin-4-yl)thiazol-2-yl)phenyl)-5-(trifluoromethyl)pyrrolidin-2-one (Example 38) (yellow residue, 14 mg, 18%). ¹H NMR (600 MHz, Methanol-*d₄*) δ 8.55 (d, *J* = 5.1 Hz, 1H), 8.42 (s, 1H), 8.27 - 8.21 (m, 1H), 8.03 (dt, *J* = 7.2, 1.7 Hz, 1H), 7.61 (d, *J* = 5.1 Hz, 1H), 7.56 - 7.50 (m, 3H), 6.77 (d, *J* = 2.3 Hz, 1H), 3.85 (s, 3H), 3.10 (d, *J* = 1.1 Hz, 3H), 2.89 (dd, *J* = 14.5, 8.6 Hz, 1H), 2.50 (dd, *J* = 14.5, 5.3 Hz, 1H).

### Example 39 and Example 40: (R)-3-hydroxy-1-methyl-3-(3-(2-(2-(((R)-1-(1-methyl-1H-pyrazol-4-yl)ethyl)amino)pyrimidin-4-yl)thiazol-4-yl)phenyl)pyrrolidin-2-one and (R)-3-hydroxy-1-methyl-3-(3-(2-(2-(((S)-1-(1-methyl-1H-pyrazol-4-yl)ethyl)amino)pyrimidin-4-yl)thiazol-4-yl)phenyl)pyrrolidin-2-one.

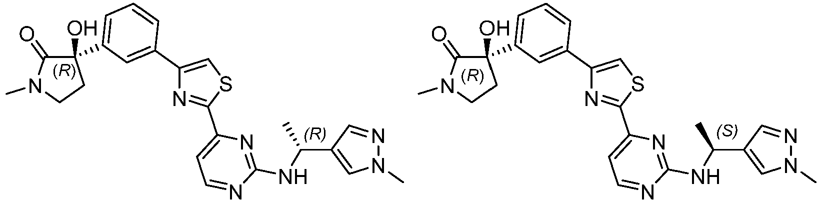

(*R*)-3-Hydroxy-1-methyl-3-(3-(2-(2-(((*R*,*S*)-1-(1-methyl-1H-pyrazol-4-yl)ethyl)amino)pyrimidin-4-yl)thiazol-4-yl)phenyl)pyrrolidin-2-one was prepared using conditions analogous to those described in Example 50, using (*R,S*)-4-(4-bromothiazol-2-yl)-N-(1-(1-methyl-1H-pyrazol-4-yl)ethyl)pyrimidin-2-amine (Intermediate 64 in place of 4-(4-bromothiazol-2-yl)-N-(1-(1-methyl-1H-pyrazol-3-yl)ethyl)pyrimidin-2-amine. The residue was purified using column C8 under basic conditions to provide (*R*)-3-hydroxy-1-methyl-3-(3-(2-(2-(((*R,S*)-1-(1-methyl-1H-pyrazol-4-yl)ethyl)amino)pyrimidin-4-yl)thiazol-4-yl)phenyl)pyrrolidin-2-one. The (*R*) and (*S*) diastereomers of (*R*)-3-hydroxy-1-methyl-3-(3-(2-(2-(((*R,S*)-1-(1-methyl-1H-pyrazol-4-yl)ethyl)amino)pyrimidin-4-yl)thiazol-4-yl)phenyl)pyrrolidin-2-one were separated by SFC (CHIRALPAK IA, 5 µm, 250 x 21 mm, 60% methanol: isopropanol (1:1), 40% CO₂, 55 mL/min) to give two products. The first eluting peak was designated diastereomer 1 of (*R*)-3-hydroxy-1-methyl-3-(3-(2-(2-(((*R,S*)-1-(1-methyl-1H-pyrazol-4-yl)ethyl)amino)pyrimidin-4-yl)thiazol-4-yl)phenyl)pyrrolidin-2-one (Example 39) (24%). ¹H NMR (500 MHz, DMSO-*d₆*) δ 8.50 (d, *J* = 4.9 Hz, 1H), 8.32 (s, 1H), 8.08 (t, *J* = 1.8 Hz, 1H), 7.93 (dt, *J* = 7.8, 1.3 Hz, 1H), 7.67 (d, *J* = 8.5 Hz, 1H), 7.61 (s, 1H), 7.45 (t, *J* = 7.7 Hz, 1H), 7.40 (s, 1H), 7.38 - 7.34 (m, 1H), 7.32 (d, *J* = 4.9 Hz, 1H), 6.10 (s, 1H), 5.22 - 5.13 (m, 1H), 4.34 (d, *J* = 4.2 Hz, 1H), 3.82 - 3.74 (m, 4H), 3.51 - 3.44 (m, 1H), 3.42 - 3.37 (m, 1H), 2.87 (s, 3H), 2.42 - 2.35 (m, 1H), 2.32 - 2.25 (m, 1H), 1.49 (d, *J* = 6.9 Hz, 3H). MS (ESI⁺): m/z = 476.2. The second eluting peak was designated diastereomer 2 of (*R*)-3-hydroxy-1-methyl-3-(3-(2-(2-(((*R,S*)-1-(1-methyl-1H-pyrazol-4-yl)ethyl)amino)pyrimidin-4-yl)thiazol-4-yl)phenyl)pyrrolidin-2-one (Example 40) (25%). ¹H NMR (500 MHz, DMSO-*d₆*) δ 8.50 (d, *J* = 4.9 Hz, 1H), 8.32 (s, 1H), 8.08 (t, *J* = 1.8 Hz, 1H), 7.93 (dt, *J* = 7.9, 1.3 Hz, 1H), 7.67 (d, *J* = 8.4 Hz, 1H), 7.61 (s, 1H), 7.45 (t, *J* = 7.7 Hz, 1H), 7.41 (s, 1H), 7.35 (ddd, *J* = 7.8, 1.9, 1.1 Hz, 1H), 7.32 (d, *J* = 4.9 Hz, 1H), 6.10 (s, 1H), 5.17 (d, *J* = 7.7 Hz, 1H), 3.77 (s, 3H), 3.48 (ddd, *J* = 9.9, 8.2, 4.8 Hz, 1H), 3.41 - 3.37 (m, 1H), 2.87 (s, 3H), 2.42 - 2.38 (m, 1H), 2.28 (ddd, *J* = 13.5, 8.2, 5.8 Hz, 1H), 1.49 (d, *J* = 7.0 Hz, 3H). MS (ESI⁺): m/z = 476.2.

### Example 41 and Example 42: (R)-3-(3-(2-(2-(((S)-1-(1H-pyrazol-3-yl)ethyl)amino)pyrimidin-4-yl)thiazol-4-yl)phenyl)-3-hydroxy-1-methylpyrrolidin-2-one and (R)-3-(3-(2-(2-(((R)-1-(1H-pyrazol-3-yl)ethyl)amino)pyrimidin-4-yl)thiazol-4-yl)phenyl)-3-hydroxy-1-methylpyrrolidin-2-one.

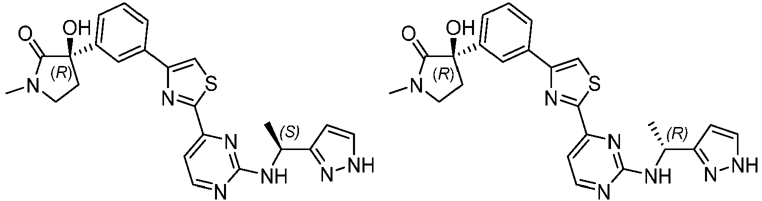

(*R*)-3-(3-(2-(2-(((*R,S*)-1-(1H-Pyrazol-3-yl)ethyl)amino)pyrimidin-4-yl)thiazol-4-yl)phenyl)-3-hydroxy-1-methylpyrrolidin-2-one was prepared using conditions analogous to those described in Example 50, using (*R,S*)-N-(1-(1H-pyrazol-3-yl)ethyl)-4-(4-bromothiazol-2-yl)pyrimidin-2-amine (Intermediate 65) in place of 4-(4-bromothiazol-2-yl)-N-(1-(1-methyl-1H-pyrazol-3-yl)ethyl)pyrimidin-2-amine. The residue was purified using column C8 under basic conditions to provide (*R*)-3-(3-(2-(2-(((*R*,*S*)-1-(1H-pyrazol-3-yl)ethyl)amino)pyrimidin-4-yl)thiazol-4-yl)phenyl)-3-hydroxy-1-methylpyrrolidin-2-one. The (*R*) and (*S*) diastereomers of (*R*)-3-(3-(2-(2-(((*R,S*)-1-(1H-pyrazol-3-yl)ethyl)amino)pyrimidin-4-yl)thiazol-4-yl)phenyl)-3-hydroxy-1-methylpyrrolidin-2-one were separated by SFC (CHIRALPAK IA, 5 µm, 250 x 21 mm, Mobile phase: 40% methanol: isopropanol (1:1) with 0.2% isopropylamine, 60% CO₂, 55 mL/min) to give two products. The first eluting peak was designated diastereomer 1 of (*R*)-3-(3-(2-(2-(((*R,S*)-1-(1H-pyrazol-3-yl)ethyl)amino)pyrimidin-4-yl)thiazol-4-yl)phenyl)-3-hydroxy-1-methylpyrrolidin-2-one (Example 41) (14%). ¹H NMR (500 MHz, DMSO-*d₆*) δ 12.70 - 12.42 (m, 1H), 8.50 (d, *J* = 4.9 Hz, 1H), 8.31 (s, 1H), 8.07 (t, *J* = 1.8 Hz, 1H), 7.92 (dt, *J* = 7.8, 1.3 Hz, 1H), 7.65 - 7.55 (m, 1H), 7.44 (t, *J* = 7.7 Hz, 1H), 7.37 - 7.30 (m, 2H), 6.27 - 6.17 (m, 1H), 6.09 (s, 1H), 5.37 - 5.25 (m, 1H), 3.50 - 3.44 (m, 1H), 3.41 - 3.34 (m, 1H), 2.86 (s, 3H), 2.41 - 2.34 (m, 1H), 2.31 - 2.24 (m, 1H), 1.51 (d, *J* = 6.9 Hz, 3H). MS (ESI⁺): m/z = 462.2.

The second eluting peak was designated as diastereomer 2 of (*R*)-3-(3-(2-(2-(((*R*,*S*)-1-(1H-pyrazol-3-yl)ethyl)amino)pyrimidin-4-yl)thiazol-4-yl)phenyl)-3-hydroxy-1-methylpyrrolidin-2-one (Example 42) (14%). ¹H NMR (500 MHz, DMSO-*d₆*) δ 8.56 (d, *J* = 4.9 Hz, 1H), 8.38 (s, 1H), 8.13 (t, *J* = 1.9 Hz, 1H), 7.99 (dt, *J* = 7.9, 1.3 Hz, 1H), 7.78 - 7.67 (m, 1H), 7.51 (t, *J* = 7.7 Hz, 1H), 7.43 - 7.40 (m, 1H), 7.40 - 7.38 (m, 1H), 6.31 - 6.26 (m, 1H), 6.15 (s, 1H), 5.41 - 5.36 (m, 1H), 3.56 - 3.50 (m, 1H), 3.47 - 3.43 (m, 2H), 2.93 (s, 3H), 2.48 - 2.43 (m, 1H), 2.38 - 2.30 (m, 2H), 1.97 (s, 2H), 1.58 (d, *J* = 6.9 Hz, 3H). MS (ESI⁺): m/z = 462.2.

### Example 43: (R)-3-Hydroxy-1-methyl-3-(3-(4-(2-((2-methylpyridin-4-yl)amino)pyrimidin-4-yl)thiazol-2-yl)phenyl)pyrrolidin-2-one.

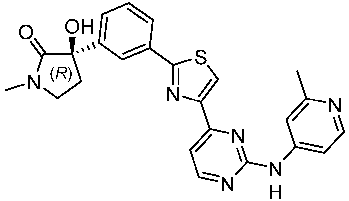

A mixture of (3R)-3-hydroxy-1-methyl-3-[3-[4-(2-methylsulfonylpyrimidin-4-yl)thiazol-2-yl]phenyl]pyrrolidin-2-one (Intermediate 12, 50 mg, 0.12 mmol), 2-methyl-4-aminopyridine (27 mg, 0.25 mmol), TFA (0.02 mL, 0.25 mmol) and DMSO (0.25 mL) was heated at 140 °C for 3.5 hr and then cooled to room temperature. The resulting solution was diluted with DMSO, filtered through a syringe filter, and then purified by RP-HPLC (basic conditions, column C2) to afford (*R*)-3-hydroxy-1-methyl-3-(3-(4-(2-((2-methylpyridin-4-yl)amino)pyrimidin-4-yl)thiazol-2-yl)phenyl)pyrrolidin-2-one (11.6 mg, 22%) as a yellow powder. ¹H NMR (500 MHz, DMSO-*d₆*) δ 10.13 (s, 1H), 8.73 (d, *J =* 5.0 Hz, 1H), 8.56 (s, 1H), 8.28 (d, *J* = 5.7 Hz, 1H), 8.14 (t, *J* = 1.8 Hz, 1H), 7.97 (ddd, *J* = 7.6, 1.9, 1.1 Hz, 1H), 7.74 (dd, *J* = 5.8, 2.1 Hz, 1H), 7.71 - 7.63 (m, 2H), 7.53 (t, *J =* 7.7 Hz, 1H), 7.49 -7.42 (m, 1H), 6.24 (s, 1H), 3.51 - 3.43 (m, 1H), 3.43 - 3.35 (m, 1H), 2.87 (s, 3H), 2.44 (s, 3H), 2.44 - 2.35 (m, 1H), 2.35 - 2.24 (m, 1H). MS (ESI⁺): m/z = 459.15.

### Example 44: (R)-3-(3-(2-(2-((1-(1,3-dihydroxypropan-2-yl)-1H-pyrazol-4-yl)amino)pyrimidin-4-yl)thiazol-4-yl)phenyl)-3-hydroxy-1-methylpyrrolidin-2-one.

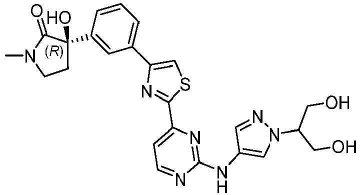

(*R*)-3-(3-(2-(2-((1-(1,3-dihydroxypropan-2-yl)-1H-pyrazol-4-yl)amino)pyrimidin-4-yl)thiazol-4-yl)phenyl)-3-hydroxy-1-methylpyrrolidin-2-one (10.8 mg, 18%) was prepared in a manner analogous to Example 43 using (*R*)-3-hydroxy-1-methyl-3-(3-(2-(2-(methylsulfonyl)pyrimidin-4-yl)thiazol-4-yl)phenyl)pyrrolidin-2-one (Intermediate 9) instead of (3R)-3-hydroxy-1-methyl-3-[3-[4-(2-methylsulfonylpyrimidin-4-yl)thiazol-2-yl]phenyl]pyrrolidin-2-one (Intermediate 12) and 1-(oxetan-3-yl)-1H-pyrazol-4-amine instead of 2-methyl-4-aminopyridine. ¹H NMR (500 MHz, DMSO-*d₆*) δ 9.80 (s, 1H), 8.64 (d, J = 4.9 Hz, 1H), 8.39 (s, 1H), 8.14 - 7.99 (m, 2H), 7.99 - 7.89 (m, 1H), 7.66 (s, 1H), 7.51 - 7.43 (m, 2H), 7.41 - 7.32 (m, 1H), 6.11 (s, 1H), 4.97 - 4.80 (m, 2H), 4.29 - 4.16 (m, 1H), 3.83 - 3.67 (m, 4H), 3.53 - 3.44 (m, 1H), 3.44 - 3.35 (m, 1H), 2.88 (s, 3H), 2.44 - 2.34 (m, 1H), 2.34 - 2.23 (m, 1H). MS (ESI⁺): m/z = 508.0.

### Example 45: (R)-3-(3-(4-(2-(((R,S)-1-(1H-benzo[d]imidazol-2-yl)ethyl)amino)pyrimidin-4-yl)thiazol-2-yl)phenyl)-3-hydroxy-1-methylpyrrolidin-2-one.

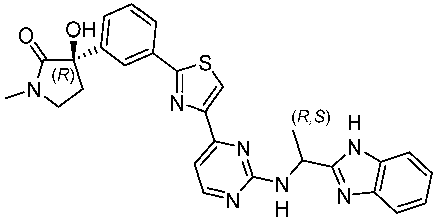

A mixture of (3R)-3-hydroxy-1-methyl-3-[3-[4-(2-methylsulfonylpyrimidin-4-yl)thiazol-2-yl]phenyl]pyrrolidin-2-one (Intermediate 12, 50 mg, 0.12 mmol), 1-(1H-benzo[d]imidazol-2-yl)ethan-1-amine (40 mg, 0.25 mmol), DIPEA (0.086 mL, 0.5 mmol) and DMA (0.5 mL) was heated at 140 °C for 3.5 hr and then cooled to room temperature. The resulting solution was diluted with DMSO, filtered through a syringe filter, and then purified by RP-HPLC (basic conditions, column C2) to afford (*R*)-3-(3-(4-(2-(((*R,S*)-1-(1H-benzo[d]imidazol-2-yl)ethyl)amino)pyrimidin-4-yl)thiazol-2-yl)phenyl)-3-hydroxy-1-methylpyrrolidin-2-one (6.8 mg, yield 11%) as a yellow powder. ¹H NMR (600 MHz, DMSO-*d₆*) δ 12.24 (s, 1H), 8.47 (s, 1H), 8.39 (s, 1H), 8.08 (s, 1H), 7.91 (d, *J* = 7.6 Hz, 1H), 7.70 (d, *J* = 7.4 Hz, 1H), 7.61 - 7.39 (m, 4H), 7.35 (d, *J =* 4.9 Hz, 1H), 7.12 (dd, *J* = 6.0, 3.1 Hz, 2H), 6.21 (s, 1H), 5.46 (s, 1H), 3.52 - 3.41 (m, 1H), 3.40 - 3.36 (m, 1H), 2.86 (s, 3H), 2.37 (dd, *J* = 12.5, 6.9 Hz, 1H), 2.32 - 2.18 (m, 1H), 1.66 (d, *J* = 7.0 Hz, 3H). MS (ESI⁺): m/z = 512.3.

### Example 46: (R)-3-Hydroxy-1-methyl-3-(5-(2-(2-((1-methyl-1H-pyrazol-5-yl)amino)pyrimidin-4-yl)thiazol-4-yl)pyridin-3-yl)pyrrolidin-2-one or (S)-3-hydroxy-1-methyl-3-(5-(2-(2-((1-methyl-1H-pyrazol-5-yl)amino)pyrimidin-4-yl)thiazol-4-yl)pyridin-3-yl)pyrrolidin-2-one.

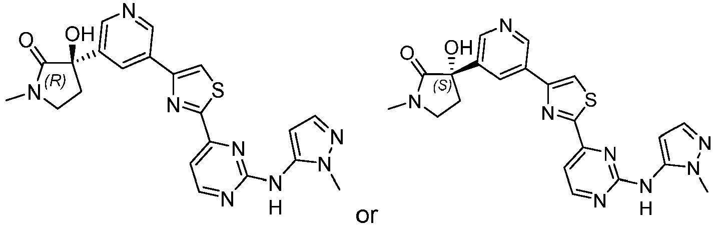

A mixture of 4-(4-bromothiazol-2-yl)-N-(1-methyl-1H-pyrazol-5-yl)pyrimidin-2-amine (Intermediate 14, 100 mg, 0.30 mmol), PdCl₂(dppf) (22 mg, 0.030 mmol), bis(pinacolato)diboron (90 mg, 0.36 mmol), KOAc (87 mg, 0.89 mmol), and 1,4-dioxane (1.3 mL) was heated at 100 °C for 1 hr and then enantiomer 1 of 3-(5-bromopyridin-3-yl)-3-hydroxy-1-methylpyrrolidin-2-one (Intermediate 55, 96 mg, 0.36 mmol) in 1,4-dioxane (0.86 mL) and K₂CO₃ (0.74 mL, 1 M aqueous solution, 0.741 mmol) were added. The resulting mixture was heated at 100 °C for 30 minutes and then cooled to room temperature. The reaction mixture was diluted with water and extracted with 1:4 IPA:DCM. Then the organic extract was dried with anhydrous MgSO₄, filtered and concentrated. The residue was purified by RP-HPLC (basic conditions, column C8) to afford (*R*)-3-hydroxy-1-methyl-3-(5-(2-(2-((1-methyl-1H-pyrazol-5-yl)amino)pyrimidin-4-yl)thiazol-4-yl)pyridin-3-yl)pyrrolidin-2-one or (*S*)-3-hydroxy-1-methyl-3-(5-(2-(2-((1-methyl-1*H*-pyrazol-5-yl)amino)pyrimidin-4-yl)thiazol-4-yl)pyridin-3-yl)pyrrolidin-2-one as a yellow solid (27 mg, yield 19%). ¹H NMR (500 MHz, DMSO-*d₆*) δ 9.75 (s, 1H), 9.19 - 9.15 (m, 1H), 8.68 (d, *J* = 5.0 Hz, 1H), 8.57 (d, *J* = 2.3 Hz, 1H), 8.57 - 8.54 (m, 1H), 8.40 (t, *J* = 2.2 Hz, 1H), 7.63 (d, *J =* 5.0 Hz, 1H), 7.39 (d, *J =* 1.9 Hz, 1H), 6.36 - 6.31 (m, 2H), 3.73 (s, 3H), 3.55 - 3.47 (m, 1H), 3.46 - 3.41 (m, 1H), 2.87 (s, 3H), 2.49 - 2.45 (m, 1H), 2.35 - 2.28 (m, 1H). MS (ESI⁺): m/z = 449.

### Example 47: (R)-3-Hydroxy-1-methyl-3-(3-(4-(2-((1-(methylsulfonyl)piperidin-4-yl)amino)pyrimidin-4-yl)thiazol-2-yl)phenyl)pyrrolidin-2-one.

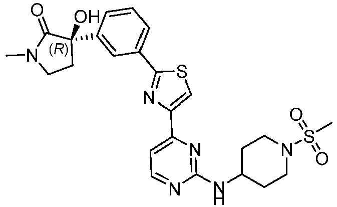

A mixture of 1-(methylsulfonyl)piperidin-4-amine (208 mg, 1.17 mmol), (R)-3-hydroxy-1-methyl-3-[3-[4-(2-methylsulfonylpyrimidin-4-yl)thiazol-2-yl]phenyl]pyrrolidin-2-one (Intermediate 12, 100 mg, 0.232 mmol), DIPEA (0.72 mL, 4.2 mmol) and DMA (0.72 mL) was heated at 150 °C in a microwave reactor for 30 minutes. The resulting mixture was cooled to room temperature, concentrated, and purified by RP-HPLC (Welch Xtimate C18 column, 5 µm, 150 x 30 mm; 30-60% ACN (v/v)/H₂O (0.05% NH₃H₂O + 10 mM NH₄HCO₃)) to afford (*R*)-3-hydroxy-1-methyl-3-(3-(4-(2-((1-(methylsulfonyl)piperidin-4-yl)amino)pyrimidin-4-yl)thiazol-2-yl)phenyl)pyrrolidin-2-one (33.8 mg, 27%). ¹H NMR (400 MHz, DMSO-*d₆*) δ 8.47 - 8.31 (m, 2H), 8.10 (s, 1H), 7.96 - 7.89 (m, 1H), 7.54 - 7.48 (m, 1H), 7.46 - 7.40 (m, 1H), 7.35 - 7.26 (m, 2H), 6.24 (s, 1H), 3.98 (br s, 1H), 3.60 - 3.52 (m, 2H), 3.51 - 3.44 (m, 1H), 3.43 - 3.38 (m, 1H), 2.96 - 2.84 (m, 8H), 2.42 - 2.32 (m, 1H), 2.32 - 2.23 (m, 1H), 2.06 - 1.96 (m, 2H), 1.66 - 1.53 (m, 2H). MS (ESI⁺): m/z = 529.1.

### Example 48: (R)-3-hydroxy-1-methyl-3-(3-(4-(2-((6-methylpyridin-2-yl)amino)pyrimidin-4-yl)thiazol-2-yl)phenyl)pyrrolidin-2-one

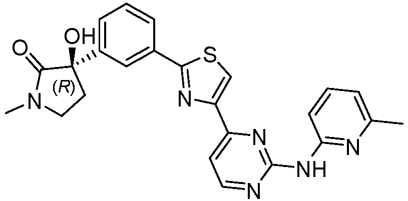

To a mixture of 6-methylpyridin-2-amine (38 mg, 0.348 mmol) and THF (2 mL) at -72 °C was added NaHMDS (0.42 mL, 0.42 mmol, 1 M in THF). After 1 h, (*R*)-3-hydroxy-1-methyl-3-[3-[4-(2-methylsulfonylpyrimidin-4-yl)thiazol-2-yl]phenyl]pyrrolidin-2-one (Intermediate 12, 60 mg, 0.14 mmol) was added and the resulting mixture was stirred for an additional hour at -72 °C. The mixture was warmed to rt and stirred for 16 hr and then diluted with MeOH, concentrated, and purified by FCC (0-5% MeOH in DCM) followed by RP-HPLC (Phenomenex C18 column, 3 µm, 75 x 30 mm; eluent: 41% to 71% (v/v) CH₃CN and H₂O with 0.05% NH₃ + 10 mM NH₄HCO₃) to afford (*R*)-3-hydroxy-1-methyl-3-(3-(4-(2-((6-methylpyridin-2-yl)amino)pyrimidin-4-yl)thiazol-2-yl)phenyl)pyrrolidin-2-one (11.8 mg, 18%) as a white solid. ¹H NMR (400 MHz, DMSO-*d₆*) δ 9.67 (d, *J* = 1.6 Hz, 1H), 8.70 (d, *J* = 4.0 Hz, 1H), 8.57 (s, 1H), 8.26 (d, *J* = 8.0 Hz, 1H), 8.13 (s, 1H), 8.00 - 7.93 (m, 1H), 7.74 - 7.70 (m, 1H), 7.63 (d, *J* = 4.0 Hz, 1H), 7.56 - 7.50 (m, 1H), 7.48 - 7.43 (m, 1H), 6.89 (d, *J* = 4.0 Hz, 1H), 6.25 (s, 1H), 3.52 - 3.46 (m, 1H), 3.42 - 3.36 (m, 1H), 2.87 (s, 3H), 2.43 (s, 3H), 2.41 - 2.35 (m, 1H), 2.34 - 2.25 (m, 1H). MS (ESI⁺): m/z = 459.1.

### Example 49: (R)-3-(3-(4-(2-((1,5-Dimethyl-1H-pyrazol-3-yl)amino)pyrimidin-4-yl)thiazol-2-yl)phenyl)-3-hydroxy-1-methylpyrrolidin-2-one.

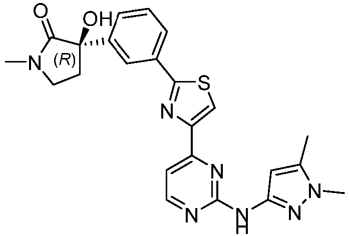

A mixture of (*R*)-3-hydroxy-1-methyl-3-[3-[4-(2-methylsulfonylpyrimidin-4-yl)thiazol-2-yl]phenyl]pyrrolidin-2-one (Intermediate 12, 250 mg, 0.58 mmol), 1,4-dioxane (5 mL), 1,5-dimethyl-1H-pyrazol-3-amine (625 mg, 5.62 mmol) and TsOH (125 mg, 0.726 mmol) was heated at 115 °C or 16 h and then cooled to rt. The resulting mixture was diluted with DCM and then washed with 2 M aqueous NaOH solution followed by brine. The organic extract was dried with anhydrous MgSO₄, filtered, and concentrated. The residue was purified by RP HPLC (Xtimate C18 column, 10 µm, 150 x 40 mm; eluent: 25% to 55% (v/v) CH₃CN and H₂O with 0.05% NH₃ + 10 mM NH₄HCO₃) to afford (*R*)-3-(3-(4-(2-((1,5-dimethyl-1*H*-pyrazol-3-yl)amino)pyrimidin-4-yl)thiazol-2-yl)phenyl)-3-hydroxy-1-methylpyrrolidin-2-one (70 mg, 25%) as a yellow solid. ¹H NMR (400 MHz, DMSO-*d*₆) δ 9.70 (s, 1H), 8.59 - 8.55 (m, 1H), 8.48 (s, 1H), 8.14 - 8.10 (m, 1H), 7.98 - 7.93 (m, 1H), 7.55 - 7.49 (m, 1H), 7.49 - 7.42 (m, 2H), 6.59 (s, 1H), 6.26 (s, 1H), 3.64 (s, 3H), 3.53 - 3.45 (m, 1H), 3.43 - 3.39 (m, 1H), 2.87 (s, 3H), 2.43 - 2.35 (m, 1H), 2.34 - 2.22 (m, 4H). MS (ESI⁺): m/z = 462.2.

### Example 50 and Example 51: (R)-3-hydroxy-1-methyl-3-(3-(2-(2-(((S)-1-(1-methyl-1H-pyrazol-3-yl)ethyl)amino)pyrimidin-4-yl)thiazol-4-yl)phenyl)pyrrolidin-2-one and (R)-3-hydroxy-1-methyl-3-(3-(2-(2-(((R)-1-(1-methyl-1H-pyrazol-3-yl)ethyl)amino)pyrimidin-4-yl)thiazol-4-yl)phenyl)pyrrolidin-2-one.

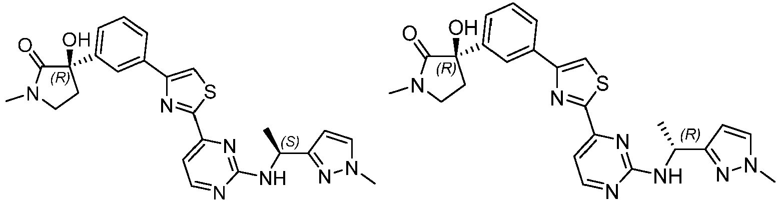

A mixture of 4-(4-bromothiazol-2-yl)-N-(1-(1-methyl-1H-pyrazol-3-yl)ethyl)pyrimidin-2-amine (Intermediate 63, 110 mg, 0.30 mmol), PdCl₂(dppf) (11 mg, 0.015 mmol), (R)-3-hydroxy-1-methyl-3-[3-(4,4,5,5-tetramethyl-1,3,2-dioxaborolan-2-yl)phenyl]pyrrolidin-2-one (116 mg, 0.37 mmol), K₂CO₃ (0.76 mL, 1 M in water, 0.76 mmol), and 1,4-dioxane (0.88 mL) was heated at 90 °C for 1 hour. The resulting mixture was cooled to rt, diluted with ethyl acetate and washed with water. The organic extracts were dried with anhydrous MgSO₄, filtered, concentrated and purified by RP-HPLC (basic conditions, column C8) to afford (*R*)-3-hydroxy-1-methyl-3-(3-(2-(2-(((*R*,*S*)-1-(1-methyl-1H-pyrazol-3-yl)ethyl)amino)pyrimidin-4-yl)thiazol-4-yl)phenyl)pyrrolidin-2-one (105 mg, yield 69%). The diastereomers of (*R*)-3-hydroxy-1-methyl-3-(3-(2-(2-(((*R*,*S*)-1-(1-methyl-1H-pyrazol-3-yl)ethyl)amino)pyrimidin-4-yl)thiazol-4-yl)phenyl)pyrrolidin-2-one were separated by SFC ((Stationary phase: CHIRALCEL OZ-H, 5 µm, 250 x 21 mm, Mobile phase: 45% methanol, 55% CO₂). Flow rate 50 mL/min, monitor at 220 nm. >99% ee) to provide a first eluting peak which was designated as diastereomer 1 of (*R*)-3-hydroxy-1-methyl-3-(3-(2-(2-(((*R,S*)-1-(1-methyl-1H-pyrazol-3-yl)ethyl)amino)pyrimidin-4-yl)thiazol-4-yl)phenyl)pyrrolidin-2-one (Example 50) (55 mg, 38%). ¹H NMR (500 MHz, DMSO-*d₆*) δ 8.49 (d, *J* = 4.9 Hz, 1H), 8.31 (s, 1H), 8.07 (t, *J* = 1.8 Hz, 1H), 7.95 - 7.91 (m, 1H), 7.62 (d, *J* = 8.4 Hz, 1H), 7.54 (d, *J* = 2.2 Hz, 1H), 7.45 (t, *J* = 7.7 Hz, 1H), 7.38 - 7.34 (m, 1H), 7.32 (d, *J* = 4.9 Hz, 1H), 6.20 (s, 1H), 6.10 (s, 1H), 5.30 - 5.22 (m, 1H), 3.79 (s, 3H), 3.51 - 3.44 (m, 1H), 3.42 - 3.36 (m, 1H), 2.87 (s, 3H), 2.42 - 2.35 (m, 1H), 2.32 - 2.25 (m, 1H), 1.50 (d, *J* = 6.9 Hz, 3H). MS (ESI⁺): m/z = 476.2.

The second eluting peak was designated as diastereomer 2 of (*R*)-3-hydroxy-1-methyl-3-(3-(2-(2-(((*R,S*)-1-(1-methyl-1H-pyrazol-3-yl)ethyl)amino)pyrimidin-4-yl)thiazol-4-yl)phenyl)pyrrolidin-2-one (Example 51) (52 mg, 36%). ¹H NMR (500 MHz, DMSO-*d₆*) δ 8.49 (d, *J* = 4.9 Hz, 1H), 8.31 (s, 1H), 8.07 (t, *J* = 1.8 Hz, 1H), 7.95 - 7.91 (m, 1H), 7.62 (d, *J* = 8.4 Hz, 1H), 7.54 (d, *J* = 2.2 Hz, 1H), 7.45 (t, *J* = 7.7 Hz, 1H), 7.38 - 7.34 (m, 1H), 7.32 (d, *J* = 4.9 Hz, 1H), 6.20 (s, 1H), 6.10 (s, 1H), 5.30 - 5.22 (m, 1H), 3.79 (s, 3H), 3.51 - 3.44 (m, 1H), 3.42 - 3.36 (m, 1H), 2.87 (s, 3H), 2.42 - 2.35 (m, 1H), 2.32 - 2.25 (m, 1H), 1.50 (d, *J* = 6.9 Hz, 3H). MS (ESI⁺): m/z = 476.2.

### Example 52: (R)-3-(3-(4-(5-Fluoro-2-((1-methyl-1H-pyrazol-3-yl)amino)pyrimidin-4-yl)thiazol-2-yl)phenyl)-3-hydroxy-1-methylpyrrolidin-2-one.

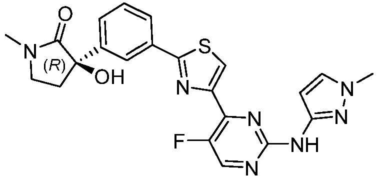

Step A: (*R*)-3-Hydroxy-1-methyl-3-(3-(4-(trimethylstannyl)thiazol-2-yl)phenyl)pyrrolidin-2-one. A mixture of (*R*)-3-(3-(4-bromothiazol-2-yl)phenyl)-3-hydroxy-1-methylpyrrolidin-2-one (Intermediate 7, 512 mg, 1.45 mmol), hexamethylditin (569 mg, 1.74 mmol), tetrakis(triphenylphosphine)palladium(0) (168 mg, 0.150 mmol) and THF (10 mL) was heated at 100 °C for 3 h. The mixture was cooled down to rt, concentrated, and purified by FCC (20-40% EtOAc / DCM) to provide (*R*)-3-hydroxy-1-methyl-3-(3-(4-(trimethylstannyl)thiazol-2-yl)phenyl)pyrrolidin-2-one (384 mg, 60%). MS (ESI): mass calcd. for C₁₇H₂₂N₂O₂SSn, 438.0; m/z found, 439.1 [M+H]⁺.

Step B: (*R*)-3-(3-(4-(2-Chloro-5-fluoropyrimidin-4-yl)thiazol-2-yl)phenyl)-3-hydroxy-1-methylpyrrolidin-2-one. A mixture of (*R*)-3-hydroxy-1-methyl-3-(3-(4-(trimethylstannyl)thiazol-2-yl)phenyl)pyrrolidin-2-one (170 mg, 0.39 mmol), 2,4-dichloro-5-fluoropyrimidine (100 mg, 0.6 mmol) and bis(triphenylphosphine)palladium(II) chloride (27 mg, 0.039 mmol) in DMSO (2 mL) was heated at 100 °C for 16 h, cooled to room temperature, and purified by RP-HPLC (CH₃CN/H₂O eluent) to provide (*R*)-3-(3-(4-(2-chloro-5-fluoropyrimidin-4-yl)thiazol-2-yl)phenyl)-3-hydroxy-1-methylpyrrolidin-2-one (81 mg, 51%). MS (ESI): mass calcd. for C₁₈H₁₄ClFN₄O₂S, 404.1; m/z found, 405.1 [M+H]⁺.

Step C: (*R*)-3-(3-(4-(5-Fluoro-2-((1-methyl-1*H*-pyrazol-3-yl)amino)pyrimidin-4-yl)thiazol-2-yl)phenyl)-3-hydroxy-1-methylpyrrolidin-2-one. A mixture of 1-methyl-1*H-*pyrazol-3-ylamine (19 mg, 0.20 mmol) and (*R*)-3-(3-(4-(2-chloro-5-fluoropyrimidin-4-yl)thiazol-2-yl)phenyl)-3-hydroxy-1-methylpyrrolidin-2-one (80 mg, 0.20 mmol) in DMSO (1 mL) was heated at 120 °C for 2 h, cooled to room temperature, and purified by RP-HPLC (CH₃CN/H₂O eluent) to provide (*R*)-3-(3-(4-(5-fluoro-2-((1-methyl-1*H*-pyrazol-3-yl)amino)pyrimidin-4-yl)thiazol-2-yl)phenyl)-3-hydroxy-1-methylpyrrolidin-2-one (21 mg, 23%). MS (ESI): mass calcd. for C₂₂H₂₀FN₇O₂S, 465.1; m/z found, 466.2 [M+H]⁺. ¹H NMR (500 MHz, Chloroform-*d*) δ 10.40 (s, 1H), 8.36 (d, *J* = 3.1 Hz, 1H), 8.23 (t, *J* = 1.9 Hz, 1H), 8.15 (d, *J* = 1.2 Hz, 1H), 7.86 - 7.76 (m, 1H), 7.57 (d, *J* = 2.6 Hz, 1H), 7.48 - 7.34 (m, 2H), 7.24 (d, *J* = 2.6 Hz, 1H), 7.08 ( s 1H), 3.87 (s, 3H), 3.68 - 3.55 (m, 1H), 3.55 - 3.42 (m, 1H), 3.08 (s, 3H), 2.63 - 2.49 (m, 1H), 2.51 - 2.37 (m, 1H).

### Example 53: (R)-2-((4-(2-(3-(3-Hydroxy-1-methyl-2-oxopyrrolidin-3-yl)phenyl)thiazol-4-yl)pyrimidin-2-yl)amino)-5-methyl-6,7-dihydropyrazolo[1,5-a]pyrazin-4(5H)-one.

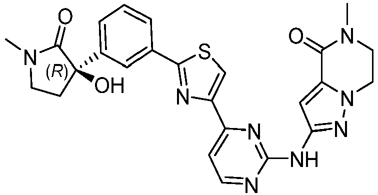

A mixture of (*R*)-3-hydroxy-1-methyl-3-(3-(4-(2-(methylsulfonyl)pyrimidin-4-yl)thiazol-2-yl)phenyl)pyrrolidin-2-one (Intermediate 12, 150 mg, 0.35 mmol), 2-amino-5-methyl-6,7-dihydropyrazolo[1,5-a]pyrazin-4(5H)-one (87 mg, 0.52 mmol), DMF (1 mL), and HCl (0.1 mL, 4 M) was heated at 160 °C for 1 h, cooled to room temperature, and purified by RP-HPLC (CH₃CN/H₂O eluent) to provide (*R*)-2-((4-(2-(3-(3-Hydroxy-1-methyl-2-oxopyrrolidin-3-yl)phenyl)thiazol-4-yl)pyrimidin-2-yl)amino)-5-methyl-6,7-dihydropyrazolo[1,5-a]pyrazin-4(5*H*)-one (12 mg, 7%). MS (ESI): mass calcd. for C₂₅H₂₄N₈O₃S, 516.2; m/z found, 517.0 [M+H]⁺. ¹H NMR (500 MHz, Methanol-*d₄*) δ 8.67 (s, 1H), 8.62 (d, *J* = 5.4 Hz, 1H), 8.25 (s, 1H), 8.03 (d, *J* = 5.3 Hz, 1H), 7.77 (d, *J* = 5.6 Hz, 1H), 7.60 - 7.52 (m, 2H), 7.24 (s, 1H), 4.40 (t, *J* = 6.3 Hz, 2H), 3.95 - 3.86 (m, 2H), 3.66 - 3.58 (m, 1H), 3.58 - 3.50 (m, 1H), 3.18 (s, 4H), 3.03 (s, 3H), 2.59 - 2.50 (m, 1H), 2.50 - 2.40 (m, 1H).

### Example 54: (R)-3-hydroxy-1-methyl-3-(3-(4-(2-((4,5,6,7-tetrahydropyrazolo[1,5-a]pyrimidin-3-yl)amino)pyrimidin-4-yl)thiazol-2-yl)phenyl)pyrrolidin-2-one.

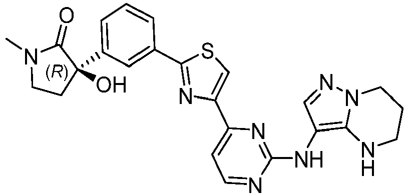

(R)-3-Hydroxy-1-methyl-3-(3-(4-(2-((4,5,6,7-tetrahydropyrazolo[1,5-a]pyrimidin-3-yl)amino)pyrimidin-4-yl)thiazol-2-yl)phenyl)pyrrolidin-2-one (16 mg, 12%) was prepared in a manner analogous to Example 53 using 4,5,6,7-tetrahydropyrazolo[1,5-a]pyrimidin-3-amine in place of 2-amino-5-methyl-6,7-dihydropyrazolo[1,5-a]pyrazin-4(5H)-one. ¹H NMR (500 MHz, Methanol-*d₄*) δ 8.49 - 8.39 (m, 2H), 8.26 - 8.18 (m, 1H), 8.02 - 7.97 (m, 1H), 7.90 (s, 1H), 7.66 (d, J = 5.5 Hz, 1H), 7.57 - 7.49 (m, 2H), 4.21 (t, J = 6.1 Hz, 2H), 3.68 - 3.59 (m, 1H), 3.58 - 3.48 (m, 1H), 3.48 - 3.42 (m, 2H), 3.02 (s, 3H), 2.61 - 2.50 (m, 1H), 2.50 - 2.42 (m, 1H), 2.30 - 2.19 (m, 2H). MS (ESI⁺): m/z = 489.3.

### Example 55: (R)-3-hydroxy-1-methyl-3-(3-(3-(2-((1-methyl-1H-pyrazol-3-yl)amino)pyrimidin-4-yl)-1H-pyrazol-1-yl)phenyl)pyrrolidin-2-one.

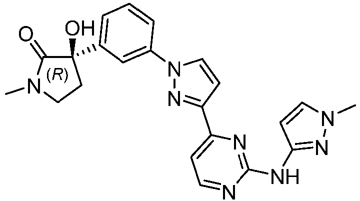

Step A: (R)-3-(3-(3-bromo-1H-pyrazol-1-yl)phenyl)-3-hydroxy-1-methylpyrrolidin-2-one. A mixture of (R)-3-(3-bromophenyl)-3-hydroxy-1-methyl-pyrrolidin-2-one (500 mg, 1.851 mmol), 3-bromopyrazole (136 mg, 0.925 mmol), cuprous iodide (18 mg, 0.093 mmol), trans-N,N'-dimethylcyclohexane-1,2-diamine (0.060 mL, 0.37 mmol), K₂CO₃ (384 mg, 2.78 mmol) and toluene (2 mL) was heated at 130 °C in a microwave reactor for 1 hr. The mixture was cooled to room temperature, diluted with water, and extracted with ethyl acetate. The organic extract was dried with anhydrous MgSO₄, filtered, and concentrated. The residue was purified by FCC (gradient 30-100% EtOAc in hexanes) to afford (R)-3-(3-(3-bromo-1H-pyrazol-1-yl)phenyl)-3-hydroxy-1-methylpyrrolidin-2-one (39 mg, 12.535%). MS (ESI⁺): m/z = 336.1.

Step B: (R)-3-hydroxy-1-methyl-3-(3-(3-(2-((1-methyl-1H-pyrazol-3-yl)amino)pyrimidin-4-yl)-1H-pyrazol-1-yl)phenyl)pyrrolidin-2-one. A mixture of (R)-3-(3-(3-bromo-1H-pyrazol-1-yl)phenyl)-3-hydroxy-1-methylpyrrolidin-2-one (39 mg, 0.116 mmol), N-(1-methyl-1H-pyrazol-3-yl)-4-(trimethylstannyl)pyrimidin-2-amine (Intermediate 1, 59 mg, 0.174 mmol), tetrakis(triphenylphosphine)palladium(0) (13 mg, 0.012 mmol), cuprous iodide (4.4 mg, 0.23 mmol), and DMF (1 mL) was heated at 80 °C for 2 hours and then at 100 °C for 16 hours. The resulting mixture was cooled to room temperature, diluted with water, and extracted with ethyl acetate. The organic extract was dried with anhydrous MgSO₄, filtered, and concentrated. The residue was purified by FCC (0-15% MeOH:DCM) followed by RP-HPLC (basic conditions, column C2) to afford (R)-3-hydroxy-1-methyl-3-(3-(3-(2-((1-methyl-1H-pyrazol-3-yl)amino)pyrimidin-4-yl)-1H-pyrazol-1-yl)phenyl)pyrrolidin-2-one (1.3 mg, 3%) as a white powder. MS (ESI⁺): m/z = 431.4.

### Example 56: (R)-3-hydroxy-1-methyl-3-(3-(4-(2-((1-methyl-1H-pyrazol-3-yl)amino)pyrimidin-4-yl)thiazol-2-yl)phenyl)pyrrolidin-2-one

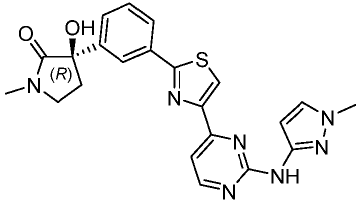

A mixture of (*R*)-3-hydroxy-1-methyl-3-(3-(4-(2-(methylsulfonyl)pyrimidin-4-yl)thiazol-2-yl)phenyl)pyrrolidin-2-one (Intermediate 12, 300 mg, 0.697 mmol) and 1-methyl-1H-pyrazol-3-amine (4 mL) was heated at 150 °C in a microwave reactor for 1 h and then cooled to room temperature. The resulting mixture was purified by RP-HPLC (Xtimate C18 150 x 40 mm x 5 µm column; eluent 26% to 56% (v/v) CH₃CN and H₂O with 0.05% NH₃H₂O) to afford (R)-3-hydroxy-1-methyl-3-(3-(4-(2-((1-methyl-1H-pyrazol-3-yl)amino)pyrimidin-4-yl)thiazol-2-yl)phenyl)pyrrolidin-2-one (17 mg, 5%). ¹H NMR (400 MHz, DMSO-*d₆*) δ 9.84 (s, 1H), 8.62 - 8.56 (m, 1H), 8.46 (s, 1H), 8.12 (s, 1H), 7.99 - 7.91 (m, 1H), 7.63 - 7.57 (m, 1H), 7.55 - 7.42 (m, 3H), 6.79 - 6.73 (m, 1H), 6.26 (s, 1H), 3.77 (s, 3H), 3.55 - 3.44 (m, 1H), 3.43 - 3.39 (m, 1H), 2.87 (s, 3H), 2.43 - 2.34 (m, 1H), 2.33 - 2.23 (m, 1H). MS (ESI⁺): m/z = 448.3.

### Example 57 and Example 58: (R)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile and (S)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile.

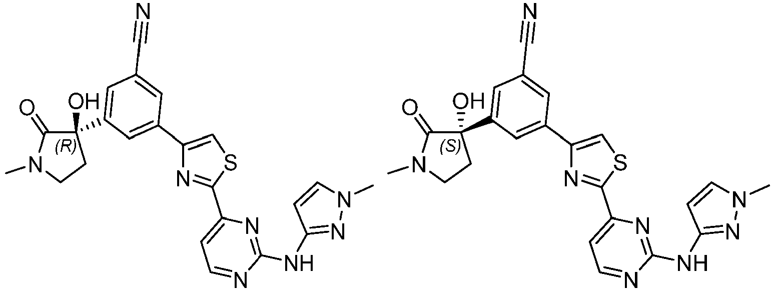

Step A: 3-bromo-5-(3-hydroxy-1-methyl-2-oxopyrrolidin-3-yl)benzonitrile. To a stirring solution of 3,5-dibromobenzonitrile (3.00 g, 11.5 mmol) and THF (20 mL) at -78 °C was added iso-propylmagnesium chloride-lithium chloride complex (8.84 mL, 1.3 M in hexanes, 11.5 mmol). The resulting mixture was stirred at -78 °C for 20 min and then 1-methylpyrrolidine-2,3-dione (1.00 g, 8.84 mmol, in 5 mL anhydrous THF) was added and the resulting mixture was stirred at -78 °C for 30 minutes. The reaction mixture was diluted with saturated aqueous NH₄Cl solution and warmed to room temperature. The resulting mixture was extracted with EtOAc and the organic extract was dried with anhydrous Na₂SO₄, filtered, and concentrated. The residue was purified by FCC (petroleum ether:EtOAc = 0:1 to 1:2) to afford 3-bromo-5-(3-hydroxy-1-methyl-2-oxopyrrolidin-3-yl)benzonitrile as a yellow solid (720 mg, 26%). MS (ESI⁺): m/z = 295.0.

Step B: (*R,S*)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile. A mixture of 4-(4-bromothiazol-2-yl)-N-(1-methyl-1H-pyrazol-4-yl)pyrimidin-2-amine (Intermediate 11, 300 mg, 0.89 mmol), bis(pinacoloto)diboron (407 mg, 1.6 mmol), Pd(dppf)Cl₂•DCM (72 mg, 0.089 mmol), KOAc (262 mg, 2.67 mmol) and 1,4-dioxane (2 mL) were heated at 100 °C for 1 h. Then 3-bromo-5-(3-hydroxy-1-methyl-2-oxopyrrolidin-3-yl)benzonitrile (262 mg, 0.89 mmol), K₂CO₃ (2.67 mL, 2.67 mmol, 1 M in H₂O), and 1,4-dioxane (3 mL) were added and the resulting mixture was heated at 100 °C for 2 h. The resulting mixture was poured into water and extracted with ethyl acetate. The organic extract was dried with anhydrous Na₂SO₄, filtered, and concentrated. The residue was purified by FCC (DCM:MeOH 1:0 to 10:1) to afford (*R,S*)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile (120 mg, 25%). MS (ESI⁺): m/z = 473.1.

Step C: (*R*)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile and (*S*)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile. The (*R*) and (*S*) enantiomers of (*R*,*S*)-3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile were separated by SFC (DAICEL CHIRALPAK AS (250 x 30 mm, 10 µm); 45% (v/v) EtOH (containing 0.1% of 25% aq. NH₃)/CO₂)) to afford a first eluting peak which was designated as enantiomer 1 of 3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile and further purified by RP-HPLC (Boston Prime C18 150 x 30 mm, 5 µm; 35 - 65% (v/v) CH₃CN (containing 0.05% of 25% aq. NH₃)/H₂O) to afford the title compound as a yellow solid (Example 57) (32 mg, 18%). ¹H NMR (400 MHz, DMSO-*d₆*) δ 9.82 (br s, 1H), 8.67 - 8.60 (m, 2H), 8.46 (s, 1H), 8.42 (s, 1H), 7.93 (s, 1H), 7.79 - 7.76 (m, 1H), 7.69 - 7.55 (m, 1H), 7.50 (d, J = 4.8 Hz, 1H), 6.42 (s, 1H), 3.86 (s, 3H), 3.56 - 3.41 (m, 2H), 2.88 (s, 3H), 2.48 - 2.44 (m, 1H), 2.36 - 2.25 (m, 1H). MS (ESI⁺): m/z = 473.2. The second eluting isomer was designated as enantiomer 2 of 3-(3-hydroxy-1-methyl-2-oxopyrrolidin-3-yl)-5-(2-(2-((1-methyl-1H-pyrazol-3-yl)amino)pyrimidin-4-yl)thiazol-4-yl)benzonitrile (Example 58) (28.9 mg, 23%). ¹H NMR (400 MHz, DMSO-*d₆*) δ 9.83 (br s, 1H), 8.68 - 8.61 (m, 2H), 8.46 (s, 1H), 8.42 (s, 1H), 7.93 (s, 1H), 7.79 - 7.76 (m, 1H), 7.69 - 7.55 (m, 1H), 7.51 (d, J = 5.2 Hz, 1H), 6.42 (s, 1H), 3.86 (s, 3H), 3.55 - 3.41 (m, 2H), 2.88 (s, 3H), 2.48 - 2.43 (m, 1H), 2.33 - 2.26 (m, 1H). MS (ESI⁺): m/z = 473.2.

### Example 59 and Example 60: (3R,5S)-3-hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one and (3S,5R)-3-hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one.

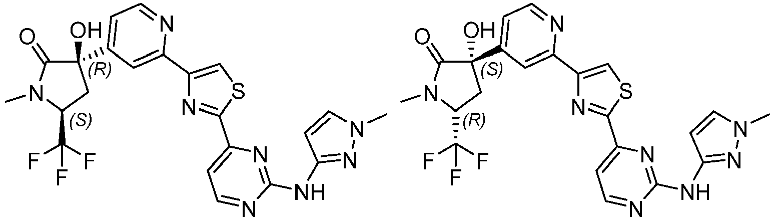

Step A: (*trans*)-3-(2-Chloropyridin-4-yl)-3-hydroxy-1-methyl-5-(trifluoromethyl)pyrrolidin-2-one. To a mixture of 4-bromo-2-chloropyridine (1.06 g, 613 µL, 5.52 mmol) and diethyl ether (15 mL) at -70 °C was added n-BuLi (2.5 M in hexanes, 2.43 mL, 6.07 mmol). After 5 minutes, a solution of 1-methyl-5-(trifluoromethyl)pyrrolidine-2,3-dione (Intermediate 61, 500 mg, 2.76 mmol) in diethyl ether (5 mL) was added and the resulting mixture was stirred for 5 minutes, and then warmed to -15 °C and stirred an additional 30 minutes. The resulting mixture was diluted with saturated aqueous NH₄Cl solution and water and extracted with ethyl acetate. The organic extract was dried with brine followed by Na₂SO₄, filtered and concentrated. The residue was purified by FCC (15-30% ethyl acetate/petroleum ether) followed by preparative TLC (PE:EA=1:1, 100 mL) to give (trans)-3-(2-chloropyridin-4-yl)-3-hydroxy-1-methyl-5-(trifluoromethyl)pyrrolidin-2-one as a light-yellow oil (280 mg, 26%). MS (ESI⁺): m/z = 294.9.

Step B: (*trans*)-3-Hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one. A mixture of 4-(4-bromothiazol-2-yl)-N-(1-methyl-1H-pyrazol-4-yl)pyrimidin-2-amine (Intermediate 11, 90 mg, 0.267 mmol), bis(pinacolato)diboron (136 mg, 0.534 mmol), Pd₂(dba)₃ (24 mg, 0.027 mmol), tricyclohexylphosphine (7.5 mg, 0.027 mmol), KOAc (79 mg, 0.800 mmol) and 1,4-dioxane (2 mL) was heated at 120 °C for 2 h. A portion of the solution (0.5 mL) was added to a mixture of (trans)-3-(2-chloropyridin-4-yl)-3-hydroxy-1-methyl-5-(trifluoromethyl)pyrrolidin-2-one (15 mg, 0.0387 mmol), KOAc (10 mg, 0.1 mmol), BrettPhos-Pd-G3 (4.5 mg, 0.005 mmol), 1,4-dioxane (0.5 mL) and water (0.1 mL) and the resulting mixture was heated at 100 °C for 1 h. The resulting solution was diluted with water and extracted with ethyl acetate. The organic extract was dried with anhydrous Na₂SO₄, filtered, concentrated and combined with two additional batches. The residue was purified by FCC (0-100% EtOAc:petroleum ether) followed by RP-HPLC (Phenomenex C18 column, 3 µm, 75 x 30 mm; eluent: 29% to 53% (v/v) CH₃CN and H₂O with 0.05% NH₃ + 10 mM NH₄HCO₃) to afford (trans)-3-hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one (20 mg). MS (ESI⁺): m/z = 517.1.

Step C: The diastereomers of (*trans*)-3-hydroxy-1-methyl-3-(2-(2-(2-((1-methyl-1H-pyrazol-3-yl)amino)pyrimidin-4-yl)thiazol-4-yl)pyrrolid-4-yl)-5-(trifluoromethyl)pyrrolidine-2-one (17 mg) were separated by SFC (Phenomenex-Cellulose-2 (250 x 30 mm, 10 µm) column; isocratic elution with IPA containing 0.1% of 25% aq. NH₃; 60% supercritical CO₂) to afford a second eluting peak which was designated as diastereomer 1 of (*trans*)-3-hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one (Example 59) (2.2 mg). ¹H NMR (400 MHz, DMSO-*d₆*) δ 9.88 - 9.75 (m, 1H), 8.68 - 8.61 (m, 2H), 8.56 (s, 1H), 8.30 (s, 1H), 7.94 (s, 1H), 7.68 - 7.55 (m, 1H), 7.49 (d, J = 4.8 Hz, 1H), 7.39 - 7.33 (m, 1H), 6.75 (s, 1H), 4.69 - 4.59 (m, 1H), 3.91 - 3.80 (m, 3H), 2.97 (s, 3H), 2.84 - 2.79 (m, 1H), 2.39 - 2.35 (m, 1H). MS (ESI⁺): m/z = 517.1. The first eluting isomer was designated as diastereomer 2 of (*trans*)-3-hydroxy-1-methyl-3-(2-(2-(2-((1-methyl-1H-pyrazol-3-yl)amino)pyrimidin-4-yl)thiazol-4-yl)pyridin-4-yl)-5-(trifluoromethyl)pyrrolidin-2-one (Example 60). ¹H NMR (400 MHz, DMSO-*d₆*) δ 9.87 - 9.76 (m, 1H), 8.69 - 8.61 (m, 2H), 8.59 - 8.47 (m, 1H), 8.30 (s, 1H), 7.97 - 7.91 (m, 1H), 7.67 - 7.55 (m, 1H), 7.49 (d, J = 4.8 Hz, 1H), 7.36 (d, J = 4.0 Hz, 1H), 6.75 (s, 1H), 4.69 - 4.59 (m, 1H), 3.85 (s, 3H), 2.98 (s, 3H), 2.84 - 2.79 (m, 1H), 2.37 - 2.35 (m, 1H). MS (ESI⁺): m/z = 517.2.

### Example 61 and Example 62: (3R,5S)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one and (3S,5R)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one.

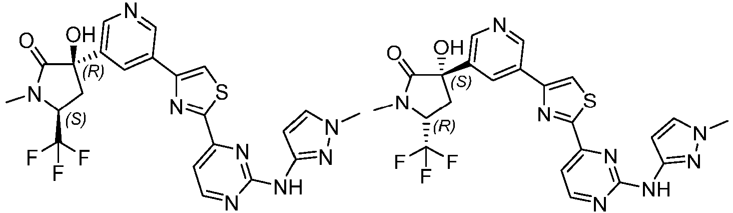

Step A: (trans)-3-(3-bromopyridin-5-yl)-3-hydroxy-1-methyl-5-(trifluoromethyl)pyrrolidine-2-one. To a stirring solution of 3,5-dibromopyridine (916 mg, 3.87 mmol) and **THF** (20 mL) at 0 °C was added dropwise i-PrMgCl•LiCl (4.46 mL, 1.3 M in **THF,** 1.5 eq). The reaction mixture was stirred at 0 °C for 0.5 h and then (R,S)-1-methyl-5-(trifluoromethyl)pyrrolidine-2,3-dione (Intermediate 61, 700 mg, 3.865 mmol) was added and the resulting mixture was stirred at 0 °C for 1 h. The reaction mixture was diluted with saturated aqueous ammonium chloride (20 mL) and then extracted with ethyl acetate. The organic extract was dried with anhydrous Na₂SO₄, filtered, and concentrated. The residue was purified by FCC to afford (trans)-3-(3-bromopyridin-5-yl)-3-hydroxy-1-methyl-5-(trifluoromethyl)pyrrolidine-2-one (214 mg, 16%) as a yellow solid. MS (ESI⁺): m/z = 340.9.

Step B: (5-((*trans*)-3-Hydroxy-1-methyl-2-oxo-5-(trifluoromethyl)pyrrolidin-3-yl)pyridin-3-yl)boronic acid. A mixture of (trans)-3-(3-bromopyridin-5-yl)-3-hydroxy-1-methyl-5-(trifluoromethyl)pyrrolidine-2-one (200 mg, 0.59 mmol), bis(pinacolato)diboron (240 mg, 0.944 mmol), KOAc (173 mg, 1.77 mmol), Pd(dppf)Cl₂ (43 mg, 0.059 mmol), and 1,4-dioxane (10 mL) was heated at 100 °C for 15 h. The mixture was cooled to room temperature, concentrated, and purified by FCC to afford (5-((trans)-3-hydroxy-1-methyl-2-oxo-5-(trifluoromethyl)pyrrolidin-3-yl)pyridin-3-yl)boronic acid (118 mg, 66%) as a gray solid. MS (ESI⁺): m/z = 305.0.

Step C: A mixture of (5-((trans)-3-hydroxy-1-methyl-2-oxo-5-(trifluoromethyl)pyrrolidin-3-yl)pyridin-3-yl)boronic acid (185 mg, 0.122 mmol), 4-(4-bromothiazol-2-yl)-N-(1-methyl-1H-pyrazol-4-yl)pyrimidin-2-amine (Intermediate 11, 49 mg, 0.146 mmol), KOAc (36 mg, 0.365 mmol), Pd(dppf)Cl₂ (9 mg, 0.012 mmol), 1,4-dioxane (5 mL) and water (0.5 mL) was heated at 100 °C for 15 h. The resulting mixture was concentrated and purified by RP-HPLC (column: Xtimate C18 150 x 40 mm, 5 µm) to afford (trans)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one (25 mg, 40%). MS (ESI⁺): m/z = 517.1.

Step D: The diastereomers of (*trans*)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one (25 mg) were separated by SFC (DAICEL CHIRALPAK AD (250 x 30 mm, 10 µm); 50% (v/v) IPA (containing 0.1% of aq. NH₃)/CO₂) to afford a second eluting peak which was designated as diastereomer 1 of (*trans*)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one (Example 61) (4.3 mg, 17%). ¹H NMR (400 MHz, MeOH-*d*₄) δ 9.17 (m, 1H), 8.62 (m, 1H), 8.60 - 8.52 (m, 2H), 8.32 (s, 1H), 8.00 (s, 1H),7.72 - 7.64 (m, 1H), 7.56 (m, 1H), 4.53 - 4.49 (m, 1H), 3.91 (s, 3H), 3.09 (s, 3H), 2.95 - 2.91 (m, 1H), 2.58 - 2.48 (m, 1H). MS (ESI⁺): m/z = 517.2.
The first eluting isomer was designated as diastereomer 2 of (*trans*)-3-hydroxy-1-methyl-3-(5-(2-(2-((1-methyl-1H-pyrazol-3-yl)amino)pyrimidin-4-yl)thiazol-4-yl)pyridin-3-yl)-5-(trifluoromethyl)pyrrolidin-2-one (Example 62) (5.1 mg, 20%). ¹H NMR (400 MHz, MeOH-*d*₄) δ 9.17 (m, 1H), 8.62 (m, 1H), 8.60 - 8.52 (m, 2H), 8.32 (s, 1H), 8.00 (s, 1H),7.72 - 7.64 (m, 1H), 7.56 (m, 1H), 4.53 - 4.49 (m, 1H), 3.91 (s, 3H), 3.09 (s, 3H), 2.95 - 2.91 (m, 1H), 2.58 - 2.48 (m, 1H). MS (ESI⁺): m/z = 517.2.

### Example 63: (R)-3-(3-(4-(2-((1-acetyl-6-methoxyindolin-5-yl)amino)pyrimidin-4-yl)thiazol-2-yl)phenyl)-3-hydroxy-1-methylpyrrolidin-2-one.

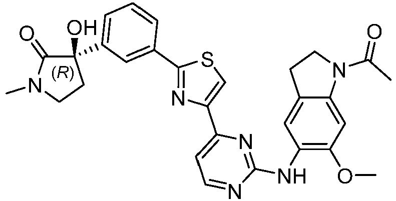

Step A. 1-(6-Methoxyindolin-1-yl)ethanone. Acetyl chloride (0.715 mL, 10.1 mmol) was added dropwise over 5 min to a 50 mL round-bottomed flask containing 6-methoxyindoline (1.00 g, 6.70 mmol), pyridine (1.62 mL, 20.1 mmol), and anhydrous DCM (20 mL) at 0 °C. The resultant mixture was stirred at r.t. for 1 h. After this time, the reaction mixture was poured into saturated aqueous NaHCO₃ (10 mL) and extracted with DCM (30 mL x 3). The combined organic extracts were washed with brine (30 mL), dried over anhydrous Na₂SO₄, filtered, and the filtrate concentrated to dryness *in vacuo* to give a light yellow oil. The light yellow oil was subjected to silica gel chromatography (20-80% EtOAc/pet ether) to afford 1-(6-methoxyindolin-1-yl)ethanone as a white solid (1.2 g, 88%). MS (ESI⁺): m/z = 192.1.

Step B. 1-(6-Methoxy-5-nitroindolin-1-yl)ethanone. 1-(6-Methoxyindolin-1-yl)ethanone (1.20 g, 6.28 mmol), KNO₃ (634 mg, 6.28 mmol), and TFA (20 mL) were added to a 50 mL round-bottomed flask. The resultant mixture was stirred at -15 °C for 2 h. After this time, the reaction mixture was poured into saturated aqueous NaHCO₃ (30 mL) and extracted with CH₂Cl₂ (30 mL x 3). The combined organic extracts were washed with brine (50 mL), dried over anhydrous Na₂SO₄, filtered, and the filtrate concentrated to dryness *in vacuo* to give a brown solid. The brown solid was subjected to silica gel chromatography (30-100% EtOAc/pet ether) to afford 1-(6-methoxy-5-nitroindolin-1-yl)ethanone as a brown solid (1.1 g, 72%). MS (ESI⁺): m/z = 236.6. ¹H NMR (400 Hz, CDCl₃) δ 8.10 (s, 1H), 7.82 (s, 1H), 4.17 (t, *J* = 8.4 Hz, 2H), 3.99 (s, 3H), 3.20 (t, *J* = 8.4 Hz, 2H), 2.29 (s, 3H).

Step C. 1-(5-Amino-6-methoxyindolin-1-yl)ethanone. 1-(6-Methoxy-5-nitroindolin-1-yl)ethanone (990 mg, 4.19 mmol), Fe powder (1.17 g, 21.0 mmol), NH₄Cl (897 mg, 16.8 mmol), anhydrous EtOH (12 mL) and H₂O (3 mL) were added to a 50 mL round-bottomed flask fitted with a reflux condenser, and the resulting mixture was heated for 1 h at 70 °C. The reaction vessel was removed from the oil bath and allowed to gradually cool to room temperature. The reaction mixture was filtered through a pad of diatomaceous earth and the pad washed with EtOAc (40 mL). The filtrate was extracted with EtOAc (20 mL x 3). The combined organic extracts were washed with brine (40 mL), dried over anhydrous Na₂SO₄, filtered, and the filtrate concentrated to dryness *in vacuo* to give a brown oil. The brown oil was subjected to silica gel chromatography (20-100% EtOAc/pet ether) to afford 1-(5-amino-6-methoxyindolin-1-yl)ethanone as a brown solid (500 mg, 48%). MS (ESI⁺): m/z = 206.8.

Step D. (*R*)-3-(3-(4-(2-((1-Acetyl-6-methoxyindolin-5-yl)amino)pyrimidin-4-yl)thiazol-2-yl)phenyl)-3-hydroxy-1-methylpyrrolidin-2-one. (*R*)-3-(3-(4-(2-Chloropyrimidin-4-yl)thiazol-2-yl)phenyl)-3-hydroxy-1-methylpyrrolidin-2-one (Intermediate 35, 100 mg, 0.258 mmol), 1-(5-amino-6-methoxyindolin-1-yl)ethanone (69.3 mg, 0.336 mmol), Pd(OAc)₂ (11.6 mg, 51.7 µmol), Cs₂CO₃ (168 mg, 0.517 mmol), (9,9-dimethyl-9*H*-xanthene-4,5-diyl)bis(diphenylphosphine) (15.0 mg, 25.8 µmol), and anhydrous 1,4-dioxane (5 mL) were added to an oven-dried and nitrogen-purged 10 mL round-bottomed flask fitted with a reflux condenser. The resultant mixture was heated at 100 °C for 1 h and turned black. The reaction vessel was removed from the oil bath and allowed to gradually cool to room temperature. The reaction mixture was poured into H₂O (10 mL) and extracted with ethyl acetate (20 mL x 3). The combined organic extracts were washed with brine (30 mL), dried over anhydrous Na₂SO₄, filtered, and the filtrate concentrated to dryness *in vacuo* to give a brown oil. The brown oil was purified by silica gel chromatography (0-10% MeOH/CH₂Cl₂) to afford the product as a yellow solid. This solid was further purified by preparative TLC (100% EA) to give (R)-3-(3-(4-(2-((1-acetyl-6-methoxyindolin-5-yl)amino)pyrimidin-4-yl)thiazol-2-yl)phenyl)-3-hydroxy-1-methylpyrrolidin-2-one as a yellow solid (10.3 mg, 7%). MS (ESI⁺): m/z = 557.3 [M+H]⁺. ¹H NMR (400 MHz, CDCl₃) δ 8.53 (d, *J* = 4.8 Hz, 1H), 8.40 (s, 1H), 8.20 (s, 1H), 8.10 (s, 1H), 8.00 (s, 1H), 7.95 - 7.91 (m, 1H), 7.73 (s, 1H), 7.57 (d, *J* = 5.2 Hz, 1H), 7.46 - 7.39 (m, 2H), 4.11 - 4.05 (m, 2H), 3.94 (s, 3H), 3.55 - 3.47 (m, 2H), 3.45 - 3.38 (m, 1H), 3.26 - 3.21 (m, 2H), 3.06 (s, 3H), 2.58 - 2.50 (m, 1H), 2.47 - 2.41 (m, 1H), 2.23 (s, 3H).
The compounds shown in Table 3A were prepared in a manner analogous to Example 43 using (*R*)-3-hydroxy-1-methyl-3-(3-(2-(2-(methylsulfonyl)pyrimidin-4-yl)thiazol-4-yl)phenyl)pyrrolidin-2-one (Intermediate 9) or (*R*)-3-hydroxy-1-methyl-3-(3-(1-(2-(methylsulfonyl)pyrimidin-4-yl)-1H-pyrazol-3-yl)phenyl)pyrrolidin-2-one (Intermediate 13) in place of (*R*)-3-hydroxy-1-methyl-3-(3-(4-(2-(methylsulfonyl)pyrimidin-4-yl)thiazol-2-yl)phenyl)pyrrolidin-2-one (Intermediate 12) and the appropriate amine in place of 2-methyl-4-aminopyridine. Purification methods are noted in the table.

**Table 3A:**

| # | Structure | Analytical data | RP HPLC conditions | Yield (%) |
|---|---|---|---|---|
| | Name | | | |
| 64 | 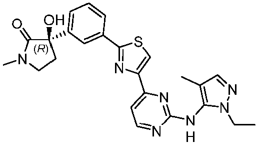 | ¹H NMR (500 MHz, DMSO-*d₆*) δ 8.99 (d, *J* = 5.1 Hz, 1H), 8.91 (d, *J* = 1.2 Hz, 1H), 8.86 (s, 1H), 8.35 (s, 2H), 8.19 - 8.11 (m, 1H), 8.09 (d, *J =* 5.1 Hz, 1H), 7.98 (ddd, *J* = 7.6, 1.9, 1.2 Hz, 1H), 7.59 - 7.50 (m, 1H), 7.48 (ddd, *J* = 7.9, 1.9, 1.2 Hz, 1H), 6.24 (s, 1H), 4.78 (q, *J* = 7.0 Hz, 2H), 3.60 - 3.18 (m, 2H), 2.87 (s, 3H), 2.44 - 2.34 (m, 1H), 2.34 - 2.19 (m, 1H), 2.06 (d, *J =* 1.0 Hz, 3H), 1.30 (t, *J* = 7.0 Hz, 3H). MS (ESI⁺): m/z = 476.10. | acidic conditions, column C1 | 8 |
| | (*R*)-3-(3-(4-(2-((1-ethyl-4-methyl-1H-pyrazol-5-yl)amino)pyrimidin-4-yl)thiazol-2-yl)phenyl)-3-hydroxy-1-methylpyrrolidin-2-one | | | |
| 65 | 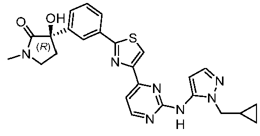 | ¹H NMR (500 MHz, DMSO-*d₆*) δ 8.79 (d, *J =* 5.1 Hz, 1H), 8.72 (s, 1H), 8.12 (t, *J* = 1.9 Hz, 1H), 7.97 (ddd, *J* = 7.6, 1.9, 1.1 Hz, 1H), 7.90 (s, 1H), 7.73 (d, *J* = 5.1 Hz, 1H), 7.53 (t, *J* = 7.7 Hz, 1H), 7.46 (ddd, *J* = 7.8, 1.8, 1.2 Hz, 1H), 6.61 (s, 1H), 3.89 (d, *J* = 6.9 Hz, 2H), 3.66 - 3.33 (m, 2H), 2.39 (ddd, *J* = 13.1, 7.8, 5.1 Hz, 1H), 2.29 (ddd, *J* = 13.4, 8.1, 5.5 Hz, 1H), 1.31 - 1.18 (m, 1H), 0.53 - 0.45 (m, 2H), 0.42 - 0.33 (m, 2H). ). MS (ESI⁺): m/z = 488.1. | acidic conditions column C1 | 6 |
| | (*R*)-3-(3-(4-(2-((1-(cyclopropylmethyl)-1H-pyrazol-5-yl)amino)pyrimidin-4-yl)thiazol-2-yl)phenyl)-3-hydroxy-1-methylpyrrolidin-2-one | | | |
| 66 | 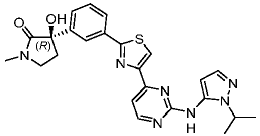 | ¹H NMR (600 MHz, DMSO-*d₆*) δ 8.79 (d, *J =* 5.1 Hz, 1H), 8.70 (s, 1H), 8.13 (t, *J* = 1.8 Hz, 1H), 8.00 - 7.95 (m, 1H), 7.92 (s, 1H), 7.73 (d, *J* = 5.1 Hz, 1H), 7.53 (t, *J = 7.7* Hz, 1H), 7.47 (dt, *J* = 7.8, 1.4 Hz, 1H), 6.60 (s, 2H), 6.23 (s, 1H), 4.58 - 4.49 (m, 1H), 3.54 - 3.46 (m, 1H), 3.43 - 3.37 (m, 2H), 2.88 (s, 3H), 2.43 - 2.35 (m, 1H), 2.33 - 2.22 (m, 1H), 1.37 (d, *J* = 6.5 Hz, 6H). MS (ESI⁺): m/z = 476.4. | acidic conditions, column C1 | 8 |
| | (*R*)-3-hydroxy-3-(3-(4-(2-((1-isopropyl-1H-pyrazol-5-yl)amino)pyrimidin-4-yl)thiazol-2-yl)phenyl)-1-methylpyrrolidin-2-one | | | |
| 67 | 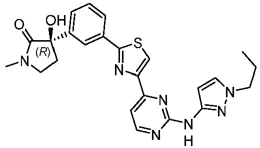 | ¹H NMR (600 MHz, DMSO-*d₆*) δ 9.79 (s, 1H), 8.58 (d, *J* = 4.9 Hz, 1H), 8.46 (s, 1H), 8.12 (t, *J =* 1.9 Hz, 1H), 7.97 - 7.91 (m, 1H), 7.63 (d, *J = 2.2* Hz, 1H), 7.52 (t, *J* = 7.7 Hz, 1H), 7.49 (d, *J* = 4.9 Hz, 1H), 7.48 - 7.43 (m, 1H), 6.77 (s, 1H), 6.23 (s, 1H), 3.98 (t, *J* = 6.9 Hz, 2H), 3.55 - 3.46 (m, 1H), 3.44 - 3.36 (m, 1H), 2.87 (s, 3H), 2.46 - 2.34 (m, 1H), 2.35 - 2.23 (m, 1H), 1.79 (h, *J* = 7.2 Hz, 2H), 0.86 (t, *J* = 7.4 Hz, 3H). MS (ESI⁺): m/z = 476.5. | acidic conditions, column C1 | 3 |
| | (*R*)-3-hydroxy-1-methyl-3-(3-(4-(2-((1-propyl-1H-pyrazol-3-yl)amino)pyrimidin-4-yl)thiazol-2-yl)phenyl)pyrrolidin-2-one | | | |
| 68 | 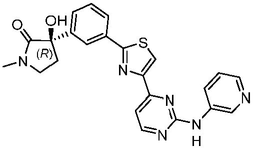 | ¹H NMR (500 MHz, DMSO-*d₆*) δ 9.44 (t, *J* = 2.1 Hz, 1H), 9.40 (ddd, J = 6.0, 1.8, 1.0 Hz, 1H), 9.27 (d, *J =* 5.1 Hz, 1H), 9.10 (s, 1H), 8.46 (d, *J =* 5.1 Hz, 1H), 8.17 (t, *J =* 1.8 Hz, 1H), 8.05 - 7.88 (m, 3H), 7.56 (t, *J* = 7.7 Hz, 1H), 7.50 (dt, *J* = 7.9, 1.4 Hz, 1H), 6.92 (s, 2H), 6.24 (s, 1H), 3.55 - 3.45 (m, 1H), 3.44 - 3.37 (m, 1H), 2.88 (s, 3H), 2.40 (ddd, *J* = 13.0, 7.8, 5.1 Hz, 1H), 2.30 (ddd, *J* = 13.4, 8.1, 5.4 Hz, 1H). MS (ESI⁺): m/z = 445.0. | acidic conditions, column C1 | 5 |
| | (*R*)-3-hydroxy-1-methyl-3-(3-(4-(2-(pyridin-3-ylamino)pyrimidin-4-yl)thiazol-2-yl)phenyl)pyrrolidin-2-one | | | |
| 69 | 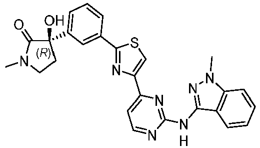 | ¹H NMR (500 MHz, DMSO-*d₆*) δ 9.83 (s, 1H), 8.55 (d, *J* = 5.0 Hz, 1H), 8.13 - 8.07 (m, 2H), 7.93 (dt, *J* = 7.7, 1.4 Hz, 1H), 7.65 (dd, *J* = 8.2, 1.1 Hz, 1H), 7.63 - 7.58 (m, 1H), 7.55 - 7.48 (m, 2H), 7.45 (dt, *J* = 7.8, 1.5 Hz, 1H), 7.40 (ddd, *J* = 8.4, 6.8, 1.1 Hz, 1H), 7.10 - 7.03 (m, 1H), 4.00 (s, 3H), 3.55 - 3.35 (m, 2H), 2.86 (s, 3H), 2.41 - 2.36 (m, 1H), 2.28 (ddd, *J =* 13.4, 8.1, 5.5 Hz, 2H). MS (ESI⁺): m/z = 498.1. | acidic conditions, column C1 | 15 |
| | (*R*)-3-hydroxy-1-methyl-3-(3-(4-(2-((1-methyl-1H-indazol-3-yl)amino)pyrimidin-4-yl)thiazol-2-yl)phenyl)pyrrolidin-2-one | | | |
| 70 | 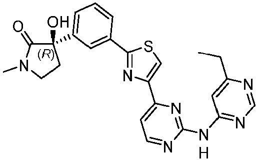 | ¹H NMR (600 MHz, DMSO-*d₆*) δ 8.83 (d, *J = 5.0* Hz, 1H), 8.78 (s, 1H), 8.57 (s, 1H), 8.38 (s, 1H), 8.14 (t, *J* = 1.9 Hz, 1H), 7.98 (dt, *J =* 7.7, 1.5 Hz, 1H), 7.80 (d, *J* = 5.0 Hz, 1H), 7.54 (t, *J* = 7.7 Hz, 1H), 7.47 (dt, *J* = 7.8, 1.5 Hz, 1H), 3.52 - 3.30 (m, 2H), 2.88 (s, 3H), 2.81 (q, *J =* 7.6 Hz, 2H), 2.42 - 2.35 (m, 1H), 2.30 (ddd, *J =* 13.4, 8.1, 5.4 Hz, 1H), 1.30 (t, *J =* 7.6 Hz, 3H). MS (ESI⁺): m/z = 474.4. | 1) basic conditions, column C2; | 2 |
| | | | 2) acidic conditions, column C1 | |
| | (*R*)-3-(3-(4-(2-((6-ethylpyrimidin-4-yl)amino)pyrimidin-4-yl)thiazol-2-yl)phenyl)-3-hydroxy-1-methylpyrrolidin-2-one | | | |
| 71 | 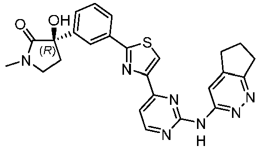 | ¹H NMR (500 MHz, DMSO-*d₆*) δ 10.31 (s, 1H), 8.70 (d, *J =* 5.1 Hz, 1H), 8.52 (s, 1H), 8.43 (d, *J =* 1.3 Hz, 1H), 8.17 - 8.08 (m, 1H), 7.97 (ddd, *J* = 7.6, 1.9, 1.1 Hz, 1H), 7.66 (d, *J* = 5.1 Hz, 1H), 7.57 - 7.48 (m, 1H), 7.46 (ddd, *J* = 7.8, 1.9, 1.1 Hz, 1H), 6.23 (s, 1H), 3.55 - 3.44 (m, 1H), 3.39 (ddd, *J =* 10.0, 7.7, 5.5 Hz, 1H), 3.09 - 2.98 (m, 4H), 2.87 (s, 3H), 2.38 (ddd, *J* = 13.1, 7.8, 5.1 Hz, 1H), 2.29 (ddd, *J =* 13.4, 8.1, 5.5 Hz, 1H), 2.11 (p, *J* = 7.5 Hz, 2H). MS (ESI⁺): m/z = 486.1. | basic conditions, column C2 | 4 |
| | (*R*)-3-(3-(4-(2-((6,7-dihydro-5H-cyclopenta[c]pyridazin-3-yl)amino)pyrimidin-4-yl)thiazol-2-yl)phenyl)-3-hydroxy-1-methylpyrrolidin-2-one | | | |
| 72 | 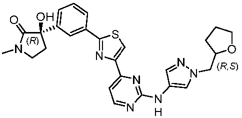 | ¹H NMR (600 MHz, DMSO-*d₆*) δ 9.58 (s, 1H), 8.65 - 8.44 (m, 2H), 8.13 (t, *J =* 1.9 Hz, 1H), 7.96 (d, *J =* 7.6 Hz, 1H), 7.58 (s, 1H), 7.53 (t, *J =* 7.7 Hz, 1H), 7.48 - 7.41 (m, 2H), 4.17 (s, 3H), 3.85 - 3.74 (m, 1H), 3.67 (q, *J* = 7.2 Hz, 1H), 3.53 - 3.40 (m, 2H), 2.88 (s, 3H), 2.44 - 2.35 (m, 1H), 2.35 - 2.26 (m, 1H), 2.01 - 1.87 (m, 1H), 1.87 - 1.73 (m, 2H), 1.62 (d, *J =* 11.5 Hz, 1H). MS (ESI⁺): m/z = 518.2. | 1) basic conditions, column C2;2) acidic conditions, column C1 | 6 |
| | (*R*)-3-hydroxy-1-methyl-3-(3-(4-(2-((1-(((*R*,*S*)-tetrahydrofuran-2-yl)methyl)-1 H-pyrazol-4-yl)amino)pyrimidin-4-yl)thiazol-2-yl)phenyl)pyrrolidin-2-one | | | |
| 73 | 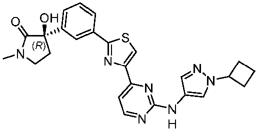 | ¹H NMR (500 MHz, DMSO-*d₆*) δ 9.57 (s, 1H), 8.66 - 8.43 (m, 2H), 8.15 - 8.10 (m, 1H), 8.05 (s, 1H), 7.95 (d, *J =* 7.7 Hz, 1H), 7.63 (s, 1H), 7.55 - 7.50 (m, 1H), 7.49 - 7.42 (m, 2H), 6.23 (s, 1H), 4.88 (s, 1H), 3.49 (ddd, *J* = 9.9, 8.1, 5.0 Hz, 1H), 3.39 (ddd, *J =* 10.0, 7.8, 5.5 Hz, 1H), 2.87 (s, 3H), 2.50 - 2.42 (m, 2H), 2.38 (ddt, *J =* 11.7, 8.8, 4.9 Hz, 3H), 2.29 (ddd, *J* = 13.4, 8.1, 5.5 Hz, 1H), 1.76 (dtd, *J =* 18.4, 10.8, 8.1 Hz, 2H). MS (ESI⁺): m/z = 488.10. | basic conditions, column C2 | 11 |
| | (*R*)-3-(3-(4-(2-((1-cyclobutyl-1H-pyrazol-4-yl)amino)pyrimidin-4-yl)thiazol-2-yl)phenyl)-3-hydroxy-1-methylpyrrolidin-2-one | | | |
| 74 | 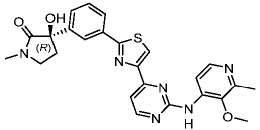 | ¹H NMR (600 MHz, DMSO-*d₆*) δ 9.58 (s, 1H), 8.89 (d, *J* = 5.1 Hz, 1H), 8.80 (d, *J =* 7.1 Hz, 1H), 8.77 (s, 1H), 8.37 (s, 1H), 8.17 (t, *J =* 1.9 Hz, 1H), 8.03 - 7.95 (m, 1H), 7.89 (d, *J* = 5.1 Hz, 1H), 7.55 (t, *J* = 7.7 Hz, 1H), 7.48 (dt, *J* = 7.7, 1.5 Hz, 1H), 6.51 (s, 1H), 4.09 (s, 3H), 3.54 - 3.35 (m, 2H), 2.88 (s, 3H), 2.63 (s, 3H), 2.44 - 2.38 (m, 1H), 2.31 (ddd, *J* = 13.4, 8.1, 5.4 Hz, 1H). MS (ESI⁺): m/z = 489.3. | 1) basic conditions, column C2; | 3 |
| | (*R*)-3-hydroxy-3-(3-(4-(2-((3-methoxy-2-methylpyridin-4-yl)amino)pyrimidin-4-yl)thiazol-2-yl)phenyl)-1-methylpyrrolidin-2-one | | | |
| | | | 2) acidic conditions, column C1 | |
| 75 | 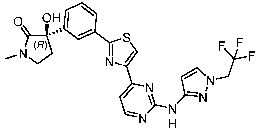 | ¹H NMR (600 MHz, DMSO-*d₆*) δ 10.04 (s, 1H), 8.60 (d, *J* = 5.0 Hz, 1H), 8.48 (s, 1H), 8.12 (t, *J =* 1.9 Hz, 1H), 7.95 (dt, *J =* 7.7, 1.5 Hz, 1H), 7.77 (d, *J =* 2.4 Hz, 1H), 7.57 - 7.34 (m, 3H), 6.94 (d, *J =* 2.4 Hz, 1H), 5.03 (q, *J =* 9.1 Hz, 2H), 2.87 (s, 3H), 2.42 - 2.33 (m, 1H), 2.33 - 2.19 (m, 1H). MS (ESI⁺): m/z = 516.2. | basic conditions, column C2 | 14 |
| | (*R*)-3-hydroxy-1-methyl-3-(3-(4-(2-((1-(2,2,2-trifluoroethyl)-1H-pyrazol-3-yl)amino)pyrimidin-4-yl)thiazol-2-yl)phenyl)pyrrolidin-2-one | | | |
| 76 | 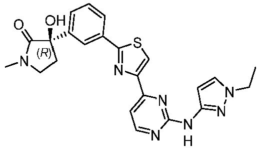 | ¹H NMR (500 MHz, DMSO-*d₆*) δ 9.80 (s, 1H), 8.58 (d, *J* = 4.9 Hz, 1H), 8.45 (s, 1H), 8.12 (t, *J =* 1.9 Hz, 1H), 7.95 (ddd, *J =* 7.6, 1.9, 1.1 Hz, 1H), 7.63 (d, *J =* 2.2 Hz, 1H), 7.57 - 7.41 (m, 3H), 6.76 (d, *J =* 2.2 Hz, 1H), 6.23 (s, 1H), 4.06 (q, *J =* 7.2 Hz, 2H), 3.49 (ddd, *J* = 9.9, 8.1, 5.0 Hz, 1H), 3.42 - 3.36 (m, 1H), 2.87 (s, 3H), 2.38 (ddd, *J* = 12.9, 7.7, 5.0 Hz, 1H), 2.29 (ddd, *J* = 13.4, 8.1, 5.5 Hz, 1H), 1.38 (t, *J* =7.2 Hz, 3H). MS (ESI⁺): m/z = 462.2. | basic conditions, column C2 | 18 |
| | (*R*)-3-(3-(4-(2-((1-ethyl-1H-pyrazol-3-yl)amino)pyrimidin-4-yl)thiazol-2-yl)phenyl)-3-hydroxy-1-methylpyrrolidin-2-one | | | |
| 77 | 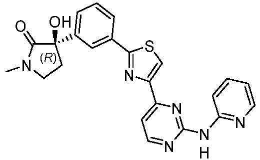 | ¹H NMR (600 MHz, DMSO-*d₆*) δ 9.73 (s, 1H), 8.64 (d, *J* = 5.0 Hz, 1H), 8.52 (s, 1H), 8.37 (dt, *J* = 8.4, 1.0 Hz, 1H), 8.27 - 8.22 (m, 1H), 8.07 (t, *J =* 1.8 Hz, 1H), 7.92 - 7.87 (m, 1H), 7.80 - 7.74 (m, 1H), 7.58 (d, *J =* 5.0 Hz, 1H), 7.46 (t, *J =* 7.7 Hz, 1H), 7.42 - 7.37 (m, 1H), 6.99 - 6.94 (m, 1H), 6.16 (s, 1H), 3.46 - 3.38 (m, 1H), 3.36 - 3.30 (m, 1H), 2.80 (s, 3H), 2.36 - 2.29 (m, 1H), 2.27 - 2.16 (m, 1H). MS (ESI⁺): m/z = 445.05 | acidic conditions, column C1 | 4 |
| | (*R*)-3-hydroxy-1-methyl-3-(3-(4-(2-(pyridin-2-ylamino)pyrimidin-4-yl)thiazol-2-yl)phenyl)pyrrolidin-2-one | | | |
| 78 | 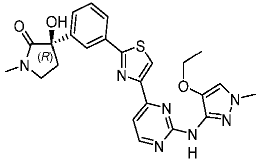 | ¹H NMR (500 MHz, DMSO-*d₆*) δ 8.50 (d, *J =* 4.9 Hz, 1H), 8.44 (s, 1H), 8.36 (s, 1H), 8.14 - 8.05 (m, 1H), 7.97 - 7.91 (m, 1H), 7.81 (s, 1H), 7.51 (t, *J =* 7.7 Hz, 1H), 7.47 - 7.42 (m, 1H), 7.41 (d, *J =* 5.0 Hz, 1H), 6.22 (s, 1H), 4.17 (q, *J =* 7.0 Hz, 2H), 3.71 (s, 3H), 3.52 - 3.43 (m, 1H), 3.43 - 3.34 (m, 1H), 2.87 (s, 3H), 2.42 - 2.33 (m, 1H), 2.33 - 2.22 (m, 1H), 1.29 (t, *J* = 7.0 Hz, 3H). MS (ESI⁺): m/z = 492.2. | basic conditions, column C2 | 19 |
| | (*R*)-3-(3-(4-(2-((4-ethoxy-1-methyl-1H-pyrazol-3-yl)amino)pyrimidin-4-yl)thiazol-2-yl)phenyl)-3-hydroxy-1-methylpyrrolidin-2-one | | | |
| 79 | 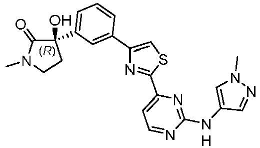 | ¹H NMR (500 MHz, DMSO-*d₆*) δ 9.77 (s, 1H), 8.63 (d, *J* = 4.9 Hz, 1H), 8.37 (s, 1H), 8.15 - 8.01 (m, 1H), 8.00 - 7.90 (m, 2H), 7.62 (s, 1H), 7.48 - 7.41 (m, 2H), 7.39 - 7.29 (m, 1H), 3.85 (s, 3H), 3.53 - 3.26 (m, 2H), 2.87 (s, 3H), 2.44 - 2.35 (m, 1H), 2.33 - 2.24 (m, 1H). MS (ESI⁺): m/z = 448.3. | acidic conditions, column C7 | 54 |
| | (*R*)-3-hydroxy-1-methyl-3-(3-(2-(2-((1-methyl-1H-pyrazol-4-yl)amino)pyrimidin-4-yl)thiazol-4-yl)phenyl)pyrrolidin-2-one | | | |
| 80 | 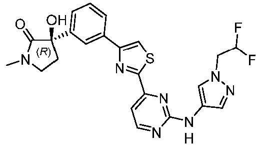 | ¹H NMR (400 MHz, DMF-d₇) δ 10.40 - 10.16 (m, 1H), 9.08 (d, *J =* 5.0 Hz, 1H), 8.82 (s, 1H), 8.59 - 8.44 (m, 2H), 8.38 (dt, *J =* 7.7, 1.4 Hz, 1H), 8.15 (s, 1H), 7.95 - 7.82 (m, 2H), 7.79 (dt, J= 7.8, 1.4 Hz, 1H), 6.95 - 6.61 (m, 1H), 5.16 - 4.96 (m, 2H), 3.94 - 3.84 (m, 1H), 3.84 - 3.78 (m, 1H), 3.30 (s, 3H), 2.86 - 2.77 (m, 1H), 2.76 - 2.66 (m, 1H). MS (ESI⁺): m/z = 498.1. | acidic conditions, column C7 | 22 |
| | (*R*)-3-(3-(2-(2-((1-(2,2-difluoroethyl)-1H-pyrazol-4-yl)amino)pyrimidin-4-yl)thiazol-4-yl)phenyl)-3-hydroxy-1-methylpyrrolidin-2-one | | | |
| 81 | 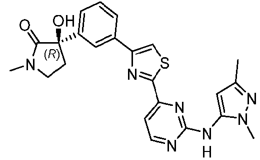 | ¹H NMR (500 MHz, DMSO-*d₆*) δ 8.86 (d, *J* = 5.1 Hz, 1H), 8.39 (s, 1H), 8.14 - 8.07 (m, 1H), 7.96 (dt, *J* = 7.7, 1.5 Hz, 1H), 7.73 (d, *J* = 5.1 Hz, 1H), 7.46 (t, *J* = 7.7 Hz, 1H), 7.36 (dt, *J* = 7.7, 1.5 Hz, 1H), 3.56 (s, 3H), 2.87 (s, 3H), 2.43 - 2.36 (m, 1H), 2.32 - 2.25 (m, 1H). MS (ESI⁺): m/z = 462.4. | acidic conditions, column C7 | 3 |
| | (*R*)-3-(3-(2-(2-((1,3-dimethyl-1H-pyrazol-5-yl)amino)pyrimidin-4-yl)thiazol-4-yl)phenyl)-3-hydroxy-1-methylpyrrolidin-2-one | | | |
| 82 | 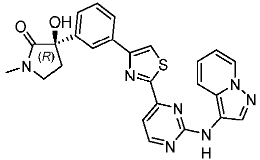 | ¹H NMR (500 MHz, DMSO-*d₆*) δ 9.84 (s, 1H), 8.70 - 8.61 (m, 1H), 8.58 (dt, *J* = 7.1, 1.1 Hz, 1H), 8.54 - 8.38 (m, 1H), 8.35 (s, 1H), 8.09 (t, *J =* 1.8 Hz, 1H), 7.98 - 7.91 (m, 1H), 7.87 (d, *J =* 9.0 Hz, 1H), 7.50 (d, *J* = 4.9 Hz, 1H), 7.46 (t, *J* = 7.7 Hz, 1H), 7.36 (ddd, *J* = 7.8, 1.9, 1.2 Hz, 1H), 7.13 (ddd, *J* = 9.0, 6.6, 1.0 Hz, 1H), 6.89 - 6.81 (m, 1H), 6.11 (s, 1H), 3.51 - 3.43 (m, 1H), 3.42 - 3.37 (m, 1H), 2.87 (s, 3H), 2.44 - 2.35 (m, 1H), 2.32 - 2.23 (m, 1H). MS (ESI⁺): m/z = 484.3. | basic conditions, column C8 | 20 |
| | (*R*)-3-hydroxy-1-methyl-3-(3-(2-(2-(pyrazolo[1,5-a]pyridin-3-ylamino)pyrimidin-4-yl)thiazol-4-yl)phenyl)pyrrolidin-2-one | | | |
| 83 | 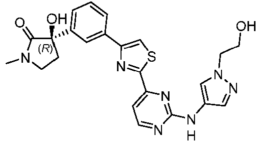 | ¹H NMR (500 MHz, DMSO-*d₆*) δ 9.97 (s, 1H), 8.82 (d, *J* = 4.9 Hz, 1H), 8.57 (s, 1H), 8.32 - 8.24 (m, 1H), 8.20 (s, 1H), 8.17 - 8.10 (m, 1H), 7.84 (s, 1H), 7.67 - 7.61 (m, 2H), 7.58 - 7.50 (m, 1H), 4.38 - 4.30 (m, 2H), 3.97 - 3.90 (m, 2H), 3.71 - 3.62 (m, 1H), 3.61 - 3.53 (m, 1H), 3.06 (s, 3H), 2.61 - 2.53 (m, 1H), 2.52 - 2.43 (m, 1H). MS (ESI⁺): m/z = 478.3. | acidic conditions, column C7 | 87 |
| | (*R*)-3-hydroxy-3-(3-(2-(2-((1-(2-hydroxyethyl)-1H-pyrazol-4-yl)amino)pyrimidin-4-yl)thiazol-4-yl)phenyl)-1-methylpyrrolidin-2-one | | | |
| 84 | 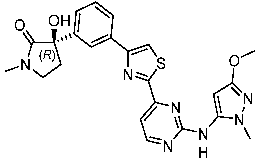 | ¹H NMR (500 MHz, DMSO-*d₆*) δ 8.83 (d, *J = 5.0* Hz, 1H), 8.39 (s, 1H), 8.10 (t, *J* = 1.9 Hz, 1H), 8.02 - 7.92 (m, 1H), 7.69 (d, *J* = 5.0 Hz, 1H), 7.47 (t, *J =* 7.7 Hz, 1H), 7.42 - 7.32 (m, 1H), 6.78 (s, 2H), 6.11 (s, 1H), 3.86 (s, 3H), 3.51 (s, 3H), 3.50 - 3.46 (m, 1H), 3.43 - 3.37 (m, 1H), 2.88 (s, 3H), 2.43 - 2.34 (m, 1H), 2.33 - 2.22 (m, 1H). MS (ESI⁺): m/z = 478.5. | basic conditions, column C8 | 11 |
| | (*R*)-3-hydroxy-3-(3-(2-(2-((3-methoxy-1-methyl-1H-pyrazol-5-yl)amino)pyrimidin-4-yl)thiazol-4-yl)phenyl)-1-methylpyrrolidin-2-one | | | |
| 85 | 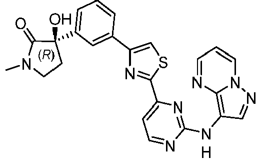 | ¹H NMR (500 MHz, DMSO-*d₆*) δ 9.43 (s, 1H), 9.13 - 8.97 (m, 1H), 8.69 - 8.52 (m, 2H), 8.53 - 8.46 (m, 1H), 8.34 (s, 1H), 8.11 - 8.05 (m, 1H), 7.98 - 7.89 (m, 1H), 7.50 (d, *J* = 4.9 Hz, 1H), 7.46 (t, *J* = 7.7 Hz, 1H), 7.40 - 7.33 (m, 1H), 7.11 - 6.95 (m, 1H), 6.11 (s, 1H), 3.52 - 3.44 (m, 1H), 3.43 - 3.36 (m, 1H), 2.88 (s, 3H), 2.44 - 2.35 (m, 1H), 2.34 - 2.22 (m, 1H). MS (ESI⁺): m/z = 485.5. | basic conditions, column C8 | 41 |
| | (*R*)-3-hydroxy-1-methyl-3-(3-(2-(2-(pyrazolo[1,5-a]pyrimidin-3-ylamino)pyrimidin-4-yl)thiazol-4-yl)phenyl)pyrrolidin-2-one | | | |
| 86 | 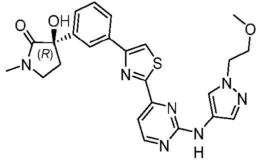 | ¹H NMR (500 MHz, DMSO-*d₆*) δ 9.79 (s, 1H), 8.64 (d, *J* = 4.9 Hz, 1H), 8.40 (s, 1H), 8.15 - 8.06 (m, 1H), 8.02 (s, 1H), 7.98 - 7.92 (m, 1H), 7.64 (s, 1H), 7.51 - 7.42 (m, 2H), 7.41 - 7.30 (m, 1H), 6.11 (s, 1H), 4.32 - 4.17 (m, 2H), 3.74 - 3.64 (m, 2H), 3.53 - 3.44 (m, 1H), 3.43 - 3.35 (m, 1H), 3.28 (s, 3H), 2.88 (s, 3H), 2.44 - 2.36 (m, 1H), 2.33 - 2.24 (m, 1H). MS (ESI⁺): m/z = 492.4. | basic conditions, column C8 | 24 |
| | (*R*)-3-hydroxy-3-(3-(2-(2-((1-(2-methoxyethyl)-1H-pyrazol-4-yl)amino)pyrimidin-4-yl)thiazol-4-yl)phenyl)-1-methylpyrrolidin-2-one | | | |
| 87 | 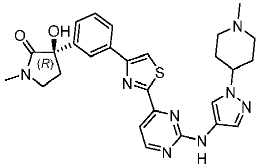 | ¹H NMR (500 MHz, DMSO-*d₆*) δ 9.80 (s, 1H), 8.63 (d, *J* = 4.9 Hz, 1H), 8.45 - 8.31 (m, 1H), 8.15 - 8.08 (m, 1H), 8.05 (s, 1H), 7.99 - 7.88 (m, 1H), 7.64 (s, 1H), 7.52 - 7.41 (m, 2H), 7.41 - 7.26 (m, 1H), 6.11 (s, 1H), 4.18 - 4.00 (m, 1H), 3.51 - 3.45 (m, 1H), 3.43 - 3.37 (m, 1H), 2.92 - 2.83 (m, 5H), 2.44 - 2.34 (m, 1H), 2.34 - 2.25 (m, 1H), 2.22 (s, 3H), 2.14 - 1.90 (m, 6H). MS (ESI⁺): m/z = 531.4. | basic conditions, column C8 | 56 |
| | (*R*)-3-hydroxy-1-methyl-3-(3-(2-(2-((1-(1-methylpiperidin-4-yl)-1H-pyrazol-4-yl)amino)pyrimidin-4-yl)thiazol-4-yl)phenyl)pyrrolidin-2-one | | | |
| 88 | 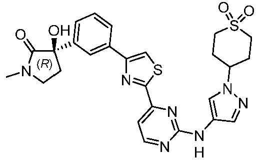 | ¹H NMR (500 MHz, DMSO-*d₆*) δ 9.84 (s, 1H), 8.65 (d, *J* = 5.0 Hz, 1H), 8.39 (s, 1H), 8.13 - 8.08 (m, 1H), 8.05 (s, 1H), 7.96 (d, *J =* 7.9 Hz, 1H), 7.71 (s, 1H), 7.53 - 7.42 (m, 2H), 7.42 - 7.31 (m, 1H), 6.11 (s, 1H), 4.72 - 4.54 (m, 1H), 3.54 - 3.37 (m, 4H), 3.28 - 3.17 (m, 2H), 2.88 (s, 3H), 2.50 - 2.34 (m, 5H), 2.34 - 2.23 (m, 1H). MS (ESI⁺): m/z = 566.4. | basic conditions, column C8 | 52 |
| | (*R*)-3-(3-(2-(2-((1-(1,1-dioxidotetrahydro-2H-thiopyran-4-yl)-1H-pyrazol-4-yl)amino)pyrimidin-4-yl)thiazol-4-yl)phenyl)-3-hydroxy-1-methylpyrrolidin-2-one | | | |
| 89 | 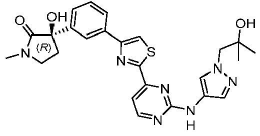 | ¹H NMR (500 MHz, DMSO-*d₆*) δ 9.79 (s, 1H), 8.64 (d, *J* = 4.9 Hz, 1H), 8.38 (s, 1H), 8.13 - 8.07 (m, 1H), 8.04 (s, 1H), 7.98 - 7.91 (m, 1H), 7.65 (s, 1H), 7.52 - 7.42 (m, 2H), 7.42 - 7.32 (m, 1H), 6.11 (s, 1H), 4.71 (s, 1H), 4.02 (s, 2H), 3.54 - 3.44 (m, 1H), 3.44 - 3.37 (m, 1H), 2.88 (s, 3H), 2.44 - 2.34 (m, 1H), 2.34 - 2.24 (m, 1H), 1.10 (s, 6H). MS (ESI⁺): m/z = 507.0. | basic conditions, column C8 | 56 |
| | (*R*)-3-hydroxy-3-(3-(2-(2-((1-(2-hydroxy-2-methylpropyl)-1H-pyrazol-4-yl)amino)pyrimidin-4-yl)thiazol-4-yl)phenyl)-1-methylpyrrolidin-2-one | | | |
| 90 | 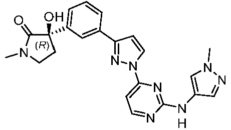 | ¹H NMR (500 MHz, DMSO-*d₆*) δ 9.71 (s, 1H), 8.71 (s, 1H), 8.53 (d, *J* = 5.4 Hz, 1H), 8.02 (t, *J* = 1.8 Hz, 1H), 7.97 (s, 1H), 7.89 - 7.83 (m, 1H), 7.53 (s, 1H), 7.45 (t, *J =* 7.7 Hz, 1H), 7.39 - 7.33 (m, 1H), 7.26 (d, *J* = 5.3 Hz, 1H), 7.14 (d, *J* = 2.7 Hz, 1H), 6.11 (s, 1H), 3.86 (s, 3H), 3.51 - 3.44 (m, 1H), 3.42 - 3.35 (m, 1H), 2.86 (s, 3H), 2.38 - 2.34 (m, 1H), 2.32 - 2.23 (m, 1H). MS (ESI⁺): m/z = 431. | acidic conditions, column C1 | 10 |
| | (*R*)-3-hydroxy-1-methyl-3-(3-(1-(2-((1-methyl-1H-pyrazol-4-yl)amino)pyrimidin-4-yl)-1H-pyrazol-3-yl)phenyl)pyrrolidin-2-one | | | |
| 91 and 92 | 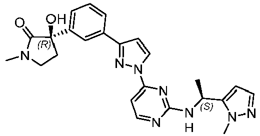 | Diastereomer 1 (Example 91): ¹H NMR (500 MHz, MeOH-*d*₄) δ 8.60 (d, *J* = 2.8 Hz, 1H), 8.36 (d, *J =* 5.5 Hz, 1H), 8.07 - 8.03 (m, 1H), 7.89 - 7.83 (m, 1H), 7.48 - 7.43 (m, 1H), 7.43 - 7.39 (m, 1H), 7.37 (d, *J* = 2.0 Hz, 1H), 7.25 (d, *J* = 5.5 Hz, 1H), 6.95 (d, *J* = 2.7 Hz, 1H), 6.31 (d, *J* = 2.0 Hz, 1H), 5.48 - 5.41 (m, 1H), 3.89 (s, 3H), 3.60 - 3.53 (m, 1H), 3.51 - 3.44 (m, 1H), 2.99 (s, 3H), 2.56 - 2.48 (m, 1H), 2.45 - 2.37 (m, 1H), 1.62 (d, *J* = 6.8 Hz, 3H). MS (ESI⁺): m/z = 459. | 1) acidic conditions, column C1; | 14 (diastere omer 1) |
| | 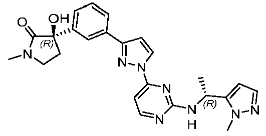 | | 2) SFC1 Mobile phase: 45% methanol, 55% CO₂); 70 mL/min, 1st eluting peak designated as diastereomer 1 (Example 91). 2^{nd} eluting peak designated as diastereomer 2 (Example 92) | |
| | | | | 13 (diastere omer 2) |
| | (*R*)-3-hydroxy-1-methyl-3-(3-(1-(2-(((S)-1-(1-methyl-1H-pyrazol-5-yl)ethyl)amino)pyrimidin -4-yl)-1H-pyrazol-3-yl)phenyl)pyrrolidin-2-one and (R)-3-hydroxy-1-methyl-3-(3-(1-(2-(((R)-1-(1-methyl-1H-pyrazol-5-yl)ethyl)amino)pyrimidin -4-yl)-1H-pyrazol-3-yl)phenyl)pyrrolidin-2-one | | | |
| | | Diastereomer 2 (Example 92): ¹H NMR (500 MHz, MeOH-*d*₄) δ 8.60 (d, *J* = 2.8 Hz, 1H), 8.36 (d, *J =* 5.5 Hz, 1H), 8.07 - 8.03 (m, 1H), 7.89 - 7.83 (m, 1H), 7.48 - 7.43 (m, 1H), 7.43 - 7.40 (m, 1H), 7.37 (d, *J* = 2.1 Hz, 1H), 7.26 (d, *J* = 5.5 Hz, 1H), 6.95 (d, *J* = 2.8 Hz, 1H), 6.31 (d, *J =* 2.0 Hz, 1H), 5.48 - 5.41 (m, 1H), 3.90 (s, 3H), 3.60 - 3.53 (m, 1H), 3.51 - 3.45 (m, 1H), 2.99 (s, 3H), 2.55 - 2.48 (m, 1H), 2.45 - 2.38 (m, 1H), 1.62 (d, *J =* 6.9 Hz, 3H). MS (ESI⁺): m/z = 459. | | |
| 93 and 94 | 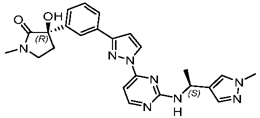 | Diastereomer 1 (Example 93): ¹H NMR (500 MHz, MeOH-*d*₄) δ 8.62 (d, *J* = 2.7 Hz, 1H), 8.33 (d, *J =* 5.5 Hz, 1H), 8.07 - 8.03 (m, 1H), 7.89 - 7.84 (m, 1H), 7.57 (s, 1H), 7.47 (s, 1H), 7.46 - 7.43 (m, 1H), 7.43 - 7.40 (m, 1H), 7.22 (d, *J* = 5.5 Hz, 1H), 6.95 (d, *J* = 2.8 Hz, 1H), 5.25 (q, *J* = 6.9 Hz, 1H), 3.84 (s, 3H), 3.60 - 3.53 (m, 1H), 3.52 - 3.45 (m, 1H), 2.99 (s, 3H), 2.56 - 2.48 (m, 1H), 2.46 - 2.38 (m, 1H), 1.57 (d, *J* = 6.9 Hz, 3H). MS (ESI⁺): m/z = 459. Diastereomer 2 (Example 94): ¹H NMR (500 MHz, MeOH-*d*₄) δ 8.62 (d, *J* = 2.7 Hz, 1H), 8.33 (d, *J =* 5.5 Hz, 1H), 8.07 - 8.03 (m, 1H), 7.89 - 7.83 (m, 1H), 7.57 (s, 1H), 7.48 - 7.46 (m, 1H), 7.46 - 7.43 (m, 1H), 7.43 - 7.39 (m, 1H), 7.22 (d, *J =* 5.5 Hz, 1H), 6.94 (d, *J* = 2.8 Hz, 1H), 5.25 (q, *J* = 6.9 Hz, 1H), 3.84 (s, 3H), 3.60 - 3.53 (m, 1H), 3.51 - 3.45 (m, 1H), 2.99 (s, 3H), 2.56 - 2.48 (m, 1H), 2.46 - 2.37 (m, 1H), 1.57 (d, *J* = 6.9 Hz, 3H). MS (ESI⁺): m/z = 459. | 1) acidic conditions, column C1; | 25 (diastere omer 1) |
| | 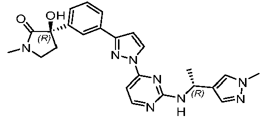 | | 2) SFC1 Mobile phase: 35% methanol, 65% CO₂); 70 mL/min, 1st eluting peak designated as diastereomer 1 (Example 93). 2nd eluting peak designated as diastereomer 2 (Example 94). | 24 (diastere omer 2) |
| | (*R*)-3-hydroxy-1-methyl-3-(3-(1-(2-(((S)-1-(1-methyl-1H-pyrazol-4-yl)ethyl)amino)pyrimidin -4-yl)-1H-pyrazol-3-yl)phenyl)pyrrolidin-2-one and (R)-3-hydroxy-1-methyl-3-(3-(1-(2-(((R)-1-(1-methyl-1H-pyrazol-4-yl)ethyl)amino)pyrimidin -4-yl)-1H-pyrazol-3-yl)phenyl)pyrrolidin-2-one | | | |
| 95 | 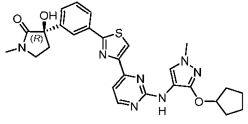 | ¹H NMR (500 MHz, DMSO-*d₆*) δ 8.50 (d, *J =* 4.9 Hz, 1H), 8.44 (s, 1H), 8.29 (s, 1H), 8.11 (s, 1H), 7.94 (d, *J* = 7.6 Hz, 1H), 7.51 (t, *J* = 7.7 Hz, 1H), 7.47 - 7.43 (m, 1H), 7.41 (d, *J =* 4.9 Hz, 1H), 6.22 (s, 1H), 4.97 (t, *J* = 4.4 Hz, 1H), 3.71 (s, 3H), 3.51 - 3.45 (m, 1H), 3.42 - 3.37 (m, 1H), 2.87 (s, 3H), 2.34 - 2.24 (m, 2H), 1.83 - 1.75 (m, 4H), 1.70 - 1.63 (m, 2H), 1.55 - 1.47 (m, 2H). MS (ESI⁺): m/z = 532. | basic conditions, column C2 | 13 |
| | (*R*)-3-(3-(4-(2-((3-(cyclopentyloxy)-1-methyl-1H-pyrazol-4-yl)amino)pyrimidin-4-yl)thiazol-2-yl)phenyl)-3-hydroxy-1-methylpyrrolidin-2-one | | | |
| 96 | 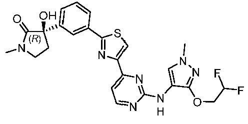 | ¹H NMR (500 MHz, DMSO-*d₆*) δ 8.53 (s, 1H), 8.51 (d, *J* = 5.0 Hz, 1H), 8.43 (s, 1H), 8.11 (t, *J =* 1.8 Hz, 1H), 7.97 - 7.91 (m, 1H), 7.85 (s, 1H), 7.51 (t, *J =* 7.8 Hz, 1H), 7.47 - 7.43 (m, 1H), 7.43 (d, *J* = 4.9 Hz, 1H), 6.35 (tt, J = 54.8, 3.8 Hz, 2H), 6.22 (s, 1H), 4.43 - 4.34 (m, 2H), 3.73 (s, 3H), 3.52 - 3.45 (m, 1H), 3.43 - 3.36 (m, 1H), 2.87 (s, 3H), 2.41 - 2.34 (m, 2H), 2.32 - 2.25 (m, 1H). MS (ESI⁺): m/z = 528. | basic conditions, column C2 | 19 |
| | (*R*)-3-(3-(4-(2-((3-(2,2-difluoroethoxy)-1-methyl-1H-pyrazol-4-yl)amino)pyrimidin-4-yl)thiazol-2-yl)phenyl)-3-hydroxy-1-methylpyrrolidin-2-one | | | |
| 97 | 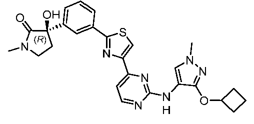 | ¹H NMR (500 MHz, DMSO-*d₆*) δ 8.51 (d, *J =* 4.9 Hz, 1H), 8.45 (s, 1H), 8.36 (s, 1H), 8.11 (t, *J* = 1.8 Hz, 1H), 7.97 - 7.92 (m, 1H), 7.80 (s, 1H), 7.51 (t, *J =* 7.7 Hz, 1H), 7.47 - 7.43 (m, 1H), 7.41 (d, *J =* 5.0 Hz, 1H), 6.22 (s, 1H), 4.88 - 4.78 (m, 1H), 3.70 (s, 3H), 3.52 - 3.44 (m, 1H), 3.42 - 3.36 (m, 1H), 2.87 (s, 3H), 2.40 - 2.35 (m, 2H), 2.34 - 2.25 (m, 3H), 2.10 - 2.00 (m, 2H), 1.76 - 1.67 (m, 1H), 1.60 - 1.49 (m, 1H). MS (ESI⁺): m/z = 518. | Basic conditions, column C2 | 11 |
| | (*R*)-3-(3-(4-(2-((3-cyclobutoxy-1-methyl-1H-pyrazol-4-yl)amino)pyrimidin-4-yl)thiazol-2-yl)phenyl)-3-hydroxy-1-methylpyrrolidin-2-one | | | |
| 98 | 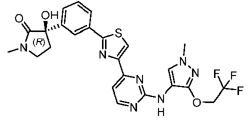 | ¹H NMR (500 MHz, DMSO-*d₆*) δ 8.61 (s, 1H), 8.51 (d, *J* = 5.0 Hz, 1H), 8.42 (s, 1H), 8.13 - 8.08 (m, 1H), 7.97 - 7.91 (m, 1H), 7.85 (s, 1H), 7.55 - 7.48 (m, 1H), 7.46 - 7.44 (m, 1H), 7.43 (d, *J =* 5.2 Hz, 1H), 6.22 (s, 1H), 4.78 (q, *J* = 9.1 Hz, 2H), 3.74 (s, 3H), 3.52 - 3.45 (m, 1H), 3.42 - 3.35 (m, 1H), 2.87 (s, 3H), 2.41 - 2.34 (m, 1H), 2.32 - 2.25 (m, 1H). MS (ESI⁺): m/z = 546. | basic conditions, column C2 | 8 |
| | (*R*)-3-hydroxy-1-methyl-3-(3-(4-(2-((1-methyl-3-(2,2,2-trifluoroethoxy)-1 H-pyrazol-4-yl)amino)pyrimidin-4-yl)thiazol-2-yl)phenyl)pyrrolidin-2-one | | | |
| 99 | 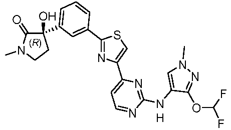 | ¹H NMR (500 MHz, DMSO-*d₆*) δ 8.85 (s, 1H), 8.54 (d, *J* = 5.0 Hz, 1H), 8.45 (s, 1H), 8.11 (t, *J =* 1.9 Hz, 1H), 7.97 (s, 1H), 7.96 - 7.91 (m, 1H), 7.51 (t, *J =* 7.7 Hz, 1H), 7.46 (d, *J* = 5.0 Hz, 1H), 7.46 - 7.42 (m, 1H), 7.18 (t, *J =* 73.7 Hz, 1H), 6.22 (s, 1H), 3.79 (s, 3H), 3.51 - 3.45 (m, 1H), 3.42 - 3.37 (m, 1H), 2.87 (s, 3H), 2.42 - 2.37 (m, 1H), 2.32 - 2.25 (m, 1H). MS (ESI⁺): m/z = 514. | basic conditions, column C2 | 4 |
| | (*R*)-3-(3-(4-(2-((3-(difluoromethoxy)-1-methyl-1H-pyrazol-4-yl)amino)pyrimidin-4-yl)thiazol-2-yl)phenyl)-3-hydroxy-1-methylpyrrolidin-2-one | | | |
| 100 | 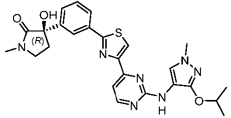 | ¹H NMR (500 MHz, DMSO-*d₆*) δ 8.51 (d, *J =* 5.0 Hz, 1H), 8.47 (s, 1H), 8.35 - 8.23 (m, 1H), 8.11 (t, *J =* 1.8 Hz, 1H), 7.94 (dt, J *=* 7.7, 1.4 Hz, 1H), 7.81 (s, 1H), 7.51 (t, *J =* 7.7 Hz, 1H), 7.47 - 7.41 (m, 2H), 4.72 (p, *J* = 6.2 Hz, 1H), 3.71 (s, 4H), 2.87 (s, 3H), 2.41 - 2.34 (m, 2H), 2.32 - 2.24 (m, 2H), 1.27 (d, *J =* 6.1 Hz, 7H). MS (ESI⁺): m/z = 506. | basic conditions, column C2 | 15 |
| | (*R*)-3-hydroxy-3-(3-(4-(2-((3-isopropoxy-1-methyl-1H-pyrazol-4-yl)amino)pyrimidin-4-yl)thiazol-2-yl)phenyl)-1-methylpyrrolidin-2-one | | | |
| 101 | 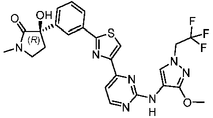 | ¹H NMR (500 MHz, DMSO-*d₆*) δ 8.66 (s, 1H), 8.55 (d, *J* = 4.9 Hz, 1H), 8.46 (s, 1H), 8.11 (t, *J =* 1.8 Hz, 1H), 8.07 (s, 1H), 7.95 (dt, *J* = 7.6, 1.4 Hz, 1H), 7.52 (t, *J* = 7.7 Hz, 1H), 7.49 - 7.43 (m, 2H), 5.03 - 4.94 (m, 2H), 3.86 (s, 3H), 3.51 - 3.49 (m, 1H), 3.48 - 3.46 (m, 1H), 2.87 (s, 3H), 2.41 - 2.35 (m, 2H), 2.32 - 2.26 (m, 1H). MS (ESI⁺): m/z = 546. | basic conditions, column C2 | 11 |
| | (*R*)-3-hydroxy-3-(3-(4-(2-((3-methoxy-1-(2,2,2-trifluoroethyl)-1H-pyrazol-4-yl)amino)pyrimidin-4-yl)thiazol-2-yl)phenyl)-1-methylpyrrolidin-2-one | | | |
| 102 | 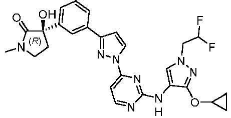 | ¹H NMR (500 MHz, DMSO-*d₆*) δ 8.76 (s, 1H), 8.57 (s, 1H), 8.48 (d, *J* = 5.4 Hz, 1H), 8.00 (t, *J* = 1.8 Hz, 1H), 7.93 (s, 1H), 7.87 - 7.82 (m, 1H), 7.44 (t, *J =* 7.7 Hz, 1H), 7.39 - 7.34 (m, 1H), 7.25 (d, *J* = 5.4 Hz, 1H), 7.12 (d, *J* = 2.7 Hz, 1H), 6.33 (tt, *J* = 55.2, 3.9 Hz, 1H), 6.10 (s, 1H), 4.51 (t, *J* = 14.8 Hz, 2H), 4.14 - 4.07 (m, 1H), 3.50 - 3.44 (m, 1H), 3.41 - 3.35 (m, 1H), 2.86 (s, 3H), 2.41 - 2.33 (m, 1H), 2.31 - 2.24 (m, 1H), 0.72 - 0.67 (m, 2H), 0.67 - 0.61 (m, 2H). MS (ESI⁺): m/z = 537. | basic conditions, column C8 | 56 |
| | (*R*)-3-(3-(1-(2-((3-cyclopropoxy-1-(2,2-difluoroethyl)-1H-pyrazol-4-yl)amino)pyrimidin-4-yl)-1H-pyrazol-3-yl)phenyl)-3-hydroxy-1-methylpyrrolidin-2-one | | | |
| 103 | 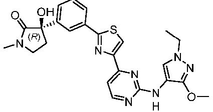 | ¹H NMR (500 MHz, DMSO-*d₆*) δ 8.49 (d, *J =* 5.0 Hz, 1H), 8.41 (s, 2H), 8.10 (t, *J* = 1.8 Hz, 1H), 7.93 (dt, *J* = 7.8, 1.4 Hz, 1H), 7.91 - 7.74 (m, 1H), 7.51 (t, *J =* 7.7 Hz, 1H), 7.44 (dt, *J =* 7.9, 1.4 Hz, 1H), 7.40 (d, *J* = 4.9 Hz, 1H) , 6.23 (s, 1H), 3.99 (q, *J* = 7.2 Hz, 2H), 3.82 (s, 3H), 3.53 - 3.48 (m, 1H), 3.48 - 3.45 (m, 1H), 2.86 (s, 3H), 2.42 - 2.33 (m, 1H), 2.32 - 2.24 (m, 1H), 1.35 (t, *J* = 7.2 Hz, 3H). MS (ESI⁺): m/z = 492. | basic conditions, column C2 | 7 |
| | (*R*)-3-(3-(4-(2-((1-ethyl-3-methoxy-1H-pyrazol-4-yl)amino)pyrimidin-4-yl)thiazol-2-yl)phenyl)-3-hydroxy-1-methylpyrrolidin-2-one | | | |
| 104 | 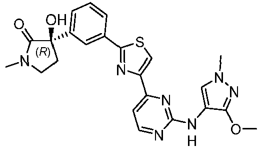 | MS (ESI⁺): m/z = 478.3. | acidic conditions, column C7 | 3 |
| | (R)-3-hydroxy-3-(3-(4-(2-((3-methoxy-1-methyl-1H-pyrazol-4-yl)amino)pyrimidin-4-yl)thiazol-2-yl)phenyl)-1-methylpyrrolidin-2-one | | | |
| 105 | 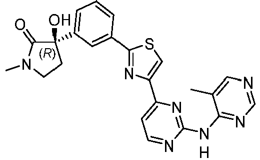 | ¹H NMR (500 MHz, DMSO-*d₆*) δ 9.97 (s, 1H), 9.95 (s, 1H), 9.32 - 9.25 (m, 2H), 9.15 (d, *J =* 5.1 Hz, 1H), 8.30 (d, *J* = 5.1 Hz, 1H), 8.16 (t, *J =* 1.8 Hz, 1H), 8.00 (dt, *J* = 7.7, 1.4 Hz, 1H), 7.55 (t, *J* = 7.7 Hz, 1H), 7.49 (dt, *J* = 7.9, 1.4 Hz, 1H), 6.24 (s, 1H), 3.54 - 3.46 (m, 1H), 3.43 - 3.38 (m, 1H), 2.87 (s, 3H), 2.40 (ddd, *J* = 13.0, 7.8, 5.1 Hz, 1H), 2.34 - 2.26 (m, 4H). MS (ESI⁺): m/z = 460.1. | acidic conditions, column C1 | 46 |
| | (*R*)-3-hydroxy-1-methyl-3-(3-(4-(2-((5-methylpyrimidin-4-yl)amino)pyrimidin-4-yl)thiazol-2-yl)phenyl)pyrrolidin-2-one | | | |
| 106 | 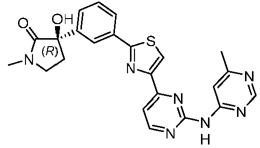 | MS (ESI⁺): m/z = 460.2. | acidic conditions, column C1 | 3 |
| | (R)-3-hydroxy-1-methyl-3-(3-(4-(2-((6-methylpyrimidin-4-yl)amino)pyrimidin-4-yl)thiazol-2-yl)phenyl)pyrrolidin-2-one | | | |
| 107 | 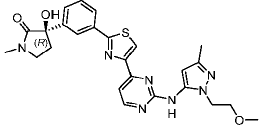 | MS (ESI⁺): m/z = 506.4. | basic conditions, column C2 | 2 |
| | (R)-3-hydroxy-3-(3-(4-(2-((1-(2-methoxyethyl)-3-methyl-1H-pyrazol-5-yl)amino)pyrimidin-4-yl)thiazol-2-yl)phenyl)-1-methylpyrrolidin-2-one | | | |

The compounds shown in Table 3B were prepared in a manner analogous to Example 45 using (*R*)-3-hydroxy-1-methyl-3-(3-(2-(2-(methylsulfonyl)pyrimidin-4-yl)thiazol-4-yl)phenyl)pyrrolidin-2-one (Intermediate 9) or (*R*)-3-hydroxy-1-methyl-3-(3-(1-(2-(methylsulfonyl)pyrimidin-4-yl)-1*H*-pyrazol-3-yl)phenyl)pyrrolidin-2-one (Intermediate 13) in place of (*R*)-3-hydroxy-1-methyl-3-(3-(4-(2-(methylsulfonyl)pyrimidin-4-yl)thiazol-2-yl)phenyl)pyrrolidin-2-one (Intermediate 12) and the appropriate amine in place of 1-(1*H*-benzo[*d*]imidazol-2-yl)ethan-1-amine. Purification methods are noted in the table.

**Table 3B:**

| # | Structure | Analytical data | RP HPLC conditions | Yield (%) |
|---|---|---|---|---|
| | Name | | | |
| 108 | 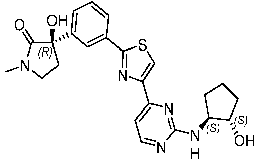 | ¹H NMR (600 MHz, DMSO-*d₆*) d 8.68 - 8.21 (m, 2H), 8.09 (td, *J* = 1.8, 0.5 Hz, 1H), 7.93 (dt, *J* = 7.8, 1.4 Hz, 1H), 7.57 - 7.49 (m, 1H), 7.48 - 7.38 (m, 1H), 7.28 (d, *J =* 5.0 Hz, 1H), 7.19 (s, 1H), 6.21 (s, 1H), 4.91 (s, 1H), 3.99 (t, *J =* 5.4 Hz, 2H), 3.56 - 3.42 (m, 1H), 3.42 - 3.37 (m, 1H), 2.86 (s, 3H), 2.44 - 2.35 (m, 1H), 2.32 - 2.22 (m, 1H), 2.08 (s, 1H), 1.93 - 1.82 (m, 1H), 1.73 - 1.59 (m, 2H), 1.59 - 1.42 (m, 2H). MS (ESI⁺): m/z = 452.2. | basic conditions, column C2 | 26 |
| | (*R*)-3-hydroxy-3-(3-(4-(2-(((1S,2S)-2-hydroxycyclopentyl)ami no)pyrimidin-4-yl)thiazol-2-yl)phenyl)-1-methylpyrrolidin-2-one | | | |
| 109 | 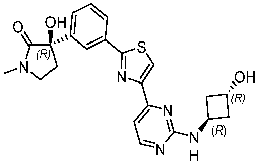 | ¹H NMR (500 MHz, DMSO-*d₆*) δ 8.42 (d, *J = 5.0* Hz, 1H), 8.35 (s, 1H), 8.09 (t, *J* = 1.8 Hz, 1H), 7.96 - 7.90 (m, 1H), 7.56 - 7.48 (m, 2H), 7.47 - 7.42 (m, 1H), 7.29 (d, *J =* 4.9 Hz, 1H), 6.22 (s, 1H), 4.98 (d, *J =* 5.3 Hz, 1H), 4.43 (s, 1H), 4.32 (dt, *J =* 11.6, 5.9 Hz, 1H), 3.48 (ddd, *J* = 9.8, 8.1, 5.0 Hz, 1H), 3.42 - 3.36 (m, 2H), 2.87 (s, 3H), 2.37 (ddd, *J =* 13.0, 7.8, 5.1 Hz, 1H), 2.32 - 2.13 (m, 5H). MS (ESI⁺): m/z = 438.15 | basic conditions, column C2 | 61 |
| | (*R*)-3-hydroxy-3-(3-(4-(2-(((1R,3R)-3-hydroxycyclobutyl)amin o)pyrimidin-4-yl)thiazol-2-yl)phenyl)-1-methylpyrrolidin-2-one | | | |
| 110 | 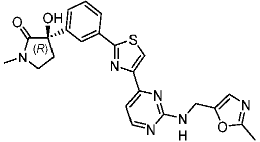 | ¹H NMR (500 MHz, DMSO-*d₆*) δ 8.47 (d, *J =* 5.0 Hz, 2H), 8.10 (t, *J* = 1.8 Hz, 1H), 7.93 (ddd, *J* = 7.6, 1.9, 1.1 Hz, 1H), 7.73 (t, *J =* 6.0 Hz, 1H), 7.56 - 7.47 (m, 1H), 7.44 (ddd, *J* = 7.9, 1.9, 1.2 Hz, 1H), 7.35 (d, *J =* 5.0 Hz, 1H), 6.92 (s, 1H), 6.22 (s, 1H), 4.57 (d, *J* = 5.9 Hz, 2H), 3.53 - 3.43 (m, 1H), 3.42 - 3.37 (m, 1H), 2.86 (s, 3H), 2.42 - 2.33 (m, 4H), 2.32 - 2.23 (m, 1H). MS (ESI⁺): m/z = 463.05 | basic conditions, column C2 | 30 |
| | (*R*)-3-hydroxy-1-methyl-3-(3-(4-(2-(((2-methyloxazol-5-yl)methyl)amino)pyrimidi n-4-yl)thiazol-2-yl)phenyl)pyrrolidin-2-one | | | |
| 111 | 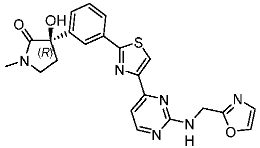 | ¹H NMR (500 MHz, DMSO-*d₆*) δ 8.46 (d, *J =* 5.0 Hz, 1H), 8.36 (s, 1H), 8.09 (t, *J* = 1.9 Hz, 1H), 8.01 (d, *J* = 0.8 Hz, 1H), 7.92 (dt, *J =* 7.6, 1.5 Hz, 1H), 7.90 - 7.80 (m, 1H), 7.51 (t, *J =* 7.7 Hz, 1H), 7.44 (dt, *J =* 7.8, 1.5 Hz, 1H), 7.36 (d, *J* = 5.0 Hz, 1H), 7.13 (s, 1H), 4.68 (d, *J* = 5.3 Hz, 2H), 3.51 - 3.38 (m, 2H), 2.86 (s, 3H), 2.41 - 2.32 (m, 1H), 2.31 - 2.22 (m, 1H). MS (ESI⁺): m/z = 449.2. | acidic conditions, column C1 | 14 |
| | (*R*)-3-hydroxy-1-methyl-3-(3-(4-(2-((oxazol-2-ylmethyl)amino)pyrimidi n-4-yl)thiazol-2-yl)phenyl)pyrrolidin-2-one | | | |
| 112 | 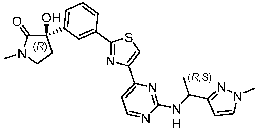 | ¹H NMR (500 MHz, DMSO-*d₆*) δ 8.43 (d, *J =* 4.9 Hz, 1H), 8.40 (s, 1H), 8.09 (t, *J* = 1.9 Hz, 1H), 7.92 (dt, *J* = 7.7, 1.4 Hz, 1H), 7.59 - 7.47 (m, 2H), 7.44 (dt, *J* = 7.9, 1.4 Hz, 1H), 7.33 (d, *J* = 8.5 Hz, 1H), 7.29 (d, *J* = 5.0 Hz, 1H), 6.33 - 6.06 (m, 2H), 5.30 (s, 1H), 3.78 (s, 3H), 3.53 - 3.45 (m, 1H), 3.42 - 3.38 (m, 1H), 2.86 (s, 3H), 2.37 (ddd, J = 13.0, 7.8, 5.1 Hz, 1H), 2.28 (ddd, *J* = 13.4, 8.1, 5.5 Hz, 1H), 1.48 (d, *J* = 6.9 Hz, 3H). MS (ESI⁺): m/z = 476.10. | basic conditions, column C2 | 51 |
| | (*R*)-3-hydroxy-1-methyl-3-(3-(4-(2-(((*R,S*)-1-(1-methyl-1H-pyrazol-3-yl)ethyl)amino)pyrimidin -4-yl)thiazol-2-yl)phenyl)pyrrolidin-2-one | | | |
| 113 | 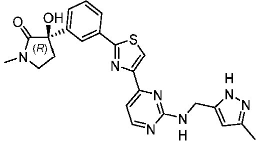 | ¹H NMR (600 MHz, DMSO-*d₆*) δ 12.18 (s, 1H), 8.68 - 8.29 (m, 2H), 8.10 (t, *J =* 1.8 Hz, 1H), 7.96 - 7.88 (m, 1H), 7.54 - 7.49 (m, 1H), 7.48 - 7.36 (m, 2H), 7.32 (d, *J =* 4.9 Hz, 1H), 6.22 (s, 1H), 5.94 (s, 1H), 4.50 (s, 2H), 3.58 - 3.46 (m, 1H), 3.46 - 3.35 (m, 1H), 2.87 (s, 3H), 2.42 - 2.32 (m, 1H), 2.32 - 2.21 (m, 1H), 2.16 (s, 3H). MS (ESI⁺): m/z = 462.3. | basic conditions, column C2 | 26 |
| | (*R*)-3-hydroxy-1-methyl-3-(3-(4-(2-(((3-methyl-1H-pyrazol-5-yl)methyl)amino)pyrimidi n-4-yl)thiazol-2-yl)phenyl)pyrrolidin-2-one | | | |
| 114 | 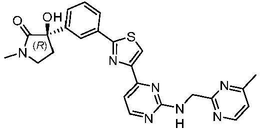 | ¹H NMR (500 MHz, DMSO-*d₆*) δ 8.59 (d, *J* = 5.1 Hz, 1H), 8.54 - 8.18 (m, 2H), 8.08 (s, 1H), 7.92 (d, *J =* 7.6 Hz, 1H), 7.59 - 7.46 (m, 2H), 7.44 (dt, *J* = 7.8, 1.4 Hz, 1H), 7.32 (d, *J* = 5.0 Hz, 1H), 7.24 (d, *J* = 5.1 Hz, 1H), 6.22 (s, 1H), 4.72 (d, *J* = 5.1 Hz, 2H), 3.55 - 3.42 (m, 1H), 3.42 - 3.38 (m, 1H), 2.86 (s, 3H), 2.46 (s, 3H), 2.41 - 2.32 (m, 1H), 2.32 - 2.22 (m, 1H). MS (ESI⁺): m/z = 474.15 | basic conditions, column C2 | 47 |
| | (*R*)-3-hydroxy-1-methyl-3-(3-(4-(2-(((4-methylpyrimidin-2-yl)methyl)amino)pyrimidi n-4-yl)thiazol-2-yl)phenyl)pyrrolidin-2-one | | | |
| 115 | 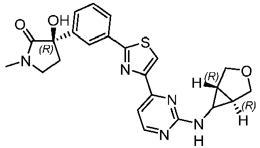 | ¹H NMR (500 MHz, DMSO-*d₆*) δ 8.43 - 8.32 (m, 1H), 8.14 - 8.03 (m, 1H), 7.99 - 7.87 (m, 1H), 7.56 - 7.40 (m, 3H), 7.35 (d, *J* = 5.0 Hz, 1H), 6.22 (s, 1H), 3.99 - 3.85 (m, 2H), 3.67 (d, *J* = 8.2 Hz, 2H), 3.53 - 3.45 (m, 1H), 3.42 - 3.38 (m, 1H), 2.87 (s, 3H), 2.59 - 2.52 (m, 1H), 2.43 - 2.34 (m, 1H), 2.34 - 2.19 (m, 1H), 1.88 - 1.80 (m, 2H). MS (ESI⁺): m/z = 450.2. | basic conditions, column C2 | 29 |
| | (*R*)-3-(3-(4-(2-(((1R,5R)-3-oxabicyclo[3.1.0]hexan-6-yl)amino)pyrimidin-4-yl)thiazol-2-yl)phenyl)-3-hydroxy-1-methylpyrrolidin-2-one | | | |
| 116 | 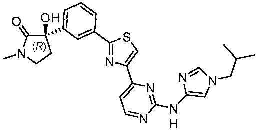 | ¹H NMR (500 MHz, DMSO-*d₆*) δ 10.42 (s, 1H), 8.70 (d, *J* = 5.1 Hz, 1H), 8.67 (s, 1H), 8.59 (s, 1H), 8.13 (t, *J* = 1.9 Hz, 1H), 7.96 (dt, *J =* 7.7, 1.5 Hz, 1H), 7.67 (d, *J* = 5.0 Hz, 1H), 7.60 (d, *J =* 1.8 Hz, 1H), 7.53 (t, *J =* 7.7 Hz, 1H), 7.46 (dt, J = 7.8, 1.5 Hz, 1H), 6.23 (s, 1H), 4.02 (d, *J* = 7.3 Hz, 2H), 3.62 - 3.31 (m, 2H), 2.87 (s, 3H), 2.43 - 2.35 (m, 1H), 2.35 - 2.23 (m, 1H), 2.16 (hept, *J* = 6.8 Hz, 1H), 0.91 (d, *J* = 6.7 Hz, 6H). MS (ESI⁺): m/z = 490.3. | acidic conditions, column C1 | 16 |
| | (*R*)-3-hydroxy-3-(3-(4-(2-((1-isobutyl-1H-imidazol-4-yl)amino)pyrimidin-4-yl)thiazol-2-yl)phenyl)-1-methylpyrrolidin-2-one | | | |
| 117 | 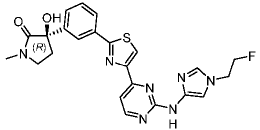 | ¹H NMR (500 MHz, DMSO-*d₆*) δ 10.30 (s, 1H), 8.68 (d, *J* = 5.0 Hz, 1H), 8.61 (s, 1H), 8.47 (s, 1H), 8.14 (t, *J* = 1.9 Hz, 1H), 7.96 (dt, *J* = 7.7, 1.4 Hz, 1H), 7.69 - 7.60 (m, 2H), 7.53 (t, *J =* 7.7 Hz, 1H), 7.45 (dt, *J =* 7.8, 1.4 Hz, 1H), 6.21 (s, 1H), 4.88 (t, *J* = 4.7 Hz, 1H), 4.79 (t, *J* = 4.6 Hz, 1H), 4.55 (t, *J* = 4.7 Hz, 1H), 4.49 (t, *J* = 4.7 Hz, 1H), 3.71 - 3.35 (m, 2H), 2.87 (s, 3H), 2.45 - 2.34 (m, 1H), 2.34 - 2.22 (m, 1H). MS (ESI⁺): m/z = 480.5. | acidic conditions, column C1 | 11 |
| | (*R*)-3-(3-(4-(2-((1-(2-fluoroethyl)-1H-imidazol-4-yl)amino)pyrimidin-4-yl)thiazol-2-yl)phenyl)-3-hydroxy-1-methylpyrrolidin-2-one | | | |
| 118 | 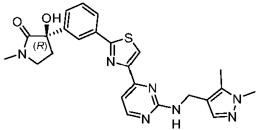 | MS (ESI+): m/z = 476.10. | basic conditions, column C2 | 54 |
| | (*R*)-3-(3-(4-(2-(((1,5-dimethyl-1H-pyrazol-4-yl)methyl)amino)pyrimidi n-4-yl)thiazol-2-yl)phenyl)-3-hydroxy-1-methylpyrrolidin-2-one | | | |
| 119 | 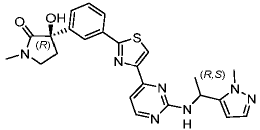 | ¹H NMR (500 MHz, DMSO-*d₆*) δ 8.46 (d, *J =* 5.0 Hz, 2H), 8.10 (t, *J =* 1.9 Hz, 1H), 7.92 (dt, *J* = 7.6, 1.4 Hz, 1H), 7.76 (d, *J* = 8.2 Hz, 1H), 7.51 (t, *J =* 7.7 Hz, 1H), 7.44 (dt, *J* = 7.8, 1.5 Hz, 1H), 7.34 (d, *J* = 5.0 Hz, 1H), 7.28 (d, *J* = 1.9 Hz, 1H), 6.25 (d, *J* = 2.0 Hz, 1H), 5.46 (s, 1H), 3.86 (s, 3H), 2.86 (s, 3H), 2.37 (ddd, *J* = 13.0, 7.8, 5.0 Hz, 1H), 2.28 (ddd, *J* = 13.5, 8.1, 5.5 Hz, 1H), 1.52 (d, *J* = 6.9 Hz, 3H). MS (ESI⁺): m/z = 476.10. | acidic conditions, column C1 | 16 |
| | (*R*)-3-hydroxy-1-methyl-3-(3-(4-(2-(((R, S)-1-(1-methyl-1H-pyrazol-5-yl)ethyl)amino)pyrimidin -4-yl)thiazol-2-yl)phenyl)pyrrolidin-2-one | | | |
| 120 | 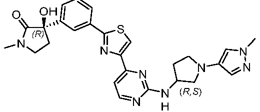 | ¹H NMR (500 MHz, DMSO-*d₆*) δ 8.48 (d, *J =* 5.0 Hz, 2H), 8.10 (t, *J* = 1.8 Hz, 1H), 7.97 - 7.83 (m, 1H), 7.67 (s, 1H), 7.51 (t, *J =* 7.7 Hz, 1H), 7.44 (ddd, *J* = 7.8, 1.8, 1.2 Hz, 1H), 7.37 (d, *J* = 5.0 Hz, 1H), 7.33 (s, 1H), 7.16 (s, 1H), 4.62 (s, 1H), 3.74 (s, 3H), 3.60 - 3.44 (m, 2H), 3.44 - 3.34 (m, 1H), 3.32 (d, *J =* 7.1 Hz, 1H), 3.23 - 3.03 (m, 2H), 2.87 (s, 3H), 2.44 - 2.32 (m, 2H), 2.32 - 2.20 (m, 1H), 2.09 - 1.93 (m, 1H). MS (ESI⁺): m/z = 517.2. | acidic conditions, column C1 | 15 |
| | (*R*)-3-hydroxy-1-methyl-3-(3-(4-(2-(((*R,S*)-1-(1-methyl-1H-pyrazol-4-yl)pyrrolidin-3-yl)amino)pyrimidin-4-yl)thiazol-2-yl)phenyl)pyrrolidin-2-one | | | |
| 121 | 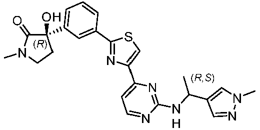 | ¹H NMR (500 MHz, DMSO-*d₆*) δ 8.44 (d, *J =* 5.0 Hz, 2H), 8.11 (td, *J =* 1.8, 0.5 Hz, 1H), 7.93 (ddd, *J* = 7.6, 1.9, 1.1 Hz, 1H), 7.61 (s, 1H), 7.51 (td, *J =* 7.7, 0.5 Hz, 1H), 7.47 - 7.37 (m, 3H), 7.30 (d, *J =* 4.9 Hz, 1H), 6.23 (s, 1H), 5.24 (s, 1H), 3.77 (s, 3H), 3.49 (ddd, *J* = 9.9, 8.1, 5.0 Hz, 1H), 3.39 (ddd, *J =* 9.9, 7.7, 5.5 Hz, 1H), 2.87 (s, 3H), 2.38 (ddd, *J* = 12.9, 7.8, 5.1 Hz, 1H), 2.29 (ddd, J = 13.4, 8.1, 5.5 Hz, 1H), 1.48 (d, *J* = 6.9 Hz, 3H). MS (ESI⁺): m/z = 476.10. | basic conditions, column C2 | 12 |
| | (*R*)-3-hydroxy-1-methyl-3-(3-(4-(2-(((*R,S*)-1-(1-methyl-1H-pyrazol-4-yl)ethyl)amino)pyrimidin -4-yl)thiazol-2-yl)phenyl)pyrrolidin-2-one | | | |
| 122 | 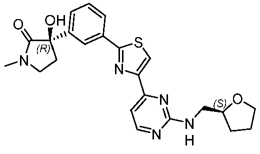 | ¹H NMR (500 MHz, DMSO-*d₆*) δ 8.44 (d, *J =* 5.0 Hz, 2H), 8.10 (dt, *J =* 1.9, 0.9 Hz, 1H), 7.93 (ddd, *J* = 7.7, 1.9, 1.2 Hz, 1H), 7.54 - 7.49 (m, 1H), 7.47 - 7.39 (m, 2H), 7.30 (d, *J* = 4.9 Hz, 1H), 6.22 (s, 1H), 3.83 - 3.70 (m, 2H), 3.63 (td, *J* = 8.0, 6.8 Hz, 1H), 3.57 - 3.43 (m, 2H), 3.43 - 3.33 (m, 3H), 2.87 (s, 3H), 2.57 (dd, *J* = 13.4, 6.7 Hz, 1H), 2.38 (ddd, *J* = 12.9, 7.8, 5.1 Hz, 1H), 2.29 (ddd, *J =* 13.4, 8.1, 5.5 Hz, 1H), 1.98 (td, *J* = 13.4, 7.7 Hz, 1H), 1.64 (dq, *J* = 13.0, 6.7 Hz, 1H). MS (ESI⁺): m/z = 452.10. | basic conditions, column C2 | 41 |
| | (*R*)-3-hydroxy-1-methyl-3-(3-(4-(2-((((S)-tetrahydrofuran-2-yl)methyl)amino)pyrimidi n-4-yl)thiazol-2-yl)phenyl)pyrrolidin-2-one | | | |
| 123 | 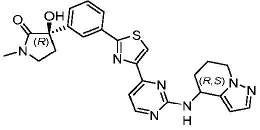 | ¹H NMR (500 MHz, DMSO-*d₆*) δ 8.61 - 8.26 (m, 2H), 8.11 (t, *J =* 1.8 Hz, 1H), 7.97 - 7.92 (m, 1H), 7.65 (d, *J* = 8.7 Hz, 1H), 7.52 (t, *J =* 7.7 Hz, 1H), 7.45 (dt, *J* = 7.6, 1.4 Hz, 1H), 7.41 - 7.31 (m, 2H), 6.22 (s, 1H), 6.11 - 6.01 (m, 1H), 5.43 (s, 1H), 4.15 (dt, *J =* 10.1, 4.9 Hz, 1H), 4.04 (td, *J* = 12.8, 11.1, 4.8 Hz, 1H), 3.49 (ddd, *J* = 9.8, 8.1, 5.0 Hz, 1H), 3.44 - 3.35 (m, 1H), 2.87 (s, 3H), 2.38 (ddd, *J =* 13.0, 7.7, 5.1 Hz, 1H), 2.29 (ddd, *J* = 13.4, 8.1, 5.5 Hz, 1H), 2.24 (dd, *J* = 11.8, 6.3 Hz, 1H), 2.20 - 2.09 (m, 1H), 2.03 (s, 1H), 1.87 (q, *J* = 11.0 Hz, 1H). MS (ESI+): m/z = 488.10 | basic conditions, column C2 | 25 |
| | (*R*)-3-hydroxy-1-methyl-3-(3-(4-(2-(((*R,S*)-4,5,6,7-tetrahydropyrazolo[1,5-a]pyridin-4-yl)amino)pyrimidin-4-yl)thiazol-2-yl)phenyl)pyrrolidin-2-one | | | |
| 124 | 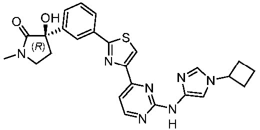 | ¹H NMR (500 MHz, DMSO-*d₆*) δ 9.59 (s, 1H), 8.59 (d, *J* = 5.0 Hz, 1H), 8.51 (s, 1H), 8.12 (t, *J* = 1.8 Hz, 1H), 8.02 - 7.91 (m, 1H), 7.59 (s, 1H), 7.56 - 7.38 (m, 4H), 6.23 (s, 1H), 4.74 (p, *J* = 8.4 Hz, 1H), 3.53 - 3.43 (m, 1H), 3.43 - 3.36 (m, 1H), 2.87 (s, 3H), 2.47 - 2.34 (m, 5H), 2.34 - 2.24 (m, 1H), 1.89 - 1.67 (m, 2H). MS (ESI⁺): m/z = 488.2. | basic conditions, column C2 | 5 |
| | (*R*)-3-(3-(4-(2-((1-cyclobutyl-1H-imidazol-4-yl)amino)pyrimidin-4-yl)thiazol-2-yl)phenyl)-3-hydroxy-1-methylpyrrolidin-2-one | | | |
| 125 | 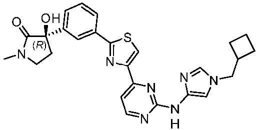 | ¹H NMR (500 MHz, DMSO-*d₆*) δ 10.39 (s, 1H), 8.69 (d, *J* = 5.1 Hz, 1H), 8.65 (s, 1H), 8.60 (s, 1H), 8.14 (t, *J* = 1.9 Hz, 1H), 7.96 (dt, *J* = 7.7, 1.4 Hz, 1H), 7.66 (d, *J* = 5.1 Hz, 1H), 7.58 (d, *J* = 1.8 Hz, 1H), 7.53 (t, *J* = 7.7 Hz, 1H), 7.46 (dt, *J* = 7.8, 1.5 Hz, 1H), 6.23 (s, 1H), 4.23 (d, *J* = 7.6 Hz, 2H), 3.55 - 3.38 (m, 2H), 2.87 (s, 3H), 2.82 (p, *J* = 7.7 Hz, 1H), 2.44 - 2.34 (m, 1H), 2.33 - 2.23 (m, 1H), 2.07 - 1.97 (m, 2H), 1.96 - 1.71 (m, 4H). MS (ESI⁺): m/z = 502.4. | acidic conditions, column C1 | 15 |
| | (*R*)-3-(3-(4-(2-((1-(cyclobutylmethyl)-1H-imidazol-4-yl)amino)pyrimidin-4-yl)thiazol-2-yl)phenyl)-3-hydroxy-1-methylpyrrolidin-2-one | | | |
| | 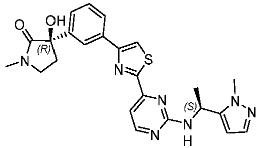 | Diastereomer 1 (Example 126): ¹H NMR (400 MHz, DMSO-*d*₆) δ 8.51 (d, *J* = 4.9 Hz, 1H), 8.32 (s, 1H), 8.06 (t, *J* = 1.8 Hz, 1H), 8.03 - 7.76 (m, 2H), 7.44 (t, *J* = 7.7 Hz, 1H), 7.39 - 7.30 (m, 2H), 7.27 (d, *J* = 1.9 Hz, 1H), 6.23 (d, *J* = 1.9 Hz, 1H), 6.09 (s, 1H), 5.35 (p, *J* = 7.1 Hz, 1H), 3.87 (s, 3H), 3.55 - 3.42 (m, 1H), 3.42 - 3.35 (m, 1H), 2.86 (s, 3H), 2.45 - 2.32 (m, 1H), 2.32 - 2.20 (m, 1H), 1.52 (d, *J* = 6.9 Hz, 3H). MS (ESI⁺): m/z = 476.1. | 1) acidic conditions, column C7; 2) SFC1 Mobile phase: 45% methanol, 55% CO₂). 52 mL/min. 1^{st} eluting peak designated as diastereomer 1 (Example 126). 2^{nd} designated as diastereomer 2 (Example 127). | |
| | 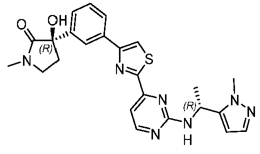 | | | |
| 126 and 127 | (*R*)-3-hydroxy-1-methyl-3-(3-(2-(2-(((S)-1-(1-methyl-1H-pyrazol-5-yl)ethyl)amino)pyrimidin -4-yl)thiazol-4-yl)phenyl)pyrrolidin-2-one and (R)-3-hydroxy-1-methyl-3-(3-(2-(2-(((R)-1-(1-methyl-1H-pyrazol-5-yl)ethyl)amino)pyrimidin -4-yl)thiazol-4-yl)phenyl)pyrrolidin-2-one | | | 15 (diastere omer 1) |
| | | Diastereomer 2 (Example 127): ¹H NMR (400 MHz, DMSO-*d*₆) δ 8.51 (d, *J* = 5.0 Hz, 1H), 8.32 (s, 1H), 8.15 - 8.03 (m, 1H), 8.03 - 7.85 (m, 2H), 7.44 (t, *J =* 7.7 Hz, 1H), 7.39 - 7.30 (m, 2H), 7.27 (d, *J* = 1.9 Hz, 1H), 6.23 (d, *J* = 1.9 Hz, 1H), 6.10 (s, 1H), 5.35 (p, *J* = 7.1 Hz, 1H), 3.87 (s, 3H), 3.53 - 3.42 (m, 1H), 3.42 - 3.36 (m, 1H), 2.86 (s, 3H), 2.43 - 2.34 (m, 1H), 2.34 - 2.18 (m, 1H), 1.52 (d, *J* = 6.9 Hz, 3H). MS (ESI⁺): m/z = 476.1. | | 18 (diastere omer 2) |
| 128 | 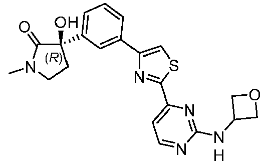 | ¹H NMR (500 MHz, DMSO-*d₆*) δ 8.53 (d, *J* = 5.0 Hz, 1H), 8.34 (s, 1H), 8.23 (d, *J* = 5.6 Hz, 1H), 8.13 - 8.03 (m, 1H), 7.95 - 7.88 (m, 1H), 7.45 (t, *J* = 7.7 Hz, 1H), 7.41 - 7.29 (m, 2H), 6.10 (s, 1H), 4.98 (h, *J =* 6.7 Hz, 1H), 4.89 - 4.74 (m, 2H), 4.59 (t, *J* = 6.3 Hz, 2H), 3.51 - 3.43 (m, 1H), 3.43 - 3.36 (m, 1H), 2.87 (s, 3H), 2.44 - 2.34 (m, 1H), 2.34 - 2.22 (m, 1H). MS (ESI⁺): m/z = 424.4. | basic conditions, column C8 | 37 |
| | (*R*)-3-hydroxy-1-methyl-3-(3-(2-(2-(oxetan-3-ylamino)pyrimidin-4-yl)thiazol-4-yl)phenyl)pyrrolidin-2-one | | | |
| 129 and 130 | 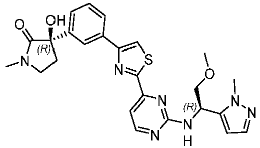 | Diastereomer 1 (Example 129): ¹H NMR (500 MHz, DMSO-*d*₆) δ 8.45 (d, *J* = 5.0 Hz, 1H), 8.26 (s, 1H), 8.00 (t, *J* = 1.8 Hz, 1H), 7.95 - 7.76 (m, 2H), 7.37 (t, *J* = 7.7 Hz, 1H), 7.33 - 7.25 (m, | 1) basic conditions, column C8; | 9 (diastere omer 1) |
| | 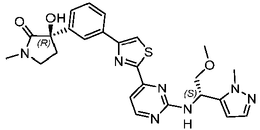 | | 2) SFC1 Mobile phase: 50% | 15 (diastere omer 2) |
| | (*R*)-3-hydroxy-3-(3-(2-(2-(((R)-2-methoxy-1-(1-methyl-1H-pyrazol-5-yl)ethyl)amino)pyrimidin -4-yl)thiazol-4-yl)phenyl)-1-methylpyrrolidin-2-one and (R)-3-hydroxy-3-(3-(2-(2-(((S)-2-methoxy-1-(1-methyl-1H-pyrazol-5-yl)ethyl)amino)pyrimidin -4-yl)thiazol-4-yl)phenyl)-1-methylpyrrolidin-2-one | 2H), 7.23 (d, *J =* 1.9 Hz, 1H), 6.27 - 6.12 (m, 1H), 6.03 (s, 1H), 5.54 - 5.30 (m, 1H), 3.85 (s, 3H), 3.75 - 3.66 (m, 1H), 3.62 - 3.50 (m, 1H), 3.44 - 3.35 (m, 1H), 3.35 - 3.28 (m, 1H), 3.25 (s, 3H), 2.79 (s, 3H), 2.36 - 2.25 (m, 1H), 2.25 - 2.14 (m, 1H). MS (ESI⁺): m/z = 506.6. | methanol, 50% CO₂). 65 mL/min. 1^{st} eluting peak designated as diastereomer 1 (Example 129). 2^{nd} eluting peak designated as diastereomer 2 (Example 130). | |
| | | Diastereomer 2 (Example 130): ¹H NMR (500 MHz, DMSO-*d*₆) δ 8.45 (d, *J* = 5.0 Hz, 1H), 8.26 (s, 1H), 8.00 (t, 1H), 7.94 - 7.74 (m, 2H), 7.37 (t, *J =* 7.7 Hz, 1H), 7.29 (dd, *J* = 11.9, 6.4 Hz, 2H), 7.23 (d, *J* = 1.9 Hz, 1H), 6.26 - 6.17 (m, 1H), 6.02 (s, 1H), 5.53 - 5.31 (m, 1H), 3.85 (s, 3H), 3.75 - 3.67 (m, 1H), 3.60 - 3.50 (m, 1H), 3.44 - 3.35 (m, 2H), 3.34 - 3.28 (m, 4H), 3.25 (s, 3H), 2.79 (s, 3H), 2.37 - 2.25 (m, 1H), 2.26 - 2.14 (m, 1H). MS (ESI⁺): m/z = 506.6. | | |
| 131 | 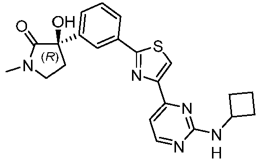 | ¹H NMR (500 MHz, DMSO-*d₆*) δ 8.43 (d, *J* = 5.2 Hz, 2H), 8.10 (t, *J* = 1.9 Hz, 1H), 7.96 - 7.90 (m, 1H), 7.78 (s, 1H), 7.51 (t, *J =* 7.7 Hz, 1H), 7.45 (dt, *J =* 7.8, 1.5 Hz, 1H), 7.33 (d, *J =* 5.1 Hz, 1H), 4.46 (s, 1H), 3.39 (ddd, *J* = 9.9, 7.7, 5.5 Hz, 1H), 2.87 (s, 3H), 2.42 - 2.34 (m, 1H), 2.34 - 2.20 (m, 3H), 2.11 - 1.96 (m, 2H), 1.79 - 1.60 (m, 2H). MS (ESI⁺): m/z = 422. | acidic conditions, column C1 | 57 |
| | (*R*)-3-(3-(4-(2-(cyclobutylamino)pyrimi din-4-yl)thiazol-2-yl)phenyl)-3-hydroxy-1-methylpyrrolidin-2-one | | | |
| 132 | 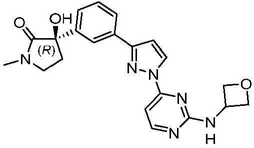 | ¹H NMR (500 MHz, DMSO-*d₆*) δ 8.66 (s, 1H), 8.42 (d, *J* = 5.4 Hz, 1H), 8.24 - 8.16 (m, 1H), 7.99 (t, *J* = 1.8 Hz, 1H), 7.86 - 7.81 (m, 1H), 7.44 (t, *J =* 7.7 Hz, 1H), 7.39 - 7.32 (m, 1H), 7.17 (d, *J* = 5.4 Hz, 1H), 7.10 (d, *J* = 2.8 Hz, 1H), 6.09 (s, 1H), 5.08 - 4.95 (m, 1H), 4.91 - 4.74 (m, 2H), 4.57 (t, *J* = 6.3 Hz, 2H), 3.51 - 3.42 (m, 1H), 3.42 - 3.34 (m, 1H), 2.86 (s, 3H), 2.42 - 2.33 (m, 1H), 2.32 - 2.22 (m, 1H). MS (ESI⁺): m/z = 407. | basic conditions, column C2 | 51 |
| | (*R*)-3-hydroxy-1-methyl-3-(3-(1-(2-(oxetan-3-ylamino)pyrimidin-4-yl)-1 H-pyrazol-3-yl)phenyl)pyrrolidin-2-one | | | |
| | 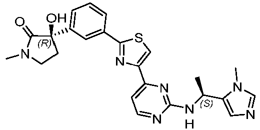 | Diastereomer 1 (Example 133); ¹H NMR (500 MHz, DMSO-*d*₆) δ 8.45 (d, *J* = 4.8 Hz, 2H), 8.09 (t, *J* = 1.9 Hz, 1H), 7.92 (dt, *J* = 7.7, 1.5 Hz, 1H), 7.50 (t, *J* = 7.7 Hz, 1H), 7.46 - 7.35 (m, 2H), 7.32 (d, *J* = 4.9 Hz, 1H), 7.03 (s, 1H), 6.78 (s, 1H), 6.23 (s, 1H), 5.42 (s, 1H), 3.67 (s, 3H), 3.51 - 3.48 (m, 1H), 3.48 - 3.45 (m, 2H), 2.86 (s, 3H), 2.40 - 2.34 (m, 1H), 2.31 - 2.25 (m, 1H), 1.53 (d, *J* = 6.6 Hz, 3H). MS (ESI⁺): m/z = 476. | 1) acidic conditions, column C7 | 9 (diastere omer 1) |
| | 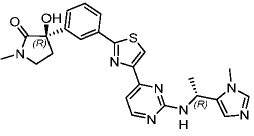 | | 2) SFC1 Mobile phase: 50% methanol with 0.2% TEA, 50% CO₂); 45 mL/min. 1st eluting peak designated as diastereomer 1 (Example 133). 2^{nd} eluting peak designated as diastereomer 2 (Example 134). | |
| 133 and 134 | (*R*)-3-hydroxy-1-methyl-3-(3-(4-(2-(((S)-1-(1-methyl-1H-imidazol-5-yl)ethyl)amino)pyrimidin -4-yl)thiazol-2-yl)phenyl)pyrrolidin-2-one and (R)-3-hydroxy-1-methyl-3-(3-(4-(2-(((R)-1-(1-methyl-1H-imidazol-5-yl)ethyl)amino)pyrimidin -4-yl)thiazol-2-yl)phenyl)pyrrolidin-2-one | | | |
| | | Diastereomer 2 (Example 134): ¹H NMR (500 MHz, DMSO-*d*₆) δ 8.45 (d, *J* = 5.1 Hz, 2H), 8.12 - 8.06 (m, 1H), 7.94 - 7.90 (m, 1H), 7.50 (t, *J* = 7.8 Hz, 1H), 7.43 (dt, *J* = 7.8, 1.4 Hz, 2H), 7.32 (d, *J* = 4.9 Hz, 1H), 7.05 (s, 1H), 6.81 (d, *J* = 5.9 Hz, 1H), 6.23 (s, 1H), 5.42 (s, 1H), 3.67 (s, 3H), 3.62 - 3.54 (m, 3H), 3.51 - 3.47 (m, 5H), 2.86 (s, 3H), 2.40 - 2.33 (m, 1H), 2.32 - 2.25 (m, 1H), 1.53 (d, *J =*6.8 Hz, 3H). MS (ESI⁺): m/z = 476. | | 32 (diastere omer 2) |
| | 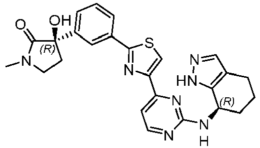 | Diastereomer 1 (Example 135): ¹H NMR (400 MHz, CD₃OD) δ 8.41 (d, *J* = 5.1 Hz, 1H), 8.34 (s, 1H), 8.19 (d, *J* = 2.3 Hz, 1H), 8.02 - 7.94 (m, 1H), 7.57 - 7.46 (m, 2H), 7.43 (d, *J* = 5.1 Hz, 1H), 7.33 (s, 1H), 5.33 (t, *J* = 6.0 Hz, 1H), 3.63 - 3.53 (m, 1H), 3.53 - 3.42 (m, 1H), 2.99 (s, 3H), 2.70 - 2.57 (m, 2H), 2.57 - 2.48 (m, 1H), 2.48 - 2.37 (m, 1H), 2.28 - 2.09 (m, 1H), 2.09 - 1.90 (m, 2H), 1.90 - 1.74 (m, 1H). MS (ESI⁺): m/z = 488. | 1) basic conditions, column C8; | |
| | 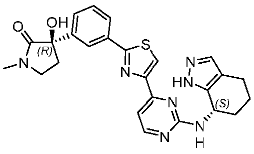 | | | |
| | | | 2) SFC1 Mobile phase: 45% methanol, 55% CO₂); 70 mL/min. 1st eluting peak designated as diastereomer 1 (Example 135). 2^{nd} eluting peak designated as diastereomer 2 (Example 136). | |
| 135 and 136 | (*R*)-3-hydroxy-1-methyl-3-(3-(4-(2-(((R)-4,5,6,7-tetrahydro-1 H-indazol-7-yl)amino)pyrimidin-4-yl)thiazol-2-yl)phenyl)pyrrolidin-2-one and (R)-3-hydroxy-1-methyl-3-(3-(4-(2-(((S)-4,5,6,7-tetrahydro-1 H-indazol-7-yl)amino)pyrimidin-4-yl)thiazol-2-yl)phenyl)pyrrolidin-2-one | | | 16 (diastere omer 1) |
| | | | | 11 (diastere omer 2) |
| | | Diastereomer 2 (Example 136): ¹H NMR (400 MHz, CD₃OD) δ 8.41 (d, *J* = 5.0 Hz, 1H), 8.37 (s, 1H), 8.20 (s, 1H), 8.01 - 7.96 (m, 1H), 7.52 (d, *J* = 6.6. Hz, 2H), 7.44 (d, *J* = 5.1 Hz, 1H), 5.34 (d, *J* = 6.1 Hz, 1H), 3.56 (d, *J* = 8.5 Hz, 1H), 3.48 (t, *J* = 1.7 Hz, 2H), 2.98 (s, 3H), 2.71 - 2.56 (m, 2H), 2.56 - 2.47 (m, 1H), 2.47 - 2.36 (m, 1H), 2.31 - 2.10 (m, 1H), 2.10 - 1.92 (m, 2H), 1.61 (s, 1H). MS (ESI⁺): m/z = 488. | | |
| 137 | 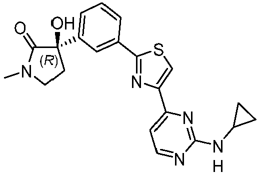 | ¹H NMR (400 MHz, DMSO-*d₆*) δ 8.46 (d, *J =* 5.0 Hz, 1H), 8.38 (s, 1H), 8.10 (t, *J* = 1.8 Hz, 1H), 7.93 (dt, *J* = 7.6, 1.4 Hz, 1H), 7.51 (t, *J* = 7.7 Hz, 1H), 7.47 - 7.41 (m, 2H), 7.33 (d, *J =* 5.0 Hz, 1H), 6.22 (s, 1H), 3.56 - 3.38 (m, 2H), 2.87 (s, 3H), 2.84 - 2.78 (m, 1H), 2.43 - 2.24 (m, 2H), 0.75 - 0.67 (m, 2H), 0.56 - 0.48 (m, 2H). MS (ESI⁺): m/z = 408.1. | basic conditions, column C9 | 65 |
| | (*R*)-3-(3-(4-(2-(cyclopropylamino)pyrim idin-4-yl)thiazol-2-yl)phenyl)-3-hydroxy-1-methylpyrrolidin-2-one | | | |
| 138 and 139 | 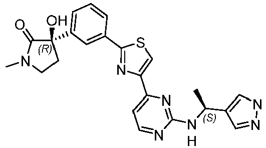 | Diastereomer 1 (Example 138): ¹H NMR (500 MHz, DMSO-*d*₆) δ 12.49 (s, 1H), 8.49 - 8.37 (m, 1H), 8.24 (s, 1H), 8.03 - 7.95 (m, 1H), 7.90 - 7.83 (m, 1H), 7.63 - 7.55 (m, 1H), 7.55 - 7.43 (m, 2H), 7.38 (t, *J* = 7.7 Hz, 1H), 7.31 - 7.26 (m, 1H), 7.25 (d, *J* = 4.9 Hz, 1H), 6.02 (s, 1H), 5.27 - 5.08 (m, 1H), 3.44 - 3.36 (m, 1H), 3.35 - 3.29 (m, 1H), 2.80 (s, 3H), 2.35 - 2.26 (m, 1H), 2.26 - 2.12 (m, 1H), 1.49 - 1.33 (m, 3H). MS (ESI⁺): m/z = 462.4. Diastereomer 2 (Example 139): ¹H NMR (500 MHz, DMSO-*d*₆) δ 12.90 - 12.06 (m, 1H), 8.53 - 8.45 (m, 1H), 8.32 (s, 1H), 8.10 - 8.05 (m, 1H), 7.97 - 7.90 (m, 1H), 7.68 - 7.62 (m, 1H), 7.58 (s, 2H), 7.47 - 7.42 (m, 1H), 7.38 - 7.34 (m, 1H), 7.32 (d, *J* = 4.9 Hz, 1H), 6.26 - 5.86 (m, 1H), 5.37 - 4.96 (m, 1H), 3.56 - 3.44 (m, 1H), 3.42 - 3.36 (m, 1H), 2.87 (s, 3H), 2.44 - 2.34 (m, 1H), 2.34 - 2.24 (m, 1H), 1.61 - 1.37 (m, 3H). MS (ESI⁺): m/z = 462.4. | 1) Basic conditions, column C8; | |
| | 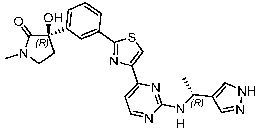 | | 2) SFC1 Mobile phase: 55% methanol, 45% CO₂); 65 mL/min. 1^{st} eluting peak designated as diastereomer 1 (Example 138). 2^{nd} eluting peak designated as diastereomer 2 (Example 139). | 3 (diastere omer 1) |
| | (*R*)-3-(3-(4-(2-(((*S*)-1-(1H-pyrazol-4-yl)ethyl)amino)pyrimidin -4-yl)thiazol-2-yl)phenyl)-3-hydroxy-1-methylpyrrolidin-2-one and (*R*)-3-(3-(4-(2-(((*R*)-1-(1H-pyrazol-4-yl)ethyl)amino)pyrimidin -4-yl)thiazol-2-yl)phenyl)-3-hydroxy-1-methylpyrrolidin-2-one | | | 9 (diastere omer 2) |
| 140 | 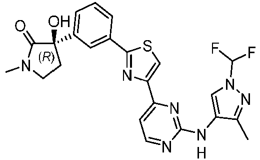 | MS (ESI⁺): m/z = 498.4. | Acidic conditions, column C1 | 2 |
| | (R)-3-(3-(4-(2-((1-(difluoromethyl)-3-methyl-1H-pyrazol-4-yl)amino)pyrimidin-4-yl)thiazol-2-yl)phenyl)-3-hydroxy-1-methylpyrrolidin-2-one | | | |
| 141 | 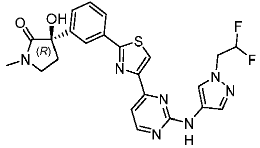 | MS (ESI⁺): m/z = 498.1. | Basic conditions, column C2 | 2 |
| | (R)-3-(3-(4-(2-((1-(2,2-difluoroethyl)-1H-pyrazol-4-yl)amino)pyrimidin-4-yl)thiazol-2-yl)phenyl)-3-hydroxy-1-methylpyrrolidin-2-one | | | |
| 142 and 143 | 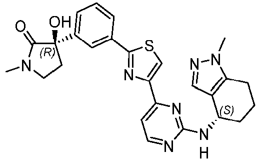 | Diastereomer 1 (Example 142): ¹H NMR (500 MHz, DMSO-*d*₆) δ 8.57 - 8.26 (m, 2H), 8.09 (t, *J* = 1.9 Hz, 1H), 7.93 (dt, *J* = 7.9, 1.4 Hz, 1H), 7.51 (t, *J* = 7.7 Hz, 1H), 7.44 (dt, *J* = 7.8, 1.3 Hz, 1H), 7.32 (d, *J* = 5.0 Hz, 1H), 7.28 - 7.15 (m, 2H), 6.23 (s, 1H), 5.24 - 5.04 (m, 1H), 3.67 (s, 3H), 3.52 - 3.48 (m, 1H), 3.48 - 3.45 (m, 1H), 2.86 (s, 3H), 2.65 - 2.55 (m, 2H), 2.42 - 2.33 (m, 1H), 2.33 - 2.25 (m, 1H), 2.08 - 1.98 (m, 1H), 1.98 - 1.89 (m, 1H), 1.83 - 1.65 (m, 2H). MS (ESI⁺): m/z = 502. | 1) basic conditions, column C8; | 20 (diastere omer 1) |
| | 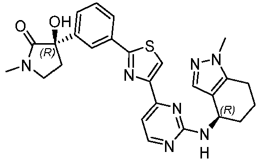 | | 2) SFC1; Mobile phase: 45% methanol, 55% CO₂); 70 mL/min. 2nd eluting peak designated as diastereomer 1 (Example 142). 1st eluting peak designated as diastereomer 2 (Example 143). | |
| | (*R*)-3-hydroxy-1-methyl-3-(3-(4-(2-(((*S*)-1-methyl-4,5,6,7-tetrahydro-1H-indazol-4-yl)amino)pyrimidin-4-yl)thiazol-2-yl)phenyl)pyrrolidin-2-one and (*R*)-3-hydroxy-1-methyl-3-(3-(4-(2-(((R)-1-methyl-4,5,6,7-tetrahydro-1H-indazol-4-yl)amino)pyrimidin-4-yl)thiazol-2-yl)phenyl)pyrrolidin-2-one | | | |
| | | | | 18 (diastere omer 2) |
| | | Diastereomer 2 (Example 143): ¹H NMR (500 MHz, DMSO-*d*₆) δ 8.63 - 8.23 (m, 2H), 8.09 (t, *J* = 1.8 Hz, 1H), 7.93 (dt, *J* = 7.6, 1.4 Hz, 1H), 7.51 (t, *J* = 7.7 Hz, 1H), 7.44 (dt, *J* = 7.9, 1.4 Hz, 1H), 7.32 (d, *J* = 5.0 Hz, 1H), 7.27 - 7.15 (m, 2H), 6.23 (s, 1H), 5.23 - 5.03 (m, 1H), 3.66 (s, 3H), 3.52 - 3.48 (m, 1H), 3.48 - 3.45 (m, 1H), 2.86 (s, 3H), 2.65 - 2.55 (m, 2H), 2.42 - 2.33 (m, 1H), 2.33 - 2.24 (m, 1H), 2.08 - 1.98 (m, 1H), 1.98 - 1.90 (m, 1H), 1.84 - 1.63 (m, 2H). MS (ESI⁺): m/z = 502. | | |

The compounds shown in Table 3C were prepared in a manner analogous to Example 46, (*R*)-3-hydroxy-1-methyl-3-(5-(2-(2-((1-methyl-1*H*-pyrazol-5-yl)amino)pyrimidin-4-yl)thiazol-4-yl)pyridin-3-yl)pyrrolidin-2-one using the appropriate coupling partner in place of 4-(4-bromothiazol-2-yl)-*N*-(1-methyl-1*H*-pyrazol-5-yl)pyrimidin-2-amine (Intermediate 14). Purification methods are noted in the table.

**Table 3C:**

| # | Structure | Analytical Data | RP HPLC Condition | Yield (%) |
|---|---|---|---|---|
| | Name | | | |
| 144 | 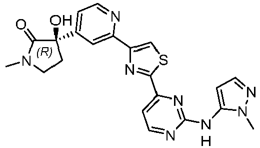 or 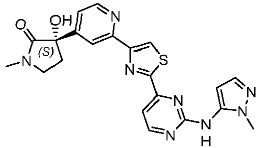 | ¹H NMR (500 MHz, DMSO-*d₆*) δ 9.79 (s, 1H), 8.70 (d, *J* = 5.0 Hz, 1H), 8.65 - 8.61 (m, 1H), 8.59 (s, 1H), 8.30 - 8.26 (m, 1H), 7.63 (d, *J* = 4.9 Hz, 1H), 7.41 (d, *J* = 1.9 Hz, 1H), 7.39 (dd, *J* = 5.2, 1.8 Hz, 1H), 6.38 - 6.33 (m, 1H), 3.74 (s, 3H), 3.57 - 3.50 (m, 1H), 3.48 - 3.41 (m, 1H), 2.89 (s, 3H), 2.45 - 2.38 (m, 1H), 2.34 - 2.27 (m, 1H). MS (ESI⁺): m/z = 449. | acidic conditions, column C7 | 23 |
| | (*R*)-3-hydroxy-1-methyl-3-(2-(2-(2-((1-methyl-1H-pyrazol-5-yl)amino)pyrimidin-4-yl)thiazol-4-yl)pyridin-4-yl)pyrrolidin-2-one or (*S*)-3-hydroxy-1-methyl-3-(2-(2-(2-((1-methyl-1H-pyrazol-5-yl)amino)pyrimidin-4-yl)thiazol-4-yl)pyridin-4-yl)pyrrolidin-2-one | | | |
| 145 | 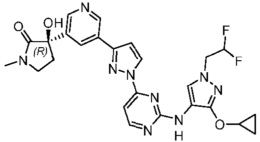 | ¹H NMR (500 MHz, DMSO-*d₆*) δ 9.09 (t, *J* = 2.7 Hz, 1H), 8.60 (d, *J* = 2.3 Hz, 1H), 8.50 (d, *J* = 5.3 Hz, 1H), 8.35 (d, *J* = 2.4 Hz, 1H), 7.94 (s, 1H), 7.28 (dd, *J* = 4.1, 1.4 Hz, 2H), 6.46 - 6.20 (m, 4H), 4.51 (t, *J* = 14.5 Hz, 3H), 4.10 (tt, *J* = 6.0, 3.0 Hz, 1H), 3.54 - 3.48 (m, 3H), 3.42 (s, 5H), 2.87 (s, 3H), 2.31 (ddd, *J* = 13.4, 8.0, 5.2 Hz, 1H), 2.03 - 1.95 (m, 1H), 1.24 (d, *J* = 3.2 Hz, 2H), 0.72 - 0.67 (m, 2H), 0.67 - 0.62 (m, 2H). MS (ESI⁺): m/z = 538. | acidic conditions, column C7 | 38 |
| | or | | | |
| | 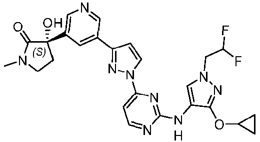 | | | |
| | (*R*)-3-(5-(1-(2-((3-cyclopropoxy-1-(2,2-difluoroethyl)-1H-pyrazol-4-yl)amino)pyrimidin-4-yl)-1H-pyrazol-3-yl)pyridin-3-yl)-3-hydroxy-1-methylpyrrolidin-2-one or (S)-3-(5-(1-(2-((3-cyclopropoxy-1-(2,2-difluoroethyl)-1H-pyrazol-4-yl)amino)pyrimidin-4-yl)-1H-pyrazol-3-yl)pyridin-3-yl)-3-hydroxy-1-methylpyrrolidin-2-one | | | |
| 146 | 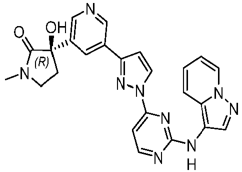 | ¹H NMR (500 MHz, DMSO-*d₆*) δ 9.78 (s, 1H), 9.11 (s, 1H), 8.66 - 8.61 (m, 1H), 8.61 - 8.58 (m, 1H), 8.58 - 8.53 (m, 1H), 8.47 - 8.35 (m, 2H), 7.81 (d, *J* = 9.0 Hz, 1H), 7.33 (d, *J* = 5.3 Hz, 1H), 7.28 (s, 1H), 7.17 - 7.12 (m, 1H), 6.85 (td, *J* = 6.8, 1.4 Hz, 1H), 5.75 (s, 1H), 2.87 (s, 3H), 2.35 - 2.32 (m, 1H), 2.32 - 2.28 (m, 1H). MS (ESI⁺): m/z = 468. | acidic conditions, column C7 | 45 |
| | or | | | |
| | 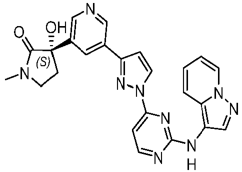 | | | |
| | (*R*)-3-hydroxy-1-methyl-3-(5-(1-(2-(pyrazolo[1,5-a]pyridin-3-ylamino)pyrimidin-4-yl)-1H-pyrazol-3-yl)pyridin-3-yl)pyrrolidin-2-one or (*S*)-3-hydroxy-1-methyl-3-(5-(1-(2-(pyrazolo[1,5-a]pyridin-3-ylamino)pyrimidin-4-yl)-1H-pyrazol-3-yl)pyridin-3-yl)pyrrolidin-2-one | | | |
| 147 | 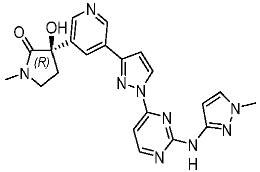 | ¹H NMR (500 MHz, CD₂Cl₂) δ 7.11 (d, *J =* 2.1 Hz, 1H), 6.65 (d, *J* = 2.2 Hz, 1H), 6.55 (d, *J* = 2.7 Hz, 1H), 6.54 (d, *J* = 5.4 Hz, 1H), 6.35 (t, *J* = 2.2 Hz, 1H), 6.22 (s, 1H), 5.45 (d, *J* = 5.4 Hz, 1H), 5.41 (d, *J* = 2.3 Hz, 1H), 4.81 (d, *J* = 2.8 Hz, 1H), 4.76 (d, *J* = 2.2 Hz, 1H), 3.40 (s, 1H), 1.96 (s, 3H), 1.71 - 1.65 (m, 1H), 1.61 - 1.55 (m, 1H), 1.18 (s, 3H), 0.72 - 0.65 (m, 1H), 0.62 - 0.56 (m, 1H). MS (ESI⁺): m/z = 432. | basic conditions, column C8 | 5 |
| | or | | | |
| | 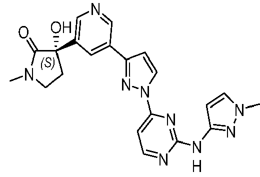 | | | |
| | (*R*)-3-hydroxy-1-methyl-3-(5-(1-(2-((1-methyl-1H-pyrazol-3-yl)amino)pyrimidin-4-yl)-1H-pyrazol-3-yl)pyridin-3-yl)pyrrolidin-2-one or (*S*)-3-hydroxy-1-methyl-3-(5-(1-(2-((1-methyl-1H-pyrazol-3-yl)amino)pyrimidin-4-yl)-1H-pyrazol-3-yl)pyridin-3-yl)pyrrolidin-2-one | | | |
| 148 | 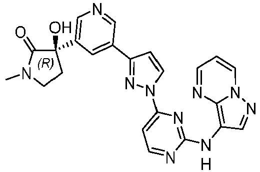 | ¹H NMR (500 MHz, CDCl₃) δ 9.01 (d, *J* = 2.1 Hz, 1H), 8.77 (s, 1H), 8.62 (dd, *J* = 7.1, 1.7 Hz, 1H), 8.55 (d, *J* = 2.7 Hz, 1H), 8.54 (d, *J* = 2.3 Hz, 1H), 8.52 (d, *J* = 5.4 Hz, 1H), 8.39 (dd, *J* = 3.9, 1.7 Hz, 1H), 8.31 (t, *J* = 2.2 Hz, 1H), 7.41 (d, *J* = 5.4 Hz, 1H), 6.83 (d, *J* = 2.7 Hz, 1H), 6.78 (dd, *J* = 7.1, 3.9 Hz, 1H), 3.57 - 3.51 (m, 1H), 3.48 - 3.42 (m, 1H), 3.06 (s, 3H), 2.62 - 2.54 (m, 1H), 2.50 - 2.43 (m, 1H). MS (ESI⁺): m/z = 469. | acidic conditions, column C8 | 8 |
| | or | | | |
| | 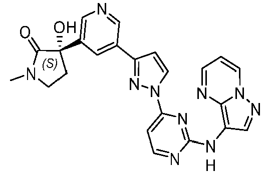 | | | |
| | (*R*)-3-hydroxy-1-methyl-3-(5-(1-(2-(pyrazolo[1,5-a]pyrimidin-3-ylamino)pyrimidin-4-yl)-1H-pyrazol-3-yl)pyridin-3-yl)pyrrolidin-2-one or (*S*)-3-hydroxy-1-methyl-3-(5-(1-(2-(pyrazolo[1,5-a]pyrimidin-3-ylamino)pyrimidin-4-yl)-1H-pyrazol-3-yl)pyridin-3-yl)pyrrolidin-2-one | | | |
| 149 | 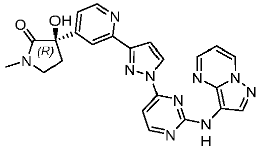 | ¹H NMR (500 MHz, CDCl₃) δ 8.65 (s, 1H), 8.60 (dd, *J* = 7.1, 1.7 Hz, 1H), 8.53 (d, *J* = 5.2 Hz, 1H), 8.51 (d, *J* = 5.4 Hz, 1H), 8.44 (s, 1H), 8.38 (dd, *J =* 3.9, 1.7 Hz, 1H), 8.16 (s, 1H), 7.40 (d, *J* = 5.4 Hz, 1H), 7.15 - 7.12 (m, 1H), 7.09 (d, *J* = 2.7 Hz, 1H), 6.76 (dd, *J* = 7.1, 3.9 Hz, 1H), 3.62 - 3.54 (m, 1H), 3.51 - 3.44 (m, 1H), 3.08 (s, 3H), 2.58 - 2.50 (m, 1H), 2.43 - 2.36 (m, 1H). MS (ESI⁺): m/z = 469. | basic conditions, column C2 | 14 |
| | or | | | |
| | 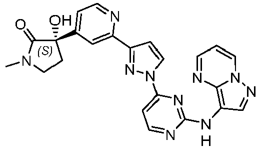 | | | |
| | (*R*)-3-hydroxy-1-methyl-3-(2-(1-(2-(pyrazolo[1,5-a]pyrimidin-3-ylamino)pyrimidin-4-yl)-1H-pyrazol-3-yl)pyridin-4-yl)pyrrolidin-2-one or (*S*)-3-hydroxy-1-methyl-3-(2-(1-(2-(pyrazolo[1,5-a]pyrimidin-3-ylamino)pyrimidin-4-yl)-1H-pyrazol-3-yl)pyridin-4-yl)pyrrolidin-2-one | | | |
| 150 | 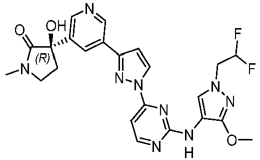 | ¹H NMR (500 MHz, DMSO-*d₆*) δ 9.07 (d, *J* = 2.0 Hz, 1H), 8.63 - 8.59 (m, 1H), 8.58 (d, *J* = 2.2 Hz, 1H), 8.50 (d, *J* = 5.3 Hz, 1H), 8.32 (t, *J* = 2.1 Hz, 1H), 7.96 - 7.89 (m, 1H), 7.30 - 7.27 (m, 1H), 7.27 - 7.24 (m, 1H), 6.45 - 6.20 (m, 3H), 4.54 - 4.43 (m, 2H), 3.84 (s, 3H), 3.54 - 3.48 (m, 2H), 3.45 - 3.42 (m, 3H), 2.87 (s, 3H), 2.48 - 2.43 (m, 1H), 2.34 - 2.27 (m, 1H). MS (ESI⁺): m/z = 512. | basic conditions, column C8 | 38 |
| | or | | | |
| | 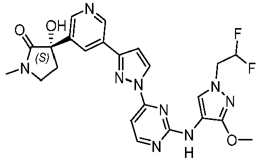 | | | |
| | (R)-3-(5-(1-(2-((1-(2,2-difluoroethyl)-3-methoxy-1H-pyrazol-4-yl)amino)pyrimidin-4-yl)-1H-pyrazol-3-yl)pyridin-3-yl)-3-hydroxy-1-methylpyrrolidin-2-one or (*S*)-3-(5-(1-(2-((1-(2,2-difluoroethyl)-3-methoxy-1H-pyrazol-4-yl)amino)pyrimidin-4-yl)-1H-pyrazol-3-yl)pyridin-3-yl)-3-hydroxy-1-methylpyrrolidin-2-one | | | |
| 151 | 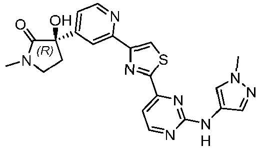 | ¹H NMR (500 MHz, DMSO-*d₆*) δ 9.81 (s, 1H), 8.72 - 8.62 (m, 2H), 8.60 (s, 1H), 8.28 (d, *J* = 1.7 Hz, 1H), 7.95 (s, 1H), 7.63 (s, 1H), 7.49 (d, J = 4.9 Hz, 1H), 7.38 (dd, *J =* 5.2, 1.8 Hz, 1H), 3.86 (s, 3H), 3.58 - 3.50 (m, 1H), 3.49 - 3.39 (m, 1H), 2.90 (s, 3H), 2.45 - 2.38 (m, 1H), 2.35 - 2.27 (m, 1H). MS (ESI⁺): m/z = 449.7. | acidic conditions, column C7 | 12 |
| | or | | | |
| | 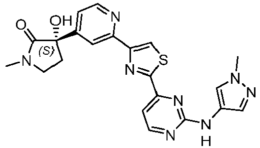 | | | |
| | (*R*)-3-hydroxy-1-methyl-3-(5-(2-(2-((1-methyl-1H-pyrazol-4-yl)amino)pyrimidin-4-yl)thiazol-4-yl)pyridin-3-yl)pyrrolidin-2-one or (*S*)-3-hydroxy-1-methyl-3-(5-(2-(2-((1-methyl-1H-pyrazol-4-yl)amino)pyrimidin-4-yl)thiazol-4-yl)pyridin-3-oryl)pyrrolidin-2-one | | | |
| 152 | 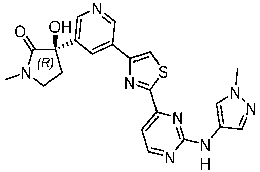 or | ¹H NMR (600 MHz, DMSO-*d₆*) δ 9.80 (s, 1H), 9.19 (d, *J* = 2.1 Hz, 1H), 8.64 (d, *J* = 4.9 Hz, 1H), 8.61 - 8.52 (m, 2H), 8.46 - 8.40 (m, 1H), 7.94 (d, *J* = 0.7 Hz, 1H), 7.63 (s, 1H), 7.50 (d, *J* = 4.9 Hz, 1H), 6.34 (s, 1H), 3.86 (s, 3H), 3.57 - 3.47 (m, 1H), 3.47 - 3.39 (m, 1H), 2.88 (s, 3H), 2.50 - 2.46 (m, 1H), 2.37 - 2.27 (m, 1H). MS (ESI⁺): m/z = 449.3. | basic conditions, column C8 | 32 |
| | 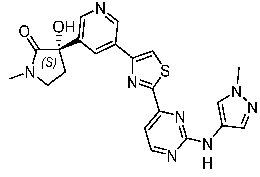 | | | |
| | (*R*)-3-hydroxy-1-methyl-3-(5-(2-(2-((1-methyl-1H-pyrazol-4-yl)amino)pyrimidin-4-yl)thiazol-4-yl)pyridin-3-yl)pyrrolidin-2-one or (*S*)-3-hydroxy-1-methyl-3-(5-(2-(2-((1-methyl-1H-pyrazol-4-yl)amino)pyrimidin-4-yl)thiazol-4-yl)pyridin-3-yl)pyrrolidin-2-one | | | |
| 153 | 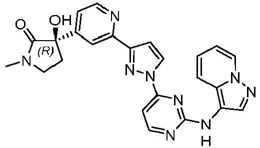 | ¹H NMR (500 MHz, DMSO-*d₆*) δ 9.79 (s, 1H), 8.63 - 8.61 (m, 1H), 8.61 - 8.59 (m, 1H), 8.58 - 8.56 (m, 1H), 8.41 - 8.37 (m, 1H), 8.18 - 8.16 (m, 1H), 7.83 - 7.80 (m, 1H), 7.37 - 7.34 (m, 1H), 7.33 (d, J = 5.3 Hz, 1H), 7.20 - 7.17 (m, 1H), 7.17 - 7.15 (m, 1H), 7.15 - 7.13 (m, 1H), 6.88 - 6.84 (m, 1H), 3.56 - 3.47 (m, 3H), 3.48 - 3.40 (m, 2H), 2.89 (s, 3H), 2.44 - 2.35 (m, 3H), 2.33 - 2.26 (m, 2H). MS (ESI⁺): m/z = 468.0. | acidic conditions, column C7 | 30 |
| | or | | | |
| | 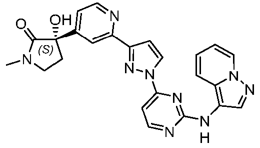 | | | |
| | (*R*)-3-hydroxy-1-methyl-3-(2-(1-(2-(pyrazolo[1,5-a]pyridin-3-ylamino)pyrimidin-4-yl)-1H-pyrazol-3-yl)pyridin-4-yl)pyrrolidin-2-one or (*S*)-3-hydroxy-1-methyl-3-(2-(1-(2-(pyrazolo[1,5-a]pyridin-3-ylamino)pyrimidin-4-yl)-1H-pyrazol-3-yl)pyridin-4-yl)pyrrolidin-2-one | | | |
| 154 | 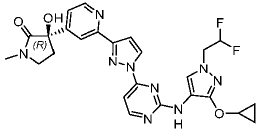 | ¹H NMR (500 MHz, DMSO-*d₆*) δ 8.64 - 8.59 (m, 2H), 8.52 (d, *J* = 5.3 Hz, 1H), 8.18 - 8.14 (m, 1H), 7.97 - 7.92 (m, 1H), 7.38 - 7.34 (m, 1H), 7.29 (d, *J* = 5.3 Hz, 1H), 7.20 - 7.16 (m, 1H), 6.46 - 6.21 (m, 3H), 4.57 - 4.46 (m, 3H), 4.14 - 4.09 (m, 2H), 3.55 - 3.49 (m, 2H), 3.47 - 3.40 (m, 2H), 2.88 (s, 3H), 2.42 - 2.35 (m, 2H), 2.33 - 2.25 (m, 2H), 0.74 - 0.68 (m, 2H), 0.68 - 0.63 (m, 2H). MS (ESI⁺): m/z = 538.0. | acidic conditions, column C7 | 34 |
| | or | | | |
| | 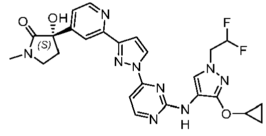 | | | |
| | (*R*)-3-(2-(1-(2-((3-cyclopropoxy-1-(2,2-difluoroethyl)-1H-pyrazol-4-yl)amino)pyrimidin-4-yl)-1H-pyrazol-3-yl)pyridin-4-yl)-3-hydroxy-1-methylpyrrolidin-2-one or (*S*)-3-(2-(1-(2-((3-cyclopropoxy-1-(2,2-difluoroethyl)-1H-pyrazol-4-yl)amino)pyrimidin-4-yl)-1H-pyrazol-3-yl)pyridin-4-yl)-3-hydroxy-1-methylpyrrolidin-2-one | | | |
| 155 | 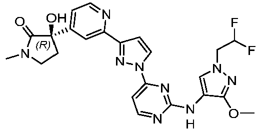 | ¹H NMR (500 MHz, DMSO-*d₆*) δ 8.62 - 8.61 (m, 1H), 8.60 - 8.58 (m, 1H), 8.53 - 8.50 (m, 1H), 8.16 - 8.14 (m, 1H), 7.96 - 7.90 (m, 1H), 7.34 (dd, *J* = 5.2, 1.8 Hz, 1H), 7.29 (d, *J* = 5.3 Hz, 1H), 7.17 (d, *J* = 2.7 Hz, 1H), 6.39 (s, 1H), 6.34 - 6.31 (m, 1H), 4.54 - 4.44 (m, 3H), 3.85 (s, 3H), 3.55 - 3.49 (m, 2H), 3.47 - 3.39 (m, 2H), 2.88 (s, 3H), 2.33 - 2.26 (m, 2H), 2.04 - 1.97 (m, 2H). MS (ESI⁺): m/z = 512.4. | basic conditions, column C2 | 29 |
| | or | | | |
| | 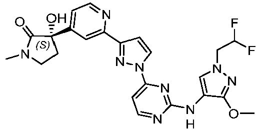 | | | |
| | (*R*)-3-(2-(1-(2-((1-(2,2-difluoroethyl)-3-methoxy-1H-pyrazol-4-yl)amino)pyrimidin-4-yl)-1H-pyrazol-3-yl)pyridin-4-yl)-3-hydroxy-1-methylpyrrolidin-2-one or (*S*)-3-(2-(1-(2-((1-(2,2-difluoroethyl)-3-methoxy-1H-pyrazol-4-yl)amino)pyrimidin-4-yl)-1H-pyrazol-3-yl)pyridin-4-yl)-3-hydroxy-1-methylpyrrolidin-2-one | | | |
| 156 and 157 | 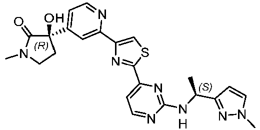 | Diastereomer 1 (Example 156): ¹H NMR (500 MHz, DMSO-*d₆*) δ 8.60 (d, 1H), 8.50 (d, *J* = 4.9 Hz, 1H), 8.48 (s, 1H), 8.23 - 8.20 (m, 1H), 7.65 (d, *J* = 8.4 Hz, 1H), 7.53 (d, *J* = 2.1 Hz, 1H), 7.35 - 7.30 (m, 2H), 6.38 (s, 1H), 6.19 (s, 1H), | 1) basic conditions column C8 | 9 (diastere omer 1) |
| | and | | 2) SFC2 Mobile phase: 40% methanol, | 7 (diastere omer 2) |
| | 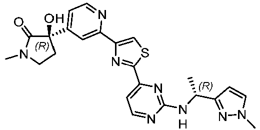 | | | |
| | or | 5.29 - 5.21 (m, 1H), 3.78 (s, 3H), 3.54 - 3.48 (m, 1H), 3.46 - 3.39 (m, 1H), 2.88 (s, 3H), 2.41 - 2.35 (m, 1H), 2.32 - 2.25 (m, 1H), 1.49 (d, *J* = 7.0 Hz, 3H). MS (ESI⁺): m/z = 477.2. Diastereomer 2 (Example 157): ¹H NMR (500 MHz, DMSO-*d₆*) δ 8.60 (d, 1H), 8.50 (d, *J* = 4.9 Hz, 1H), 8.48 (s, 1H), 8.23 - 8.20 (m, 1H), 7.65 (d, *J* = 8.4 Hz, 1H), 7.53 (d, *J* = 2.1 Hz, 1H), 7.35 - 7.30 (m, 2H), 6.38 (s, 1H), 6.19 (s, 1H), 5.29 - 5.21 (m, 1H), 3.78 (s, 3H), 3.54 - 3.48 (m, 1H), 3.46 - 3.39 (m, 1H), 2.88 (s, 3H), 2.41 - 2.35 (m, 1H), 2.32 - 2.25 (m, 1H), 1.49 (d, *J* = 7.0 Hz, 3H). MS (ESI⁺): m/z = 477.2. | 60% CO₂); 50 mL/min. 1st eluting peak designated as diastereomer 1 (Example 156) 2nd eluting peak designated as diastereomer 2 (Example 157) | |
| | 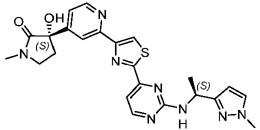 | | | |
| | and | | | |
| | 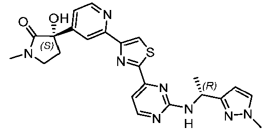 | | | |
| | either (*R*)-3-hydroxy-1-methyl-3-(2-(2-(2-(((*S*)-1-(1-methyl-1H-pyrazol-3-yl)ethyl)amino)pyrimidin -4-yl)thiazol-4-yl)pyridin-4-yl)pyrrolidin-2-one and (*R*)-3-hydroxy-1-methyl-3-(2-(2-(2-(((*R*)-1-(1-methyl-1H-pyrazol-3-yl)ethyl)amino)pyrimidin -4-yl)thiazol-4-yl)pyridin-4-yl)pyrrolidin-2-one | | | |
| | or | | | |
| | (*S*)-3-hydroxy-1-methyl-3-(2-(2-(2-(((*S*)-1-(1-methyl-1H-pyrazol-3-yl)ethyl)amino)pyrimidin -4-yl)thiazol-4-yl)pyridin-4-yl)pyrrolidin-2-one and (*S*)-3-hydroxy-1-methyl-3-(2-(2-(2-(((*R*)-1-(1-methyl-1H-pyrazol-3-yl)ethyl)amino)pyrimidin -4-yl)thiazol-4-yl)pyridin-4-yl)pyrrolidin-2-one | | | |
| 158 | 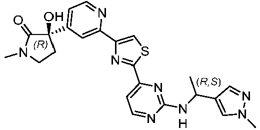 | ¹H NMR (500 MHz, DMSO-*d₆*) δ 8.62 - 8.59 (m, 1H), 8.53 - 8.50 (m, 1H), 8.49 (s, 1H), 8.24 - 8.21 (m, 1H), 7.74 - 7.68 (m, 1H), 7.63 - 7.58 (m, 1H), 7.44 - 7.37 (m, 1H), 7.36 - 7.33 (m, 1H), 7.33 - 7.31 (m, 1H), 6.39 (s, 1H), 5.22 - 5.13 (m, 1H), 3.77 (s, 3H), 3.55 - 3.49 (m, 1H), 3.47 - 3.40 (m, 1H), 2.89 (s, 3H), 2.42 - 2.38 (m, 1H), 2.33 - 2.27 (m, 1H), 1.52 - 1.45 (m, 3H). MS (ESI⁺): m/z = 477.2. | Basic conditions, column C8 | 9 |
| | or | | | |
| | 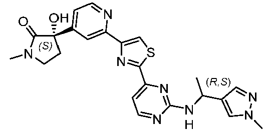 | | | |
| | (*R*)-3-hydroxy-1-methyl-3-(2-(2-(2-(((*R,S*)-1-(1-methyl-1H-pyrazol-4-yl)ethyl)amino)pyrimidin -4-yl)thiazol-4-yl)pyrrolid-4-yl)pyrrolidine-2-one or (S)-3-hydroxy-1-methyl-3-(2-(2-(2-(((*R,S*)-1-(1-methyl-1H-pyrazol-4-yl)ethyl)amino)pyrimidin -4-yl)thiazol-4-yl)pyrrolid-4-yl)pyrrolidine-2-one | | | |

The compounds shown in Table 3D were prepared in a manner analogous to Example 48 using the appropriate amine in place of 6-methylpyridin-2-amine. Purification conditions are noted in the table.

**Table 3D:**

| # | Structure | Analytical data | Purification conditions | Yield (%) |
|---|---|---|---|---|
| | Name | | | |
| 159 | 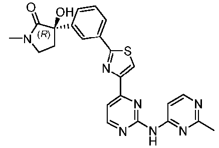 | ¹H NMR (400 MHz, DMSO-*d₆*) δ 10.43 (s, 1H), 8.78 (d, *J* = 4.0 Hz, 1H), 8.68 - 8.63 (m, 1H), 8.54 (d, *J* = 8.0 Hz, 1H), 8.32 (d, *J* = 8.0 Hz, 1H), 8.17 - 8.12 (m, 1H), 7.97 (d, *J* = 8.0 Hz, 1H), 7.75 (d, *J* = 4.0 Hz, 1H), 7.56 - 7.50 (m, 1H), 7.49 - 7.42 (m, 1H), 6.29 - 6.22 (m, 1H), 3.52 - 3.47 (m, 1H), 3.42 - 3.39 (m, 1H), 2.87 (s, 3H), 2.53 (s, 3H), 2.43 - 2.35 (m, 1H), 2.34 - 2.26 (m, 1H). MS (ESI⁺): m/z = 460.2. | 1) FCC: eluent 5% MeOH/DCM; | 7 |
| | (*R*)-3-Hydroxy-1-methyl-3-(3-(4-(2-((2-methylpyrimidin-4-yl)amino)pyrimidin-4-yl)thiazol-2-yl)phenyl)pyrrolidin-2-one | | 2) RP HPLC: Column C10; eluent: 35% to 65% (v/v) CH₃CN and H₂O with 0.05% NH₃ + 10 mM NH₄HCO₃) | |
| 160 | 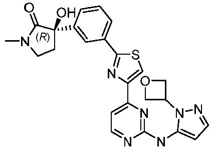 | ¹H NMR (400 MHz, DMSO-*d₆*) δ 9.59 (s, 1H), 8.58 (d, *J* = 4.8 Hz, 1H), 8.31 (s, 1H), 8.11 - 8.09 (m, 1H), 7.96 - 7.91 (m, 1H), 7.60 (d, *J* = 2.0 Hz, 1H), 7.57 (d, *J* = 5.2 Hz, 1H), 7.54 - 7.49 (m, 1H), 7.47 - 7.43 (m, 1H), 6.33 - 6.23 (m, 2H), 5.57 - 5.50 (m, 1H), 4.99 - 4.93 (m, 2H), 4.83 - 4.80 (m, 2H), 3.52 - 3.46 (m, 1H), 3.43 - 3.40 (m, 1H), 2.87 (s, 3H), 2.42 - 2.36 (m, 1H), 2.32 - 2.25 (m, 1H). MS (ESI⁺): m/z = 490.2. | 1) FCC: eluent 5% MeOH/DCM; | 1 |
| | | | 2) RP HPLC: Phenomenex C18 column, 3 µm, 75 x 30 mm; eluent: 27% to 57% (v/v) CH₃CN and H₂O with 0.05% NH₃ + 10 mM NH₄HCO₃) | |
| | (*R*)-3-Hydroxy-1-methyl-3-(3-(4-(2-((1-(oxetan-3-yl)-1H-pyrazol-5-yl)amino)pyrimidin-4-yl)thiazol-2-yl)phenyl)pyrrolidin-2-one | | 3) SFC: DAICEL CHIRALCEL OJ-H column, 5 µm, 250 x 30 mm; 40% (v/v) EtOH (containing 0.1% of 25% aq. NH₃)/CO₂) | |
| 161 | 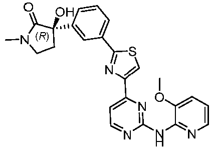 | ¹H NMR (400 MHz, DMSO-*d₆*) δ 9.19 (s, 1H), 8.55 (d, *J* = 5.2 Hz, 1H), 8.28 (s, 1H), 8.13 - 8.07 (m, 1H), 8.00 - 7.89 (m, 2H), 7.56 - 7.36 (m, 4H), 7.23 - 7.16 (m, 1H), 6.25 (s, 1H), 3.80 (s, 3H), 3.52 - 3.41 (m, 2H), 2.87 (s, 3H), 2.38 - 2.27 (m, 2H). MS (ESI⁺): m/z = 475.2. | 1) FCC: eluent 16% MeOH/DCM; | 7 |
| | (*R*)-3-Hydroxy-3-(3-(4-(2-((3-methoxypyridin-2-yl)amino)pyrimidin-4-yl)thiazol-2-yl)phenyl)-1-methylpyrrolidin-2-one | | 2) RP HPLC: Boston Prime C18 column, 3 µm, 75 x 30 mm; eluent: 23% to 53% (v/v) CH₃CN and H₂O with 0.05% NH₃) | |
| 162 | 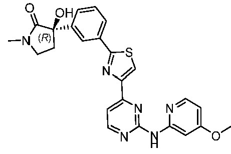 | ¹H NMR (400 MHz, DMSO-*d₆*) δ 9.79 (s, 1H), 8.75 - 8.70 (m, 1H), 8.48 (s, 1H), 8.18 - 8.05 (m, 3H), 7.99 - 7.94 (m, 1H), 7.65 (d, *J* = 4.8 Hz, 1H), 7.57 - 7.40 (m, 2H), 6.66 - 6.62 (m, 1H), 6.25 (s, 1H), 3.90 (s, 3H), 3.56 - 3.43 (m, 2H), 2.87 (s, 3H), 2.43 - 2.27 (m, 2H). MS (ESI⁺): m/z = 475.2. | HPLC: Phenomenex C18 column, 3 µm, 75 x 30 mm; eluent: 35% to 65% (v/v) CH₃CN and H₂O with 0.05% NH₃) | 15 |
| | (*R*)-3-Hydroxy-3-(3-(4-(2-((4-methoxypyridin-2-yl)amino)pyrimidin-4-yl)thiazol-2-yl)phenyl)-1-methylpyrrolidin-2-one | | | |
| 163 | 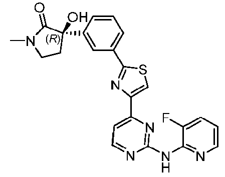 | ¹H NMR (400 MHz, DMSO-*d₆*) δ 9.91 (s, 1H), 8.59 (d, *J* = 4.8 Hz, 1H), 8.30 - 8.19 (m, 2H), 8.10 (s, 1H), 7.97 - 7.91 (m, 1H), 7.81 - 7.72 (m, 1H), 7.58 (d, *J* = 4.8 Hz, 1H), 7.55 - 7.49 (m, 1H), 7.47 - 7.43 (m, 1H), 7.35 - 7.28 (m, 1H), 6.24 (s, 1H), 3.52 - 3.39 (m, 2H), 2.87 (s, 3H), 2.32 - 2.24 (m, 2H). MS (ESI⁺): m/z = 463.2. | 1) RP HPLC: Phenomenex C18 column, 3 µm, 75 x 30 mm; eluent: 28% to 58% (v/v) CH₃CN and H₂O with 0.05% NH₃ + 10 mM NH₄HCO₃) | 10 |
| | (*R*)-3-(3-(4-(2-((3-Fluoropyridin-2-yl)amino)pyrimidin-4-yl)thiazol-2-yl)phenyl)-3-hydroxy-1-methylpyrrolidin-2-one | | | |
| | | | 2) SFC: DAICEL CHIRALCEL OD column, 10 µm, 250 x 30 mm; 50% (v/v) EtOH (containing 0.1% of 25% aq. NH₃)/CO₂) | |
| 164 | 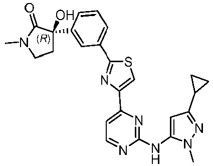 | ¹H NMR (400 MHz, DMSO-*d₆*) δ 9.48 (s, 1H), 8.60 (d, *J* = 5.2 Hz, 1H), 8.36 (s, 1H), 8.13 - 8.09 (m, 1H), 7.98 - 7.92 (m, 1H), 7.57 - 7.54 (m, *J* = 5.2 Hz, 1H), 7.54 - 7.49 (m, 1H), 7.47 - 7.43 (m, 1H), 6.26 (s, 1H), 6.02 (s, 1H), 3.61 (s, 3H), 3.52 - 3.43 (m, 2H), 2.87 (s, 3H), 2.41 - 2.26 (m, 2H), 1.87 - 1.78 (m, 1H), 0.86 - 0.80 (m, 2H), 0.67 - 0.62 (m, 2H). MS (ESI⁺): m/z = 488.2. | 1) HPLC: Column C10: 35% to 65% (v/v) CH₃CN and H₂O with 0.05% NH₃ + 10 mM NH₄HCO₃) | 4 |
| | (*R*)-3-(3-(4-(2-((3-Cyclopropyl-1-methyl-1H-pyrazol-5-yl)amino)pyrimidin-4-yl)thiazol-2-yl)phenyl)-3-hydroxy-1-methylpyrrolidin-2-one | | 2) SFC: DAICEL CHIRALCEL OD-H column, 5 µm, 250 x 30 mm; 45% (v/v) EtOH (containing 0.1% of 25% aq. NH₃)/CO₂) | |
| 165 | 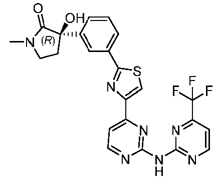 | ¹H NMR (400 MHz, DMSO-*d₆*) δ 10.97 (br s, 1H), 8.99 - 8.95 (m, 1H), 8.79 (d, *J* = 5.2 Hz, 1H), 8.44 (s, 1H), 8.15 - 8.10 (m, 1H), 7.99 - 7.93 (m, 1H), 7.79 (d, *J* = 5.2 Hz, 1H), 7.57 - 7.50 (m, 2H), 7.49 - 7.44 (m, 1H), 6.26 (s, 1H), 3.54 - 3.44 (m, 2H), 2.87 (s, 3H), 2.43 - 2.35 (m, 1H), 2.35 - 2.25 (m, 1H). MS (ESI⁺): m/z = 514.2. | HPLC: Column C10; eluent: 40% to 70% (v/v) CH₃CN and H₂O with 0.05% NH₃ + 10 mM NH₄HCO₃) | 10 |
| | (*R*)-3-Hydroxy-1-methyl-3-(3-(4-(2-((4-(trifluoromethyl)pyri midin-2-yl)amino)pyrimidin-4-yl)thiazol-2-yl)phenyl)pyrrolidin-2-one | | | |
| 166 | 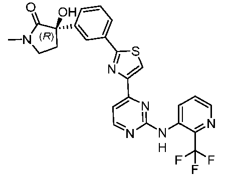 | ¹H NMR (400 MHz, DMSO-*d₆*) δ 9.22 (br s, 1H), 8.62 - 8.53 (m, 2H), 8.32 (s, 1H), 8.27 (d, *J* = 7.7 Hz, 1H), 8.12 - 8.09 (m, 1H), 7.96 - 7.91 (m, 1H), 7.80 - 7.75 (m, 1H), 7.58 (d, *J* = 5.1 Hz, 1H), 7.54 - 7.49 (m, 1H), 7.47 - 7.42 (m, 1H), 6.24 (s, 1H), 3.51 - 3.45 (m, 1H), 3.42 - 3.39 (m, 1H), 2.86 (s, 3H), 2.40 - 2.35 (m, 1H), 2.31 - 2.24 (m, 1H). MS (ESI⁺): m/z = 513.2. | HPLC: Column C10; eluent: 45% to 75% (v/v) CH₃CN and H₂O with 0.05% NH₃ + 10 mM NH₄HCO₃) | 3 |
| | (*R*)-3-Hydroxy-1-methyl-3-(3-(4-(2-((2-(trifluoromethyl)pyrid in-3-yl)amino)pyrimidin-4-yl)thiazol-2-yl)phenyl)pyrrolidin-2-one | | | |
| 167 | 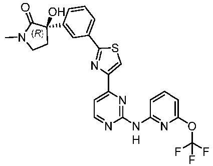 | ¹H NMR (400 MHz, DMSO-*d₆*) δ 10.18 (s, 1H), 8.75 (d, *J* = 5.0 Hz, 1H), 8.62 (s, 1H), 8.44 (d, *J* = 8.3 Hz, 1H), 8.14 (s, 1H), 8.06 - 8.00 (m, 1H), 7.97 (d, *J* = 7.8 Hz, 1H), 7.71 (d, *J* = 5.0 Hz, 1H), 7.56 - 7.50 (m, 1H), 7.49 - 7.44 (m, 1H), 6.88 (d, *J* = 7.8 Hz, 1H), 6.26 (s, 1H), 3.52 - 3.47 (m, 2H), 2.87 (s, 3H), 2.42 - 2.35 (m, 1H), 2.32 - 2.25 (m, 1H). MS (ESI⁺): m/z = 529.2. | HPLC: Column C10; eluent: 60% to 90% (v/v) CH₃CN and H₂O with 0.05% NH₃ + 10 mM NH₄HCO₃) | 12 |
| | (*R*)-3-Hydroxy-1-methyl-3-(3-(4-(2-((6-(trifluoromethoxy)pyr idin-2-yl)amino)pyrimidin-4-yl)thiazol-2-yl)phenyl)pyrrolidin-2-one | | | |
| 168 | 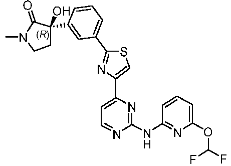 | ¹H NMR (400 MHz, DMSO-*d₆*) δ 9.97 (s, 1H), 8.78 - 8.71 (m, 1H), 8.62 (s, 1H), 8.26 (d, *J* = 8.0 Hz, 1H), 8.17 - 8.12 (m, 1H), 8.00 - 7.43 (m, 6H), 6.67 (d, *J* = 8.0 Hz, 1H), 6.26 (s, 1H), 3.54 - 3.42 (m, 2H), 2.87 (s, 3H), 2.44 - 2.26 (m, 2H). MS (ESI⁺): m/z = 511.2. | HPLC: Column C10; eluent: 60% to 90% (v/v) CH₃CN and H₂O with 0.05% NH₃ + 10 mM NH₄HCO₃) | 25 |
| | (*R*)-3-(3-(4-(2-((6-(Difluoromethoxy)pyr idin-2-yl)amino)pyrimidin-4-yl)thiazol-2-yl)phenyl)-3-hydroxy-1-methylpyrrolidin-2-one | | | |
| 169 | 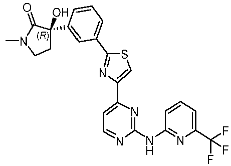 | ¹H NMR (400 MHz, DMSO-*d₆*) δ 10.47 (s, 1H), 8.79 - 8.68 (m, 2H), 8.62 (s, 1H), 8.18 - 8.07 (m, 2H), 8.01 - 7.93 (m, 1H), 7.74 - 7.69 (m, 1H), 7.58 - 7.42 (m, 3H), 6.28 (s, 1H), 3.54 - 3.46 (m, 1H), 3.43 - 3.37 (m, 1H), 2.87 (s, 3H), 2.43 - 2.34 (m, 1H), 2.34 - 2.25 (m, 1H). MS (ESI⁺): m/z = 513.2. | HPLC: Column C10; eluent: 50% to 80% (v/v) CH₃CN and H₂O with 0.075% TFA) | 23 |
| | (*R*)-3-Hydroxy-1-methyl-3-(3-(4-(2-((6-(trifluoromethyl)pyrid in-2-yl)amino)pyrimidin-4-yl)thiazol-2-yl)phenyl)pyrrolidin-2-one | | | |

The compounds shown in Table 3E were prepared in a manner analogous to Example 48 using the appropriate amine in place of 6-methylpyridin-2-amine. Purification conditions are noted in the table.

**Table 3E:**

| # | Structure | Analytical data | Purification conditions | Yield (%) |
|---|---|---|---|---|
| | Name | | | |
| 170 | 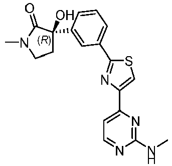 | ¹H NMR (400 MHz, DMSO-*d₆*) δ 8.40 (s, 1H), 8.38 - 8.32 (m, 2H), 8.10 - 8.03 (m, 2H), 7.88 - 7.81 (m, 1H), 7.55 - 7.46 (m, 2H), 6.46 (s, 1H), 3.37 - 3.35 (m, 2H), 2.78 (s, 3H), 2.44 - 2.41 (m, 1H), 2.21 - 2.13 (m, 1H), 1.60 (s, 9H). MS (ESI⁺): m/z = 382.1. | HPLC: Phenomenex Gemini-NX C18 column, 3 µm, 75 x 30 mm (eluent: 25-55% (v/v) CH₃CN/H₂O with 0.05% NH₃H₂O +10 mM NH₄HCO₃ | 61 |
| | (*R*)-3-Hydroxy-1-methyl-3-(3-(4-(2-(methylamino)pyrimi din-4-yl)thiazol-2-yl)phenyl)pyrrolidin-2-one | | | |
| 171 | 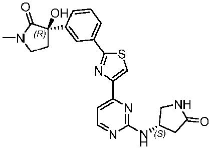 | ¹H NMR (400 MHz, DMSO-*d₆*) δ 8.54 - 8.37 (m, 2H), 8.12 - 8.07 (m, 1H), 7.96 - 7.89 (m, 1H), 7.68 - 7.61 (m, 2H), 7.54 - 7.48 (m, 1H), 7.47 - 7.41 (m, 1H), 7.35 (d, J = 4.8 Hz, 1H), 6.23 (s, 1H), 4.70 - 4.53 (m, 1H), 3.53 - 3.44 (m, 1H), 3.42 - 3.36 (m, 2H), 3.22 - 3.15 (m, 1H), 2.87 (s, 3H), 2.62 - 2.53 (m, 1H), 2.42 - 2.33 (m, 1H), 2.32 - 2.24 (m, 2H). MS (ESI⁺): m/z = 451.1. | HPLC: Column C10; 20-50% (v/v) CH₃CN/H₂O with 0.05% NH₃H₂O + 10 mM NH₄HCO₃ | 21 |
| | (*R*)-3-Hydroxy-1-methyl-3-(3-(4-(2-(((S)-5-oxopyrrolidin-3-yl)amino)pyrimidin-4-yl)thiazol-2-yl)phenyl)pyrrolidin-2-one | | | |
| 172 | 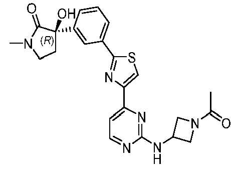 | ¹H NMR (400 MHz, DMSO-*d₆*) δ 8.51 - 8.30 (m, 2H), 8.07 (s, 1H), 7.96 - 7.92 (m, 1H), 7.92 - 7.89 (m, 1H), 7.51 - 7.45 (m, 1H), 7.44 - 7.38 (m, 1H), 7.35 (d, *J* = 5.1 Hz, 1H), 6.21 (s, 1H), 4.66 - 4.57 (m, 1H), 4.49 - 4.38 (m, 1H), 4.20 - 4.08 (m, 1H), 4.04 - 3.97 (m, 1H), 3.85 - 3.77 (m, 1H), 3.47 - 3.42 (m, 1H), 3.39 - 3.36 (m, 1H), 2.84 (s, 3H), 2.39 - 2.31 (m, 1H), 2.29 - 2.21 (m, 1H), 1.75 (s, 3H). MS (ESI⁺): m/z = 465.1. | HPLC: Phenomenex Gemini-NX C18 column, 3 µm, 75 x 30 mm; 22-52% (v/v) CH₃CN/H₂O with 0.05% NH₃H₂O +10 mM NH₄HCO₃ | 26 |
| | (*R*)-3-(3-(4-(2-((1-Acetylazetidin-3-yl)amino)pyrimidin-4-yl)thiazol-2-yl)phenyl)-3-hydroxy-1-methylpyrrolidin-2-one | | | |
| 173 | 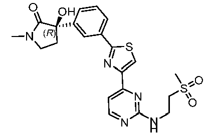 | ¹H NMR (400 MHz, DMSO-*d₆*) δ 8.39 - 8.55 (m, 2H), 8.07 - 8.14 (m, 1H), 7.94 (d, *J* = 7.60 Hz, 1H), 7.49 - 7.54 (m, 1H), 7.42 - 7.48 (m, 2H), 7.37 (d, *J* = 4.80 Hz, 1H), 6.25 (s, 1H), 3.73 - 3.83 (m, 2H), 3.38 - 3.53 (m, 4H), 3.06 (s, 3H), 2.87 (s, 3H), 2.23 - 2.43 (m, 2H). MS (ESI⁺): m/z = 474.1. | HPLC: Xtimate C18 column, 5 µm, 150 x 40 mm; 23-53% (v/v) CH₃CN/H₂O with 0.05% NH₃H₂O | 75 |
| | (*R*)-3-Hydroxy-1-methyl-3-(3-(4-(2-((2-(methylsulfonyl)ethyl )amino)pyrimidin-4-yl)thiazol-2-yl)phenyl)pyrrolidin-2-one | | | |
| 174 | 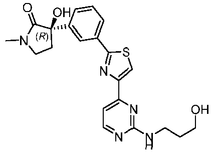 | ¹H NMR (400 MHz, DMSO-*d₆*) δ 8.42 - 8.38 (m, 1H), 8.36 - 8.32 (m, 1H), 8.09 (s, 1H), 7.92 - 7.87 (m, 1H), 7.52 - 7.43 (m, 2H), 7.28 - 7.24 (m, 1H), 6.87 - 6.81 (m, 1H), 5.92 (br s, 1H), 4.23 (br s, 1H), 3.58 - 3.32 (m, 6H), 2.86 (s, 3H), 2.44 - 2.26 (m, 2H), 1.81 - 1.71 (m, 2H). MS (ESI⁺): m/z = 426.1. | HPLC: Column C12; 20-50% ACN (v/v)/H₂O (0.05% NH₃H₂O + 10 mM NH₄HCO₃) | 54 |
| | (*R*)-3-Hydroxy-3-(3-(4-(2-((3-hydroxypropyl)amino )pyrimidin-4-yl)thiazol-2-yl)phenyl)-1-methylpyrrolidin-2-one | | | |
| 175 | 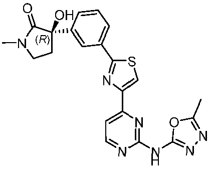 | ¹H NMR (400 MHz, DMSO-*d₆*) δ 11.18 (br s, 1H), 8.72 (d, *J* = 5.0 Hz, 1H), 8.48 (s, 1H), 8.12 (s, 1H), 7.99 - 7.91 (m, 1H), 7.73 (d, *J* = 5.0 Hz, 1H), 7.55 - 7.49 (m, 1H), 7.48 - 7.44 (m, 1H), 6.25 (s, 1H), 3.53 - 3.45 (m, 1H), 3.43 - 3.39 (m, 1H), 2.87 (s, 3H), 2.52 - 2.51 (m, 3H), 2.41 - 2.36 (m, 1H), 2.31 - 2.26 (m, 1H). MS (ESI⁺): m/z = 450.2. | HPLC:Phenomenex Gemini-NX C18 column, 3 µm, 75 x 30mm; 23-53% (v/v) ACN/H₂O with 0.05% NH₃•H₂O + 10 mM NH₄HCO₃ | 6 |
| | (*R*)-3-Hydroxy-1-methyl-3-(3-(4-(2-((5-methyl-1,3,4-oxadiazol-2-yl)amino)pyrimidin-4-yl)thiazol-2-yl)phenyl)pyrrolidin-2-one | | | |
| 176 | 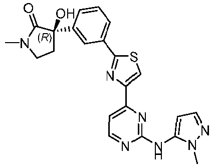 | ¹H NMR (400 MHz, DMSO-*d₆*) δ 9.58 (s, 1H), 8.62 (d, *J* = 5.0 Hz, 1H), 8.38 (s, 1H), 8.16 - 8.07 (m, 1H), 7.95 (d, J = 7.8 Hz, 1H), 7.57 (d, *J* = 5.0 Hz, 1H), 7.55 - 7.49 (m, 1H), 7.48 - 7.43 (m, 1H), 7.37 (d, *J* = 1.8 Hz, 1H), 6.37 - 6.30 (m, 1H), 6.25 (d, *J* = 1.0 Hz, 1H), 3.73 (s, 3H), 3.52 - 3.46 (m, 1H), 3.43 - 3.38 (m, 1H), 2.87 (s, 3H), 2.43 - 2.36 (m, 1H), 2.32 - 2.25 (m, 1H). MS (ESI⁺): m/z = 448.2. | SFC: DAICEL CHIRALCEL OJ column, 10 µm, 250 mm x 30 mm; 65% (v/v) EtOH (containing 0.1% of 25% aq. NH₃)/CO₂ | 5 |
| | (*R*)-3-Hydroxy-1-methyl-3-(3-(4-(2-((1-methyl-1H-pyrazol-5-yl)amino)pyrimidin-4-yl)thiazol-2-yl)phenyl)pyrrolidin-2-one | | | |
| 177 | 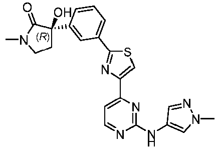 | ¹H NMR (400 MHz, DMSO-*d₆*) δ 9.60 (s, 1H), 8.73 - 8.49 (m, 2H), 8.13 (s, 1H), 8.10 - 7.93 (m, 2H), 7.57 - 7.49 (m, 2H), 7.48 - 7.41 (m, 2H), 6.25 (s, 1H), 3.86 (s, 3H), 3.51 - 3.47 (m, 1H), 3.42 - 3.40 (m, 1H), 2.87 (s, 3H), 2.41 - 2.36 (m, 1H), 2.31 - 2.25 (m, 1H). MS (ESI⁺): m/z = 448.2. | HPLC: Phenomenex Gemini-NX C18 column, 3 µm, 75 x 30 mm; 22-52% (v/v) ACN/H₂O with 0.05% NH₃•H₂O + 10 mM NH₄HCO₃ | 17 |
| | (*R*)-3-Hydroxy-1-methyl-3-(3-(4-(2-((1-methyl-1H-pyrazol-4-yl)amino)pyrimidin-4-yl)thiazol-2-yl)phenyl)pyrrolidin-2-one | | | |
| 178 | 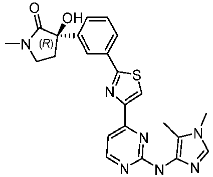 | ¹H NMR (400 MHz, DMSO-*d₆*) δ 8.69 (s, 1H), 8.47 - 8.41 (m, 1H), 8.31 - 8.24 (m, 1H), 8.10 (s, 1H), 7.96 - 7.90 (m, 1H), 7.55 - 7.48 (m, 1H), 7.47 - 7.42 (m, 1H), 7.41 - 7.36 (m, 2H), 6.24 (s, 1H), 3.57 (s, 3H), 3.52 - 3.45 (m, 1H), 3.42 - 3.38 (m, 1H), 2.87 (s, 3H), 2.43 - 2.24 (m, 2H), 2.05 (s, 3H). MS (ESI⁺): m/z = 462.2. | HPLC: Column C12; 20-50% ACN (v/v) / water with 0.05% NH₃•H₂O + 10 mM NH₄HCO₃ | 6 |
| | (*R*)-3-(3-(4-(2-((1,5-Dimethyl-1H-imidazol-4-yl)amino)pyrimidin-4-yl)thiazol-2-yl)phenyl)-3-hydroxy-1-methylpyrrolidin-2-one | | | |
| 179 | 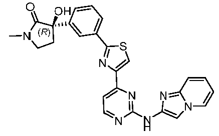 | ¹H NMR (400 MHz, DMSO-*d₆*) δ 9.96 (d, *J* = 6.8 Hz, 1H), 8.92 (d, *J* = 5.1 Hz, 1H), 8.82 (s, 1H), 8.17 (s, 1H), 7.99 (d, *J* = 7.7 Hz, 1H), 7.74 (d, *J* = 5.1 Hz, 1H), 7.57 - 7.51 (m, 1H), 7.49 - 7.45 (m, 1H), 7.44 - 7.38 (m, 2H), 7.11 - 7.04 (m, 1H), 6.91 (s, 2H), 6.28 (s, 1H), 3.53 - 3.47 (m, 1H), 3.43 - 3.40 (m, 1H), 2.88 (s, 3H), 2.43 - 2.37 (m, 1H), 2.33 - 2.26 (m, 1H). MS (ESI⁺): m/z = 484.2. | HPLC: Column C10; (45-75% (v/v) ACN/H₂O 0.05% NH₃•H₂O + 10 mM NH₄HCO₃ | 70 |
| | (*R*)-3-Hydroxy-3-(3-(4-(2-(imidazo[1,2-a]pyridin-2-ylamino)pyrimidin-4-yl)thiazol-2-yl)phenyl)-1-methylpyrrolidin-2-one | | | |
| 180 | 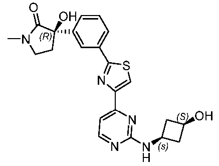 | ¹H NMR (400 MHz, DMSO-*d₆*) δ 8.49 - 8.31 (m, 2H), 8.11 - 8.07 (m, 1H), 7.94 - 7.90 (m, 1H), 7.54 - 7.48 (m, 1H), 7.48 - 7.41 (m, 2H), 7.28 (d, J = 5.2 Hz, 1H), 6.27 (s, 1H), 5.15 (d, J = 6.0 Hz, 1H), 4.01 - 3.78 (m, 2H), 3.43 - 3.34 (m, 2H), 2.86 (s, 3H), 2.69 - 2.57 (m, 2H), 2.43 - 2.22 (m, 2H), 1.93 - 1.78 (m, 2H). MS (ESI⁺): m/z = 438.2. | HPLC: Xtimate C18, 5 µm, 150 x 40 mm; 35-60% ACN/water with 0.05% NH₃•H₂O + 10 mM NH₄HCO₃ | 62 |
| | (*R*)-3-Hydroxy-3-(3-(4-(2-(((1s,3S)-3-hydroxycyclobutyl)a mino)pyrimidin-4-yl)thiazol-2-yl)phenyl)-1-methylpyrrolidin-2-one | | | |
| 181 | 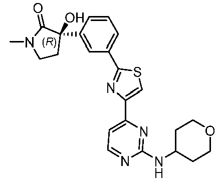 | ¹H NMR (400 MHz, DMSO-*d₆*) δ 8.48 - 8.33 (m, 2H), 8.11 - 8.07 (m, 1H), 7.92 (d, *J* = 8.0 Hz, 1H), 7.54 - 7.48 (m, 1H), 7.46 - 7.40 (m, 1H), 7.31 - 7.27 (m, 1H), 7.24 (d, *J* = 8.0 Hz, 1H), 6.26 (s, 1H), 4.08 - 3.95 (m, 1H), 3.93 - 3.84 (m, 2H), 3.52 - 3.48 (m, 2H), 3.40 - 3.34 (m, 2H), 2.86 (s, 3H), 2.42 - 2.23 (m, 2H), 1.94 - 1.81 (m, 2H), 1.63 - 1.46 (m, 2H). MS (ESI⁺): m/z = 452.2. | HPLC: Xtimate C18, 5 µm, 150 x 40 mm; 39-64% ACN/water with 0.05% NH₃•H₂O + 10 mM NH₄HCO₃ | 57 |
| | (*R*)-3-Hydroxy-1-methyl-3-(3-(4-(2-((tetrahydro-2H-pyran-4-yl)amino)pyrimidin-4-yl)thiazol-2-yl)phenyl)pyrrolidin-2-one | | | |
| 182 | 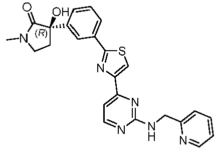 | ¹H NMR (400 MHz, DMSO-*d₆*) δ 8.55 - 8.50 (m, 1H), 8.48 - 8.42 (m, 1H), 8.36 (br s, 1H), 8.09 (s, 1H), 7.96 - 7.90 (m, 1H), 7.86 - 7.79 (m, 1H), 7.78 - 7.71 (m, 1H), 7.54 - 7.48 (m, 1H), 7.47 - 7.42 (m, 1H), 7.41 - 7.31 (m, 2H), 7.28 - 7.22 (m, 1H), 6.28 (s, 1H), 4.69 (d, J = 4.8 Hz, 2H), 3.42 - 3.35 (m, 2H), 2.87 (s, 3H), 2.42 - 2.24 (m, 2H). MS (ESI⁺): m/z = 459.2. | HPLC: Column C12; 17-57% ACN/water with 0.05% NH₃•H₂O + 10 mM NH₄HCO₃ | 55 |
| | (*R*)-3-Hydroxy-1-methyl-3-(3-(4-(2-((pyridin-2-ylmethyl)amino)pyri midin-4-yl)thiazol-2-yl)phenyl)pyrrolidin-2-one | | | |
| 183 | 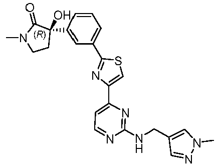 | ¹H NMR (400 MHz, DMSO-*d₆*) δ 8.53 - 8.34 (m, 2H), 8.12 - 8.06 (m, 1H), 7.96 - 7.89 (m, 1H), 7.61 (s, 1H), 7.55 - 7.48 (m, 2H), 7.46 - 7.37 (m, 2H), 7.33 - 7.28 (m, 1H), 6.26 (s, 1H), 4.38 (d, *J* = 6.0 Hz, 2H), 3.76 (s, 3H), 3.39 - 3.35 (m, 2H), 2.86 (s, 3H), 2.42 - 2.23 (m, 2H). MS (ESI⁺): m/z = 462.2. | HPLC: Column C12; 15-55% ACN/water with 0.05% NH₃•H₂O + 10 mM NH₄HCO₃ | 71 |
| | (*R*)-3-Hydroxy-1-methyl-3-(3-(4-(2-(((1-methyl-1H-pyrazol-4-yl)methyl)amino)pyri midin-4-yl)thiazol-2-yl)phenyl)pyrrolidin-2-one | | | |
| 184 | 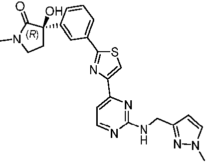 | ¹H NMR (400 MHz, DMSO-*d₆*) δ 8.48 - 8.38 (m, 2H), 8.10 (s, 1H), 7.97 - 7.90 (m, 1H), 7.57 - 7.42 (m, 4H), 7.34 - 7.28 (m, 1H), 6.25 (s, 1H), 6.17 (br s, 1H), 4.52 (d, *J* = 5.6 Hz, 2H), 3.78 (s, 3H), 3.53 - 3.43 (m, 2H), 2.87 (s, 3H), 2.43 - 2.24 (m, 2H). MS (ESI⁺): m/z = 462.2. | HPLC: Column C12; 15-55% ACN/water with 0.05% NH₃•H₂O + 10 mM NH₄HCO₃ | 46 |
| | (*R*)-3-Hydroxy-1-methyl-3-(3-(4-(2-(((1-methyl-1H-pyrazol-3-yl)methyl)amino)pyri midin-4-yl)thiazol-2-yl)phenyl)pyrrolidin-2-one | | | |
| 185 | 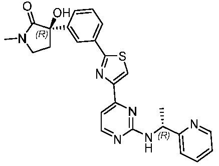 | ¹H NMR (400 MHz, DMSO-*d₆*) δ 8.55 - 8.50 (m, 1H), 8.47 - 8.19 (m, 2H), 8.08 (s, 1H), 7.95 - 7.87 (m, 1H), 7.77 - 7.67 (m, 2H), 7.54 - 7.40 (m, 3H), 7.32 - 7.26 (m, 1H), 7.26 - 7.19 (m, 1H), 6.24 (s, 1H), 5.23 (br s, 1H), 3.51 - 3.40 (m, 2H), 2.86 (s, 3H), 2.41 - 2.23 (m, 2H), 1.51 (d, *J* = 7.2 Hz, 3H). MS (ESI⁺): m/z = 473.2. | HPLC: Column C12; 22-62% ACN/water with 0.05% NH₃•H₂O + 10 mM NH₄HCO₃ | 33 |
| | (*R*)-3-Hydroxy-1-methyl-3-(3-(4-(2-(((*R*)-1-(pyridin-2-yl)ethyl)amino)pyrimi din-4-yl)thiazol-2-yl)phenyl)pyrrolidin-2-one | | | |
| 186 | 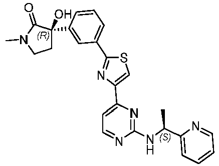 | ¹H NMR (400 MHz, DMSO-*d₆*) δ 8.57 - 8.49 (m, 1H), 8.46 - 8.21 (m, 2H), 8.08 (s, 1H), 7.96 - 7.87 (m, 1H), 7.77 - 7.65 (m, 2H), 7.55 - 7.39 (m, 3H), 7.32 - 7.26 (m, 1H), 7.25 - 7.19 (m, 1H), 6.24 (s, 1H), 5.30 - 5.15 (m, 1H), 3.52 - 3.44 (m, 2H), 2.86 (s, 3H), 2.41 - 2.23 (m, 2H), 1.51 (d, *J* = 7.2 Hz, 3H). MS (ESI⁺): m/z = 473.2. | HPLC: Column C12; 22-62% ACN/water with 0.05% NH₃•H₂O + 10 mM NH₄HCO₃ | 62 |
| | (*R*)-3-Hydroxy-1-methyl-3-(3-(4-(2-(((*S*)-1-(pyridin-2-yl)ethyl)amino)pyrimi din-4-yl)thiazol-2-yl)phenyl)pyrrolidin-2-one | | | |
| 187 | 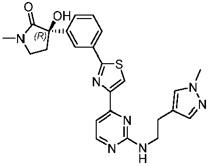 | ¹H NMR (400 MHz, DMSO-*d₆*) δ 8.49 - 8.33 (m, 2H), 8.10 (s, 1H), 7.96 - 7.89 (m, 1H), 7.59 - 7.41 (m, 3H), 7.35 - 7.25 (m, 3H), 6.24 (s, 1H), 3.77 (s, 3H), 3.54 - 3.45 (m, 3H), 3.42 - 3.39 (m, 1H), 2.87 (s, 3H), 2.76 - 2.65 (m, 2H), 2.43 - 2.24 (m, 2H). MS (ESI⁺): m/z = 476.2. | HPLC: Column C12; 17-57% ACN/water with 0.05% NH₃•H₂O + 10 mM NH₄HCO₃ | 56 |
| | (*R*)-3-Hydroxy-1-methyl-3-(3-(4-(2-((2-(1-methyl-1H-pyrazol-4-yl)ethyl)amino)pyrimi din-4-yl)thiazol-2-yl)phenyl)pyrrolidin-2-one | | | |
| 188 | 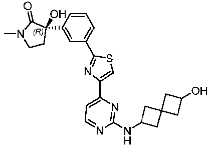 | ¹H NMR (400 MHz, DMSO-*d₆*) δ 8.48 - 8.28 (m, 2H), 8.11 - 8.06 (m, 1H), 7.96 - 7.88 (m, 1H), 7.55 - 7.39 (m, 3H), 7.30 - 7.23 (m, 1H), 6.26 (s, 1H), 4.97 (d, *J* = 6.4 Hz, 1H), 4.39 - 4.23 (m, 1H), 4.03 - 3.91 (m, 1H), 3.41 - 3.34 (m, 2H), 2.86 (s, 3H), 2.44 - 2.32 (m, 3H), 2.32 - 2.21 (m, 2H), 2.20 - 2.12 (m, 1H), 2.05 - 1.94 (m, 2H), 1.92 - 1.78 (m, 2H). MS (ESI⁺): m/z = 478.2. | HPLC: Column C12; 17-57% ACN/water with 0.05% NH₃•H₂O + 10 mM NH₄HCO₃ | 57 |
| | (*R*)-3-Hydroxy-3-(3-(4-(2-((6-hydroxyspiro[3.3]hep tan-2-yl)amino)pyrimidin-4-yl)thiazol-2-yl)phenyl)-1-methylpyrrolidin-2-one | | | |
| 189 | 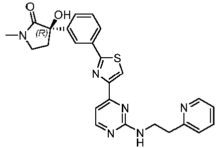 | ¹H NMR (400 MHz, DMSO-*d₆*) δ 8.54 - 8.49 (m, 1H), 8.48 - 8.34 (m, 2H), 8.10 (s, 1H), 7.97 - 7.89 (m, 1H), 7.75 - 7.67 (m, 1H), 7.55 - 7.47 (m, 1H), 7.47 - 7.41 (m, 1H), 7.37 - 7.27 (m, 3H), 7.25 - 7.18 (m, 1H), 6.24 (s, 1H), 3.78 - 3.65 (m, 2H), 3.53 - 3.44 (m, 1H), 3.43 - 3.39 (m, 1H), 3.06 (t, *J* = 7.2 Hz, 2H), 2.87 (s, 3H), 2.43 - 2.22 (m, 2H). MS (ESI⁺): m/z = 473.2. | HPLC: Column C12; 19-59% ACN/water with 0.05% NH₃•H₂O + 10 mM NH₄HCO₃ | 64 |
| | (*R*)-3-Hydroxy-1-methyl-3-(3-(4-(2-((2-(pyridin-2-yl)ethyl)amino)pyrimi din-4-yl)thiazol-2-yl)phenyl)pyrrolidin-2-one | | | |
| 190 | 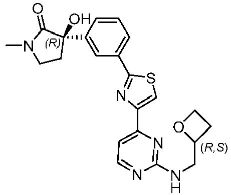 | ¹H NMR (400 MHz, DMSO-*d₆*) δ 8.48 - 8.35 (m, 2H), 8.10 (s, 1H), 7.96 - 7.88 (m, 1H), 7.54 - 7.48 (m, 1H), 7.46 - 7.42 (m, 1H), 7.36 - 7.29 (m, 2H), 6.24 (s, 1H), 4.95 - 4.81 (m, 1H), 4.58 - 4.40 (m, 2H), 3.74 - 3.55 (m, 2H), 3.52 - 3.44 (m, 1H), 3.43 - 3.39 (m, 1H), 2.87 (s, 3H), 2.71 - 2.57 (m, 1H), 2.48 - 2.42 (m, 1H), 2.42 - 2.24 (m, 2H). MS (ESI⁺): m/z = 438.2. | HPLC: Column C12; 15-55% ACN/water with 0.05% NH₃•H₂O + 10 mM NH₄HCO₃ | 31 |
| | (*R*)-3-hydroxy-1-methyl-3-(3-(4-(2-((((*R, S*)-oxetan-2-yl)methyl)amino)pyri midin-4-yl)thiazol-2-yl)phenyl)pyrrolidin-2-one | | | |
| 191 | 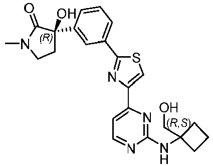 | ¹H NMR (400 MHz, DMSO-*d₆*) δ 8.42 (d, *J* = 4.9 Hz, 1H), 8.34 (s, 1H), 8.13 - 8.07 (m, 1H), 7.93 (d, *J* = 7.6 Hz, 1H), 7.56 - 7.48 (m, 1H), 7.47 - 7.40 (m, 1H), 7.29 (d, *J* = 5.0 Hz, 1H), 7.12 (s, 1H), 6.27 (s, 1H), 4.96 - 4.88 (m, 1H), 3.77 - 3.65 (m, 4H), 2.87 (s, 3H), 2.42 - 2.27 (m, 4H), 2.23 - 2.15 (m, 2H), 1.93 - 1.70 (m, 2H). MS (ESI⁺): m/z = 452.2. | HPLC: Xtimate C18 column, 5 µm, 150 x 25 mm; 15-55% (v/v) MeCN/H₂O with 0.05% NH₃•H₂O + 10 mM NH₄HCO₃ | 46 |
| | (*R*)-3-Hydroxy-3-(3-*(4-(2-(((R, S)-1-*(hydroxymethyl)cycl obutyl)amino)pyrimid in-4-yl)thiazol-2-yl)phenyl)-1-methylpyrrolidin-2-one | | | |
| 192 | 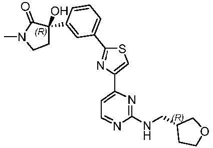 | ¹H NMR (400 MHz, DMSO-*d₆*) δ 8.52 - 8.31 (m, 2H), 8.13 - 8.07 (m, 1H), 7.96 - 7.90 (m, 1H), 7.55 - 7.48 (m, 1H), 7.47 - 7.39 (m, 2H), 7.30 (d, *J* = 5.1 Hz, 1H), 6.24 (s, 1H), 3.79 - 3.71 (m, 2H), 3.66 - 3.48 (m, 6H), 2.87 (s, 3H), 2.61 - 2.55 (m, 1H), 2.42 - 2.24 (m, 2H), 2.04 - 1.91 (m, 1H), 1.69 - 1.58 (m, 1H). MS (ESI⁺): m/z = 452.2. | HPLC: Xtimate C18 column, 5 µm, 150 x 25 mm; 17-57% (v/v) MeCN/H₂O with 0.05% NH₃•H₂O + 10 mM NH₄HCO₃ | 70 |
| | (R)-3-Hydroxy-1-methyl-3-(3-(4-(2-*((((R)-*tetrahydrofuran-3-yl)methyl)amino)pyri midin-4-yl)thiazol-2-yl)phenyl)pyrrolidin-2-one | | | |
| 193 | 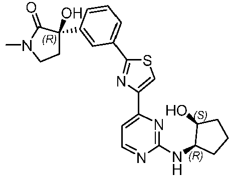 | ¹H NMR (400 MHz, DMSO-*d₆*) δ 8.51 - 8.37 (m, 2H), 8.14 - 8.06 (m, 1H), 7.97 - 7.89 (m, 1H), 7.56 - 7.48 (m, 1H), 7.47 - 7.41 (m, 1H), 7.31 (d, *J* = 4.9 Hz, 1H), 6.55 - 6.43 (m, 1H), 6.25 (s, 1H), 4.82 - 4.73 (m, 1H), 4.21 - 4.01 (m, 2H), 3.52 - 3.44 (m, 2H), 2.87 (s, 3H), 2.42 - 2.24 (m, 2H), 2.03 - 1.92 (m, 1H), 1.86 - 1.71 (m, 2H), 1.66 - 1.49 (m, 3H). MS (ESI⁺): m/z = 452.2. | HPLC: Xtimate C18 column, 5 µm, 150 x 25 mm; 18-58% (v/v) water (ACN/NH₃H₂O) | 68 |
| | and | | | |
| | 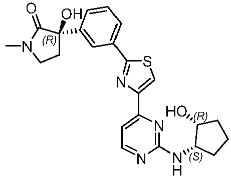 (racemic) | | | |
| | (*R*)-3-Hydroxy-3-(3-(4-(2-((cis-2-hydroxycyclopentyl) amino)pyrimidin-4-yl)thiazol-2-yl)phenyl)-1-methylpyrrolidin-2-one | | | |
| 194 | 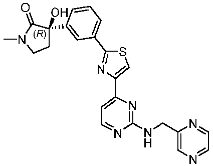 | ¹H NMR (400 MHz, DMSO-*d₆*) δ 8.77 - 8.63 (m, 1H), 8.62 - 8.56 (m, 1H), 8.54 - 8.48 (m, 1H), 8.48 - 8.42 (m, 1H), 8.41 - 8.31 (m, 1H), 8.09 (s, 1H), 7.97 - 7.87 (m, 2H), 7.55 - 7.47 (m, 1H), 7.47 - 7.41 (m, 1H), 7.39 - 7.31 (m, 1H), 6.24 (s, 1H), 4.77 - 4.67 (m, 2H), 3.50 - 3.48 (m, 2H), 2.86 (s, 3H), 2.41 - 2.24 (m, 2H). MS (ESI⁺): m/z = 460.2. | HPLC: Xtimate C18 column, 5 µm, 150 x 25 mm; 12-52% (v/v) water (ACN/NH₃H₂O) | 59 |
| | (*R*)-3-Hydroxy-1-methyl-3-(3-(4-(2-((pyrazin-2-ylmethyl)amino)pyri midin-4-yl)thiazol-2-yl)phenyl)pyrrolidin-2-one | | | |
| 195 | 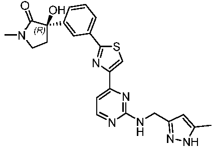 | ¹H NMR (400 MHz, DMSO-*d₆*) δ 8.52 - 8.40 (m, 2H), 8.10 (s, 1H), 7.96 - 7.90 (m, 1H), 7.55 - 7.47 (m, 2H), 7.47 - 7.42 (m, 1H), 7.34 - 7.30 (m, 1H), 6.24 (s, 1H), 5.93 (s, 1H), 4.53 - 4.45 (m, 2H), 3.53 - 3.45 (m, 2H), 2.86 (s, 3H), 2.42 - 2.23 (m, 2H), 2.15 (s, 3H). MS (ESI⁺): m/z = 462.2. | basic conditions, column C2 | 26 |
| | (*R*)-3-Hydroxy-1-methyl-3-(3-(4-(2-(((5-methyl-1H-pyrazol-3-yl)methyl)amino)pyri midin-4-yl)thiazol-2-yl)phenyl)pyrrolidin-2-one | | | |
| 196 | 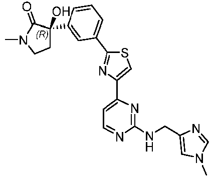 | ¹H NMR (400 MHz, DMSO-*d₆*) δ 8.53 - 8.36 (m, 2H), 8.14 - 8.06 (m, 1H), 7.93 (d, *J* = 7.6 Hz, 1H), 7.55 - 7.41 (m, 3H), 7.40 - 7.28 (m, 2H), 6.98 (br s, 1H), 6.26 (s, 1H), 4.47 - 4.40 (m, 2H), 3.59 - 3.57 (m, 5H), 2.87 (s, 3H), 2.43 - 2.24 (m, 2H). MS (ESI⁺): m/z = 462.2. | HPLC: Xtimate C18 column, 5 µm, 150 x 25 mm; 34-59% (v/v) MeCN/H₂O with 0.05% NH₃•H₂O + 10 mM NH₄HCO₃ | 27 |
| | (*R*)-3-Hydroxy-1-methyl-3-(3-(4-(2-(((1-methyl-1H-imidazol-4-yl)methyl)amino)pyri midin-4-yl)thiazol-2-yl)phenyl)pyrrolidin-2-one | | | |
| 197 | 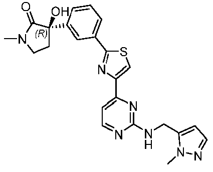 | ¹H NMR (400 MHz, DMSO-*d₆*) δ 8.54 - 8.38 (m, 2H), 8.12 - 8.07 (m, 1H), 7.96 - 7.90 (m, 1H), 7.82 - 7.76 (m, 1H), 7.55 - 7.49 (m, 1H), 7.47 - 7.41 (m, 1H), 7.35 (d, *J* = 5.0 Hz, 1H), 7.30 - 7.25 (m, 1H), 6.28 - 6.19 (m, 2H), 4.65 - 4.60 (m, 2H), 3.88 - 3.83 (m, 5H), 2.86 (s, 3H), 2.43 - 2.23 (m, 2H). MS (ESI⁺): m/z = 462.2. | HPLC: Xtimate C18 column, 5 µm, 150 x 25 mm; 15-55% (v/v) MeCN/H₂O with 0.05% NH₃•H₂O + 10 mM NH₄HCO₃ | 59 |
| | (*R*)-3-Hydroxy-1-methyl-3-(3-(4-(2-(((1-methyl-1H-pyrazol-5-yl)methyl)amino)pyri midin-4-yl)thiazol-2-yl)phenyl)pyrrolidin-2-one | | | |
| 198 | 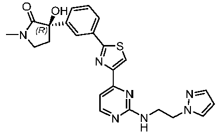 | ¹H NMR (400 MHz, DMSO-*d₆*) δ 8.61 - 8.29 (m, 2H), 8.13 - 8.07 (m, 1H), 7.97 - 7.91 (m, 1H), 7.79 - 7.71 (m, 1H), 7.56 - 7.49 (m, 1H), 7.48 - 7.41 (m, 2H), 7.37 - 7.28 (m, 2H), 6.28 (s, 1H), 6.25 - 6.20 (m, 1H), 4.41 - 4.31 (m, 2H), 3.81 - 3.70 (m, 2H), 3.40 - 3.36 (m, 2H), 2.87 (s, 3H), 2.42 - 2.33 (m, 1H), 2.33 - 2.25 (m, 1H). MS (ESI⁺): m/z = 462.2. | HPLC: Xtimate C18 column, 5 µm, 150 x 25 mm; 38-63% (v/v)MeCN/H₂O with 0.05% NH₃•H₂O + 10 mM NH₄HCO₃ | 44 |
| | (*R*)-3-(3-(4-(2-((2-(1H-Pyrazol-1-yl)ethyl)amino)pyrimi din-4-yl)thiazol-2-yl)phenyl)-3-hydroxy-1-methylpyrrolidin-2-one | | | |
| 199 | 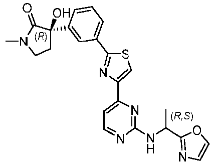 | ¹H NMR (400 MHz, DMSO-*d₆*) δ 8.46 (d, J = 4.9 Hz, 1H), 8.34 (s, 1H), 8.11 - 8.07 (m, 1H), 8.01 - 7.99 (m, 1H), 7.94 - 7.90 (m, 1H), 7.85 (d, J = 7.7 Hz, 1H), 7.54 - 7.48 (m, 1H), 7.46 - 7.42 (m, 1H), 7.34 (d, J = 4.9 Hz, 1H), 7.13 (s, 1H), 6.25 (s, 1H), 5.39 - 5.30 (m, 1H), 3.52 - 3.46 (m, 2H), 2.86 (s, 3H), 2.38 - 2.27 (m, 2H), 1.58 (d, J = 7.0 Hz, 3H). MS (ESI⁺): m/z = 463.2. | HPLC: Xtimate C18 column, 5 µm, 150 x 25 mm; 18-58% (v/v) water (ACN/NH₃H₂O) | 16 |
| | (*R*)-3-hydroxy-1-methyl-3-(3-(4-(2-(((*R, S*)-1-(oxazol-2-yl)ethyl)amino)pyrimi din-4-yl)thiazol-2-yl)phenyl)pyrrolidin-2-one | | | |
| 200 | 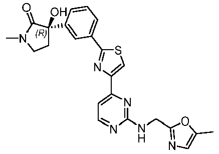 | ¹H NMR (400 MHz, DMSO-*d₆*) δ 8.49 - 8.44 (m, 1H), 8.41 - 8.37 (m, 1H), 8.12 - 8.07 (m, 1H), 7.93 (d, *J* = 7.5 Hz, 1H), 7.83 - 7.76 (m, 1H), 7.55 - 7.48 (m, 1H), 7.47 - 7.42 (m, 1H), 7.39 - 7.33 (m, 1H), 6.73 (s, 1H), 6.28 - 6.23 (m, 1H), 4.64 - 4.59 (m, 2H), 3.86 - 3.83 (m, 2H), 2.88 - 2.85 (m, 3H), 2.42 - 2.23 (m, 5H). MS (ESI⁺): m/z = 463.2. | HPLC (Xtimate C18 column, 5 µm, 150 x 25 mm; 15-55% (v/v) MeCN/H₂O with 0.05% NH₃•H₂O + 10 mM NH₄HCO₃) | 29 |
| | (*R*)-3-Hydroxy-1-methyl-3-(3-(4-(2-(((5-methyloxazol-2-yl)methyl)amino)pyri midin-4-yl)thiazol-2-yl)phenyl)pyrrolidin-2-one | | | |
| 201 | 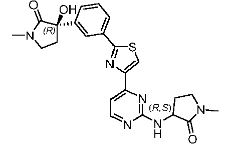 | ¹H NMR (400 MHz, DMSO-*d₆*) δ 8.45 (d, *J* = 5.0 Hz, 1H), 8.38 (s, 1H), 8.12 - 8.08 (m, 1H), 7.95 - 7.91 (m, 1H), 7.55 - 7.49 (m, 1H), 7.46 - 7.41 (m, 2H), 7.34 (d, *J* = 5.0 Hz, 1H), 6.26 (s, 1H), 4.75 - 4.66 (m, 1H), 3.37 - 3.33 (m, 4H), 2.87 (s, 3H), 2.80 (s, 3H), 2.42 - 2.34 (m, 2H), 2.32 - 2.25 (m, 1H), 2.09 - 1.97 (m, 1H). MS (ESI⁺): m/z = 465.2. | HPLC (Xtimate C18 column, 5 µm, 150 x 25 mm; 15-55% (v/v) MeCN/H₂O with 0.05% NH₃•H₂O + 10 mM NH₄HCO₃) | 42 |
| | (*R*)-3-hydroxy-1-methyl-3-(3-(4-(2-(((*R,S*)-1-methyl-2-oxopyrrolidin-3-yl)amino)pyrimidin-4-yl)thiazol-2-yl)phenyl)pyrrolidin-2-one | | | |
| 202 | 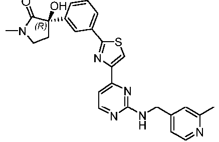 | ¹H NMR (400 MHz, DMSO-*d₆*) δ 8.48 - 8.42 (m, 1H), 8.40 - 8.32 (m, 2H), 8.12 - 8.07 (m, 1H), 7.96 - 7.86 (m, 2H), 7.55 - 7.48 (m, 1H), 7.47 - 7.42 (m, 1H), 7.34 (d, *J* = 5.0 Hz, 1H), 7.28 - 7.13 (m, 2H), 6.26 (s, 1H), 4.61 - 4.53 (m, 2H), 3.39 - 3.37 (m, 2H), 2.86 (s, 3H), 2.43 (s, 3H), 2.40 - 2.34 (m, 1H), 2.32 - 2.25 (m, 1H). MS (ESI⁺): m/z = 473.2. | HPLC (Xtimate C18 column, 5 µm, 150 x 25 mm; 39-64% (v/v) MeCN/H₂O with 0.05% NH₃•H₂O + 10 mM NH₄HCO₃) | 46 |
| | (*R*)-3-Hydroxy-1-methyl-3-(3-(4-(2-(((2-methylpyridin-4-yl)methyl)amino)pyri midin-4-yl)thiazol-2-yl)phenyl)pyrrolidin-2-one | | | |
| 203 | 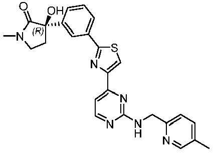 | ¹H NMR (400 MHz, DMSO-*d₆*) δ 8.48 - 8.32 (m, 3H), 8.11 - 8.07 (m, 1H), 7.92 (d, J = 7.6 Hz, 1H), 7.81 - 7.74 (m, 1H), 7.58 - 7.48 (m, 2H), 7.46 - 7.42 (m, 1H), 7.35 - 7.23 (m, 2H), 6.26 (s, 1H), 4.68 - 4.59 (m, 2H), 3.54 - 3.53 (m, 2H), 2.86 (s, 3H), 2.41 - 2.35 (m, 1H), 2.32 - 2.25 (m, 4H). MS (ESI⁺): m/z = 473.2. | HPLC (Xtimate C18 column, 5 µm, 150 x 25 mm; 42-67% (v/v)MeCN/H2O with 0.05% NH₃•H₂O + 10 mM NH₄HCO₃) | 45 |
| | (*R*)-3-Hydroxy-1-methyl-3-(3-(4-(2-(((5-methylpyridin-2-yl)methyl)amino)pyri midin-4-yl)thiazol-2-yl)phenyl)pyrrolidin-2-one | | | |
| 204 | 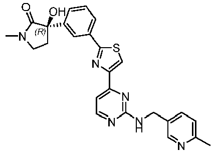 | ¹H NMR (400 MHz, DMSO-*d₆*) δ 8.58 - 8.35 (m, 3H), 8.10 (s, 1H), 7.93 (d, J = 7.6 Hz, 1H), 7.90 - 7.85 (m, 1H), 7.69 (br s, 1H), 7.54 - 7.48 (m, 1H), 7.46 - 7.42 (m, 1H), 7.32 (d, J = 5.0 Hz, 1H), 7.19 (d, J = 7.9 Hz, 1H), 6.25 (s, 1H), 4.63 - 4.40 (m, 2H), 3.53 - 3.45 (m, 1H), 3.42 - 3.39 (m, 1H), 2.86 (s, 3H), 2.41 (s, 3H), 2.39 - 2.33 (m, 1H), 2.32 - 2.23 (m, 1H). MS (ESI⁺): m/z = 473.2. | HPLC (Xtimate C18 column, 5 µm, 150 x 25 mm; 19-59% (v/v) MeCN/H₂O with 0.05% NH₃•H₂O + 10 mM NH₄HCO₃) | 57 |
| | (*R*)-3-Hydroxy-1-methyl-3-(3-(4-(2-(((6-methylpyridin-3-yl)methyl)amino)pyri midin-4-yl)thiazol-2-yl)phenyl)pyrrolidin-2-one | | | |
| 205 | 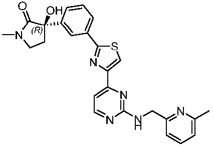 | ¹H NMR (400 MHz, DMSO-*d₆*) δ 8.48 - 8.41 (m, 1H), 8.37 (s, 1H), 8.09 (s, 1H), 7.99 - 7.88 (m, 1H), 7.85 - 7.77 (m, 1H), 7.68 - 7.58 (m, 1H), 7.53 - 7.48 (m, 1H), 7.46 - 7.40 (m, 1H), 7.33 (d, *J* = 4.9 Hz, 1H), 7.21 - 7.05 (m, 2H), 6.24 (s, 1H), 4.63 (s, 2H), 3.52 - 3.44 (m, 1H), 3.42 - 3.39 (m, 1H), 2.86 (s, 3H), 2.47 (s, 3H), 2.38 - 2.24 (m, 2H). MS (ESI⁺): m/z = 473.2. | HPLC (Xtimate C18 column, 5 µm, 150 x 25 mm; 21-61% (v/v) MeCN/H₂O with 0.05% NH₃•H₂O + 10 mM NH₄HCO₃) | 57 |
| | (*R*)-3-Hydroxy-1-methyl-3-(3-(4-(2-(((6-methylpyridin-2-yl)methyl)amino)pyri midin-4-yl)thiazol-2-yl)phenyl)pyrrolidin-2-one | | | |
| 206 | 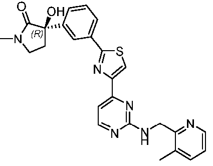 | ¹H NMR (400 MHz, DMSO-*d₆*) δ 8.50 - 8.45 (m, 2H), 8.38 (d, *J* = 4.5 Hz, 1H), 8.10 (s, 1H), 7.93 (d, *J* = 7.5 Hz, 1H), 7.61 (d, *J* = 7.5 Hz, 1H), 7.54 - 7.49 (m, 1H), 7.47 - 7.42 (m, 1H), 7.42 - 7.38 (m, 1H), 7.35 (d, *J* = 4.8 Hz, 1H), 7.28 - 7.20 (m, 1H), 6.24 (s, 1H), 4.68 (s, 2H), 3.53 - 3.45 (m, 1H), 3.43 - 3.39 (m, 1H), 2.87 (s, 3H), 2.40 - 2.33 (m, 4H), 2.32 - 2.24 (m, 1H). MS (ESI⁺): m/z = 473.2. | HPLC (Xtimate C18 column, 5 µm, 150 x 25 mm; 26-66% (v/v) MeCN/H₂O with 0.05% NH₃•H₂O + 10 mM NH₄HCO₃) | 53 |
| | (*R*)-3-Hydroxy-1-methyl-3-(3-(4-(2-(((3-methylpyridin-2-yl)methyl)amino)pyri midin-4-yl)thiazol-2-yl)phenyl)pyrrolidin-2-one | | | |
| 207 | 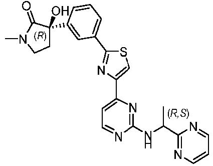 | ¹H NMR (400 MHz, DMSO-*d₆*) δ 8.77 (d, *J* = 4.9 Hz, 2H), 8.41 (br s, 1H), 8.07 (br s, 1H), 7.91 (d, *J* = 7.6 Hz, 1H), 7.71 - 7.54 (m, 1H), 7.53 - 7.47 (m, 1H), 7.46 - 7.40 (m, 1H), 7.38 - 7.32 (m, 1H), 7.31 - 7.24 (m, 1H), 6.25 (s, 1H), 5.28 - 5.15 (m, 1H), 3.52 - 3.43 (m, 2H), 2.86 (s, 3H), 2.40 - 2.34 (m, 1H), 2.31 - 2.23 (m, 1H), 1.55 (d, J = 7.2 Hz, 3H). MS (ESI⁺): m/z = 474.2. | HPLC (Xtimate C18 column, 5 µm, 150 x 25 mm; 14-54% (v/v) MeCN and water with 0.05% NH₃•H₂O + 10 mM NH₄HCO₃) | 48 |
| | (*R*)-3-hydroxy-1-methyl-3-(3-(4-(2-*(((R, S*)-1-(pyrimidin-2-yl)ethyl)amino)pyrimi din-4-yl)thiazol-2-yl)phenyl)pyrrolidin-2-one | | | |
| 208 | 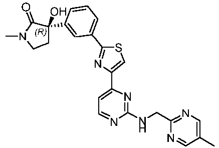 | ¹H NMR (400 MHz, DMSO-*d₆*) δ 8.60 (s, 2H), 8.49 - 8.20 (m, 2H), 8.08 (s, 1H), 7.97 - 7.86 (m, 1H), 7.61 - 7.55 (m, 1H), 7.54 - 7.47 (m, 1H), 7.46 - 7.40 (m, 1H), 7.31 (d, *J* = 5.0 Hz, 1H), 6.23 (s, 1H), 4.81 - 4.64 (m, 2H), 3.51 - 3.45 (m, 2H), 2.86 (s, 3H), 2.41 - 2.33 (m, 1H), 2.32 - 2.26 (m, 1H), 2.24 (s, 3H). MS (ESI⁺): m/z = 474.2. | HPLC (Xtimate C18 column, 5 µm, 150 x 25 mm; 14-54% (v/v) MeCN/H₂O with 0.05% NH₃•H₂O + 10 mM NH₄HCO₃) | 57 |
| | (*R*)-3-Hydroxy-1-methyl-3-(3-(4-(2-(((5-methylpyrimidin-2-yl)methyl)amino)pyri midin-4-yl)thiazol-2-yl)phenyl)pyrrolidin-2-one | | | |
| 209 | 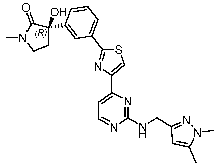 | ¹H NMR (400 MHz, DMSO-*d₆*) δ 8.48 - 8.37 (m, 2H), 8.12 - 8.06 (m, 1H), 7.97 - 7.89 (m, 1H), 7.55 - 7.47 (m, 1H), 7.47 - 7.41 (m, 2H), 7.30 (d, *J* = 5.0 Hz, 1H), 6.23 (s, 1H), 5.95 (s, 1H), 4.49 - 4.38 (m, 2H), 3.68 - 3.61 (m, 3H), 3.52 - 3.44 (m, 1H), 3.42 - 3.39 (m, 1H), 2.86 (s, 3H), 2.41 - 2.35 (m, 1H), 2.31 - 2.24 (m, 1H), 2.18 (s, 3H). MS (ESI⁺): m/z = 476.8. | HPLC (Xtimate C18 column, 5 µm, 150 x 25 mm; 18-58% (v/v) MeCN/H₂O with 0.05% NH₃•H₂O + 10 mM NH₄HCO₃) | 57 |
| | (*R*)-3-(3-(4-(2-(((1,5-Dimethyl-1*H-*pyrazol-3-yl)methyl)amino)pyri midin-4-yl)thiazol-2-yl)phenyl)-3-hydroxy-1-methylpyrrolidin-2-one | | | |
| 210 | 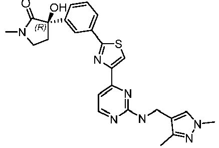 | ¹H NMR (400 MHz, DMSO-*d₆*) δ 8.57 - 8.33 (m, 2H), 8.13 - 8.07 (m, 1H), 7.98 - 7.89 (m, 1H), 7.56 - 7.49 (m, 2H), 7.48 - 7.40 (m, 2H), 7.31 (d, *J* = 5.0 Hz, 1H), 6.29 (s, 1H), 4.44 - 4.26 (m, 2H), 3.68 (s, 3H), 3.42 - 3.36 (m, 2H), 2.87 (s, 3H), 2.42 - 2.34 (m, 1H), 2.33 - 2.25 (m, 1H), 2.16 (s, 3H). MS (ESI⁺): m/z = 476.2. | HPLC (Xtimate C18 column, 5 µm, 150 x 25 mm; 17-57% (v/v) (ACN/water with 0.05% NH₃•H₂O + 10 mM NH₄HCO₃) | 5 |
| | (*R*)-3-(3-(4-(2-(((1,3-Dimethyl-1*H-*pyrazol-4-yl)methyl)amino)pyri midin-4-yl)thiazol-2-yl)phenyl)-3-hydroxy-1-methylpyrrolidin-2-one | | | |
| 211 | 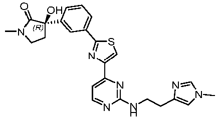 | ¹H NMR (400 MHz, DMSO-*d₆*) δ 8.50 - 8.33 (m, 2H), 8.12 - 8.07 (m, 1H), 7.93 (d, *J* = 7.7 Hz, 1H), 7.57 - 7.48 (m, 2H), 7.47 - 7.40 (m, 1H), 7.30 (d, *J* = 4.9 Hz, 1H), 7.26 - 7.18 (m, 1H), 6.96 (s, 1H), 6.24 (s, 1H), 3.61 (s, 3H), 3.59 - 3.53 (m, 2H), 3.52 - 3.46 (m, 2H), 2.87 (s, 3H), 2.81 - 2.73 (m, 2H), 2.43 - 2.34 (m, 1H), 2.33 - 2.25 (m, 1H). MS (ESI⁺): m/z = 476.2. | HPLC (Xtimate C18 column, 5 µm, 150 x 40 mm; 36-61% (v/v) MeCN/H₂O with 0.05% NH₃•H₂O + 10 mM NH₄HCO₃) | 50 |
| | (*R*)-3-Hydroxy-1-methyl-3-(3-(4-(2-((2-(1-methyl-1*H-*imidazol-4-yl)ethyl)amino)pyrimi din-4-yl)thiazol-2-yl)phenyl)pyrrolidin-2-one | | | |
| 212 | 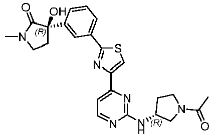 | ¹H NMR (400 MHz, DMSO-*d₆*) δ 8.57 - 8.32 (m, 2H), 8.10 (s, 1H), 7.93 (d, *J* = 7.7 Hz, 1H), 7.61 - 7.48 (m, 2H), 7.47 - 7.42 (m, 1H), 7.37 - 7.31 (m, 1H), 6.24 (s, 1H), 4.61 - 4.39 (m, 1H), 3.93 - 3.57 (m, 2H), 3.55 - 3.48 (m, 2H), 3.32 - 3.27 (m, 2H), 2.87 (s, 3H), 2.41 - 2.34 (m, 1H), 2.31 - 2.23 (m, 1H), 2.19 - 2.08 (m, 1H), 2.05 - 1.97 (m, 1H), 1.97 - 1.91 (m, 3H). MS (ESI⁺): m/z = 479.2. | SFC (DAICEL CHIRALCEL OJ column, 10 µm, 250 mm x 30 mm; 55% (v/v) CO₂/EtOH (0.1% NH₃H₂O)) | 50 |
| | (*R*)-3-(3-(4-(2-(((*R*)-1-Acetylpyrrolidin-3-yl)amino)pyrimidin-4-yl)thiazol-2-yl)phenyl)-3-hydroxy-1-methylpyrrolidin-2-one | | | |
| 213 | 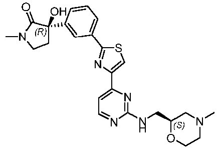 | ¹H NMR (400 MHz, DMSO-*d₆*) δ 8.50 - 8.32 (m, 2H), 8.12 - 8.05 (m, 1H), 7.93 (d, *J* = 7.6 Hz, 1H), 7.55 - 7.48 (m, 1H), 7.47 - 7.41 (m, 1H), 7.31 (d, *J* = 4.9 Hz, 1H), 7.27 - 7.19 (m, 1H), 6.25 (s, 1H), 3.82 - 3.74 (m, 1H), 3.71 - 3.62 (m, 1H), 3.54 - 3.44 (m, 3H), 3.42 - 3.39 (m, 2H), 2.87 (s, 3H), 2.81 - 2.74 (m, 1H), 2.60 - 2.55 (m, 1H), 2.41 - 2.35 (m, 1H), 2.33 - 2.24 (m, 1H), 2.16 (s, 3H), 2.02 - 1.92 (m, 1H), 1.80 - 1.68 (m, 1H). MS (ESI⁺): m/z = 481.2. | HPLC (Xtimate C18 column, 5 µm, 150 x 25 mm; 14-54% (v/v) MeCN/H₂O with 0.05% NH₃•H₂O + 10 mM NH₄HCO₃) | 53 |
| | (*R*)-3-Hydroxy-1-methyl-3-(3-(4-(2-((((*S*)-4-methylmorpholin-2-yl)methyl)amino)pyri midin-4-yl)thiazol-2-yl)phenyl)pyrrolidin-2-one | | | |
| 214 | 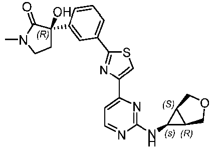 | ¹H NMR (400 MHz, DMSO-*d₆*) δ 8.56 - 8.27 (m, 2H), 8.13 - 8.06 (m, 1H), 7.93 (d, *J* = 7.7 Hz, 1H), 7.56 - 7.48 (m, 2H), 7.46 - 7.41 (m, 1H), 7.35 (d, *J* = 4.9 Hz, 1H), 6.26 (s, 1H), 3.95 (br s, 2H), 3.71 - 3.64 (m, 2H), 3.52 - 3.45 (m, 1H), 3.42 - 3.40 (m, 1H), 2.87 (s, 3H), 2.55 - 2.53 (m, 1H), 2.42 - 2.24 (m, 2H), 1.85 (s, 2H). MS (ESI⁺): m/z = 450.2. | HPLC (Xtimate C18 column, 5 µm, 150 x 25 mm; 14% to 54% (v/v) MeCN/H₂O with 0.05% NH₃•H₂O + 10 mM NH₄HCO₃) | 48 |
| | (*R*)-3-(3-(4-(2-((1*R*,5*S*,6*s*)-3-Oxabicyclo[3.1.0]hex an-6-ylamino)pyrimidin-4-yl)thiazol-2-yl)phenyl)-3-hydroxy-1-methylpyrrolidin-2-one | | | |
| 215 | 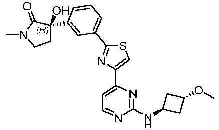 | ¹H NMR (400 MHz, DMSO-*d₆*) δ 8.51 - 8.31 (m, 2H), 8.13 - 8.07 (m, 1H), 7.96 - 7.89 (m, 1H), 7.63 (d, *J* = 6.6 Hz, 1H), 7.55 - 7.48 (m, 1H), 7.47 - 7.41 (m, 1H), 7.30 (d, *J* = 5.0 Hz, 1H), 6.25 (s, 1H), 4.45 (br s, 1H), 4.06 - 3.93 (m, 1H), 3.52 - 3.43 (m, 2H), 3.17 (s, 3H), 2.87 (s, 3H), 2.37 - 2.19 (m, 6H). MS (ESI⁺): m/z = 452.2. | HPLC (Xtimate C18 column, 5 µm, 150 x 25 mm; 42-62% (v/v) MeCN/water with 0.05% NH₃•H₂O + 10 mM NH₄HCO₃) | 25 |
| | (*R*)-3-Hydroxy-3-(3-(4-(2-((*trans*-3-methoxycyclobutyl)a mino)pyrimidin-4-yl)thiazol-2-yl)phenyl)-1-methylpyrrolidin-2-one | | | |
| 216 | 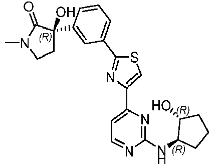 | ¹H NMR (400 MHz, DMSO-*d₆*) δ 8.64 - 8.31 (m, 2H), 8.14 - 8.07 (m, 1H), 7.94 (d, *J* = 7.6 Hz, 1H), 7.57 - 7.49 (m, 1H), 7.48 - 7.42 (m, 1H), 7.29 (d, *J* = 5.0 Hz, 1H), 7.25 - 7.15 (m, 1H), 6.27 (br s, 1H), 5.03 (br s, 1H), 4.07 - 3.92 (m, 2H), 3.42 - 3.36 (m, 2H), 2.86 (s, 3H), 2.45 - 2.36 (m, 1H), 2.33 - 2.23 (m, 1H), 2.17 - 2.04 (m, 1H), 1.95 - 1.83 (m, 1H), 1.74 - 1.63 (m, 2H), 1.58 - 1.42 (m, 2H). MS (ESI⁺): m/z = 452.2. | HPLC (Xtimate C18 column, 5 µm, 150 x 25 mm; 25-55% (v/v) MeCN/H₂O with 0.05% NH₃•H₂O + 10 mM NH₄HCO₃) | 52 |
| | (*R*)-3-Hydroxy-3-(3-(4-(2-(((1*R*,2*R*)-2-hydroxycyclopentyl) amino)pyrimidin-4-yl)thiazol-2-yl)phenyl)-1-methylpyrrolidin-2-one | | | |
| 217 | 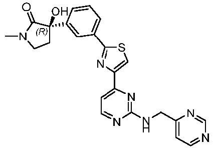 | ¹H NMR (400 MHz, DMSO-*d₆*) δ 9.11 (s, 1H), 8.70 (d, *J* = 5.2 Hz, 1H), 8.53 - 8.20 (m, 2H), 8.08 (s, 1H), 7.99 - 7.87 (m, 2H), 7.54 - 7.42 (m, 3H), 7.36 (d, *J* = 5.0 Hz, 1H), 6.24 (s, 1H), 4.74 - 4.55 (m, 2H), 3.52 - 3.44 (m, 1H), 3.42 - 3.39 (m, 1H), 2.86 (s, 3H), 2.41 - 2.34 (m, 1H), 2.32 - 2.24 (m, 1H). MS (ESI⁺): m/z = 460.1. | HPLC (Xtimate C18 column, 5 µm, 150 x 25 mm; 11-51% (v/v) MeCN/H₂O with 0.05% NH₃•H₂O + 10 mM NH₄HCO₃) | 41 |
| | (*R*)-3-Hydroxy-1-methyl-3-(3-(4-(2-((pyrimidin-4-ylmethyl)amino)pyri midin-4-yl)thiazol-2-yl)phenyl)pyrrolidin-2-one | | | |
| 218 | 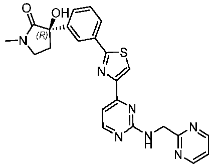 | ¹H NMR (400 MHz, DMSO-*d₆*) δ 8.76 (d, *J* = 4.9 Hz, 2H), 8.56 - 8.13 (m, 2H), 8.08 (s, 1H), 7.97 - 7.84 (m, 1H), 7.70 - 7.57 (m, 1H), 7.54 - 7.47 (m, 1H), 7.46 - 7.41 (m, 1H), 7.39 - 7.35 (m, 1H), 7.31 (d, *J* = 4.9 Hz, 1H), 6.22 (s, 1H), 4.87 - 4.68 (m, 2H), 3.53 - 3.44 (m, 1H), 3.42 - 3.38 (m, 1H), 2.86 (s, 3H), 2.39 - 2.35 (m, 1H), 2.31 - 2.26 (m, 1H). MS (ESI⁺): m/z = 460.2. | HPLC (Xtimate C18 column, 5 µm, 150 x 25 mm; (11-51% (v/v) ACN/H₂O with 0.05% NH₃•H₂O + 10 mM NH₄HCO₃) | 65 |
| | (*R*)-3-Hydroxy-1-methyl-3-(3-(4-(2-((pyrimidin-2-ylmethyl)amino)pyri midin-4-yl)thiazol-2-yl)phenyl)pyrrolidin-2-one | | | |
| 219 | 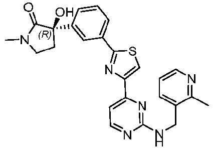 | ¹H NMR (400 MHz, DMSO-*d₆*) δ 8.45 (d, *J* = 5.0 Hz, 1H), 8.39 (s, 1H), 8.32 - 8.27 (m, 1H), 8.12 - 8.07 (m, 1H), 7.94 - 7.90 (m, 1H), 7.88 - 7.82 (m, 1H), 7.67 (br s, 1H), 7.54 - 7.47 (m, 1H), 7.46 - 7.41 (m, 1H), 7.33 (d, *J* = 4.9 Hz, 1H), 7.20 - 7.11 (m, 1H), 6.24 (s, 1H), 4.58 (br s, 2H), 3.51 - 3.45 (m, 1H), 3.41 - 3.39 (m, 1H), 2.86 (s, 3H), 2.56 (br s, 3H), 2.41 - 2.35 (m, 1H), 2.32 - 2.24 (m, 1H). MS (ESI⁺): m/z = 473.2. | HPLC (Xtimate C18 column, 5 µm, 150 x 25 mm; (16-56% (v/v) ACN/H₂O with 0.05% NH₃•H₂O + 10 mM NH₄HCO₃) | 64 |
| | (*R*)-3-Hydroxy-1-methyl-3-(3-(4-(2-(((2-methylpyridin-3-yl)methyl)amino)pyrimidin-4-yl)thiazol-2-yl)phenyl)pyrrolidin-2-one | | | |
| 220 | 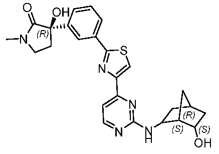 | ¹H NMR (400 MHz, DMSO-*d₆*) δ 8.53 - 8.23 (m, 2H), 8.12 - 8.07 (m, 1H), 7.93 (d, *J* = 7.7 Hz, 1H), 7.54 - 7.48 (m, 1H), 7.46 - 7.42 (m, 1H), 7.29 (d, *J* = 4.9 Hz, 1H), 7.25 (d, *J* = 6.8 Hz, 1H), 6.24 (s, 1H), 4.31 (d, *J* = 4.2 Hz, 1H), 4.19 - 4.07 (m, 1H), 4.04 - 3.95 (m, 1H), 3.52 - 3.40 (m, 2H), 2.87 (s, 3H), 2.43 - 2.34 (m, 2H), 2.31 - 2.24 (m, 1H), 2.20 - 2.14 (m, 1H), 1.93 - 1.79 (m, 2H), 1.64 - 1.54 (m, 1H), 1.29 - 1.20 (m, 2H), 1.09 - 0.97 (m, 1H). MS (ESI⁺): m/z = 478.2. | HPLC (Xtimate C18 column, 5 µm, 150 x 25 mm; (17-57% (v/v) ACN/H₂O with 0.05% NH₃•H₂O + 10 mM NH₄HCO₃) | 51 |
| | (*R*)-3-Hydroxy-3-(3-(4-(2-(((1*S*,4*R*,6*S*)-6-hydroxybicyclo[2.2.1 ]heptan-2-yl)amino)pyrimidin-4-yl)thiazol-2-yl)phenyl)-1-methylpyrrolidin-2-one | | | |
| 221 | 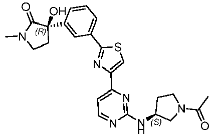 | ¹H NMR (400 MHz, DMSO-*d₆*) δ 8.69 - 8.23 (m, 2H), 8.10 (s, 1H), 7.96 - 7.91 (m, 1H), 7.63 - 7.55 (m, 1H), 7.55 - 7.49 (m, 1H), 7.47 - 7.41 (m, 1H), 7.38 - 7.33 (m, 1H), 6.27 (s, 1H), 4.63 - 4.31 (m, 1H), 3.68 - 3.58 (m, 4H), 3.32 - 3.29 (m, 2H), 2.87 (s, 3H), 2.41 - 2.36 (m, 1H), 2.33 - 2.22 (m, 2H), 2.00 - 1.92 (m, 4H). MS (ESI⁺): m/z = 479.2. | HPLC (Xtimate C18 column, 5 µm, 150 x 40 mm; (34-59% (v/v) ACN/H₂O with 0.05% NH₃•H₂O + 10 mM NH₄HCO₃) | 28 |
| | (*R*)-3-(3-(4-(2-(((*S*)-1-Acetylpyrrolidin-3-yl)amino)pyrimidin-4-yl)thiazol-2-yl)phenyl)-3-hydroxy-1-methylpyrrolidin-2-one | | | |
| 222 | 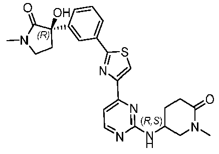 | ¹H NMR (400 MHz, DMSO-*d₆*) δ 8.48 (d, *J* = 5.0 Hz, 2H), 8.14 - 8.07 (m, 1H), 7.93 (d, *J* = 8.0 Hz, 1H), 7.53 - 7.48 (m, 1H), 7.48 - 7.42 (m, 2H), 7.34 (d, *J =* 5.0 Hz, 1H), 6.23 (s, 1H), 4.33 (br s, 1H), 3.61 - 3.53 (m, 1H), 3.52 - 3.45 (m, 1H), 3.42 - 3.39 (m, 1H), 3.29 - 3.27 (m, 1H), 2.87 (s, 3H), 2.82 (s, 3H), 2.41 - 2.35 (m, 3H), 2.31 - 2.24 (m, 1H), 2.05 - 1.97 (m, 1H), 1.96 - 1.85 (m, 1H). MS (ESI⁺): m/z = 479.2. | HPLC (Xtimate C18 column, 5 µm, 150 x 25 mm; (11-51% (v/v) MeCN/H₂O with 0.05% NH₃•H₂O + 10 mM NH₄HCO₃) | 62 |
| | (*R*,*S*)-5-((4-(2-(3-((*R*)-3-hydroxy-1-methyl-2-oxopyrrolidin-3-yl)phenyl)thiazol-4-yl)pyrimidin-2-yl)amino)-1-methylpiperidin-2-one (R) | | | |
| 223 | 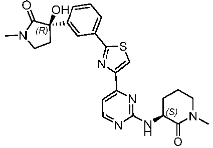 | ¹H NMR (400 MHz, DMSO-*d₆*) δ 8.44 (d, *J* = 5.0 Hz, 1H), 8.39 (s, 1H), 8.10 (s, 1H), 7.93 (d, *J* = 7.7 Hz, 1H), 7.54 - 7.48 (m, 1H), 7.46 - 7.42 (m, 1H), 7.32 (d, *J* = 5.0 Hz, 1H), 7.27 - 7.17 (m, 1H), 6.23 (s, 1H), 4.57 - 4.38 (m, 1H), 3.53 - 3.44 (m, 1H), 3.42 - 3.37 (m, 2H), 3.32 - 3.31 (m, 1H), 2.87 (s, 3H), 2.85 (s, 3H), 2.41 - 2.35 (m, 1H), 2.31 - 2.25 (m, 1H), 2.18 - 2.08 (m, 1H), 1.97 - 1.87 (m, 3H). MS (ESI⁺): m/z = 479.2. | HPLC (Xtimate C18 column, 5 µm, 150 x 25 mm; (15-55 % (v/v) MeCN/H₂O with 0.05% NH₃•H₂O + 10 mM NH₄HCO₃) | 65 |
| | (*S*)-3-((4-(2-(3-((*R*)-3-Hydroxy-1-methyl-2-oxopyrrolidin-3-yl)phenyl)thiazol-4-yl)pyrimidin-2-yl)amino)-1-methylpiperidin-2-one | | | |
| 224 | 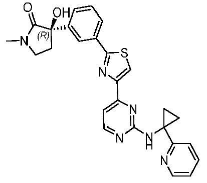 | ¹H NMR (400 MHz, DMSO-*d₆*) δ 8.67 - 8.25 (m, 3H), 8.19 (s, 1H), 8.08 (br s, 1H), 7.94 - 7.89 (m, 1H), 7.64 - 7.58 (m, 1H), 7.53 - 7.47 (m, 1H), 7.46 - 7.40 (m, 1H), 7.37 - 7.32 (m, 2H), 7.15 - 7.05 (m, 1H), 6.24 (s, 1H), 3.50 - 3.46 (m, 2H), 2.86 (s, 3H), 2.38 - 2.35 (m, 1H), 2.31 - 2.27 (m, 1H), 1.64 - 1.55 (m, 2H), 1.27 - 1.24 (m, 2H). MS (ESI⁺): m/z = 485.2. | HPLC (Xtimate C18 column, 5 µm, 150 x 40 mm; (42-67 % (v/v) MeCN/H₂O with 0.05% NH₃•H₂O + 10 mM NH₄HCO₃) | 15 |
| | (*R*)-3-Hydroxy-1-methyl-3-(3-(4-(2-((1-(pyridin-2-yl)cyclopropyl)amino )pyrimidin-4-yl)thiazol-2-yl)phenyl)pyrrolidin-2-one | | | |
| 225 | 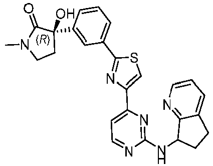 | ¹H NMR (400 MHz, DMSO-*d₆*) δ 8.46 (d, *J* = 5.0 Hz, 1H), 8.42 - 8.25 (m, 2H), 8.14 - 8.03 (m, 1H), 8.02 - 7.87 (m, 1H), 7.68 (d, J = 7.5 Hz, 1H), 7.57 - 7.41 (m, 3H), 7.34 (d, *J* = 4.9 Hz, 1H), 7.27 7.14 (m, 1H), 6.24 (s, 1H), 5.73 - 5.58 (m, 1H), 3.52 - 3.45 (m, 2H), 3.03 - 2.96 (m, 1H), 2.92 - 2.83 (m, 4H), 2.64 - 2.57 (m, 1H), 2.42 - 2.37 (m, 1H), 2.31 - 2.24 (m, 1H), 2.09 - 1.92 (m, 1H). MS (ESI⁺): m/z = 485.2. | HPLC (Xtimate C18 column, 5 µm, 150 x 25 mm; (24-64% (v/v) MeCN/H₂O with 0.05% NH₃•H₂O + 10 mM NH₄HCO₃)) | 49 |
| | (*R*)-3-(3-(4-(2-((6,7-Dihydro-5*H-*cyclopenta[b]pyridin-7-yl)amino)pyrimidin-4-yl)thiazol-2-yl)phenyl)-3-hydroxy-1-methylpyrrolidin-2-one | | | |
| 226 | 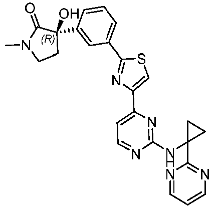 | ¹H NMR (400 MHz, DMSO-*d₆*) δ 8.60 (d, *J* = 4.9 Hz, 2H), 8.37 (d, *J* = 5.0 Hz, 1H), 8.15 - 8.00 (m, 2H), 7.90 - 7.85 (m, 1H), 7.69 (s, 1H), 7.50 - 7.44 (m, 2H), 7.30 (d, *J* = 5.0 Hz, 1H), 7.21 - 7.13 (m, 1H), 5.93 (s, 1H), 3.52 - 3.43 (m, 1H), 3.40 - 3.32 (m, 1H), 2.86 (s, 3H), 2.38 - 2.34 (m, 1H), 2.31 - 2.25 (m, 1H), 1.70 - 1.65 (m, 2H), 1.39 - 1.36 (m, 2H). MS (ESI⁺): m/z = 486.2. | HPLC (Xtimate C18 column, 5 µm, 150 x 25 mm; (17-57% (v/v) MeCN/H₂O with 0.05% NH₃•H₂O + 10 mM NH₄HCO₃)) | 26 |
| | (*R*)-3-Hydroxy-1-methyl-3-(3-(4-(2-((1-(pyrimidin-2-yl)cyclopropyl)amino )pyrimidin-4-yl)thiazol-2-yl)phenyl)pyrrolidin-2-one | | | |
| 227 | 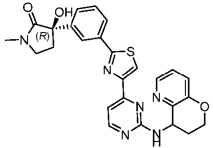 | ¹H NMR (400 MHz, DMSO-*d₆*) δ 8.74 - 8.28 (m, 2H), 8.16 - 8.06 (m, 2H), 7.96 - 7.90 (m, 1H), 7.64 (d, *J =*7.7 Hz, 1H), 7.55 - 7.48 (m, 1H), 7.47 - 7.41 (m, 1H), 7.36 (d, *J* = 5.0 Hz, 1H), 7.26 - 7.20 (m, 2H), 6.24 (s, 1H), 5.43 - 5.27 (m, 1H), 4.39 - 4.29 (m, 2H), 3.52 - 3.45 (m, 2H), 2.87 (s, 3H), 2.39 - 2.35 (m, 1H), 2.32 - 2.24 (m, 2H), 2.20 - 2.12 (m, 1H). MS (ESI⁺): m/z = 501.2. | HPLC (Xtimate C18 column, 5 µm, 150 x 25 mm; (24-64% (v/v) MeCN/H₂O with 0.05% NH₃•H₂O + 10 mM NH₄HCO₃)) | 57 |
| | (*R*)-3-(3-(4-(2-((3,4-Dihydro-2*H-*pyrano[3,2-*b*]pyridin-4-yl)amino)pyrimidin-4-yl)thiazol-2-yl)phenyl)-3-hydroxy-1-methylpyrrolidin-2-one | | | |
| 228 | 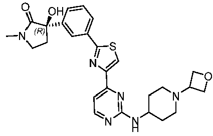 | ¹H NMR (400 MHz, DMSO-*d₆*) δ 8.50 - 8.25 (m, 2H), 8.13 - 8.03 (m, 1H), 7.97 - 7.88 (m, 1H), 7.54 - 7.47 (m, 1H), 7.46 - 7.41 (m, 1H), 7.27 (d, *J* = 5.0 Hz, 1H), 7.21 (d, *J* = 7.7 Hz, 1H), 6.23 (s, 1H), 4.57 - 4.50 (m, 2H), 4.46 - 4.40 (m, 2H), 3.82 (br s, 1H), 3.53 - 3.40 (m, 3H), 2.86 (s, 3H), 2.73 - 2.68 (m, 2H), 2.41 - 2.37 (m, 1H), 2.30 - 2.24 (m, 1H), 1.97 - 1.83 (m, 4H), 1.61 - 1.50 (m, 2H). MS (ESI⁺): m/z = 507.2. | HPLC (Xtimate C18 column, 5 µm, 150 x 25 mm; (14-54 % (v/v) MECN/H2O WITH 0.05% NH₃•H₂O + 10 mM NH₄HCO₃) | 14 |
| | (*R*)-3-Hydroxy-1-methyl-3-(3-(4-(2-((1-(oxetan-3-yl)piperidin-4-yl)amino)pyrimidin-4-yl)thiazol-2-yl)phenyl)pyrrolidin-2-one | | | |
| 229 | 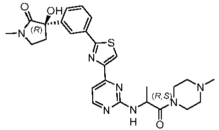 | ¹H NMR (400 MHz, DMSO-*d₆*) δ 8.50 - 8.37 (m, 2H), 8.13 - 8.07 (m, 1H), 7.98 - 7.89 (m, 1H), 7.55 - 7.48 (m, 1H), 7.46 - 7.42 (m, 1H), 7.36 - 7.30 (m, 1H), 7.29 - 7.08 (m, 1H), 6.24 (s, 1H), 4.96 (br s, 1H), 3.79 - 3.54 (m, 2H), 3.52 - 3.42 (m, 4H), 2.86 (s, 3H), 2.42 - 2.34 (m, 3H), 2.30 - 2.21 (m, 3H), 2.17 (s, 3H), 1.32 (br d, *J* = 6.8 Hz, 3H). MS (ESI⁺): m/z = 522.2. | HPLC (Xtimate C18 column, 5 µm, 150 x 25 mm; (12-52% (v/v) MeCN/H₂O with 0.05% NH₃•H₂O + 10 mM NH₄HCO₃)) | 44 |
| | (*R*)-3-hydroxy-1-methyl-3-(3-(4-(2-(((*R*,*S*)-1-(4-methylpiperazin-1-yl)-1-oxopropan-2-yl)amino)pyrimidin-4-yl)thiazol-2-yl)phenyl)pyrrolidin-2-one | | | |
| 230 | 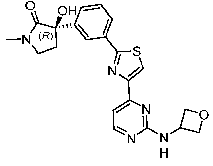 | ¹H NMR (400 MHz, Chloroform-*d*) δ 8.51 (bs, 1H), 8.25 (bs, 1H), 7.69 (d, *J* = 7.7 Hz, 1H), 7.57 (s, 1H), 7.40 (t, *J* = 7.7 Hz, 1H), 7.30 (d, *J = 5.0* Hz, 1H), 7.07 (d, *J* = 7.7 Hz, 1H), 4.70 - 4.72 (m, 1H), 4.58 - 4.31 (m, 3H), 4.09 (bs, 1H), 3.67-3.71 (m, 1H), 3.50-3.55 (m, 1H), 3.08 (s, 3H), 2.59 - 2.33 (m, 2H). MS (ESI⁺): m/z = 424.15. | RP-HPLC (Column 9, basic conditions) | 42 |
| | (R)-3-hydroxy-1-methyl-3-(3-(4-(2-(oxetan-3-ylamino)pyrimidin-4-yl)thiazol-2-yl)phenyl)pyrrolidin-2-one | | | |
| 231 | 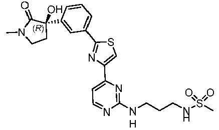 | ¹H NMR (500 MHz, Methanol-*d₄*) δ 8.43 - 8.29 (m, 2H), 8.16-8.19 (m, 1H), 7.95-7.99 (m, 1H), 7.57 - 7.46 (m, 2H), 7.37-7.42 (m, 1H), 3.66 - 3.42 (m, 4H), 3.19 (td, J = 6.7, 1.1 Hz, 2H), 3.00 (s, 3H), 2.92 (s, 3H), 2.49-2.55 (m, 1H), 2.40-2.46 (m, 1H), 1.88-1.94 (m, 2H). MS (ESI+): m/z = 503.15. | 1) FCC: (100% DCM increasing to 5% MeOH-DCM) 2) RP-HPLC (Column 9, basic conditions) | 73 |
| | (R)-N-(3-((4-(2-(3-(3-hydroxy-1-methyl-2-oxopyrrolidin-3-yl)phenyl)thiazol-4-yl)pyrimidin-2-yl)amino)propyl)met hanesulfonamide | | | |
| 232 | 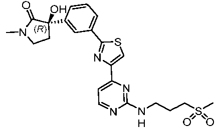 | ¹H NMR (500 MHz, Methanol-*d₄*) δ 8.42 - 8.30 (m, 2H), 8.17-8.19 (m, 1H), 8.01 - 7.93 (m, 1H), 7.55 - 7.47 (m, 2H), 7.41 (d, J = 5.1 Hz, 1H), 3.68 - 3.55 (m, 3H), 3.46-3.51 (m, 1H), 3.28 - 3.17 (m, 2H), 3.00 (s, 3H), 2.97 (s, 3H), 2.48-2.55 (m, 1H), 2.40-2.46 (m, 1H), 2.22 - 2.11 (m, 2H). MS (ESI+): m/z = 488.20. | 1) FCC: (100% DCM increasing to 5% MeOH-DCM) | 63 |
| | (R)-3-hydroxy-1-methyl-3-(3-(4-(2-((3-(methylsulfonyl)prop yl)amino)pyrimidin-4-yl)thiazol-2-yl)phenyl)pyrrolidin-2-one | | 2) RP-HPLC (Column 9, basic conditions) | |
| 233 | 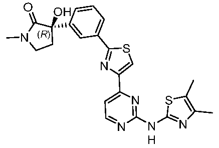 | ¹H NMR (500 MHz, Chloroform-*d*) δ 8.50 (bs, 1H), 7.91 (s, 1H), 7.76-7.82 (m, 1H), 7.52-7.56 (m, 1H), 7.44 (s, 1H), 7.40 - 7.27 (m, 2H), 5.71 (s, 1H), 3.62-3.67 (m, 1H), 3.58 - 3.43 (m, 1H), 3.10 (s, 3H), 2.65 - 2.46 (m, 2H), 2.33 (s, 3H), 2.33 (s, 3H). MS (ESI⁺): m/z = 479.10. | 1) FCC: (100% EtOAc increasing to 5% MeOH-EtOAc) | 23 |
| | (R)-3-(3-(4-(2-((4,5-dimethylthiazol-2-yl)amino)pyrimidin-4-yl)thiazol-2-yl)phenyl)-3-hydroxy-1-methylpyrrolidin-2-one | | 2) RP-HPLC (Column 9, basic conditions) | |
| 234 and 235 | 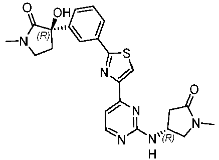 | Diastereromer 1 (Example 234): ¹H NMR (400 MHz, Methanol-*d₄*) δ 8.40 (d, *J* = 5.1 Hz, 1H), 8.35 (s, 1H), 8.15-8.20 (m, 1H), 7.94-7.98 (m, 1H), 7.53 - 7.49 (m, 2H), 7.46 (d, *J* = 5.1 Hz, 1H), 4.67 (br s, 1H), 3.96 - 3.86 (m, 1H), 3.55-3.61 (m, 1H), 3.45-3.56 (m, 1H), 3.40-3.43 (m, 1H), 3.00 (s, 3H), 2.88 (s, 3H), 2.58 - 2.36 (m, 3H). MS (ESI⁺): m/z = 465.20. Diastereomer 2 (Example 235): ¹H NMR (400 MHz, Methanol-*d₄*) δ 8.40 (d, *J* = 5.1 Hz, 1H), 8.35 (s, 1H), 8.15-8.20 (m, 1H), 7.94-7.98 (m, 1H), 7.53 - 7.49 (m, 2H), 7.46 (d, *J* = 5.1 Hz, 1H), 4.67 (br s, 1H), 3.96 - 3.86 (m, 1H), 3.55-3.61 (m, 1H), 3.45-3.56 (m, 1H), 3.40-3.43 (m,1H), 3.00 (s, 3H), 2.88 (s, 3H), 2.58 - 2.36 (m, 3H). MS (ESI⁺): m/z = 465.20. | 1) FCC (100% DCM increasing to 7% MeOH-DCM) | 24 (diaster eomer 1) |
| | 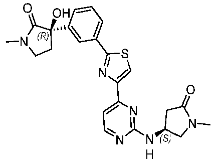 | | 2) RP-HPLC (Column 9, basic conditions). | 27 (diaster eomer 2) |
| | (R)-3-hydroxy-1-methyl-3-(3-(4-(2-(((R)-1-methyl-5-oxopyrrolidin-3-yl)amino)pyrimidin-4-yl)thiazol-2-yl)phenyl)pyrrolidin-2-one and (R)-3-hydroxy-1-methyl-3-(3-(4-(2-(((S)-1-methyl-5-oxopyrrolidin-3-yl)amino)pyrimidin-4-yl)thiazol-2-yl)phenyl)pyrrolidin-2-one | | 3) Chiral SFC CHIRALCEL OD3 5 µm, 250 x 21 mm, Mobile phase: 35% Methanol, 65% CO₂); 52 mg/min; 1^{st} eluting peak designated as diastereomer 1 (Example 234). 2nd eluting peak designated as diastereomer 2 (Example 235). | |
| 236 | 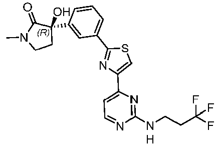 | ¹H NMR (400 MHz, Methanol-*d₄*) δ 8.44 (d, *J =* 5.0 Hz, 1H), 8.13 (br s, 1H), 8.02 (br s, 1H), 7.92-7.96 (m, 1H), 7.55 - 7.34 (m, 3H), 3.75 - 3.64 (m, 2H), 3.63 - 3.41 (m, 2H), 3.00 (s, 3H), 2.64 - 2.49 (m, 3H), 2.38-2.46 (m, 1H). MS (ESI⁺): m/z = 464.20. | 1) FCC: (100% EtOAc increasing to 5% MeOH-EtOAc) | 38 |
| | (R)-3-hydroxy-1-methyl-3-(3-(4-(2-((3,3,3-trifluoropropyl)amino)pyrimidin-4-yl)thiazol-2-yl)phenyl)pyrrolidin-2-one | | 2) RP-HPLC (Column 9, basic conditions) | |
| 237 | 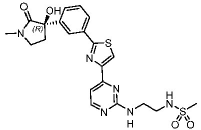 | ¹H NMR (400 MHz, Methanol-*d₄*) δ 8.42 (d, *J* = 5.1 Hz, 1H), 8.13 (t, *J* = 1.7 Hz, 1H), 8.01 (s, 1H), 7.94 (dt, *J* = 7.5, 1.6 Hz, 1H), 7.50 - 7.35 (m, 3H), 3.68 - 3.40 (m, 4H), 3.36 (d, *J* = 6.3 Hz, 2H), 2.99 (s, 3H), 2.95 (s, 3H), 2.50-2.57 (m, 1H), 2.38-2.46 (m, 1H). MS (ESI⁺): m/z = 489.10. | 1) FCC (100% DCM increasing to 10% MeOH-DCM) 2) RP-HPLC (Column 9, basic conditions). | 77 |
| | (R)-N-(2-((4-(2-(3-(3-hydroxy-1-methyl-2-oxopyrrolidin-3-yl)phenyl)thiazol-4-yl)pyrimidin-2-yl)amino)ethyl)meth anesulfonamide | | | |
| 238 | 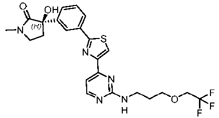 | ¹H NMR (400 MHz, Methanol-*d₄*) δ 8.37 - 8.34 (m, 2H), 8.19-8.17 (m, 1H), 7.99 - 7.96 (m, 1H), 7.54 - 7.48 (m, 2H), 7.39 (d, *J =* 5.2 Hz, 1H), 3.93 (q, *J* = 9.0 Hz, 2H), 3.75 (t, *J* = 6.1 Hz, 2H), 3.65 - 3.42 (m, 4H), 3.00 (s, 3H), 2.58 - 2.38 (m, 2H), 1.92-1.98 (m, 2H). ¹⁹F NMR (376 MHz, Chloroform-d) δ -74.14. MS (ESI⁺): m/z = 508.10. | 1) FCC (100% DCM increasing to 5% MeOH-DCM) 2) RP-HPLC (Column 9, basic conditions). | 75 |
| | (R)-3-hydroxy-1-methyl-3-(3-(4-(2-((3-(2,2,2-trifluoroethoxy)propy l)amino)pyrimidin-4-yl)thiazol-2-yl)phenyl)pyrrolidin-2-one | | | |
| 239 | 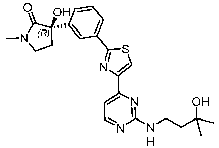 | ¹H NMR (500 MHz, Methanol-*d₄*) δ 8.40 - 8.29 (m, 2H), 8.22 - 8.12 (m, 1H), 7.95-8.00 (m, 1H), 7.57 - 7.47 (m, 2H), 7.45 - 7.34 (m, 1H), 3.45-3.61 (m, 4H), 3.00 (s, 3H), 2.48-2.55 (m, 1H), 2.39-2.51 (m, 1H), 1.87 - 1.78 (m, 2H), 1.29 (s, 6H). MS (ESI+): m/z = 454.30. | 1) FCC (100% DCM increasing to 5% MeOH-DCM) 2) RP-HPLC (Column 9, basic conditions). | 78 |
| | (R)-3-hydroxy-3-(3-(4-(2-((3-hydroxy-3-methylbutyl)amino)p yrimidin-4-yl)thiazol-2-yl)phenyl)-1-methylpyrrolidin-2-one | | | |
| 240 | 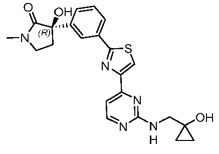 | ¹H NMR (400 MHz, Methanol-*d₄*) δ 8.42 - 8.27 (m, 2H), 8.16-8.19 (m, 1H), 8.04 - 7.93 (m, 1H), 7.58 - 7.46 (m, 2H), 7.38-7.42 (m, 1H), 3.65 (s, 2H), 3.63 - 3.41 (m, 2H), 2.99 (s, 3H), 2.69 - 2.29 (m, 2H), 0.85 - 0.60 (m, 4H). MS (ESI+): m/z = 438.20. | 1) FCC (100% DCM increasing to 5% MeOH-DCM) 2) RP-HPLC (Column 9, basic conditions). | 72 |
| | (R)-3-hydroxy-3-(3-(4-(2-(((1-hydroxycyclopropyl) methyl)amino)pyrimi din-4-yl)thiazol-2-yl)phenyl)-1-methylpyrrolidin-2-one | | | |
| 241 | 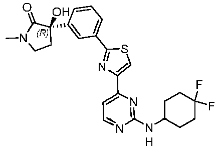 | ¹H NMR (400 MHz, Methanol-*d₄*) δ 8.37 (d, *J* = 5.1 Hz, 1H), 8.35 (s, 1H), 8.16-8.19 (m, 1H), 8.01 - 7.94 (m, 1H), 7.57 - 7.47 (m, 2H), 7.40 (d, J = 5.1 Hz, 1H), 4.14 - 3.98 (m, 1H), 3.57-3.60 (m, 1H), 3.48-3.55 (m, 1H), 3.00 (s, 3H), 2.59 - 2.34 (m, 2H), 2.22 - 1.85 (m, 7H), 1.70-1.73 (m, 2H). MS (ESI+): m/z = 486.20. | 1) FCC (100% DCM increasing to 5% MeOH-DCM) | 35 |
| | (R)-3-(3-(4-(2-((4,4-difluorocyclohexyl)a mino)pyrimidin-4-yl)thiazol-2-yl)phenyl)-3-hydroxy-1-methylpyrrolidin-2-one | | 2) RP-HPLC (Column 9, basic conditions). | |
| 242 | 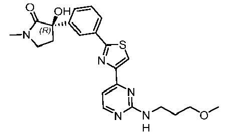 | ¹H NMR (400 MHz, Methanol-*d₄*) δ 8.36 - 8.27 (m, 2H), 8.17 (dt, *J* = 1.9, 1.0 Hz, 1H), 7.98 - 7.91 (m, 1H), 7.54 - 7.42 (m, 2H), 7.36 (d, *J* = 5.1 Hz, 1H), 3.62 - 3.41 (m, 6H), 3.31 (s, 1H), 2.99 (s, 3H), 2.55 - 2.32 (m, 2H), 1.97 - 1.82 (m, 2H). MS (ESI⁺): m/z = 440.20. | 1) FCC (100% DCM increasing to 5% MeOH-DCM) 2) RP-HPLC (Column 9, basic conditions). | 83 |
| | (R)-3-hydroxy-3-(3-(4-(2-((3-methoxypropyl)amin o)pyrimidin-4-yl)thiazol-2-yl)phenyl)-1-methylpyrrolidin-2-one | | | |
| 243 | 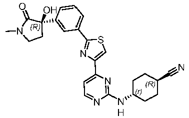 | 1H NMR (600 MHz, Chloroform-d) δ 8.48 (s, 1H), 8.32 (s, 1H), 8.00 (s, 1H), 7.76 (s, 1H), 7.40 (q, J = 6.9, 6.1 Hz, 2H), 3.74 (s, 1H), 3.62 (s, 1H), 3.48 (td, J = 9.7, 8.8, 5.2 Hz, 1H), 3.06 (s, 4H), 2.54 (ddd, J = 13.4, 8.2, 5.1 Hz, 1H), 2.43 (ddt, J = 13.4, 7.9, 4.2 Hz, 2H), 2.14 (d, J = 8.0 Hz, 2H), 2.10 - 1.97 (m, 1H), 1.82 (s, 1H), 1.77 - 1.53 (m, 3H), 1.43 - 1.15 (m, 2H), 1.03 (s, 1H).MS (ESI⁺): m/z = 475.25. | 1) FCC (100% DCM increasing to 10% MeOH-DCM) 2) RP-HPLC (Column 9, basic conditions). | 16 |
| | (1R,4r)-4-((4-(2-(3-((R)-3-hydroxy-1-methyl-2-oxopyrrolidin-3-yl)phenyl)thiazol-4-yl)pyrimidin-2-yl)amino)cyclohexan e-1-carbonitrile | | | |
| 244 | 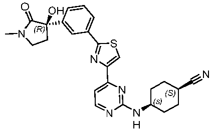 | ¹H NMR (500 MHz, Methanol-*d₄*) δ 8.42 - 8.30 (m, 2H), 8.17 - 8.19 (m, 1H), 7.94 - 7.98 (m, 1H), 7.56 - 7.46 (m, 2H), 7.40 (d, J = 5.1 Hz, 1H), 3.89 - 3.94 (m, 1H), 3.55-3.60 (m, 1H), 3.45-3.52 (m, 1H), 3.02 - 3.05 (m, 1H), 3.00 (s, 3H), 2.48-2.55 (m, 1H), 2.39 - 2.45 (m, 1H), 2.09 - 1.98 (m, 4H), 1.79-1.85 (m, 2H), 1.78 - 1.64 (m, 2H). MS (ESI⁺): m/z = 475.20. | 1) FCC (100% DCM increasing to 10% MeOH-DCM) 2) RP-HPLC (Column 9, basic conditions). | 20 |
| | (1S,4s)-4-((4-(2-(3-((R)-3-hydroxy-1-methyl-2-oxopyrrolidin-3-yl)phenyl)thiazol-4-yl)pyrimidin-2-yl)amino)cyclohexan e-1-carbonitrile | | | |
| 245 | 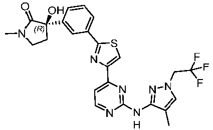 | ¹H NMR (500 MHz, Methanol-*d₄*) δ 8.44 (d, *J* = 5.1 Hz, 1H), 8.26 (s, 1H), 8.18 (br d, 1H), 8.00 - 7.93 (m, 1H), 7.58 (d, *J* = 5.1 Hz, 1H), 7.55 (s, 1H), 7.53 - 7.47 (m, 2H), 4.78-4.84 (m, 2H), 3.52-3.61 (m, 1H), 3.43-3.50 (m, 1H), 2.99 (s, 3H), 2.45-2.55 (m, 1H), 2.39-2.44 (m, 1H), 2.00 (d, J = 0.9 Hz, 3H). MS (ESI⁺): m/z = 530.20. | 1) FCC (100% DCM increasing to 10% MeOH-DCM) 2) RP-HPLC (Column 9, basic conditions). | 81 |
| | (R)-3-hydroxy-1-methyl-3-(3-(4-(2-((4-methyl-1-(2,2,2-trifluoroethyl)-1H-pyrazol-3-yl)amino)pyrimidin-4-yl)thiazol-2-yl)phenyl)pyrrolidin-2-one | | | |
| 246 | 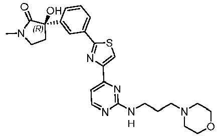 | ¹H NMR (400 MHz, Methanol-*d₄*) δ 8.38 - 8.31 (m, 2H), 8.16-8.19 (m, 1H), 7.94-7.99 (m, 1H), 7.55 - 7.48 (m, 2H), 7.39 (d, J = 5.1 Hz, 1H), 3.75 - 3.66 (m, 4H), 3.55-3.62 (m, 1H), 3.54 - 3.44 (m, 3H), 3.00 (s, 3H), 2.57 - 2.36 (m, 8H), 1.97 - 1.79 (m, 2H). MS (ESI⁺): m/z = 495.20. | 1) FCC (100% DCM increasing to 10% MeOH-DCM) 2) RP-HPLC (Column 9, basic conditions). | 84 |
| | (R)-3-hydroxy-1-methyl-3-(3-(4-(2-((3-morpholinopropyl)amino)pyrimidin-4-yl)thiazol-2-yl)phenyl)pyrrolidin-2-one | | | |
| 247 | 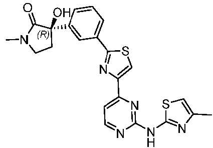 | ¹H NMR (500 MHz, Methanol-*d₄*) δ 8.74 (d, J = 5.0 Hz, 1H), 8.17 (t, J = 1.8 Hz, 1H), 8.14 (s, 1H), 7.98 (dt, J = 7.6, 1.5 Hz, 1H), 7.81 (d, J = 5.0 Hz, 1H), 7.55 - 7.38 (m, 2H), 6.65 (s, 1H), 3.55-3.60 (m, 1H), 3.46-3.53 (m, 2H), 3.00 (s, 3H), 2.52-2.57 (m, 1H), 2.40-2.46 (m, 1H), 2.34 (d, J = 1.1 Hz, 3H). MS (ESI⁺): m/z = 465.10. | 1) FCC (100% DCM increasing to 10% MeOH-DCM) 2) RP-HPLC (Column 9, basic conditions). | 27 |
| | (R)-3-hydroxy-1-methyl-3-(3-(4-(2-((4-methylthiazol-2-yl)amino)pyrimidin-4-yl)thiazol-2-yl)phenyl)pyrrolidin-2-one | | | |
| 248 | 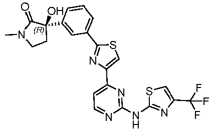 | ¹H NMR (400 MHz, Methanol-*d₄*) δ 8.74 (dd, *J* = 35.8, 5.3 Hz, 1H), 8.23 - 8.09 (m, 2H), 7.98 (t, *J* = 7.6 Hz, 1H), 7.94 - 7.84 (m, 2H), 7.63 (s, 1 H), 7.47 (dd, *J =* 15.7, 8.2 Hz, 2H), 3.66 - 3.44 (m, 2H), 3.00 (d, *J =* 2.8 Hz, 3H), 2.53 (dd, *J =* 8.0, 4.4 Hz, 1H), 2.49 - 2.34 (m, 1H). MS (ESI⁺): m/z = 519.1. | 1) FCC (100% DCM increasing to 10% MeOH-DCM) 2) RP-HPLC (Column 9, basic conditions). | 26 |
| | (R)-3-hydroxy-1-methyl-3-(3-(4-(2-((4-(trifluoromethyl)thiaz ol-2-yl)amino)pyrimidin-4-yl)thiazol-2-yl)phenyl)pyrrolidin-2-one | | | |
| 249 | 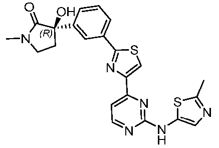 | ¹H NMR (400 MHz, Methanol-*d₄*) δ 8.59 (d, *J* = 5.2 Hz, 1H), 8.22 - 8.10 (m, 2H), 8.01 - 7.93 (m, 1H), 7.88 (d, J = 5.1 Hz, 1H), 7.56 - 7.46 (m, 2H), 7.36 (bs, 1H), 3.50-3.63 (m, 1H), 3.45-3.53 (m, 1H), 2.99 (s, 3H), 2.58 - 2.29 (m, 5H). MS (ESI+): m/z = 465.2. | 1) FCC (100% DCM increasing to 10% MeOH-DCM) 2) RP-HPLC (Column 9, basic conditions). | 18 |
| | (R)-3-hydroxy-1-methyl-3-(3-(4-(2-((2-methylthiazol-5-yl)amino)pyrimidin-4-yl)thiazol-2-yl)phenyl)pyrrolidin-2-one | | | |
| 250 | 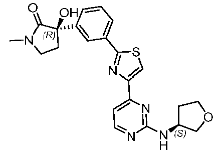 | ¹H NMR (500 MHz, Methanol-*d₄*) δ 8.45 - 8.27 (m, 2H), 8.17 (s, 1H), 8.02 - 7.87 (m, 1H), 7.61 - 7.34 (m, 3H), 4.58 (s, 1H), 4.09 - 3.92 (m, 2H), 3.92 - 3.81 (m, 1H), 3.81 - 3.68 (m, 1H), 3.64 - 3.53 (m, 1H), 3.53 - 3.40 (m, 1H), 2.99 (s, 3H), 2.56 - 2.29 (m, 3H), 2.03 - 1.94 (m, 1H). MS (ESI⁺): m/z = 438.2. | RP-HPLC (Column 9, basic conditions) | 56 |
| | (*R*)-3-Hydroxy-1-methyl-3-(3-(4-(2-(((*S*)-tetrahydrofuran-3-yl)amino)pyrimidin-4-yl)thiazol-2-yl)phenyl)pyrrolidin-2-one | | | |
| 251 | 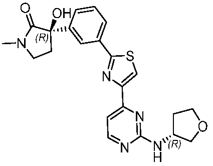 | ¹H NMR (500 MHz, Methanol-*d₄*) δ 8.38 (d, *J =* 5.1 Hz, 1H), 8.34 (s, 1H), 8.22 - 8.15 (m, 1H), 8.04 - 7.92 (m, 1H), 7.55 - 7.48 (m, 2H), 7.43 (d, *J* = 5.1 Hz, 1H), 4.66 - 4.56 (m, 1H), 4.10 - 3.97 (m, 2H), 3.94 - 3.82 (m, 1H), 3.73 (dd, J *=*9.0, 3.9 Hz, 1H), 3.63 - 3.53 (m, 1H), 3.53 - 3.47 (m, 1H), 3.00 (s, 3H), 2.58 - 2.49 (m, 1H), 2.48 - 2.39 (m, 1H), 2.38 - 2.30 (m, 1H), 2.04 - 1.91 (m, 1H). MS (ESI⁺): m/z = 438.1. | RP-HPLC (Column 9, basic conditions) | 60 |
| | (*R*)-3-Hydroxy-1-methyl-3-(3-(4-(2-*(((R)-*tetrahydrofuran-3-yl)amino)pyrimidin-4-yl)thiazol-2-yl)phenyl)pyrrolidin-2-one | | | |
| 252 | 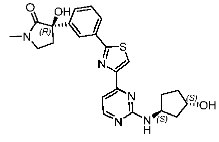 | ¹H NMR (400 MHz, Chloroform-d) δ 8.49 (d, *J* = 5.1 Hz, 1H), 8.29 (s, 1H), 8.03 (s, 1H), 7.78 (d, *J* = 7.6 Hz, 1H), 7.51 - 7.35 (m, 2H), 7.27 (s, 1H), 6.10 (s, 1H), 4.47 - 4.22 (m, 2H), 3.71 - 3.56 (m, 1H), 3.54 - 3.42 (m, 1H), 3.08 (s, 3H), 2.62 - 2.51 (m, 1H), 2.51 - 2.39 (m, 1H), 2.17 - 1.90 (m, 3H), 1.82 - 1.72 (m, 1H), 1.63 - 1.52 (m, 2H), 1.40 - 1.25 (m, 1H). MS (ESI⁺): m/z = 452.2. | RP-HPLC (Column 9, basic conditions) | 75 |
| | (*R*)-3-Hydroxy-3-(3-(4-(2-(((1*S*,3*S*)-3-hydroxycyclopentyl) amino)pyrimidin-4-yl)thiazol-2-yl)phenyl)-1-methylpyrrolidin-2-one. | | | |
| 253 | 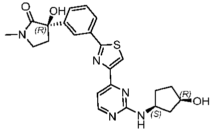 | ¹H NMR (400 MHz, Chloroform-*d*) δ 8.52 (d, *J =* 5.1 Hz, 1H), 8.39 (s, 1H), 7.98 (s, 1H), 7.70 (d, *J* = 7.5 Hz, 1H), 7.45 - 7.32 (m, 2H), 7.21 (d, *J* = 7.9 Hz, 1H), 6.86 (s, 1H), 4.34 - 4.20 (m, 1H), 4.12 (s, 1H), 3.75 - 3.62 (m, 1H), 3.59 - 3.29 (m, 2H), 3.06 (s, 3H), 2.62 - 2.53 (m, 1H), 2.50 - 2.34 (m, 1H), 2.18 - 2.00 (m, 1H), 1.84 - 1.73 (m, 3H), 1.40-1.25 (m, 1H). MS (ESI⁺): m/z = 452.2. | RP-HPLC (Column 9, basic conditions) | 56 |
| | (R)-3-Hydroxy-3-(3-(4-(2-(((1*S*,3*R*)-3-hydroxycyclopentyl) amino)pyrimidin-4-yl)thiazol-2-yl)phenyl)-1-methylpyrrolidin-2-one. | | | |
| 254 | 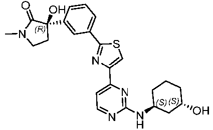 | ¹H NMR (400 MHz, Methanol-*d₄*) δ 8.43 - 8.29 (m, 2H), 8.23 - 8.13 (m, 1H), 8.03 - 7.89 (m, 1H), 7.58 - 7.47 (m, 2H), 7.38 (d, *J* = 5.1 Hz, 1H), 4.35 (s, 1H), 4.11 (d, *J* = 5.3 Hz, 1H), 3.66 - 3.43 (m, 2H), 3.00 (s, 3H), 2.58 - 2.38 (m, 2H), 2.05 - 1.92 (m, 2H), 1.92 - 1.78 (m, 1H), 1.78 - 1.60 (m, 4H), 1.54 - 1.41 (m, 1H). MS (ESI⁺): m/z = 466.3. | RP-HPLC (Column 9, basic conditions) | 35 |
| | (*R*)-3-Hydroxy-3-(3-(4-(2-(((1*S*,3*S*)-3-hydroxycyclohexyl)a mino)pyrimidin-4-yl)thiazol-2-yl)phenyl)-1-methylpyrrolidin-2-one. | | | |
| 255 | 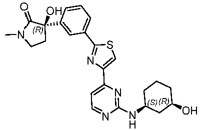 | ¹H NMR (500 MHz, Methanol-*d₄*) δ 8.34 (d, *J =* 5.2 Hz, 1H), 8.30 (s, 1H), 8.22 - 8.13 (m, 1H), 8.03 - 7.91 (m, 1H), 7.54 - 7.45 (m, 2H), 7.36 (d, *J* = 5.1 Hz, 1H), 3.94 (br, 1H), 3.77 - 3.65 (m, 1H), 3.64 - 3.55 (m, 1H), 3.55 - 3.45 (m, 1H), 2.99 (s, 3H), 2.55 - 2.39 (m, 2H), 2.39 - 2.28 (m, 1H), 2.04 - 1.88 (m, 2H), 1.88 - 1.79 (m, 1H), 1.48 - 1.35 (m, 1H), 1.35 - 1.17 (m, 3H). MS (ESI⁺): m/z = 466.3. | RP-HPLC (Column 9, basic conditions) | 26 |
| | (*R*)-3-Hydroxy-3-(3-(4-(2-(((1*S*,3*R*)-3-hydroxycyclohexyl)a mino)pyrimidin-4-yl)thiazol-2-yl)phenyl)-1-methylpyrrolidin-2-one. | | | |

The compounds shown in Table 3F were prepared in a manner analogous to (R)-3-(3-(4-(2-((1,5-dimethyl-1*H*-pyrazol-3-yl)amino)pyrimidin-4-yl)thiazol-2-yl)phenyl)-3-hydroxy-1-methylpyrrolidin-2-one (Example 49) using the appropriate amine in place of 1,5-dimethyl-1*H*-pyrazol-3-amine. Purification methods are noted in the table.

**Table 3F:**

| # | Structure | Analytical data | Purification conditions | Yield (%) |
|---|---|---|---|---|
| | Name | | | |
| 256 | 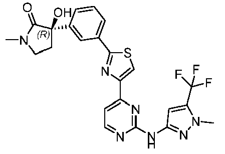 | ¹H NMR (400 MHz, DMSO-*d*₆) δ 10.27 (s, 1H), 8.64 (d, *J* = 5.2 Hz, 1H), 8.43 (s, 1H), 8.13 - 8.08 (m, 1H), 7.99 - 7.93 (m, 1H), 7.56 (d, *J* = 5.2 Hz, 1H), 7.55 - 7.50 (m, 1H), 7.48 - 7.43 (m, 1H), 7.29 (s, 1H), 6.25 (s, 1H), 3.90 (s, 3H), 3.54 - 3.40 (m, 2H), 2.87 (s, 3H), 2.43 - 2.24 (m, 2H). MS (ESI⁺): m/z = 516.2. | HPLC: Column C10; eluent: 50% to 80% (v/v) CH₃CN and H₂O with 0.05% NH₃ + 10 mM NH₄HCO₃) | 14 |
| | (*R*)-3-Hydroxy-1-methyl-3-(3-(4-(2-((1-methyl-5-(trifluoromethyl)-1*H*-pyrazol-3-yl)amino)pyrimidin-4-yl)thiazol-2-yl)phenyl)pyrrolidin -2-one | | | |
| 257 | 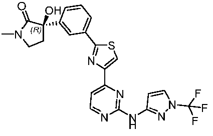 | ¹H NMR (400 MHz, DMSO-*d*₆) δ 10.53 (s, 1H), 8.67 (d, *J* = 5.0 Hz, 1H), 8.53 (s, 1H), 8.35 (d, *J* = 2.7 Hz, 1H), 8.18 - 8.09 (m, 1H), 7.96 (d, *J* = 7.7 Hz, 1H), 7.62 (d, *J =* 5.0 Hz, 1H), 7.56 - 7.50 (m, 1H), 7.48 - 7.43 (m, 1H), 7.28 (d, *J* = 2.7 Hz, 1H), 6.26 (s, 1H), 3.50 - 3.47 (m, 2H), 2.87 (s, 3H), 2.43 - 2.35 (m, 1H), 2.32 - 2.25 (m, 1H). MS (ESI⁺): m/z = 502.2. | HPLC: Column C10; eluent: 50% to 80% (v/v) CH₃CN and H₂O with 0.05% NH₃ + 10 mM NH₄HCO₃) | 26 |
| | (*R*)-3-Hydroxy-1-methyl-3-(3-(4-(2-((1-(trifluoromethyl)-1*H*-pyrazol-3-yl)amino)pyrimidin-4-yl)thiazol-2-yl)phenyl)pyrrolidin -2-one | | | |
| 258 | 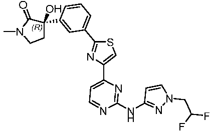 | ¹H NMR (400 MHz, DMSO-*d*₆) δ 9.70 (s, 1H), 8.59 - 8.55 (m, 1H), 8.48 (s, 1H), 8.14 - 8.10 (m, 1H), 7.98 - 7.93 (m, 1H), 7.55 - 7.49 (m, 1H), 7.49 - 7.42 (m, 2H), 6.59 (s, 1H), 6.26 (s, 1H), 3.64 (s, 3H), 3.53 - 3.45 (m, 1H), 3.43 - 3.39 (m, 1H), 2.87 (s, 3H), 2.43 - 2.35 (m, 1H), 2.34 - 2.22 (m, 4H). MS (ESI⁺): m/z = 462.2. | 1) HPLC: Xtimate C18 column, 5 µm, 150 x 40 mm; eluent: 30% to 60% (v/v) CH₃CN and H₂O with 0.05% NH₃ + 10 mM NH₄HCO₃); | 31 |
| | (*R*)-3-(3-(4-(2-((1-(2,2-Difluoroethyl)-1*H*-pyrazol-3-yl)amino)pyrimidin-4-yl)thiazol-2-yl)phenyl)-3-hydroxy-1-methylpyrrolidin-2-one | | 2) SFC: DAICEL CHIRALPAK AS column, 10 µm, 250 x 30 mm; 45% (v/v) EtOH (containing 0.1% of 25% aq. NH₃)/CO₂) | |
| 259 | 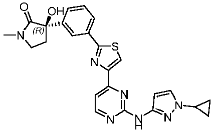 | ¹H NMR (400 MHz, DMSO-*d*₆) δ 9.82 (s, 1H), 8.58 (d, *J* = 5.0 Hz, 1H), 8.45 (s, 1H), 8.11 (s, 1H), 7.95 (d, *J* = 7.6 Hz, 1H), 7.66 (d, *J* = 2.3 Hz, 1H), 7.56 - 7.42 (m, 3H), 6.76 (d, *J* = 2.0 Hz, 1H), 6.25 (s, 1H), 3.65 - 3.58 (m, 1H), 3.49 - 3.46 (m, 2H), 2.87 (s, 3H), 2.41 - 2.35 (m, 1H), 2.31 - 2.23 (m, 1H), 1.04 - 0.97 (m, 2H), 0.97 - 0.90 (m, 2H). MS (ESI⁺): m/z = 474.3. | 1) HPLC: Welch Xtimate C18 column, 5 µm, 150 x 30 mm; eluent: 40% to 70% (v/v) CH₃CN and H₂O with 0.05% NH₃ + 10 mM NH₄HCO₃) | 44 |
| | (*R*)-3-(3-(4-(2-((1-Cyclopropyl-1*H-*pyrazol-3-yl)amino)pyrimidin-4-yl)thiazol-2-yl)phenyl)-3-hydroxy-1-methylpyrrolidin-2-one | | 2) HPLC: Phenomenex C18 column, 3 µm, 75 x 30 mm; eluent: 30% to 60% (v/v) CH₃CN and H₂O with 0.05% NH₃ + 10 mM NH₄HCO₃) | |
| 260 | 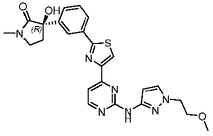 | ¹H NMR (400 MHz, DMSO-*d*₆) δ 9.88 (s, 1H), 8.62 - 8.56 (m, 1H), 8.46 (s, 1H), 8.14 - 8.09 (m, 1H), 7.99 - 7.92 (m, 1H), 7.64 - 7.59 (m, 1H), 7.55 - 7.43 (m, 3H), 6.81 - 6.74 (m, 1H), 6.26 (s, 1H), 4.18 (t, *J* = 5.2 Hz, 2H), 3.68 (t, *J* = 5.2 Hz, 2H), 3.53 - 3.45 (m, 1H), 3.43 - 3.37 (m, 1H), 3.25 (s, 3H), 2.87 (s, 3H), 2.44 - 2.24 (m, 2H). MS (ESI⁺): m/z = 492.2. | HPLC: Column C10; eluent: 35% to 65% (v/v) CH₃CN and H₂O with 0.05% NH₃ + 10 mM NH₄HCO₃) | 31 |
| | (*R*)-3-Hydroxy-3-(3-(4-(2-((1-(2-methoxyethyl)-1*H-*pyrazol-3-yl)amino)pyrimidin-4-yl)thiazol-2-yl)phenyl)-1-methylpyrrolidin-2-one | | | |
| 261 | 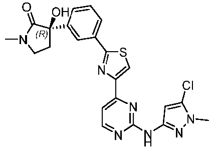 | ¹H NMR (400 MHz, DMSO-*d*₆) δ 10.05 (s, 1H), 8.60 (d, *J* = 5.0 Hz, 1H), 8.48 (s, 1H), 8.14 - 8.08 (m, 1H), 7.98 - 7.94 (m, 1H), 7.55 - 7.49 (m, 2H), 7.48 - 7.41 (m, 1H), 6.87 (s, 1H), 6.25 (s, 1H), 3.73 (s, 3H), 3.52 - 3.46 (m, 1H), 3.43 - 3.40 (m, 1H), 2.87 (s, 3H), 2.41 - 2.35 (m, 1H), 2.31 - 2.25 (m, 1H). MS (ESI⁺): m/z = 482.2. | HPLC: Column C10; eluent: 40% to 70% (v/v) CH₃CN and H₂O with 0.05% NH₃ + 10 mM NH₄HCO₃) | 5 |
| | (*R*)-3-(3-(4-(2-((5-Chloro-1-methyl-1*H*-pyrazol-3-yl)amino)pyrimidin-4-yl)thiazol-2-yl)phenyl)-3-hydroxy-1-methylpyrrolidin-2-one | | | |
| 262 | 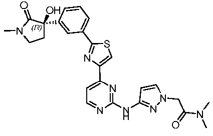 | ¹H NMR (400 MHz, DMSO-*d*₆) δ 9.84 (s, 1H), 8.59 - 8.58 (m, 1H), 8.48 (s, 1H), 8.12 (s, 1H), 7.95 - 7.94 (m, 1H), 7.58 - 7.57 (m, 1H), 7.55 - 7.41 (m, 3H), 6.82 - 6.81 (m, 1H), 6.25 (br s, 1H), 5.00 (s, 2H), 3.53 - 3.47 (m, 2H), 3.04 (s, 3H), 2.87 (s, 3H), 2.86 (s, 3H), 2.43 - 2.35 (m, 1H), 2.34 - 2.24 (m, 1H). MS (ESI⁺): m/z = 519.3. | HPLC: Boston Green ODS column, 5 µm, 150 x 30 mm; eluent: 20% to 50% (v/v) CH₃CN/H₂O with 0.225% HCOOH) | 26 |
| | (*R*)-2-(3-((4-(2-(3-(3-Hydroxy-1-methyl-2-oxopyrrolidin-3-yl)phenyl)thiazol-4-yl)pyrimidin-2-yl)amino)-1*H-*pyrazol-1-yl)-*N,N-*dimethylacetamide | | | |
| 263 | 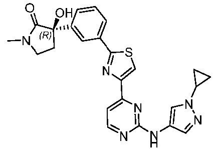 | ¹H NMR (400 MHz, DMSO-*d*₆) δ 9.59 (s, 1H), 8.63 - 8.43 (m, 2H), 8.12 (s, 1H), 8.06 (s, 1H), 7.96 (d, *J* = 7.6 Hz, 1H), 7.61 - 7.49 (m, 2H), 7.47 - 7.42 (m, 2H), 6.25 (s, 1H), 3.82 - 3.69 (m, 1H), 3.53 - 3.45 (m, 1H), 3.43 - 3.38 (m, 1H), 2.87 (s, 3H), 2.43 - 2.34 (m, 1H), 2.33 - 2.25 (m, 1H), 1.08 - 1.01 (m, 2H), 0.99 - 0.91 (m, 2H). MS (ESI⁺): m/z = 474.2. | HPLC: Boston Green ODS column, 5 µm, 150 x 30 mm; eluent: 35% to 65% (v/v) CH₃CN/H₂O with 0.225% HCOOH) | 11 |
| | (*R*)-3-(3-(4-(2-((1-Cyclopropyl-1*H-*pyrazol-4-yl)amino)pyrimidin-4-yl)thiazol-2-yl)phenyl)-3-hydroxy-1-methylpyrrolidin-2-one | | | |
| 264 | 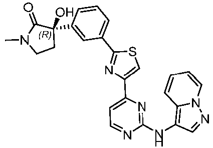 | ¹H NMR (400 MHz, DMSO-*d*₆) δ 9.66 (br s, 1H), 8.62 - 8.45 (m, 3H), 8.41 (s, 1H), 8.12 (s, 1H), 7.95 (d, *J* = 7.6 Hz, 1H), 7.90 - 7.84 (m, 1H), 7.54 - 7.44 (m, 3H), 7.15 - 7.08 (m, 1H), 6.84 - 6.80 (m, 1H), 6.25 (s, 1H), 3.52 - 3.46 (m, 1H), 3.43 - 3.39 (m, 1H), 2.87 (s, 3H), 2.42 - 2.26 (m, 2H). MS (ESI⁺): m/z = 484.2. | HPLC: Boston Green ODS column, 5 µm, 150 x 30 mm; eluent: 30% to 60% (v/v) CH₃CN/H₂O with 0.225% HCOOH) | 43 |
| | (*R*)-3-Hydroxy-1-methyl-3-(3-(4-(2-(pyrazolo[1,5-a]pyridin-3-ylamino)pyrimidin-4-yl)thiazol-2-yl)phenyl)pyrrolidin -2-one | | | |
| 265 | 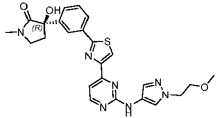 | ¹H NMR (400 MHz, DMSO-*d*₆) δ 9.60 (s, 1H), 8.57 - 8.56 (m, 2H), 8.13 (s, 1H), 8.07 (s, 1H), 7.96 (d, *J* = 7.6 Hz, 1H), 7.58 (s, 1H), 7.55 - 7.50 (m, 1H), 7.47 - 7.45 (m, 1H), 7.44 (s, 1H), 6.26 (s, 1H), 4.28 (s, 2H), 3.70 - 3.69 (m, 2H), 3.53 - 3.46 (m, 1H), 3.43 - 3.40 (m, 1H), 3.26 (s, 3H), 2.87 (s, 3H), 2.43 - 2.34 (m, 1H), 2.34 - 2.25 (m, 1H). MS (ESI⁺): m/z = 492.2. | HPLC: Boston Green ODS column, 5 µm, 150 x 30 mm; eluent: 35% to 65% (v/v) CH₃CN/H₂O with 0.225% HCOOH) | 3 |
| | (*R*)-3-Hydroxy-3-(3-(4-(2-((1-(2-methoxyethyl)-1*H-*pyrazol-4-yl)amino)pyrimidin-4-yl)thiazol-2-yl)phenyl)-1-methylpyrrolidin-2-one | | | |
| 266 | 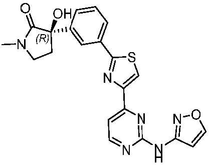 | ¹H NMR (400 MHz, DMSO-*d*₆) δ 10.61 (s, 1H), 8.78 (d, *J* = 2.0 Hz, 1H), 8.69 (d, *J* = 5.2 Hz, 1H), 8.55 (s, 1H), 8.14 - 8.10 (m, 1H), 7.98 - 7.93 (m, 1H), 7.65 (d, *J* = 5.2 Hz, 1H), 7.56 - 7.50 (m, 1H), 7.48 - 7.44 (m, 1H), 7.26 (d, *J* = 2.0 Hz, 1H), 6.26 (s, 1H), 3.53 - 3.49 (m, 2H), 2.87 (s, 3H), 2.44 - 2.36 (m, 1H), 2.34 - 2.25 (m, 1H). MS (ESI⁺): m/z = 435.1. | HPLC: Boston Green ODS column, 5 µm, 150 x 30 mm; eluent: 35% to 65% (v/v) CH₃CN/H₂O with 0.225% HCOOH) | 2 |
| | (*R*)-3-Hydroxy-3-(3-(4-(2-(isoxazol-3-ylamino)pyrimidin-4-yl)thiazol-2-yl)phenyl)-1-methylpyrrolidin-2-one | | | |
| 267 | 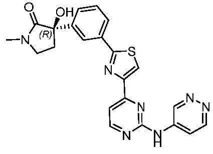 | ¹H NMR (400 MHz, DMSO-*d*₆) δ 9.47 - 9.45 (m, 1H), 9.07 (d, *J* = 5.2 Hz, 1H), 8.91 (s, 1H), 8.46 (s, 1H), 8.42 (s, 1H), 8.19 (d, *J* = 5.2 Hz, 1H), 8.14 (s, 1H), 7.99 (d, *J* = 7.6 Hz, 1H), 7.58 - 7.51 (m, 1H), 7.50 - 7.44 (m, 1H), 6.96 (d, *J* = 7.6 Hz, 1H), 6.26 (s, 1H), 3.38 - 3.36 (m, 2H), 2.87 (s, 3H), 2.38 - 2.37 (m, 1H), 2.35 - 2.30 (m, 1H). MS (ESI⁺): m/z = 446.1. | HPLC: Boston Green ODS column, 5 µm, 150 x 30 mm; eluent: 5% to 40% (v/v) CH₃CN/H₂O with 0.225% HCOOH) | 7 |
| | (*R*)-3-hydroxy-1-methyl-3-(3-(4-(2-(pyridazin-4-ylamino)pyrimidin-4-yl)thiazol-2-yl)phenyl)pyrrolidin -2-one | | | |
| 268 | 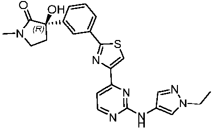 | ¹H NMR (400 MHz, DMSO-*d*₆) δ 9.60 (s, 1H), 8.58 - 8.57 (m, 1H), 8.69 - 8.27 (m, 1H), 8.16 - 8.11 (m, 1H),8.07 - 8.05 (m, 1H), 7.97 - 7.95 (m, 1H), 7.58 - 7.57 (m, 1H), 7.55 - 7.49 (m, 1H), 7.47 - 7.42 (m, 2H), 6.27 (br s, 1H), 4.17 - 4.15 (m, 2H), 3.54 - 3.45 (m, 2H), 2.87 (s, 3H), 2.44 - 2.35 (m, 1H), 2.33 - 2.23 (m, 1H), 1.41 - 1.35 (m, 3H). MS (ESI⁺): m/z = 462.2. | HPLC: Bn Xtimate C18 C18 column, 5 µm, 150 x 25 mm; eluent: 13% to 53% (v/v) CH₃CN/H₂O with 0.225% HCOOH) | 38 |
| | (*R*)-3-(3-(4-(2-((1-ethyl-1*H*-pyrazol-4-yl)amino)pyrimidin-4-yl)thiazol-2-yl)phenyl)-3-hydroxy-1-methylpyrrolidin-2-one | | | |
| 269 | 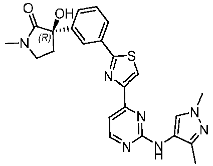 | ¹H NMR (400 MHz, DMSO-*d*₆) δ 8.91 - 8.90 (m, 1H), 8.54 - 8.53 (m, 1H), 8.47 (s, 1H), 8.12 - 8.11 (m, 1H), 8.06 - 7.88 (m, 2H), 7.56 - 7.49 (m, 1H), 7.47 - 7.40 (m, 2H), 6.27 (s, 1H), 3.79 (s, 3H), 3.54 - 3.47 (m, 4H), 2.87 (s, 3H), 2.43 - 2.34 (m, 1H), 2.33 - 2.24 (m, 1H), 2.16 (s, 3H). MS (ESI⁺): m/z = 462.2. | HPLC: Bn Xtimate C18 C18 column, 5 µm, 150 x 25 mm; eluent: 9% to 49% (v/v) CH₃CN/H₂O with 0.225% HCOOH) | 5 |
| | (*R*)-3-(3-(4-(2-((1,3-dimethyl-1*H-*pyrazol-4-yl)amino)pyrimidin-4-yl)thiazol-2-yl)phenyl)-3-hydroxy-1-methylpyrrolidin-2-one | | | |
| 270 | 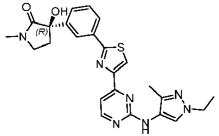 | ¹H NMR (400 MHz, DMSO-*d*₆) δ 9.00 -8.90 (m, 1H), 8.54 - 8.53 (m, 1H), 8.45 (s, 1H), 8.12 - 8.11 (m, 1H), 8.05 - 7.80 (m, 2H), 7.56 - 7.48 (m, 1H), 7.46 - 7.41 (m, 2H), 6.25 (s, 1H), 4.07 (q, *J =* 7.2 Hz, 2H), 3.51 - 3.46 (m, 1H), 3.42 - 3.39 (m, 1H), 2.87 (s, 3H), 2.41 - 2.35 (m, 1H), 2.33 - 2.26 (m, 1H), 2.17 (s, 3H), 1.37 (t, *J* = 7.2 Hz, 3H). MS (ESI⁺): m/z = 476.2. | HPLC: Bn Xtimate C18 C18 column, 5 µm, 150 x 25 mm; eluent: 12% to 52% (v/v) CH₃CN/H₂O with 0.225% HCOOH) | 47 |
| | (*R*)-3-(3-(4-(2-((1-Ethyl-3-methyl-1*H-*pyrazol-4-yl)amino)pyrimidin-4-yl)thiazol-2-yl)phenyl)-3-hydroxy-1-methylpyrrolidin-2-one | | | |
| 271 | 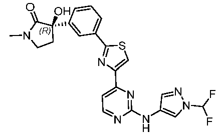 | ¹H NMR (400 MHz, DMSO-*d*₆) δ 9.92 (s, 1H), 8.73 - 8.41 (m, 3H), 8.13 (s, 1H), 7.99 -7.66 (m, 3H), 7.56 - 7.50 (m, 2H), 7.46 - 7.42 (m, 1H), 6.25 (s, 1H), 3.53 - 3.45 (m, 1H), 3.43 - 3.36 (m, 1H), 2.87 (s, 3H), 2.43 - 2.35 (m, 1H), 2.33 - 2.24 (m, 1H). MS (ESI⁺): m/z = 484.2. | HPLC: Xtimate C18 150 x 25 mm x 5 µm, column; eluent: 24% to 64% (v/v) ACN and H₂O with 0.05% ammonia hydroxide and 10 mM NH₄HCO₃). | 8 |
| | (*R*)-3-(3-(4-(2-((1-(Difluoromethyl)-1*H*-pyrazol-4-yl)amino)pyrimidin-4-yl)thiazol-2-yl)phenyl)-3-hydroxy-1-methylpyrrolidin-2-one | | | |
| 272 | 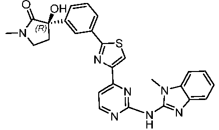 | ¹H NMR (400 MHz, DMSO-*d*₆) δ 9.11 - 9.07 (m, 1H), 8.73 - 8.64 (m, 1H), 8.54 (s, 1H), 8.14 - 8.10 (m, 1H), 8.02 - 7.94 (m, 1H), 7.91 - 7.86 (m, 1H), 7.65 - 7.59 (m, 1H), 7.58 -7.50 (m, 2H), 7.50 - 7.41 (m, 2H), 7.31 - 7.11 (m, 3H), 6.28 - 6.24 (m, 1H), 3.66 (s, 3H), 3.52 - 3.47 (m, 2H), 2.87 (s, 3H), 2.42 - 2.36 (m, 1H), 2.34 - 2.26 (m, 1H). MS (ESI⁺): m/z = 498.2. | HPLC: Xtimate C18 150 x 25 mm x 5 µm column; eluent: 34% to 74% (v/v) ACN and H₂O with 0.05% ammonia hydroxide and 10 mM NH₄HCO₃ | 5 |
| | (*R*)-3-Hydroxy-1-methyl-3-(3-(4-(2-((1-methyl-1*H-*benzo[d]imidazol-2-yl)amino)pyrimidin-4-yl)thiazol-2-yl)phenyl)pyrrolidin -2-one | | | |
| 273 | 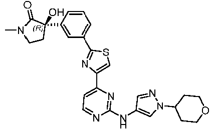 | ¹H NMR (400 MHz, DMSO-*d*₆) δ 9.61 (s, 1H), 8.64 - 8.45 (m, 2H), 8.16 - 8.11 (m, 1H), 8.10 - 8.03 (m, 1H), 7.99 - 7.94 (m, 1H), 7.63 (br s, 1H), 7.56 - 7.50 (m, 1H), 7.47 - 7.43 (m, 2H), 6.26 (s, 1H), 4.44 (br s, 1H), 4.02 - 3.93 (m, 2H), 3.50 - 3.46 (m, 2H), 3.43 - 3.40 (m, 2H), 2.87 (s, 3H), 2.43 - 2.35 (m, 1H), 2.35 - 2.25 (m, 1H), 2.01 - 1.94 (m, 4H). MS (ESI⁺): m/z = 518.2. | HPLC: Boston Green ODS column, 5 µm, 150 x 30 mm; eluent: 25% to 55% (v/v) CH₃CN/H₂O with 0.225% HCOOH | 40 |
| | (*R*)-3-Hydroxy-1-methyl-3-(3-(4-(2-((1-(tetrahydro-2*H-*pyran-4-yl)-1*H-*pyrazol-4-yl)amino)pyrimidin-4-yl)thiazol-2-yl)phenyl)pyrrolidin -2-one | | | |
| 274 | 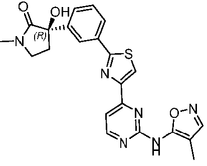 | ¹H NMR (400 MHz, DMSO-*d*₆) δ 9.24 - 9.11 (m, 1H), 8.89 (s, 1H), 8.44 - 8.34 (m, 1H), 8.18 - 8.11 (m, 1H), 8.01 - 7.96 (m, 1H), 7.59 - 7.44 (m, 2H), 6.25 (s, 1H), 3.53 (s, 3H), 3.52 - 3.46 (m, 1H), 3.43 - 3.36 (m, 1H), 3.30 (s, 1H), 2.87 (s, 3H), 2.44 - 2.35 (m, 1H), 2.33 - 2.24 (m, 1H), 1.24 (s, 1H). MS (ESI⁺): m/z = 448.1. | HPLC: Xtimate C18 150 x 40 mm x 5 µm column; eluent: 39% to 64% (v/v) ACN and H₂O with 0.05% ammonia hydroxide and 10 mM NH₄HCO₃ | 8 |
| | (*R*)-3-Hydroxy-1-methyl-3-(3-(4-(2-((4-methylisoxazol-5-yl)amino)pyrimidin-4-yl)thiazol-2-yl)phenyl)pyrrolidin -2-one | | | |
| 275 | 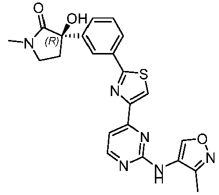 | ¹H NMR (400 MHz, DMSO-*d*₆) δ 9.44 (s, 1H), 9.25 (br s, 1H), 8.70 - 8.59 (m, 2H), 8.16 - 8.09 (m, 1H), 7.99 - 7.92 (m, 1H), 7.60 - 7.56 (m, 1H), 7.55 - 7.50 (m, 1H), 7.49 - 7.44 (m, 1H), 6.26 (s, 1H), 3.53 - 3.46 (m, 2H), 2.87 (s, 3H), 2.43 - 2.37 (m, 1H), 2.37 - 2.35 (m, 3H), 2.32 - 2.26 (m, 1H). MS (ESI⁺): m/z = 449.2. | HPLC: Boston Green ODS column, 5 µm, 150 x 30 mm; eluent: 30% to 60% (v/v) CH₃CN/H₂O (0.225% HCOOH) | 7 |
| | (*R*)-3-Hydroxy-1-methyl-3-(3-(4-(2-((3-methylisoxazol-4-yl)amino)pyrimidin-4-yl)thiazol-2-yl)phenyl)pyrrolidin -2-one | | | |
| 276 | 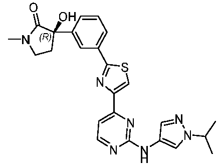 | ¹H NMR (400 MHz, DMSO-*d*₆) δ 9.59 (s, 1H), 8.62 - 8.43 (m, 2H), 8.16 - 8.10 (m, 1H), 8.03 (br s, 1H), 7.98 - 7.91 (m, 1H), 7.66 - 7.56 (m, 1H), 7.55 - 7.49 (m, 1H), 7.47 - 7.41 (m, 2H), 6.27 (s, 1H), 4.60 - 4.47 (m, 1H), 3.55 - 3.47 (m, 2H), 2.87 (s, 3H), 2.44 - 2.35 (m, 1H), 2.33 - 2.24 (m, 1H), 1.44 (s, 3H), 1.42 (s, 3H). MS (ESI⁺): m/z = 476.2. | HPLC: Boston Green ODS column, 5 µm, 150 x 30 mm; eluent: 28% to 58% (v/v) CH₃CN/H₂O with 0.225% HCOOH | 49 |
| | (*R*)-3-Hydroxy-3-(3-(4-(2-((1-isopropyl-1*H-*pyrazol-4-yl)amino)pyrimidin-4-yl)thiazol-2-yl)phenyl)-1-methylpyrrolidin-2-one | | | |
| 277 | 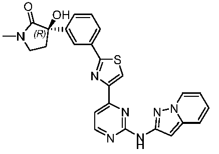 | ¹H NMR (400 MHz, DMSO-*d*₆) δ 8.92 - 8.86 (m, 1H), 8.78 (s, 1H), 8.54 - 8.44 (m, 2H), 8.20 - 8.14 (m, 1H), 8.02 - 7.97 (m, 1H), 7.80 - 7.76 (m, 1H), 7.58 - 7.51 (m, 1H), 7.49 - 7.41 (m, 2H), 6.94 - 6.86 (m, 1H), 6.78 - 6.71 (m, 2H), 6.29 (s, 1H), 3.54 - 3.47 (m, 2H), 2.88 (s, 3H), 2.45 - 2.36 (m, 1H), 2.35 - 2.25 (m, 1H). MS (ESI⁺): m/z = 484.2. | HPLC: Boston Green ODS column, 5 µm, 150 x 30 mm; eluent: 39% to 69% (v/v) CH₃CN/H₂O with 0.225% HCOOH | 33 |
| | (*R*)-3-Hydroxy-1-methyl-3-(3-(4-(2-(pyrazolo[1,5-a]pyridin-2-ylamino)pyrimidin-4-yl)thiazol-2-yl)phenyl)pyrrolidin -2-one | | | |
| 278 | 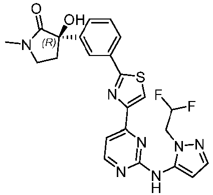 | ¹H NMR (400 MHz, DMSO-*d*₆) δ 8.88 - 8.74 (m, 2H), 8.12 (s, 1H), 8.04 - 7.93 (m, 2H), 7.78 - 7.74 ( m, 1H), 7.59 - 7.41 (m, 2H), 6.83 (s, 2H), 6.52 - 6.17 (m, 2H), 4.58 - 4.47 (m, 2H), 3.65 - 3.42 (m, 2H), 2.87 (s, 3H), 2.43 - 2.23 (m, 2H). MS (ESI⁺): m/z = 498.2. | 1) HPLC: Welch Xtimate C18 column, 5 µm, 150 x 25 mm; eluent: 34% to 64% (v/v) CH₃CN and H₂O with 0.225% HCOOH; 2) SFC: DAICEL CHIRALPAK OJ column, 10 µm, 250 x 30 mm; 40% IPA (containing 0.1% of 25% aq. NH₃)/supercritica I CO₂. | 47 |
| | (*R*)-3-(3-(4-(2-((1-(2,2-Difluoroethyl)-1*H*-pyrazol-5-yl)amino)pyrimidin-4-yl)thiazol-2-yl)phenyl)-3-hydroxy-1-methylpyrrolidin-2-one | | | |
| 279 | 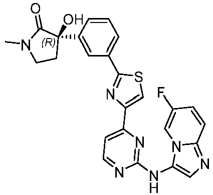 | ¹H NMR (400 MHz, DMSO-*d*₆) δ 8.81 - 8.75 (m, 1H), 8.60 (s, 1H), 8.54 - 8.47 (m, 1H), 8.14 - 8.11 (m, 1H), 8.07 - 8.01 (m, 1H), 7.99 - 7.92 (m, 1H), 7.91 - 7.83 (m, 1H), 7.80 - 7.73 (m, 1H), 7.58 - 7.49 (m, 1H), 7.48 - 7.38 (m, 1H), 6.25 (s, 1H), 5.28 (m, 2H), 3.53 - 3.45 (m, 1H), 3.43 - 3.36 (m, 1H), 2.87 (s, 3H), 2.43 - 2.34 (m, 1H), 2.34 - 2.22 (m, 1H). MS (ESI⁺): m/z = 502.2. | HPLC: Xtimate C18 150 x 40 mm x 5 µm Column; eluent: 48% to 73% (v/v) ACN and H₂O with 0.05% ammonia hydroxide and 10 mM NH₄HCO₃ | 13 |
| | (*R*)-3-(3-(4-(2-((6-Fluoroimidazo[1,2-*a*]pyridin-3-yl)amino)pyrimidin-4-yl)thiazol-2-yl)phenyl)-3-hydroxy-1-methylpyrrolidin-2-one | | | |
| 280 | 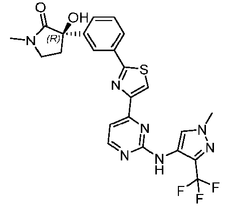 | ¹H NMR (400 MHz, DMSO-*d*₆) δ 8.86 (s, 1H), 8.59 - 8.53 (m, 1H), 8.44 (s, 1H), 8.19 (s, 1H), 8.11 (s, 1H), 7.98 - 7.92 (m, 1H), 7.57 - 7.39 (m, 3H), 6.25 (s, 1H), 3.96 (s, 3H), 3.54 - 3.45 (m, 1H), 3.43 - 3.37 (m, 1H), 2.87 (s, 3H), 2.43 - 2.34 (m, 1H), 2.33 - 2.25 (m, 1H). MS (ESI⁺): m/z = 516.2. | HPLC: Xtimate C18 150 x 40 mm x 5 µm column; eluent: 47% to 72% (v/v) ACN and H₂O with 0.05% ammonia hydroxide and 10 mM NH₄HCO₃ | 35 |
| | (*R*)-3-Hydroxy-1-methyl-3-(3-(4-(2-((1-methyl-3-(trifluoromethyl)-1*H*-pyrazol-4-yl)amino)pyrimidin-4-yl)thiazol-2-yl)phenyl)pyrrolidin -2-one | | | |
| 281 | 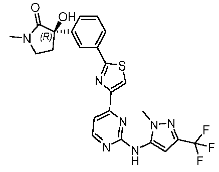 | ¹H NMR (400 MHz, DMSO-*d*₆) δ 9.92 (br s, 1H), 8.71 - 8.57 (m, 1H), 8.40 (s, 1H), 8.11 (s, 1H), 8.02 - 7.88 (m, 1H), 7.70 - 7.59 (m, 1H), 7.56 - 7.39 (m, 2H), 6.80 (s, 1H), 6.25 (s, 1H), 3.83 (s, 3H), 3.55 - 3.46 (m, 1H), 3.42 - 3.37 (m, 1H), 2.87 (s, 3H), 2.46 - 2.33 (m, 1H), 2.33 - 2.22 (m, 1H). MS (ESI⁺): m/z = 516.2. | HPLC: Xtimate C18 150 x 40 mm x 5 µm column; eluent: 48% to 73% (v/v) ACN and H₂O with 0.05% ammonia hydroxide and 10 mM NH₄HCO₃ | 13 |
| | (*R*)-3-Hydroxy-1-methyl-3-(3-(4-(2-((1-methyl-3-(trifluoromethyl)-1*H*-pyrazol-5-yl)amino)pyrimidin-4-yl)thiazol-2-yl)phenyl)pyrrolidin -2-one | | | |
| 282 | 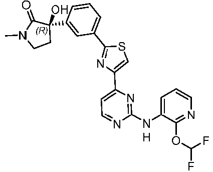 | ¹H NMR (400 MHz, DMSO-*d*₆) δ 8.84 (s, 1H), 8.64 (d, *J* = 5.0 Hz, 1H), 8.55 - 8.44 (m, 2H), 8.12 (s, 1H), 8.03 - 7.70 (m, 3H), 7.61 (d, *J* = 5.0 Hz, 1H), 7.57 - 7.49 (m, 1H), 7.48 - 7.42 (m, 1H), 7.37 - 7.31 (m, 1H), 6.24 (s, 1H), 3.52 - 3.45 (m, 1H), 3.42 - 3.37 (m, 1H), 2.87 (s, 3H), 2.42 - 2.35 (m, 1H), 2.32 - 2.25 (m, 1H). MS (ESI⁺): m/z = 511.1. | HPLC: Column C10; eluent: 55% to 85% (v/v) ACN/H₂O (0.05% NH₃•H₂O+10 mM NH₄HCO₃) | 31 |
| | (*R*)-3-(3-(4-(2-((2-(Difluoromethoxy)p yridin-3-yl)amino)pyrimidin-4-yl)thiazol-2-yl)phenyl)-3-hydroxy-1-methylpyrrolidin-2-one | | | |
| 283 | 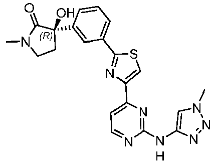 | ¹H NMR (400 MHz, Chloroform-d) δ 8.57 (d, *J* = 5.1 Hz, 1H), 8.08 (d, *J* = 7.7 Hz, 2H), 8.00 (s, 1H), 7.79-7.83 (m, 1H), 7.56 (d, *J* = 5.1 Hz, 1H), 7.47 - 7.32 (m, 2H), 4.12 (s, 3H), 3.51-3.61 (m, 1H), 3.39-3.48 (m, 1H), 3.08 (s, 3H), 2.49-2.59 (m, 1H), 2.37-2.46 (m, 1H). MS (ESI⁺): m/z = 449.80. | 1) FCC (100% DCM increasing to 5% MeOH-DCM); | 56 |
| | (*R*)-3-hydroxy-1-methyl-3-(3-(4-(2-((1-methyl-1*H-*1,2,3-triazol-4-yl)amino)pyrimidin-4-yl)thiazol-2-yl)phenyl)pyrrolidin -2-one | | 2) RP-HPLC (Column C9, basic conditions) | |
| 284 | 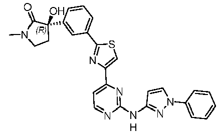 | ¹H NMR (400 MHz, Chloroform-d) δ 8.57 (d, *J* = 5.1 Hz, 1H), 8.43 (s, 1H), 8.18 (s, 1H), 8.14 - 8.02 (m, 1H), 7.93 - 7.81 (m, 2H), 7.63-7.69 (m, 2H), 7.59-7.61 (m, 1H), 7.50 - 7.33 (m, 4H), 7.20-7.25 (m, 1H), 7.07-7.11 (m, 1H), 3.50-3.57 (m, 1H), 3.40-3.47 (m, 1H), 3.07 (s, 3H), 2.50-2.59 (m, 1H), 2.38-2.45 (m, 1H). MS (ESI⁺): m/z = 510.80. | 1) FCC (100% DCM increasing to 5% MeOH-DCM); | 10 |
| | (R)-3-hydroxy-1-methyl-3-(3-(4-(2-((1-phenyl-1H-pyrazol-3-yl)amino)pyrimidin-4-yl)thiazol-2-yl)phenyl)pyrrolidin -2-one | | 2) RP-HPLC (Column C9, basic conditions) | |
| 285 | 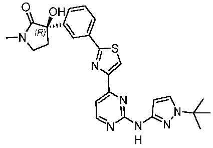 | ¹H NMR (500 MHz, Chloroform-d) δ 8.52 (d, *J* = 5.0 Hz, 1H), 8.18 (s, 1H), 8.09 (td, *J* = 1.8, 0.7 Hz, 1H), 7.92 (dt, *J* = 7.2, 1.7 Hz, 1H), 7.83 (s, 1H), 7.56 (d, *J* = 5.0 Hz, 1H), 7.48 - 7.37 (m, 3H), 6.78 (d, *J* = 2.3 Hz, 1H), 3.59 - 3.30 (m, 2H), 3.06 (s, 3H), 2.64 - 2.32 (m, 2H), 1.80 - 1.32 (m, 9H). MS (ESI⁺): m/z = 490.2. | 1) FCC (100% DCM increasing to 5% MeOH-DCM); | 26 |
| | (*R*)-3-(3-(4-(2-((1-(tert-butyl)-1H-pyrazol-3-yl)amino)pyrimidin-4-yl)thiazol-2-yl)phenyl)-3-hydroxy-1-methylpyrrolidin-2-one | | 2) RP-HPLC (Column C9, basic conditions) | |
| 286 | 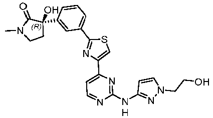 | ¹H NMR (500 MHz, Methanol-*d*₄) δ 8.49 (d, *J* = 5.1 Hz, 1H), 8.42 (s, 1H), 8.20 (br s, 1H), 8.13 (br s 1H), 7.97-8.01 (m, 1H), 7.64 (s, 1H), 7.50-7.54 (m, 3H), 4.24 (t, J = 5.4 Hz, 2H), 3.92 (t, J = 5.4 Hz, 2H), 3.55-3.62 (m, 1H), 3.48-3.52 (m, 1H), 3.00 (s, 3H), 2.50-2.56 (m, 1H), 2.39-2.45 (m, 1H). MS (ESI⁺): m/z = 478.9. | 1) FCC (100% DCM increasing to 5% MeOH-DCM); | 69 |
| | (R)-3-hydroxy-3-(3-(4-(2-((1-(2-hydroxyethyl)-1H-pyrazol-3-yl)amino)pyrimidin-4-yl)thiazol-2-yl)phenyl)-1-methylpyrrolidin-2-one | | 2) RP-HPLC (Column C9, basic conditions) | |
| 287 | 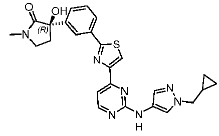 | ¹H NMR (400 MHz, Methanol-*d*₄) δ 8.47-8.49 (m, 1H), 8.35 (s, 1H), 8.19 (s, 1H), 8.10 (s, 1H), 7.95-7.98 (m, 1H), 7.63 (s, 1H), 7.58 - 7.40 (m, 3H), 4.00 (d, J = 7.1 Hz, 2H), 3.48-3.62 (m, 2H), 3.00 (s, 3H), 2.60 - 2.34 (m, 2H), 1.29-1.33 (m, 1H), 0.73 - 0.54 (m, 2H), 0.40-0.44 (m, 2H). MS (ESI⁺): m/z = 488.9. | 1) FCC (100% DCM increasing to 5% MeOH-DCM); | 57 |
| | (*R*)-3-(3-(4-(2-((1-(cyclopropylmethyl )-1H-pyrazol-4-yl)amino)pyrimidin-4-yl)thiazol-2-yl)phenyl)-3-hydroxy-1-methylpyrrolidin-2-one | | 2) RP-HPLC (Column C9, basic conditions) | |
| 288 | 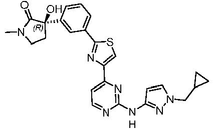 | ¹H NMR (400 MHz, Methanol-*d*₄) δ 8.54 (d, *J* = 5.1 Hz, 1H), 8.41 (s, 1H), 8.21 (br s, 1H), 8.04 - 7.96 (m, 1H), 7.69 - 7.58 (m, 2H), 7.58 - 7.45 (m, 2H), 6.77 (s, 1H), 3.95 (d, *J* = 7.1 Hz, 2H), 3.50-3.58 (m, 2H), 3.02 (s, 3H), 2.65 - 2.37 (m, 2H), 1.43 - 1.21 (m, 1H), 0.71 - 0.58 (m, 2H), 0.40-0.44 (m, 2H). MS (ESI⁺): m/z = 488.20. | 1) FCC (100% EtOAc increasing to 5% MeOH-EtOAc) | 5 |
| | (R)-3-(3-(4-(2-((1-(cyclopropylmethyl )-1H-pyrazol-3-yl)amino)pyrimidin-4-yl)thiazol-2-yl)phenyl)-3-hydroxy-1-methylpyrrolidin-2-one | | 2) RP-HPLC (Column C9, basic conditions) | |
| 289 | 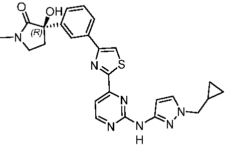 | ¹H NMR (400 MHz, Methanol-*d₄*) δ 8.57 (d, *J* = 5.0 Hz, 1H), 8.15 (t, *J* = 1.8 Hz, 1H), 8.06 (s, 1H), 7.96 (dt, *J* = 7.5, 1.6 Hz, 1H), 7.67 - 7.56 (m, 2H), 7.54 - 7.35 (m, 2H), 6.81 (d, *J* = 2.4 Hz, 1H), 3.93 (d, *J* = 7.0 Hz, 2H), 3.64 - 3.41 (m, 2H), 3.00 (s, 3H), 2.62 - 2.37 (m, 2H), 1.41 - 1.20 (m, 1H), 0.70 - 0.55 (m, 2H), 0.39-0.42 (m, 2H). MS (ESI⁺): m/z = 488.20. | 1) FCC (100% DCM increasing to 5% MeOH-DCM) | 43 |
| | (R)-3-(3-(2-(2-((1-(cyclopropylmethyl )-1H-pyrazol-3-yl)amino)pyrimidin-4-yl)thiazol-4-yl)phenyl)-3-hydroxy-1-methylpyrrolidin-2-one | | 2) RP-HPLC (Column C9, basic conditions) | |

### Example 290: (R)-3-Hydroxy-1-methyl-3-(2-(2-(2-(((S)-1-(1-methyl-1H-pyrazol-5-yl)ethyl)amino)pyrimidin-4-yl)thiazol-4-yl)pyridin-4-yl)pyrrolidin-2-one.

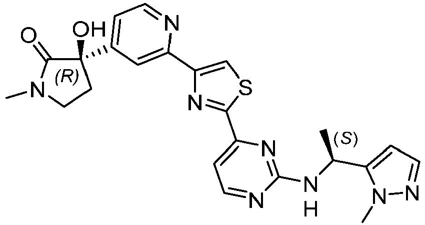

The title compound and its TFA salt were made in a manner analogous to Example 46, (*R*)-3-hydroxy-1-methyl-3-(5-(2-(2-((1-methyl-1*H*-pyrazol-5-yl)amino)pyrimidin-4-yl)thiazol-4-yl)pyridin-3-yl)pyrrolidin-2-one, using the appropriate coupling partner in place of 4-(4-bromothiazol-2-yl)-*N*-(1-methyl-1*H*-pyrazol-5-yl)pyrimidin-2-amine (Intermediate 14). The compound was purified by reverse-phase HPLC purification using acidic media and column C7 to provide the title compound (16% yield) as its TFA salt. ¹H NMR (500 MHz, DMSO-*d*₆) δ 8.63 (dd, *J* = 5.2, 0.7 Hz, 1H), 8.56 (s, 1H), 8.55 (d, *J* = 5.0 Hz, 1H), 8.27 - 8.25 (m, 1H), 8.04 (s, 1H), 7.40 - 7.36 (m, 2H), 7.29 (d, *J =* 1.8 Hz, 1H), 6.24 (d, *J* = 1.9 Hz, 1H), 5.36 (p, *J* = 7.0 Hz, 1H), 3.89 (s, 4H), 3.56 - 3.49 (m, 1H), 3.47 - 3.41 (m, 1H), 2.88 (s, 3H), 2.44 - 2.37 (m, 1H), 2.33 - 2.27 (m, 1H), 1.53 (d, *J* = 6.9 Hz, 3H). MS (ESI⁺): m/z = 477.3. The TFA salt could be converted to the corresponding free base by passage through a carbonate cartridge.

### Example 291: (R)-3-Hydroxy-1-methyl-3-(2-(2-(2-(((R)-1-(1-methyl-1H-pyrazol-5-yl)ethyl)amino)pyrimidin-4-yl)thiazol-4-yl)pyridin-4-yl)pyrrolidin-2-one.

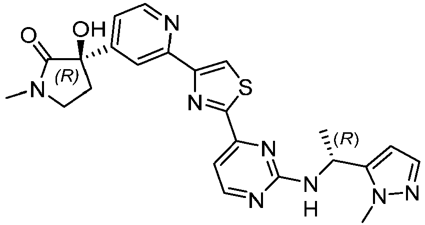

The title compound and its TFA salt were made in a manner analogous to Example 46, (*R*)-3-hydroxy-1-methyl-3-(5-(2-(2-((1-methyl-1*H*-pyrazol-5-yl)amino)pyrimidin-4-yl)thiazol-4-yl)pyridin-3-yl)pyrrolidin-2-one, using the appropriate coupling partner in place of 4-(4-bromothiazol-2-yl)-*N*-(1-methyl-1*H*-pyrazol-5-yl)pyrimidin-2-amine (Intermediate 14). The compound was purified by reverse-phase HPLC purification using acidic media and column C7 to provide the title compound (24% yield) as its TFA salt. ¹H NMR (500 MHz, DMSO-*d*₆) δ 8.62 (dd, *J* = 5.2, 0.8 Hz, 1H), 8.56 (s, 1H), 8.55 (d, *J* = 5.0 Hz, 1H), 8.27 - 8.24 (m, 1H), 8.04 (s, 1H), 7.41 - 7.36 (m, 2H), 7.29 (d, *J =* 1.8 Hz, 1H), 6.24 (d, *J* = 1.9 Hz, 1H), 5.36 (p, *J* = 7.0 Hz, 1H), 3.95 - 3.82 (m, 3H), 3.56 - 3.49 (m, 1H), 3.47 - 3.41 (m, 1H), 2.89 (s, 3H), 2.43 - 2.36 (m, 1H), 2.34 - 2.27 (m, 1H), 1.53 (d, *J* = 6.9 Hz, 3H). MS (ESI⁺): m/z = 477.3. The TFA salt could be converted to the corresponding free base by passage through a carbonate cartridge.

### Example 292: (R)-3-(2-(1-(2-((1-(2,2-Difluoroethyl)-3-methyl-1H-pyrazol-4-yl)amino)pyrimidin-4-yl)-1H-pyrazol-3-yl)pyridin-4-yl)-3-hydroxy-1-methylpyrrolidin-2-one.

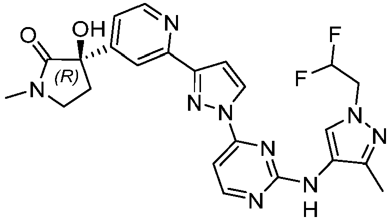

The title compound was made in a manner analogous to Example 46, (R)-3-hydroxy-1-methyl-3-(5-(2-(2-((1-methyl-1*H*-pyrazol-5-yl)amino)pyrimidin-4-yl)thiazol-4-yl)pyridin-3-yl)pyrrolidin-2-one, using the appropriate coupling partner in place of 4-(4-bromothiazol-2-yl)-*N*-(1-methyl-1*H*-pyrazol-5-yl)pyrimidin-2-amine (Intermediate 14). The compound was purified by reverse-phase HPLC purification using basic media and column C8 to provide the title compound (9% yield). ¹H NMR (500 MHz, DMSO-*d*₆) δ 9.32 - 9.08 (m, 1H), 8.64 (d, *J* = 2.3 Hz, 1H), 8.61 (d, *J* = 5.3 Hz, 1H), 8.54 (d, *J* = 5.3 Hz, 1H), 8.17 - 8.13 (m, 1H), 8.11 - 8.00 (m, 1H), 7.34 (dd, *J* = 5.1, 1.9 Hz, 1H), 7.30 (d, *J* = 5.5 Hz, 1H), 7.17 (d, *J* = 2.8 Hz, 1H), 6.39 (s, 1H), 6.35 (tt, *J* = 55.3, 4.0 Hz, 1H), 4.65 - 4.50 (m, 2H), 3.55 - 3.49 (m, 1H), 3.47 - 3.41 (m, 1H), 2.88 (s, 3H), 2.35 - 2.42 (m, 1H), 2.25 - 2.32 (m, 1H), 2.19 (s, 3H). MS (ESI⁺): m/z = 496.2.

### Example 293 and Example 294: (R)-3-(3-(4-(2-(((R)-1-(1H-Pyrazol-4-yl)ethyl)amino)pyrimidin-4-yl)thiazol-2-yl)phenyl)-3-hydroxy-1-methylpyrrolidin-2-one and (R)-3-(3-(4-(2-(((S)-1-(1H-pyrazol-4-yl)ethyl)amino)pyrimidin-4-yl)thiazol-2-yl)phenyl)-3-hydroxy-1-methylpyrrolidin-2-one.

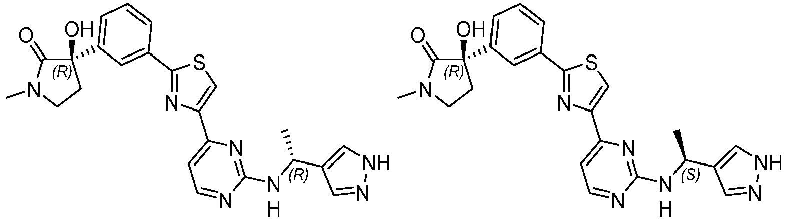

The title compounds were made in a manner analogous to Example 45 using (*R*)-3-hydroxy-1-methyl-3-(3-(4-(2-(methylsulfonyl)pyrimidin-4-yl)thiazol-2-yl)phenyl)pyrrolidin-2-one (Intermediate 12) and the appropriate amine in place of 1-(1*H*-benzo[d]imidazol-2-yl)ethan-1-amine. The reaction mixture was purified by reverse phase HPLC using basic media and column C8 to give (*R*)-3-(3-(4-(2-(((*R*,*S*)-1-(1*H*-pyrazol-4-yl)ethyl)amino)pyrimidin-4-yl)thiazol-2-yl)phenyl)-3-hydroxy-1-methylpyrrolidin-2-one. The (*R*) and (*S*) diastereomers of (*R*)-3-(3-(4-(2-(((*R*, *S*)-1-(1*H*-pyrazol-4-yl)ethyl)amino)pyrimidin-4-yl)thiazol-2-yl)phenyl)-3-hydroxy-1-methylpyrrolidin-2-one were separated by SFC (CHIRALPAK IA, 5 µm, 250 x 30 mm; 35% methanol, 65% CO₂; flow rate 70 mL/min; monitoring at 220 nm) to give two products, the first eluting peak was designated as diastereomer 1 of (*R*)-3-(3-(4-(2-(((*R*, *S*)-1-(1*H*-pyrazol-4-yl)ethyl)amino)pyrimidin-4-yl)thiazol-2-yl)phenyl)-3-hydroxy-1-methylpyrrolidin-2-one (Example 293) (24% yield). ¹H NMR (500 MHz, DMSO-*d*₆) δ 12.43 (s, 1H), 8.64 (s, 1H), 8.38 - 8.29 (m, 1H), 7.92 (t, *J* = 1.9 Hz, 1H), 7.76 (dt, *J* = 7.8, 1.4 Hz, 1H), 7.56 - 7.44 (m, 2H), 7.37 (t, *J* = 7.7 Hz, 1H), 7.31 - 7.26 (m, 1H), 7.06 - 6.98 (m, 2H), 6.02 (s, 1H), 5.23 - 5.13 (m, 1H), 3.44 - 3.36 (m, 1H), 3.35 - 3.27 (m, 1H), 2.79 (s, 3H), 2.35 - 2.26 (m, 1H), 2.24 - 2.16 (m, 1H), 1.41 (d, *J* = 6.9 Hz, 3H), 1.17 (s, 1H). MS (ESI⁺): *m*/*z =* 445.2. The second eluting peak was designated as diastereomer 2 of (*R*)-3-(3-(4-(2-(((*R*,*S*)-1-(1H-pyrazol-4-yl)ethyl)amino)pyrimidin-4-yl)thiazol-2-yl)phenyl)-3-hydroxy-1-methylpyrrolidin-2-one (Example 294) (2% yield). ¹H NMR (500 MHz, DMSO-*d*₆) δ 8.41 (s, 1H), 7.99 (s, 1H), 7.84 (d, *J =* 7.7 Hz, 1H), 7.59 (s, 3H), 7.44 (t, *J* = 7.7 Hz, 1H), 7.38 - 7.34 (m, 1H), 7.09 (d, *J* = 2.7 Hz, 2H), 6.10 (s, 1H), 5.28 - 5.22 (m, 1H), 3.50 - 3.45 (m, 1H), 3.41 - 3.35 (m, 2H), 2.87 (s, 3H), 2.38 (ddd, *J* = 12.7, 7.6, 4.9 Hz, 1H), 2.28 (ddd, *J* = 13.5, 8.1, 5.8 Hz, 1H), 1.49 (d, *J* = 6.9 Hz, 3H), 1.24 (s, 3H). MS (ESI⁺): *m*/*z* = 445.2.

Compounds of the present disclosure were tested in biological assays. The results of the assays are presented in Table 4 below which is entitled Results of Biological Assays. The results are presented as an average of values obtained.

### Assay 1: Inhibition of auto-phosphorylation of recombinant human NF-kappaB-inducing kinase (NIK/MAP3K14) activity (AlphaScreen^{™})

NIK/MAP3K14 auto-phosphorylation activity was measured using the AlphaScreen^{™} (αscreen) format (Perkin Elmer). All compounds tested were dissolved in dimethyl sulfoxide (DMSO) and further dilutions were made in assay buffer. The final DMSO concentration was 0.7% (v/v) in assays. The assay buffer was 50 mM Tris pH 7.5 containing 1 mM EGTA (ethylene glycol tetraacetic acid), 1 mM DTT (dithiothreitol), 0.1 mM Na₃VO₄, 5 mM MgCl₂, and 0.01% Tween^{®} 20. The assays were carried out in 384 well Proxiplates (Perkin Elmer). The incubations consisted of the compound, 5 µM Adenosine-5'-triphosphate (ATP), and 1 nM NIK/MAP3K14. Incubations were initiated by the addition of GST-tagged NIK/MAP3K14 enzyme, carried out for 2 h at 25 °C and terminated by addition of stop buffer containing anti-phospho-IKK Ser176/180 antibody. Protein A Acceptor and Glutathione-Donor beads were added before reading using an EnVision Multilabel Plate Reader (Perkin Elmer). The signal obtained in the wells was normalized using high (full enzyme activity, 0.7% DMSO) and low controls (no enzyme activity, 0.7% DMSO, no ATP). IC₅₀'s were determined by fitting a sigmoidal curve to % inhibition of control versus Log₁₀ compound concentration.

### Assay 2: Effect of compounds on p-IKKa levels in L363 (NIK translocated multiple myeloma) cells

All compounds tested were dissolved and serially diluted in DMSO, 1:3 dilution for 11 points in an Echo compatible plate. 100% DMSO was added to columns 12 and 24 of the plate to serve as high and low signal controls. This compound plate was used to spot 20 nL of compound or DMSO into a Greiner 384 well TC plate (781080). The final DMSO concentration was 0.3% (v/v) in cell assays. Human L363 cells (ATCC) were cultured in RPMI1640 medium supplemented with GlutaMax, non-essential amino acids, sodium pyruvate and 10% fetal bovine serum. Cells were routinely maintained at densities of 0.2x10⁶ cells per ml - 2x10⁸ cells per mL at 37°C in a humidified 5% CO₂ atmosphere incubator. Cells were passaged twice a week splitting back to obtain the low density. The day before the assay, cells were washed twice in HBSS (Hank's Balanced Salt Solution), resuspended in Dulbecco's Modified Eagle Medium (DMEM) + 0.5% IgG and protease free BSA (Jackson Immuno Research Laboratories), +/- 250 ng/ml recombinant human B-cell activating factor (BAFF/BLyS/TNFSF13B) and incubated overnight at 37 °C in a humidified 5% CO₂ atmosphere (bulk stimulation with or without BAFF). The next day, the cell concentration was adjusted to 1x10⁷ cells /ml in DMEM +/- 250 ng/ml BAFF +/- 10 µM MG132 and plated at 10 µl/ well into compound or DMSO spotted 384 well TC plates. Seeded cells were incubated at 37 °C in a humidified 5% CO₂ atmosphere for 6 h. After 6 h, the plates were removed from the incubator and cell lysis was achieved by the addition of 2.5 µl 5x lysis buffer containing protease and phosphatase inhibitors, followed by shaking on a plate shaker at room temperature for 15 min. At the end of this incubation, lysed cells were sequentially treated and incubated with acceptor and donor bead mixes according to the manufacturer's protocol for a 1 plate/ 2-incubation suspension cell assay (AlphaLISA SureFire Ultra p-IKKa (Ser 176/180) Assay Kit (Perkin Elmer). Plates were read using an EnVision Multilabel Plate Reader (Perkin Elmer). Within an experiment, a concentration response curve for each compound was run in duplicate. The signal obtained in the test wells was normalized using high signal (BAFF stimulated cells, DMSO, MG132) and low signal (unstimulated cells, DMSO) controls. To determine the IC₅₀, a sigmoidal curve was fitted to the plot of % inhibition versus Log₁₀ compound concentration.

Table 4 below provides IC₅₀ data for certain compounds described herein on NIK inhibition. In cases where the compound was tested more than once, the IC₅₀ value was calculated as a simple average of the measured values. The average was then used to categorize the compound within one of the following ranges.
A: IC₅₀ < 100 nM;
B: 100 nM ≤ IC₅₀ ≤ 500 nM;
C: IC₅₀ > 501 nM
- Not available

**Table 4. Results of Biological Assays**

| Example | Assay 1 IC₅₀ | Assay 2 IC₅₀ |
|---|---|---|
| 1 | A | B |
| 2 | A | B |
| 3 | A | A |
| 4 | A | C |
| 5 | A | A |
| 6 | B | C |
| 7 | A | B |
| 8 | A | B |
| 9 | A | A |
| 10 | A | B |
| 11 | A | A |
| 12 | A | B |
| 13 | A | B |
| 14 | A | B |
| 15 | A | B |
| 16 | B | C |
| 17 | A | C |
| 18 | A | B |
| 19 | A | A |
| 20 | B | C |
| 21 | nt | nt |
| 22 | C | C |
| 23 | C | C |
| 24 | C | C |
| 25 | B | C |
| 26 | C | C |
| 27 | A | B |
| 28 | A | A |
| 29 | B | C |
| 30 | A | B |
| 31 | C | C |
| 32 | A | C |
| 33 | C | C |
| 34 | A | B |
| 35 | C | C |
| 36 | A | B |
| 37 | C | C |
| 38 | A | B |
| 39 | A | A |
| 40 | A | C |
| 41 | A | B |
| 42 | A | C |
| 43 | A | B |
| 44 | A | C |
| 45 | A | C |
| 46 | A | A |
| 47 | A | C |
| 48 | A | C |
| 49 | A | B |
| 50 | B | C |
| 51 | A | B |
| 52 | B | C |
| 53 | A | B |
| 54 | A | B |
| 55 | A | B |
| 56 | A | B |
| 57 | A | C |
| 58 | C | C |
| 59 | A | B |
| 60 | B | C |
| 61 | A | B |
| 62 | C | C |
| 63 | nt | nt |
| 64 | C | C |
| 65 | B | C |
| 66 | B | C |
| 67 | A | B |
| 68 | C | C |
| 69 | A | C |
| 70 | A | C |
| 71 | A | C |
| 72 | A | B |
| 73 | A | B |
| 74 | B | C |
| 75 | A | B |
| 76 | A | B |
| 77 | A | C |
| 78 | A | A |
| 79 | A | C |
| 80 | A | A |
| 81 | B | C |
| 82 | A | A |
| 83 | A | C |
| 84 | C | C |
| 85 | A | A |
| 86 | A | A |
| 87 | A | B |
| 88 | A | B |
| 89 | A | A |
| 90 | A | B |
| 91 | A | B |
| 92 | A | A |
| 93 | A | B |
| 94 | A | C |
| 95 | A | B |
| 96 | A | A |
| 97 | A | A |
| 98 | A | A |
| 99 | A | A |
| 100 | A | A |
| 101 | A | A |
| 102 | A | A |
| 103 | A | B |
| 104 | A | A |
| 105 | nt | C |
| 106 | A | C |
| 107 | C | C |
| 108 | B | C |
| 109 | B | C |
| 110 | A | C |
| 111 | A | C |
| 112 | A | C |
| 113 | A | C |
| 114 | B | C |
| 115 | B | C |
| 116 | A | C |
| 117 | A | B |
| 118 | A | C |
| 119 | A | B |
| 120 | B | C |
| 121 | A | B |
| 122 | A | C |
| 123 | A | C |
| 124 | A | C |
| 125 | A | C |
| 126 | A | B |
| 127 | A | B |
| 128 | A | B |
| 129 | A | B |
| 130 | A | B |
| 131 | A | C |
| 132 | A | B |
| 133 | A | C |
| 134 | C | C |
| 135 | C | C |
| 136 | B | C |
| 137 | A | C |
| 138 | A | C |
| 139 | A | A |
| 140 | A | A |
| 141 | A | A |
| 142 | nt | nt |
| 143 | nt | nt |
| 144 | A | B |
| 145 | A | A |
| 146 | A | A |
| 147 | A | B |
| 148 | A | C |
| 149 | A | C |
| 150 | A | A |
| 151 | A | A |
| 152 | A | B |
| 153 | A | A |
| 154 | A | A |
| 155 | A | A |
| 156 | A | B |
| 157 | B | C |
| 158 | A | B |
| 159 | A | C |
| 160 | A | C |
| 161 | C | C |
| 162 | A | C |
| 163 | B | C |
| 164 | A | B |
| 165 | B | C |
| 166 | A | C |
| 167 | A | C |
| 168 | A | C |
| 169 | A | C |
| 170 | B | C |
| 171 | A | C |
| 172 | B | C |
| 173 | B | C |
| 174 | A | C |
| 175 | B | C |
| 176 | A | A |
| 177 | A | A |
| 178 | C | C |
| 179 | C | C |
| 180 | B | C |
| 181 | B | C |
| 182 | B | C |
| 183 | A | C |
| 184 | A | C |
| 185 | A | C |
| 186 | B | C |
| 187 | A | C |
| 188 | B | C |
| 189 | B | C |
| 190 | B | C |
| 191 | A | C |
| 192 | B | C |
| 193 | B | C |
| 194 | B | C |
| 195 | A | C |
| 196 | B | C |
| 197 | A | C |
| 198 | B | C |
| 199 | A | C |
| 200 | A | C |
| 201 | C | C |
| 202 | A | C |
| 203 | A | C |
| 204 | A | C |
| 205 | A | C |
| 206 | A | C |
| 207 | A | C |
| 208 | C | C |
| 209 | A | C |
| 210 | A | C |
| 211 | C | C |
| 212 | A | C |
| 213 | C | C |
| 214 | B | C |
| 215 | A | C |
| 216 | B | C |
| 217 | B | C |
| 218 | B | C |
| 219 | A | C |
| 220 | B | C |
| 221 | B | C |
| 222 | A | C |
| 223 | C | C |
| 224 | B | C |
| 225 | B | C |
| 226 | C | C |
| 227 | A | C |
| 228 | C | C |
| 229 | B | C |
| 230 | A | B |
| 231 | B | C |
| 232 | B | C |
| 233 | A | B |
| 234 | B | C |
| 235 | A | C |
| 236 | A | C |
| 237 | A | C |
| 238 | B | C |
| 239 | B | C |
| 240 | B | C |
| 241 | A | C |
| 242 | B | C |
| 243 | A | C |
| 244 | A | C |
| 245 | C | C |
| 246 | C | C |
| 247 | A | B |
| 248 | A | B |
| 249 | C | C |
| 250 | A | C |
| 251 | A | C |
| 252 | B | C |
| 253 | A | C |
| 254 | B | C |
| 255 | B | C |
| 256 | A | C |
| 257 | A | C |
| 258 | A | B |
| 259 | A | B |
| 260 | A | B |
| 261 | A | B |
| 262 | A | C |
| 263 | A | B |
| 264 | A | A |
| 265 | A | B |
| 266 | A | B |
| 267 | C | C |
| 268 | A | B |
| 269 | A | A |
| 270 | A | A |
| 271 | A | B |
| 272 | C | C |
| 273 | A | A |
| 274 | C | C |
| 275 | A | A |
| 276 | A | A |
| 277 | B | C |
| 278 | B | C |
| 279 | C | C |
| 280 | A | B |
| 281 | A | C |
| 282 | A | B |
| 283 | A | B |
| 284 | A | B |
| 285 | A | B |
| 286 | A | B |
| 287 | A | B |
| 288 | A | B |
| 289 | C | C |
| 290 | A | B |
| 291 | A | B |
| 292 | A | A |
| 293 | A | B |
| 294 | A | C |

Accordingly, the compounds provided for and described herein can be used to mediate NIK activity and treat diseases and conditions associated with NIK, such as those described herein.

## Claims

1. A compound of Formula I: 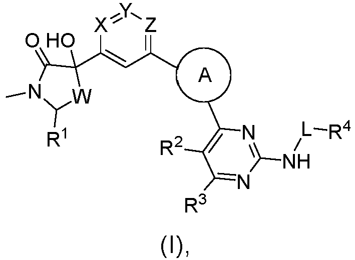 or a pharmaceutically acceptable salt thereof, wherein:
A is a 5- to 6-membered heteroaryl that is optionally substituted with one to three -C₍₁₋₄₎alkyl groups;
W is CH₂, CHF, or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein the -C₍₁₋₆₎alkyl, -C₍₃₋₁₀₎cycloalkyl, 3- to 10-membered heterocyclyl, 5- to 12-membered bi- or tricyclic ring system containing one or more heteroatoms, -C(O)-(3- to 10-membered heterocyclyl), -C₍₆₋₁₀₎aryl, and 5- to 10-membered heteroaryl are each optionally substituted with one to five R^{4x} groups;
each R^{4x}, independently for each occurrence, is halo, -OH, -N(R^{N1})(R^{N2}), -CN, - C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, - C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H, -C₍₁₋₃₎alkyl, or -C₍₁₋₃₎haloalkyl;
provided that at least one of X, Y, and Z are C-H.

2. The compound of claim 1, or a pharmaceutically acceptable salt thereof, wherein:
A is a 5- to 6-membered heteroaryl that is optionally substituted with one to three -C₍₁₋₄₎alkyl groups;
W is CH₂ or CF₂;
X is N, C-H, or C-R^{X};
Y is N, C-H, or C-R^{Y};
Z is N, C-H, or C-R^{Z};
R^{X}, R^{Y}, and R^{Z} are each, independently, halo, -CN, -C₍₁₋₄₎alkyl, or -C₍₁₋₄₎haloalkyl;
R¹ is hydrogen, -C₍₁₋₄₎alkyl, or -C₍₁₋₄₎haloalkyl;
R² is hydrogen, halo, -C₍₁₋₄₎alkyl, or -C₍₁₋₄₎haloalkyl;
R³ is hydrogen, halo, -C₍₁₋₄₎alkyl, or -C₍₁₋₄₎haloalkyl;
L is absent, -C₍₁₋₄₎alkylene or -C₍₃₋₆₎cycloalkylene, wherein the -C₍₁₋₄₎alkylene and -C₍₃₋₆₎cycloalkylene are optionally substituted with one to three groups selected from halo, -C₍₁₋₃₎alkyl, and -OC₍₁₋₃₎alkyl;
R⁴ is hydrogen, -C₍₁₋₆₎alkyl, -C₍₃₋₁₀₎cycloalkyl, 3- to 10-membered heterocyclyl, a 5- to 12-membered bi- or tricyclic ring system containing one or more heteroatoms, - C(O)-(3- to 10-membered heterocyclyl), -C₍₆₋₁₀₎aryl, or 5- to 10-membered heteroaryl, wherein: the -C₍₁₋₆₎alkyl is optionally substituted with one to five R^{4a} groups, the -C₍₃₋₁₀₎cycloalkyl is optionally substituted with one to five R^{4b} groups, the 3- to 10-membered heterocyclyl is optionally substituted with one to five R^{4c} groups, the 5- to 12-membered bi- or tricyclic ring system containing one or more heteroatoms is optionally substituted with one to five R^{4d} groups, the -C(O)-(3- to 10-membered heterocyclyl) is optionally substituted with one to five R^{4e} groups, the -C₍₆₋₁₀₎aryl is optionally substituted with one to five R^{4f} groups, and the 5- to 10-membered heteroaryl is optionally substituted with one to five R^{4g} groups;
each R^{4a} independently for each occurrence is halo, -OH, -N(R^{N1})(R^{N2}), -CN, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₆₋₁₀₎aryl, or 5- to 10-membered heteroaryl, wherein the -C₍₁₋₆₎haloalkyl, - C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₆₋₁₀₎aryl, and 5- to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, -OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
R^{4b}, R^{4c}, R^{4d}, and R^{4e} are each independently for each occurrence halo, -OH, - N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C(O)C₍₁₋₄₎alkyl, -C(O)N(R^{N1})(R^{N2}), -S(O)₂C₍₁₋₄₎alkyl, - N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl,-OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl;
R^{4f} and R^{4g} are each independently for each occurrence halo, -OH, -N(R^{N1})(R^{N2}), -CN, -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, -OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, -C(O)C₍₁₋₄₎alkyl, - C(O)N(R^{N1})(R^{N2}), -S(O)₂C₍₁₋₄₎alkyl, -N(H)S(O)₂C₍₁₋₄₎alkyl, 3- to 8-membered heterocyclyl, -C₍₁₋₃₎alkyl(3- to 8-membered heterocyclyl), -C₍₆₋₁₀₎aryl, or 5- to 10-membered heteroaryl, wherein the -C₍₁₋₆₎alkyl, -C₍₁₋₆₎haloalkyl, -C₍₃₋₈₎cycloalkyl, -OC₍₁₋₆₎alkyl, -OC₍₁₋₆₎haloalkyl, - OC₍₃₋₈₎cycloalkyl, -C₍₁₋₃₎alkylC₍₃₋₁₀₎cycloalkyl, -C₍₃₋₁₀₎cycloalkylC₍₁₋₃₎alkyl, 3- to 8-membered heterocyclyl, -C₍₁₋₃₎alkyl(3- to 8-membered heterocyclyl), -C₍₆₋₁₀₎aryl, and 5-to 10-membered heteroaryl are optionally further substituted with one to five groups selected from halo, -OH, -N(R^{N1})(R^{N2}), -CN, -C₍₁₋₄₎alkyl, -C₍₁₋₄₎haloalkyl, -OC₍₁₋₄₎alkyl, - OC₍₁₋₆₎haloalkyl, -C(O)C₍₁₋₄₎alkyl, -C(O)N(R^{N1})(R^{N2}), -S(O)₂C₍₁₋₄₎alkyl, and -N(H)S(O)₂C₍₁₋₄₎alkyl; and
R^{N1} and R^{N2} are each independently for each occurrence H or -C₍₁₋₃₎alkyl;
provided that at least one of X, Y, and Z are C-H.

3. The compound of any one of claims 1-2, or a pharmaceutically acceptable salt thereof, wherein A is: 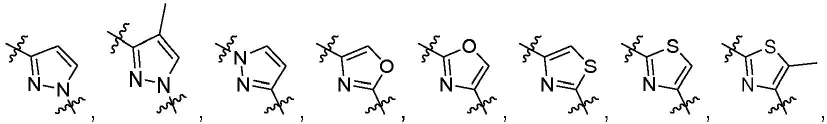 or

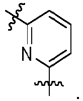

4. The compound of any one of claims 1-3, or a pharmaceutically acceptable salt thereof, wherein W is CH₂.

5. The compound of any one of claims 1-3, or a pharmaceutically acceptable salt thereof, wherein two of X, Y, and Z are C-H.

6. The compound of any one of claims 1-3, or a pharmaceutically acceptable salt thereof, wherein no more than one of X, Y, and Z is N.

7. The compound of any one of claims 1-6, or a pharmaceutically acceptable salt thereof, wherein R¹ is hydrogen or -CF₃.

8. The compound of any one of claims 1-7, or a pharmaceutically acceptable salt thereof, wherein R² and R³ are hydrogen.

9. The compound of any one of claims 1-8, or a pharmaceutically acceptable salt thereof, which is a compound of any one of Formulas If-1 to If-8:

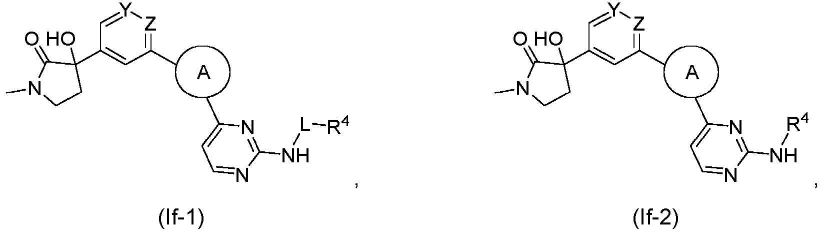

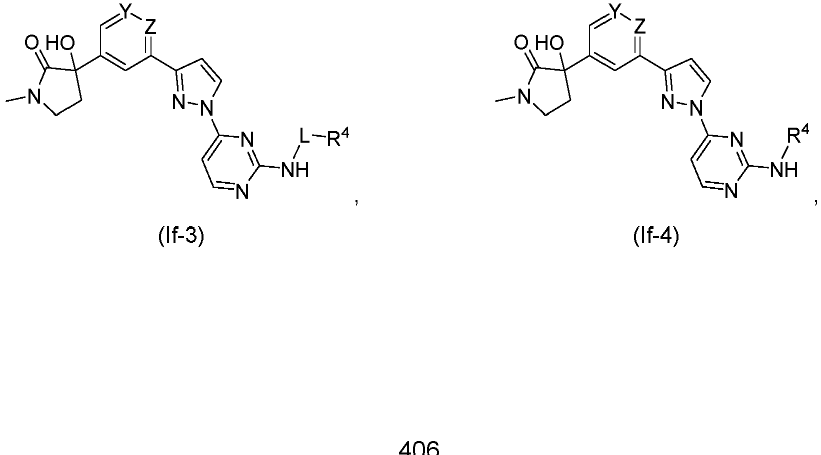

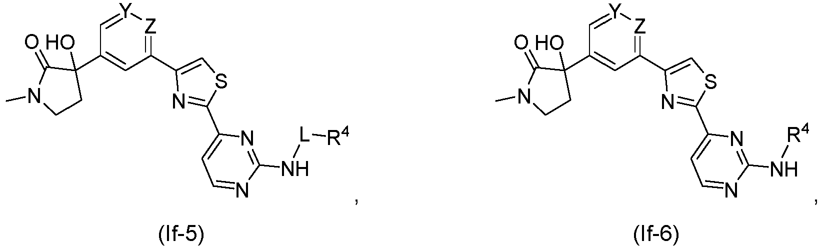

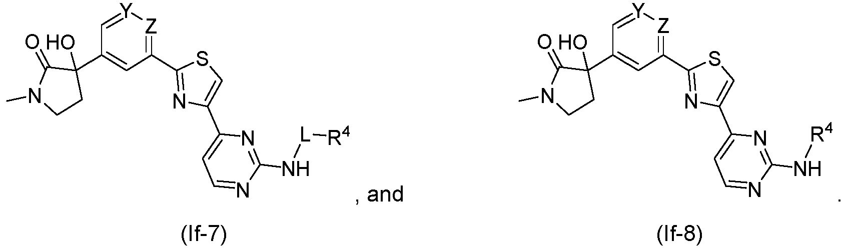

10. The compound of any one of claims 1-9, or a pharmaceutically acceptable salt thereof, wherein R⁴ is: 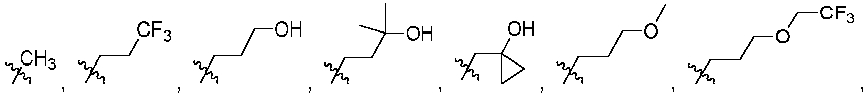 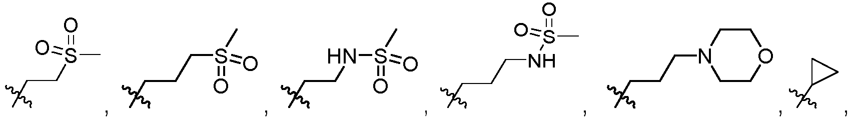 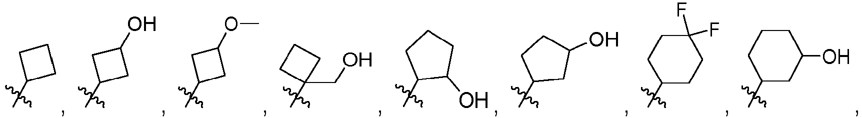 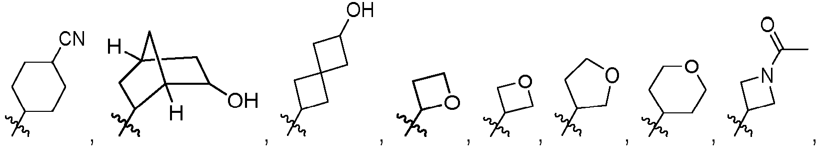 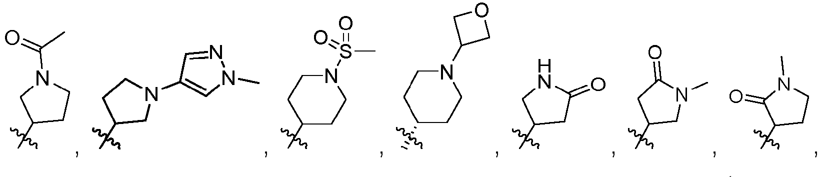 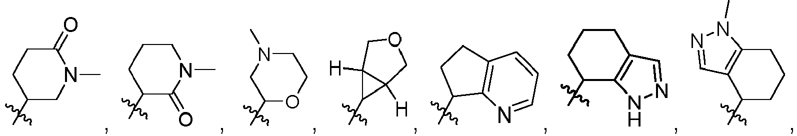 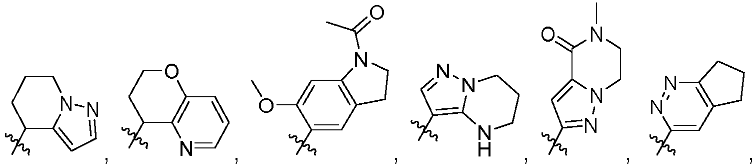 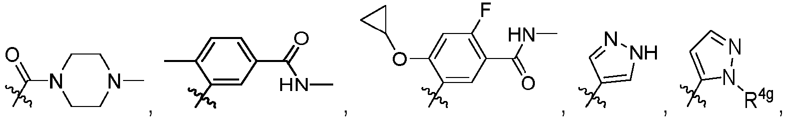 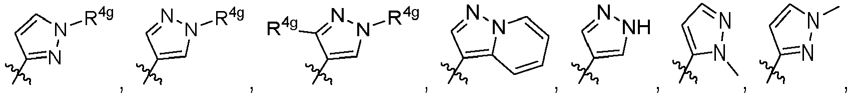 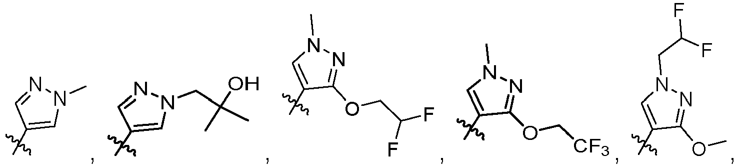 or

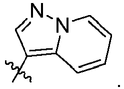

11. The compound of claim 1 or claim 2, or a pharmaceutically acceptable salt thereof, which is a compound of Formula If-1: 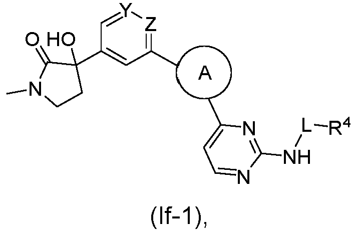
A is a pyrazolyl or thiazolyl;
Y is N, C-H, or C-F;
Z is N, C-H, or C-F;
L is absent or -C₍₁₋₄₎alkylene;
R⁴ is 5- to 10-membered heteroaryl, which is optionally substituted with one to five R^{4g} groups;
each R^{4g} independently for each occurrence is -C₍₁₋₆₎alkyl, -C₍₁₋₆₎haloalkyl, -OC₍₁₋₆₎alkyl, or -OC₍₁₋₆₎haloalkyl, each of which is optionally further substituted with one to five groups selected from halo and -OH;
provided that at least one of Y and Z is C-H.

12. The compound of claim 11, or a pharmaceutically acceptable salt thereof, having a structure selected from the group consisting of:

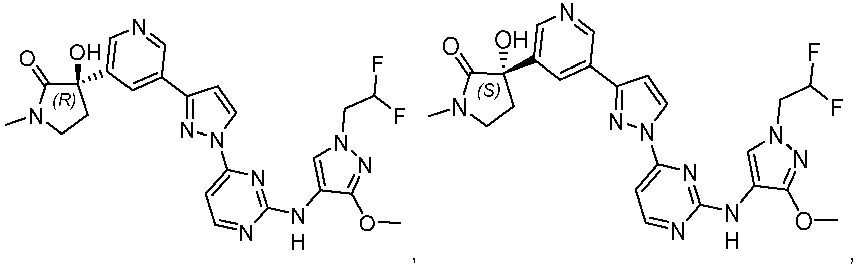

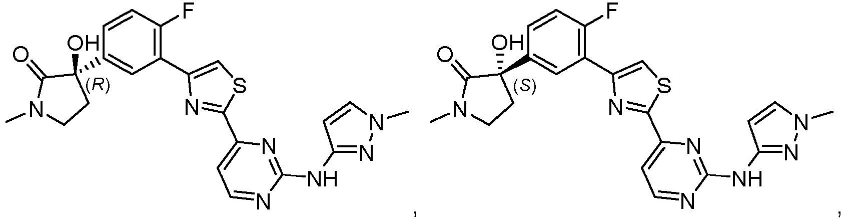

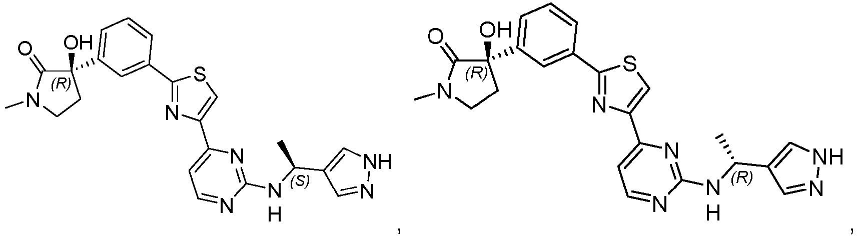

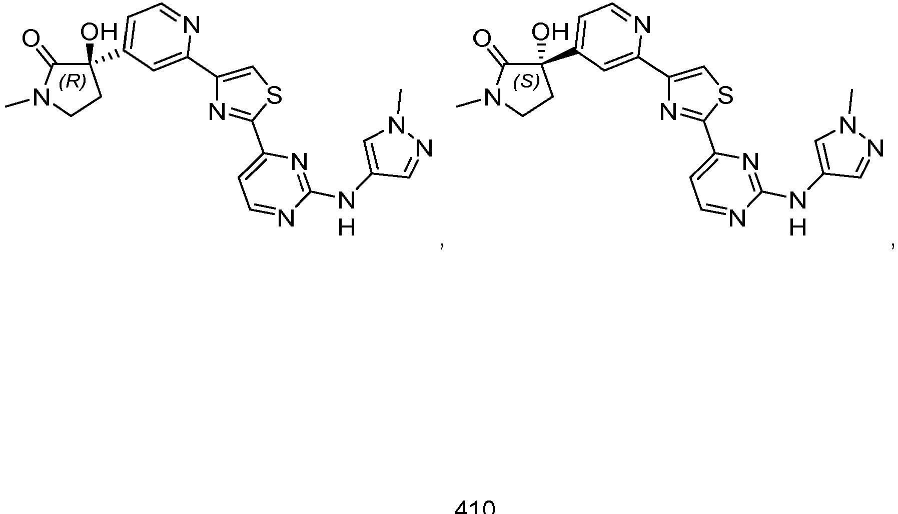

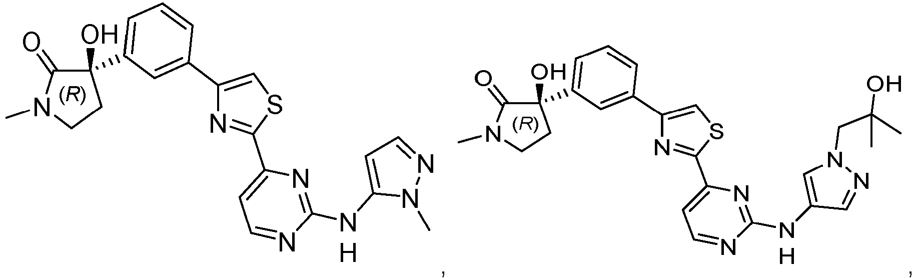

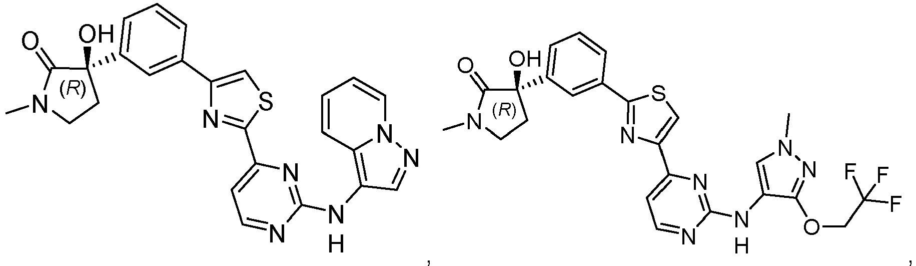

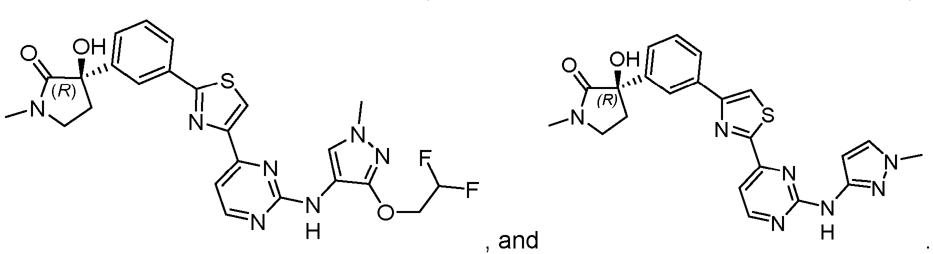

13. A pharmaceutical composition comprising a compound of any one of claims 1-12, or a pharmaceutically acceptable salt thereof, and a pharmaceutically acceptable carrier.

14. A compound of any one of claims 1-12, or a pharmaceutically acceptable carrier thereof, or a pharmaceutical composition of claim 13, for use in a method of treating a disease, disorder, or medical condition mediated by NIK activity, comprising administering to a subject in need of such treatment an effective amount of said compound or pharmaceutical composition.

15. The compound, pharmaceutically acceptable carrier thereof, or pharmaceutical composition for use according to claim 14, wherein the disease, disorder or medical condition mediated by NIK activity is selected from the group consisting of systemic lupus erythematosus ("SLE"), rheumatoid arthritis ("RA"), Sjogren's syndrome, lupus nephritis, inflammatory bowel disease ("IBD"), ANCA associated vasculitis, myositis, IgG4 associated diseases, bullous pemphigoid, neuromyelitis optica spectrum disorders ("NMOSD"), atopic dermatitis "AD"), hidradenitis supperativa ("HS"), steatosis, nonalcoholic steatohepatitis ("NASH"), primary biliary cirrhosis, leukemias, lymphomas, pancreatic cancer, breast cancer, melanoma, obesity, diabetes, acute kidney injury, IgAN, autosomal dominant polycystic kidney disease ("ADCKD"), membranous nephropathy, osteoporosis, bone resorption (periodontitis), multiple sclerosis ("MS"), immune thrombocytopenic purpura, transplantation, myasthenia gravis, scleroderma, myositis, IgG4 associated diseases, and bullous pemphigoid.

## Patentansprüche

1. Verbindung der Formel I: 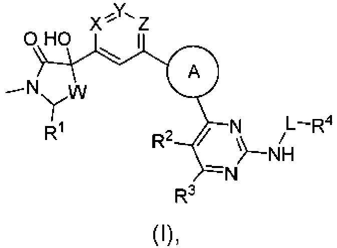 oder ein pharmazeutisch akzeptables Salz davon, wobei:
A ein 5- bis 6-gliedriges Heteroaryl ist, das gegebenenfalls mit einer bis drei -C₍₁₋₄₎-Alkylgruppen substituiert ist;
W CH₂, CHF oder CF₂ ist;
X N, C-H oder C-R^{X} ist;
Y N, C-H oder C-R^{Y} ist;
Z N, C-H oder C-R^{Z} ist;
R^{X}, R^{Y} und R^{Z} jeweils unabhängig Halogen, -CN, -C₍₁₋₄₎-Alkyl oder -C₍₁₋₄₎-Halogenalkyl sind;
R¹ Wasserstoff, -C₍₁₋₄₎-Alkyl oder -C₍₁₋₄₎-Halogenalkyl ist;
R² Wasserstoff, Halogen, -C₍₁₋₄₎-Alkyl oder -C₍₁₋₄₎-Halogenalkyl ist;
R³ Wasserstoff, Halogen, -C₍₁₋₄₎-Alkyl oder -C₍₁₋₄₎-Halogenalkyl ist;
L fehlt, -C₍₁₋₄₎-Alkylen oder -C₍₃₋₆₎-Cycloalkylen ist, wobei das -C₍₁₋₄₎-Alkylen und -C₍₃₋₆₎-Cycloalkylen gegebenenfalls mit einer bis drei Gruppen substituiert sind, die aus Halogen, -C₍₁₋₃₎-Alkyl und -OC₍₁₋₃₎-Alkyl ausgewählt sind;
R⁴ Wasserstoff, -C₍₁₋₆₎-Alkyl, -C₍₃₋₁₀₎-Cycloalkyl, 3- bis 10-gliedriges Heterocyclyl, ein 5- bis 12-gliedriges bi- oder tricyclisches Ringsystem, das ein oder mehrere Heteroatome enthält, -C(O)-(3- bis 10-gliedriges Heterocyclyl), -C₍₆₋₁₀₎-Aryl oder 5- bis 10-gliedriges Heteroaryl ist, wobei das -C₍₁₋₆₎-Alkyl, -C₍₃₋₁₀₎-Cycloalkyl, 3- bis 10-gliedrige Heterocyclyl, 5- bis 12-gliedrige bi- oder tricyclische Ringsystem, das ein oder mehrere Heteroatome enthält, -C(O)-(3- bis 10-gliedriges Heterocyclyl), -C₍₆₋₁₀₎-Aryl und 5- bis 10-gliedrige Heteroaryl jeweils gegebenenfalls mit einer bis fünf R^{4x}-Gruppen substituiert sind;
jedes R^{4x} unabhängig bei jedem Auftreten, Halogen, -OH, -N(R^{N1})(R^{N2}), -CN, -C₍₁₋₆₎-Alkyl, -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎₋-Cycloalkyl, -C₍₁₋₃₎-Alkyl-C₍₃₋₁₀₎-Cycloalkyl, -C₍₃₋₁₀₎-Cycloalkyl-C₍₁₋₃₎-Alkyl, -C(O)C₍₁₋₄₎-Alkyl, -C(O)N(R^{N1})(R^{N2}), -S(O)₂C₍₁₋₄₎-Alkyl, -N(H)S(O)₂C₍₁₋₄₎Alkyl, 3-bis 8-gliedriges Heterocyclyl, -C₍₁₋₃₎-Alkyl-(3- bis 8-gliedriges Heterocyclyl), -C₍₆₋₁₀₎-Aryl oder 5- bis 10-gliedriges Heteroaryl ist, wobei das -C₍₁₋₆₎-Alkyl, -C₍₁₋₆₎-Halogenalkyl, - C₍₃₋₈₎-Cycloalkyl, -OC₍₁₋₆₎-Alkyl, -OC₍₁₋₆₎-Halogenalkyl, -OC₍₃₋₈₎-Cycloalkyl, -C₍₁₋₃₎-Alkyl-C₍₃₋₁₀₎-Cycloalkyl, -C₍₃₋₁₀₎-Cycloalkyl-C₍₁₋₃₎-Alkyl, 3- bis 8-gliedriges Heterocyclyl, -C₍₁₋₃₎-Alkyl-(3- bis 8-gliedriges Heterocyclyl), -C₍₆₋₁₀₎-Aryl und 5- bis 10-gliedrige Heteroaryl gegebenenfalls weiter mit einer bis fünf Gruppen substituiert sind, die aus Halogen, - OH, -N(R^{N1})(R^{N2}), -CN, -C₍₁₋₄₎-Alkyl, -C₍₁₋₄₎-Halogenalkyl, -OC₍₁₋₄₎-Alkyl, -OC₍₁₋₆₎-Halogenalkyl, -C(O)C₍₁₋₄₎-Alkyl, -C(O)N(R^{N1})(R^{N2}), -S(O)₂C₍₁₋₄₎-Alkyl, und - N(H)S(O)₂C₍₁₋₄₎-Alkyl ausgewählt sind; und
R^{N1} und R^{N2} jeweils unabhängig bei jedem Auftreten H, -C₍₁₋₃₎-Alkyl oder - C₍₁₋₃₎-Halogenalkyl sind;
mit der Maßgabe, dass mindestens eines von X, Y und Z C-H ist.

2. Verbindung nach Anspruch 1 oder ein pharmazeutisch akzeptables Salz davon, wobei:
A ein 5- bis 6-gliedriges Heteroaryl ist, das gegebenenfalls mit einer bis drei -C₍₁₋₄₎-Alkylgruppen substituiert ist;
W CH₂ oder CF₂ ist;
X N, C-H oder C-R^{X} ist;
Y N, C-H oder C-R^{Y} ist;
Z N, C-H oder C-R^{Z} ist;
R^{X}, R^{Y} und R^{Z} jeweils unabhängig Halogen, -CN, -C₍₁₋₄₎-Alkyl oder -C₍₁₋₄₎-Halogenalkyl sind;
R¹ Wasserstoff, -C₍₁₋₄₎-Alkyl oder -C₍₁₋₄₎-Halogenalkyl ist;
R² Wasserstoff, Halogen, -C₍₁₋₄₎-Alkyl oder -C₍₁₋₄₎-Halogenalkyl ist;
R³ Wasserstoff, Halogen, -C₍₁₋₄₎-Alkyl oder -C₍₁₋₄₎-Halogenalkyl ist;
L fehlt, -C₍₁₋₄₎-Alkylen oder -C₍₃₋₆₎-Cycloalkylen ist, wobei das -C₍₁₋₄₎-Alkylen und -C₍₃₋₆₎-Cycloalkylen gegebenenfalls mit einer bis drei Gruppen substituiert sind, die aus Halogen, -C₍₁₋₃₎-Alkyl und -OC₍₁₋₃₎-Alkyl ausgewählt sind;
R⁴ Wasserstoff, -C₍₁₋₆₎-Alkyl, -C₍₃₋₁₀₎-Cycloalkyl, 3- bis 10-gliedriges Heterocyclyl, ein 5- bis 12-gliedriges bi- oder tricyclisches Ringsystem, das ein oder mehrere Heteroatome enthält, -C(O)-(3- bis 10-gliedriges Heterocyclyl), -C₍₆₋₁₀₎-Aryl oder 5- bis 10-gliedriges Heteroaryl ist, wobei: das -C₍₁₋₆₎-Alkyl gegebenenfalls mit einer bis fünf R^{4a}-Gruppen substituiert ist, das -C₍₃₋₁₀₎-Cycloalkyl gegebenenfalls mit einer bis fünf R^{4b}-Gruppen substituiert ist, das 3- bis 10-gliedrige Heterocyclyl gegebenenfalls mit einer bis fünf R^{4c}-Gruppen substituiert ist, das 5- bis 12-gliedrige bi- oder tricyclische Ringsystem, das ein oder mehrere Heteroatome enthält, gegebenenfalls mit einer bis fünf R^{4d}-Gruppen substituiert ist, das -C(O)-(3- bis 10-gliedrige Heterocyclyl) gegebenenfalls mit einer bis fünf R^{4e}-Gruppen substituiert ist, das -C₍₆₋₁₀₎-Aryl gegebenenfalls mit einer bis fünf R^{4f}-Gruppen substituiert ist, und das 5- bis 10-gliedrige Heteroaryl gegebenenfalls mit einer bis fünf R^{4g}-Gruppen substituiert ist;
jedes R^{4a} unabhängig bei jedem Auftreten Halogen, -OH, -N(R^{N1})(R^{N2}), - CN, -C₍₁₋₆₎-Halogenalkyl, -C₍₃₋₈₎-Cycloalkyl, -OC₍₁₋₆₎-Alkyl, -OC₍₁₋₆₎-Halogenalkyl, - OC₍₃₋₈₎-Cycloalkyl, -C(O)C₍₁₋₄₎-Alkyl, -C(O)N(R^{N1})(R^{N2}), -S(O)₂C₍₁₋₄₎-Alkyl, - N(H)S(O)₂C₍₁₋₄₎-Alkyl, 3- bis 8-gliedriges Heterocyclyl, -C₍₆₋₁₀₎-Aryl oder 5- bis 10-gliedriges Heteroaryl ist, wobei das -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎-Cycloalkyl, 3- bis 8-gliedrige Heterocyclyl, - C₍₆₋₁₀₎-Aryl und 5- bis 10-gliedrige Heteroaryl gegebenenfalls weiter mit einer bis fünf Gruppen substituiert sind, die aus Halogen, -OH, -N(R^{N1})(R^{N2}), -CN, -C₍₁₋₄₎-Alkyl, -C₍₁₋₄₎-Halogenalkyl, -OC₍₁₋₄₎-Alkyl, -OC₍₁₋₆₎-Halogenalkyl, -C(O)C₍₁₋₄₎-Alkyl, - C(O)N(R^{N1})(R^{N2}), -S(O)₂C₍₁₋₄₎-Alkyl und -N(H)S(O)₂C₍₁₋₄₎-Alkyl ausgewählt sind;
R^{4b}, R^{4c}, R^{4d}, und R^{4e} jeweils unabhängig bei jedem Auftreten Halogen, - OH, -N(R^{N1})(R^{N2}), -CN, -C₍₁₋₆₎-Alkyl, -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎-Cycloalkyl, -C(O)C₍₁₋₄₎-Alkyl, - C(O)N(R^{N1})(R^{N2}), -S(O)₂C₍₁₋₄₎-Alkyl, -N(H)S(O)₂C₍₁₋₄₎-Alkyl, 3- bis 8-gliedriges Heterocyclyl, -C₍₁₋₃₎-Alkyl-(3- bis 8-gliedriges Heterocyclyl), -C₍₆₋₁₀₎-Aryl oder 5- bis 10-gliedriges Heteroaryl sind, wobei das -C₍₁₋₆₎-Alkyl, -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎-Cycloalkyl, 3- bis 8-gliedrige Heterocyclyl, -C₍₁₋₃₎-Alkyl-(3- bis 8-gliedriges Heterocyclyl), -C₍₆₋₁₀₎-Aryl und 5- bis 10-gliedrige Heteroaryl gegebenenfalls weiter mit einer bis fünf Gruppen substituiert sind, die aus Halogen, -OH, -N(R^{N1})(R^{N2}), -CN, -C₍₁₋₄₎-Alkyl, -C₍₁₋₄₎-Halogenalkyl, -OC₍₁₋₄₎-Alkyl, -OC₍₁₋₆₎-Halogenalkyl, -C(O)C₍₁₋₄₎-Alkyl, - C(O)N(R^{N1})(R^{N2}), -S(O)₂C₍₁₋₄₎-Alkyl, und-N(H)S(O)₂C₍₁₋₄₎-Alkyl ausgewählt sind;
R^{4f} und R^{4g} jeweils unabhängig bei jedem Auftreten Halogen, -OH, - N(R^{N1})(R^{N2}), -CN, -C₍₁₋₆₎-Alkyl, -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎-Cycloalkyl, -C₍₁₋₃₎-Alkyl-C₍₃₋₁₀₎-Cycloalkyl, -C₍₃₋₁₀₎-Cycloalkyl-C₍₁₋₃₎-Alkyl, -C(O)C₍₁₋₄₎-Alkyl, -C(O)N(R^{N1})(R^{N2}), -S(O)₂C₍₁₋₄₎-Alkyl, - N(H)S(O)₂C₍₁₋₄₎-Alkyl, 3- bis 8-gliedriges Heterocyclyl, -C₍₁₋₃₎-Alkyl-(3- bis 8-gliedriges Heterocyclyl), -C₍₆₋₁₀₎-Aryl oder 5- bis 10-gliedriges Heteroaryl sind, wobei das -C₍₁₋₆₎-Alkyl, -C₍₁₋₆₎-Halogenalkyl, -C₍₃₋₈₎-Cycloalkyl, -OC₍₁₋₆₎-Alkyl, -OC₍₁₋₆₎-Halogenalkyl, -OC₍₃₋₈₎-Cycloalkyl, -C₍₁₋₃₎-Alkyl-C₍₃₋₁₀₎-Cycloalkyl, -C₍₃₋₁₀₎-Cycloalkyl-C₍₁₋₃₎-Alkyl, 3- bis 8-gliedrige Heterocyclyl, -C₍₁₋₃₎-Alkyl-(3- bis 8-gliedriges Heterocyclyl), -C₍₆₋₁₀₎-Aryl und 5- bis 10-gliedrige Heteroaryl gegebenenfalls weiter mit einer bis fünf Gruppen substituiert sind, die aus Halogen, -OH, -N(R^{N1})(R^{N2}), -CN, -C₍₁₋₄₎-Alkyl, -C₍₁₋₄₎-Halogenalkyl, -OC₍₁₋₄₎-Alkyl, - OC₍₁₋₆₎-Halogenalkyl, -C(O)C₍₁₋₄₎-Alkyl, -C(O)N(R^{N1})(R^{N2}), -S(O)₂C₍₁₋₄₎-Alkyl, und-N(H)S(O)₂C₍₁₋₄)-Alkyl ausgewählt sind; und
R^{N1} und R^{N2} jeweils unabhängig bei jedem Auftreten H oder -C₍₁₋₃₎-Alkyl sind; mit der Maßgabe, dass mindestens eines von X, Y und Z C-H ist.

3. Verbindung nach einem der Ansprüche 1 bis 2 oder ein pharmazeutisch akzeptables Salz davon, wobei A ist: 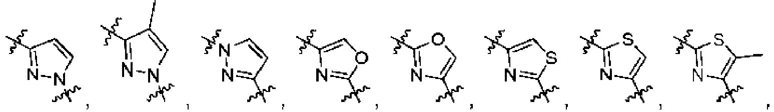 oder

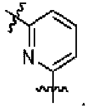

4. Verbindung nach einem der Ansprüche 1 bis 3 oder ein pharmazeutisch akzeptables Salz davon, wobei W CH₂ ist.

5. Verbindung nach einem der Ansprüche 1 bis 3 oder ein pharmazeutisch akzeptables Salz davon, wobei zwei von X, Y, und Z C-H sind.

6. Verbindung nach einem der Ansprüche 1 bis 3 oder ein pharmazeutisch akzeptables Salz davon, wobei nicht mehr als eines von X, Y und Z N ist.

7. Verbindung nach einem der Ansprüche 1 bis 6 oder ein pharmazeutisch akzeptables Salz davon, wobei R¹ Wasserstoff oder -CF₃ ist.

8. Verbindung nach einem der Ansprüche 1 bis 7 oder ein pharmazeutisch akzeptables Salz davon, wobei R² und R³ Wasserstoff sind.

9. Verbindung nach einem der Ansprüche 1 bis 8 oder ein pharmazeutisch akzeptables Salz davon, das eine Verbindung einer beliebigen der Formeln If-1 bis If-8 ist:

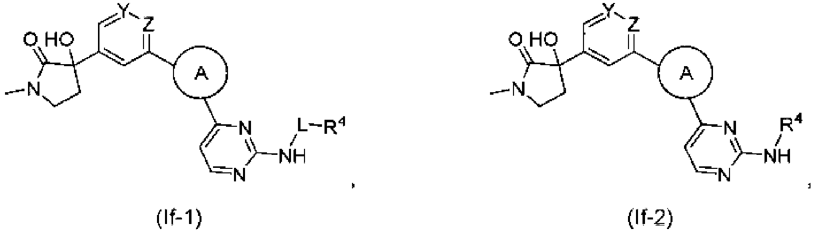

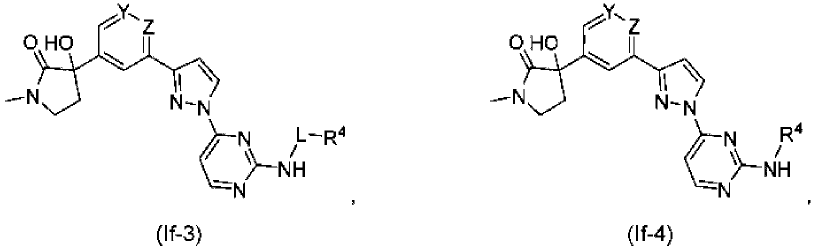

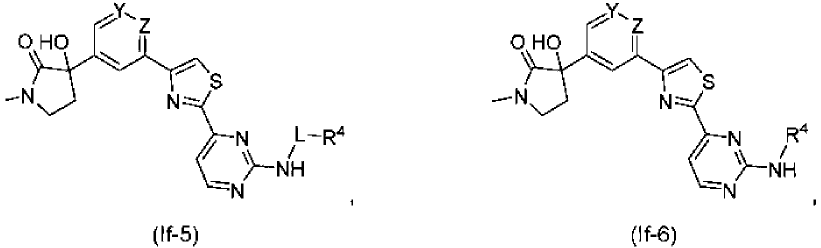

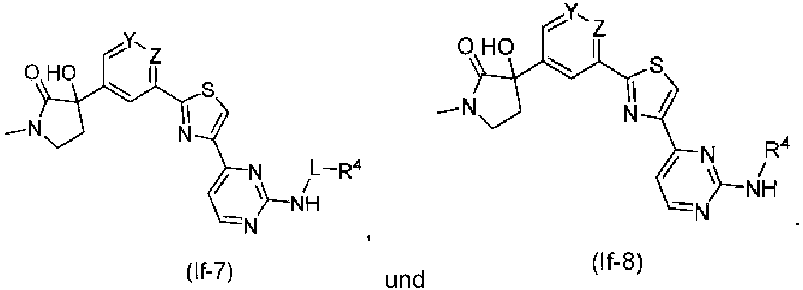

10. Verbindung nach einem der Ansprüche 1 bis 9 oder ein pharmazeutisch akzeptables Salz davon, wobei R⁴ ist:

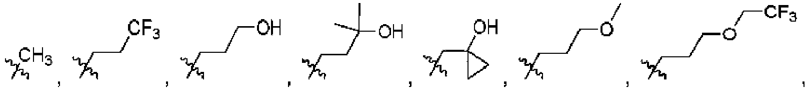

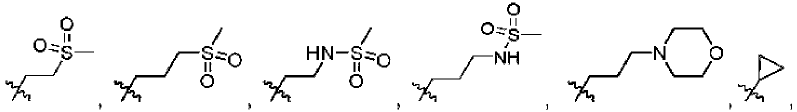

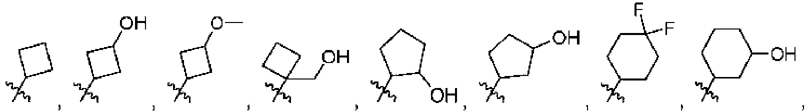

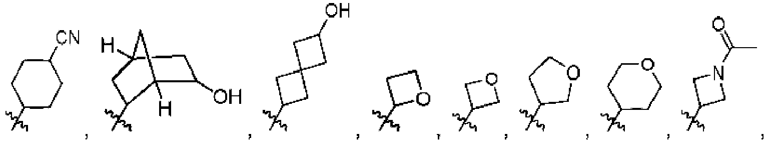

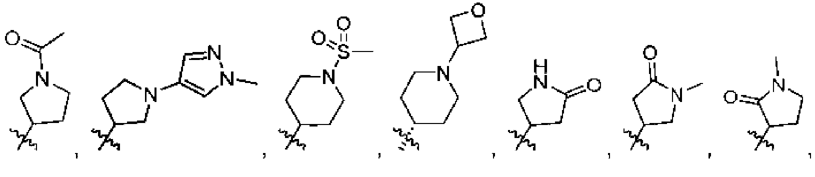

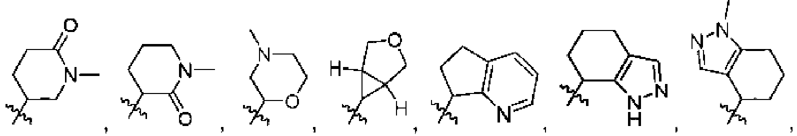

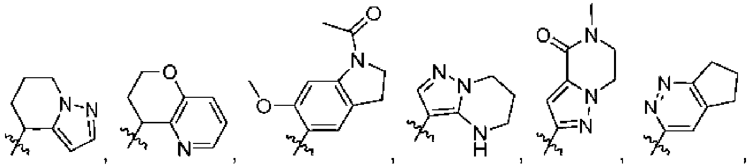

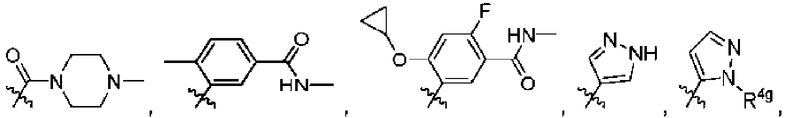

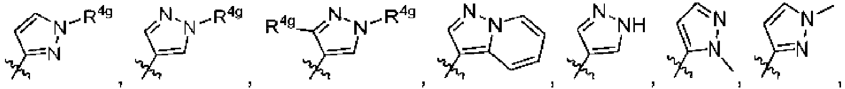

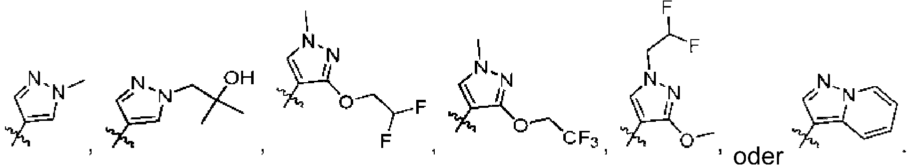

11. Verbindung nach Anspruch 1 oder 2 oder ein pharmazeutisch akzeptables Salz davon, das eine Verbindung der Formel If-1 ist: 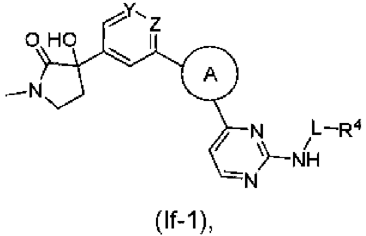
A ist ein Pyrazolyl oder Thiazolyl;
Y ist N, C-H oder C-F;
Z ist N, C-H oder C-F;
L fehlt oder ist -C₍₁₋₄₎-Alkylen;
R⁴ ist 5- bis 10-gliedriges Heteroaryl, das gegebenenfalls mit einer bis fünf R^{4g}-Gruppen substituiert ist;
jedes R^{4g} für jedes Auftreten ist unabhängig -C₍₁₋₆₎-Alkyl, -C₍₁₋₆₎-Halogenalkyl, -OC₍₁₋₆₎-Alkyl oder -OC₍₁₋₆₎-Halogenalkyl, von denen jedes gegebenenfalls weiter mit einer bis fünf Gruppen substituiert ist, die aus Halogen und -OH ausgewählt sind;
mit der Maßgabe, dass mindestens eines von Y und Z C-H ist.

12. Verbindung nach Anspruch 11 oder ein pharmazeutisch akzeptables Salz davon, die/das eine Struktur auswählt, die aus der Gruppe ausgewählt ist, bestehend aus:

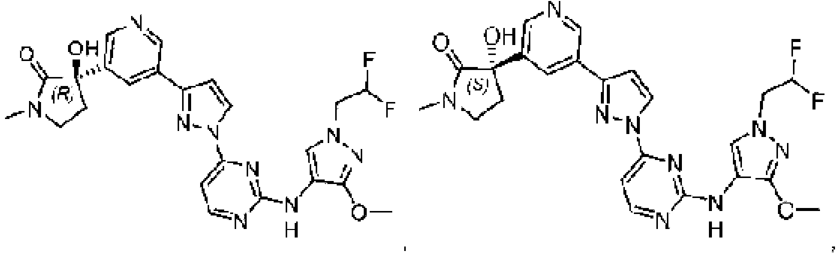

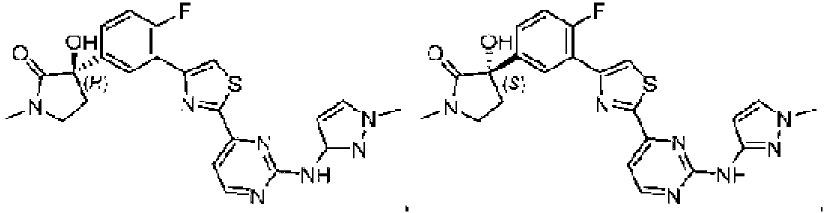

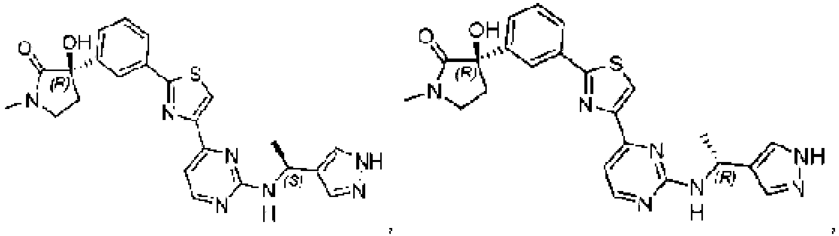

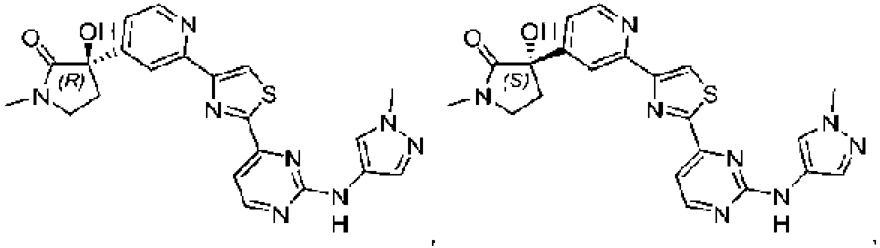

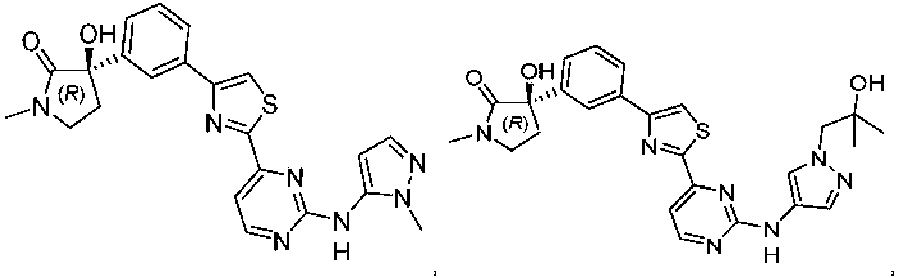

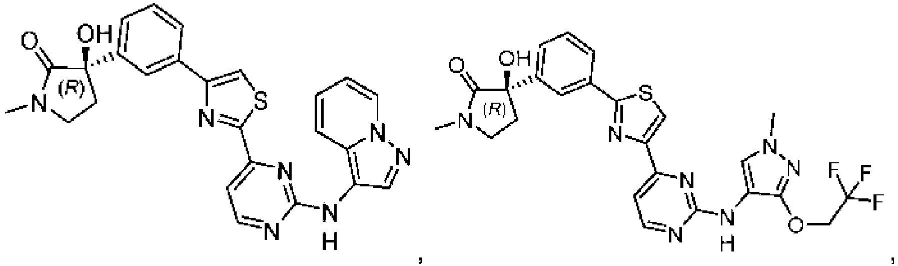

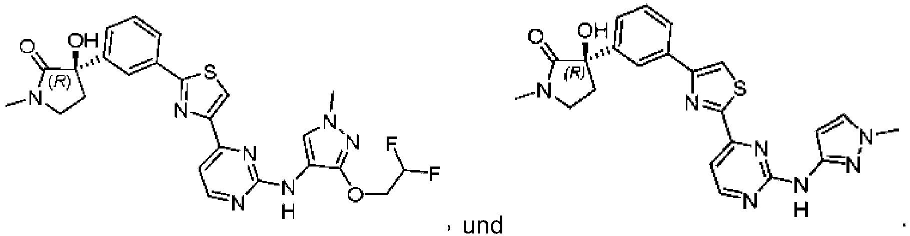

13. Pharmazeutische Zusammensetzung, umfassend eine Verbindung nach einem der Ansprüche 1 bis 12 oder ein pharmazeutisch akzeptables Salz davon und einen pharmazeutisch akzeptablen Träger.

14. Verbindung nach einem der Ansprüche 1 bis 12 oder ein pharmazeutisch akzeptabler Träger davon oder eine pharmazeutische Zusammensetzung nach Anspruch 13 zur Verwendung in einem Verfahren zum Behandeln einer Erkrankung, Störung oder eines medizinischen Zustands, die/der durch NIK-Aktivität herbeigeführt wird, umfassend ein Verabreichen einer wirksamen Menge der Verbindung oder pharmazeutischen Zusammensetzung an ein Subjekt, das einer solchen Behandlung bedarf.

15. Verbindung, der pharmazeutisch akzeptable Träger davon oder die pharmazeutische Zusammensetzung zur Verwendung nach Anspruch 14, wobei die Erkrankung, Störung oder der medizinische Zustand, die/der durch NIK-Aktivität herbeigeführt wird, aus der Gruppe ausgewählt ist, bestehend aus systemischem Lupus erythematodes ("SLE"), rheumatoider Arthritis ("RA"), Sjögren-Syndrom, Lupus-Nephritis, entzündlicher Darmerkrankung ("IBD"), ANCAassoziierter Vaskulitis, Myositis, IgG4-assoziierten Erkrankungen, bullösem Pemphigoid, Neuromyelitis-optica-Spektrum-Erkrankungen ("NMOSD"), atopischer Dermatitis ("AD"), Hidradenitis suppurativa ("HS"), Steatose, nichtalkoholischer Steatohepatitis ("NASH"), primär biliärer Zirrhose, Leukämien, Lymphomen, Pankreaskrebs, Brustkrebs, Melanom, Adipositas, Diabetes, akuter Nierenschädigung, IgAN, autosomal-dominanter polyzystischer Nierenerkrankung ("ADCKD"), membranöser Nephropathie, Osteoporose, Knochenresorption (Parodontitis), Multipler Sklerose ("MS"), immunthrombozytopenischer Purpura, Transplantation, Myasthenia gravis, Sklerodermie, Myositis, IgG4-assoziierten Erkrankungen und bullösem Pemphigoid.

## Revendications

1. Composé de Formule I : 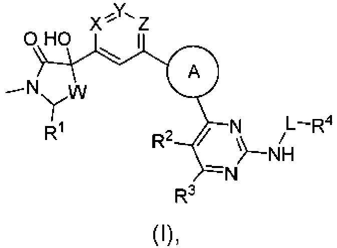 ou un sel pharmaceutiquement acceptable de celui-ci, dans lequel :
A est un hétéroaryle à 5 à 6 chaînons qui est facultativement substitué par un à trois groupes -C₍₁₋₄₎alkyle ;
W est CH₂, CHF, ou CF₂ ;
X est N, C-H, ou C-R^{X} ;
Y est N, C-H, ou C-R^{Y} ;
Z est N, C-H, ou C-R^{Z} ;
R^{X}, R^{Y}, et R^{Z} sont chacun, indépendamment, halo, -CN, -C₍₁₋₄₎alkyle, ou-C₍₁₋₄₎haloalkyle ;
R¹ est hydrogène, -C₍₁₋₄₎alkyle ou -C₍₁₋₄₎haloalkyle ;
R² est hydrogène, halo, -C₍₁₋₄₎alkyle, ou -C₍₁₋₄₎haloalkyle ;
R³ est hydrogène, halo, -C₍₁₋₄₎alkyle, ou -C₍₁₋₄₎haloalkyle ;
L est absent, -C₍₁₋₄₎alkylène ou -C₍₃₋₆₎cycloalkylène, dans lequel les -C₍₁₋₄₎alkylène et -C₍₃₋₆₎cycloalkylène sont facultativement substitués par un à trois groupes choisis parmi halo, -C₍₁₋₃₎alkyle, et -OC₍₁₋₃₎alkyle ;
R⁴ est hydrogène, -C₍₁₋₆₎alkyle, -C₍₃₋₁₀₎cycloalkyle, hétérocyclyle à 3 à 10 chaînons, un système cyclique bi- ou tricyclique à 5 à 12 chaînons contenant un ou plusieurs hétéroatomes, -C(O)-(hétérocyclyle à 3 à 10 chaînons), -C₍₆₋₁₀₎aryle, ou hétéroaryle à 5 à 10 chaînons, dans lequel les -C₍₁₋₆₎alkyle, -C₍₃₋₁₀₎cycloalkyle, hétérocyclyle à 3 à 10 chaînons, système cyclique bi- ou tricyclique à 5 à 12 chaînons contenant un ou plusieurs hétéroatomes, -C(O)-(hétérocyclyle à 3 à 10 chaînons), -C₍₆₋₁₀₎aryle, et hétéroaryle à 5 à 10 chaînons, sont chacun facultativement substitués par un à cinq groupes R^{4x} ;
chaque R^{4x}, indépendamment pour chaque occurrence, est halo, -OH, - N(R^{N1})(R^{N2}), -CN, -C₍₁₋₆₎alkyle, -C₍₁₋₆₎haloalkyle, -C₍₃₋₈₎cycloalkyle, -OC₍₁₋₆₎alkyle, - OC₍₁₋₆₎haloalkyle, -OC₍₃₋₈₎cycloalkyle, -C₍₁₋₃₎alkylC₍₃₋₁₀₎cycloalkyle, -C₍₃₋₁₀₎cycloalkylC₍₁₋₃₎alkyle, -C(O)C₍₁₋₄₎alkyle, -C(O)N(R^{N1})(R^{N2}), -S(O)₂C₍₁₋₄₎alkyle, - N(H)S(O)₂C₍₁₋₄₎alkyle, hétérocyclyle à 3 à 8 chaînons, -C₍₁₋₃₎alkyl(hétérocyclyle à 3 à 8 chaînons), -C₍₆₋₁₀₎aryle, ou hétéroaryle à 5 à 10 chaînons, dans lequel les -C₍₁₋₆₎alkyle, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, -C₍₁₋₃₎alkylC₍₃₋₁₀₎cycloalkyle, -C₍₃₋₁₀₎cycloalkylC₍₁₋₃₎alkyle, hétérocyclyle à 3 à 8 chaînons, -C₍₁₋₃₎alkyl(hétérocyclyle à 3 à 8 chaînons), -C₍₆₋₁₀₎aryle, et hétéroaryle à 5 à 10 chaînons sont facultativement substitués par un à cinq groupes choisis parmi halo, -OH, -N(R^{N1})(R^{N2}), -CN, -C₍₁₋₄₎alkyle, -C₍₁₋₄₎haloalkyle, -OC₍₁₋₄₎alkyle, -OC₍₁₋₆₎haloalkyle, -C(O)C₍₁₋₄₎alkyle, -C(O)N(R^{N1})(R^{N2}), -S(O)₂C₍₁₋₄₎alkyle, et -N(H)S(O)₂C₍₁₋₄₎alkyle ; et
R^{N1} et R^{N2} sont chacun, indépendamment pour chaque occurrence, H, - C₍₁₋₃₎alkyle, ou -C₍₁₋₃₎haloalkyle ;
à condition qu'au moins l'un parmi X, Y, et Z soit C-H.

2. Composé selon la revendication 1, ou un sel pharmaceutiquement acceptable de celui-ci, dans lequel :
A est un hétéroaryle à 5 à 6 chaînons qui est facultativement substitué par un à trois groupes -C₍₁₋₄₎alkyle ;
W est CH₂ ou CF₂ ;
X est N, C-H, ou C-R^{X} ;
Y est N, C-H, ou C-R^{Y} ;
Z est N, C-H, ou C-R^{Z} ;
R^{X}, R^{Y}, et R^{Z} sont chacun, indépendamment, halo, -CN, -C₍₁₋₄₎alkyle, ou-C₍₁₋₄₎haloalkyle ;
R¹ est hydrogène, -C₍₁₋₄₎alkyle, ou -C₍₁₋₄₎haloalkyle ;
R² est hydrogène, halo, -C₍₁₋₄₎alkyle, ou -C₍₁₋₄₎haloalkyle ;
R³ est hydrogène, halo, -C₍₁₋₄₎alkyle, ou -C₍₁₋₄₎haloalkyle ;
L est absent, -C₍₁₋₄₎alkylène ou -C₍₃₋₆₎cycloalkylène, dans lequel les -C₍₁₋₄₎alkylène et -C₍₃₋₆₎cycloalkylène sont facultativement substitués par un à trois groupes choisis parmi halo, -C₍₁₋₃₎alkyle, et -OC₍₁₋₃₎alkyle ;
R⁴ est hydrogène, -C₍₁₋₆₎alkyle, -C₍₃₋₁₀₎cycloalkyle, hétérocyclyle à 3 à 10 chaînons, un système cyclique bi- ou tricyclique à 5 à 12 chaînons contenant un ou plusieurs hétéroatomes, -C(O)-(hétérocyclyle à 3 à 10 chaînons), -C₍₆₋₁₀₎aryle, ou hétéroaryle à 5 à 10 chaînons, dans lequel : le -C₍₁₋₆₎alkyle est facultativement substitué par un à cinq groupes R^{4a}, le -C₍₃₋₁₀₎cycloalkyle est facultativement substitué par un à cinq groupes R^{4b}, l'hétérocyclyle à 3 à 10 chaînons est facultativement substitué par un à cinq groupes R^{4c}, le système cyclique bi- ou tricyclique à 5 à 12 chaînons contenant un ou plusieurs hétéroatomes est facultativement substitué par un à cinq groupes R^{4d}, le -C(O)-(hétérocyclyle à 3 à 10 chaînons) est facultativement substitué par un à cinq groupes R^{4e}, le -C₍₆₋₁₀₎aryle est facultativement substitué par un à cinq groupes R^{4f}, et l'hétéroaryle à 5 à 10 chaînons est facultativement substitué par un à cinq groupes R^{4g} ;
chaque R^{4a}, indépendamment pour chaque occurrence, est halo, -OH, - N(R^{N1})(R^{N2}), -CN, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, -C(O)C₍₁₋₄₎alkyle, -C(O)N(R^{N1})(R^{N2}), -S(O)₂C₍₁₋₄₎alkyle, -N(H)S(O)₂C₍₁₋₄₎alkyle, hétérocyclyle à 3 à 8 chaînons, -C₍₆₋₁₀₎aryle, ou un hétéroaryle à 5 à 10 chaînons, dans lequel les -C₍₁₋₆₎haloalkyle, -C₍₃₋₈₎cycloalkyle, - OC₍₁₋₆₎alkyle, -OC₍₁₋₆₎haloalkyle, -OC₍₃₋₈₎cycloalkyle, hétérocyclyle à 3 à 8 chaînons, -C₍₆₋₁₀₎aryle, et hétéroaryle à 5 à 10 chaînons sont facultativement substitués par un à cinq groupes choisis parmi halo, -OH, -N(R^{N1})(R^{N2}), -CN, -C₍₁₋₄₎alkyle, -C₍₁₋₄₎haloalkyle, -OC₍₁₋₄₎alkyle, -OC₍₁₋₆₎haloalkyle, -C(O)C₍₁₋₄₎alkyle, - C(O)N(R^{N1})(R^{N2}), -S(O)₂C₍₁₋₄₎alkyle, et -N(H)S(O)₂C₍₁₋₄₎alkyle ;
R^{4b}, R^{4c}, R^{4d}, et R^{4e} sont chacun, indépendamment pour chaque occurrence, halo, -OH, -N(R^{N1})(R^{N2}), -CN, -C₍₁₋₆₎alkyle, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, -C(O)C₍₁₋₄₎alkyle, -C(O)N(R^{N1})(R^{N2}), -S(O)₂C₍₁₋₄₎alkyle, -N(H)S(O)₂C₍₁₋₄₎alkyle, hétérocyclyle à 3 à 8 chaînons, -C₍₁₋₃₎alkyl(hétérocyclyle à 3 à 8 chaînons), -C₍₆₋₁₀₎aryle, ou hétéroaryle à 5 à 10 chaînons, dans lequel les -C₍₁₋₆₎alkyle, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, hétérocyclyle à 3 à 8 chaînons, -C₍₁₋₃₎alkyl(hétérocyclyle à 3 à 8 chaînons), -C₍₆₋₁₀₎aryle, et hétéroaryle à 5 à 10 chaînons sont facultativement substitués par un à cinq groupes choisis parmi halo, -OH, -N(R^{N1})(R^{N2}), -CN, -C₍₁₋₄₎alkyle, -C₍₁₋₄₎haloalkyle, -OC₍₁₋₄₎alkyle, -OC₍₁₋₆₎haloalkyle, -C(O)C₍₁₋₄₎alkyle, -C(O)N(R^{N1})(R^{N2}), -S(O)₂C₍₁₋₄₎alkyle, et -N(H)S(O)₂C₍₁₋₄₎alkyle ;
R^{4f} et R^{4g} sont chacun, indépendamment pour chaque occurrence, halo, - OH, -N(R^{N1})(R^{N2}), -CN, -C₍₁₋₆₎alkyle, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, -C₍₁₋₃₎alkylC₍₃₋₁₀₎cycloalkyle, -C₍₃₋₁₀₎cycloalkylC₍₁₋₃₎alkyle, -C(O)C₍₁₋₄₎alkyle, -C(O)N(R^{N1})(R^{N2}), -S(O)₂C₍₁₋₄₎alkyle, - N(H)S(O)₂C₍₁₋₄₎alkyle, hétérocyclyle à 3 à 8 chaînons, -C₍₁₋₃₎alkyl(hétérocyclyle à 3 à 8 chaînons), -C₍₆₋₁₀₎aryle, ou hétéroaryle à 5 à 10 chaînons, dans lequel les -C₍₁₋₆₎alkyle, -C₍₁₋₆₎haloalkyle, -C₍₃₋₈₎cycloalkyle, -OC₍₁₋₆₎alkyle, -OC₍₁₋₆₎haloalkyle, -OC₍₃₋₈₎cycloalkyle, -C₍₁₋₃₎alkylC₍₃₋₁₀₎cycloalkyle, -C₍₃₋₁₀₎cycloalkylC₍₁₋₃₎alkyle, hétérocyclyle à 3 à 8 chaînons, -C₍₁₋₃₎alkyl(hétérocyclyle à 3 à 8 chaînons), -C₍₆₋₁₀₎aryle, et hétéroaryle 5 à 10 chaînons sont facultativement substitués par un à cinq groupes choisis parmi halo, -OH, -N(R^{N1})(R^{N2}), -CN, -C₍₁₋₄₎alkyle, -C₍₁₋₄₎haloalkyle, -OC₍₁₋₄₎alkyle, -OC₍₁₋₆₎haloalkyle, -C(O)C₍₁₋₄₎alkyle, -C(O)N(R^{N1})(R^{N2}), -S(O)₂C₍₁₋₄₎alkyle, et -N(H)S(O)₂C₍₁₋₄₎alkyle ; et
R^{N1} et R^{N2} sont chacun, indépendamment pour chaque occurrence, H ou - C₍₁₋₃₎alkyle ; à condition qu'au moins l'un parmi X, Y, et Z soit C-H.

3. Composé selon l'une quelconque des revendications 1 à 2, ou un sel pharmaceutiquement acceptable de celui-ci, dans lequel A est :

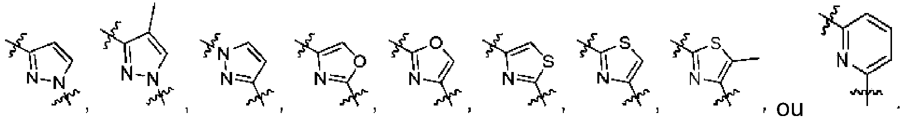

4. Composé selon l'une quelconque des revendications 1 à 3, ou un sel pharmaceutiquement acceptable de celui-ci, dans lequel W est CH₂.

5. Composé selon l'une quelconque des revendications 1 à 3, ou un sel pharmaceutiquement acceptable de celui-ci, dans lequel deux parmi X, Y, et Z sont C-H.

6. Composé selon l'une quelconque des revendications 1 à 3, ou un sel pharmaceutiquement acceptable de celui-ci, dans lequel pas plus d'un parmi X, Y, et Z n'est N.

7. Composé selon l'une quelconque des revendications 1 à 6, ou un sel pharmaceutiquement acceptable de celui-ci, dans lequel R¹ est hydrogène ou -CF₃.

8. Composé selon l'une quelconque des revendications 1 à 7, ou un sel pharmaceutiquement acceptable de celui-ci, dans lequel R² et R³ sont hydrogène.

9. Composé selon l'une quelconque des revendications 1 à 8, ou un sel pharmaceutiquement acceptable de celui-ci, qui est un composé selon l'une quelconque des Formules If-1 à If-8 :

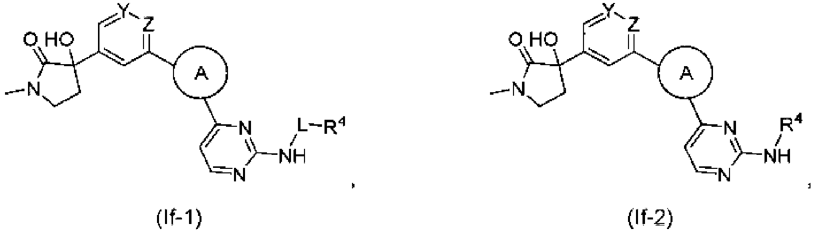

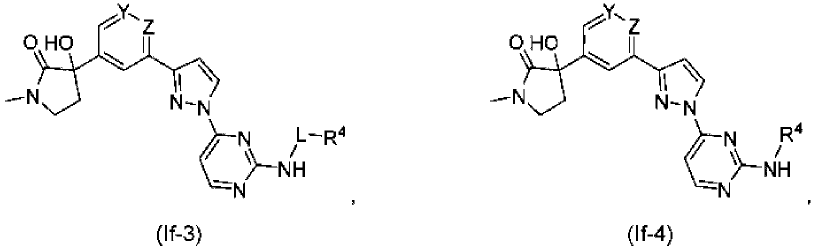

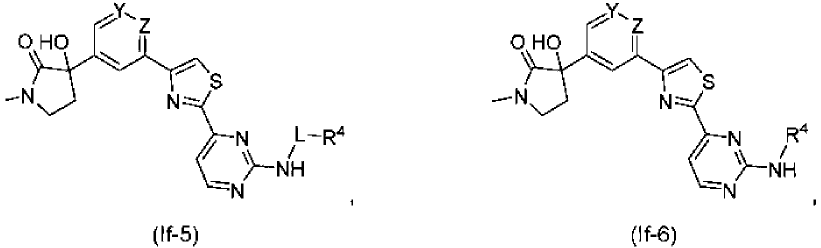

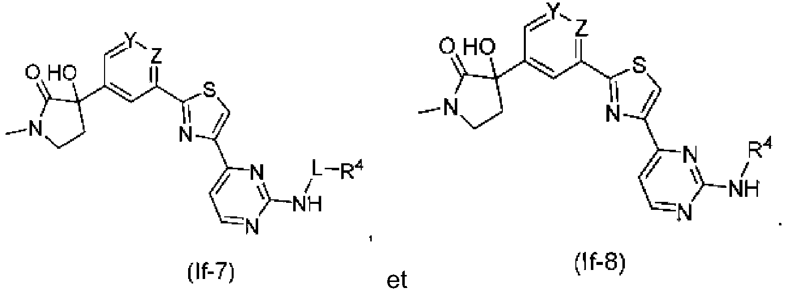

10. Composé selon l'une quelconque des revendications 1 à 9, ou un sel pharmaceutiquement acceptable de celui-ci, dans lequel R⁴ est :

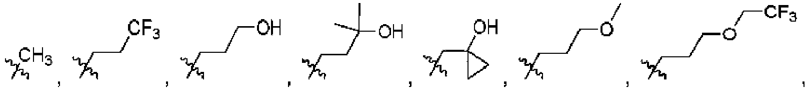

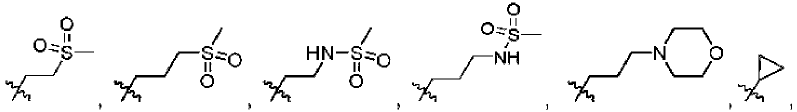

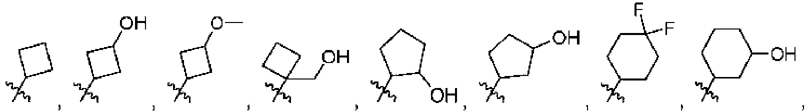

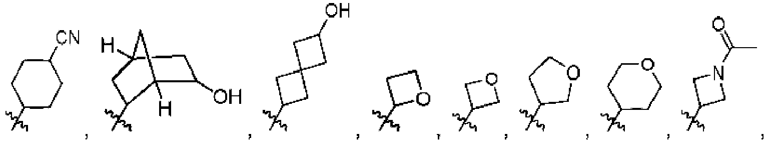

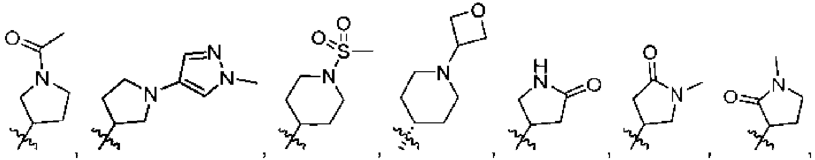

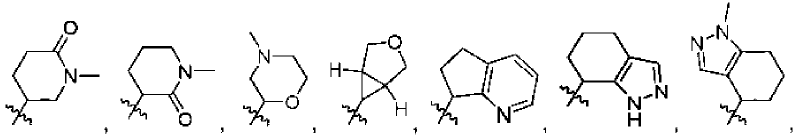

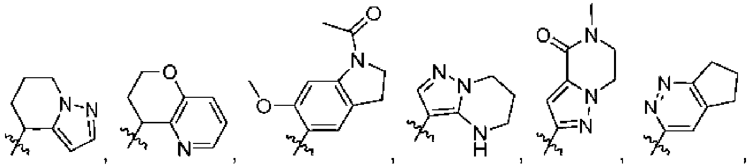

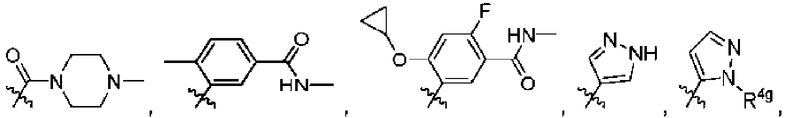

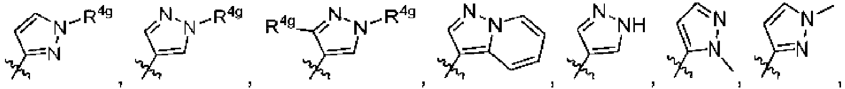

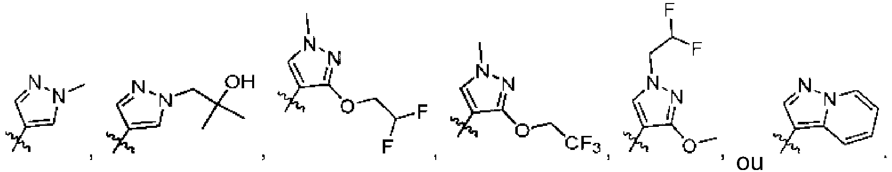

11. Composé selon la revendication 1 ou la revendication 2, ou un sel pharmaceutiquement acceptable de celui-ci, qui est un composé de Formule If-1 : 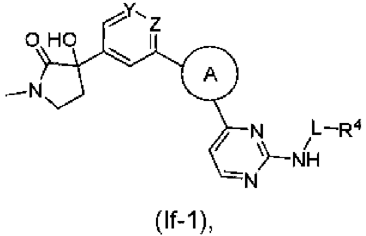
A est un pyrazolyle ou thiazolyle ;
Y est N, C-H, ou C-F ;
Z est N, C-H, ou C-F ;
L est absent ou -C₍₁₋₄₎ alkylène ;
R⁴ est hétéroaryle à 5 à 10 chaînons, qui est facultativement substitué par un à cinq groupes R^{4g} ;
chaque R^{4g} est, indépendamment pour chaque occurrence, -C₍₁₋₆₎alkyle, - C₍₁₋₆₎haloalkyle, -OC₍₁₋₆₎alkyle, ou -OC₍₁₋₆₎haloalkyle, dont chacun est facultativement substitué par un à cinq groupes choisis parmi halo et -OH ;
à condition qu'au moins l'un parmi Y et Z soit C-H.

12. Composé selon la revendication 11, ou un sel pharmaceutiquement acceptable de celui-ci, ayant une structure choisie dans le groupe constitué de :

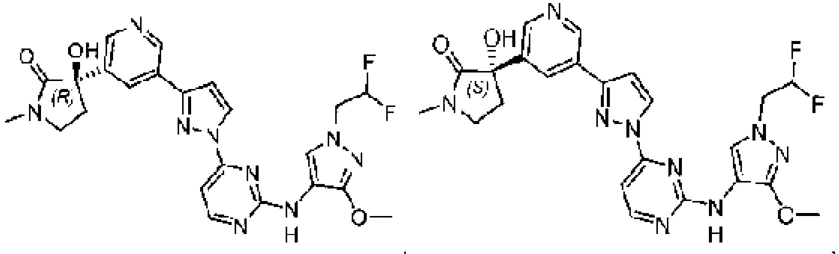

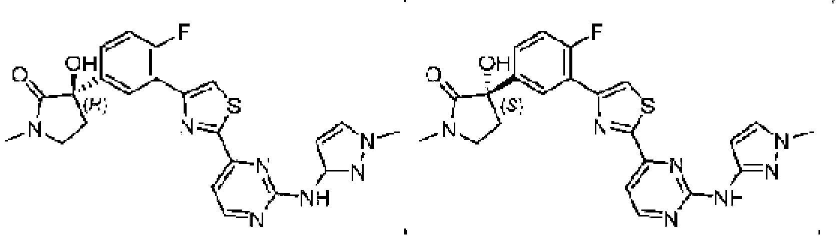

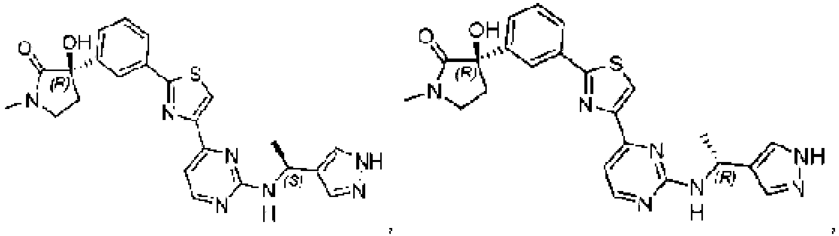

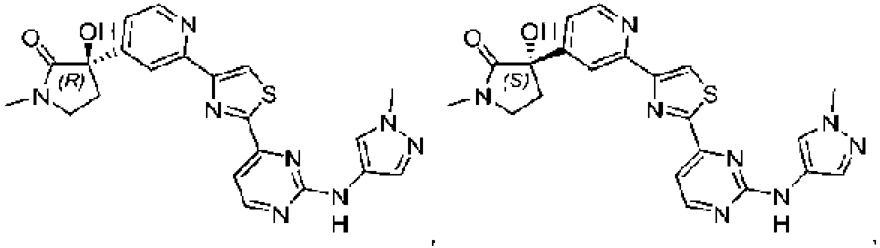

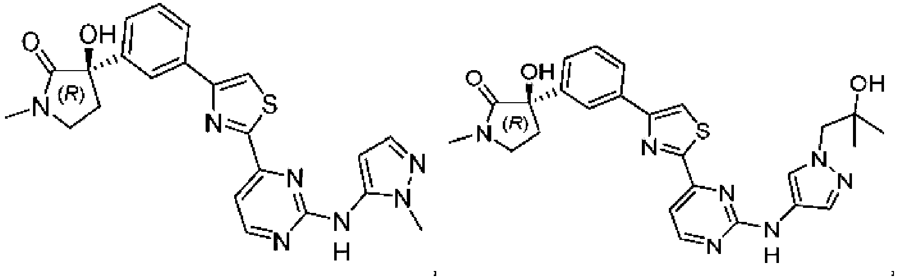

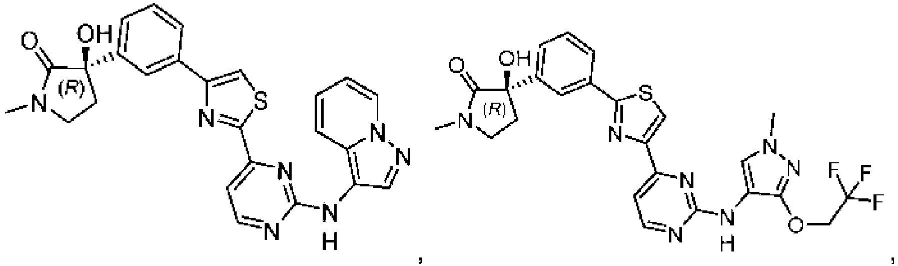

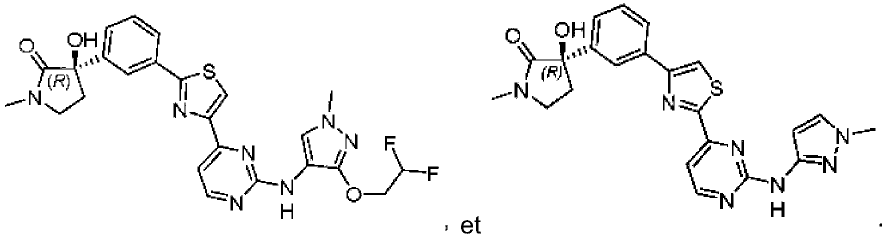

13. Composition pharmaceutique comprenant un composé selon l'une quelconque des revendications 1 à 12, ou un sel pharmaceutiquement acceptable de celui-ci, et un support pharmaceutiquement acceptable.

14. Composé selon l'une quelconque des revendications 1 à 12, ou un support pharmaceutiquement acceptable de celui-ci, ou composition pharmaceutique selon la revendication 13, pour une utilisation dans un procédé de traitement d'une maladie, d'un trouble, ou d'un état médical médié par l'activité NIK, comprenant l'administration à un sujet nécessitant un tel traitement d'une quantité efficace dudit composé ou de ladite composition pharmaceutique.

15. Composé, support pharmaceutiquement acceptable de celui-ci, ou composition pharmaceutique pour une utilisation selon la revendication 14, la maladie, le trouble ou l'état médical médié par l'activité NIK étant choisi dans le groupe constitué de lupus érythémateux disséminé (« LED»), polyarthrite rhumatoïde (« PR »), syndrome de Sjögren, néphrite lupique, maladie inflammatoire chronique de l'intestin (« MICI »), vascularite associée à ANCA, myosite, maladies associées à l'lgG4, pemphigoïde bulleux, troubles du spectre de la neuromyélite optique (« NMOSD »), dermatite atopique (« DA »), hidradénite suppurée (« HS »), stéatose, stéatohépatite non alcoolique (« NASH »), cirrhose biliaire primitive, leucémies, lymphomes, cancer du pancréas, cancer du sein, mélanome, obésité, diabète, lésion rénale aiguë, IgAN, polykystose rénale autosomique dominante (« ADCKD »), néphropathie membraneuse, ostéoporose, résorption osseuse (parodontite), sclérose en plaques (« SEP »), purpura thrombocytopénique immunitaire, transplantation, myasthénie grave, sclérodermie, myosite, maladies associées à l'lgG4, et pemphigoïde bulleux.
